(19) Europäisches Patentamt
European Patent Office
Office européen des brevets

(11) EP 4 032 547 A1

(12) EUROPEAN PATENT APPLICATION

(43) Date of publication:
**27.07.2022 Bulletin 2022/30**

(21) Application number: **21152594.4**

(22) Date of filing: **20.01.2021**

(51) International Patent Classification (IPC):
***A61K 39/245*** *(2006.01)* ***C07K 14/035*** *(2006.01)*

(52) Cooperative Patent Classification (CPC):
**A61K 39/245; A61K 39/12; C07K 14/005;**
A61K 2039/505; C12N 2710/16622;
C12N 2710/16634

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **GlaxoSmithKline Biologicals S.A.**
**1330 Rixensart (BE)**

(72) Inventor: **The designation of the inventor has not yet been filed**

(74) Representative: **West, Heloise**
**GlaxoSmithKline**
**Global Patents (CN925.1)**
**980 Great West Road**
**Brentford, Middlesex TW8 9GS (GB)**

# (54) HSV1 FCE DERIVED FRAGEMENTS FOR THE TREATMENT OF HSV

(57) The present invention relates to a HSV2 Fc receptor or immunogenic fragment or variant thereof for use in generating a cross reactive immune response against HSV1 in a subject. Also provided is a HSV1 Fc receptor or immunogenic fragment or variant thereof for use in generating a cross reactive immune response against HSV2 when administered to a subject.

EP 4 032 547 A1

**Description**

**FIELD OF THE INVENTION**

The present invention relates to a Herpes Simplex Virus (HSV, including HSV1 and HSV2) Fc receptor or an immunogenic fragment or variant thereof for treating a HSV infection in a subject. In particular, the invention provides a HSV2 or HSV1 Fc receptor or an immunogenic fragment or variant thereof that can induce a cross reactive immune response against HSV-1 or HSV-2 in a subject.

**BACKGROUND**

Herpes Simplex Viruses (HSVs, including HSV1 and HSV2) are members of the subfamily *Alphaherpesvirinae (α-herpesvirus)* in the family *Herpesviridae.* They are enveloped, doublestranded DNA viruses containing at least 74 genes encoding functional proteins. HSV1 and HSV2 infect mucosal epithelial cells and establish lifelong persistent infection in sensory neurons innervating the mucosa in which the primary infection had occurred. Both HSV1 and HSV2 can reactivate periodically from latency established in neuronal cell body, leading to either herpes labialis (cold sores) or genital herpes (GH).

The global prevalence of genital herpes is estimated at 417 million in individuals between the ages of 15 and 49, with a disproportionate burden of disease in Africa. HSV1 is approximately as common as HSV2 as the cause of first time genital herpes in resource-rich countries. Recurrent infections are less common after HSV1 than HSV2 genital infections; therefore, HSV2 remains the predominant cause of recurrent genital herpes. Some infected individuals have severe and frequent outbreaks of genital ulcers, while others have mild or subclinical infections, yet all risk transmitting genital herpes to their intimate partners.

Recurrent GH is the consequence of reactivation of HSV2 (and to some extent of HSV1) from the sacral ganglia, followed by an anterograde migration of the viral capsid along the neuron axon leading to viral particles assembly, cell to cell fusion, viral spread and infection of surrounding epithelial cells from the genital mucosa.

Antivirals such as acyclovir; valacyclovir and famciclovir are used for the treatment of GH, both in primary or recurrent infections and regardless of the HSV1 or HSV2 origin. These drugs do not eradicate the virus from the host, as their biological mechanism of action blocks or interferes with the viral replication machinery. Randomized controlled trials demonstrated that short-term therapy with any of these three drugs reduced the severity and duration of symptomatic recurrences by one to two days when started early after the onset of symptoms or clinical signs of recurrence. However, such intermittent regimen does not reduce the number of recurrences per year.

Current treatment options for HSV recurrences have limitations, including incomplete antiviral efficacy, short term efficacy, compliance to treatment regimen, appearance of antiviral resistance, cost of treatment, and side effects. Presently, no strategies that result in long term prevention of symptomatic recurrences are known.

Accordingly, there is a need in the art for improved treatment of recurrent herpes virus infections, in particular HSV2 and HSV1 infections.

**SUMMARY OF THE INVENTION**

Provided herein are the following aspects of the invention:

- a HSV-2 Fc receptor or an immunogenic fragment or variant thereof for use in inducing a cross reactive immune response against HSV-1 when administered to a subject.

- a HSV-1 Fc receptor or an immunogenic fragment or variant thereof for use in inducing a cross reactive immune response against HSV-2 when administered to a subject.

- a pharmaceutical composition comprising a HSV-2 Fc receptor or an immunogenic fragment or variant thereof, a binding partner from HSV-2 or a fragment thereof, and a pharmaceutically acceptable carrier, for use in inducing a cross reactive immune response against HSV-1 when administered to a subject.

- a pharmaceutical composition comprising a HSV-1 Fc receptor or an immunogenic fragment or variant thereof, a binding partner from HSV-1 or a fragment thereof, and a pharmaceutically acceptable carrier, for use in inducing a cross reactive immune response against HSV-2 when administered to a subject.

- a method of treating a herpes virus infection or herpes virus related disease in a subject in need thereof comprising administering an immunologically effective amount of a HSV2 Fc receptor or immunogenic fragment or variant thereof to the subject wherein the HSV2 Fc receptor or immunogenic fragment or variant thereof can induce a cross reactive immune response against HSV 1 when administered to a subject.

- a method of treating a herpes virus infection or herpes virus related disease in a subject in need thereof comprising administering an immunologically effective amount of a HSV1 Fc receptor or immunogenic fragment or variant thereof to the subject wherein the HSV1 Fc receptor or immunogenic fragment or variant thereof can induce a cross reactive immune response against HSV2 when administered to a subject.

## BRIEF DESCRIPTION OF THE DRAWINGS

**Figure 1 - Total HSV-2 gE- or gI-specific IgG antibody titers measured in serum samples collected after immunizations with different mutated versions of AS01-adjuvanted HSV-2 gE/gI protein**. At days 0, 14 & 28, CB6F1 mice (n=6/gr1-5) were intramuscularly (i.m) immunized with different HSV-2 gE/gI mutant candidates adjuvanted with AS01 (5μg/each). An additional group of mice was i.m injected with a saline solution (NaCl 150mM) following the same schedule of immunization and used as negative control group (n=4/gr6). On days 14 (14PI), 28 (14PII) & 42 (14PIII), serum samples were collected to evaluate the total HSV-2 gE- **(Fig 1A)** or gI- **(Fig 1B)** specific IgG antibody titer by ELISA. Each symbol represents individual animal at 14PI (dot), 14PII (diamond) or 14PIII (triangle) while the black bars represents the Geometric mean (GM) of each group. GM and number of animals (N) for each group is indicated on the x axis.

**Figure 2 - Head to head comparison of HSV-2 gE- or gI-specific IgG antibody responses between vaccinated and unvaccinated groups 14 days after the third immunization in CB6F1mice.** At days 0, 14 & 28, CB6F1 mice (n=6/gr1-5) were intramuscularly (i.m) immunized with different HSV-2 gE/gI mutant candidates adjuvanted with AS01 (5μg/each). An additional group of mice was i.m injected with a saline solution (NaCl 150mM) following the same schedule of immunization and used as negative control group (n=4/gr6). On day 42 (14PIII), serum sample were collected to evaluate the total HSV-2 gE- **(Fig 2A)** or gI- **(Fig 2B)** specific IgG antibody titer by ELISA. The black error bar represents the 95% Confidence Interval (CI) of each geometric mean ratio (GMR). The geometric means (GMs) of the two groups compared and GMRs are indicated at the right of the graph.

**Figure 3 - Timepoint comparison of HSV-2 gE- or gI-specific IgG antibody responses in each vaccinated group of CB6F1mice.** At days 0, 14 & 28, CB6F1 mice (n=6/gr1-5) were intramuscularly (i.m) immunized with different HSV-2 gE/gI mutant candidates adjuvanted with AS01 (5μg/each). An additional group of mice was i.m injected with a saline solution (NaCl 150mM) following the same schedule of immunization and used as negative control group (n=4/gr6). On days 14 (14PI), 28 (14PII) & 42 (14PIII), serum samples were collected to evaluate the total HSV-2 gE-specific IgG antibody titer by ELISA. The black error bar represents the 95% Confidence Interval (CI) of each geometric mean ratio (GMR). The geometric means (GMs) of the two time points and GMRs are indicated at the right of the graph. **Fig 3A** illustrates PII over PI & **Fig 3B** illustrates PIII over PII for HSV2 gE-specific response, whereas **Fig 3C** illustrates PII over PI & **Fig 3D** illustrates PIII over PII for HSV2 gI-specific response.

**Figure 4 - Head to head comparison of HSV-2 gE- or gI IgG antibody responses between vaccinated groups 14 days after the third immunization of CB6F1 mice.** At days 0, 14 & 28, CB6F1 mice (n=6/gr1-5) were intramuscularly (i.m) immunized with different HSV-2 gE/gI mutant candidates adjuvanted with AS01 (5μg/each). An additional group of mice was i.m injected with a saline solution (NaCl 150mM) following the same schedule of immunization and used as negative control group (n=4/gr6). On day 42 (14PIII), serum samples were collected to evaluate the total HSV-2 gE- **(Fig 4A)** or gI- **(Fig 4B)** specific IgG antibody titer by ELISA. The black error bar represents the 95% Confidence Interval (CI) of each geometric mean ratio (GMR). The geometric means (GMs) of the two groups compared and GMRs are indicated at the right of the graph.

**Figure 5 - Total HSV-1 gE/gI cross-reactive IgG antibody titers measured in serum samples collected 14 days after the third immunization with different mutated versions of AS01-adjuvanted HSV-2 gE/gI protein.** At days 0, 14 & 28, CB6F1 mice (n=6/gr1-5) were intramuscularly (i.m) immunized with different HSV-2 gE/gI mutant candidates adjuvanted with AS01 (5μg/each). An additional group of mice was i.m injected with a saline solution (NaCl 150mM) following the same schedule of immunization and used as negative control group (n=4/gr6). On day 42 (14PIII), serum samples were collected to evaluate the total HSV-1 gE/gI cross-reactive IgG antibody titer by ELISA. Each symbol represents individual animal at 14PIII while the black bars represents the Geometric mean (GM) of each group. GM and number of animals (N) for each group is indicated on the x axis.

**Figure 6 - Head to head comparison of HSV-1 gE/gI cross-reactive IgG antibody response between vaccinated and unvaccinated groups 14 days after the third immunization in CB6F1mice.** At days 0, 14 & 28, CB6F1 mice (n=6/gr1-5) were intramuscularly (i.m) immunized with different HSV-2 gE/gI mutant candidates adjuvanted with AS01 (5μg/each). An additional group of mice was i.m injected with a saline solution (NaCl 150mM) following the same schedule of immunization and used as negative control group (n=4/gr6). On day 42 (14PIII), serum sample were collected to evaluate the total HSV-1 gE/gI cross-reactive IgG antibody titer by ELISA. The black error bar represents the 95% Confidence Interval (CI) of each geometric mean ratio (GMR). The geometric means (GMs) of the two groups compared and GMRs are indicated at the right of the graph.

**Figure 7 - Head to head comparison of HSV-1 gE/gI cross-reactive antibody responses between vaccinated groups 14 days after the third immunization of CB6F1 mice.** At days 0, 14 & 28, CB6F1 mice (n=6/gr1-5) were intramuscularly (i.m) immunized with different HSV-2 gE/gI mutant candidates adjuvanted with AS01 (5μg/each). An additional group of mice was i.m injected with a saline solution (NaCl 150mM) following the same schedule of immunization and used as negative control group (n=4/gr6). On day 42 (14PIII), serum samples were collected to evaluate the total HSV-1 gE/gI cross-reactive IgG antibody titer by ELISA. The black error bar represents the 95% Confidence Interval (CI) of each geometric mean ratio (GMR). The geometric means (GMs) of the two groups compared and GMRs are indicated at the right of the graph.

**Figure 8 - HSV-2 MS-specific neutralizing antibody titers measured in serum samples collected 14 days after the third immunization with different mutated versions of AS01-adjuvanted HSV-2 gE/gI protein.** At days 0, 14 & 28, CB6F1 mice (n=6/gr1-5) were intramuscularly (i.m) immunized with different HSV-2 gE/gI mutant candidates adjuvanted with AS01 (5μg/each). An additional group of mice was i.m injected with a saline solution (NaCl 150mM) following the same schedule of immunization and used as negative control group (n=4/gr6). On days 42 (14PIII), serum samples were collected to evaluate the titer of neutralizing antibodies towards HSV-2 MS strain (400 $TCID_{50}$). **Fig 8A:** Each dot represents individual animal titer. The positivity threshold value corresponds to the $1^{st}$ sample dilution. Negative samples are illustrated by the $1^{st}$ samples dilution/2 (neutralizing titer = 5). The number of mice by group (N) and the Geometric mean (GM) for each group are indicated below the x axis of the graph. **Fig 8B:** GMR with 95% of confidence interval (CI) of HSV-2-specific neutralizing antibody titers. The black error bar represents the 95% Confidence Interval (CI) of each geometric mean ratio (GMR). The GMs of the two groups compared and GMRs are indicated at the right of the graph.

**Figure 9 - HSV-1 cross-reactive neutralizing antibody titers measured in serum samples collected 14 days after the third immunization with different mutated versions of AS01-adjuvanted HSV-2 gE/gI heterodimer protein.** At days 0, 14 & 28, CB6F1 mice (n=6/gr1-5) were intramuscularly (i.m) immunized with different HSV-2 gE/gI mutant candidates adjuvanted with AS01 (5μg/each). An additional group of mice was i.m injected with a saline solution (NaCl 150mM) following the same schedule of immunization and used as negative control group (n=4/gr6). On days 42 (14PIII), serum samples were collected to evaluate the titer of cross-neutralizing antibodies towards HSV-1 VR1789 strain (400 $TCID_{50}$). **Fig 9A:** Each dot represents individual animal titer. The positivity threshold value corresponds to the $1^{st}$ sample dilution. Negative samples are illustrated by the $1^{st}$ samples dilution/2 (neutralizing titer = 5). The number of mice by group (N) and the Geometric mean (GM) for each group are indicated below the x axis of the graph. **Fig 9B:** GMR with 95% of confidence interval (CI) of HSV-1 cross-reactive neutralizing antibody titers. The black error bar represents the 95% Confidence Interval (CI) of each geometric mean ratio (GMR). The GMs of the two groups compared and GMRs are indicated at the right of the graph.

**Figure 10 - Evaluation of the ability of vaccine-specific antibodies to decrease, in-vitro, human IgG Fc binding by HSV-2 gE/gI antigen 14 days after third immunizations with different mutated versions of AS01-adjuvanted HSV-2 gE/gI protein.** At days 0, 14 & 28, CB6F1 mice (n=6/gr1-5) were intramuscularly (i.m) immunized with different HSV-2 gE/gI mutant candidates adjuvanted with AS01 (5μg/each). An additional group of mice was i.m injected with a saline solution (NaCl 150mM) following the same schedule of immunization and used as negative control group (n=4/gr6). On days 42 (14PIII), serum samples were collected to evaluate the ability of vaccine-specific antibodies to compete and decrease hIgG Fc binding by HSV-2 gE/gI protein. Each curve represents individual mice data. **Fig 10A:** AS01/HSV-2 gE/gI V340W over NaCl; **Fig 10B:** AS01/HSV-2 gE/gI A248T over NaCl; **Fig 10C:** AS01/HSV-2 gE/gI A246W over NaCl; **Fig 10D:** AS01/HSV-2 gE/gI P318I over NaCl; **Fig10E:** AS01/HSV-2 gE/gI A248T_V340W over NaCl.

**Figure 11 - Comparison of the ability of vaccine-specific antibodies to decrease, in-vitro, human IgG Fc binding by HSV-2 gE/gI antigen 14 days after third immunizations with different mutated versions of AS01-adjuvanted HSV-2 gE/gI protein.** At days 0, 14 & 28, CB6F1 mice (n=6/gr1-5) were intramuscularly (i.m) immunized with different HSV-2 gE/gI mutant proteins adjuvanted in AS01 (5μg/each). An additional group of mice was i.m injected with a saline solution (NaCl 150mM) following the same schedule of immunization and used as negative control group (n=4/gr6). On days 42 (14PIII), serum samples were collected to evaluate the ability of vaccine-specific antibodies to compete and decrease hIgG Fc binding by HSV-2 gE/gI protein. **Fig 11A:** Each dot represents ED50 titer with 95% CIs from individual mice. The positivity threshold value corresponds to the 1st sample dilution. Negative samples are illustrated by the 1st samples dilution/2. The number of mice by group (N) and the Geometric mean (GM) for each group is indicated below the x axis of the graph. **Fig 11B:** GMR with 95% of confidence interval (CI). The black error bar represents the 95% Confidence Interval (CI) of each geometric mean ratio (GMR). The GMs of the two groups compared and GMRs are indicated at the right of the graph.

**Figure 12 - Evaluation of the ability of HSV-2 gE/gI antibodies to bind and activate mFcγRIII after incubation with HSV-2 gE/gI positive cells 14 days after third immunizations with different mutated versions of AS01-adjuvanted HSV-2 gE/gI protein.** At days 0, 14 & 28, CB6F1 mice (n=6/gr1-5) were intramuscularly (i.m) immunized with different HSV-2 gE/gI mutant candidates adjuvanted with AS01 (5μg/each). An additional group of mice was i.m injected with a saline solution (NaCl 150mM) following the same schedule of immunization and used as negative control group (n=4/gr6). On days 42 (14PIII), serum samples were collected to evaluate the ability of vaccine-specific antibodies to bind HSV-2 gE/gI positive 3T3 cells and activate the mouse $F_c$γRIII expressed by modified Jurkat reporter cells. **Fig 12A-E** each curve illustrate data from pools of 2 mouse sera immunized with different AS01-HSV-2 gE/gI mutants over NaCl while **Fig 12F** illustrates Geoemetric mean of each AS01-HSV-2 gE/gI vaccinated group over NaCl.

**Figure 13 - Percentage of vaccine-specific CD4+/CD8+T cell response induced in CB6F1 mice 14 days after third immunizations with different mutated versions of AS01-adjuvanted HSV-2 gE/gI protein.** At days 0, 14 & 28, CB6F1 mice (n=6/gr1-5) were intramuscularly (i.m) immunized with different HSV-2 gE/gI mutant candidates adjuvanted with AS01 (5μg/each). An additional group of mice was i.m injected with a saline solution (NaCl 150mM) following the same schedule of immunization and used as negative control group (n=4/gr6). On days 42 (14PIII), serum sample were collected to evaluate vaccine-specific CD4+/CD8+T cell response in the spleen. Splenocytes were stimulated *ex-vivo* during 6 hours with pools of 15mer peptides covering the whole amino acids sequence of the gE or gI protein from HSV-2. β-actin peptides pool stimulation was used to evaluate non-specific response. The frequencies of CD4+/CD8+ T cells secreting IL-2, IFN-γ and/or TNF-α were measured by intracellular cytokine staining. Circle, triangle and diamond shapes represent individual % of CD4+ **(Fig 13A)** /CD8+ **(Fig 13B)** T cell response detected for each antigen (HSV-2 gE or HSV-2 gI antigens, or human β-actin). Black line represents the geometric mean (GM) of the response and black dotted line indicates the percentile 95th (P95) obtained with all the stimulations in the saline group. The number of animals/group with valid result (N) and GM of each group are indicated under the graph.

**Figure 14 - Head to head comparison of % of vaccine-specific CD4+/CD8+T cell responses between vaccinated and unvaccinated groups 14 days after third immunizations in CB6F1mice.** At days 0, 14 & 28, CB6F1 mice (n=6/gr1-5) were intramuscularly (i.m) immunized with different HSV-2 gE/gI mutant candidates adjuvanted with AS01 (5μg/each). An additional group of mice was i.m injected with a saline solution (NaCl 150mM) following the same schedule of immunization and used as negative control group (n=4/gr6). On days 42 (14PIII) mice were culled to evaluate vaccine-specific CD4+/CD8+T cell response in the spleen. The black error bar represents the 95% Confidence Interval (CI) of each geometric mean ratio (GMR). The geometric means (GMs) of the two groups compared and GMRs are indicated at the right of the graph. **Fig 14A:** GMRs with 95%CIs of HSV-2 gE CD4+ T cells; **Fig 14B:** GMRs with 95%CIs of HSV-2 gI specific CD4+ T cells; **Fig 14C :** GMRs with 95%CIs of HSV-2 gE specific CD8+ T cells.

**Figure 15 - Head to head comparison of % of vaccine-specific CD4+/CD8+T cell responses between vaccinated groups 14 days after the third immunization of CB6F1 mice.** At days 0, 14 & 28, CB6F1 mice (n=6/gr1-5) were intramuscularly (i.m) immunized with different HSV-2 gE/gI mutant candidates adjuvanted with AS01 (5μg/each). An additional group of mice was i.m injected with a saline solution (NaCl 150mM) following the same schedule of immunization and used as negative control group (n=4/gr6). On days 42 (14PIII) mice were culled to evaluate vaccine-specific CD4/CD8+T cell response in the spleen. The black error bar represents the 95% Confidence Interval (CI) of each geometric mean ratio (GMR). The geometric means (GMs) of the two groups compared and GMRs are indicated at the right of the graph. **Fig 15A:** GMRs with 95%CIs of HSV-2 gE-specific CD4+ T cells; **Fig 15B:** GMRs with 95% CIs of HSV-2 gI-specific CD4+ T cells; **Fig 15C:** GMRs with 95%CIs of HSV-2 gE- specific CD8+ T cells.

**Figure 16 - Total HSV-2 gE- or gI-specific IgG antibody titers measured in serum samples collected after one, two or three immunizations with different mutated versions of SAM HSV-2 gE/gI vector formulated in Lipid nanoparticles (LNP).** At days 0, 21 & 42, CB6F1 mice (n=6/gr1-6) were intramuscularly (i.m) immunized with 0.8μg of different mutated versions of SAM-HSV-2 gE/gI vector formulated in Lipid nanoparticles (LNP). An additional group of mice was i.m injected with a saline solution (NaCl 150mM) following the same schedule of immunization and used as negative control group (n=4/gr7). On days 21 (21PI), 42 (21PII) & 63 (21PIII), serum sample from each animal was collected to evaluate the total HSV-2 gE- **(Fig 16A)** or gI- **(Fig 16B)** specific IgG antibody titer by ELISA. Each symbol represents individual animal at 21PI (dot), 21PII (triangle) or 21PIII (diamond) while the black bars represents the Geometric mean (GM) of each group. GM and number of animals (N) for each group is indicated on the x axis.

**Figure 17 - Head to head comparison of HSV-2 gE- or gI-specific IgG antibody responses between vaccinated and unvaccinated groups 21days after the third immunization in CB6F1mice.** At days 0, 21 & 42, CB6F1 mice (n=6/gr1-6) were intramuscularly (i.m) immunized with 0.8μg of different mutated versions of SAM-HSV-2 gE/gI vector formulated in Lipid nanoparticles (LNP). An additional group of mice was i.m injected with a saline solution (NaCl 150mM) following the same schedule of immunization and used as negative control group (n=4/gr7). On day 63 (21PIII), serum sample from each animal was collected to evaluate the total HSV-2 gE-(fig2A) or gI- (fig2B) specific IgG antibody titer by ELISA. The black error bar represents the 95% Confidence Interval (CI) of each geometric mean ratio (GMR). The geometric means (GMs) of the two groups compared and GMRs are indicated at the right of the graph. **Fig 17A:** GMR with 95%CIs of HSV-2 gE-specific antibody titers (EU/mL); **Fig 17B:** GMR with 95%CIs of HSV-2 gI-specific antibody titers (EU/mL).

**Figure 18 - Timepoint comparison of HSV-2 gE- or gI-specific IgG antibody responses between each LNP-SAM HSV-2 gE/gI vaccinated group of CB6F1mice.** At days 0, 21 & 42, CB6F1 mice (n=6/gr1-6) were intramuscularly (i.m) immunized with 0.8μg of different mutated versions of SAM-HSV-2 gE/gI vector formulated in Lipid nanoparticles (LNP). An additional group of mice was i.m injected with a saline solution (NaCl150mM) following the same schedule of immunization and used as negative control group (n=4/gr7). On days 21 (21PI), 42 (21PII) & 63 (21PIII), serum sample from each animal was collected to evaluate the total HSV-2 gE-or gI-specific IgG antibody titer by ELISA. The black error bar represents the 95% Confidence Interval (CI) of each geometric mean ratio (GMR). The geometric means (GMs) of the two time points and GMRs are indicated at the right of the graph. **Fig 18A** illustrates PII over PI & **Fig 18B** illustrates PIII over PII for HSV-2 gE-specific response, whereas **Fig 18C** illustrates PII over PI & **Fig 18D** illustrates PIII over PII for HSV-2 gI-specific response.

**Figure 19 - Head to head comparison of HSV-2 gE- or gI- IgG antibody responses between vaccinated groups 21 days after the third immunization of CB6F1 mice.** At days 0, 21 & 42, CB6F1 mice (n=6/gr1-6) were intramuscularly (i.m) immunized with 0.8μg of different mutated versions of SAM-HSV-2 gE/gI vector formulated in Lipid nanoparticles (LNP). An additional group of mice was i.m injected with a saline solution (NaCl 150mM) following the same schedule of immunization and used as negative control group (n=4/gr7). On day 63 (21PIII), serum sample from each animal was collected to evaluate the total HSV-2 gE- **(Fig 19A)** or gI- **(Fig 19B)** specific IgG antibody titer by ELISA. The black error bar represents the 95% Confidence Interval (CI) of each geometric mean ratio (GMR). The geometric means (GMs) of the two groups compared and GMRs are indicated at the right of the graph.

**Figure 20 - Total HSV-1 gE/gI cross-reactive IgG antibody titers measured in serum samples collected 21 days after the third immunization with different SAM HSV-2 gE/gI mutants formulated in Lipid nanoparticles (LNP).** At days 0, 21 & 42, CB6F1 mice (n=6/gr1-6) were intramuscularly (i.m) immunized with 0.8μg of different mutated versions of SAM-HSV-2 gE/gI vector formulated in Lipid nanoparticles (LNP). An additional group of mice was i.m injected with a saline solution (NaCl 150mM) following the same schedule of immunization and used as negative control group (n=4/gr7). On day 63 (21PIII), serum sample from each animal was collected to evaluate the total HSV-1 gE/gI cross-reactive IgG antibody titer by ELISA. Each symbol represents individual animal at 21PIII while the black bars represents the Geometric mean (GM) of each group. GM and number of animals (N) for each group is indicated on the x axis.

**Figure 21 - Head to head comparison of HSV-1 gE/gI cross-reactive IgG antibody response between vaccinated and unvaccinated groups 21 days after the third immunization in CB6F1mice.** At days 0, 21 & 42, CB6F1 mice (n=6/gr1-6) were intramuscularly (i.m) immunized with 0.8μg of different mutated versions of SAM-HSV-2 gE/gI vector formulated in Lipid nanoparticles (LNP). An additional group of mice was i.m injected with a saline solution (NaCl 150mM) following the same schedule of immunization and used as negative control group (n=4/gr7). On day 63 (21PIII), serum sample from each animal was collected to evaluate the total HSV-1 gE/gI cross-reactive IgG antibody titer by ELISA. The black error bar represents the 95% Confidence Interval (CI) of each geometric mean ratio (GMR). The geometric means (GMs) of the two groups compared and GMRs are indicated at the right of the graph.

**Figure 22 - Head to head comparison of HSV-1 gE/gI cross-reactive IgG antibody response between vaccinated groups 21 days after the third immunization of CB6F1 mice.** At days 0, 21 & 42, CB6F1 mice (n=6/gr1-6) were intramuscularly (i.m) immunized with 0.8μg of different mutated versions of SAM-HSV-2 gE/gI vector formulated in Lipid nanoparticles (LNP). An additional group of mice was i.m injected with a saline solution (NaCl 150mM) following the same schedule of immunization and used as negative control group (n=4/gr7). On day 63 (21PIII), serum sample from each animal was collected to evaluate the total HSV-1 gE/gI cross-reactive IgG antibody titer by ELISA. The black error bar represents the 95% Confidence Interval (CI) of each geometric mean ratio (GMR). The geometric means (GMs) of the two groups compared and GMRs are indicated at the right of the graph.

**Figure 23 - HSV-2 MS-specific neutralizing antibody titer measured in serum samples collected 21 days after the third immunization with different LNP-formulated SAM-HSV-2 gE/gI mutants.** At days 0, 21 & 42, CB6F1 mice (n=6/gr1-6) were intramuscularly (i.m) immunized with 0.8μg of different mutated versions of SAM-HSV-2 gE/gI vector formulated in Lipid nanoparticles (LNP). An additional group of mice was i.m injected with a saline solution (NaCl 150mM) following the same schedule of immunization and used as negative control group (n=4/gr7). On days 63 (21PIII), serum sample from each animal was collected to evaluate the titer of neutralizing antibodies towards HSV-2 MS strain (400 $TCID_{50}$). **Fig 23A:** Each symbol represents individual animal titer while each bar represents Geomean (GM) + 95% Confidence intervals (CIs). The positivity threshold value corresponds to the $1^{st}$ sample dilution. Negative samples are illustrated by the $1^{st}$ samples dilution/2 (neutralizing titer = 5). The number of mice by group (N) and the GM for each group are indicated below the x axis of the graph. **Fig 23B:** The black error bar represents the 95% Confidence Interval (CI) of each geometric mean ratio (GMR). The GMs of the two groups compared and GMRs are indicated at the right of the graph.

**Figure 24 - HSV-1 cross-reactive neutralizing antibody titer measured in serum samples collected 21 days after the third immunization with different LNP-formulated SAM-HSV-2 gE/gI mutants.** At days 0, 21 & 42, CB6F1 mice (n=6/gr1-6) were intramuscularly (i.m) immunized with 0.8μg of different mutated versions of SAM-HSV-2 gE/gI vector formulated in Lipid nanoparticles (LNP). An additional group of mice was i.m injected with a saline solution (NaCl 150mM) following the same schedule of immunization and used as negative control group (n=4/gr7). On days 63 (21PIII), serum sample from each animal was collected to evaluate the titer of neutralizing antibodies towards HSV-1 VR1789 strain (400 $TCID_{50}$). **Fig 24A:** Each symbol represents individual animal titer while each bar represents Geomean (GM) + 95% Confidence intervals (CIs). The positivity threshold value corresponds to the $1^{st}$ sample dilution. Negative samples are illustrated by the $1^{st}$ samples dilution/2 (neutralizing titer = 5). The number of mice by group (N) and the GM for each group are indicated below the x axis of the graph. **Fig 24B:** The black error bar represents the 95% Confidence Interval (CI) of each geometric mean ratio (GMR). The GMs of the two groups compared and GMRs are indicated at the right of the graph.

**Figure 25 - Evaluation of the ability of vaccine-specific antibodies to decrease, in-vitro, hIgG Fc binding by HSV-2 gE/gI antigen 21 days after the third immunization with different LNP-formulated SAM-HSV-2 gE/gI mutants.** At days 0, 21 & 42, CB6F1 mice (n=6/gr1-6) were intramuscularly (i.m) immunized with 0.8μg of different mutated versions of SAM-HSV-2 gE/gI vector formulated in Lipid nanoparticles (LNP). An additional group of mice was i.m injected with a saline solution (NaCl 150mM) following the same schedule of immunization and used as negative control group (n=4/gr7). On days 63 (21PIII), serum sample from each animal was collected to evaluate the ability of vaccine-specific antibodies to compete and decrease hIgG Fc binding by HSV-2 gE/gI protein. Each curve represents individual mice. LNP/SAM-HSV-2 gE/gI V340W over NaCl group **(Fig 25A);** LNP/SAM-HSV-2 gE/gI A248T over NaCl group **(Fig 25B);** LNP/SAM-HSV-2 gE/gI A246W over NaCl group **(Fig 25C);** LNP/SAM-HSV-2 gE/gI P318I over NaCl group **(Fig 25D);** LNP/SAM-HSV-2 gE/gI A248T_V340W over NaCl group **(Fig 25E);** LNP/SAM-HSV-2 gE/gI insert ARAA over NaCl group; **(Fig 25F).**

**Figure 26 - Comparison of the ability of vaccine-specific antibodies to decrease, in-vitro, human IgG Fc binding by HSV-2 gE/gI antigen 21 days after third immunizations with different LNP-formulated SAM-HSV-2 gE/gI mutants in CB6F1 mice.** At days 0, 21 & 42, CB6F1 mice (n=6/gr1-6) were intramuscularly (i.m) immunized with 0.8μg of different mutated versions of SAM-HSV-2 gE/gI vector formulated in Lipid nanoparticles (LNP). An additional group of mice was i.m injected with a saline solution (NaCl 150mM) following the same schedule of immunization and used as negative control group (n=4/gr7). On days 63 (21PIII), serum sample from each animal was collected to evaluate the ability of vaccine-specific antibodies to compete and decrease hIgG Fc binding by HSV-2 gE/gI protein. Each dot represents ED50 titer with 95% CIs from individual mice. The positivity threshold value corresponds to the 1st sample dilution. Negative samples are illustrated by the 1st samples dilution/2 (titer =5). The number of mice by group (N) and the Geometric mean (GM) for each group is indicated below the x axis of the graph.

**Figure 27 - Evaluation of the ability of HSV-2 gE/gI antibodies to bind and activate mFcγRIII after incubation with HSV-2 gE/gI positive cells 21 days after three immunizations with different mutated versions of LNP-formulated SAM-HSV-2 gE/gI protein.** At days 0, 21 & 42, CB6F1 mice (n=6/gr1-6) were intramuscularly (i.m) immunized with 0.8μg of different mutated versions of SAM-HSV-2 gE/gI vector formulated in Lipid nanoparticles (LNP). An additional group of mice was i.m injected with a saline solution (NaCl 150mM) following the same schedule of immunization and used as negative control group (n=4/gr7). On days 63 (21PIII), serum samples were collected to evaluate the ability of vaccine-specific antibodies to bind HSV-2 gE/gI positive 3T3 cells and activate the mouse $F_c\gamma$RIII expressed by modified Jurkat reporter cells. **Fig 27A-F:** each curve illustrates pools of 2 mouse sera immunized with different LNP-SAM HSV-2 gE/gI mutants over NaCl while **Fig 27G:** illustrates Geoemetric mean of each LNP-SAM HSV-2 gE/gI vaccinated group over NaCl.

**Figure 28 - Percentage of vaccine-specific or HSV-1 gE cross reactive CD4+/CD8+T cell responses induced in CB6F1 mice 21 days after third immunization with different SAM HSV-2 gE/gI mutants formulated in Lipid nanoparticles (LNP).** At days 0, 21 & 42, CB6F1 mice (n=6/gr1-6) were intramuscularly (i.m) immunized with 0.8μg of different mutated versions of SAM HSV-2 gE/gI vector formulated in Lipid nanoparticles (LNP). An additional group of mice was i.m injected with a saline solution (NaCl 150mM) following the same schedule of immunization and used as negative control group (n=4/gr7). On days 63 (21PIII) mice were culled to evaluate HSV-2 gE and HSV-2 gI-specific or HSV-1 gE cross reactive CD4+/CD8+T cell responses in the spleen. Splenocytes were stimulated *ex-vivo* during 6 hours with pools of 15mer peptides covering the whole amino acids sequence of the gE or gI protein from HSV-2, as well as gE protein from HSV-1. Human β-actin peptides pool stimulation was used to evaluate non-specific response. The frequencies of CD4+ **(Fig 28A)** /CD8+ **(Fig28B)** T cells secreting IL-2, IFN-γ and/or TNF-α were measured by intracellular cytokine staining. Circle, triangle, square and diamond shapes represent individual % of CD4+/CD8+ T cell responses detected for each antigen (HSV-1 or 2 gE or HSV-2 gI antigens, or human β-actin). Black line represents the geometric means (GM) of the response and black dotted line indicates the percentile 95th (P95) obtained in the saline group when combining the four antigens (HSV-1 or 2 gE or HSV-2 gI antigens or Human β-actin). The number of animals/group with valid result (N) and the GM of each group are indicated under the graph.

**Figure 29 - Head to head comparison of vaccine-specific CD4+T cell response between vaccinated and unvaccinated groups 21days after third immunization in CB6F1 mice.** At days 0, 21 & 42, CB6F1 mice (n=6/gr1-6) were intramuscularly (i.m) immunized with 0.8μg of different mutated versions of SAM HSV-2 gE/gI vector formulated in Lipid nanoparticles (LNP). An additional group of mice was i.m injected with a saline solution (NaCl 150mM) following the same schedule of immunization and used as negative control group (n=4/gr7). On days 63 (21PIII) mice were culled to evaluate HSV-2 gE **(Fig 29A)** & HSV-2 gI **(Fig 29B)**-specific CD4+T cell responses in the spleen. The black error bar represents the 95% Confidence Interval (CI) of each the geometric mean ratio (GMR). The geometric means (GMs) of the two groups compared and GMRs are indicated at the right of the graph.

**Figure 30 - Head to head comparison of HSV-2 gE-specific or HSV-1 gE cross reactive CD8+T cell response between vaccinated and unvaccinated groups 21 days after third immunization in CB6F1 mice.** At days 0, 21 & 42, CB6F1 mice (n=6/gr1-6) were intramuscularly (i.m) immunized with 0.8μg of different mutated versions of SAM HSV-2 gE/gI vector formulated in Lipid nanoparticles (LNP). An additional group of mice was i.m injected with a saline solution (NaCl 150mM) following the same schedule of immunization and used as negative control group (n=4/gr7). On days 63 (21PIII), mice were culled to evaluate HSV-2 gE-specific **(Fig 30A)** or HSV-1 gE cross reactive **(Fig 30B)** CD8+T cell responses in the spleen. The black error bar represents the 95% Confidence Interval (CI) of each geometric mean ratio (GMR). The geometric means (GMs) of the two groups compared and GMRs are indicated at the right of the graph.

**Figure 31 - Head to head comparison of vaccine-specific CD4+T cell response between vaccinated groups 21 days after third immunization in CB6F1 mice.** At days 0, 21 & 42, CB6F1 mice (n=6/gr1-6) were intramuscularly (i.m) immunized with 0,8μg of different mutated versions of SAM HSV-2 gE/gI vector formulated in Lipid nanoparticles (LNP). An additional group of mice was i.m injected with a saline solution (NaCl150mM) following the same schedule of immunization and used as negative control group (n=4/gr7). On days 63 (21PIII) mice were culled to evaluate HSV-2 gE- **(Fig 31A)** & HSV-2 gI- **(Fig 31B)** specific CD4+T cell responses in the spleen. The black error bar represents the 95% Confidence Interval (CI) of each geometric mean ratio (GMR). The geometric means (GMs) of the two groups compared and GMRs are indicated at the right of the graph.

**Figure 32 - Head to head comparison of HSV-2 gE-specific or HSV-1 gE cross reactive CD8+T cell response between vaccinated groups 21 days after third immunization in CB6F1 mice.** At days 0, 21 & 42, CB6F1 mice (n=6/gr1-6) were intramuscularly (i.m) immunized with 0,8μg of different mutated versions of SAM HSV-2 gE/gI vector formulated in Lipid nanoparticles (LNP). An additional group of mice was i.m injected with a saline solution (NaCl 150mM) following the same schedule of immunization and used as negative control group (n=4/gr7 On days 63 (21PIII), mice were culled to evaluate HSV-2 gE-specific **(Fig 32A)** or HSV-1 gE cross reactive **(Fig 32B)** CD8+T cell responses in the spleen. The black error bar represents the 95% Confidence Interval (CI) of each geometric mean ratio (GMR). The geometric means (GMs) of the two groups compared and GMRs are indicated at the right of the graph.

**Figure 33 - Anti-HSV-1-specific gE/gI IgG antibody response measured in serum samples after immunizations with different versions of AS01-adjuvanted HSV-1 gE/gI protein.** At days 0, 14 & 28, CB6F1 mice (n=6/gr1-6) were intramuscularly (i.m) immunized with different versions of HSV-1 gE/gI protein adjuvanted with AS01 (5μg/each). An additional group of mice was i.m injected with a saline solution (NaCl 150mM) following the same schedule of immunization and used as negative control group (n=4/gr7). On days 13 (13PI), 27 (13PII) & 42 (14PIII), serum sample from each animal was collected to evaluate the total anti-HSV-1 gE/gI-specific IgG antibody titer by ELISA. Each shape represents individual animal at different timepoints (dot = 13PI; triangle = 13PII; diamond = 14PIII) while the black bars represents the Geometric mean of each group. Geometric mean (GM) and number of animals (N) for each group is indicated on the x axis.

**Figure 34 - Head to head comparison of anti-HSV-1-specific gE/gI-specific IgG antibody response between vaccinated and unvaccinated groups 14 days after the third immunization in CB6F1mice.** At days 0, 14 & 28, CB6F1 mice (n=6/gr1-6) were intramuscularly (i.m) immunized with different versions of HSV-1 gE/gI protein adjuvanted with AS01 (5μg/each). An additional group of mice was i.m injected with saline solution (NaCl 150mM) following the same schedule of immunization and used as negative control group (n=4/gr7). On days 42 (14PIII), serum sample from each animal was collected to evaluate the total anti-HSV-1 gE/gI-specific IgG antibody titer by ELISA. The black error bar represents the 95% Confidence Interval (CI) of each geometric mean ratio (GMR). The geometric means (GMs) of the two groups compared and GMRs are indicated at the right of the graph.

**Figure 35 - Time point comparison of anti--HSV-1 gE/gI-specific IgG antibody response in each vaccinated group of CB6F1 mice.** At days 0, 14 & 28, CB6F1 mice (n=6/gr1-6) were intramuscularly (i.m) immunized with different versions of HSV-1 gE/gI protein adjuvanted with AS01 (5μg/each). An additional group of mice was i.m injected with saline solution (NaCl 150mM) following the same schedule of immunization and used as negative control group (n=4/gr7). On days 13 (13PI), 27 (13PII) & 42 (14PIII), serum sample from each animal was collected to evaluate the total anti-HSV-1 gE/gI-specific IgG antibody titer by ELISA. The black error bar represents the 95% Confidence Interval (CI) of each geometric mean ratio (GMR). The geometric means (GMs) of the two time points and GMRs are indicated at the right of the graph. **Fig 35A** = PII over PI & **Fig 35B** = PIII over PII.

**Figure 36 - Head to head comparison of anti-HSV-1 gE/gI-specific IgG antibody response between vaccinated groups 14 days after the third immunization in CB6F1mice.** At days 0, 14 & 28, CB6F1 mice (n=6/gr1-6) were intramuscularly (i.m) immunized with different versions of HSV-1 gE/gI protein) adjuvanted with AS01 (5μg/each). An additional group of mice was i.m injected with a saline solution (NaCl 150mM) following the same schedule of immunization and used as negative control group (n=4/gr7). On days 42 (14PIII), serum sample from each animal was collected to evaluate the total anti-HSV-1 gE/gI- specific IgG antibody titer by ELISA. The black error bar represents the 95% Confidence Interval (CI) of each geometric mean ratio (GMR). The geometric means (GMs) of the two groups compared and GMRs are indicated at the right of the graph.

**Figure 37 - Anti-HSV-2 gE or HSV-2 gI cross-reactive IgG antibody response measured in serum samples after immunizations with different versions of AS01-adjuvanted HSV-1 gE/gI protein.** At days 0, 14 & 28, CB6F1 mice (n=6/gr1-6) were intramuscularly (i.m) immunized with different versions of HSV-1 gE/gI protein adjuvanted with AS01 (5μg/each). An additional group of mice was i.m injected with a saline solution (NaCl 150mM) following the same schedule of immunization and used as negative control group (n=4/gr7). On day 42 (14PIII), serum sample from each animal was collected to evaluate the total anti-HSV-2 gE **(Fig 37A)** or HSV-2 gI **(Fig 37B)** cross-reactive IgG antibody titer by ELISA. Each shape represents individual animal at timepoint 14PIII while the black bars represents the Geometric mean of each group. Geometric mean (GM) and number of animals (N) for each group is indicated on the x axis.

**Figure 38 - Head to head comparison of anti-HSV-2 gE or HSV-2 gI cross-reactive IgG antibody response between vaccinated and unvaccinated groups 14 days after the third immunization in CB6F1mice.** At days 0, 14 & 28, CB6F1 mice (n=6/gr1-6) were intramuscularly (i.m) immunized with different versions of HSV-1 gE/gI protein adjuvanted with AS01 (5μg/each). An additional group of mice was i.m injected with saline solution (NaCl 150mM) following the same schedule of immunization and used as negative control group (n=4/gr7). On days 42 (14PIII), serum sample from each animal was collected to evaluate the total anti-HSV-2 gE **(Fig 38A)** or HSV-2 gI **(Fig 38B)** cross-reactive IgG antibody titer by ELISA. The black error bar represents the 95% Confidence Interval (CI) of each geometric mean ratio (GMR). The geometric means (GMs) of the two groups compared and GMRs are indicated at the right of the graph.

**Figure 39 - Head to head comparison of anti-HSV-2 gE or HSV-2 gI cross-reactive IgG antibody response between vaccinated groups 14 days after the third immunization in CB6F1mice.** At days 0, 14 & 28, CB6F1 mice (n=6/gr1-6) were intramuscularly (i.m) immunized with different versions of HSV-1 gE/gI protein) adjuvanted with AS01 (5μg/each). An additional group of mice was i.m injected with a saline solution (NaCl 150mM) following the same schedule of immunization and used as negative control group (n=4/gr7). On days 42 (14PIII), serum sample from each animal was collected to evaluate the total anti-HSV-2 gE **(Fig 39A)** or HSV-2 gI **(Fig 39B)** cross-reactive IgG antibody titer by ELISA. The black error bar represents the 95% Confidence Interval (CI) of each geometric mean ratio (GMR). The geometric means (GMs) of the two groups compared and GMRs are indicated at the right of the graph.

**Figure 40 - HSV-1-specific neutralizing antibody titers measured in serum samples collected 14 days after the third immunization with different versions of AS01-adjuvanted HSV-1 gE/gI protein.** At days 0, 14 & 28, CB6F1 mice (n=6/gr1-6) were intramuscularly (i.m) immunized with different versions of HSV-1 gE/gI protein adjuvanted with AS01 (5μg/each). An additional group of mice was i.m injected with a saline solution (NaCl 150mM) following the same schedule of immunization and used as negative control group (n=4/gr7). On days 42 (14PIII), serum sample was collected to evaluate the titer of neutralizing antibodies towards HSV-1 VR-1789 strain (400 $TCID_{50}$). **Fig 40A:** Each dot represents individual animal titer. The positivity threshold value corresponds to the 1$^{st}$ sample dilution. Negative samples are illustrated by the 1$^{st}$ samples dilution/2 (neutralizating titer = 5). The number of mice by group (N) and the Geometric mean (GM) for each group are indicated below the x axis of the graph. **Fig 40B:** GMR with 95% of confidence interval (CI). The black error bar represents the 95% Confidence Interval (CI) of each GMR. The GMs of the two groups compared and GMRs are indicated at the right of the graph.

**Figure 41 - HSV-2 cross-reactive neutralizing antibody titers measured in serum samples collected 14 days after the third immunization with different versions of AS01-adjuvanted HSV-1 gE/gI protein.** At days 0, 14 & 28, CB6F1 mice (n=6/gr1-6) were intramuscularly (i.m) immunized with different versions of HSV-1 gE/gI protein adjuvanted with AS01 (5μg/each). An additional group of mice was i.m injected with a saline solution (NaCl 150mM) following the same schedule of immunization and used as negative control group (n=4/gr7). On days 42 (14PIII), serum sample was collected to evaluate the titer of cross-reactive neutralizing antibodies towards HSV-2 MS strain (400 $TCID_{50}$). Each dot represents individual animal titer. The positivity threshold value corresponds to the 1$^{st}$ sample dilution. Negative samples are illustrated by the 1$^{st}$ samples dilution/2 (neutralizating titer = 5). The number of mice by group (N) and the Geometric mean (GM) for each group are indicated below the x axis of the graph.

**Figure 42 - Evaluation of the ability of vaccine-specific antibodies to decrease, in-vitro, hIgG Fc binding by HSV-1 gE/gI antigen 14 days after the third immunization with different versions of AS01-adjuvanted HSV-1 gE/gI protein.** At days 0, 14 & 28, CB6F1 mice (n=6/gr1-6) were intramuscularly (i.m) immunized with different versions of HSV-1 gE/gI protein adjuvanted with AS01 (5μg/each). An additional group of mice was i.m injected with a saline solution (NaCl 150mM) following the same schedule of immunization and used as negative control group (n=4/gr7). On days 42 (14PIII), serum sample was collected to evaluate the ability of vaccine-specific antibodies to compete and decrease hIgG Fc binding by HSV-1 gE/gI protein. **Fig 42A=** AS01-HSV-1 gE/gI unmutated over NaCl; **Fig 42B=** AS01-HSV-1 gE_P319R/gI over NaCl; **Fig 42C=** AS01-HSV-1 gE_P321D/gI over NaCl; **Fig 42D=** AS01-HSV-1 gE_R322D/gI over NaCl; **Fig 42E=** AS01-HSV-1 gE_N243A_R322D/gI over NaCl; **Fig 42F=** AS01-HSV-1 gE_A340G_S341G_V342G/gI over NaCl.

**Figure 43 - Comparison of the ability of vaccine-specific antibodies to decrease, in-vitro, hIgG Fc binding by HSV-1 gE/gI antigen 14 days after the third immunization with different versions of AS01-adjuvanted HSV-1 gE/gI protein.** At days 0, 14 & 28, CB6F1 mice (n=6/gr1-6) were intramuscularly (i.m) immunized with different versions of HSV-1 gE/gI protein adjuvanted in AS01 (5μg/each). An additional group of mice was i.m injected with saline solution (NaCl 150mM) following the same schedule of immunization and used as negative control group (n=4/gr7). On days 42 (14PIII), serum sample was collected to evaluate the ability of vaccine-specific antibodies to compete and decrease hIgG Fc binding by HSV-1 gE/gI protein. **Fig 43A:** Each dot represents ED50 value from individual mice while each bar represents GMT + 95% CIs. The positivity threshold value corresponds to the 1st sample dilution. Negative samples are illustrated by the 1st samples dilution/2 (ED50 value = 5). The number of mice by group (N) and the Geometric mean (GM) for each group is indicated below the x axis of the graph. **Fig 43B:** GMR with 95% CIs of inhibition of hIgG Fc binding on HSV-1 gE/gI protein (ED50). The black error bar represents the 95% Confidence Interval (CI) of each GMR. The GMs of the two groups compared and GMRs are indicated at the right of the graph.

**Figure 44 - Evaluation of the ability of vaccine-specific antibodies to decrease, in-vitro, hIgG Fc binding by HSV-2 gE/gI antigen 14 days after the third immunization with different versions of AS01-adjuvanted HSV-1 gE/gI protein.** At days 0, 14 & 28, CB6F1 mice (n=6/gr1-6) were intramuscularly (i.m) immunized with different versions of HSV-1 gE/gI protein adjuvanted with AS01 (5μg/each). An additional group of mice was i.m injected with a saline solution (NaCl 150mM) following the same schedule of immunization and used as negative control group (n=4/gr7). On days 42 (14PIII), serum sample was collected to evaluate the ability of vaccine-specific antibodies to compete and decrease hIgG Fc binding by HSV-2 gE/gI protein. **Fig 44A=** AS01-HSV-1 gE/gI unmutated over NaCl; **Fig 44B=** AS01-HSV-1 gE_P319R/gI over NaCl; **Fig 44C=** AS01-HSV-1 gE_P321D/gI over NaCl; **Fig 44D=** AS01-HSV-1 gE_R322D/gI over NaCl; **Fig 44E=** AS01-HSV-1 gE_N243A_R322D/gI over NaCl; **Fig 44F=** AS01-HSV-1 gE_A340G_S341G_V342G/gI over NaCl.

**Figure 45 - Comparison of the ability of vaccine-specific antibodies to decrease, in-vitro, hIgG Fc binding by HSV-2 gE/gI antigen 14 days after the third immunization with different versions of AS01-adjuvanted HSV-1 gE/gI protein.** At days 0, 14 & 28, CB6F1 mice (n=6/gr1-6) were intramuscularly (i.m) immunized with different versions of HSV-1 gE/gI protein adjuvanted in AS01 (5μg/each). An additional group of mice was i.m injected with saline solution (NaCl 150mM) following the same schedule of immunization and used as negative control group (n=4/gr7). On days 42 (14PIII), serum sample was collected to evaluate the ability of vaccine-specific antibodies to compete and decrease hIgG Fc binding by HSV-2 gE/gI protein. **Fig 45A:** Each dot represents ED50 value from individual mice while each bar represents GMT + 95% CIs. The positivity threshold value corresponds to the 1st sample dilution. Negative samples are illustrated by the 1st samples dilution/2 (ED50 value = 5). The number of mice by group (N) and the Geometric mean (GM) for each group is indicated below the x axis of the graph. **Fig 45B**: GMR with 95% CIs of inhibition of hIgG Fc binding on HSV-2 gE/gI protein (ED50). The black error bar represents the 95% Confidence Interval (CI) of each GMR. The GMs of the two groups compared and GMRs are indicated at the right of the graph.

**Figure 46 - Evaluation of the ability of HSV-2 gE/gI cross reactive antibodies collected 14 days after the third immunization with different versions of AS01-adjuvanted HSV-1 gE/gI protein to bind and activate mouse FcγRIII after incubation with HSV-2 gE/gI transfected cells.** At days 0, 14 & 28, CB6F1 mice (n=6/gr1-6) were intramuscularly (i.m) immunized with different versions of HSV-1 gE/gI protein adjuvanted in AS01 (5μg/each). An additional group of mice was i.m injected with saline solution (NaCl 150mM) following the same schedule of immunization and used as negative control group (n=4/gr7). On days 42 (14PIII), serum samples were collected to evaluate the ability of vaccine-specific antibodies to cross-bind and activate mouse FcγRIII expressed by modified Jurkat reporter cells after incubation with HSV-2 gE/gI transfected 3T3 cells.

**Fig 46A-F:** Each curve illustrates data from pools of 2 mouse sera immunized with different versions of AS01-adjuvanted HSV-1 gE/gI protein or treated with a NaCl 150mM solution.

**Figure 47 - Comparison of the ability of HSV-2 gE/gI cross reactive antibodies collected 14 days after the third immunization with different versions of AS01-adjuvanted HSV-1 gE/gI protein to bind and activate mouse FcγRIII after incubation with HSV-2 gE/gI transfected cells.** At days 0, 14 & 28, CB6F1 mice (n=6/gr1-6) were intramuscularly (i.m) immunized with different versions of HSV-1 gE/gI protein adjuvanted in AS01 (5μg/each). An additional group of mice was i.m injected with saline solution (NaCl 150mM) following the same schedule of immunization and used as negative control group (n=4/gr7). On days 42 (14PIII), serum samples were collected to evaluate the ability of vaccine-specific antibodies to cross-react and activate mouse FcγRIII expressed by modified Jurkat reporter cells after incubation with HSV-2 gE/gI transfected 3T3 cells. Each dot represents a pool of 2 individual mouse and is represented as the ratio of the area under the curve (AUC) normalized on control positive sample.

**Figure 48 - Percentage of vaccine-specific or HSV-2 gE cross reactive CD4+/CD8+T cell responses induced in CB6F1 mice 14 days after the third immunization with different versions of HSV-1 gE/gI protein adjuvanted in AS01.** At days 0, 14 & 28, CB6F1 mice (n=6/gr1-6) were intramuscularly (i.m) immunized with different versions of HSV-1 gE/gI protein adjuvanted with AS01 (5μg/each). An additional group of mice was i.m injected with a saline solution (NaCl 150mM), following the same schedule of immunization, and used as negative control group (n=4/gr7). On days 42 (14PIII) mice were culled to evaluate HSV-1 gE and HSV-1 gI-specific & HSV-2 gE cross-reactive CD4+/CD8+T cell response in the spleen. Splenocytes were stimulated *ex-vivo* during 6 hours with pools of 15mer peptides covering the whole amino acids sequence of the gE or gI protein from HSV-1, as well as gE protein from HSV-2. Human β-actin peptides pool stimulation was used to evaluate non-specific response. The frequencies of CD4+/CD8+ T cells secreting IL-2, IFN-γ and/or TNF-α were measured by intracellular cytokine staining and results are shown in Fig 48A and 48B. Circle, triangle, square and diamond shapes represent individual % of CD4+/CD8+ T cell response detected for each antigen (HSV-1 or HSV-2 gE or gI antigens, or Human β-actin). Black line represents the geometric means (GM) of the response and black dotted line indicates the percentile 95$^{th}$ (P95) obtained with all the stimulations in the saline group. The number of animals/group with valid result (N) and the geometric mean (GM) of each group are indicated under the graph.

**Figure 49 - Head to head comparison of vaccine-specific or HSV-2 gE cross reactive CD4+T cell responses between vaccinated and unvaccinated groups 14 days after the third immunization in CB6F1 mice.** At days 0, 14 & 28, CB6F1 mice (n=6/gr1-6) were intramuscularly (i.m) immunized with different versions of HSV-1 gE/gI protein adjuvanted with AS01 (5μg/each). An additional group of mice was i.m injected with a saline solution (NaCl 150mM) following the same schedule of immunization and used as negative control group (n=4/gr7). On days 42 (14PIII) mice were culled to evaluate HSV-1 gE and HSV-1 gI-specific & HSV-2 gE cross-reactive CD4+T cell responses in the spleen. The black error bar represents the 95% Confidence Interval (CI) of each geometric mean ratio (GMR). The Geometric means (GM)s of the two groups compared and GMRs are indicated at the right of the graph. **Fig 49A:** GMR with 95% CIs of HSV-1 gE-specific CD4+ T cells; **Fig 49B:** GMR with 95% CIs of HSV-2 gE-specific CD4+ T cells; **Fig 49C:** GMR with 95% CIs of HSV-1 gI-specific CD4+ T cells. The black error bar represents the 95% Confidence Interval (CI) of each GMR. The GMs of the two groups compared and GMRs are indicated at the right of the graph.

**Figure 50 - Head to head comparison of HSV-1 gI-specific CD8+T cell response between vaccinated and unvaccinated groups 14 days after the third immunization in CB6F1 mice.** At days 0, 14 & 28, CB6F1 mice (n=6/gr1-6) were intramuscularly (i.m) immunized with different versions of HSV-1 gE/gI protein adjuvanted with AS01 (5μg/each). An additional group of mice was i.m injected with a saline solution (NaCl 150mM) following the same schedule of immunization and used as negative control group (n=4/gr7). On days 42 (14PIII) mice were culled to evaluate HSV-1 gI-specific CD8+T cell response in the spleen. The black error bar represents the 95% Confidence Interval (CI) of each GMR. The GMs of the two groups compared and GMRs are indicated at the right of the graph.

**Figure 51 - Head to head comparison of HSV-1 gE- specific or HSV-2 gE cross reactive CD4+T cell responses between vaccinated groups 14 days after the third immunization in CB6F1 mice.** At days 0, 14 & 28, CB6F1 mice (n=6/gr1-6) were intramuscularly (i.m) immunized with different versions of HSV-1 gE/gI protein adjuvanted with AS01 (5μg/each). An additional group of mice was i.m injected with a saline solution (NaCl 150mM), following the same schedule of immunization, and used as negative control group (n=4/gr7). On days 42 (14PIII) mice were culled to evaluate HSV-1 gE **(Fig 51A)**-specific and HSV-2 gE **(Fig 51B)** cross-reactive CD4+T cell responses in the spleen. The black error bar represents the 95% Confidence Interval (CI) of each GMR. The GMs of the two groups compared and GMRs are indicated at the right of the graph.

**Figure 52 - Head to head comparison of HSV-1 gI -specific CD4+T cell response between vaccinated groups 14 days after the third immunization in CB6F1 mice.** At days 0, 14 & 28, CB6F1 mice (n=6/gr1-6) were intramuscularly (i.m) immunized with different version of HSV-1 gE/gI protein adjuvanted with AS01 (5μg/each). An additional group of mice was i.m injected with a saline solution (NaCl 150mM) following the same schedule of immunization and used as negative control group (n=4/gr7). On days 42 (14PIII) mice were culled to evaluate HSV-1 gI-specific CD4+T cell response in the spleen. The black error bar represents the 95% Confidence Interval (CI) of each GMR. The GMs of the two groups compared and GMRs are indicated at the right of the graph.

**Figure 53 - Anti-HSV-1 gE/gI-specific IgG antibody response measured 28 days after the first or 21days after the second immunization with different mutated versions of SAM HSV-1 gE/gI vector formulated in Lipid nanoparticles (LNP).** At days 0 & 28, CB6F1 mice (n=6/gr1-5) were intramuscularly (i.m) immunized with 1μg of different mutated versions of SAM HSV-1 gE/gI vector formulated in LNP. An additional group of mice was i.m injected with a saline solution (NaCl 150mM) and used as negative control group (n=6/gr6). On days 28 & 49 (28PI & 21PII), serum sample from each animal was collected to evaluate the total anti-HSV-1 gE/gI-specific IgG antibody titer by ELISA. Each shape represents individual animal at different timepoints (dot = 28PI; triangle = 21PII) while the black bars represents the Geometric mean of each group. Geometric mean (GM) and number of animals (N) for each group is indicated on the x axis.

**Figure 54 - Head to head comparison of anti-HSV-1 gE/gI-specific IgG antibody response between LNP/SAM HSV-1 vaccinated and unvaccinated groups 21 days after the second immunization in CB6F1mice.** At days 0 & 28, CB6F1 mice (n=6/gr1-5) were intramuscularly (i.m) immunized with 1μg of different mutated versions of SAM HSV-1 gE/gI vector formulated in LNP. An additional group of mice was i.m injected with a saline solution (NaCl 150mM), following the same schedule of immunization, and used as negative control group (n=6/gr6). On days 28 & 49 (28PI & 21PII), serum sample from each animal was collected to evaluate the total anti-HSV-1 gE/gI-specific IgG antibody titer by ELISA. The black error bar represents the 95% Confidence Interval (CI) of each geometric mean ratio (GMR). The geometric means (GMs) of the two groups compared and GMRs are indicated at the right of the graph.

**Figure 55 - Timepoint comparison of anti-HSV-1 gE/gI-specific IgG antibody response measured 28 days after the first and 21days after the second immunization with different mutated versions of SAM HSV-1 gE/gI vector formulated in Lipid nanoparticles (LNP).** At days 0 & 28, CB6F1 mice (n=6/gr1-5) were intramuscularly (i.m) immunized with 1μg of different mutated versions of SAM HSV-1 gE/gI vector formulated in LNP. An additional group of mice was i.m injected with a saline solution (NaCl 150mM) and used as negative control group (n=6/gr6). On days 28 & 49 (28PI & 21PII), serum sample from each animal was collected to evaluate the total anti-HSV-1 gE/gI-specific IgG antibody titer by ELISA. The black error bar represents the 95% Confidence Interval (CI) of each geometric mean ratio (GMR). The geometric means (GMs) of the two time points and GMRs are indicated at the right of the graph.

**Figure 56 - Head to head comparison of anti-HSV-1 gE/gI-specific IgG antibody response measured in serum samples collected 21days after the second immunization with different mutated versions of SAM HSV-1 gE/gI vector formulated in Lipid nanoparticles (LNP).** At days 0 & 28, CB6F1 mice (n=6/gr1-5) were intramuscularly (i.m) immunized with 1μg of different mutated versions of SAM HSV-1 gE/gI vector formulated in LNP. An additional group of mice was i.m injected with a saline solution (NaCl 150mM) and used as negative control group (n=6/gr6). On days 28 & 49 (28PI & 21PII), serum sample from each animal was collected to evaluate the total anti-HSV-1 gE/gI-specific IgG antibody titer by ELISA. The black error bar represents the 95% Confidence Interval (CI) of each geometric mean ratio (GMR). The geometric means (GMs) of the two groups and GMRs are indicated at the right of the graph.

**Figure 57 - Anti-HSV-2 gE or gI cross-reactive IgG antibody responses measured 21 days after the second immunization with different mutated versions of SAM HSV-1 gE/gI vector formulated in Lipid nanoparticles (LNP).** At days 0 & 28, CB6F1 mice (n=6/gr1-5) were intramuscularly (i.m) immunized with 1μg of different mutated versions of SAM HSV-1 gE/gI vector formulated in LNP. An additional group of mice was i.m injected with a saline solution (NaCl 150mM) and used as negative control group (n=6/gr6). On day 49 (21PII), serum sample from each animal was collected to evaluate the total anti-HSV-2 gE **(Fig 57A)** or gI **(Fig 57B)** cross-reactive IgG antibody titers by ELISA. Each shape represents individual animal at timepoint 21PII while the black bars represents the Geometric mean of each group. Geometric mean (GM) and number of animals (N) for each group is indicated on the x axis.

**Figure 58 - Head to head comparison of anti-HSV-2 gE or HSV-2 gI cross-reactive IgG antibody responses between LNP/SAM HSV-1 vaccinated and unvaccinated groups 21 days after the second immunization in CB6F1mice.** At days 0 & 28, CB6F1 mice (n=6/gr1-5) were intramuscularly (i.m) immunized with 1μg of different mutated versions of SAM HSV-1 gE/gI vector formulated in LNP. An additional group of mice was i.m injected with a saline solution (NaCl 150mM), following the same schedule of immunization, and used as negative control group (n=6/gr6). On day 49 (21PII), serum sample from each animal was collected to evaluate the total anti-HSV-2 gE **(Fig 58A)** or HSV-2 gI **(Fig 58B)** cross-reactive IgG antibody titer by ELISA. The black error bar represents the 95% Confidence Interval (CI) of each geometric mean ratio (GMR). The geometric means (GMs) of the two groups compared and GMRs are indicated at the right of the graph.

**Figure 59 - Head to head comparison of anti-HSV-2 gE or HSV-2 gI cross-reactive IgG antibody responses measured in serum samples collected 21 days after the second immunization with different mutated versions of SAM HSV-1 gE/gI vector formulated in Lipid nanoparticles (LNP).** At days 0 & 28, CB6F1 mice (n=6/gr1-5) were intramuscularly (i.m) immunized with 1μg of different mutated versions of SAM HSV-1 gE/gI vector formulated in LNP. An additional group of mice was i.m injected with a saline solution (NaCl 150mM) and used as negative control group (n=6/gr6). On day 49 (21PII), serum sample from each animal was collected to evaluate the total anti-HSV-2 gE **(Fig 59A)** or HSV-2 gI **(Fig 59B)** cross-reactive IgG antibody titer by ELISA. The black error bar represents the 95% Confidence Interval (CI) of each geometric mean ratio (GMR). The geometric means (GMs) of the two groups and GMRs are indicated at the right of the graph.

**Figure 60 - HSV-1-specific neutralizing antibody titers measured in serum samples collected 21 days after the second immunization with different mutated versions of LNP-formulated HSV-1 gE/gI vector.** At days 0 & 28, CB6F1 mice (n=6/gr1-5) were intramuscularly (i.m) immunized with 1μg of different mutated versions of SAM HSV-1 gE/gI vector formulated in LNP. An additional group of mice was i.m injected with a saline solution (NaCl 150mM) following the same schedule of immunization and used as negative control group (n=5/gr6). On days 49 (21PII), serum sample was collected to evaluate the titer of neutralizing antibodies towards HSV-1 VR1789 strain (viral load 400 $TCID_{50}$). **Fig 60A:** Each dot represents individual animal titer while bar represents Geometric mean (GM) + 95% confidence intervals (CIs). The positivity threshold value corresponds to the 1st sample dilution. Negative samples are illustrated by the 1st samples dilution/2 (neutralizing titer = 5). The number of mice by group (N) and the GM for each group are indicated below the x axis of the graph. **Fig 60B:** GMR with 95% of confidence interval (CI). The black error bar represents the 95% Confidence Interval (CI) of each GMR. The GMs of the two groups compared and GMRs are indicated at the right of the graph.

**Figure 61 - HSV-2 cross-reactive neutralizing antibody titers measured in serum samples collected 21 days after the second immunization with different mutated versions of LNP-formulated HSV-1 gE/gI vector.** At days 0 & 28, CB6F1 mice (n=6/gr1-5) were intramuscularly (i.m) immunized with 1μg of different mutated versions of SAM HSV-1 gE/gI vector formulated in LNP. An additional group of mice was i.m injected with a saline solution (NaCl 150mM) following the same schedule of immunization and used as negative control group (n=5/gr6). On days 49 (21PII), serum sample was collected to evaluate the titer of cross-reactive neutralizing antibodies towards HSV-2 MS strain (viral load 400 $TCID_{50}$). Each dot represents individual animal titer while bar represents Geometric mean (GM) + 95% confidence intervals (CIs). The positivity threshold value corresponds to the 1st sample dilution. Negative samples are illustrated by the 1st samples dilution/2 (neutralizing titer = 5). The number of mice by group (N) and the GM for each group are indicated below the x axis of the graph.

**Figure 62 - Evaluation of the ability of vaccine-specific antibodies to decrease, in-vitro, hIgG Fc binding by HSV-1 gE/gI protein 21 days after the second immunization with different mutated versions of LNP-formulated SAM-HSV-1 gE/gI vector.** At days 0 & 28, CB6F1 mice (n=6/gr1-5) were intramuscularly (i.m) immunized with 1μg of different mutated versions of SAM HSV-1 gE/gI vector formulated in LNP. An additional group of mice was i.m injected with a saline solution (NaCl 150mM) and used as negative control group (n=5/gr6). On days 49 (21PII), serum sample was collected to evaluate the ability of vaccine-specific antibodies to compete and decrease hIgG Fc binding by HSV-1 gE/gI protein. Each curve represents individual mice. LNP/SAM-HSV-1 gE_P319R/gI over NaCl **(Fig 62A);** LNP/SAM-HSV-1 gE_P321D/gI over NaCl **(Fig 62B);** LNP/SAM-HSV-1 gER322D/gI over NaCl **(Fig 62C);** LNP/SAM-HSV-1 gE N243AR322D/gI over NaCl **(Fig 62D);** LNP/SAM-HSV-1 gE_A340G_S341G_V342G/gI over NaCl **(Fig 62E).**

**Figure 63 - Comparison of the ability of vaccine-specific antibodies to decrease, in-vitro, human IgG Fc binding by HSV-1 gE/gI antigen 21 days after the second immunization with different mutated versions of LNP-formulated SAM HSV-1 gE/gI vector.** At days 0 & 28, CB6F1 mice (n=6/gr1-5) were intramuscularly (i.m) immunized with 1μg of different mutated versions of SAM HSV-1 gE/gI vector formulated in LNP. An additional group of mice was i.m injected with a saline solution (NaCl 150mM) and used as negative control group (n=5/gr6). On days 49 (21PII), serum sample was collected to evaluate the ability of vaccine-specific antibodies to compete and decrease hIgG Fc binding by HSV-1 gE/gI protein. **Fig 63A:** Each dot represents ED50 titer with 95% CIs from individual mice. The positivity threshold value corresponds to the 1st sample dilution. Negative samples are illustrated by the 1st samples dilution/2 (ED50 titer = 5). The number of mice by group (N) and the Geometric mean (GM) for each group is indicated below the x axis of the graph. **Fig 63B:** GMR with 95% of confidence interval (CI). The black error bar represents the 95% Confidence Interval (CI) of each geometric mean ratio (GMR). The GMs of the two groups compared and GMRs are indicated at the right of the graph.

**Figure 64 - Evaluation of the ability of vaccine-specific antibodies to decrease, in-vitro, hIgG Fc binding by HSV-2 gE/gI protein 21days after the second immunizations with different mutated versions of LNP-formulated SAM-HSV-1 gE/gI vector.** At days 0 & 28, CB6F1 mice (n=6/gr1-5) were intramuscularly (i.m) immunized

with 1μg of different mutated versions of SAM HSV-1 gE/gI vector formulated in LNP. An additional group of mice was i.m injected with a saline solution (NaCl 150mM) and used as negative control group (n=5/gr6). On days 49 (21PII), serum sample was collected to evaluate the ability of vaccine-specific antibodies to compete and decrease hIgG Fc binding by HSV-2 gE/gI protein. Each curve represents individual mice. LNP/SAM-HSV-1 gE_P319R/gI over NaCl **(Fig 64A);** LNP/SAM-HSV-1 gE_P321D/gI over NaCl **(Fig 64B);** LNP/SAM-HSV-1 gER322D/gI over NaCl **(Fig 64C);** LNP/SAM-HSV-1 gE N243AR322D/gI over NaCl **(Fig 64D);** LNP/SAM-HSV-1 gE_A340G_S341G_V342G/gI over NaCl **(Fig 64E).**

**Figure 65 - Comparison of the ability of vaccine-specific antibodies to decrease, in-vitro, human IgG Fc binding by HSV-2 gE/gI protein 21 days after the second immunization with different mutated versions of LNP-formulated SAM HSV-1 gE/gI vector.** At days 0 & 28, CB6F1 mice (n=6/gr1-5) were intramuscularly (i.m) immunized with 1μg of different mutated versions of SAM HSV-1 gE/gI vector formulated in LNP. An additional group of mice was i.m injected with a saline solution (NaCl 150mM) and used as negative control group (n=5/gr6). On days 49 (21PII), serum sample was collected to evaluate the ability of vaccine-specific antibodies to compete and decrease hIgG Fc binding by HSV-2 gE/gI protein. **Fig 65A:** Each dot represents ED50 titer with 95% CIs from individual mice. The positivity threshold value corresponds to the 1$^{st}$ sample dilution. Negative samples are illustrated by the 1$^{st}$ samples dilution/2 (ED50 titer = 5). The number of mice by group (N) and the Geometric mean (GM) for each group is indicated below the x axis of the graph. **Fig 65B:** GMR with 95% of confidence interval (CI). The black error bar represents the 95% Confidence Interval (CI) of each geometric mean ratio (GMR). The GMs of the two groups compared and GMRs are indicated at the right of the graph.

**Figure 66 - Evaluation of the ability of HSV-2 gE/gI cross reactive antibodies collected 21 days after the second immunization with different mutated versions of LNP-formulated SAM HSV-1 gE/gI vector to bind and activate mouse FcγRIII after incubation with HSV-2 gE/gI transfected cells.** At days 0 & 28, CB6F1 mice (n=6/gr1-5) were intramuscularly (i.m) immunized with 1μg of different mutated versions of SAM-HSV-1 gE/gI vector formulated in LNP. An additional group of mice was i.m injected with a saline solution (NaCl 150mM) and used as negative control group (n=5/gr6). On days 49 (21PII), serum sample was collected to evaluate the ability of HSV-2 gE/gI cross reactive antibodies to bind and activate mouse FcγRIII expressed by modified Jurkat reporter cells after incubation with HSV-2 gE/gI transfected 3T3 cells. **Fig 66A-E:** Each curve illustrates data from pools of 2 sera from mice immunized with different mutated versions of LNP/SAM-HSV-1 gE/gI or with a NaCl 150mM solution.

**Figure 67 - Percentage of vaccine-specific and HSV-2 gE cross-reactive CD4+/CD8+T cell responses induced 21 days after the second immunization with different mutated versions of SAM HSV-1 gE/gI vector formulated in LNP in CB6F1 mice.** At days 0 & 28, CB6F1 mice (n=6/gr1-5) were intramuscularly (i.m) immunized with 1μg of different SAM-HSV-1 gE/gI mutant candidates formulated in lipid nanoparticles (LNP). An additional group of mice was i.m injected with a saline solution (NaCl 150mM) and used as negative control group (n=6/gr6). On day 49 (21PII), mice were culled to evaluate vaccine-specific and HSV_2 gE cross-reactive CD4+/CD8+T cell responses in the spleen. Splenocytes were stimulated *ex-vivo* during 6 hours with pools of 15mer peptides covering the whole amino acids sequence of the gE or gI protein from HSV-1, as well as gE protein from HSV-2. Human β-actin peptides pool stimulation was used to evaluate non-specific response. The frequencies of CD4+/CD8+ T cells secreting IL-2, IFN-γ and/or TNF-α were measured by intracellular cytokine staining. Circle, triangle, square and diamond shapes represent individual % of CD4+ **(Fig 67A)** and CD8+ **(Fig 67B)** T cell responses detected for each antigen (HSV-1 or HSV-2 gE or HSV-2 gI antigens, or Human β-actin). Black line represents the geometric means (GM) of the response and black dotted line indicates the percentile 95$^{th}$ (P95) obtained with all the stimulations in the saline group. The number of animals/group with valid result (N) and the GM of each group are indicated under the graph.

**Figure 68 - Head to head comparison of vaccine-specific and HSV-2 gE cross-reactive CD4+/ CD8+T cell responses between vaccinated and unvaccinated groups in CB6F1 mice 21 days after the second immunization in CB6F1 mice.** At days 0 & 28, CB6F1 mice (n=6/gr1-5) were intramuscularly (i.m) immunized with 1μg of different SAM-HSV-1 gE/gI mutant candidates formulated in LNP. An additional group of mice was i.m injected with a saline solution (NaCl 150mM) following the same schedule of immunization and used as negative control group (n=6/gr6). On day 49 (21PII), mice were culled to evaluate vaccine-specific and HSV-2 gE cross-reactive CD4+ and CD8+T cell responses in the spleen. The black error bar represents the 95% Confidence Interval (CI) of each the geometric mean ratio (GMR). The geometric means (GMs) of the two groups compared and GMRs are indicated at the right of the graph. **Fig 68A:** GMR with 95% CI of HSV-1 gE-specific CD4+ T cells; **Fig 68B:** GMR with 95% CI of HSV-2 gE-cross reactive CD4+ T cells; **Fig 68C:** GMR with 95% CI of HSV-1 gI-specific CD4+ T cells; **Fig 68D:** GMR with 95% CI of HSV-1 gI-specific CD8+ T cells.

**Figure 69 - Head to head comparison of vaccine-specific and HSV-2 gE cross-reactive CD4+T cell responses between vaccinated groups 21days after the second immunization in CB6F1 mice.** At days 0 & 28, CB6F1 mice (n=6/gr1-5) were intramuscularly (i.m) immunized with 1$\mu$g of different SAM-HSV-1 gE/gI mutant candidates formulated in LNP. An additional group of mice was i.m injected with a saline solution (NaCl 150mM) following the same schedule of immunization and used as negative control group (n=6/gr6). On day 49 (21PII), mice were culled to evaluate vaccine-specific and HSV-2 gE cross-reactive CD4+T cell responses in the spleen. The black error bar represents the 95% Confidence Interval (CI) of each geometric mean ratio (GMR). The geometric means (GMs) of the two groups compared and GMRs are indicated at the right of the graph. **Fig 69A:** GMR with 95% CI of HSV-1 gE-specific CD4+ T cells; **Fig 69B:** GMR with 95% CI of HSV-2 gE cross-reactive CD8+ T cells.

**Figure 70 - Head to head comparison of HSV-1 gI-specific CD4+/CD8+T cell responses between vaccinated groups 21 days after the second immunization in CB6F1 mice.** At days 0 & 28, CB6F1 mice (n=6/gr1-5) were intramuscularly (i.m) immunized with 1$\mu$g of different SAM HSV-1 gE/gI mutant candidates formulated in LNP. An additional group of mice was i.m injected with saline solution (NaCl 150mM) following the same schedule of immunization and used as negative control group (n=6/gr6). On day 49 (21PII), mice were culled to evaluate anti-HSV-1 gI-specific CD4+/CD8+T cell responses in the spleen. The black error bar represents the 95% Confidence Interval (CI) of each geometric mean ratio (GMR). The geometric means (GMs) of the two groups compared and GMRs are indicated at the right of the graph. **Fig 70A:** GMR with 95% CI of HSV-1 gI-specific CD4+ T cells; **Fig 70B:** GMR with 95% CI of HSV-1 gI-specific CD8+ T cells.

**Figure 71 - Evaluation of the ability of HSV-1 gE/gI cross reactive antibodies to decrease, in-vitro, human IgG Fc binding by HSV-1 gE/gI antigen 14 days after third immunizations with different mutated versions of AS01-adjuvanted HSV-2 gE/gI protein.** At days 0, 14 & 28, CB6F1 mice (n=6/gr1-5) were intramuscularly (i.m) immunized with different HSV-2 gE/gI mutant proteins adjuvanted in AS01 (5$\mu$g/each). An additional group of mice was i.m injected with a saline solution (NaCl 150mM) following the same schedule of immunization and used as negative control group (n=4/gr6). On days 42 (14PIII), serum samples were collected to evaluate the ability of HSV-1 cross reactive antibodies to compete and decrease hIgG Fc binding by HSV-1gE/gI protein. Each curve represents individual mice data. **Fig 71A:** AS01/HSV-2 gE/gI V340W over NaCl; **Fig 71B:** AS01/HSV-2 gE/gI A248T over NaCl; **Fig 71C:** AS01/HSV-2 gE/gI A246W over NaCl; **Fig 71D:** AS01/HSV-2 gE/gI P318I over NaCl; **Fig 71E:** AS01/HSV-2 gE/gI A248T_V340W over NaCl.

**Figure 72 - Evaluation of the ability of HSV-1 gE/gI cross reactive antibodies to bind and activate mFc$\gamma$RIII after incubation with HSV-1 gE/gI positive cells 14 days after third immunizations with different mutated versions of AS01-adjuvanted HSV-2 gE/gI protein.** At days 0, 14 & 28, CB6F1 mice (n=6/gr1-5) were intramuscularly (i.m) immunized with different HSV-2 gE/gI mutant candidates adjuvanted with AS01 (5$\mu$g/each). An additional group of mice was i.m injected with a saline solution (NaCl 150mM) following the same schedule of immunization and used as negative control group (n=4/gr6). On days 42 (14PIII), serum samples were collected to evaluate the ability of vaccine-specific antibodies to bind HSV-1 gE/gI positive 3T3 cells and activate the mouse $F_c\gamma$RIII expressed by modified Jurkat reporter cells. **Fig 72A-E** each curve illustrate data from pools of 2 mouse sera immunized with different AS01-HSV-2 gE/gI mutants over NaCl while **Fig 12F** illustrates Geoemetric mean of each AS01-HSV-2 gE/gI vaccinated group over NaCl.

**Figure 73 - Evaluation of the ability of HSV-1 gE/gI cross reactive antibodies to decrease, in-vitro, hIgG Fc binding by HSV-1 gE/gI protein 21 days after the third immunization with different LNP-formulated SAM-HSV-2 gE/gI mutants.** At days 0, 21 & 42, CB6F1 mice (n=6/gr1-6) were intramuscularly (i.m) immunized with 0.8$\mu$g of different mutated versions of SAM-HSV-2 gE/gI vector formulated in Lipid nanoparticles (LNP). An additional group of mice was i.m injected with a saline solution (NaCl 150mM) following the same schedule of immunization and used as negative control group (n=4/gr7). On days 63 (21PIII), serum sample from each animal was collected to evaluate the ability of HSV-1 cross reactive antibodies to compete and decrease hIgG Fc binding by HSV-1 gE/gI protein. Each curve represents individual mice. LNP/SAM-HSV-2 gE/gI V340W over NaCl group **(Fig 73A);** LNP/SAM-HSV-2 gE/gI A248T over NaCl group **(Fig 73B);** LNP/SAM-HSV-2 gE/gI A246W over NaCl group **(Fig 73C);** LNP/SAM-HSV-2 gE/gI P318I over NaCl group **(Fig 73D);** LNP/SAM-HSV-2 gE/gI A248T_V340W over NaCl group **(Fig 73E);** LNP/SAM-HSV-2 gE/gI insert ARAA over NaCl group; **(Fig 73F).**

**Figure 74 - Evaluation of the ability of HSV-1 gE/gI-specific antibodies collected 14 days after the third immunization with different versions of AS01-adjuvanted HSV-1 gE/gI protein to bind and activate mouse Fc$\gamma$RIII after incubation with HSV-1 gE/gI transfected cells.** At days 0, 14 & 28, CB6F1 mice (n=6/gr1-6) were intramuscularly (i.m) immunized with different versions of HSV-1 gE/gI protein adjuvanted in AS01 (5$\mu$g/each). An

additional group ofmice was i.m injected with saline solution (NaCl 150mM) following the same schedule of immunization and used as negative control group (n=4/gr7). On days 42 (14PIII), serum samples were collected to evaluate the ability of vaccine-specific antibodies to bind and activate mouse FcγRIII expressed by modified Jurkat reporter cells after incubation with HSV-1 gE/gI transfected 3T3 cells. **Fig 74A-F:** Each curve illustrates data from pools of 2 mouse sera immunized with different versions of AS01-adjuvanted HSV-1 gE/gI protein or treated with a NaCl 150mM solution. **Fig 74G:** illustrates Geoemetric mean of each AS01-HSV-1 gE/gI vaccinated group over NaCl.

**Figure 75 - Annotated amino acid sequences for HSV2 gE (UniprotKB: A7U881) and HSV1 gE (UniprotKB: Q703E9).** Sequence alignment on EBIO using GAP, Gap Weight: 8, Length Weight: 2, Similarity: 78.68 %, Identity: 76.10 %. Underlined: Signal peptide (SP); **bold underlined:** transmembrane domain; *Italic underlined:* Fc-binding region; ***bold italic:*** region required for heterodimer complex formation.

**Figure 76 - Annotated amino acid sequences for HSV2 gI (UniprotKB: A8U5L5) and HSV1 gI (UniprotKB: P06487).** Sequence alignment on EBIO using GAP; Gap Weight: 8; Length Weight: 2; Similarity: 73.37%; Identity: 70.38%. Underlined: Signal peptide (SP); **Bold underlined:** transmembrane domain; ***bold italic:*** region required for heterodimer complex formation.

**Figure 77 - Alignment of HSV2 gE ectodomain protein sequences.** Black / dark grey / light grey shading: 100% /80% /60% similarity respectively across all aligned sequences.

**Figure 78 - Alignment of HSV2 gI ectodomain protein sequences.** Black / dark grey / light grey shading: 100% /80% / 60% similarity respectively across all aligned sequences.

**Figure 79 - DNA sequence of the plasmid that expresses the RNA sequence for the SAM-gEgI constructs.** Upper case: SAM backbone; Lower case: non-SAM sequence; underlined: 5' UTR of SAM; **bold underlined:** 3' UTR of SAM; grey shade: Insert encoding the gEgI heterodimer.

**Figure 80 - Design of several ThHSV SAM vectors encoding for gEgI heterodimer.** Cloning was performed into VEEV TC-83 SAM vector (Venezuelan Equine Encephalitis virus-attenuated strain). HSV2 gE P317R mutant (Fc binding KO) versions were also generated. A) Screening of different regulatory elements to drive gI expression. The selected regulatory elements were i) Enterovirus 71 Internal Ribosome entry site (EV71 IRES), ii) two 2A peptide sequences (GSG-P2A: Porcine teschovirus-1 2A with GSG linker, F2A: 2A peptide from the foot-and-mouth disease virus (F2A)) and iii) the promoter for 26S RNA (26S prom). Size (bp) of each regulatory element is indicated. B) Same constructs as in A), including an HA-tag in C-term of HSV2 gE and gI proteins.

## DETAILED DESCRIPTION OF THE INVENTION

The present invention relates to the use of a HSV Fc receptor or an immunogenic fragment or variant thereof, in particular glycoprotein gE from HSV1 or HSV2, alone or with its binding partner gI in a therapeutic vaccine against HSV, wherein the HSV Fc receptor or immunogenic fragment or variant thereof induces a cross reactive immune response against HSV1 or HSV2 when administered to a subject. The inventors have shown that immunisation of mice with the ectodomain of HSV2 Fc receptor (in particular gE/gI) or variants thereof with mutations of gE/gI that reduce or abolish binding to the Fc portion of a human antibody, results in a functional cross reactive immune response against HSV1 viruses or HSV1 antigens. It was also demonstrated that the immunisation of mice with the ectodomain of HSV1 Fc receptor (in particular gE/gI) or variants thereof with mutations of gE/gI that reduce or abolish binding to a human antibody, results in a functional cross reactive immune response against HSV2 viruses or HSV2 antigens. Amino acid sequence alignments between HSV1 KOS strain and HSV2 SD90e strain revealed gE and gI proteins to respectively share 76.10%/67.4% and 78.68%/75.5% of identity/similarity. But a functional cross-reactive immune response between HSV-1 and HSV-2 gE/gI was unexpected. Even at significantly higher levels of sequence similarity between antigens, this does not imply a functional cross-reactive immune response. For example, some influenza virus HA antigens (from strains post 2009) share approximately 90% amino acid sequence similarity yet show no functional cross-reactive immune response. Hence, it was surprising that administration of a nucleic acid encoding a first HSV serotype would show cross-reactivity and elicit an immune response in a subject for a different HSV serotype.

Hence, there is provided an HSV2 Fc receptor or an immunogenic fragment or variant thereof for use in inducing a cross reactive immune response against HSV-1 when administered to a subject. Also provided is an HSV1 Fc receptor or an immunogenic fragment or variant thereof for use in inducing a cross reactive immune response against HSV2 when administered to a subject. The HSV1 Fc receptor or an immunogenic fragment or variant thereof may be used in treating a subject infected with HSV1 and/or HSV2, preferably for use as a pan-HSV vaccine. The HSV2 Fc receptor or an immunogenic fragment or variant thereof may be used in treating a subject infected with HSV1 and/or HSV2, preferably for use as a pan-HSV vaccine. The HSV1 or HSV2 Fc receptor or immunogenic fragment or variant of the invention may be used in particular against recurrent infections with HSV1 and/or HSV2 and related clinical and sub-clinical manifestations.

*Cross reactive immune response*

The immune response is cross reactive in that the HSV Fc receptor or immunogenic fragment or variant thereof can induce an antigen specific humoral and/or cellular immune response against an antigen from a serotype of HSV other than the one from which it is derived, i.e. it induces a cross serotype response. For example, in the case of an HSV2 Fc receptor or immunogenic fragment or variant thereof, it can induce a humoral and/or cellular immune response against HSV1, in the case of an HSV1 Fc receptor or immunogenic fragment or variant thereof, it can induce a humoral and/or cellular immune response against HSV2. Thus, in one embodiment, the HSV Fc receptor or an immunogenic fragment or variant thereof induces a humoral and/or cellular immune response to both homologous and heterologous serotypes of HSV. Hence, the HSV Fc receptor or an immunogenic fragment or variant thereof, may induce an immune response against both the serotype of HSV from which it is derived and against another serotype of HSV from which the Fc receptor is not derived. As there are two serotypes of HSV (HSV1 and HSV2), the HSV Fc receptor or immunogenic fragment or variant thereof may induce a humoral and/or cellular immune response against both HSV1 and HSV2. The humoral immune response may comprise the generation of antibodies that bind to an antigen from a serotype of HSV other than the one from which the HSV Fc receptor or immunogenic fragment or variant thereof is derived. Alternatively, or in addition, the cross-reactive immune response may lead to the generation of a specific T lymphocyte cell response, for example a CD4+ T cell response and/or a CD8+ T cell response.

The immune response may be functional, for example not only binding of an antibody to the Fc receptor antigen or antigen associated molecule or cell, but that initiates downstream effector functions that contribute to an effective immune response. In one embodiment, a functional antibody response is induced. For example, a functional antibody response may include the activation of complement, granulocytes and/or cytotoxic T cells, neutralisation of viral particles, or other means to inhibit or immobilise HSV or inhibition of HSV's immune evasion activity. As a cross reactive functional immune response, the HSV Fc receptor or immunogenic fragment or variant thereof may initiate downstream effector functions (e.g. activation of complement, granulocytes and/or cytotoxic T cells, neutralisation, or other means to inhibit or immobilise HSV or inhibition of HSV's immune evasion activity) against a serotype of HSV from which it is not derived, i.e. generating a cross serotype response. Preferably, the functional antibody response comprises inhibition of HSV's immune evasion activity. The latter may be achieved by generation of antibodies that bind HSV Fc receptor and inhibit the binding of HSV Fc receptor to human IgG. In this way, the functional immune response may include inhibition of antibody bipolar bridging, for example bipolar bridging of antibodies bound to the surface of HSV-infected cells.

The generation of cross reactive antibodies that bind for example, HSV Fc receptor, can be measured by techniques well known to those skilled in the art, for example, an ELISA, or indirect ELISA for detection of cross reactive antibodies that bind a serotype of HSV from which the Fc receptor antigen was not derived. The ability to inhibit immune evasion activity may be measured for example, by a competitive ELISA to assess the ability of vaccine specific antibodies to decrease human IgG Fc binding by HSV Fc receptor in vitro. A neutralisation assay can be used to assess the presence of cross neutralising antibodies. Neutralising antibodies may prevent the replication of HSV in permissive host cells, cells which allow the penetration and multiplication of HSV. A neutralisation assay can assess the ability of the antibodies to neutralise the virus strain, for example by assessing the cytopathic effect of virus infected host cells in the presence or absence of serum containing such antibodies.

The functional immune response may comprise the generation of a cross reactive (e.g. cross serotype) T cell response. Two main cellular effectors of the specific T cell response are the CD4+ helper T cells and CD8+ T lymphocytes (or cytotoxic T lymphocytes or CTLs). The activation of CD4+ T cells causes them to release cytokines which influence the activity of many cell types such as B lymphocytes, CTLs and antigen presenting cells (APCs). CD4+ T cells can be further subdivided into Th1 and Th2 which produce Th1-type cytokines (e.g. IL-2 and IFN-$\gamma$) and Th2-type cytokines. The activation of CD4+ T cells and secretion of cytokines can stimulate both cell-mediated and antibody-mediated branches of the immune system. Preferably, the cross reactive CD4+ T cell response is primarily a Th1 cell-mediated immune response, for example inducing IL-2 and/or IFN-$\gamma$. CD8+ T cells (or cytotoxic T cells (CTLs)) may also be involved in T cell-medicated immune responses and can induce the death of cells infected with viruses. CD8+ T cells can produce cytokines (e.g. IFN-$\gamma$) upon activation following specific antigen stimulation. The release of cytokines upon activation of CD4+ helper T cells or CD8+ T cells can be measured to assess and quantify this activation. Techniques well known to those skilled in the art may be used to measure the cytokine response associated with CD4+ /CD8+ T cell activation and include but are not limited to use of a specific ELISA or ELISPOT or flow cytometry designed for measurement of cytokines such as IFN-$\gamma$, IL-2 and/or others. In particular, an intracellular cytokine staining assay may be used to detect intracellular cytokines in these cells and then measured for example using flow cytometry. Cytotoxicity can be assessed using for example in vitro cytolytic assays whereby antigen-loaded target cells are lysed by specific cytotoxic T cells that are generated in vivo following vaccination or infection. Techniques for this are well known to those skilled in the art and include for example a chromium release assay or an antibody-dependent cell-mediated cytotoxicity (ADCC) bioassay. ADCC bioassays may detect killing of target cells (e.g. by PBMCs or NK cells) after cytotoxic pathways are activated when membrane surface antigens are bound by specific antibodies. Alternative assays such as the Promega ADCC Reporter Bioassay may detect FcγR mediated activation of luciferase activity in cell lines stably expressing human FcγRIIIa induced luciferase. Following engagement with the Fc region of a relevant antibody bound to a target cell, the bioassay effector cells expressing FcγRIIIa transduce intracellular signals resulting in luciferase activity that can be easily quantified. In a further embodiment, is provided a method for determining a cross reactive immune response in a subject following administration of an immunologically effective amount of a HSV1 Fc receptor or immunogenic fragment or variant thereof, comprising taking a tissue (e.g. blood) sample from the subject and performing an in vitro assay to detect a cross reactive immune response against HSV2. In a further embodiment, is provided a method for determining a cross reactive immune response in a subject following administration of an immunologically effective amount of a HSV2 Fc receptor or immunogenic fragment or variant thereof, comprising taking a tissue (e.g. blood) sample from the subject and performing an in vitro assay to detect a cross reactive immune response against HSV1. In one embodiment, the in vitro assay is used to detect cross reactive antibodies in a blood sample.

In one embodiment is provided a HSV2 Fc receptor or an immunogenic fragment or variant thereof for the use described herein wherein the cross-reactive immune response comprises a cross serotype antibody response against HSV1, preferably for use as a pan-HSV vaccine. In a further embodiment is provided a HSV1 Fc receptor or an immunogenic fragment or variant thereof for the use described herein wherein the cross-reactive immune response comprises a cross serotype antibody response against HSV2, preferably for use as a pan-HSV vaccine.

In one embodiment is provided a HSV Fc receptor or an immunogenic fragment or variant thereof for use in inducing a cross reactive immune response against HSV of a different serotype when administered to a subject.

In one embodiment is provided a HSV2 Fc receptor or an immunogenic fragment or variant thereof for the use described herein wherein:

- the cross-reactive immune response comprises a cross serotype antibody response against HSV1 and
- the HSV2 Fc receptor is part of a heterodimer with a binding partner from HSV or a fragment thereof, preferably wherein the HSV2 Fc receptor is HSV2 gE2 or an immunogenic fragment or variant thereof.

In a further embodiment is provided a HSV1 Fc receptor or an immunogenic fragment or variant thereof for the use described herein wherein:

- the cross-reactive immune response comprises a cross serotype antibody response against HSV2 and
- the HSV1 Fc receptor is part of a heterodimer with a binding partner from HSV or a fragment thereof, preferably wherein the HSV1 Fc receptor is HSV1 gE1 or an immunogenic fragment or variant thereof.

In one embodiment is provided a HSV2 Fc receptor or an immunogenic fragment or variant thereof for use in inducing a cross reactive immune response against HSV1 when administered to a subject and for use as a pan-HSV vaccine, wherein:

- the cross-reactive immune response is a cross serotype antibody response against HSV1 and
- the HSV2 Fc receptor is part of a heterodimer with a binding partner from HSV or a fragment thereof, preferably wherein the HSV2 Fc receptor is HSV2 gE2 or an immunogenic fragment or variant thereof.

In a further embodiment is provided a HSV1 Fc receptor or an immunogenic fragment or variant thereof for use in inducing a cross reactive immune response against HSV2 when administered to a subject and for use as a pan-HSV vaccine, wherein:

- the cross-reactive immune response is a cross serotype antibody response against HSV2 and
- the HSV1 Fc receptor is part of a heterodimer with a binding partner from HSV or a fragment thereof, preferably wherein the HSV1 Fc receptor is HSV1 gE1 or an immunogenic fragment or variant thereof.

In one embodiment is provided a HSV2 Fc receptor or an immunogenic fragment or variant thereof for the use described herein wherein:

- the cross-reactive immune response comprises a cross serotype antibody response against HSV1 and
- said use does not comprise administration to the subject together with HSV2 gD2.

In a further embodiment is provided a HSV1 Fc receptor or an immunogenic fragment or variant thereof for the use described herein wherein:

- the cross-reactive immune response is a cross serotype antibody response against HSV2 and
- said use does not comprise administration to the subject together with HSV1 gD 1.

In one embodiment, there is provided a HSV2 Fc receptor or an immunogenic fragment or variant thereof for the use described herein, wherein the cross reactive immune response comprises a cross serotype antibody response, a cross serotype cytotoxic antibody response, a cross serotype T cell response and/or generation of antibodies that can inhibit the immune evasion activity of HSV.

In a further embodiment, there is provided a HSV1 Fc receptor or an immunogenic fragment or variant thereof for the use described herein, wherein the cross reactive immune response comprises a cross serotype antibody response, a cross serotype cytotoxic antibody response, a cross serotype T cell response and/or generation of antibodies that can inhibit the immune evasion activity of HSV.

In one embodiment is provided a HSV2 Fc receptor or an immunogenic fragment or variant thereof for the use described herein wherein:

- the cross-reactive immune response comprises the generation of antibodies that can inhibit the immune evasion activity of HSV and
- said use does not comprise administration to the subject together with HSV2 gD2 or a fragment thereof comprising immunodominant epitopes.

In a further embodiment is provided a HSV1 Fc receptor or an immunogenic fragment or variant thereof for the use described herein wherein:

- the cross-reactive immune response comprises the generation of antibodies that can inhibit the immune evasion activity of HSV and
- said use does not comprise administration to the subject together with HSV1 gD1 or a fragment thereof comprising immunodominant epitopes.

In one embodiment is provided a HSV2 Fc receptor or an immunogenic fragment or variant thereof for the use described herein wherein:

- the cross-reactive immune response comprises a cross serotype T cell response and
- said use does not comprise administration to the subject together with HSV2 gD2 or a fragment thereof comprising immunodominant epitopes.

In a further embodiment is provided a HSV1 Fc receptor or an immunogenic fragment or variant thereof for the use described herein wherein:

- the cross-reactive immune response comprises a cross serotype T cell response and
- said use does not comprise administration to the subject together with HSV1 gD1 or a fragment thereof comprising immunodominant epitopes.

In one embodiment is provided a HSV2 Fc receptor or an immunogenic fragment or variant thereof for the use described herein wherein:

- the cross-reactive immune response comprises a cross serotype T cell response and
- said use does not comprise administration to the subject together with HSV2 gD2 or a fragment thereof comprising immunodominant epitopes.

In a further embodiment is provided a HSV1 Fc receptor or an immunogenic fragment or variant thereof for the use described herein wherein:

- the cross-reactive immune response comprises a cross serotype T cell response and
- said use does not comprise administration to the subject together with HSV1 gD1 or a fragment thereof comprising immunodominant epitopes.

*Herpes simplex virus and immune evasion*

Alphaherpesviruses, such as herpes simplex virus (HSV), have evolved specialized mechanisms enabling virus spread in epithelial and neuronal tissues. Primary infection involves entry into mucosal epithelial cells, followed by rapid virus spread between these cells. During this phase of virus replication and spread, viruses enter sensory neurons by fusion of the virion envelope with neuronal membranes so that capsids are delivered into the cytoplasm. Capsids undergo retrograde axonal transport on microtubules toward neuronal cell bodies or nuclei in ganglia, where latency is established. Later, following stimulation of neurons, latent virus reactivates and there is production of virus particles that undergo fast axonal transport on microtubules in the anterograde direction from cell bodies to axon tips. An essential phase of the life cycle of herpes simplex virus (HSV) and other alphaherpesviruses is the capacity to reactivate from latency and then spread from infected neurons to epithelial tissues. This spread involves at least two steps: (i) anterograde transport to axon tips followed by (ii) exocytosis and extracellular spread from axons to epithelial cells. HSV gE/gI is a heterodimer formed from two viral membrane glycoproteins, gE and gI. The HSV gE/gI heterodimer has been shown to facilitate virus spread. (Howard, Paul W., et al. "Herpes simplex virus gE/gI extracellular domains promote axonal transport and spread from neurons to epithelial cells." Journal of virology 88.19 (2014): 11178-11186.)

When HSV1 or HSV2 reactivates in an infected cell, the virus becomes more visible to the immune system and therefore more vulnerable. Typically, host IgG recognize viral antigens on the virion or at the cell surface of infected cells and the host IgG Fc domain can mediate important antibody effector activities by interacting with Fc gamma receptor on NK cells, granulocytes and macrophages to trigger antibody-dependent cellular cytotoxicity (ADCC), and by interacting with Fc gamma receptor on macrophages, monocytes, neutrophils and dendritic cells to trigger antibody-dependent cellular phagocytosis (ADCP).

HSV1 or HSV2 gE can form a noncovalent heterodimer complex with HSV1 or HSV2 (respectively) glycoprotein I (gI). The gEgI heterodimer functions as a viral Fc gamma receptor (FcγR), meaning it has the capacity to interact with the Fc portion of human IgG. Indeed, HSV1 or HSV2 gE or gE/gI heterodimer, when displayed at the cell surface of HSV infected cells, binds host IgG through their Fc portion. The interaction between gE and gI is thought to increase Fc binding affinity by a factor of about a hundred as compared to gE alone. This interaction has been linked to an immune evasion mechanism. Indeed, human IgGs which can bind HSV1 or HSV2 antigens (for example gD) on the virion or infected cell through the IgG Fab domain can also bind the Fc binding domain on the viral gE through their Fc domain, leading to endocytosis of the immune complex through a clathrin-mediated mechanism. This mechanism is referred to as antibody bipolar bridging and is postulated to be a major immune evasion strategy competing with innate immune cell activation. Through antibody Fc binding, the viral FcγR inhibits IgG Fc-mediated activities, including complement binding and antibody-dependent cellular cytotoxicity (ADCC) allowing the virus to circumvent the recognition by the immune system. (Ndjamen, Blaise, et al. "The herpes virus Fc receptor gE-gI mediates antibody bipolar bridging to clear viral antigens from the cell surface." PLoS pathogens 10.3 (2014): e1003961.; Dubin, G., et al. "Herpes simplex virus type 1 Fc receptor protects infected cells from antibody-dependent cellular cytotoxicity." Journal of virology 65.12 (1991): 7046-7050.; Sprague, Elizabeth R., et al. "Crystal structure of the HSV1 Fc receptor bound to Fc reveals a mechanism for antibody bipolar bridging." PLoS biology 4.6 (2006): e148.)

HSV2 prophylactic subunit gD2 vaccines did not efficiently prevent HSV-2 disease or infection in human trials (Johnston, Christine, Sami L. Gottlieb, and Anna Wald. "Status of vaccine research and development of vaccines for herpes simplex virus." Vaccine 34.26 (2016): 2948-2952). Another HSV2 vaccine candidate based on a truncated gD2 and ICP4.2 antigens adjuvanted with Matrix-M2 reduced genital HSV2 shedding and lesion rates in a phase 2 trial (Van Wagoner, Nicholas, et al. "Effects of different doses of GEN-003, a therapeutic vaccine for genital herpes simplex virus-2, on viral shedding and lesions: results of a randomized placebo-controlled trial." The Journal of infectious diseases 218.12 (2018): 1890-1899.) A trivalent adjuvanted vaccine including a virus entry molecule (gD2) and two antigens that block HSV2 immune evasion, gC2 which inhibits complement and gE2, showed efficacy in animal models. (Awasthi, Sita, et al. "Blocking herpes simplex virus 2 glycoprotein E immune evasion as an approach to enhance efficacy of a trivalent subunit antigen vaccine for genital herpes." Journal of virology 88.15 (2014): 8421-8432.; Awasthi, Sita, et al. "An HSV2 trivalent vaccine is immunogenic in rhesus macaques and highly efficacious in guinea pigs." PLoS pathogens 13.1 (2017): e1006141.; Awasthi, Sita, et al. "A trivalent subunit antigen glycoprotein vaccine as immunotherapy for genital herpes in the guinea pig genital infection model." Human vaccines & immunotherapeutics 13.12 (2017): 2785-2793.; Hook, Lauren M., et al. "A trivalent gC2/gD2/gE2 vaccine for herpes simplex virus generates antibody responses that block immune evasion domains on gC2 better than natural infection." Vaccine 37.4 (2019): 664-669.)

Directing an immune response against the Fc binding domain of gE may prevent or interfere with the above described immune evasion mechanism (antibody bipolar bridging), allowing natural immunity to viral proteins, in particular the immune-dominant HSV1 or HSV2 gD antigen, to become more potent. Specific targeting of gE or the gE/gI heterodimer through vaccination may enhance subdominant immune responses, in particular antibody dependent cellular cytotoxicity (ADCC) and antibody dependent cellular phagocytosis (ADCP), and redirect the immune system to protective mechanisms. Without wishing to be bound by theory, the present inventors thus believe that specifically raising a cross reactive immune response through vaccination with gE alone or in combination with its heterodimer binding partner gI could effectively treat subjects infected with HSV1 or HSV2 or prevent subjects from being infected with HSV1 or HSV2. In some aspects, specifically raising a cross-reactive immune response through vaccination with gE alone or in combination with its heterodimer binding partner gI could effectively treat subjects to reduce or abolish latent viral infection with HSV1 or HSV2 and/or treat or reduce acute infection arising from the latent viral infection with HSV1 or HSV2.

For the treatment of subjects that have already been infected by a herpes virus (seropositive subjects), whether they are in a symptomatic or asymptomatic phase, it may not be necessary to include an immunodominant antigen such as HSV gD in a therapeutic composition. Indeed, gD is a dominant antigen and seropositive subjects, whether symptomatic or asymptomatic, already have high levels of naturally generated neutralising antibodies against gD (Cairns, Tina M., et al. "Patient-specific neutralizing antibody responses to herpes simplex virus are attributed to epitopes on gD, gB, or both and can be type specific." Journal of virology 89.18 (2015): 9213-9231.). By inducing an immune response against a viral Fc receptor such as HSV gE or gE/gI, the present inventors have hypothesized the gE/gI immune evasion mechanism will be circumvented and the natural immunity will more fully play its role, in particular the natural antibody responses directed against immunodominant antigens such as gD. In addition to acting on the immune evasion mechanism, the gE or gE/gI antigen may also induce a humoral response (anti gE or anti gE and gI antibodies) that would lead to the destruction of infected cells by cytotoxic and/or phagocytic mechanisms (ADCC/ADCP). In the case of seropositive subjects, the ADCC and/or ADCP mechanisms may be more efficient than neutralising antibody mechanisms (such as the response driven by the dominant HSV antigen gD) to control at early stage viral replication. Finally, it is hypothesised that the induction of CD4+ T cells with a gE or gE/gI antigen would also be helpful against recurrent HSV infections.

*Fc receptor*

In a first aspect, the invention provides a HSV-2 Fc receptor or an immunogenic fragment or variant thereof for use in inducing a cross reactive immune response against HSV-1 when administered to a subject. In a second aspect, is provided a HSV-1 Fc receptor or an immunogenic fragment or variant thereof for use in inducing a cross reactive immune response against HSV-2 when administered to a subject.

As used herein, an **"Fc receptor"** (or "**FcR**") is a protein found at the surface of certain cells and which has the ability to bind the Fc region of an antibody. Fc receptors are classified based on the type of antibody that they recognize. Fc receptors which bind IgG, the most common class of antibody, are referred to as **"Fc-gamma receptors"** (or "**FcγR**"), those that bind IgA are called "Fc-alpha receptors" (or "FcaR") and those that bind IgE are called "Fc-epsilon receptors" (or "FcεR"). Herein, Fc receptors displayed on the surface of cells from a given multicellular organism as a result of the expression of endogenous genes are referred to as **"host Fc receptors".** Host Fc receptors are found in particular on the surface of host immune effector cells such as B lymphocytes, follicular dendritic cells, natural killer (NK) cells, macrophages, neutrophils, eosinophils, basophils, human platelets, and mast cells. The binding of host Fc receptors to the Fc region of antibodies that are bound to infected cells or invading pathogens through their Fab region triggers phagocytosis or destruction of the infected cells or invading pathogens by antibody-mediated cellular phagocytosis (ADCP) or antibody-dependent cellular cytotoxicity (ADCC).

Suitably, the FcR or immunogenic fragment thereof is in a subunit form, which means that it is not part of a whole virus. Suitably, the FcR or immunogenic fragment thereof is isolated.

Some viruses, in particular herpes viruses, express viral Fc receptors that bind the Fc portion of the host IgGs, thereby preventing binding of the IgG to host Fc receptors on immune effector cells and allowing the virus to evade host ADCC or ADCP immune responses.

In one embodiment, the HSV Fc receptor is a FcyR. Preferably, the FcyR is selected from HSV2 gE2 and HSV1gE1.

Herein, a **"HSV2 gE2"** (or **"HSV2 gE"**) is a HSV2 gE glycoprotein encoded by HSV2 gene US8 and displayed on the surface of infected cells and which functions as a viral FcγR. Suitably, the HSV2 gE2 is selected from the HSV2 gE glycoproteins shown in Table 1 or variants therefrom which are at least 60%, 65%, 70%, 75% 80%, 85%, 90%, 95%, 96%, 97%, 98%, 99% or 99,5% identical thereto.

**Table 1 - HSV2 gE glycoproteins and ectodomains**

| Genbank accession number | SEQ ID NO | Ectodomain |
|---|---|---|
| AHG54732.1 | 1 | 1-419 |
| AKC59449.1 | 13 | 1-419 |
| AKC42830.1 | 14 | 1-419 |
| ABU45436.1 | 15 | 1-419 |
| ABU45439.1 | 16 | 1-419 |
| ABU45437.1 | 17 | 1-419 |
| ABU45438.1 | 18 | 1-419 |
| AMB66104.1 | 19 | 1-416 |
| AMB66173.1 | 20 | 1-416 |
| AMB66246.1 | 21 | 1-419 |
| AKC59520.1 | 22 | 1-419 |
| AKC59591.1 | 23 | 1-419 |
| AKC59307.1 | 24 | 1-419 |
| AMB66465.1 | 25 | 1-419 |
| AKC59378.1 | 26 | 1-419 |
| AEV91407.1 | 27 | 1-416 |
| CAB06715.1 | 28 | 1-416 |
| YP_009137220.1 | 29 | 1-416 |
| ABW83306.1 | 30 | 1-419 |
| ABW83324.1 | 31 | 1-419 |
| ABW83308.1 | 32 | 1-419 |
| ABW83310.1 | 33 | 1-419 |
| ABW83312.1 | 34 | 1-419 |
| ABW83314.1 | 35 | 1-419 |
| ABW83316.1 | 36 | 1-419 |
| ABW83318.1 | 37 | 1-419 |
| ABW83320.1 | 38 | 1-419 |
| ABW83322.1 | 39 | 1-419 |
| ABW83398.1 | 40 | 1-419 |
| ABW83380.1 | 41 | 1-419 |
| ABW83396.1 | 42 | 1-416 |
| ABW83382.1 | 43 | 1-419 |
| ABW83384.1 | 44 | 1-419 |
| ABW83394.1 | 45 | 1-416 |
| ABW83386.1 | 46 | 1-419 |
| ABW83388.1 | 47 | 1-419 |
| ABW83390.1 | 48 | 1-419 |
| ABW83392.1 | 49 | 1-419 |

(continued)

| Genbank accession number | SEQ ID NO | Ectodomain |
|---|---|---|
| ABW83400.1 | 50 | 1-419 |
| ABW83342.1 | 51 | 1-419 |
| ABW83340.1 | 52 | 1-419 |
| ABW83346.1 | 53 | 1-419 |
| ABW83348.1 | 54 | 1-419 |
| ABW83326.1 | 55 | 1-419 |
| ABW83350.1 | 56 | 1-419 |
| ABW83352.1 | 57 | 1-419 |
| ABW83336.1 | 58 | 1-419 |
| ABW83334.1 | 59 | 1-419 |
| ABW83354.1 | 60 | 1-419 |
| ABW83338.1 | 61 | 1-419 |

In a preferred embodiment, the HSV2 gE2 is the gE from HSV2 strain SD90e (Genbank accession number AHG54732.1, UniProtKB accession number: A7U881) which has the amino acid sequence shown in SEQ ID NO:1, or a variant therefrom which is at least 60%, 65%, 70%, 75% 80%, 85%, 90%, 95%, 96%, 97%, 98%, 99% or 99,5% identical thereto.

As used herein, a **"Variant"** is a peptide sequence that differs in sequence from a reference antigen sequence but retains at least one essential property of the reference antigen. Changes in the sequence of peptide variants may be limited or conservative, so that the sequences of the reference peptide and the variant are closely similar overall and, in many regions, identical. A variant and reference antigen can differ in amino acid sequence by one or more substitutions, additions or deletions in any combination. A variant of an antigen can be naturally occurring such as an allelic variant, or can be a variant that is not known to occur naturally. Non-naturally occurring variants of nucleic acids and polypeptides may be made by mutagenesis techniques or by direct synthesis. In a preferred embodiment, the essential property retained by the variant is the ability to induce an immune response, suitably a humoral or T cell response, which is similar to the immune response induced by the reference antigen. Suitably, the variant induces a humoral or T cell response in mice which is not more than 10-fold lower, more suitably not more than 5-fold lower, not more than 2-fold lower or not lower, than the immune response induced by the reference antigen.

Herein, a **"HSV1 gE1"** (or "**HSV1 gE**") is a HSV1 gE glycoprotein encoded by HSV1 gene US8 and displayed on the surface of infected cells and which functions as a viral FcγR. Suitably, the HSV1 gE1 is the gE from HSV1 strain KOS321 (UniProtKB accession number: Q703E9) which has the amino acid sequence shown in SEQ ID NO:3, or a variant therefrom which is at least 60%, 65%, 70%, 75% 80%, 85%, 90%, 95%, 96%, 97%, 98%, 99% or 99,5% identical thereto.

In one embodiment, an immunogenic fragment of a HSV Fc receptor is used.

As used herein, an **"immunogenic fragment"** refers to a fragment of a reference antigen containing one or more epitopes (e.g., linear, conformational or both) capable of stimulating a host's immune system to make a humoral and/or cellular antigen-specific immunological response (i.e. an immune response which specifically recognizes a naturally occurring polypeptide, e.g., a viral or bacterial protein). An **"epitope"** is that portion of an antigen that determines its immunological specificity. T- and B-cell epitopes can be identified empirically (e.g. using PEPSCAN or similar methods). In a preferred embodiment, the immunogenic fragment induces an immune response, suitably a humoral or T cell response, which is similar to the immune response induced by the reference antigen. Suitably, the immunogenic fragment induces a humoral or T cell response in mice which is not more than 10-fold lower, more suitably not more than 5-fold lower, not more than 2-fold lower or not lower, than the immune response induced by the reference antigen.

As used herein, an **"immunogenic fragment of a HSV Fc receptor"** refers to a fragment of a naturally-occurring viral Fc receptor of at least 10, 15, 20, 30, 40, 50, 60, 100, 200, 300 or more amino acids, or a peptide having an amino acid sequence of at least 60%, 65%, 70%, 75%, 80%, 85%, 90%, 95%, 97%, 98%, 99%, or 99.5% sequence identity to a naturally-occurring viral Fc receptor (or to a fragment of a naturally-occurring viral Fc receptor of at least about 10, 15, 20, 30, 40, 50, 60 or more amino acids). Thus, an immunogenic fragment of an antigenic viral Fc receptor may be a fragment of a naturally occurring viral Fc receptor, of at least 10 amino acids, and may comprise one or more amino acid substitutions, deletions or additions.

Any of the encoded HSV Fc receptor immunogenic fragments may additionally comprise an initial methionine residue where required.

Suitably, the HSV Fc receptor or immunogenic fragment thereof does not comprise a functional transmembrane domain. Suitably, the HSV Fc receptor or immunogenic fragment thereof does not comprise a cytoplasmic domain. Preferably, the HSV Fc receptor or immunogenic fragment thereof neither comprises a functional transmembrane domain, nor a cytoplasmic domain. In other words, in a preferred embodiment, the HSV Fc receptor or immunogenic fragment consists of a HSV FcR ectodomain (or extracellular domain). More preferably, the HSV Fc receptor or immunogenic fragment comprises or consists of a HSV2 gE2 ectodomain or a HSV1 gE1 ectodomain.

In a preferred embodiment, the HSV FcR ectodomain is a HSV2 gE2 ectodomain which comprises or consists of the amino acid sequence shown on SEQ ID NO: 7 (corresponding to amino acid residues 1-419 of SEQ ID NO: 1), or a sequence which is at least 60%, 65%, 70%, 75% 80%, 85%, 90%, 95%, 96%, 97%, 98%, 99% or 99,5% identical thereto. Suitably, the viral FcR ectodomain has a sequence selected from the sequences shown in Table 1 or FIG. 77. In a preferred embodiment, the viral FcR ectodomain is a HSV2 gE2 ectodomain which is at least 90%, 95%, 96%, 97%, 98%, 99%, 99,5% or 100% identical to SEQ ID NO: 7.

Suitably, the HSV2 Fc receptor ectodomain may comprise one or more amino acid residue substitutions, deletions, or insertion relative to the amino acid sequence shown at SEQ ID NO: 7, for example 1, 2, 3, 4, 5, 6, 7, 8, 9 or 10 amino acid residue substitutions, deletions, or insertions.

In another embodiment, the HSV2 FcR ectodomain is a HSV2 gE2 ectodomain which comprises or consists of the amino acid sequence corresponding to amino acid residues 1-417 of SEQ ID NO: 1, or a sequence which is at least 60%, 65%, 70%, 75% 80%, 85%, 90%, 95%, 96%, 97%, 98%, 99% or 99,5% identical thereto. Suitably, the HSV2 Fc receptor ectodomain may comprise one or more amino acid residue substitution, deletion, or insertion relative to the amino acid sequence corresponding to amino acid residues 1-417 of SEQ ID NO: 1, for example 1, 2, 3, 4, 5, 6, 7, 8, 9 or 10 amino acid residue substitutions, deletions, or insertions.

In another embodiment, the HSV1 FcR ectodomain is a HSV1 gE1 ectodomain which comprises or consists of the amino acid sequence shown in SEQ ID NO: 9 (corresponding to amino acid residues 1-421 of SEQ ID NO: 3), or a sequence which is at least 60%, 65%, 70%, 75% 80%, 85%, 90%, 95%, 96%, 97%, 98%, 99% or 99,5% identical thereto. In a preferred embodiment, the HSV1 FcR ectodomain is a HSV1 gE1 ectodomain which is at least 90%, 95%, 96%, 97%, 98%, 99%, 99,5% or 100% identical to SEQ ID NO: 9.

Suitably, the HSV Fc receptor HSV1 ectodomain may comprise one or more amino acid residue substitution, deletion, or insertion relative to the amino acid sequence shown in SEQ ID NO: 9, for example 1, 2, 3, 4, 5, 6, 7, 8, 9 or 10 amino acid residue substitutions, deletions, or insertions.

In another embodiment, the HSV1 FcR HSV1 ectodomain comprises or consists of the amino acid sequence corresponding to amino acid residues 1-419 of SEQ ID NO: 3, or a sequence which is at least 60%, 65%, 70%, 75% 80%, 85%, 90%, 95%, 96%, 97%, 98%, 99% or 99,5% identical thereto. Suitably, the HSV1 Fc receptor HSV1 ectodomain may comprise one or more amino acid residue substitutions, deletions, or insertions relative to the amino acid sequence corresponding to amino acid residues 1-419 of SEQ ID NO: 3, for example 1, 2, 3, 4, 5, 6, 7, 8, 9 or 10 amino acid residue substitutions, deletions, or insertions.

In another embodiment, the immunogenic fragment of a HSV1 or HSV2 FcR comprises or consists of a Fc binding domain from a HSV FcR, or a variant thereof.

In one embodiment, the immunogenic fragment of a HSV2 FcR comprises or consists of a Fc binding domain from a HSV2 gE, for example the amino acid sequence corresponding to amino acid residues 233-378 of SEQ ID NO: 1, or a sequence which is at least 60%, 65%, 70%, 75% 80%, 85%, 90%, 95%, 96%, 97%, 98%, 99% or 99,5% identical thereto. Suitably, the viral Fc receptor HSV2 Fc binding domain may comprise one or more amino acid residue substitutions, deletions, or insertions relative to the amino acid sequence corresponding to amino acid residues 233-378 of SEQ ID NO: 1, for example 1, 2, 3, 4, 5, 6, 7, 8, 9 or 10 amino acid residue substitutions, deletions, or insertions.

In one embodiment, the immunogenic fragment of a HSV1 FcR comprises or consists of a Fc binding domain from a HSV1 gE, for example the amino acid sequence corresponding to amino acid residues 235-380 of SEQ ID NO: 3, or a sequence which is at least 60%, 65%, 70%, 75% 80%, 85%, 90%, 95%, 96%, 97%, 98%, 99% or 99,5% identical thereto. Suitably, the HSV1 Fc receptor HSV1 Fc binding domain may comprise one or more amino acid residue substitutions, deletions, or insertions relative to the amino acid sequence corresponding to amino acid residues 235-380 of SEQ ID NO: 3, for example 1, 2, 3, 4, 5, 6, 7, 8, 9 or 10 amino acid residue substitutions, deletions, or insertions.

In a preferred embodiment, the ability of the HSV Fc receptor or immunogenic fragment or variant thereof to bind to a human antibody Fc domain is reduced or abolished compared to the corresponding native HSV Fc receptor. Suitably, the HSV Fc receptor or immunogenic fragment thereof comprises one or more amino acid substitutions, deletions or insertions compared to the native sequence of the HSV Fc receptor or immunogenic fragment thereof, that reduce or abolish the binding affinity between the HSV FcR or immunogenic fragment or variant thereof and the antibody Fc domain compared to the native HSV Fc receptor.

The binding affinity between the HSV FcR or immunogenic fragment or variant thereof and the antibody Fc domain can be determined by methods well known to those skilled in the art. For example, the association rate ($k_{on}$), dissociation rate ($k_{off}$), equilibrium dissociation constant ($K_D = k_{off} / k_{on}$) and equilibrium association constant ($K_A = 1/ K_D = k_{on} / k_{off}$) can be determined by BiLayer Interferometry.

In a preferred embodiment, the $k_{on}$ between the HSV FcR or immunogenic fragment or variant thereof and human IgGs is lower than the $k_{on}$ between the corresponding native HSV FcR and human IgGs (slow binder).

In a preferred embodiment, the $k_{off}$ between the HSV FcR or immunogenic fragment or variant thereof and human IgGs is higher than the $k_{off}$ between the corresponding native HSV FcR and human IgGs (fast releaser).

In a more preferred embodiment, the $k_{on}$ between the HSV FcR or immunogenic fragment or variant thereof and human IgGs is lower than the $k_{on}$ between the corresponding native HSV FcR and human IgGs, and the $k_{off}$ between the HSV FcR or immunogenic fragment or variant thereof and human IgGs is higher than the $k_{off}$ between the corresponding native HSV FcR and human IgGs (slow binder / fast releaser).

In a preferred embodiment, the equilibrium dissociation constant ($K_D$) between the HSV FcR or immunogenic fragment or variant thereof and human IgGs is higher than the $K_D$ between the corresponding native HSV FcR and human IgGs.

The relative affinity between the HSV FcR or immunogenic fragment or variant thereof and human IgGs can be determined by dividing the $K_D$ determined for the native HSV FcR by the $K_D$ determined for the HSV FcR or immunogenic fragment or variant thereof.

In a preferred embodiment, the relative affinity between the HSV FcR or immunogenic fragment or variant thereof and human IgGs is less than 100%, for example less than 90%, 80%, 70%, 60%, 50%, 40%, 30%, 20%, 15% or 10% of the affinity between the corresponding native HSV FcR and human IgGs. In a more preferred embodiment, the relative affinity between the HSV FcR or immunogenic fragment or variant thereof and human IgGs is less than 15%, more preferably still less than 10% of the affinity between the corresponding native HSV FcR and human IgGs.

In a preferred embodiment, the equilibrium dissociation constant ($K_D$) between the HSV FcR or immunogenic fragment or variant thereof and human IgGs is higher than $2 \times 10^{-7}$ M, preferably higher than $5 \times 10^{-7}$ M, more preferably higher than $1 \times 10^{-6}$ M.

Alternatively, the ability of the HSV Fc receptor or immunogenic fragment thereof to bind to a human antibody Fc domain can be assessed by measuring the response (expressed in nm) in a BiLayer Interferometry assay.

In a preferred embodiment, the response in a BiLayer Interferometry assay corresponding to the binding between the HSV Fc receptor or immunogenic fragment or variant thereof and human IgGs is less than 80%, suitably less than 70%, 60%, 50%, 40% of the response obtained with the corresponding native HSV Fc receptor. In a preferred embodiment, the response in a BiLayer Interferometry assay corresponding to the binding between the HSV Fc receptor or immunogenic fragment or variant thereof and human IgGs is lower than 0.4 nm, suitably lower than 0.3 nm, 0.2 nm or 0.1 nm.

Suitably, the HSV2 gE2 or immunogenic fragment or variant thereof comprises one or more mutations (insertions, substitutions or deletions) at positions selected from N241, H245, A246, A248, R314, P317, P318, P319, F322, R320, A337, S338 or V340 of the HSV2 gE2 sequence shown in SEQ ID NO: 1.

Exemplary mutations that may be used herein to reduce or abolish the binding affinity between HSV2 gE2 or immunogenic fragment or variant thereof and an antibody Fc domain include the single point substitution mutations of the sequence shown in SEQ ID NO: 1 selected from H245A, H245K, P317R, P319A, P319R, P319G, P319K, P319T, A337G, P319D, P319S, S338D, N241A, R320D, H245E, H245V, H245R, H245D, H245Q, H245G, H245I, H245K, H245S, H245T, A246W, A248K, A248T, A248G, R314A, R314N, R314D, R314Q, R314E, R314G, R314I, R314L, R314K, R314M, R314F, R314P, R314S, R314T, R314Y, R314V, P317N, P317G, P317I, P317L, P317K, P317F, P317S, P318R, P318D, P318Q, P318I, P318S, P318T, P318Y, P319L, R320A, R320S, R320N, R320Q, R320E, R320G, R320H, R320I, R320L, R320M, R320P, R320T, R320V, F322A, F322N, F322I, F322K, F322P, F322T, S338G, S338E, S338L, S338T, V340A, V340R, V340D, V340Q, V340M, V340F, V340P and V340W.

Exemplary mutations that may be used herein to reduce or abolish the binding affinity between HSV2 gE2 or immunogenic fragment thereof and an antibody Fc domain also include the double point substitution mutations of the sequence shown in SEQ ID NO: 1 selected from H245A and P319A; H245A and P319R; H245A and P319G; H245A and P319K; H245A and P319T; N241A and R320D; N241A and P319D; A246W and P317K; A246W and P317F; A246W and P317S; A246W and R320D; A246W and R320G; A246W and R320T; A248K and V340R; A248K and V340M; A248K and V340W; A248T and V340R; A248T and V340M; A248T and V340W; A248G and V340R; A248G and V340M; A248G and V340W; A248K and F322A; A248K and F322I; A248K and F322P; A248T and F322A; A248T and F322I; A248T and F322P; A248G and F322A; A248G and F322I; A248G and F322P; H245A and R320D; H245A and R320G; H245A and R320T; H245G and R320D; H245G and R320G; H245G and R320T; H245S and R320D; H245S and R320G; H245S and R320T; H245A and P319G; H245A and P319L; H245G and P319G; H245G and P319L; H245S and P319G; H245S and P319L; R314G and P318R; R314G and P318D; R314G and P318I; R314L and P318R; R314L and P318D; R314L and P318I; R314P and P318R; R314P and P318D; R314P and P318I; R314G and F322A; R314G and F322I; R314G and F322P; R314L and F322A; R314L and F322I; R314L and F322P; R314P and F322A; R314P and F322I; R314P and F322P; R314G and V340R; R314G and V340M; R314G and V340W; R314L and V340R; R314L and V340M;

R314L and V340W; R314P and V340R; R314P and V340M; R314P and V340W; P317K and V340R; P317K and V340M; P317K and V340W; P317F and V340R; P317F and V340M; P317F and V340W; P317S and V340R; P317S and V340M; P317S and V340W; P317K and S338G; P317K and S338H; P317K and S338L; P317F and S338G; P317F and S338H; P317F and S338L; P317S and S338G; P317S and S338H; P317S and S338L; P318R and S338G; P318R and S338H; P318R and S338L; P318D and S338G; P318D and S338H; P318D and S338L; P318I and S338G; P318I and S338H; P318I and S338L; P319G and V340R; P319G and V340M; P319G and V340W; P319L and V340R; P319L and V340M; P319L and V340W; P317R and P319D; P317R and R320D; P319D and R320D.

Exemplary mutations that may be used herein to reduce or abolish the binding affinity between HSV2 gE2 or immunogenic fragment or variant thereof and an antibody Fc domain also include deletion mutations at positions P319 and/or R320 of the sequence shown in SEQ ID NO: 1, alone or in combination with substitution mutations, in particular mutations selected from P319 deletion; R320 deletion; P319 deletion / R320 deletion; P319 deletion / R320 deletion / P317G / P318G; P319 deletion / R320 deletion / P318E; P319 deletion / R320 deletion /P318G; P319 deletion / R320 deletion / P318K; P319 deletion / R320 deletion / P317R / P318E; P319 deletion / R320 deletion / P317R / P318G; P319 deletion / R320 deletion / P317R / P318K; P319 deletion / R320 deletion / P317G / P318K.

Exemplary mutations that may be used herein to reduce or abolish the binding affinity between HSV2 gE2 or immunogenic fragment or variant thereof and an antibody Fc domain also include the insertion mutations selected from:

- insertion of peptide sequence LDIGE between amino acid residues Y275 and E276 of SEQ ID NO: 1 (275_insert_LDIGE),
- insertion of peptide sequence ADIGL between amino acid residues S289 and P290 of SEQ ID NO: 1 (289_insert_ADIGL),
- insertion of peptide sequence ARAA between amino acid residues A337 and S338 of SEQ ID NO: 1 (337_insert_ARAA),
- insertion of peptide sequence ARAA between amino acid residues S338 and T339 of SEQ ID NO: 1 (338_insert_ARAA), and
- insertion of peptide sequence ADIT between amino acid residues H346 and A347 of SEQ ID NO: 1 (346_insert_ADIT).

In a preferred embodiment, the HSV2 gE2 or immunogenic fragment or variant thereof comprises a mutation or a combination of mutations with respect to the sequence shown in SEQ ID NO: 1 selected from 289_insert ADIGL; 338_insert ARAA; H245K; P317R; P319R; P319G; P319K; H245A_P319R; H245A_P319G; H245A_P319K; H245A_P319T; P319D; S338D; R320D; N241A_R320D; A248K_V340M; P318Y; A248K_V340R; A248T_V340W; A248K_V340W; A246W_R320G; A246W_P317K; A246W_R320D; A246W_R320T; V340W; A248G_V340W; H245G_R320D; P318D; A246W_P317F; P319G_V340W; A248T_V340M; P317K_V340W; V340F; V340D; H245A_R320D; P317F_V340W; A246W_P317S; H245S_R320D; R314G_P318D; A248T; P318S; P317K; P317S_V340W; H245D; R314P_V340W; R314L_318D; P319L_V340W; P317F; P318D_S338G; R314G_V340W; P317K_S338H; R314L_V340W; P318R; P318Q; P317F_S338G; R314G_P318I; H245G P319G; P317L; P318I; A248T_F322A; H245E; P318T; P318R_S338G; P318D_S338H; P317F_S338H; A248T_V340R; A248T_F322I; H245A_R320G; P318R_S338H; H245S_R320G; P317K S338G; A248T_F322P; V340R; R314L P318R; H245S_R320T; R314G_P318R; R320E; H245G_R320G; H245A_R320T; A246W; P318I_S338G; P317K_V340M; P317I; R320H; R314P_P318I; P318I_S338H; P317F_V340M; H245A_P319G; H245A_P319L; R320P; H245G_R320T; R314L_V340R; P319G V340R; R314G F322I; R314LP318I; R320A; R314N; P317F V340R; P318D_S338L; A248G_V340R; R314E; R314P_P318D; H245S_P319G; V340Q; A248K_F322I; R320G; H245S_P319L; R314F; P319L; P317K_S338L; P319L_V340M; P317G; R320S; R320Q; R314P_V340R; V340A; H245G_P319L; R320T; R314P_P318R; A248G_F322I; R320N; P317N; R314D; R314Y; R314P_F322I; P319G_V340M; P317S_V340R; R314V; P317R_P319D; P317R_R320D; P319D_R320D; Δ319_Δ320; P317G_P318G_Δ319_ Δ320; P318E_Δ319_Δ320; P318G_Δ319_Δ320; P318K_A319_Δ320; P317R_P318E_A319_320; P317R_P318G_Δ319_Δ320 and P317G_P318K_A319_Δ320. (Δ means deleted residue).

In a more preferred embodiment, the HSV2 gE2 or immunogenic fragment or variant thereof comprises a mutation or a combination of mutations with respect to the sequence shown in SEQ ID NO: 1 selected from 338_insert ARAA; P317R; P319D; R320D; A248T_V340W; V340W; A248T; P318I and A246W.

Corresponding mutations in other HSV2 gE2 sequences, for example the sequences listed in Table 1 and shown on the alignment presented in Figure 77, to the exemplary substitution, selection and insertion mutations listed above are also in the scope of the present invention.

All possible combinations of the exemplary single and double substitution mutations and insertion mutations listed above are also in the scope of the present invention.

Suitably, the HSV1 gE1 or immunogenic fragment or variant thereof comprises one or more mutations (insertions, substitutions or deletions) at positions selected from H247, P319 and P321 of the HSV1 gE1 sequence shown in SEQ ID NO: 3.

Exemplary mutations that may be used herein to reduce or abolish the binding affinity between HSV 1 gE1 or immunogenic fragment thereof and an antibody Fc domain include the single point substitution mutations of the sequence shown in SEQ ID NO: 3 selected from H247A, H247K, P319R, P321A, P321R, P321G, P321K, P321T, A339G, P321D, P321S, A340D, N243A and R322D, and the double point substitutions mutations of the sequence shown in SEQ ID NO: 3 selected from H247A/P321A, H247A/P321R, H247A/P321G, H247A/P321K, H247A/P321T, N243A/R322D, N243A/P321D, H247G/P319G, P319G/P321G and A340G/S341G/V342G.

Exemplary mutations that may be used herein to reduce or abolish the binding affinity between HSV 1 gE1 or immunogenic fragment or variant thereof and an antibody Fc domain also include the insertion mutations selected from:

- insertion of peptide sequence LDIGE between amino acid residues Y277 and E278 of SEQ ID NO: 3 (277_insert_LDIGE);
- insertion of peptide sequence ADIGL between amino acid residues S291 and P292 of SEQ ID NO: 3 (291_insert_ADIGL);
- insertion of peptide sequence ARAA between amino acid residues A339 and A340 of SEQ ID NO: 3 (339_inset_ARAA);
- insertion of peptide sequence ARAA between amino acid residues A340 and S341 of SEQ ID NO: 3 (340_inset_ARAA); and
- insertion of peptide sequence ADIT between amino acid residues D348 and A349 of SEQ ID NO: 3 (348_inset_ADIT).

In a preferred embodiment, the HSV1 gE1 or immunogenic fragment or variant thereof comprises a mutation or a combination of mutations with respect to the sequence shown in SEQ ID NO: 3 selected from P321K; P321D; R322D; N243A_R322D; N243A_P321D; A340G_S341G_V342G; H247G_P319G; P321R; H247A_P321K; 291insert ADIGL; 339_insert ARAA; P319R; P319G_P321G and H247A_P321R.

In a more preferred embodiment, the HSV1 gE1 or immunogenic fragment or variant thereof comprises a mutation or a combination of mutations with respect to the sequence shown in SEQ ID NO: 3 selected from P319R, P321D, R322D, N243A_R322D or A340G_S341G_V342G.

Corresponding mutations in other HSV1 gE1 sequences to the exemplary single and double substitution mutations and insertion mutations listed above are also in the scope of the present invention.

All possible combinations of the exemplary single and double substitution mutations and insertion mutations listed above are also in the scope of the present invention.

In a preferred embodiment, when the HSV Fc receptor or immunogenic fragment or variant thereof is a HSV FcR ectodomain, the ability of the ectodomain to bind to an antibody Fc domain is reduced or abolished compared to the native HSV Fc receptor. Suitably, the HSV FcR ectodomain comprises one or more amino acid substitutions, deletions or insertions compared to the native sequence of the HSV FcR ectodomain, that reduce or abolish the binding affinity between the HSV FcR ectodomain and the antibody Fc domain compared to the native HSV FcR.

The binding affinity between the HSV FcR ectodomain and the antibody Fc domain can be determined by methods described above.

In a preferred embodiment, the $k_{on}$ between the HSV FcR ectodomain and human IgGs is lower than the $k_{on}$ between the corresponding native HSV FcR ectodomain and human IgGs (slow binder).

In a preferred embodiment, the $k_{off}$between the HSV FcR ectodomain and human IgGs is higher than the $k_{off}$between the corresponding native HSV FcR ectodomain and human IgGs (fast releaser).

In a more preferred embodiment, the $k_{on}$ between the HSV FcR ectodomain and human IgGs is lower than the $k_{on}$ between the corresponding native HSV FcR ectodomain and human IgGs, and the $k_{off}$ between the HSV FcR ectodomain and human IgGs is higher than the $k_{off}$between the corresponding native HSV FcR ectodomain and human IgGs (slow binder / fast releaser).

In a preferred embodiment, the equilibrium dissociation constant ($K_D$) between the HSV FcR ectodomain and human IgGs is higher than the $K_D$ between the corresponding native HSV FcR ectodomain and human IgGs.

In a preferred embodiment, the relative affinity between the HSV FcR ectodomain and human IgGs is less than 100%, for example less than 90%, 80%, 70%, 60%, 50%, 40%, 30%, 20%, 15% or 10% of the affinity between the corresponding native HSV FcR ectodomain and human IgGs. In a more preferred embodiment, the relative affinity between the HSV FcR ectodomain and human IgGs is less than 15%, more preferably still less than 10% of the affinity between the corresponding native HSV FcR ectodomain and human IgGs.

In a preferred embodiment, the equilibrium dissociation constant ($K_D$) between the HSV FcR ectodomain and human IgGs is higher than $2 \times 10^{-7}$ M, preferably higher than $5 \times 10^{-7}$ M, more preferably higher than $1 \times 10^{-6}$ M.

Alternatively, the ability of the HSV FcR ectodomain to bind to a human antibody Fc domain can be assessed by measuring the response (expressed in nm) in a BiLayer Interferometry assay. In a preferred embodiment, the response in a BiLayer Interferometry assay corresponding to the binding between the HSV FcR ectodomain and human IgGs is less than 80%, suitably less than 70%, 60%, 50%, 40% of the response obtained with the corresponding native HSV FcR ectodomain. In a preferred embodiment, the response in a BiLayer Interferometry assay corresponding to the binding between the viral FcR ectodomain and human IgGs is lower than 0.6 nm, suitably lower than 0.5 nm, 0.4 nm, 0.3 nm or 0.2 nm.

Suitably, the HSV2 gE2 ectodomain comprises one or more mutations (insertions, substitutions or deletions) at positions selected from N241, H245, A246, A248, R314, P317, P318, P319, F322, R320, A337, S338 or V340 of the HSV2 gE2 ectodomain sequence shown in SEQ ID NO: 7.

Exemplary mutations that may be used herein to reduce or abolish the binding affinity between HSV2 gE2 ectodomain and an antibody Fc domain include the single point substitution mutations of the sequence shown in SEQ ID NO: 7 selected from H245A, H245K, P317R, P319A, P319R, P319G, P319K, P319T, A337G, P319D, P319S, S338D, N241A, R320D, H245E, H245V, H245R, H245D, H245Q, H245G, H245I, H245K, H245S, H245T, A246W, A248K, A248T, A248G, R314A, R314N, R314D, R314Q, R314E, R314G, R314I, R314L, R314K, R314M, R314F, R314P, R314S, R314T, R314Y, R314V, P317N, P317G, P317I, P317L, P317K, P317F, P317S, P318R, P318D, P318Q, P318I, P318S, P318T, P318Y, P319L, R320A, R320S, R320N, R320Q, R320E, R320G, R320H, R320I, R320L, R320M, R320P, R320T, R320V, F322A, F322N, F322I, F322K, F322P, F322T, S338G, S338E, S338L, S338T, V340A, V340R, V340D, V340Q, V340M, V340F, V340P and V340W.

Exemplary mutations that may be used herein to reduce or abolish the binding affinity between HSV2 gE2 ectodomain and an antibody Fc domain also include the double point substitution mutations of the sequence shown in SEQ ID NO: 7 selected from H245A and P319A; H245A and P319R; H245A and P319G; H245A and P319K; H245A and P319T; N241A and R320D; N241A and P319D; A246W and P317K; A246W and P317F; A246W and P317S; A246W and R320D; A246W and R320G; A246W and R320T; A248K and V340R; A248K and V340M; A248K and V340W; A248T and V340R; A248T and V340M; A248T and V340W; A248G and V340R; A248G and V340M; A248G and V340W; A248K and F322A; A248K and F322I; A248K and F322P; A248T and F322A; A248T and F322I; A248T and F322P; A248G and F322A; A248G and F322I; A248G and F322P; H245A and R320D; H245A and R320G; H245A and R320T; H245G and R320D; H245G and R320G; H245G and R320T; H245S and R320D; H245S and R320G; H245S and R320T; H245A and P319G; H245A and P319L; H245G and P319G; H245G and P319L; H245S and P319G; H245S and P319L; R314G and P318R; R314G and P318D; R314G and P318I; R314L and P318R; R314L and P318D; R314L and P318I; R314P and P318R; R314P and P318D; R314P and P318I; R314G and F322A; R314G and F322I; R314G and F322P; R314L and F322A; R314L and F322I; R314L and F322P; R314P and F322A; R314P and F322I; R314P and F322P; R314G and V340R; R314G and V340M; R314G and V340W; R314L and V340R; R314L and V340M; R314L and V340W; R314P and V340R; R314P and V340M; R314P and V340W; P317K and V340R; P317K and V340M; P317K and V340W; P317F and V340R; P317F and V340M; P317F and V340W; P317S and V340R; P317S and V340M; P317S and V340W; P317K and S338G; P317K and S338H; P317K and S338L; P317F and S338G; P317F and S338H; P317F and S338L; P317S and S338G; P317S and S338H; P317S and S338L; P318R and S338G; P318R and S338H; P318R and S338L; P318D and S338G; P318D and S338H; P318D and S338L; P318I and S338G; P318I and S338H; P318I and S338L; P319G and V340R; P319G and V340M; P319G and V340W; P319L and V340R; P319L and V340M; and P319L; V340W; P317R and P319D; P317R and R320D; P319D and R320D.

Exemplary mutations that may be used herein to reduce or abolish the binding affinity between HSV2 gE2 ectodomain and an antibody Fc domain also include deletion mutations at positions P319 and/or R320 of the sequence shown in SEQ ID NO: 7 alone or in combination with substitution mutations, in particular mutations selected from P319 deletion; R320 deletion; P319 deletion / R320 deletion; P319 deletion / R320 deletion / P317G / P318G; P319 deletion / R320 deletion / P318E; P319 deletion / R320 deletion /P318G; P319 deletion / R320 deletion / P318K; P319 deletion / R320 deletion / P317R / P318E; P319 deletion / R320 deletion / P317R / P318G; P319 deletion / R320 deletion / P317R / P318K; P319 deletion / R320 deletion / P317G / P318K.

Exemplary mutations that may be used herein to reduce or abolish the binding affinity between HSV2 gE2 ectodomain and an antibody Fc domain also include the insertion mutations selected from:

- insertion of peptide sequence LDIGE between amino acid residues Y275 and E276 of SEQ ID NO: 7 (275_insert_LDIGE),
- insertion of peptide sequence ADIGL between amino acid residues S289 and P290 of SEQ ID NO: 7 (289_insert_ADIGL),
- insertion of peptide sequence ARAA between amino acid residues A337 and S338 of SEQ ID NO: 7 (337_insert_ARAA),
- insertion peptide sequence ARAA between amino acid residues S338 and T339 of SEQ ID NO: 7 (338_insert_ARAA), and
- insertion peptide sequence ADIT between amino acid residues H346 and A347 of SEQ ID NO: 7 (346_insert_ADIT).

In a preferred embodiment, the HSV2 gE2 ectodomain comprises a mutation or a combination of mutations with respect to the sequence shown in SEQ ID NO: 7 selected from 289_insert ADIGL; 338insert ARAA; H245K; P317R;

P319R; P319G; P319K; H245A_P319R; H245A_P319G; H245A_P319K; H245A_P319T; P319D; S338D; R320D; N241A_R320D; A248K_V340M; P318Y; A248K_V340R; A248T_V340W; A248K_V340W; A246W_R320G; A246W_P317K; A246W_R320D; A246W_R320T; V340W; A248G_V340W; H245G_R320D; P318D; A246W_P317F; P319G_V340W; A248T_V340M; P317K_V340W; V340F; V340D; H245A_R320D; P317F_V340W; A246W_P317S; H245S_R320D; R314G_P318D; A248T; P318S; P317K; P317S_V340W; H245D; R314P_V340W; R314L_318D; P319L_V340W; P317F; P318D_S338G; R314G_V340W; P317K_S338H; R314L_V340W; P318R; P318Q; P317F_S338G; R314G_P318I; H245G_P319G; P317L; P318I; A248T_F322A; H245E; P318T; P318R_S338G; P318D_S338H; P317F_S338H; A248T_V340R; A248T_F322I; H245A_R320G; P318R_S338H; H245S_R320G; P317K_S338G; A248T F322P; V340R; R314L P318R; H245S_R320T; R314G_P318R; R320E; H245G_R320G; H245A R320T; A246W; P318I_S338G; P317K_V340M; P317I; R320H; R314P_P318I; P318I_S338H; P317F_V340M; H245A_P319G; H245A_P319L; R320P; H245G_R320T; R314L_V340R; P319G_V340R; R314G F322I; R314L_P318I; R320A; R314N; P317F_V340R; P318D_S338L; A248G_V340R; R314E; R314P_P318D; H245S_P319G; V340Q; A248K_F322I; R320G; H245S_P319L; R314F; P319L; P317K_S338L; P319L_V340M; P317G; R320S; R320Q; R314P V340R; V340A; H245G P319L; R320T; R314P_P318R; A248G_F322I; R320N; P317N; R314D; R314Y; R314P_F322I; P319G_V340M; P317S_V340R; R314V; P317R_P319D; P317R_R320D; P319D_R320D; A319_A320; P317G_P318G_Δ319_A320; P318E_A319_A320; P318G_Δ319_ Δ320; P318K_Δ319_Δ320; P317R_P318E_A319_320; P317R_P318G_L1319 Δ320 and P317G_P318K_Δ319_Δ320.

In a more preferred embodiment, the HSV2 gE2 ectodomain comprises a mutation or a combination of mutations with respect to the sequence shown in SEQ ID NO: 7 selected from 338_insert ARAA; P317R; P319D; R320D; A248T_V340W; V340W; A248T; P318I and A246W.

Corresponding mutations in other HSV2 gE2 ectodomain sequences, for example the sequences listed in Table 1 and shown on the alignment presented in Figure 77, to the exemplary substitution, deletion and insertion mutations listed above are also in the scope of the present invention.

All possible combinations of the exemplary single and double substitution mutations and insertion mutations listed above are also in the scope of the present invention.

Suitably, the HSV1 gE1 ectodomain comprises one or more mutations (insertions, substitutions or deletions) at positions selected from H247, P319 and P321 of the HSV1 gE1 ectodomain sequence shown in SEQ ID NO: 9.

Exemplary mutations that may be used herein to reduce or abolish the binding affinity between HSV 1 gE1 ectodomain and an antibody Fc domain include the single point substitution mutations of the sequence shown in SEQ ID NO: 9 selected from H247A, H247K, P319R, P321A, P321R, P321G, P321K, P321T, A339G, P321D, P321S, A340D, N243A and R322D, and the double point substitutions mutations of the sequence shown in SEQ ID NO: 9 selected from H247A/P321A, H247A/P321R H247A/P321G, H247A/P321K, H247A/P321T, N243A/R322D, N243A/P321D, H247G/P319G, P319G/P321G and A340G/S341G/V342G.

Exemplary mutations that may be used herein to reduce or abolish the binding affinity between HSV 1 gE1 ectodomain and an antibody Fc domain also include the insertion mutations selected from:

- insertion of peptide sequence LDIGE between amino acid residues Y277 and E278 of SEQ ID NO: 9 (277_insert_LDIGE);
- insertion of peptide sequence ADIGL between amino acid residues S291 and P292 of SEQ ID NO: 9 (291_inset_ADIGL);
- insertion of peptide sequence ARAA between amino acid residues A339 and A340 of SEQ ID NO: 9 (339_inset_ARAA);
- insertion of peptide sequence ARAA between amino acid residues A340 and S341 of SEQ ID NO: 9 (340_inset_ARAA); and
- insertion of peptide sequence ADIT between amino acid residues D348 and A349 of SEQ ID NO: 9 (348_insert_ADIT).

In a preferred embodiment, the HSV1 gE1 ectodomain comprises a mutation or a combination of mutations with respect to the sequence shown in SEQ ID NO: 9 selected from P321K; P321D; R322D; N243A_R322D; N243A_P321D; A340G_S341G_V342G; H247G_P319G; P321R; H247A_P321K; 291_insert ADIGL; 339_insert ARAA; P319R; P319G_P321G and H247A_P321R.

In a more preferred embodiment, the HSV1 gE ectodomain comprises a mutation or a combination of mutations with respect to the sequence shown in SEQ ID NO: 9 selected from P321D; R322D; A340G_S341G_V342G and P319R.

Corresponding mutations in other HSV1 gE1 ectodomain sequences to the exemplary single and double substitution mutations and insertion mutations listed above are also in the scope of the present invention.

All possible combinations of the exemplary single and double substitution mutations and insertion mutations listed above are also in the scope of the present invention.

In a preferred embodiment, the HSV Fc receptor or immunogenic fragment or variant thereof is part of a heterodimer with a binding partner from said virus or a fragment thereof.

As used herein, a **"binding partner"** is a viral protein (or glycoprotein) or fragment thereof which forms a noncovalent heterodimer complex with the Fc receptor or immunogenic fragment or variant thereof.

In a preferred embodiment, the HSV Fc receptor is HSV2 gE2 or an immunogenic fragment or variant thereof and the binding partner is HSV2 gI2 or a fragment thereof.

Herein, a **"HSV2 gI2"** (or **"HSV2 gI"**) is a HSV2 gI glycoprotein encoded by HSV2 gene US7 and displayed on the surface of infected cells where it associates with HSV2 gE2 to form a heterodimer. Suitably, the HSV2 gI2 is selected from the HSV2 gI glycoproteins shown in Table 2 or variants therefrom which are at least 60%, 65%, 70%, 75% 80%, 85%, 90%, 95%, 96%, 97%, 98%, 99% or 99,5% identical thereto.

**Table 2 - HSV2 gI glycoproteins and ectodomains**

| Genbank accession numbers | SEQ ID NO | Ectodomain |
|---|---|---|
| AHG54731.1 | 2 | 1-256 |
| AKC42829.1 | 62 | 1-256 |
| AKC59519.1 | 63 | 1-256 |
| AKC59590.1 | 64 | 1-256 |
| AKC59306.1 | 65 | 1-256 |
| AKC59377.1 | 66 | 1-256 |
| ABW83313.1 | 67 | 1-256 |
| ABW83397.1 | 68 | 1-256 |
| ABW83385.1 | 69 | 1-256 |
| ABW83327.1 | 70 | 1-256 |
| ABW83341.1 | 71 | 1-256 |
| ABW83339.1 | 72 | 1-256 |
| ABW83325.1 | 73 | 1-256 |
| ABW83351.1 | 74 | 1-256 |
| ABW83337.1 | 75 | 1-256 |
| ABW83355.1 | 76 | 1-256 |
| ABW83343.1 | 77 | 1-256 |
| ABW83329.1 | 78 | 1-256 |
| ABW83357.1 | 79 | 1-256 |
| ABW83365.1 | 80 | 1-256 |
| ABW83367.1 | 81 | 1-256 |
| ABW83371.1 | 82 | 1-256 |
| ABW83377.1 | 83 | 1-256 |
| AKC59448.1 | 84 | 1-256 |
| ABW83319.1 | 85 | 1-256 |
| ABW83379.1 | 86 | 1-256 |
| ABW83381.1 | 87 | 1-256 |
| ABW83383.1 | 88 | 1-256 |
| ABW83389.1 | 89 | 1-256 |
| ABW83347.1 | 90 | 1-256 |
| ABW83349.1 | 91 | 1-256 |
| ABW83335.1 | 92 | 1-256 |
| ABW83333.1 | 93 | 1-256 |

(continued)

| Genbank accession numbers | SEQ ID NO | Ectodomain |
|---|---|---|
| ABW83353.1 | 94 | 1-256 |
| ABW83359.1 | 95 | 1-256 |
| ABW83363.1 | 96 | 1-256 |
| ABW83331.1 | 97 | 1-256 |
| ABW83369.1 | 98 | 1-256 |
| ABW83375.1 | 99 | 1-256 |
| AMB66172.1 | 100 | 1-256 |
| YP_009137219.1 | 101 | 1-256 |
| CAB06714.1 | 102 | 1-256 |
| AEV91406.1 | 103 | 1-256 |
| ABW83305.1 | 104 | 1-256 |
| ABW83323.1 | 105 | 1-256 |
| ABW83307.1 | 106 | 1-256 |
| ABW83311.1 | 107 | 1-256 |
| ABW83315.1 | 108 | 1-256 |
| ABW83317.1 | 109 | 1-256 |
| ABW83321.1 | 110 | 1-256 |
| ABW83395.1 | 111 | 1-256 |
| ABW83393.1 | 112 | 1-256 |
| ABW83387.1 | 113 | 1-256 |
| ABW83391.1 | 114 | 1-256 |
| ABW83399.1 | 115 | 1-256 |
| ABW83345.1 | 116 | 1-256 |
| ABW83361.1 | 117 | 1-256 |
| ABW83309.1 | 118 | 1-256 |
| ABW83373.1 | 119 | 1-256 |
| AMB66029.1 | 120 | 1-256 |
| AMB66103.1 | 121 | 1-256 |
| AMB66322.1 | 122 | 1-256 |
| AMB66245.1 | 123 | 1-256 |

In a preferred embodiment, the HSV2 gI2 is the gI from HSV2 strain SD90e (Genbank accession numbers AHG54731.1, UniProtKB accession number: A8U5L5, SEQ ID NO: 2) which has the amino acid sequence shown in SEQ ID NO:2, or a variant therefrom which is at least 60%, 65%, 70%, 75% 80%, 85%, 90%, 95%, 96%, 97%, 98%, 99% or 99,5% identical thereto.

In another preferred embodiment, the HSV Fc receptor is HSV1 gE1 or an immunogenic fragment or variant thereof and the binding partner is HSV1 gI1 or a fragment thereof.

Herein, a **"HSV1 gI1"** (or "**HSV1 gI**") is a HSV1 gI glycoprotein encoded by HSV1 gene US7 and displayed on the surface of infected cells where it associates with HSV1 gE1 to form a heterodimer. Suitably, the HSV1 gI1 is the gI from HSV1 strain 17 (UniProtKB accession number: P06487, SEQ ID NO: 4) which has the amino acid sequence shown in SEQ ID NO: 4, or a variant therefrom which is at least 60%, 65%, 70%, 75% 80%, 85%, 90%, 95%, 96%, 97%, 98%, 99% or 99,5% identical thereto.

In one embodiment, a fragment of the HSV FcR binding partner is used.

As used herein, the term "**fragment**" as applied to a protein or peptide refers to a subsequence of a larger protein or peptide. A "fragment" of a protein or peptide is at least about 10 amino acids in length (amino acids naturally occurring as consecutive amino acids; e.g., as for a single linear epitope); for example at least about 15, 20, 30, 40, 50, 60, 100, 200, 300 or more amino acids in length (and any integer value in between).

In a preferred embodiment, the HSV FcR binding partner fragment is an immunogenic fragment.

As used herein, an **"fragment of a HSV FcR binding partner"** refers to a fragment of a naturally-occurring HSV FcR binding partner of at least 10, 15, 20, 30, 40, 50, 60, 100, 200, 300 or more amino acids, or a peptide having an amino acid sequence of at least 60%, 65%, 70%, 75%, 80%, 85%, 90%, 95%, 97%, 98%, 99%, or 99.5% sequence identity to a naturally-occurring HSV FcR binding partner (or to a fragment of a naturally-occurring HSV FcR binding partner of at least about 10, 15, 20, 30, 40, 50, 60 or more amino acids). Thus, a fragment of a HSV FcR binding partner may be a fragment of a naturally occurring HSV FcR binding partner, of at least 10 amino acids, and may comprise one or more amino acid substitutions, deletions or additions.

Any of the encoded HSV FcR binding partner fragments may additionally comprise an initial methionine residue where required.

A transmembrane protein is a type of integral membrane protein that has the ability to span across a cell membrane under normal culture conditions. Herein a transmembrane domain is the section of a transmembrane protein that finds itself within the cell membrane under normal culture conditions. Herein, a cytoplasmic domain is the section of a transmembrane protein that finds itself on the cytosolic side of the cell membrane under normal culture conditions. Herein, an ectodomain is the section of a transmembrane protein that finds itself on the external side of the cell membrane under normal culture conditions.

Suitably, the HSV FcR binding partner or fragment thereof does not comprise a transmembrane domain. Suitably, the HSV FcR binding partner or immunogenic fragment thereof does not comprise a cytoplasmic domain. Preferably, the HSV FcR binding partner or immunogenic fragment thereof neither comprises a transmembrane domain, nor a cytoplasmic domain. In other words, in a preferred embodiment, the HSV FcR binding partner or immunogenic fragment consists of a HSV FcR binding partner ectodomain (or extracellular domain). More preferably, the HSV FcR binding partner or immunogenic fragment is selected from a HSV2 gI2 ectodomain and a HSV1 gI1 ectodomain.

In a preferred embodiment, the HSV FcR binding partner ectodomain is a HSV2 gI2 ectodomain which comprises or consists of the amino acid sequence shown on SEQ ID NO: 8 (corresponding to amino acid residues 1-256 of SEQ ID NO: 2), or a sequence which is at least 60%, 65%, 70%, 75% 80%, 85%, 90%, 95%, 96%, 97%, 98%, 99% or 99,5% identical thereto. Suitably, the HSV FcR ectodomain has a sequence selected from the sequences shown in Table 2 or Figure 78. In a preferred embodiment, the HSV FcR ectodomain is a HSV2 gE2 ectodomain which is at least 90%, 95%, 96%, 97%, 98%, 99%, 99,5% or 100% identical to SEQ ID NO: 8.

Suitably, the HSV FcR binding partner HSV2 gI2 ectodomain may comprise one or more amino acid residue substitution, deletion, or insertion relative to the amino acid sequence shown at SEQ ID NO: 8, for example 1, 2, 3, 4, 5, 6, 7, 8, 9 or 10 amino acid residue substitutions, deletions, or insertions.

In another embodiment, the HSV FcR binding partner is a HSV2 gI2 ectodomain which comprises or consists of the amino acid sequence corresponding to amino acid residues 1-262 of SEQ ID NO: 2, or a sequence which is at least 60%, 65%, 70%, 75% 80%, 85%, 90%, 95%, 96%, 97%, 98%, 99% or 99,5% identical thereto. Suitably, the HSV FcR binding partner HSV2 gI2 ectodomain may comprise one or more amino acid residue substitutions, deletions, or insertions relative to the amino acid sequence corresponding to amino acid residues 1-262 of SEQ ID NO: 2, for example 1, 2, 3, 4, 5, 6, 7, 8, 9 or 10 amino acid residue substitution, deletion, or insertions.

In another embodiment, the HSV FcR binding partner ectodomain is a HSV1 gI1 ectodomain which comprises or consists of the amino acid sequence shown on SEQ ID NO: 10 (corresponding to amino acid residues 1-270 of SEQ ID NO: 4), or a sequence which is at least 60%, 65%, 70%, 75% 80%, 85%, 90%, 95%, 96%, 97%, 98%, 99% or 99,5% identical thereto. In a preferred embodiment, the HSV FcR ectodomain is a HSV1 gE1 ectodomain which is at least 90%, 95%, 96%, 97%, 98%, 99%, 99,5% or 100% identical to SEQ ID NO: 10.

Suitably, the HSV FcR binding partner HSV1 gI1 ectodomain may comprise one or more amino acid residue substitutions, deletions, or insertions relative to the amino acid sequence shown at SEQ ID NO: 10, for example 1, 2, 3, 4, 5, 6, 7, 8, 9 or 10 amino acid residue substitutions, deletions, or insertions.

In another embodiment, the HSV FcR binding partner is a HSV1 gI1 ectodomain which comprises or consists of the amino acid sequence corresponding to amino acid residues 1-276 of SEQ ID NO: 4, or a sequence which is at least 60%, 65%, 70%, 75% 80%, 85%, 90%, 95%, 96%, 97%, 98%, 99% or 99,5% identical thereto. Suitably, the HSV FcR binding partner HSV1 gI1 ectodomain may comprise one or more amino acid residue substitution, deletion, or insertion relative to the amino acid sequence corresponding to amino acid residues 1-276 of SEQ ID NO: 4, for example 1, 2, 3, 4, 5, 6, 7, 8, 9 or 10 amino acid residue substitution, deletion, or insertions.

Antibodies against gD, gB and gC are detected in subjects infected with HSV, and gH/gL to a lesser extent. The dominant neutralising response was to gD (Cairns, Tina M., et al. "Dissection of the antibody response against herpes simplex virus glycoproteins in naturally infected humans." Journal of virology 88.21 (2014): 12612-12622.).

In one embodiment, the HSV Fc receptor or immunogenic fragment or variant thereof is not administered to the subject in combination with an immunodominant HSV antigen (e.g. gD 1 or gD2).

Immunodominance is the immunological phenomenon in which immune responses are mounted against only a subset of the antigenic peptides produced by a pathogen. Immunodominance has been evidenced for antibody-mediated and cell-mediated immunity. As used herein, an **"immunodominant antigen"** is an antigen which comprises immunodominant epitopes. An immunodominant viral antigen is an immunodominant antigen of viral origin. In contrast, a **"subdominant antigen"** is an antigen which does not comprise immunodominant epitopes, or in other terms, only comprises subdominant epitopes. As used herein, an **"immunodominant epitope"** is an epitope that is dominantly targeted, or targeted to a higher degree, by neutralising antibodies during an immune response to a pathogen as compared to other epitopes from the same pathogen. As used herein, a **"subdominant epitope"** is an epitope that is not targeted, or targeted to a lower degree, by neutralising antibodies during an immune response to a pathogen as compared to other epitopes from the same pathogen. For example, gD2 is an immunodominant antigen for HSV2 and gD1 is an immunodominant antigen for HSV1. In contrast, gB2, gC2, gE2/gI2 and gH2/gL2 are subdominant antigens of HSV2 and gB1, gC1, gE1/gI1 and gH1/gL1 heterodimer are subdominant antigens of HSV1.

Suitably, where the HSV Fc receptor is HSV2 gE2 or HSV1 gE1, the Fc receptor or immunogenic fragment or variant thereof is not administered to the subject together with HSV2 gD2 or HSV1 gD1, or a fragment thereof comprising immunodominant epitopes. In a particular embodiment where the HSV Fc receptor is HSV2 gE2, the HSV Fc receptor or immunogenic fragment or variant thereof is not administered to the subject together with HSV2 gD2 or a fragment thereof comprising immunodominant epitopes. In another particular embodiment where the HSV Fc receptor is HSV1 gE1, the HSV Fc receptor or immunogenic fragment or variant thereof is not administered to the subject together with HSV1 gD1 or a fragment thereof comprising immunodominant epitopes.

Glycoprotein gC from HSV1 and HSV2 is also involved in an immune escape mechanism by inhibiting complement (Awasthi, Sita, et al. "Blocking herpes simplex virus 2 glycoprotein E immune evasion as an approach to enhance efficacy of a trivalent subunit antigen vaccine for genital herpes." Journal of virology 88.15 (2014): 8421-8432.).

In one embodiment, the HSV Fc receptor is HSV2 gE2 and is administered to the subject together with HSV2 gC2, or an immunogenic fragment or variant thereof.

In one embodiment, the HSV Fc receptor is HSV1 gE1 and is administered to the subject together with HSV1 gC1, or an immunogenic fragment or variant thereof.

In one aspect, the invention provides a recombinant HSV FcR or immunogenic fragment or variant thereof, wherein the ability of the HSV FcR or immunogenic fragment or variant thereof to bind to a human antibody Fc domain is reduced or abolished compared to the corresponding native HSV Fc receptor.

Suitably, the recombinant HSV Fc receptor or immunogenic fragment or variant thereof comprises one or more amino acid substitutions, deletions or insertions compared to the native sequence of the HSV Fc receptor or immunogenic fragment thereof, that reduce or abolish the binding affinity between the HSV FcR or immunogenic fragment or variant thereof and the antibody Fc domain compared to the native HSV Fc receptor.

In a preferred embodiment, the $k_{on}$ between the recombinant HSV FcR or immunogenic fragment or variant thereof and human IgGs is lower than the $k_{on}$ between the corresponding native HSV FcR and human IgGs (slow binder). In a preferred embodiment, the $k_{off}$ between the recombinant HSV FcR or immunogenic fragment or variant thereof and human IgGs is higher than the $k_{off}$ between the corresponding native HSV FcR and human IgGs (fast releaser). In a more preferred embodiment, the $k_{on}$ between the recombinant HSV FcR or immunogenic fragment or variant thereof and human IgGs is lower than the $k_{on}$ between the corresponding native HSV FcR and human IgGs, and the $k_{off}$ between the recombinant HSV FcR or immunogenic fragment or variant thereof and human IgGs is higher than the $k_{off}$ between the corresponding native HSV FcR and human IgGs (slow binder / fast releaser).

In a preferred embodiment, the equilibrium dissociation constant ($K_D$) between the recombinant HSV FcR or immunogenic fragment or variant thereof and human IgGs is higher than the $K_D$ between the corresponding native HSV FcR and human IgGs.

In a preferred embodiment, the relative affinity between the recombinant HSV FcR or immunogenic fragment or variant thereof and human IgGs is less than 100%, for example less than 90%, 80%, 70%, 60%, 50%, 40%, 30%, 20%, 15% or 10% of the affinity between the corresponding native viral FcR and human IgGs. In a more preferred embodiment, the relative affinity between the recombinant HSV FcR or immunogenic fragment or variant thereof and human IgGs is less than 15%, more preferably still less than 10% of the affinity between the corresponding native HSV FcR and human IgGs.

In a preferred embodiment, the equilibrium dissociation constant ($K_D$) between the recombinant HSV FcR or immunogenic fragment or variant thereof and human IgGs is higher than 2 x $10^{-7}$ M, preferably higher than 5 x $10^{-7}$ M, more preferably higher than 1 x $10^{-6}$ M.

Alternatively, the ability of the HSV FcR or immunogenic fragment or variant thereof to bind to a human antibody Fc domain can be assessed by measuring the response (expressed in nm) in a BiLayer Interferometry assay.

In a preferred embodiment, the response in a BiLayer Interferometry assay corresponding to the binding between the HSV FcR or immunogenic fragment or variant thereof and human IgGs is less than 80%, suitably less than 70%, 60%, 50%, 40% of the response obtained with the corresponding native HSV FcR. In a preferred embodiment, the response in a BiLayer Interferometry assay corresponding to the binding between the HSV FcR or immunogenic fragment or variant thereof and human IgGs is lower than 0.4 nm, suitably lower than 0.3 nm, 0.2 nm or 0.1 nm.

In a preferred embodiment, the recombinant HSV FcR or immunogenic fragment or variant thereof is HSV2 gE2 or an immunogenic fragment or variant thereof. Suitably, the recombinant HSV2 gE2 or immunogenic fragment or variant thereof comprises one or more mutations (insertions, substitutions or deletions) at positions selected from N241, H245, A246, A248, R314, P317, P318, P319, F322, R320, A337, S338 or V340 of the HSV2 gE2 sequence shown in SEQ ID NO: 1.

Exemplary mutations that may be used herein to reduce or abolish the binding affinity between the recombinant HSV2 gE2 or immunogenic fragment or variant thereof and an antibody Fc domain include the single point substitution mutations of the sequence shown in SEQ ID NO: 1 selected from H245A, H245K, P317R, P319A, P319R, P319G, P319K, P319T, A337G, P319D, P319S, S338D, N241A, R320D, H245E, H245V, H245R, H245D, H245Q, H245G, H245I, H245K, H245S, H245T, A246W, A248K, A248T, A248G, R314A, R314N, R314D, R314Q, R314E, R314G, R3141, R314L, R314K, R314M, R314F, R314P, R314S, R314T, R314Y, R314V, P317N, P317G, P317I, P317L, P317K, P317F, P317S, P318R, P318D, P318Q, P318I, P318S, P318T, P318Y, P319L, R320A, R320S, R320N, R320Q, R320E, R320G, R320H, R320I, R320L, R320M, R320P, R320T, R320V, F322A, F322N, F322I, F322K, F322P, F322T, S338G, S338E, S338L, S338T, V340A, V340R, V340D, V340Q, V340M, V340F, V340P and V340W.

Exemplary mutations that may be used herein to reduce or abolish the binding affinity between the recombinant HSV2 gE2 or immunogenic fragment or variant thereof and an antibody Fc domain also include the double point substitution mutations of the sequence shown in SEQ ID NO: 1 selected from H245A and P319A; H245A and P319R; H245A and P319G; H245A and P319K; H245A and P319T; N241A and R320D; N241A and P319D; A246W and P317K; A246W and P317F; A246W and P317S; A246W and R320D; A246W and R320G; A246W and R320T; A248K and V340R; A248K and V340M; A248K and V340W; A248T and V340R; A248T and V340M; A248T and V340W; A248G and V340R; A248G and V340M; A248G and V340W; A248K and F322A; A248K and F322I; A248K and F322P; A248T and F322A; A248T and F322I; A248T and F322P; A248G and F322A; A248G and F322I; A248G and F322P; H245A and R320D; H245A and R320G; H245A and R320T; H245G and R320D; H245G and R320G; H245G and R320T; H245S and R320D; H245S and R320G; H245S and R320T; H245A and P319G; H245A and P319L; H245G and P319G; H245G and P319L; H245S and P319G; H245S and P319L; R314G and P318R; R314G and P318D; R314G and P318I; R314L and P318R; R314L and P318D; R314L and P318I; R314P and P318R; R314P and P318D; R314P and P318I; R314G and F322A; R314G and F322I; R314G and F322P; R314L and F322A; R314L and F322I; R314L and F322P; R314P and F322A; R314P and F322I; R314P and F322P; R314G and V340R; R314G and V340M; R314G and V340W; R314L and V340R; R314L and V340M; R314L and V340W; R314P and V340R; R314P and V340M; R314P and V340W; P317K and V340R; P317K and V340M; P317K and V340W; P317F and V340R; P317F and V340M; P317F and V340W; P317S and V340R; P317S and V340M; P317S and V340W; P317K and S338G; P317K and S338H; P317K and S338L; P317F and S338G; P317F and S338H; P317F and S338L; P317S and S338G; P317S and S338H; P317S and S338L; P318R and S338G; P318R and S338H; P318R and S338L; P318D and S338G; P318D and S338H; P318D and S338L; P318I and S338G; P318I and S338H; P318I and S338L; P319G and V340R; P319G and V340M; P319G and V340W; P319L and V340R; P319L and V340M; P319L and V340W; P317R and P319D; P317R and R320D; P319D and R320D.

Exemplary mutations that may be used herein to reduce or abolish the binding affinity between the recombinant HSV2 gE2 or immunogenic fragment or variant thereof and an antibody Fc domain also include deletion mutations at positions P319 and/or R320 of the sequence shown in SEQ ID NO: 1, alone or in combination with substitution mutations, in particular mutations selected from P319 deletion; R320 deletion; P319 deletion / R320 deletion; P319 deletion / R320 deletion / P317G / P318G; P319 deletion / R320 deletion / P318E; P319 deletion / R320 deletion /P318G; P319 deletion / R320 deletion / P318K; P319 deletion / R320 deletion / P317R / P318E; P319 deletion / R320 deletion / P317R / P318G; P319 deletion / R320 deletion / P317R / P318K; P319 deletion / R320 deletion / P317G / P318K.

Exemplary mutations that may be used herein to reduce or abolish the binding affinity between the recombinant HSV2 gE2 or immunogenic fragment or variant thereof and an antibody Fc domain also include the insertion mutations selected from:

- insertion of peptide sequence LDIGE between amino acid residues Y275 and E276 of SEQ ID NO: 1 (275_insert_LDIGE),
- insertion of peptide sequence ADIGL between amino acid residues S289 and P290 of SEQ ID NO: 1 (289_insert_ADIGL),
- insertion of peptide sequence ARAA between amino acid residues A337 and S338 of SEQ ID NO: 1 (337_insert_ARAA),
- insertion of peptide sequence ARAA between amino acid residues S338 and T339 of SEQ ID NO: 1 (338_insert_ARAA), and
- insertion of peptide sequence ADIT between amino acid residues H346 and A347 of SEQ ID NO: 1 (346_insert_ADIT).

In a preferred embodiment, the recombinant HSV2 gE2 or immunogenic fragment or variant thereof comprises a mutation or a combination of mutations with respect to the sequence shown in SEQ ID NO: 1 selected from 289_insert ADIGL; 338_insert ARAA; H245K; P317R; P319R; P319G; P319K; H245A_P319R; H245A_P319G; H245A_P319K; H245A_P319T; P319D; S338D; R320D; N241A_R320D; A248K_V340M; P318Y; A248K_V340R; A248T_V340W; A248K_V340W; A246W_R320G; A246W_P317K; A246W_R320D; A246W_R320T; V340W; A248G_V340W; H245G_R320D; P318D; A246W_P317F; P319G_V340W; A248T_V340M; P317K_V340W; V340F; V340D; H245A_R320D; P317F_V340W; A246W_P317S; H245S_R320D; R314G_P318D; A248T; P318S; P317K; P317S_V340W; H245D; R314P_V340W; R314L_318D; P319L_V340W; P317F; P318D_S338G; R314G_V340W; P317K_S338H; R314L_V340W; P318R; P318Q; P317F_S338G; R314G_P318I; H245G_P319G; P317L; P318I; A248T_F322A; H245E; P318T; P318R_S338G; P318D_S338H; P317F_S338H; A248T_V340R; A248T_F322I; H245A_R320G; P318R_S338H; H245S_R320G; P317K_S338G; A248T_F322P; V340R; R314L_P318R; H245S_R320T; R314G_P318R; R320E; H245G_R320G; H245A_R320T; A246W; P318I_S338G; P317K_V340M; P317I; R320H; R314P_P318I; P318I_S338H; P317F_V340M; H245A_P319G; H245A_P319L; R320P; H245G_R320T; R314L_V340R; P319G_V340R; R314G_F322I; R314L_P318I; R320A; R314N; P317F_V340R; P318D_S338L; A248G_V340R; R314E; R314P_P318D; H245S_P319G; V340Q; A248K_F322I; R320G; H245S_P319L; R314F; P319L; P317K_S338L; P319L_V340M; P317G; R320S; R320Q; R314P_V340R; V340A; H245G_P319L; R320T; R314P_P318R; A248G_F322I; R320N; P317N; R314D; R314Y; R314P_F322I; P319G_V340M; P317S_V340R; R314V; P317R_P319D; P317R_R320D; P319D_R320D; A319_Δ320; P317G_P318GΔ319_ Δ320; P318E_Δ319_Δ320; P318G_Δ319_Δ320; P318K_A319_Δ320; P317R_P318E_A319_320; P317R_P318G_Δ319_Δ320 and P317G_P318K_Δ319_Δ320.

In a more preferred embodiment, the HSV2 gE2 or immunogenic fragment or variant thereof comprises a mutation or a combination of mutations with respect to the sequence shown in SEQ ID NO: 1 selected from 338_insert ARAA; P317R; P319D; R320D; A248T_V340W; V340W; A248T; P318I and A246W.

Corresponding mutations in other HSV2 gE2 sequences, for example the sequences listed in Table 1 and shown on the alignment presented in Figure 77, to the exemplary single and double substitution, selection and mutations and insertion mutations listed above are also in the scope of the present invention.

All possible combinations of the exemplary single and double substitution mutations and insertion mutations listed above are also in the scope of the present invention.

In a preferred embodiment, the recombinant HSV2 gE2 or immunogenic fragment or variant thereof is a recombinant HSV2 gE2 ectodomain as described herein.

In another embodiment, the recombinant HSV FcR or immunogenic fragment or variant thereof is a recombinant HSV1 gE1 or an immunogenic fragment or variant thereof. Suitably, the recombinant HSV1 gE1 or immunogenic fragment or variant thereof comprises one or more mutations (insertions, substitutions or deletions) at positions selected from H247, P319 and P321 of the HSV1 gE1 sequence shown in SEQ ID NO: 3.

Exemplary mutations that may be used herein to reduce or abolish the binding affinity between the recombinant HSV1 gE1 or immunogenic fragment or variant thereof and an antibody Fc domain include the single point substitution mutations of the sequence shown in SEQ ID NO: 3 selected from H247A, H247K, P319R, P321A, P321R, P321G, P321K, P321T, A339G, P321D, P321S, A340D, N243A and R322D, and the double point substitutions mutations of the sequence shown in SEQ ID NO: 3 selected from H247A/P321A, H247A/P321R, H247A/P321G, H247A/P321K, H247A/P321T, N243A/R322D, N243A/P321D, H247G/P319G, P319G/P321G and A340G/S341GN342G.

Exemplary mutations that may be used herein to reduce or abolish the binding affinity between the recombinant HSV1 gE1 or immunogenic fragment or variant thereof and an antibody Fc domain also include the insertion mutations selected from:

- insertion of peptide sequence LDIGE between amino acid residues Y277 and E278 of SEQ ID NO: 3 (277_insert_LDIGE);
- insertion of peptide sequence ADIGL between amino acid residues S291 and P292 of SEQ ID NO: 3 (291_insert_ADIGL);
- insertion of peptide sequence ARAA between amino acid residues A339 and A340 of SEQ ID NO: 3 (339_inset_ARAA);
- insertion of peptide sequence ARAA between amino acid residues A340 and S341 of SEQ ID NO: 3 (340_inset_ARAA); and
- insertion of peptide sequence ADIT between amino acid residues D348 and A349 of SEQ ID NO: 3 (348_insert_ADIT).

In a preferred embodiment, the HSV1 gE1 or immunogenic fragment or variant thereof comprises a mutation or a combination of mutations with respect to the sequence shown in SEQ ID NO: 3 selected from P321K; P321D; R322D; N243A_R322D; N243A_P321D; A340G_S341G_V342G; H247G P319G; P321R; H247A_P321K; 291_insert ADIGL; 339_insert ARAA; P319R; P319G_P321G and H247A_P321R.

In a more preferred embodiment, the HSV1 gE1 or immunogenic fragment or variant thereof comprises a mutation or a combination of mutations with respect to the sequence shown in SEQ ID NO: 3 selected from P321D; R322D; A340G_S341G_V342G and P319R.

In one embodiment is provided a HSV2 Fc receptor or an immunogenic fragment or variant thereof for the use described herein wherein:

- the ability of said variant thereof to bind to a human antibody Fc domain is reduced or abolished compared to the corresponding native HSV Fc receptor

- said use does not comprise administration to the subject together with HSV2 gD2 or a fragment thereof comprising immunodominant epitopes.

Preferably, the encoded Fc receptor is gE2 (optionally in combination with its binding partner gI2) and the variant thereof comprises a mutation or a combination of mutations with respect to the sequence shown in SEQ ID NO: 1 selected from 338_insert ARAA; P317R; P319D; R320D; A248T_V340W; V340W; A248T; P318I and A246W.

In a further embodiment is provided a nucleic acid encoding a HSV1 Fc receptor or an immunogenic fragment or variant thereof for the use described herein wherein:

- the ability of said variant thereof to bind to a human antibody Fc domain is reduced or abolished compared to the corresponding native HSV Fc receptor and
- said use does not comprise administration to the subject together with HSV1 gD1 or a fragment thereof comprising immunodominant epitopes

Preferably, the encoded Fc receptor is gE1 (optionally in combination with its binding partner gI1) and the variant thereof comprises a mutation or a combination of mutations with respect to the sequence shown in SEQ ID NO: 3 selected from H247A, H247K, P319R, P321A, P321R, P321G, P321K, P321T, A339G, P321D, P321S, A340D, N243A and R322D, H247A/P321A, H247A/P321R H247A/P321G, H247A/P321K, H247A/P321T, N243A/R322D, N243A/P321D, H247G/P319G, P319G/P321G and A340G/S341G/V342G.

Corresponding mutations in other HSV1 gE1 sequences to the exemplary single and double substitution mutations and insertion mutations listed above are also in the scope of the present invention.

All possible combinations of the exemplary single and double substitution mutations and insertion mutations listed above are also in the scope of the present invention.

In a preferred embodiment, the recombinant HSV1 gE1 or immunogenic fragment or variant thereof is a recombinant HSV1 gE1 ectodomain as described herein.

In a preferred embodiment, the recombinant HSV FcR or immunogenic fragment or variant thereof is part of a heterodimer with a binding partner from said virus or a fragment thereof.

In a preferred embodiment, the recombinant HSV Fc receptor is recombinant HSV2 gE2 or an immunogenic fragment or variant thereof and the binding partner is HSV2 gI2 or a fragment thereof as described herein.

In another preferred embodiment, the recombinant HSV Fc receptor is recombinant HSV1 gE1 or an immunogenic fragment or variant thereof and the binding partner is HSV1 gI1 or a fragment thereof or a fragment thereof as described herein.

In another embodiment, the HSV FcR or immunogenic fragment or variant thereof is part of a heterodimer with a binding partner from said HSV virus or a fragment thereof. In a further embodiment, the HSV Fc receptor is HSV2 gE2 and the binding partner is HSV2 gI2. In another embodiment, the HSV Fc receptor is HSV1 gE1 and the binding partner is HSV1 gI1.

*Therapeutic uses and compositions*

In another aspect, the invention provides a pharmaceutical composition comprising a HSV2 Fc receptor or an immunogenic fragment or variant thereof and a pharmaceutically acceptable carrier for use in inducing a cross reactive immune response against HSV1 when administered to a subject. In one embodiment of the pharmaceutical composition, the HSV2 Fc receptor is HSV2 gE2 and the binding partner is HSV2 gI2. Preferably, the pharmaceutical composition is for use as a pan-HSV vaccine.

In a further aspect of the invention, is provided a pharmaceutical composition comprising a HSV1 Fc receptor or an immunogenic fragment or variant thereof and a pharmaceutically acceptable carrier for use in inducing a cross reactive immune response against HSV2 when administered to a subject. In one embodiment of the pharmaceutical composition, the HSV1 Fc receptor is HSV1 gE1 and the binding partner is HSV1 gI1. Preferably, the pharmaceutical composition is for use as a pan-HSV vaccine.

In another aspect, the invention provides an immunogenic composition comprising a HSV2 Fc receptor or an immunogenic fragment or variant thereof and a pharmaceutically acceptable carrier for use in inducing a cross reactive immune response against HSV1 when administered to a subject. In a further aspect, is provided an immunogenic composition comprising a HSV1 Fc receptor or an immunogenic fragment or variant thereof and a pharmaceutically acceptable carrier for use in inducing a cross reactive immune response against HSV2 when administered to a subject. Suitably, the immunogenic composition may be prepared for administration by being suspended or dissolved in a pharmaceutically or physiologically acceptable carrier. Preferably, the immunogenic compositions of the invention are suitable for use as therapeutic vaccines. Most preferably, the immunogenic compositions are for use as pan-HSV vaccines.

A **"pharmaceutically acceptable carrier"** includes any carrier that does not itself induce the production of antibodies harmful to the individual receiving the composition. Suitable carriers are typically large, slowly metabolized macromolecules such as proteins, polysaccharides, polylactic acids, polyglycolic acids, polymeric amino acids, amino acid copolymers, sucrose, trehalose, lactose, and lipid aggregates (such as oil droplets or liposomes). Such carriers are well known to those of ordinary skill in the art. The compositions may also contain a pharmaceutically acceptable diluent, such as water, saline, glycerol, etc. Additionally, auxiliary substances, such as wetting or emulsifying agents, pH buffering substances, and the like, may be present. Sterile pyrogen-free, phosphate-buffered physiologic saline is a typical carrier. The appropriate carrier may depend in large part upon the route of administration.

Suitably, the HSV Fc receptor or immunogenic fragment or variant thereof is to be administered to a subject by any route as is known in the art, including intramuscular, intravaginal, intravenous, intraperitoneal, subcutaneous, epicutaneous, intradermal, nasal, intratumoral or oral administration.

In one embodiment, the subject is a vertebrate, such as a mammal, e.g. a human, a non-human primate, or a veterinary mammal (livestock or companion animals). In a preferred embodiment, the subject is a human.

In a preferred embodiment, the subject has been infected by the virus (i.e. is seropositive), for example HSV2 and/or HSV1 prior to being treated with the HSV (HSV1 or HSV2) FcR or immunogenic fragment or variant thereof. The subject which has been infected with the virus prior to being treated with the HSV FcR or immunogenic fragment or variant thereof may have shown clinical signs of the infection (symptomatic subject) or may not have shown clinical sings of the viral infection (asymptomatic subject). In one embodiment, the symptomatic subject has shown several episodes with clinical symptoms of infections over time (recurrences) separated by periods without clinical symptoms.

In one aspect, the invention provides a HSV2 Fc receptor or immunogenic fragment or variant thereof, or a nucleic acid encoding said HSV2 FcR or immunogenic fragment or variant thereof, for use in i) inducing a cross reactive immune response against HSV-1 when administered to a subject and ii) in the treatment of recurrent herpes infection, or in a method for prevention or reduction of the frequency of recurrent herpes virus infection in a subject, preferably a human subject. In one embodiment, the HSV2 Fc receptor is HSV2 gE2 or an immunogenic fragment or variant thereof. In another embodiment, the HSV Fc receptor is a HSV2 gE2 / gI2 heterodimer or immunogenic fragment or variant thereof. In a further embodiment, the HSV2 Fc receptor or immunogenic fragment or variant thereof for the use described herein is for use as a pan-HSV vaccine.

In a further aspect, the invention provides a HSV1 Fc receptor or immunogenic fragment or variant thereof, or a nucleic acid encoding said HSV1 FcR or immunogenic fragment or variant thereof, for use in i) inducing a cross reactive immune response against HSV-2 when administered to a subject and ii) in the treatment of recurrent herpes infection, or in a method for prevention or reduction of the frequency of recurrent herpes virus infection in a subject, preferably a human subject. In one embodiment, the HSV1 Fc receptor is HSV1 gE1 or an immunogenic fragment or variant thereof. In another embodiment, the HSV Fc receptor is HSV1 gE1/gI1 heterodimer or immunogenic fragment or variant thereof. In a further embodiment, the HSV1 Fc receptor or immunogenic fragment or variant thereof for the use described herein is for use as a pan-HSV vaccine.

In one aspect, the invention provides a HSV2 Fc receptor or immunogenic fragment or variant thereof, or a nucleic acid encoding said HSV2 FcR or immunogenic fragment or variant thereof, as described herein for use in the manufacture of an immunogenic composition which when administered to a subject induces a cross reactive immune response against HSV1.

In a further aspect, the invention provides a HSV1 Fc receptor or immunogenic fragment or variant thereof, or a nucleic acid encoding said HSV1 FcR or immunogenic fragment or variant thereof, as described herein for use in the manufacture of an immunogenic composition which when administered to a subject induces a cross reactive immune response against HSV2.

In one aspect, the invention provides the use of a HSV2 Fc receptor or immunogenic fragment or variant thereof, or a nucleic acid encoding said HSV2 FcR or immunogenic fragment or variant thereof, as described herein in the manufacture of a medicament for the prevention or treatment of herpes infection or herpes-related disease wherein the HSV2 Fc receptor or immunogenic fragment or variant thereof can induce a cross reactive immune response against HSV1 when administered to a subject.

In a further aspect, the invention provides the use of a HSV1 Fc receptor or immunogenic fragment or variant thereof, or a nucleic acid encoding said HSV1 FcR or immunogenic fragment or variant thereof, as described herein in the manufacture of a medicament for the prevention or treatment of herpes infection or herpes-related disease wherein the HSV1 Fc receptor or immunogenic fragment or variant thereof can induce a cross reactive immune response against HSV2 when administered to a subject.

In one aspect, the invention provides a HSV2 gE2 or gE2 / gI2 heterodimer, an immunogenic fragment or variant thereof, or a nucleic acid encoding said HSV2 gE2 or immunogenic fragment or variant thereof, as described herein for use in the manufacture of an immunogenic composition wherein the immunogenic composition can induce a cross reactive immune response against HSV 1 when administered to a subject.

In a further aspect, the invention provides a HSV1 gE1 or gE1 / gI1 heterodimer, an immunogenic fragment or variant thereof, or a nucleic acid encoding said HSV1 gE1 or immunogenic fragment or variant thereof, as described herein for use in the manufacture of an immunogenic composition wherein the immunogenic composition can induce a cross reactive immune response against HSV2 when administered to a subject.

In one aspect, the invention provides the use of a HSV2 gE2 or gE2 / gI2 heterodimer, an immunogenic fragment or variant thereof, or a nucleic acid encoding said HSV2 gE2 or gE2 / gI2 heterodimer or immunogenic fragment or variant thereof, as described herein in the manufacture of a medicament for the prevention or treatment of i) HSV2 infection or HSV2-related disease and ii) HSV1 infection or HSV 1-related disease, wherein the HSV2 gE2 or gE2/gI2 heterodimer or an immunogenic fragment or variant thereof can induce a cross reactive immune response against HSV 1 when administered to a subject.

In a further aspect, the invention provides the use of a HSV1 gE1 or gE1 / gI1 heterodimer, an immunogenic fragment or variant thereof, or a nucleic acid encoding said HSV1 gE1 or gE1 / gI1 heterodimer or immunogenic fragment or variant thereof, as described herein in the manufacture of a medicament for the prevention or treatment of i) HSV 1 infection or HSV1-related disease and ii) HSV2 infection or HSV2-related disease, wherein the HSV1 gE1 or gE1/gI1 heterodimer or an immunogenic fragment or variant thereof can induce a cross reactive immune response against HSV2 when administered to a subject.

In one aspect, the invention provides a method of preventing or treating a herpes virus infection or herpes virus related disease in a subject in need thereof comprising administering an immunologically effective amount of a HSV2 Fc receptor or immunogenic fragment or variant thereof, or a nucleic acid encoding said HSV2 FcR or immunogenic fragment or variant thereof, to the subject wherein the HSV2 Fc receptor or immunogenic fragment or variant thereof can induce a cross reactive immune response against HSV1 when administered to a subject. In one embodiment the herpes virus infection is a HSV1 infection and the herpes virus related disease is a HSV1 related disease. In a further embodiment, the HSV2 Fc receptor is HSV2 gE2 or gE2/gI2 heterodimer, or an immunogenic fragment or variant thereof.

In a further aspect, the invention provides a method of preventing or treating a herpes virus infection or herpes virus related disease in a subject in need thereof comprising administering an immunologically effective amount of a HSV1 Fc receptor or immunogenic fragment or variant thereof, or a nucleic acid encoding said HSV1 FcR or immunogenic fragment or variant thereof, to the subject wherein the HSV1 Fc receptor or immunogenic fragment or variant thereof can induce a cross reactive immune response against HSV2 when administered to a subject. In one embodiment the herpes virus infection is a HSV2 infection and the herpes virus related disease is a HSV2 related disease. In a further embodiment, the HSV1 Fc receptor is HSV1 gE1 or gE1/gI1 heterodimer, or an immunogenic fragment or variant thereof.

A further aspect of the invention provides a method of treating a subject infected with HSV1 or preventing HSV1 infection in a subject comprising administering an immunologically effective amount of a HSV2 Fc receptor or an immunogenic fragment or variant thereof to the subject and inducing a cross reactive immune response against HSV1.

A yet further aspect of the invention provides a method of treating a subject infected with HSV2 or preventing HSV2 infection in a subject comprising administering an immunologically effective amount of a HSV1 Fc receptor or an immunogenic fragment or variant thereof to the subject and inducing a cross reactive immune response against HSV2.

Also provided is a method comprising administering to the subject a Fc receptor that is HSV2 gE2 or HSV1 gE1, and not administering together with an immunodominant viral antigen (e.g., HSV2 gD2 or HVS1gD1).

Also provided is a method comprising administering, in an effective immunological amount to a subject, a HSV Fc receptor or an immunogenic fragment or variant thereof together with a HSV2 gC2 or an immunogenic fragment thereof or a HSV1 gC1 or an immunogenic fragment thereof.

Also provided is a method comprising administering, in an effective immunological amount to a subject, a pharmaceutical composition comprising a HSV2 Fc receptor or an immunogenic fragment or variant thereof and a pharmaceutically acceptable carrier, and inducing a cross reactive immune response against HSV1 when administered to the subject.

Also provided is a method comprising administering, in an effective immunological amount to a subject, a pharmaceutical composition comprising a HSV1 Fc receptor or an immunogenic fragment or variant thereof and a pharmaceutically acceptable carrier, and inducing a cross reactive immune response against HSV2 when administered to the subject.

As used herein, the terms **"treat"** and **"treatment"** as well as words stemming therefrom, are not meant to imply a "cure" of the condition being treated in all individuals, or 100% effective treatment in any given population. Rather, there are varying degrees of treatment which one of ordinary skill in the art recognizes as having beneficial therapeutic effect(s). In this respect, the inventive methods and uses can provide any level of treatment of herpes virus infection and in particular HSV2 and/or HSV1 related disease in a subject in need of such treatment, and may comprise reduction in the severity, duration, or number of recurrences over time, of one or more conditions or symptoms of herpes virus infection, and in particular HSV2 and/or HSV1 related disease.

As used herein, **"therapeutic immunization"** or **"therapeutic vaccination"** refers to administration of the immunogenic compositions of the invention to a subject, preferably a human subject, who is known to be infected with a virus such as a herpes virus and in particular HSV2 and/or HSV1 at the time of administration, to treat the viral infection or virus-related disease. As used herein, **"prophylactic immunization"** or **"prophylactic vaccination"** refers to administration of the immunogenic compositions of the invention to a subject, preferably a human subject, who has not been infected with a virus such as a herpes virus and in particular HSV2 and/or HSV1 at the time of administration, to prevent the viral infection or virus-related disease.

For the purpose of the present invention, treatment of HSV infection aims at preventing reactivation events from the latent HSV infection state or at controlling at early stage viral replication to reduce viral shedding and clinical manifestations that occur subsequent to primary HSV infection, i.e. recurrent HSV infection. Treatment thus prevents either or both of HSV symptomatic and asymptomatic reactivation (also referred to as recurrent HSV infection), including asymptomatic viral shedding. Treatment may thus reduce the severity, duration, and/or number of episodes of recurrent HSV infections following reactivation in symptomatic individuals. Preventing asymptomatic reactivation and shedding from mucosal sites may also reduce or prevent transmission of the infection to those individuals naive to the HSV virus (i.e. HSV2, HSV1, or both). This includes prevention of transmission of HSV through sexual intercourse, in particular transmission of HSV2 but also potential transmission of HSV1 through sexual intercourse. Thus the immunogenic construct of the present invention may achieve any of the following useful goals: preventing or reducing asymptomatic viral shedding, reducing or preventing symptomatic disease recurrences, reducing duration or severity of symptomatic disease, reducing frequency of recurrences, prolonging the time to recurrences, increasing the proportion of subjects that are recurrence-free at a given point in time, reducing the use of antivirals, and preventing transmission between sexual partners.

In particular, the HSV2 Fc receptor or an immunogenic fragment or variant thereof and immunogenic compositions described herein for use in inducing a cross reactive immune response against HSV-1 when administered to a subject, are useful as therapeutic vaccines, to treat or prevent recurrent viral infections in a subject in need of such treatment. Preferably, the therapeutic vaccine is a pan-HSV therapeutic vaccine. Preferably, the subject is a human.

Also, the HSV1 Fc receptor or an immunogenic fragment or variant thereof and immunogenic compositions described herein for use in inducing a cross reactive immune response against HSV2 when administered to a subject, are useful as therapeutic vaccines, to treat or prevent recurrent viral infections in a subject in need of such treatment. Preferably, the therapeutic vaccine is a pan-HSV therapeutic vaccine. Preferably, the subject is a human.

A pan-HSV vaccine shows a humoral and/or cellular immune response against both HSV 1 and HSV2 i.e. both serotypes of HSV. Hence, a HSV1 or HSV2 Fc receptor or immunogenic fragment or variant thereof as described herein can be used as a pan-HSV vaccine for use against both types of HSV, HSV1 and HSV2. In this way, the manufacture of an antigen prepared from just one type of HSV is sufficient to make a vaccine against all serotypes of HSV, without the additional manufacture requirements and complexity of making a vaccine with antigens from both serotypes of HSV.

Suitably, the HSV1 or HSV2 Fc receptor or an immunogenic fragment or variant thereof and immunogenic compositions for the uses described herein are not part of a prophylactic vaccine.

Methods of use as provided herewith may be directed at both HSV2 and HSV1 infections (and thus at both HSV2 and HSV1 related disease, i.e., genital herpes and herpes labialis, respectively), or at HSV2 infections (thus primarily aiming at treatment of genital herpes), or at HSV1 infections (thus primarily aiming at treatment of herpes labialis).

By **"immunologically effective amount"** is intended that the administration of that amount of antigen (or immunogenic composition containing the antigen) to a subject, either in a single dose or as part of a series, is effective for inducing a measurable immune response against the administered antigen in the subject. This amount varies depending upon the health and physical condition of the individual to be treated, age, the taxonomic group of individual to be treated (e.g. human, non-human primate, etc.), the capacity of the individual's immune system to synthesize antibodies, the degree of protection desired, the formulation of the composition or vaccine, the treating doctor's assessment of the medical situation, the severity of the disease, the potency of the compound administered, the mode of administration, and other relevant factors. Vaccines as disclosed herein are typically therapeutic. In some embodiments, the immunogenic compositions disclosed herein may induce an effective immune response against a herpes virus infection, i.e., a response sufficient for treatment or prevention of herpes virus infection, such as recurrent HSV infection. Further uses of immunogenic compositions or vaccines comprising the nucleic acid constructs as described herein are provided herein below. It will be readily understood that the HSV Fc receptor or an immunogenic fragment or variant thereof and immunogenic compositions described herein are suited for use in regimens involving repeated delivery of the HSV Fc receptor or immunogenic fragment or variant thereof over time for therapeutic purposes. Suitably, a prime-boost regimen may be used. Prime-boost refers to eliciting two separate immune responses in the same individual: (i) an initial priming of the immune system followed by (ii) a secondary or boosting of the immune system weeks or months after the primary immune response has been established. Preferably, a boosting composition is administered about two to about 12 weeks after administering the priming composition to the subject, for example about 2, 3, 4, 5 or 6 weeks after administering the priming composition. In one embodiment, a boosting composition is administered one or two months after the priming composition. In one embodiment, a first boosting composition is administered one or two months after the priming composition and a second boosting composition is administered one or two months after the first boosting composition.

Dosages will depend primarily on factors such as the route of administration, the condition being treated, the age, weight and health of the subject, and may thus vary among subjects. For example, a therapeutically effective adult human dosage of the HSV Fc receptor or an immunogenic fragment or variant thereof may contain 1 to 250 μg, for example 2 to 100 μg of the HSV FcR or immunogenic fragment or variant thereof, e.g. about 5, 10, 20, 30, 40, 50, 60, 70, 80, 90 or 100 μg of the HSV FcR or immunogenic fragment or variant thereof.

When a HSV FcR binding partner or fragment thereof is administered to the subject together with the HSV FcR or immunogenic fragment or variant thereof, a therapeutically effective adult human dosage of the HSV FcR binding partner or fragment thereof may contain 5 to 250 μg, for example 10 to 100 μg of the HSV FcR binding partner or fragment thereof, e.g. about 10, 20, 30, 40, 50, 60, 70, 80, 90 or 100 μg of the HSV FcR binding partner or fragment thereof.

In a preferred embodiment, when a HSV FcR binding partner or fragment thereof is administered to the subject together with the HSV FcR or immunogenic fragment or variant thereof, the doses of the HSV FcR immunogenic fragment or variant thereof and the HSV FcR binding partner or fragment thereof are at a stochiometric ratio of about 1:1.

Generally, a human dose will be in a volume of between 0.1 ml and 2 ml. Thus, the composition described herein can be formulated in a volume of, for example, about 0.1, 0.15, 0.2, 0.5, 1.0, 1.5 or 2.0 ml human dose per individual or combined immunogenic components.

One of skill in the art may adjust these doses, depending on the route of administration and the subject being treated.

The therapeutic immune response against the HSV Fc receptor or an immunogenic fragment or variant thereof can be monitored to determine the need, if any, for boosters. Following an assessment of the immune response (e.g., of CD4+ T cell response, CD8+ T cell response, antibody titers in the serum), optional booster immunizations may be administered.

*In vitro* or *in vivo* testing methods suitable for assessing the immune response against the HSV Fc receptor or fragment thereof according to the invention are known to those of skill in the art. For example, a HSV Fc receptor or fragment or variant thereof can be tested for its effect on induction of proliferation or effector function of the particular lymphocyte type of interest, e.g., B cells, T cells, T cell lines, and T cell clones. For example, spleen cells from immunized mice can be isolated and the capacity of cytotoxic T lymphocytes to lyse autologous target cells that contain a HSV Fc receptor or fragment thereof according to the invention can be assessed. In addition, T helper cell differentiation can be analyzed by measuring proliferation or production of TH1 (IL-2, TNF-α and IFN-γ) cytokines in CD4+ T cells by cytoplasmic cytokine staining and flow cytometry analysis. The HSV Fc receptor or fragment or variant thereof according to the invention can also be tested for ability to induce humoral immune responses, as evidenced, for example, by investigating the activation of B cells in the draining lymph node, by measuring B cell production of antibodies specific for an HSV antigen of interest in the serum. These assays can be conducted using, for example, peripheral B lymphocytes from immunized individuals.

*Nucleic acid*

In a further aspect, the invention provides a **nucleic acid** encoding a HSV1 or HSV2 Fc receptor or immunogenic fragment or variant thereof of the invention.

The term **"nucleic acid"** in general means a polymeric form of nucleotides of any length, which contain deoxyribonucleotides, ribonucleotides, and/or their analogs. It includes DNA, RNA, DNA/RNA hybrids. It also includes DNA or RNA analogs, such as those containing modified backbones (e.g. peptide nucleic acids (PNAs) or phosphorothioates) or modified bases. Thus, the nucleic acid of the disclosure includes mRNA, DNA, cDNA, recombinant nucleic acids, branched nucleic acids, plasmids, vectors, etc. Where the nucleic acid takes the form of RNA, it may or may not have a 5' cap. Nucleic acid molecules as disclosed herein can take various forms (e.g. single-stranded, double-stranded). Nucleic acid molecules may be circular or branched, but will generally be linear.

The nucleic acids used herein are preferably provided in purified or substantially purified form i.e. substantially free from other nucleic acids (e.g. free from naturally-occurring nucleic acids), generally being at least about 50% pure (by weight), and usually at least about 90% pure.

The nucleic acid molecules may be produced by any suitable means, including recombinant production, chemical synthesis, or other synthetic means. Suitable production techniques are well known to those of skill in the art. Typically, the nucleic acids will be in recombinant form, i.e. a form which does not occur in nature. For example, the nucleic acid may comprise one or more heterologous nucleic acid sequences (e.g. a sequence encoding another antigen and/or a control sequence such as a promoter or an internal ribosome entry site) in addition to the nucleic acid sequences encoding the viral Fc receptor or fragment thereof or heterodimer. The sequence or chemical structure of the nucleic acid may be modified compared to naturally-occurring sequences which encode the viral Fc receptor or fragment thereof or heterodimer. The sequence of the nucleic acid molecule may be modified, e.g. to increase the efficacy of expression or replication of the nucleic acid, or to provide additional stability or resistance to degradation.

The nucleic acid molecule encoding the HSV Fc receptor or fragment or variant thereof may be codon optimized. Herein **"codon optimized"** is intended to refer to modification with respect to codon usage that may increase translation efficacy and/or half- life of the nucleic acid. A poly A tail (e.g., of about 30 adenosine residues or more) may be attached to the 3' end of the RNA to increase its half-life. The 5' end of the RNA may be capped with a modified ribonucleotide with the structure m7G (5') ppp (5') N (cap 0 structure) or a derivative thereof, which can be incorporated during RNA synthesis or can be enzymatically engineered after RNA transcription (e.g., by using Vaccinia Virus Capping Enzyme (VCE) consisting of mRNA triphosphatase, guanylyl- transferase and guanine-7-methytransferase, which catalyzes the construction of N7-monomethylated cap 0 structures). Cap 0 structure plays an important role in maintaining the stability and translational efficacy of the RNA molecule. The 5' cap of the RNA molecule may be further modified by a 2 '-O-Methyltransferase which results in the generation of a cap 1 structure (m7Gppp [m2 '-O] N), which may further increase translation efficacy.

In a preferred embodiment, the nucleic acid encodes a HSV2 or HSV1 Fc receptor or immunogenic fragment or variant thereof that is a heterodimer with a binding partner from HSV or a fragment thereof wherein the expression of the HSV2 or HSV1 FcR or immunogenic fragment or variant thereof is under the control of a subgenomic promoter, suitably the 26S subgenomic promoter shown in SEQ ID NO: 126, or a variant therefrom which is at least 60%, 65%, 70%, 75% 80%, 85%, 90%, 95%, 96%, 97%, 98%, 99% or 99,5% identical thereto.

In a preferred embodiment, the HSV1 or HSV2 FcR or immunogenic fragment or variant thereof and its binding partner or fragment thereof are separated by an internal ribosomal entry site (IRES) sequence. In a preferred embodiment, the IRES sequence is an IRES EV71 sequence. In a preferred embodiment, the IRES sequence comprises or consists of the sequence shown in SEQ ID NO: 127, or a variant therefrom which is at least 60%, 65%, 70%, 75% 80%, 85%, 90%, 95%, 96%, 97%, 98%, 99% or 99,5% identical thereto.

In another embodiment, the sequences encoding the HSV2 or HSV1 FcR or immunogenic fragment or variant thereof and its binding partner or fragment thereof are separated by a 2A "self-cleaving" peptide sequences. In one embodiment, the 2A "self-cleaving" peptide sequence is a GSG-P2A sequence, suitably comprising or consisting of the sequence shown in SEQ ID NO: 124, or a variant therefrom which is at least 60%, 65%, 70%, 75% 80%, 85%, 90%, 95%, 96%, 97%, 98%, 99% or 99,5% identical thereto. In one embodiment, the 2A "self-cleaving" peptide sequence is a F2A sequence, suitably comprising or consisting of the sequence shown in SEQ ID NO: 125, or a variant therefrom which is at least 60%, 65%, 70%, 75% 80%, 85%, 90%, 95%, 96%, 97%, 98%, 99% or 99,5% identical thereto.

In yet another embodiment, the sequences encoding the HSV2 or HSV1 FcR or immunogenic fragment or variant thereof and its binding partner or fragment thereof are separated by a subgenomic promoter. In one embodiment, the subgenomic promoter is a 26S subgenomic promoter, suitably comprising or consisting of the sequence shown in SEQ ID NO: 126, or a variant therefrom which is at least 60%, 65%, 70%, 75% 80%, 85%, 90%, 95%, 96%, 97%, 98%, 99% or 99,5% identical thereto

The nucleic acid molecule for the use of the invention may, for example, be RNA or DNA, such as a plasmid DNA. In a preferred embodiment, the nucleic acid molecule is an RNA molecule.

*Vectors, host cells*

Described herein is a **vector** comprising a nucleic acid for use according to the invention.

A vector may be any suitable nucleic acid molecule including naked DNA or RNA, a plasmid, a virus, a cosmid, phage vector such as lambda vector, an artificial chromosome such as a BAC (bacterial artificial chromosome), or an episome. Alternatively, a vector may be a transcription and/or expression unit for cell-free in vitro transcription or expression, such as a T7-compatible system. The vectors may be used alone or in combination with other vectors such as adenovirus sequences or fragments, or in combination with elements from non-adenovirus sequences. Suitably, the vector has been substantially altered (e.g., having a gene or functional region deleted and/or inactivated) relative to a wild type sequence, and replicates and expresses the inserted polynucleotide sequence, when introduced into a host cell.

Also described herein is a **cell** comprising a HSV2 or HSV1 Fc receptor or fragment or variant thereof, or a nucleic acid or a vector encoding it.

The HSV Fc receptor or immunogenic fragment or variant thereof, or the HSV FcR binding partner or fragment thereof for use according to the invention are suitably produced by recombinant technology. **"Recombinant"** means that the polynucleotide is the product of at least one of cloning, restriction or ligation steps, or other procedures that result in a polynucleotide that is distinct from a polynucleotide found in nature. A recombinant vector is a vector comprising a recombinant polynucleotide.

In one embodiment, a heterodimer as described herein is expressed from a multicistronic vector. Suitably, the heterodimer is expressed from a single vector in which the nucleic sequences encoding the HSV FcR or immunogenic fragment or variant thereof and its binding partner or fragment thereof are separated by an internal ribosomal entry site (IRES) sequence (Mokrejš, Martin, et al. "IRESite: the database of experimentally verified IRES structures (www. iresite. org)." Nucleic acids research 34.suppl_1 (2006): D125-D130.). In a preferred embodiment, the IRES is an IRES EV71 sequence. In a preferred embodiment, the IRES comprises or consists of the sequence shown in SEQ ID NO: 127, or a variant therefrom which is at least 60%, 65%, 70%, 75% 80%, 85%, 90%, 95%, 96%, 97%, 98%, 99% or 99,5% identical thereto.

Alternatively, the two nucleic sequences can be separated by a viral 2A or '2A-like' sequence, which results in production of two separate polypeptides. 2A sequences are known from various viruses, including foot-and-mouth disease virus, equine rhinitis A virus, *Thosea asigna* virus, and porcine theschovirus-1. See *e.g.,* Szymczak et al., Nature Biotechnology 22:589-594 (2004), Donnelly et al., J Gen Virol.; 82(Pt 5): 1013-25 (2001).

Optionally, to facilitate expression and recovery, the HSV2 or HSV1 Fc receptor or immunogenic fragment or variant thereof and/or the HSV FcR binding partner or fragment thereof may include a signal peptide at the N-terminus. A signal peptide can be selected from among numerous signal peptides known in the art, and is typically chosen to facilitate production and processing in a system selected for recombinant expression. In one embodiment, the signal peptide is the one naturally present in the native HSV Fc protein or binding partner. The signal peptide of the HSV2 gE from strain SD90e is located at residues 1-20 of SEQ ID NO: 1. Signal peptide for gE proteins from other HSV strains can be identified by sequence alignment. The signal peptide of the HSV2 gI from strain SD90e is located at residues 1-20 of SEQ ID NO:2. Signal peptide for gI proteins from other HSV strains can be identified by sequence alignment.

Optionally, the HSV Fc receptor or immunogenic fragment or variant thereof and/or the HSV FcR binding partner or fragment thereof can include the addition of an amino acid sequence that constitutes a tag, which can facilitate detection (e.g. an epitope tag for detection by monoclonal antibodies) and/or purification (e.g. a polyhistidine-tag to allow purification on a nickel-chelating resin) of the proteins. In a certain embodiment, cleavable linkers may be used. This allows for the tag to be separated from the purified complex, for example by the addition of an agent capable of cleaving the linker. A number of different cleavable linkers are known to those of skill in the art.

When a host cell herein is cultured under suitable conditions, the nucleic acid can express the HSV Fc receptor or immunogenic fragment or variant thereof, the HSV FcR binding partner or fragment thereof, and/or both peptides of the heterodimer. The HSV Fc receptor or immunogenic fragment or variant thereof, the HSV FcR binding partner or fragment thereof, and/or the heterodimer may then be secreted from the host cell. Suitable host cells include, for example, insect cells (e.g., *Aedes aegypti, Autographa californica, Bombyx mori, Drosophila melanogaster, Spodoptera frugiperda, and Trichoplusia ni*), mammalian cells (e.g., human, non-human primate, horse, cow, sheep, dog, cat, and rodent (e.g., hamster), avian cells (e.g., chicken, duck, and geese), bacteria (e.g., *E. coli, Bacillus subtilis, and Streptococcus spp.*), yeast cells (*e.g., Saccharomyces cerevisiae, Candida albicans, Candida maltosa, Hansenual polymorpha, Kluyveromyces fragilis, Kluyveromyces lactis, Pichia guillerimondii, Pichia pastoris, Schizosaccharomyces pombe and Yarrowia lipolytica*), Tetrahymena cells (e.g., *Tetrahymena thermophila*) or combinations thereof. Suitably, the host cell should be one that has enzymes that mediate glycosylation. Bacterial hosts are generally not suitable for such modified proteins, unless the host cell is modified to introduce glycosylation enzymes; instead, a eukaryotic host, such as insect cell, avian cell, or mammalian cell should be used.

Suitable insect cell expression systems, such as baculovirus systems, are known to those of skill in the art and described in, e.g., Summers and Smith, Texas Agricultural Experiment Station Bulletin No. 1555 (1987). Suitable insect cells include, for example, Sf9 cells, Sf21 cells, Tn5 cells, Schneider S2 cells, and High Five cells (a clonal isolate derived from the parental *Trichoplusia ni* BTI-TN-5B1-4 cell line (Invitrogen)).

Avian cell expression systems are also known to those of skill in the art and described in, e.g., U.S. Patent Nos. 5,340,740; 5,656,479; 5,830,510; 6,114,168; and 6,500,668; European Patent No. EP 0787180B; European Patent Application No. EP03291813.8; WO 03/043415; and WO 03/076601. Suitable avian cells include, for example, chicken embryonic stem cells (e.g., EBx® cells), chicken embryonic fibroblasts, chicken embryonic germ cells, duck cells (e.g., AGE1.CR and AGE1.CR.pIX cell lines (ProBioGen) which are described, for example, in Vaccine 27:4975-4982 (2009) and WO2005/042728), EB66 cells, and the like.

Preferably, the host cells are mammalian cells (e.g., human, non-human primate, horse, cow, sheep, dog, cat, and rodent (e.g., hamster)). Suitable mammalian cells include, for example, Chinese hamster ovary (CHO) cells, human embryonic kidney cells (HEK-293 cells, typically transformed by sheared adenovirus type 5 DNA), NIH-3T3 cells, 293-T cells, Vero cells, HeLa cells, PERC.6 cells (ECACC deposit number 96022940), Hep G2 cells, MRC-5 (ATCC CCL-171), WI-38 (ATCC CCL-75), fetal rhesus lung cells (ATCC CL-160), Madin-Darby bovine kidney ("MDBK") cells, Madin-Darby canine kidney ("MDCK") cells (e.g., MDCK (NBL2), ATCC CCL34; or MDCK 33016, DSM ACC 2219), baby hamster kidney (BHK) cells, such as BHK21-F, HKCC cells, and the like.

In certain embodiments, the recombinant nucleic acids encoding the HSV Fc receptor or immunogenic fragment or variant thereof, the HSV FcR binding partner or fragment thereof, and/or the heterodimer are codon optimized for expression in a selected prokaryotic or eukaryotic host cell.

The HSV Fc receptor or immunogenic fragment or variant thereof, the HSV FcR binding partner or fragment thereof, and/or the heterodimer can be recovered and purified from recombinant cell cultures by any of a number of methods well known in the art, including ammonium sulfate or ethanol precipitation, acid extraction, anion or cation exchange chromatography, phosphocellulose chromatography, hydrophobic interaction chromatography, affinity chromatography (e.g., using any of the tagging systems noted herein), hydroxyapatite chromatography, and lectin chromatography. Protein refolding steps can be used, as desired, in completing configuration of the mature protein.

Finally, high performance liquid chromatography (HPLC) can be employed in the final purification steps. In addition to the references noted above, a variety of purification methods are well known in the art, including, e.g., those set forth in Sandana (1997) Bioseparation of Proteins, Academic Press, Inc.; and Bollag et al. (1996) Protein Methods, 2nd Edition Wiley-Liss, NY; Walker (1996) The Protein Protocols Handbook Humana Press, NJ, Harris and Angal (1990) Protein Purification Applications: A Practical Approach IRL Press at Oxford, Oxford, U.K.; Scopes (1993) Protein Purification: Principles and Practice 3rd Edition Springer Verlag, NY; Janson and Ryden (1998) Protein Purification: Principles, High Resolution Methods and Applications, Second Edition Wiley-VCH, NY; and Walker (1998) Protein Protocols on CD-ROM Humana Press, NJ.

The term "**purification**" or "**purifying**" refers to the process of removing components from a composition or host cell or culture, the presence of which is not desired. Purification is a relative term, and does not require that all traces of the undesirable component be removed from the composition. In the context of vaccine production, purification includes such processes as centrifugation, dialyzation, ion-exchange chromatography, and size-exclusion chromatography, affinity-purification or precipitation. Thus, the term "purified" does not require absolute purity; rather, it is intended as a relative term. A preparation of substantially pure nucleic acid or protein can be purified such that the desired nucleic acid, or protein, represents at least 50% of the total nucleic acid content of the preparation. In certain embodiments, a substantially pure nucleic acid, or protein, will represent at least 60%, at least 70%, at least 80%, at least 85%, at least 90%, or at least 95% or more of the total nucleic acid or protein content of the preparation. Immunogenic molecules or antigens or antibodies which *have not* been subjected to any purification steps (*i.e.,* the molecule as it is found in nature) are not suitable for pharmaceutical (*e.g.*, vaccine) use.

Suitably, the recovery yield for the HSV Fc receptor or immunogenic fragment or variant thereof, the HSV FcR binding partner or fragment thereof, and/or the heterodimer is higher than 2mg per liter, preferably higher than 5, 10, 15 or 20 mg per liter, more preferably still higher than 25 mg per liter.

Suitably, the level of aggregation for the HSV Fc receptor or immunogenic fragment or variant thereof, the HSV FcR binding partner or fragment or variant thereof, and/or the heterodimer is lower 20%, preferably lower than 15 or 10 %, more preferably still lower than 5%.

*Adjuvants*

In a preferred embodiment, the HSV2 or HSV1 Fc receptor or fragment or variant thereof is administered to the subject together with an adjuvant. An "**adjuvant**" as used herein refers to a composition that enhances the immune response to an antigen in the intended subject, such as a human subject.

Examples of suitable adjuvants include but are not limited to inorganic adjuvants (*e.g.* inorganic metal salts such as aluminium phosphate or aluminium hydroxide), organic adjuvants (*e.g.* saponins, such as QS21, or squalene), oil-based adjuvants (*e.g.* Freund's complete adjuvant and Freund's incomplete adjuvant), oil-in-water emulsions, cytokines (*e.g.* IL-1β, IL-2, IL-7, IL-12, IL-18, GM-CFS, and INF-γ) particulate adjuvants (*e.g.* immuno-stimulatory complexes (ISCOMS), liposomes, or biodegradable microspheres), virosomes, bacterial adjuvants (*e.g.* monophosphoryl lipid A, such as 3-de-O-acylated monophosphoryl lipid A (3D-MPL), or muramyl peptides), synthetic adjuvants (*e.g.* non-ionic block copolymers, muramyl peptide analogues, or synthetic lipid A), synthetic polynucleotides adjuvants (*e.g* polyarginine or polylysine), Toll-like receptor (TLR) agonists (including TLR-1, TLR-2, TLR-3, TLR-4, TLR-5, TLR-6, TLR-7, TLR-8 and TLR-9 agonists) and immunostimulatory oligonucleotides containing unmethylated CpG dinucleotides ("CpG").

In a preferred embodiment, the adjuvant comprises a TLR agonist and/or an immunologically active saponin. Preferably still, the adjuvant may comprise or consist of a TLR agonist and a saponin in a liposomal formulation. The ratio of TLR agonist to saponin may be 5:1, 4:1, 3:1, 2:1 or 1:1.

The use of TLR agonists in adjuvants is well-known in art and has been reviewed e.g. by Lahiri *et al.* (2008) Vaccine 26:6777. TLRs that can be stimulated to achieve an adjuvant effect include TLR2, TLR4, TLR5, TLR7, TLR8 and TLR9. TLR2, TLR4, TLR7 and TLR8 agonists, particularly TLR4 agonists, are preferred.

Suitable TLR4 agonists include lipopolysaccharides, such as monophosphoryl lipid A (MPL) and 3-O-deacylated monophosphoryl lipid A (3D-MPL). US patent 4,436,727 discloses MPL and its manufacture. US patent 4,912,094 and reexamination certificate B1 4,912,094 discloses 3D-MPL and a method for its manufacture. Another TLR4 agonist is glucopyranosyl lipid adjuvant (GLA), a synthetic lipid A-like molecule (see, e.g. Fox et al. (2012) Clin. Vaccine Immunol 19:1633). In a further embodiment, the TLR4 agonist may be a synthetic TLR4 agonist such as a synthetic disaccharide molecule, similar in structure to MPL and 3D-MPL or may be synthetic monosaccharide molecules, such as the aminoalkyl glucosaminide phosphate (AGP) compounds disclosed in, for example, WO9850399, WO0134617, WO0212258, WO3065806, WO04062599, WO06016997, WO0612425, WO03066065, and WO0190129. Such molecules have also been described in the scientific and patent literature as lipid A mimetics. Lipid A mimetics suitably share some functional and/or structural activity with lipid A, and in one aspect are recognised by TLR4 receptors. AGPs as described herein are sometimes referred to as lipid A mimetics in the art. In a preferred embodiment, the TLR4 agonist is 3D-MPL.TLR4 agonists, such as 3-O-deacylated monophosphoryl lipid A (3D-MPL), and their use as adjuvants in vaccines has e.g. been described in WO 96/33739 and WO2007/068907 and reviewed in Alving et al. (2012) Curr Opin in Immunol 24:310.

Suitably, the adjuvant comprises an immunologically active saponin, such as an immunologically active saponin fraction, such as QS21.

Adjuvants comprising saponins have been described in the art. Saponins are described in: Lacaille-Dubois and Wagner (1996) A review of the biological and pharmacological activities of saponins, Phytomedicine vol 2:363. Saponins are known as adjuvants in vaccines. For example, Quil A (derived from the bark of the South American tree *Quillaja Saponaria Molina*), was described by Dalsgaard et al. in 1974 ("Saponin adjuvants", Archiv. fur die gesamte Virusforschung, Vol. 44, Springer Verlag, Berlin, 243) to have adjuvant activity. Purified fractions of Quil A have been isolated by HPLC which retain adjuvant activity without the toxicity associated with Quil A (Kensil et al. (1991) J. Immunol. 146: 431). Quil A fractions are also described in US 5,057,540 and "Saponins as vaccine adjuvants", Kensil, C. R., Crit Rev Ther Drug Carrier Syst, 1996, 12 (1-2):1-55.

Two Quil A such fractions, suitable for use in the present invention, are QS7 and QS21 (also known as QA-7 and QA-21). QS21 is a preferred immunologically active saponin fraction for use in the present invention. QS21 has been reviewed in Kensil (2000) In O'Hagan: Vaccine Adjuvants: preparation methods and research protocols, Homana Press, Totowa, New Jersey, Chapter 15. Particulate adjuvant systems comprising fractions of Quil A, such as QS21 and QS7, are e.g. described in WO 96/33739, WO 96/11711 and WO2007/068907.

In addition to the other components, the adjuvant preferably comprises a sterol. The presence of a sterol may further reduce reactogenicity of compositions comprising saponins, see e.g. EP0822831. Suitable sterols include beta-sitosterol, stigmasterol, ergosterol, ergocalciferol and cholesterol. Cholesterol is particularly suitable. Suitably, the immunologically active saponin fraction is QS21 and the ratio of QS21:sterol is from 1:100 to 1:1 w/w, such as from 1:10 to 1:1 w/w, e.g. from 1:5 to 1:1 w/w.

In a preferred embodiment, the adjuvant comprises a TLR4 agonist and an immunologically active saponin. In a more preferred embodiment, the TLR4 agonist is 3D-MPL and the immunologically active saponin is QS21.

In some embodiments, the adjuvant is presented in the form of an oil-in-water emulsion, e.g. comprising squalene, alpha-tocopherol and a surfactant (see e.g. WO95/17210) or in the form of a liposome. A liposomal presentation is preferred.

The term "liposome" when used herein refers to uni- or multilamellar (particularly 2, 3, 4, 5, 6, 7, 8, 9, or 10 lamellar depending on the number of lipid membranes formed) lipid structures enclosing an aqueous interior. Liposomes and liposome formulations are well known in the art. Liposomal presentations are e.g. described in WO 96/33739 and WO2007/068907. Lipids which are capable of forming liposomes include all substances having fatty or fat-like properties. Lipids which can make up the lipids in the liposomes may be selected from the group comprising glycerides, glycerophospholipides, glycerophosphinolipids, glycerophosphonolipids, sulfolipids, sphingolipids, phospholipids, isoprenolides, steroids, stearines, sterols, archeolipids, synthetic cationic lipids and carbohydrate containing lipids. In a particular embodiment of the invention the liposomes comprise a phospholipid. Suitable phospholipids include (but are not limited to): phosphocholine (PC) which is an intermediate in the synthesis of phosphatidylcholine; natural phospholipid derivates: egg phosphocholine, egg phosphocholine, soy phosphocholine, hydrogenated soy phosphocholine, sphingomyelin as natural phospholipids; and synthetic phospholipid derivates: phosphocholine (didecanoyl-L-a-phosphatidylcholine [DDPC], dilauroylphosphatidylcholine [DLPC], dimyristoylphosphatidylcholine [DMPC], dipalmitoyl phosphatidylcholine [DPPC], Distearoyl phosphatidylcholine [DSPC], Dioleoyl phosphatidylcholine, [DOPC], 1-palmitoyl, 2-oleoylphosphatidylcholine [POPC], Dielaidoyl phosphatidylcholine [DEPC]), phosphoglycerol (1,2-Dimyristoyl-sn-glycero-3-phosphoglycerol [DMPG], 1,2-dipalmitoyl-sn-glycero-3-phosphoglycerol [DPPG], 1,2-distearoyl-sn-glycero-3-phosphoglycerol [DSPG], 1-palmitoyl-2-oleoyl-sn- glycero-3-phosphoglycerol [POPG]), phosphatidic acid (1,2-dimyristoyl-sn-glycero-3-phosphatidic acid [DMPA], dipalmitoyl phosphatidic acid [DPPA], distearoyl-phosphatidic acid [DSPA]), phosphoethanolamine (1,2-dimyristoyl-sn-glycero-3-phosphoethanolamine [DMPE], 1,2-Dipalmitoyl-sn-glycero-3-phosphoethanolamine [DPPE], 1,2-distearoyl-sn-glycero-3-phosphoethanolamine [DSPE], 1,2-Dioleoyl-sn-Glycero-3-Phosphoethanolamine [DOPE]), phoshoserine, polyethylene glycol [PEG] phospholipid.

Liposome size may vary from 30 nm to several μm depending on the phospholipid composition and the method used for their preparation. In particular embodiments of the invention, the liposome size will be in the range of 50 nm to 500 nm and in further embodiments 50 nm to 200 nm. Dynamic laser light scattering is a method used to measure the size of liposomes well known to those skilled in the art.

In a particularly suitable embodiment, liposomes used in the invention comprise DOPC and a sterol, in particular cholesterol. Thus, in a particular embodiment, compositions for use according to the invention comprise QS21 in any amount described herein in the form of a liposome, wherein said liposome comprises DOPC and a sterol, in particular cholesterol.

In a more preferred embodiment, the adjuvant comprises a 3D-MPL and QS21 in a liposomal formulation.

In one embodiment, the adjuvant comprises between 25 and 75, such as between 35 and 65 micrograms (for example about or exactly 50 micrograms) of 3D-MPL and between 25 and 75, such as between 35 and 65 (for example about or exactly 50 micrograms) of QS21 in a liposomal formulation.

In another embodiment, the adjuvant comprises between 12.5 and 37.5, such as between 20 and 30 micrograms (for example about or exactly 25 micrograms) of 3D-MPL and between 12.5 and 37.5, such as between 20 and 30 micrograms (for example about or exactly 25 micrograms) of QS21 in a liposomal formulation.

In another embodiment of the present invention, the adjuvant comprises or consists of an oil-in-water emulsion. Suitably, an oil-in-water emulsion comprises a metabolisable oil and an emulsifying agent. A particularly suitable metabolisable oil is squalene. Squalene (2,6,10,15,19,23-Hexamethyl-2,6,10,14,18,22-tetracosahexaene) is an unsaturated oil which is found in large quantities in shark-liver oil, and in lower quantities in olive oil, wheat germ oil, rice bran oil, and yeast. In one embodiment, the metabolisable oil is present in the immunogenic composition in an amount of 0.5% to 10% (v/v) of the total volume of the composition. A particularly suitable emulsifying agent is polyoxyethylene sorbitan monooleate (POLYSORBATE 80 or TWEEN 80). In one embodiment, the emulsifying agent is present in the immunogenic composition in an amount of 0.125 to 4% (v/v) of the total volume of the composition. The oil-in-water emulsion may optionally comprise a tocol. Tocols are well known in the art and are described in EP0382271 B1. Suitably, the tocol may be alpha-tocopherol or a derivative thereof such as alpha-tocopherol succinate (also known as vitamin E succinate). In one embodiment, the tocol is present in the adjuvant composition in an amount of 0.25% to 10% (v/v) of the total volume of the immunogenic composition. The oil-in-water emulsion may also optionally comprise sorbitan trioleate (SPAN 85).

In an oil-in-water emulsion, the oil and emulsifier should be in an aqueous carrier. The aqueous carrier may be, for example, phosphate buffered saline or citrate.

In particular, the oil-in-water emulsion systems used in the present invention have a small oil droplet size in the sub-micron range. Suitably the droplet sizes will be in the range 120 to 750 nm, more particularly sizes from 120 to 600 nm in diameter. Even more particularly, the oil-in water emulsion contains oil droplets of which at least 70% by intensity are less than 500 nm in diameter, more particular at least 80% by intensity are less than 300 nm in diameter, more particular at least 90% by intensity are in the range of 120 to 200 nm in diameter.

It will be understood that the HSV Fc receptor or fragment or variant thereof and the adjuvant may be stored separately and admixed prior to administration (*ex tempo*) to a subject. The HSV Fc receptor or fragment or variant thereof and the adjuvant may also be administered separately but concomitantly to a subject.

In one aspect, there is provided a **kit** comprising or consisting of a HSV2 or HSV1 Fc receptor or immunogenic fragment or variant thereof as described herein and an adjuvant.

*Sequence Comparison*

For the purposes of comparing two closely-related polynucleotide or polypeptide sequences, the "sequence identity" or "% identity" between a first sequence and a second sequence may be calculated using an alignment program, such as BLAST® (available at blast.ncbi.nlm.nih.gov, last accessed 12 September 2016) using standard settings. The percentage identity is the number of identical residues divided by the length of the alignment, multiplied by 100. An alternative definition of identity is the number of identical residues divided by the number of aligned residues, multiplied by 100. Alternative methods include using a gapped method in which gaps in the alignment, for example deletions in one sequence relative to the other sequence, are considered. Polypeptide or polynucleotide sequences are said to be identical to other polypeptide or polynucleotide sequences, if they share 100% sequence identity over their entire length.

A "difference" between two sequences refers to an insertion, deletion or substitution, e.g., of a single amino acid residue in a position of one sequence, compared to the other sequence.

For the purposes of comparing a first, reference polypeptide sequence to a second, comparison polypeptide sequence, the number of additions, substitutions and/or deletions made to the first sequence to produce the second sequence may be ascertained. An addition is the addition of one amino acid residue into the sequence of the first polypeptide (including addition at either terminus of the first polypeptide). A substitution is the substitution of one amino acid residue in the sequence of the first polypeptide with one different amino acid residue. A deletion is the deletion of one amino acid residue from the sequence of the first polypeptide (including deletion at either terminus of the first polypeptide).

Suitably substitutions in the sequences of the present invention may be conservative substitutions. A conservative substitution comprises the substitution of an amino acid with another amino acid having a physico-chemical property similar to the amino acid that is substituted (see, for example, Stryer et al, Biochemistry, 5th Edition 2002, pages 44-49). Preferably, the conservative substitution is a substitution selected from the group consisting of: (i) a substitution of a basic amino acid with another, different basic amino acid; (ii) a substitution of an acidic amino acid with another, different acidic amino acid; (iii) a substitution of an aromatic amino acid with another, different aromatic amino acid; (iv) a substitution of a non-polar, aliphatic amino acid with another, different non-polar, aliphatic amino acid; and (v) a substitution of a polar, uncharged amino acid with another, different polar, uncharged amino acid. A basic amino acid is preferably selected from the group consisting of arginine, histidine, and lysine. An acidic amino acid is preferably aspartate or glutamate. An aromatic amino acid is preferably selected from the group consisting of phenylalanine, tyrosine and tryptophan. A non-polar, aliphatic amino acid is preferably selected from the group consisting of alanine, valine, leucine, methionine and isoleucine. A polar, uncharged amino acid is preferably selected from the group consisting of serine, threonine, cysteine, proline, asparagine and glutamine. In contrast to a conservative amino acid substitution, a non-conservative amino acid substitution is the exchange of one amino acid with any amino acid that does not fall under the above-outlined conservative substitutions (i) through (v).

*Terms*

**"Encoding"** refers to the inherent property of specific sequences of nucleotides in a polynucleotide, to act as a template for synthesis of other polymers and macromolecules in biological processes, e.g., synthesis of peptides or proteins. Both the coding strand of a double-stranded nucleotide molecule (the sequence of which is usually provided in sequence listings), and the non-coding strand (used as the template for transcription of a gene or cDNA), can be referred to as encoding the peptide or protein. Unless otherwise specified, as used herein a "nucleotide sequence encoding an amino acid sequence" includes all nucleotide sequences that are degenerate versions of each other and that encode the same amino acid sequence.

The term **"expression"** or **"expressing"** as used herein is defined as the transcription and/or translation of a particular nucleotide sequence driven by its operably linked promoter.

Unless otherwise explained in the context of this disclosure, all technical and scientific terms used herein have the same meaning as commonly understood by one of ordinary skill in the art to which this disclosure belongs. Definitions of common terms in molecular biology can be found in Benjamin Lewin, Genes V, published by Oxford University Press, 1994 (ISBN 0-19-854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).

The singular terms "a," "an," and "the" include plural referents unless context clearly indicates otherwise. Similarly, the word "or" is intended to include "and" unless the context clearly indicates otherwise. The term "plurality" refers to two or more. It is further to be understood that all base sizes or amino acid sizes, and all molecular weight or molecular mass values, given for nucleic acids or polypeptides are approximate, and are provided for description. Additionally, numerical limitations given with respect to concentrations or levels of a substance, such as an antigen, are intended to be approximate. Thus, where a concentration is indicated to be at least (for example) 200 pg, it is intended that the concentration be understood to be at least approximately (or "about" or "~") 200 pg.

The term "comprises" means "includes." Thus, unless the context requires otherwise, the word "comprises," and variations such as "comprise" and "comprising" will be understood to imply the inclusion of a stated compound or composition (*e.g.*, nucleic acid, polypeptide, antigen) or step, or group of compounds or steps, but not to the exclusion of any other compounds, composition, steps, or groups thereof.

Amino acid sequences provided herein are designated by either single-letter or three-letter nomenclature, as is known in the art (see, e.g., Eur. J. Biochem. 138:9-37(1984)).

Although methods and materials similar or equivalent to those described herein can be used in the practice or testing of this disclosure, suitable methods and materials are described below.

The present invention will now be further described by means of the following non-limiting examples.

## EXAMPLES

### EXAMPLE 1

The general objective of this study was to generate immunogenicity data with five new recombinant HSV-2 gE/gI proteins containing mutations in gE Fc binding domain to drop the avidity to human Immunoglobulin G Fc.

The primary objective was to compare to NaCl control group, the anti-HSV-2 gE-specific polyclonal antibody (pAb) response induced by several AS01-adjuvanted recombinant HSV-2 gE/gI proteins containing mutations in the amino acid sequence 14 days post third immunization.

As secondary objectives, 14 days post third immunization, the anti-HSV-2 gI-specific polyclonal antibody (pAb) response and the anti-HSV-2 gE/gI-specific CD4+T cell responses induced by different mutants of HSV-2 gE/gI protein were compared to NaCl control group. In addition, head-to-head comparison of all vaccine candidates in term of anti-HSV-2 gE and anti-HSV-2 gI-specific IgG antibody responses and CD4+ T cell responses were performed 14 days post third immunization.

Finally, the exploratory objectives were to evaluate, in different groups of mice immunized with HSV-2 gE/gI mutants, the anti-HSV-2 gE and the anti-HSV-2 gI-specific antibody responses after one and two immunizations, the anti-HSV-1 gE/gI cross-reactive antibody response after the three immunizations, the functions of vaccine-specific and HSV-1 gE/gI cross reactive polyclonal antibodies after three immunizations and the levels of anti-HSV-2 gE- and anti-HSV-2 gI-specific CD8+T cell responses after the third immunization.

***Introduction***

Herpesviruses have evolved diverse immune evasion strategies to survive in their natural hosts. Human Simplex Viruses (HSVs) encode namely the glycoprotein E (gE), that targets the humoral immune system. gE can form a noncovalent heterodimer complex with glycoprotein I (gI) that functions as an immunoglobulin G (IgG) Fc receptor (Fc$\gamma$R). Interactions between gE and gI increase human Fc binding affinity by about 100 times. Through antibody binding, the Fc$\gamma$R inhibits IgG Fc-mediated activities, including complement binding and antibody-dependent cellular cytotoxicity.

Previous experiments performed in mice showed that human IgG could bind to the gE Fc binding domain of the unmutated recombinant HSV-2 gE/gI protein and mask protective epitopes implicated in HSV-2 gE-mediated immune evasion mechanism. These results suggested that a similar situation could happen in clinic and negatively impact the HSV-2 vaccine therapeutic efficacy. To avoid negative immunological interference in humans, point mutations have been performed within the Fc binding domain of HSV-2 gE/gI protein to reduce the affinity to hIgG Fc domain and the HSV-2 gE-P317R/gI mutant construct was generated. Further constructs containing mutations in the gE Fc binding domain have been generated and their immunogenicity assessed. In particular, the immunogenicity in mice of five different HSV-2 gE/gI mutant proteins formulated in AS01 has been assessed.

***Study Design***

Female CB6F1 inbred mice aged 6-8 weeks old from Harlan laboratory (OlaHsd) were randomly assigned to the study groups (n=6 gr1-5 & n=4/gr6) and kept at the institutional animal facility under specified pathogen-free conditions. CB6F1 mice (gr1-5) were intramuscularly (i.m) immunized at days 0, 14 & 28 with 5$\mu$g of different HSV-2 gE/gI mutant proteins formulated in AS01 (5$\mu$g). An additional group of mice was i.m injected with a saline solution (NaCl 150mM), following the same schedule of immunization, and used as negative control group (gr6).

Serum samples were collected at days 14, 28 & 42 post prime immunization (14PI, 14PII, 14PIII) to measure anti-HSV-2 gE- and anti-HSV-2 gI-specific and anti-HSV-1 gE/gI cross-reactive IgG antibody responses and to characterize the function of vaccine-specific and cross reactive polyclonal antibodies. Spleens were collected 14 days post third immunization (14PIII) to evaluate ex-vivo systemic CD4+/CD8+ T cell responses towards HSV-2 gE and gI antigens. Details for each group is described in the Table 3.

**Table 3 - summary of study design and formulation tested**

| Group | Number of animals | Treatment | | | | | Immunization schedule (Days) | Sample collection *(organs, blood volume)* and days |
|---|---|---|---|---|---|---|---|---|
| | | Vaccine name and dose | Lot numbers | Adjuvant dose *(if applicable)* | Adjuvant lot number | Volume and route of injection | | |
| 1 | 6 | HSV-2 gE/gI **V340W** (5µg) | BMP 1271 | AS01 | LIQ-2019/2014 | 50µL i.m | Days 0, 14 & 28 | Days 14 & 28: Serum - partial bleeding Day 42: Serum - final bleeding + Spleen |
| 2 | 6 | HSV-2 gE/gI **A248T** (5µg) | BMP 1272 | | | | | |
| 3 | 6 | HSV-2 gE/gI **A246W** (5µg) | BMP 1278 | | | | | |
| 4 | 6 | HSV-2 gE/gI **P318I** (5µg) | BMP 1279 | | | | | |
| 5 | 6 | HSV-2 gE/gI **A248T_ V340W** (5µg) | BMP 1273 | | | | | |
| 6 | 4 | NaCl150mM | N/A | N/A | | | | |

***Materials and methods***

The antigens used in this study were the ectodomain of the purified recombinant gE/gI mutant proteins. The material was produced by using ExpiCHO™ expression system and was used as a research lot only.

AS01 is a liposome based adjuvant system (AS) containing QS-21 (a triterpene glycoside purified from the bark of Quillaja saponaria) and MPL (3-D Monophosphoryl lipid A), with liposomes as vehicles for these immunoenhancers and a buffer including NaCl as isotonic agent (variant 3).

A single human dose of the AS01b Adjuvant System (0.5 mL) contains 50μg of QS-21 and 50μg of MPL. In this study, the volume injected in mice is 1/10$^{th}$ of a human dose corresponding to a 5μg QS-21 and 5μg MPL per dose.

*Animal model*

It is well accepted that small animal models are useful tools to study the immunogenicity profile of new vaccine candidates. In order to get a general overview of the capacity of our vaccine candidates to induce T cell immune responses, CB6F1 mice (hybrid of C57B1/6 and Balb/C mice) have been used in this study.

CB6F1 mice have been shown to generate potent CD4+/CD8+ T cell and humoral immune responses following vaccination with various types of immunogens, including adjuvanted proteins and viral vectors. The profile of the vaccine-induced immune responses generated in these mice compared to expected responses in humans may nevertheless be impacted by some differences pertaining to TLR expression, HLA background and antigen presentation. However, the capacity for inducing CD4+/CD8+ T immune responses has shown comparable trends between these mice and humans.

*Detection of total anti-HSV-2 gE & gI specific IgG antibodies by ELISA*

Quantification of the total anti-gE or gI specific IgG antibodies was performed using indirect ELISA. Recombinant gE (~51kDa) (BMP1049 or BMP1291) or gI protein (~46kDa) (BMP1052b or BMP 1292) from HSV-2 were used as coating antigens. These proteins were produced using the ExpiHEK293F™ expression system.

Polystyrene 96-well ELISA plate (Nunc F96 Maxisorp cat 439454) were coated with 100μL/well of antigen diluted at a concentration of 2 μg/mL (gE) and 1 μg/mL (gI) in carbonate/bicarbonate 50mM pH 9.5 buffer and incubated overnight at 4°C. After incubation, the coating solution was removed and the plates were blocked with 200μL/well of Difkomilk 10% diluted in PBS (blocking buffer) (ref 232100, Becton Dickinson, USA) for 1 h at 37°C. After incubation, the blocking solution was removed and a three-fold serial dilution (in PBS + 0.1% Tween20 + 1% BSA buffer, of each serum sample was performed and added to the coated plates and incubated for 1h at 37°C. The plates were then washed four times with PBS 0.1% Tween20 (washing buffer) and Horseradish Peroxydase conjugated AffiniPure Goat anti-mouse IgG (H+L) (ref 115-035-003, Jackson, USA) was used as secondary antibody. One hundred microliters per well of the secondary antibody diluted at a concentration of 1:500 in PBS + 0.1% Tween20 + 1% BSA buffer was added to each well and the plates were incubated for 45min at 37°C.

The plates were then washed four times with washing buffer and 2 times with deionised water and incubated for 15min at RT (room temperature) with 100 μL/well of a solution of 75% single-component TMB Peroxidase ELISA Substrate (ref 172-1072, Bio-Rad, USA) diluted in sodium Citrate 0.1M pH5.5 buffer. Enzymatic color development was stopped with 100 μL of 0,4N Sulfuric Acid 1M (H2SO4) per well and the plates were read at an absorbance of 450/620nm using the Versamax ELISA reader.

Optical densities (OD) were captured and analysed using the SoftMaxPro GxP v5.3 software. A standard curve was generated by applying a 4-parameter logistic regression fit to the reference standard results (reference standard anti-gE = 20180011 14PIII - Pool of mice 1.1 to 1.20 immunized with AS01/gE (5μg of each/dose); reference standard anti-gI = 20190021 14PII - Pool of mice 2.1 to 2.5 immunized with AS01/gI (5μg of each/dose). Antibody titer in the samples was calculated by interpolation of the standard curve. The antibody titer of the samples was obtained by averaging the values from dilutions that fell within the 20-80% dynamic range of the standard curve. Titers were expressed in EU/mL (ELISA Units per mL).

*Detection of total anti-HSV-1 gE/gI cross-reactive IgG antibodies by ELISA*

Quantification of the total HSV-1 gE/gI cross-reactive IgG antibodies was performed using indirect ELISA. Recombinant gE/gI heterodimer protein (BMP 1299) from HSV-1 were used as coating antigen. This protein was produced using the ExpiCHO™ expression system.

Polystyrene 96-well ELISA plate (Nunc F96 Maxisorp cat 439454) were coated with 100μL/well of recombinant HSV-1 gE/gI heterodimer protein diluted at a concentration of 2 μg/mL in carbonate/bicarbonate 50mM pH 9.5 buffer and incubated overnight at 4°C. After incubation, the coating solution was removed and the plates were blocked with 200μL/well of Difkomilk 10% diluted in PBS (blocking buffer) (ref 232100, Becton Dickinson, USA) for 1 h at 37°C. After incubation, the blocking solution was removed and a three-fold serial dilution (in PBS + 0.1% Tween20 + 1% BSA buffer) of each serum sample was performed and added to the coated plates and incubated for 1h at 37°C. The plates were then washed four times with PBS 0.1% Tween20 (washing buffer) and Horseradish Peroxidase conjugated AffiniPure Goat anti-mouse IgG (H+L) (ref 115-035-003, Jackson, USA) was used as secondary antibody. One hundred microliters per well of the secondary antibody diluted at a concentration of 1:500 in PBS + 0.1% Tween20 + 1% BSA buffer was added to each well and the plates were incubated for 45min at 37°C.

The plates were then washed four times with washing buffer and 2 times with deionised water and incubated for 15min at RT (room temperature) with 100 μL/well of a solution of 75% single-component TMB Peroxidase ELISA Substrate (ref 172-1072, Bio-Rad, USA) diluted in sodium Citrate 0.1M pH5.5 buffer. Enzymatic color development was stopped with 100 μL of 0,4N Sulfuric Acid 1M ($H_2SO_4$) per well and the plates were read at an absorbance of 450/620nm using the Versamax ELISA reader.

Optical densities (OD) were captured and analysed using the SoftMaxPro GxP v5.3 software. A standard curve was generated by applying a 4-parameter logistic regression fit to the reference standard results (reference standard = 20200023 14PIII - Pool of mice 1.1 to 1.20 immunized with AS01-HSV-1 gE/gI HEK (5μg of each/dose). Antibody titer in the samples was calculated by interpolation of the standard curve. The antibody titer of the samples was obtained by averaging the values from dilutions that fell within the 20-80% dynamic range of the standard curve. Titers were expressed in EU/mL (ELISA Units per mL).

*Anti-HSV-2 gE and gI-specific CD4+/CD8+ T cell responses measured by ICS assay*

The frequencies of anti-HSV-2 gE & anti-HSV-2 gI-specific CD4+ and CD8+ T-cells producing IL-2 and/or IFN-γ and/or TNF-α were evaluated in splenocytes collected 14 days post third immunization after ex-vivo stimulation with HSV-2 gE or gI peptides pools.

<u>Isolation of splenocytes</u>

Spleens were collected from individual mouse 14 days after third immunization and placed in RPMI 1640 medium supplemented with RPMI additives (Glutamine, Penicillin/streptomycin, Sodium Pyruvate, non-essential amino-acids & 2- mercaptoethanol) (= RPMI/additives). Cell suspensions were prepared from each spleen using a tissue grinder. The splenic cell suspensions were filtered (cell stainer100μm) and then the filter was rinsed with 40mL of cold PBS-EDTA 2mM. After centrifugation (335g, 10min at 4°C), cells were resuspended in 7mL of cold PBS-EDTA 2mM. A second washing step was performed as previously described and the cells were finally resuspended in 1mL of RPMI/additives supplemented with 5% FCS (Capricorn scientific, FBS-HI-12A). Cell suspensions were then diluted 20x (10μL) in PBS buffer (190μL) for cell counting (using MACSQuant Analyzer). After counting, cells were centrifuged (335g, 10min at RT) and resuspended at $10^7$ Cells/mL in RPMI/additives supplemented with 5% FCS.

<u>Cell preparation</u>

Fresh splenocytes were seeded in round bottom 96-well plates at $10^6$ cells/well (100μL). The cells were then stimulated for 6 hours (37°C, 5% CO2) with anti-CD28 (BD Biosciences, clone 37.51) and anti-CD49d antibodies (BD Biosciences, clone 9C10 (MFR4.B)) at 1μg/mL per well, containing 100μL of either:-

- 15 mers overlapping peptide pool covering the sequence of gE protein from HSV-2 (1μg/mL per peptide per well).
- 15 mers overlapping peptide pool covering the sequence of gI protein from HSV-2 (1μg/mL per peptide per well).
- 15 mers overlapping peptide pool covering the sequence of Human β-actin protein (1μg/mL per peptide per well) (irrelevant stimulation).
- RPMI/additives medium (as negative control of the assay).
- PMA - ionomycin solution (Sigma, P8139) at working concentrations of 0,25 μg/mL and 2,5 μg/mL respectively (as positive control of the assay).

After 2 hours of ex vivo stimulation, Brefeldin A (Golgi plug ref 555029, BD Bioscience) diluted 1/200 in RPMI/additives supplemented with 5% FCS was added for 4 additional hours to inhibit cytokine secretion. Plates were then transferred at 4°C for overnight incubation.

Intracellular Cytokine Staining

After overnight incubation at 4°C, cells were transferred to V-bottom 96-well plates, centrifuged (189g, 5min at 4°C) and washed in 250μL of cold PBS +1% FCS (Flow buffer). After a second centrifugation (189g, 5min at 4°C), cells were resuspended to block unspecific antibody binding (10 min at 4°C) in 50μL of Flow buffer containing anti-CD16/32 antibodies (BD Biosciences, clone 2.4G2) diluted 1/50. Then, 50 μL Flow Buffer containing mouse anti-CD4-V450 antibodies (BD Biosciences, clone RM4-5, diluted at 1/100), anti-CD8-PerCp-Cy5.5 antibodies (BD Biosciences, clone 53-6.7, diluted at 1/50) and Live/DeadTM Fixable Yellow dead cell stain (Molecular probes, L34959, diluted at 1/500) was added for 30min in obscurity at 4°C. After incubation, 100μL of Flow buffer was added into each well and cells were then centrifuged (189g for 5 min at 4°C). A second washing step was performed with 200μL of Flow buffer and after centrifugation, cells were fixed and permeabilized by adding 200μL of Cytofix-Cytoperm solution (BD Biosciences, 554722) for 20min at 4°C in the obscurity. After plates centrifugation (500g for 5 min at 4°C), cells were washed with 200μL of Perm/Wash buffer (BD Biosciences, 554723), centrifuged (500g for 5 min 4°C) and resuspended in 50μE of Perm/Wash buffer containing mouse anti-IL2-FITC (BD Biosciences, clone JES6-5H4, diluted 1/400), anti-IFN-γ-APC (BD Biosciences, clone XMG1.2, diluted 1/200) and anti-TNF-α-PE (BD Biosciences, clone MP6-XT22, diluted 1/700) antibodies, for 1 hour at 4°C in the obscurity. After incubation, 100μL of Flow buffer was added into each well and cells were then finally washed with 200μL of Perm/Wash buffer (centrifugation 500g for 5 min at 4°C) and resuspended in 220μL PBS.

Cell acquisition and analysis

Stained cells were analyzed by flow cytometry using a LSRII flow cytometer and the FlowJo software. Live cells were identified with the Live/Dead staining and then lymphocytes were isolated based on Forward/Side Scatter lights (FSC/SSC) gating. The acquisition was performed on ~ 20.000 CD4+/CD8+ T-cell events. The percentages of IFN-γ+/- IL-2+/-and TNF-α+/- producing cells were calculated on CD4+ and CD8+ T cell populations. For each sample, unspecific signal detected after medium stimulation was removed from the specific signal detected after peptide pool stimulation.

*Evaluation of the ability of polyclonal sera to bind and activate mouse FcγRIII after incubation with HSV-2 or HSV-1 gE/gI transfected cells (ADCC bioassay - Promega)*

The mouse FcγRIII Antibody Dependent Cell Cytotoxicity (ADCC) Reporter Bioassay (Cat.# M1201), developed by Promega laboratory, is a bioluminescent cell-based assay which can be used to measure the ability of antibodies to specifically bind and activate the mouse FcγRIII expressed by modified Jurkat reporter cells.

Briefly, 3T3 cells, initially purchased from ATCC laboratories (clone A31, ATCC ref CCL-163), were grown in DMEM + 10% FBS decomplemented +1% L-glutamine 2mM + 1% Penicillin/streptomycin media and seeded at 2x$10^6$ cells in T175 flasks on Day 0 of experiment to ensure cells were in optimal growth phase for the next day.

On day 1, 6-well plates were prepared by adding 2mL of growth media (DMEM (Prep Mil, Log377BA), 1% L-Glutamine 2mM (Prep Mil, Log010D), 10% of Ultra low IgG FBS (Gibco, A33819-01)) to each well (one well per electroporation). Plates were kept warm in a 37°C incubator (5% CO2). The electroporator (Gene Pulser, BIO-RAD) was prepared to deliver 325V, 350 μF capacitance, infinite resistance, 1 pulse for a 4mm cuvette. 3T3 cells in growth phase were harvested into growth media and counted using a cell counter (TC20, BIO-RAD). For each electroporation, 5x$10^5$ 3T3 cells (500μL of cells at 1x$10^6$ cells/mL) and 20μg of HSV-2 gE/gI or HSV-1 gE/gI plasmid DNA (20μL at 1μg/μL) was used. For negative control, 10μL of water was used. Cells and HSV-2 or HSV-1 gE/gI plasmid DNA mixture were transferred to 4mm cuvette (Gene Pulser Electroporation Cuvettes, BIO-RAD) and immediately subjected to one pulse of electroporation using the parameters described above. After electroporation, all cuvettes were pooled to homogenise cell suspension and 500μL of cell suspension/well were transferred into 6-well plates in 2mL of pre-warmed media. Before incubation, the 6-well plates were slid back and forth and side-to-side several times to distribute cells evenly and then incubated at 37°C, 5% $CO_2$ during 48h.

After 48h incubation, HSV-2 or HSV-1 gE/gI transfected 3T3 cells (target cells (T)) were collected and pooled from the different 6-well plates. Cell suspension was centrifuged (10min, 340g, at RT) and resuspended in Promega assay buffer (96% RPMI (G7080) + 4% of low IgG serum (G7110); Promega) for cell counting (TC20, BIO-RAD). Then a solution at 96.000 3T3 cells/mL was prepared in Promega assay buffer and 25μL of this suspension (24.000cells/25μL/well) was added in 96-well plates. In a round-bottom 96-well plates (Nunc, ref 168136), a 3-fold serial dilution of each mouse serum sample (starting dilution 1/500 for HSV-2 and 1/200 for HSV-1) in 200μL was performed in Promega assay buffer and 25μL of each dilution was transferred to the corresponding well containing already the HSV-2 or HSV-1 gE/gI-transfected 3T3 cells. Finally, 25μL of genetically engineered Jurkat cells expressing mouse FcyRIII (Effector Cells (E)) at a concentration of 240.0000 cells/mL (60.000 cells/25μL/well) were added in each well

(~E/T 2,5/1) and plates were incubated for 6h at 37°C - 5% $CO_2$.

After incubation, plates were put at RT for 15min and 75μL of Bio-Glow reagent were added in each well. The plates were finally incubated for 20min at RT and read using luminometer (BioTek Synergy HI).

*Competitive ELISA to evaluate the ability of vaccine-specific antibodies to decrease human IgG Fc binding by HSV-2 gE/gI protein*

The ability of polyclonal sera collected in different groups of mice to decrease in-vitro hIgG antibodies binding by recombinant HSV-2 gE/gI protein was investigated by competitive ELISA. Recombinant HSV-2 gE/gI protein (BMP1063), produced using the ExpiHEK293F™ expression system was used as coating antigen.

Polystyrene 96-well ELISA plate (Nunc F96 Maxisorp cat 439454) were coated with 50μL/well of HSV-2 gE/gI protein diluted at a concentration of 4μg/mL in free Calcium/Magnesium PBS buffer and incubated overnight at 4°C. After incubation, the coating solution was removed and the plates were blocked with 100μL/well of PBS supplemented with 0,1% Tween-20 + 1% BSA (blocking buffer) for 1 h at 37°C.

In a 96-well Clear V-Bottom Polypropylene microplate (Falcon, ref 353263) a two-fold serial dilution (starting dilution 1/10) in blocking buffer for each individual serum was prepared in 60μl/well and mixed with 60μl/well of biotinylated-hIgG antibodies (Invitrogen, ref 12000C) pre-diluted at 0,7μg/mL in blocking buffer.

Then, after 1h of incubation with blocking buffer, the blocking solution was removed from the coated plates and 100μL of the mixture containing both hIgG and mice sera was transferred in the corresponding HSV-2 gE/gI coated wells and incubated 24h at 37°C. Positive control of the assay was a pool of anti-HSV-2 gbE/gI serum samples from previous studies. Negative control of the assay was a pool of irrelevant HPV serum samples diluted 1/1000 and mixed with hIgG too.

After 24h of incubation, the plates were washed four times with PBS 0.1% + Tween20 (washing buffer) and 50μL/well of Streptavidin-horsedish Peroxydase AMDEX (Amersham, ref RPN4401V) diluted 2000x were added on the wells and plates were incubated for 30min at 37°C. Plates were then washed four times with washing buffer and 50μL/well of a solution containing 75% single-component TMB Peroxidase ELISA Substrate (ref 172-1072, Bio-Rad, USA) diluted in sodium Citrate 0.1M pH5.5 buffer were added for 10min at room temperature. Enzymatic color development was stopped with 50μL/well of 0,4N Sulfuric Acid 1M ($H_2SO_4$) and the plates were read at an absorbance of 450/620nm using the Versamax ELISA reader. Optical densities (OD) were captured and fitted in curve in excel program.

Titers were expressed as the effective dilution at which 50% (i.e. $ED_{50}$) of the signal was achieved by sample dilution.

For each plate and using a reference sample (i.e. irrelevant serum), the reference $ED_{50}$ value was estimated using the following formula:

$$ED_{50} = \mathrm{OD}_{0\%} + 0.5 * (OD_{100\%} - OD_{0\%})$$

where $OD_{100\%}$ is the highest OD obtained with similar samples and $OD_{0\%}$ is the lowest achievable signal. For each plate, the former was obtained by averaging (mean) 6 replicates while the latter was set at zero.

Samples $ED_{50}$ titers were computed by way of linear interpolation between the left and right measurements closest to the $ED_{50}$ estimate within the plate. The approximation was obtained, on the untransformed OD and the logarithm base 10 transformed dilutions, with the *approx* function of the *stats* R base package.

Sample were not assigned a titer in the following cases:

◦ no measurement was available above or below the $ED_{50}$,
◦ curve crossed at least twice the $ED_{50}$ and
◦ one of the dilution steps (left or right) closest to the $ED_{50}$ was missing

*Competitive ELISA to evaluate the ability of HSV-1 cross-reactive antibodies to decrease human IgG Fc binding by HSV-1 gE/gI protein*

The ability of polyclonal sera collected in different groups of mice to decrease in-vitro hIgG antibodies binding by recombinant HSV-1 gE/gI protein was investigated by competitive ELISA. Recombinant HSV-1 gE/gI heterodimer protein (BMP 1299), produced using the ExpiCHO™ expression system was used as coating antigen.

Polystyrene 96-well ELISA plate (Nunc F96 Maxisorp cat 439454) were coated with 50μL/well of HSV-1 gE/gI protein diluted at a concentration of 2μg/mL in free Calcium/Magnesium PBS buffer and incubated overnight at 4°C. After incubation, the coating solution was removed and the plates were blocked with 100μL/well of PBS supplemented with 0,1% Tween-20 + 1% BSA (blocking buffer) for 1 h at 37°C.

In another 96-well Clear V-Bottom Polypropylene microplate (Falcon, ref 353263) a two-fold serial dilution (starting dilution 1/10) in blocking buffer for each serum were prepared in 60μl/well and mixed with 60μl/well of biotinylated-hIgG antibodies (Invitrogen, ref 12000C) pre-diluted at 0,7μg/mL in blocking buffer.

Then, after 1h of incubation with blocking buffer, the blocking solution was removed from the coated plates and 100μL of the mixture containing both hIgG and mice sera was transferred in the corresponding wells and incubated 24h at 37°C. Positive control of the assay was a pool of anti-HSV-1 gE/gI serum samples. Negative control of the assay was a pool of irrelevant HPV serum samples diluted 1/1000 and mix with same concentration hIgG than the one used with the samples.

After 1h of incubation, the plates were washed four times with PBS 0.1% Tween20 (washing buffer) and 50μL/well of Steptavidin-horsedish Peroxydase AMDEX (Amersham, ref RPN4401V) diluted 2000x were added on the wells and plates were incubated for 30min at 37°C. Plates were then washed four times with washing buffer and 50μL/well of a solution containing 75% single-component TMB Peroxidase ELISA Substrate (ref 172-1072, Bio-Rad, USA) diluted in sodium Citrate 0.1M pH5.5 buffer were added for 10min at room temperature. Enzymatic color development was stopped with 50μL/well of 0,4N Sulfuric Acid 1M ($H_2SO_4$) and the plates were read at an absorbance of 450/620nm using the Versamax ELISA reader. Optical densities (OD) were captured and fitted in curve in excel program.

Titers were expressed as the effective dilution at which 50% (i.e. $ED_{50}$) of the signal was achieved by sample dilution.

For each plate and using a reference sample (i.e. irrelevant serum), the reference $ED_{50}$ value was estimated using the following formula:

$$ED_{50} = OD_{0\%} + 0.5 * (OD_{100\%} - OD_{0\%})$$

where $OD_{100\%}$ is the highest OD obtained with similar samples and $OD_{0\%}$ is the lowest achievable signal. For each plate, the former was obtained by averaging (mean) 6 replicates while the latter was set at zero.

Samples $ED_{50}$ titers were computed by way of linear interpolation between the left and right measurements closest to the $ED_{50}$ estimate within the plate. The approximation was obtained, on the untransformed OD and the logarithm base 10 transformed dilutions, with the *approx* function of the *stats* R base package.

Sample were not assigned a titer in the following cases:

- no measurement was available above or below the $ED_{50}$,
- curve crossed at least twice the $ED_{50}$ and
- one of the dilution step (left or right) closest to the $ED_{50}$ was missing

*In-vitro HSV-2 MS neutralization assay*

An in-vitro neutralization assay was developed to detect and quantify HSV-2 MS neutralizing antibody titers in serum samples from different animal species. Sera (50μL/well at starting dilution 1/10) were diluted by performing a 2-fold serial dilution in HSV medium (DMEM supplemented with 1% Neomycin and 1% gentamycin) in flat-bottom 96-well plates (Nunclon Delta Surface, Nunc, Denmark, ref 167008). Sera were then incubated for 2h at 37°C (5% $CO_2$) with 400 TCID50/50μL/well of HSV-2 MS strain (ref ATCC VR-540) pre-diluted in HSV medium supplemented with 2% of guinea pig serum complement (Harlan, ref C-0006E). Edges of the plates were not used and one column of each plate was left without virus & sera (TC) or with virus but w/o serum (TV) and used as the negative or positive control of infection respectively. Positive control sera of the assay are pooled serum samples from mice immunized with different doses (0.22; 0.66; 2; 6μg/dose) of HSV-2 gD/AS01(2.5μg) and collected at 14 days post second (14PII) or third (14PIII) immunization. After the incubation of antibody-virus mixture, 10.000 Vero cells/100μL were added to each well of each plate and plates were incubated for 4 days at 37°C under 5% $CO_2$. Four days post-infection, supernatant was removed from the plates and cells were incubated for 5h at 37°C (5% $CO_2$) with a WST-1 solution (reagent for measuring cell viability, Roche, ref 11644807001) diluted 15x in HSV revelation medium (DMEM supplemented with 1% Neomycin and 1% gentamycin + 2% FBS).

To calculate neutralizing antibody titers, sets of data were normalized based on the mean of WST-1 optical density (O.D.) in "cells w/o virus" wells and "cells w/o serum" wells to 0 and 100% cytopathic effect (CPE) respectively. Percentage of inhibition of CPE at a dilution *i* was then given by:

$$\%\,inhibition = \left(O.D._{i} - Mean\,O.D._{cells\,w/o\,serum}\right) / \left(Mean\,O.D._{cells\,w/o\,virus} - Mean\,O.D._{cells\,w/o\,serum}\right)$$

The reciprocal of the dilution giving a 50% reduction of CPE was then extrapolated using nonlinear regression with the Softmaxpro Software.

*In-vitro HSV-1 neutralization assay*

An in-vitro neutralization assay was developed to detect and quantify HSV-1 cross-reactive neutralizing antibody titers in serum samples from different animal species. A 2-fold serial dilution of each serum (50μL/well at starting dilution 1/10) in HSV medium (DMEM supplemented with 1% Neomycin and 1% gentamycin) were performed in flat-bottom 96-well plates (Nunclon Delta Surface, Nunc, Denmark, ref 167008). Sera were then incubated for 2h at 37°C (5% $CO_2$) with 400 TCID50/50μL/well of HSV-1 strain (ref ATCC VR-1789) pre-diluted in HSV medium supplemented with 2% of guinea pig serum complement (Harlan, ref C-0006E). Edges of the plates were not used and one column of each plate was left without virus & sera (TC) or with virus but w/o serum (TV) and used as the negative or positive control of infection respectively. Positive control sera of the assay are pooled serum samples from mice immunized with different doses (0.22; 0.66; 2; 6μg/dose) of HSV-2 gD/AS01(2.5μg) and collected at 14 days post second (14PII) or third (14PIII) immunization. After the incubation of antibody-virus mixture, 10.000 Vero cells/100μL were added to each well of each plate and plates were incubated for 4 days at 37°C under 5% CO2. Four days post-infection, supernatant was removed from the plates and cells were incubated for 5h at 37°C (5% $CO_2$) with a WST-1 solution (reagent for measuring cell viability, Roche, ref 11644807001) diluted 15x in HSV revelation medium (DMEM supplemented with 1% Neomycin and 1% gentamycin + 25% FBS).

To calculate neutralizing antibody titers, sets of data were normalized based on the mean of WST-1 optical density (O.D.) in "cells w/o virus" wells and "cells w/o serum" wells to 0 and 100% cytopathic effect (CPE) respectively. Percentage of inhibition of CPE at a dilution i was then given by:

$$\%\,inhibition = \left(O.D._i - Mean\ O.D._{cells\ w/o\ serum}\right)/\left(Mean\ O.D._{cells\ w/o\ virus} - Mean\ O.D._{cells\ w/o\ serum}\right)$$

The reciprocal of the dilution giving a 50% reduction of CPE was then extrapolated using nonlinear regression with the Softmaxpro Software.

*Statistical methods*

The distribution of each response was assumed to be lognormal.

For each vaccine-specific IgG antibody response (gE or gI), a two-way analysis of variance (ANOVA) model was fitted on log 10 titers by including groups (all except the NaCl one), time points (Day14 (14PI), Day28 (14PII) and Day42 (14PIII)) and their interactions as fixed effects.

The NaCl group was not included as (almost) no response and variability was observed. Variance-covariance model selection was based on AICC criterion and individual data plot examination.

gE-specific variance-covariance for time points was modelled via an Heterogenous Compound Symmetry:

$$\begin{bmatrix} \sigma_1^2 + \sigma_4 & \sigma_4 & \sigma_4 \\ \sigma_4 & \sigma_2^2 + \sigma_4 & \sigma_4 \\ \sigma_4 & \sigma_4 & \sigma_3^2 + \sigma_4 \end{bmatrix}$$

**CSH- Heterogeneous CS**

The compound symmetry considers same correlation between timepoints, heterogenous refers to the fact that different variances were assumed for each timepoint. A different variance-covariance matrix was modelled for each vaccine group, indicating different variances and different timepoint correlations between groups.

gI-specific variance-covariance for time points was modelled via an Heterogenous First Order Autoregressive ARH (1) structure:

$$\begin{bmatrix} \sigma_1^2 & \sigma_1\sigma_2\rho & \sigma_1\sigma_3\rho^2 \\ \sigma_1\sigma_2\rho & \sigma_2^2 & \sigma_1\sigma_2\rho \\ \sigma_1\sigma_3\rho^2 & \sigma_2\sigma_3\rho & \sigma_3^2 \end{bmatrix}$$

**ARH(1)-Heterogeneous AR(1)**

The autoregressive structure considers correlations to be highest for adjacent times, and a systematically decreasing correlation with increasing distance between time points. Heterogenous refers to the fact that different variances were assumed for each timepoint. The same variance-covariance matrix was modelled for each vaccine group, indicating same variance between groups.

Geometric means and their 95% CIs are derived from these models.

Despite the fact that the NaCl group was not included in the ANOVA models, the comparisons associated to the primary objective were computed as follows: geometric means and 95% CI of vaccinated groups derived from the above models were divided by titer given to all the NaCl recipients for gE, or the geometric mean titer of NaCl group for gI, at the last timepoint. The resulting ratios should be understood as geometric mean ratios with their corresponding 95% CI.

For head to head comparison of vaccinated groups (at the last time point) and time point comparisons (PII/PI, PIII/PII, and PIII/PI) within each group, all the geometric mean ratios and their 95% CIs were derived from the models.

For HSV-1 gE/gI cross-reactive IgG antibody response, a one-way analysis of variance (ANOVA) model was fitted on log 10 titers by including groups (all except the NaCl one) as fixed effect. The NaCl group was not included as no response and variability was observed. No clear heterogeneity of variance was detected and therefore identical variances were assumed for the different groups. Geometric means and their 95% CIs are derived from these models. For comparison of vaccinated to NaCl groups, geometric means and 95% CI of vaccinated groups derived from the above model were divided by titer given to all the NaCl recipients. The resulting ratios should be understood as geometric mean ratios with their corresponding 95% CI. For head to head comparison of vaccinated groups, all the geometric mean ratios and their 95% CIs were derived from the model.

For % of gE or gI-specific CD4+ or CD8+ T-cell responses, a one-way analysis of variance (ANOVA) model was fitted on log 10 frequencies by including groups (all groups including the NaCl group) as fixed effect. No clear heterogeneity of variance was detected and therefore identical variances were assumed for the different groups. For both % of CD4+ and CD8+ T cell responses, the NaCl threshold was based on P95 of data across stimulation in NaCl negative control group. No modelling was performed on gI-specific CD8+T cells since response was below the P95 NaCl threshold for all vaccine groups. Geometric means and geometric mean ratios (with their corresponding 95% Cis) were derived from these models.

For the evaluation of the dissociation of the human IgG Fc binding by the pAbs, ED50 response was calculated for each sample. On this response, a one-way analysis of variance (ANOVA) model was fitted on log 10 values by including groups (all groups excluding the NaCl group) as fixed effect. Different variances for each group were modeled. Geometric means and geometric mean ratios (their corresponding 95% Cis) were derived from these models.

For HSV-2 MS-specific neutralizing antibody titers, a one-way analysis of variance (ANOVA) model was fitted on log 10 values by including groups (all groups excluding the NaCl group) as fixed effect. Different variances for each group were modeled. Geometric means and geometric mean ratios (their corresponding 95% Cis) were derived from these models. For HSV-1 cross-reactive neutralizing antibody titers, the same method was used except that identical variances were assumed for the different groups (no clear heterogeneity of variance was detected).

**Results**

***Anti-HSV-2 gE & gI-specific and anti-HSV-1 gE/gI cross-reactive IgG antibodies were induced with different mutated versions of HSV-2 gE/gI protein***

The anti-HSV-2 gE & gI-vaccine-specific and anti-HSV-1 gE/gI cross-reactive IgG antibody responses were investigated by ELISA.

Compared to NaCl control group, high anti-HSV-2 gE or gI- vaccine-specific IgG antibody responses were induced by all mutated versions of AS01-adjuvanted HSV-2 gE/gI protein 14 days post third immunization (all GMRs > 34.000 for gE and all GMRs > 6000 for gI) **(Figure A, Figure B, Figure A, Figure B).** As expected, no anti-HSV-2 gE or gI response was observed in NaCl control group (<30 EU/ml for all mice except for one with a value of 135EU/mL in HSV-2 gI response).

In all groups of mice immunized with AS01-adjuvanted HSV-2 gE/gI mutant proteins, levels of anti-HSV-2 gE and gI-specific IgG antibody responses increased after the second immunization (day28 (14PII)) compared to the first one (day14 (14PI)), with a fold increase ranging from 5 to 63 **(Figure A, Figure 3C).**

For all HSV-2 gE/gI mutant proteins, a booster effect on anti-HSV-2 gE-specific antibody response was also observed after third immunization (day42(14PIII)) compared to the second one (day28 (14PII)), with a fold increase ranging from 2 to 4 (with CI not containing 1) **(Figure B).**

A boosted effect on anti-HSV-2 gI-specific antibody response after third immunization compared to the second one was only detected in the groups of mice immunized with AS01-adjuvanted HSV-2 gE/gI A246W (group 3) and P318I (group 4) mutant proteins. Note that in A248T (group 3), one animal had a very low anti-HSV-2 gI-response compared to the others after two immunisations but was very similar to the others after the three immunizations. This might have amplified the booster effect observed in that group. No booster effect was observed in the three other groups (<2-fold change and CI containing 1) **(Figure 3D).**

Overall, these results suggest similar anti-HSV-2 gE- or anti-HSV-2 gI-specific antibody responses between the different mutated versions of HSV-2 gE/gI protein at 14days post third immunization. Only P318I mutant (group4) seemed to induce higher anti-HSV-2 gE- and anti-HSV-2 gI-specific antibody responses compared to V340W mutant (group1) with a fold increase close to 2 (GMRs of 2.09 and 2.39, respectively with CIs not containing 1) **(Figure A & Figure B).** P318I mutant (group4) seemed also to induce higher anti-HSV-2 gI-specific antibody response compared to A246W mutant (group3) with a fold increase of 2.42 (with CI not containing 1) **(Figure B).** No other differences between mutants were observed (with GMRs <2-fold change and/or CIs containing 1).

Anti-HSV-1 gE/gI cross-reactive response was not observed in NaCl control group (<20 EU/mL for all mice). At 14 days post third immunization and compared to NaCl control group, high anti-HSV-1 gE/gI cross-reactive IgG antibody response was induced in all groups of mice immunized by different mutated versions of AS01-adjuvanted HSV-2 gE/gI protein (all GMRs > 20.000) **(Figure 5, Figure 6).**

Several differences between HSV-2 gE/gI mutant candidates seemed to be observed at 14 days post third immunization. Indeed, cross-reactive response induced by both P318I (group 4) and A248T_V340W (group 5) mutants seemed to be about three times higher than the one induced by V340W (group 1) and A248T (group 3) mutants (GMRs of 3.1 with CIs not containing 1 for groups 4 or 5 versus 1, and 2.7 with lower limits of CI equal to 1 for groups 4 or 5 versus 3). No other differences between mutants seemed to be observed (with GMRs <2-fold change and/or CIs containing 1) **(Figure 7).**

*All recombinant HSV-2 gE/gI mutant proteins can induce anti-HSV-2 functional gE/gI specific and anti-HSV-1 gE/gI cross-reactive antibodies*

In this study, anti-HSV-2-specific & anti-HSV-1 cross-reactive antibody functions were only investigated in the sera collected at 14 days post third immunization. In this regard, the ability of pAbs to neutralize HSV-2 MS or HSV-1 VR1789 viruses, to bind and activate mouse FcγRIII expressed by Jurkat reporter cell line after binding of HSV-2 or HSV-1 gE/gI transfected cells and to decrease *in-vitro* binding of human IgG Fc by HSV-2 or HSV-1 gE/gI proteins was investigated.

Very low but consistent neutralizing (nAb) antibody responses directed to HSV-2 MS and HSV-1 VR1789 strains were detected in all groups of mice immunized with different mutated versions of AS01-adjuvanted HSV-2 gE/gI protein. In term of anti-HSV-2 specific nAb response, no clear evidence of difference between the mutated versions of HSV-2 gE/gI protein was observed (GMRs ≤2-fold change with all CIs containing 1). Comparisons between P318I (group 4) versus V340W (group 1) and A246W (group3) mutants indicate a 2-fold change that just fail to reach significance (lower limits of CI just below 1) **(Figure A; Figure B).** On the side of the cross-reactive nAb response directed against HSV-1 VR1789 strain, results also suggest similar response between the different mutated versions of HSV-2 gE/gI protein. Only P318I mutant (group4) seemed to induce higher response compared to A246W mutant (group3) with a fold increase close to 2 (GMR of 2.06 with CI not containing 1). Comparison between P318I (group 4) versus V340W (group 1) mutants indicates a 1.8-fold change that just fails to reach significance (lower limit of CI just below 1). No other differences between mutants were observed (GMRs <2-fold change and CIs containing 1)**(Figure A & Figure B).**

Then, the ability of sera from mice immunized with different HSV-2 gE/gI mutant candidates to compete and decrease hIgG binding by HSV-2 or HSV-1 gE/gI protein was assessed, *in-vitro,* by competitive ELISA. Different mutated versions of HSV-2 gE/gI protein elicited vaccine-specific and HSV-1 cross reactive polyclonal antibody response able to compete with hIgG binding by HSV-2 or HSV-1 gE/gI protein **(Figure and Figure 71).** The dissociation curve of hIgG Fc binding by HSV-2 or HSV-1 gE/gI protein was quite similar between all groups of mice. However, a trend for higher inhibitory response of hIgG Fc binding towards HSV-2 gE/gI protein was observed with polyclonal antibodies from mice immunized with A248T_V340W mutant (group5) compared to A248T mutant (group 2) with a fold increase of 1.96 (and CIs almost not including 1) **(Figure A; Figure B).**

Finally, the ability of a vaccine-specific or HSV-1 gE/gI cross reactive antibody response, induced 14 days post third immunization, to bind and activate *in-vitro* mouse FcyRIII was investigated on HSV-2 and HSV-1 gE/gI-transfected cells. Data shown on **Figure A-E and Figures 72A-E** suggested that all groups of mice immunized with the different mutated versions of AS01-HSV-2 gE/gI protein could induce HSV-2 gE/gI-specific and HSV-1 gE/gI cross reactive antibody responses able to specifically bind and activate Jurkat reporter cells expressing FcγRIII. As expected, activation of FcγRIII was not detected with sera from unvaccinated mice. No major difference was observed between the different groups of mice in terms of FcγRIII activation after incubation with HSV-2 or HSV-1 gE/gI transfected cells **(Figure F and Figure 72F).**

In conclusion, these data suggest that different mutated versions of AS01/HSV-2 gE/gI protein can induce functional vaccine-specific and HSV-1 gE/gI cross reactive antibodies able to bind and activate FcγRIII after incubation with HSV-2 or HSV-1 gE/gI transfected cells, to decrease human IgG Fc binding by HSV-2 or HSV-1 gE/gI protein and to neutralize at low intensity HSV-2 and HSV-1 viruses.

*All mutated versions of recombinant HSV-2 gE/gI protein can induce vaccine-specific CD4+ and CD8+ T cell responses*

Compared to the NaCl control group, higher anti-HSV-2 gE and anti-HSV-2 gI-specific CD4+T cell responses and anti-HSV-2 gE-specific CD8+T cell responses were detected 14 days after the third immunization in all groups of mice immunized with different mutated versions of AS01-adjuvanted HSV-2 gE/gI protein (>16-fold change between vaccine and NaCl groups with CIs not containing 1). No anti-HSV-2 gI-specific CD8+T cell response was detected in any of vaccinated groups compared to the NaCl control group **(Figure & Figure).**

In this study, similar anti-HSV-2 gE- or anti-HSV-2 gI-specific CD4+T cell responses were detected between the different HSV-2 gE/gI mutant candidates. Only A248T_V340W mutant (group5) seemed to induce higher anti-HSV-2 gE- and anti-HSV-2 gI-specific CD4+T cell responses compared to A248T mutant (group2) with a fold increase close to 2 (GMRs of 1.93 and 2.44, respectively with CIs not containing 1) **(Figure A & Figure B).**

Similarly, for anti-HSV-2 gE-specific CD8+T cell response, A248T_V340W mutant (group5) seemed to induce higher response compared to A248T mutant (group 2) with a fold increase of 2.66 (and CI does not include 1). In addition, response seemed to be higher with A248T_V340W (group5) compared to A246W (group3), and with P318I (group4) compared to A246W (grp3), with fold increases of 2.78 and 2.11, respectively (and CIs not including 1). There is a trend for higher response with V340W (group 1) compared to A248T (group2) or to A246W (group3), and with P318I (group4) compared to A248T (group2) with a fold increase of 2 (and CIs almost not including 1). No other differences between mutants were observed (with GMRs <2-fold change and/or CIs containing 1) **(Figure C).**

The overall results suggest that the vaccine-specific T cell responses is slightly higher in group of mice immunized with the A248T_V340W mutant compared to group of mice immunized with the A248T mutant.

EXAMPLE 2

**Objective**

The general objective of this experiment is to evaluate the immunogenicity in mice of six different Lipid nanoparticle (LNP)-formulated Self Amplifying mRNA (SAM) vectors expressing new mutated versions of HSV-2 gE/gI sequence.

The primary objective was to compare to NaCl control group, the gE-specific polyclonal antibody (pAb) response induced by several LNP-formulated SAM HSV-2 gE/gI mutants 21days post third immunization. At 21days post third immunization, the anti-HSV-2 gI-specific polyclonal antibody response and the anti-HSV-2 gE and anti-HSV-2 gI-specific CD4+T cell responses induced by several LNP-formulated SAM HSV-2 gE/gI mutants were compared to NaCl control group. In addition, head-to-head comparison of all vaccine candidates in term of anti-HSV-2 gE and anti-HSV-2 gI-specific IgG antibody responses and CD4+ T cell responses were performed 21 days post third immunization.

Finally, the exploratory objectives were to evaluate, in different LNP-formulated SAM HSV-2 gE/gI mutants, the anti-HSV-2 gE and anti-HSV-2 gI-specific antibody responses after one and two immunizations, the anti-HSV-1 gE/gI cross-reactive antibody responses after three immunizations, the functions of vaccine-specific or anti-HSV-1 gE/gI cross reactive polyclonal antibodies and the levels of anti-HSV-2 gE- and anti-HSV-2 gI-specific CD8+T cell responses. Finally, the ability of vaccine-specific T cells, induced after three immunizations, to cross-react with HSV-1 gE antigens were also investigated in this study.

**Study design**

Female CB6F1 inbred mice aged 6-8 weeks old from Harlan laboratory (OlaHsd) were randomly assigned to the study groups (n=6 gr1-6 & n=4/gr7) and kept at the institutional animal facility under specified pathogen-free conditions. CB6F1 mice (gr1-6) were intramuscularly (i.m) immunized at days 0, 21 & 42 with 0,8μg of different versions of SAM HSV-2 gE/gI mutants formulated in Lipid nanoparticles (LNP). An additional group of mice was i.m injected with a saline solution (NaCl 150mM) following the same schedule of immunization and used as negative control group (gr7). A total of 40 animals was used for the whole study.

Serum samples were collected at days 21, 42 & 63 post prime immunization (21PI, 21PII, 21PIII) to measure anti-HSV-2 gE- and anti-HSV-2 gI-specific and anti-HSV-1 gE/gI cross reactive IgG antibody responses and characterize the functions of vaccine-specific and HSV-1 cross reactive polyclonal antibodies. The spleens were collected at day 63 post prime immunization (21PIII) to evaluate ex-vivo systemic CD4+/CD8+ T cell responses towards HSV-2 gE and gI and HSV-1 gE antigens. Details for each group is described in the Table 4.

**Table 4 Summary of the study design and formulation tested**

| **Group** | **Number of animals** | **Treatment** | | | **Immunization schedule (Days)** | **Sample collection *(organs, blood volume)* and days** |
|---|---|---|---|---|---|---|
| | | **Vaccine name and dose** | **Lot numbers** | **Volume and route of injection** | | |
| 1 | 6 | LNP/SAM HSV-2 gE/gI **V340W** (0,8μg) | N71700-28-1088 | 50μL i.m | Days 0 & 28 | Days 21 & 42: Serum - partial bleeding Day 63: Serum - final bleeding + Spleen |
| 2 | 6 | LNP/SAM HSV-2 gE/gI **A248T** (0,8μg) | N71700-28-1089 | | | |
| 3 | 6 | LNP/SAM HSV-2 gE/gI **A246W** (0,8μg) | N71700-28-1095 | | | |
| 4 | 6 | LNP/SAM HSV-2 gE/gI **P318I** (0,8μg) | N71700-28-1096 | | | |
| 5 | 6 | LNP/SAM HSV-2 gE/gI **A248T_ V340W** (0,8μg) | N71700-28-1090 | | | |
| 6 | 6 | LNP/SAM HSV-2 gE/gI **insert ARAA** (0,8μg) | N71700-28-1055 | | | |
| 7 | 4 | NaCl150mM | N/A | | | |

**Materials and methods**

**Investigational products**

SAM HSV-2 gE/gI mutants were generated by In vitro Transcription (IVT) according to established protocols. Preparation of LNP with SAM followed established methods. In this method, SAM-LNP is prepared by rapidly mixing ethanolic solutions of lipids with aqueous buffers that contain SAM. The rapid mixing results in a supersaturation of hydrophobic components that have ionically paired with SAM. The SAM/lipid complexes condensation and precipitate through nucleation mediated precipitation, yielding small and narrowly disperse nanoparticles. Following this mixing step, the lipid nanoparticles mature to entrap the RNA and then are transferred to a final Tris/sucrose storage buffer through a buffer exchange step. The LNP solutions are then characterized for size, lipid content, RNA entrapment, final SAM concentration, and in vitro potency. The materials can be frozen at -80°C for over 6 months after the addition of 5% (w/v) sucrose.

*Total anti-HSV-2 gE & gI-specific IgG antibodies measured by ELISA*

As described for Example 1 above.

*Total anti-HSV-1 gE/gI cross reactive IgG antibodies measured by ELISA*

As described for Example 1 above.

*HSV-2 gE- and gI-specific and HSV-1 gE cross-reactive CD4+/CD8+T cell responses measured by ICS assay*

As described for Example 1 above, except that additionally, cross reactive CD4+ and CD8+ T cell responses towards HSV-1 gE antigen were analyzed after ex-vivo stimulation with HSV-1 gE peptides pool. For this, the Cell preparation section differs in that the cells were stimulated for 6 hours (37°C, 5% CO2) with anti-CD28 (BD Biosciences, clone 37.51) and anti-CD49d antibodies (BD Biosciences, clone 9C10 (MFR4.B)) at 1μg/mL per well, containing 100μL of either

- 15 mers overlapping peptides pool covering the sequence of gE protein from HSV-2 (1μg/mL per peptide per well).
- 15 mers overlapping peptides pool covering the sequence of gI protein from HSV-2 (1μg/mL per peptide per well).
- 15 mers overlapping peptides pool covering the sequence of gE protein from HSV-1 (1μg/mL per peptide per well).
- 15 mers overlapping peptides pool covering the sequence of Human β-actin protein (1μg/mL per peptide per well) (irrelevant stimulation).
- RPMI/additives medium (as negative control of the assay).
- PMA - ionomycin solution (Sigma, P8139) at working concentrations of 0,25 μg/mL and 2,5 μg/mL respectively (as positive control of the assay).

*Evaluation of the ability of polyclonal sera to bind and activate mouse FcγRIII after incubation with HSV-2 gE/gI transfected cells (ADCC bioassay - Promega)*

As described for Example 1.

*Competitive ELISA to evaluate the ability of vaccine specific antibodies to decrease human IgG Fc binding by HSV-2 gE/gI protein*

As described for Example 1.

*Competitive ELISA to evaluate the ability of HSV-1 cross-reactive antibodies to decrease human IgG Fc binding by HSV-1 gE/gI protein*

As described for Example 1.

*In-vitro HSV-2 neutralization assay*

As described for Example 1.

*In-vitro HSV-1 neutralization assay*

As described for Example 1.

*Statistical methods*

The distribution of each response was assumed to be lognormal.

For each vaccine-specific IgG antibody response (gE or gI), a two-way analysis of variance (ANOVA) model was fitted on $log_{10}$ titers by including groups (all except the NaCl one), time points (Day21 (21PI), Day42 (21PII) and Day63 (21PIII)) and their interactions as fixed effects. The NaCl group was not included as no response and variability was observed. Variance-covariance model selection was based on AICC criterion and individual data plot examination.

On each vaccine-specific response, the variance-covariance for time points was modelled via a Compound Symmetry matrix.

$$\begin{bmatrix} \sigma^2+\sigma_1 & \sigma_1 & \sigma_1 \\ \sigma_1 & \sigma^2+\sigma_1 & \sigma_1 \\ \sigma_1 & \sigma_1 & \sigma^2+\sigma_1 \end{bmatrix}$$

**CS- Compound Symmetry**

The compound symmetry considers same variance and same correlation between timepoints. The same variance-covariance matrix was modelled for each vaccine group, indicating same variance between groups.

Geometric means and their 95% CIs are derived from these models.

Despite the fact that the NaCl group was not included in the ANOVA models, the comparisons associated to the primary objective were computed as follows: geometric means and 95% CI of vaccinated groups derived from the above models were divided by titer given to all the NaCl recipients at the last timepoint. The resulting ratios should be understood as geometric mean ratios with their corresponding 95% CI.

For head to head comparison of vaccinated groups (at the last time point) and time point comparisons (PII/PI, PIII/PII, and PIII/PI) within each group, all the geometric mean ratios and their 95% CIs were derived from the models.

For gE/gI cross-reactive IgG antibody response, a one-way analysis of variance (ANOVA) model was fitted on $log_{10}$ titers by including groups (all except the NaCl one) as fixed effect. The NaCl group was not included as no response and variability was observed. No clear heterogeneity of variance was detected and therefore identical variances were assumed for the different groups. Geometric means and their 95% CIs are derived from this model. For comparison of vaccinated to NaCl groups, geometric means and 95% CI of vaccinated groups derived from the above model were divided by titer given to all the NaCl recipients. The resulting ratios should be understood as geometric mean ratios with their corresponding 95% CI. For head to head comparison of vaccinated groups, all the geometric mean ratios and their 95% CIs were derived from the model.

On each vaccine-specific or cross-reactive % of CD4+/ CD8+ T-cell responses (gE or gI), a one-way analysis of variance (ANOVA) model was fitted on log10 frequencies by including groups (all groups including the NaCl group) as fixed effect. No clear heterogeneity of variance was detected for % of HSV-2 gE-specific CD4+ T-cell response and therefore identical variances were assumed for the different groups. For other responses, different variances were modeled for the different groups. For both % of CD4+ and CD8+ T cell responses, the NaCl threshold was based on P95 of data across stimulation in NaCl negative control group. No modelling was performed on % of HSV-1 gE cross-reactive CD4+ T-cells and on % of HSV-2 gI-specific CD8+ T-cells since response were below the P95 NaCl threshold for all vaccine groups. Geometric means and geometric mean ratios (with their corresponding 95% CIs) were derived from these models.

For HSV-2 MS-specific neutralizing antibody titers, a one-way analysis of variance (ANOVA) model was fitted on $log_{10}$ values by including groups (all groups excluding the NaCl group) as fixed effect. No clear heterogeneity of variance was detected and therefore identical variances were assumed for the different groups. Geometric means and geometric mean ratios (their corresponding 95% CIs) were derived from this model. The same model was also performed on HSV-1 cross-reactive neutralizing antibody titers.

## Results

*All LNP/SAM HSV-2 gE/gI mutants induced vaccine-specific IgG antibody responses*

Note that one sample in LNP/SAM-HSV-2 gE/gI A248T_V340W group (sample 4.5) was not evaluated by ELISA 21days post second immunization due to not enough serum available.

The anti-HSV-2 gE & gI-vaccine-specific and anti-HSV-1 gE/gI cross-reactive IgG antibody responses were investigated by ELISA.

As expected, anti-HSV-2 gE-specific or anti-HSV-2 gI responses were not observed in NaCl control group (<20 EU/mL for all mice) while all the LNP-formulated SAM HSV-2 gE/gI vaccinated-mice produced a response above 30.000 EU/mL at the last time point **(Figure 16A & Figure 16B).** At 21 days post third immunization and compared to NaCl control group, high anti-HSV-2 gE or anti-HSV-2 gI- specific IgG antibody responses were induced in all groups of mice immunized with the different versions (mutated and amino acid insertion) of LNP-formulated SAM HSV-2 gE/gI vector (all GMRs > 17.000 for gE and all GMRs > 1.800 for gI) **(Figure 17A & Figure 17B)**.

In all groups of mice immunized with LNP-formulated SAM HSV-2 gE/gI mutants, levels of anti-HSV-2 gE and anti-HSV-2 gI-specific IgG antibody responses increased after the second immunization (day42 (21PII)) compared to the first one (day21 (21PI)), with a fold increase ranging from 2 to 8 **(Figure 18A & Figure 18C).**

For all HSV-2 gE/gI mutants except A248T mutant (group2), a booster effect on HSV-2 gE-specific antibody response was also observed 21 days after the third immunization (day 63(21PIII)) compared to the second one (day42 (21PII)), with a fold increase ranging from 2.46 to 4.49 (with CI not containing 1). For A248T mutant, a fold increase of 1.77 was observed (CI not containing 1) **(Figure 18B).**

Between the second and third vaccine dose, a clear booster effect on the levels of HSV-2 gI-specific antibody response was only detected for the groups of mice immunized with LNP-formulated SAM HSV-2 gE/gI V340W (group 1) and insert ARAA (group 6) mutants (2 and 2.3-fold increases with CIs not containing 1). A smaller booster effect was also observed with the other mutants with a 1.6-1.9-fold increase (CIs not containing 1) **(Figure 18D).**

In overall, these results suggest similar HSV-2 gE-specific antibody response between the different HSV-2 gE/gI mutant candidates at 21 days post third immunization (with GMRs close to 1-fold change and CIs containing 1) **(Figure 19A).** In contrast, for HSV-2 gI-specific antibody response, several differences between HSV-2 gE/gI mutant candidates seem to be observed at 21days post third immunization. Indeed, response induced by both A248T (group 2) and A248T_V340W (group 5) mutants seemed to be twice higher than the one induced by V340W (group 1), A246W (group 3) and P318I (group 4) mutants (GMRs of 2 with CIs either not containing 1 which indicates an effect, or CIs almost not containing 1 which indicates a trend for an effect). No other differences between mutants were observed (with GMRs <2-fold change and CIs containing 1) **(Figure 19B).**

As expected, anti-HSV-1 gE/gI cross-reactive response was not observed in NaCl control group (11 EU/mL for all mice). At 21 days post third immunization and compared to NaCl control group, high anti-HSV-1 gE/gI cross-reactive IgG antibody response was induced in all groups of mice immunized by different versions (mutated and amino acid insertion) of LNP-formulated SAM HSV-2 gE/gI vector (all GMRs > 11.000) **(Figure 20, Figure 21).** In overall, head to head comparison of vaccine-immunized groups of mice suggests similar anti-HSV-1 gE/gI cross-reactive IgG antibody response 21 days post third immunization (with most GMRs <2-fold change and CIs containing 1). Only A246W mutant (group 3) seemed to induce higher response compared to V340W mutant (group 1) with a fold increase of 2.28 (CI not containing 1) **(Figure 22).**

*Different LNP/SAM HSV-2 gE/gI mutants can induce functional vaccine-specific and HSV-1 gE/gI cross-reactive antibody response*

In this study, vaccine HSV-2 gE/gI-specific & HSV-1 gE/gI cross-reactive antibody function were only investigated in the sera collected at 21days post third immunization. In this regard, the ability of vaccine-induced pAbs to neutralize HSV-2 MS or HSV-1 VR1789 viruses, to bind and activate mFcγRIII expressed by Jurkat reporter cell line after incubation with HSV-2 gE/gI transfected cells and to decrease in-vitro binding of human IgG Fc by HSV-2 or HSV-1gE/gI protein was investigated.

Very low specific neutralizing (nAb) antibody responses directed to HSV-2 MS or HSV-1 VR1789 strains strain was were detected in all groups of mice immunized with different versions (mutated and amino acid insertion) of LNP-formulated SAM-HSV-2 gE/gI vector. Results suggest no clear difference in term of vaccine-specific or cross-reactive antibody neutralizing activity between the different mutants (GMRs ≤2-fold change with all CIs containing 1). A few vaccine comparisons (in particular, A248T_V340W (group 5) versus V340W (group 1)) showed a 2-fold change but failed to reach significance due to large variability **(Figure 23A, & Figure 23B).** On the side of the cross-reactive nAb response directed against HSV-1 VR1789 strain no difference was observed between the different candidates (see **Figure 24A & Figure 24B**).

Then, the ability of sera from mice immunized with different LNP-formulated SAM-HSV-2 gE/gI mutant candidates to compete and decrease hIgG binding by HSV-2 or HSV-1 gE/gI protein was assessed in-vitro by competitive ELISA. All LNP-SAM HSV-2 gE/gI mutants elicited vaccine-specific polyclonal antibody response able to compete in-vitro with hIgG Fc binding by HSV-2 or HSV-1 gE/gI proteins. **(Figure 25 & Figure 73)**. The dissociation curve of hIgG Fc binding by HSV-2 gE/gI protein was quite similar between all groups of mice and the calculation of the $ED_{50}$ shown similar response between group of mice **(Figure 26)**. In addition, the dissociation curve of hIgG Fc binding by HSV-1 gE/gI protein was also quite similar between all groups of mice suggesting no significant difference between the mutants **(Figure 73)**.

Finally, the ability of vaccine-specific antibody response, induced 14 days post third immunization, to bind and activate in-vitro mouse FcγRIII after incubation with HSV-2 gE/gI transfected cells was investigated. Data shown on the **Figure 27A-F** suggested that all groups of mice immunized with the different LNP-SAM HSV-2 gE/gI mutants could induce anti-HSV-2 gE/gI-specific antibody response able to specifically bind and activate Jurkat reporter cells expressing FcγRIII. As expected, activation of FcγRIII was not detected with sera from unvaccinated mice. No major difference was observed between the different group of mice in term of FcγRIII activation **(Figure 27G)**.

In conclusion, these data suggest that different LNP-SAM HSV-2 gE/gI mutants can induce vaccine-specific antibodies able to bind and activate FcγRIII after incubation with HSV-2 gE/gI transfected cells, to decrease human IgG Fc binding by HSV-2 or HSV-1 gE/gI protein and to neutralize at low intensity HSV-2 MS and HSV-1 VR1789 viruses.

*All LNP/SAM HSV-2 gE/gI mutants induced anti-HSV-2 gE-specific and anti-HSV-1 gE cross reactive CD8+ T cells and low level of anti-HSV-2 gI-specific CD4+T cells*

Twenty-one days after the third immunization with different LNP-SAM HSV-2 gE/gI mutants, low levels of anti-HSV-2 gE-specific CD4+T cell response (GMs ≤02%) was detected in all mice from group 3 (A246W), most of the mice from groups 1 (V340W), 2 (A248T) and 4 (P318I), half of the mice from group 6 (insert ARAA) and none of the mice from group 5 (A248T_V340W) when compared to the P95 threshold based on NaCl control group **(Figure 28A; Figure 29A).** Compared to the NaCl control group, higher anti-HSV-2 gI-specific CD4+T cell response was detected in all groups of mice immunized with different LNP-formulated SAM HSV-2 gE/gI mutants (about 0.4%, with all mice at or above the P95 threshold, and GMRs over NaCl ranging from 14 to 19 with CIs not containing 1) **(Figure 28A & Figure 29B)**. In contrast, no HSV-1 gE cross-reactive CD4+ T cell response was detected in any of the vaccinated groups compared to NaCl negative control group **(Figure 28A)**.

High levels of anti-HSV-2 gE-specific and anti-HSV-1gE cross-reactive CD8+T cell response were detected in all vaccinated groups compared to the NaCl control group (GMRs of around 100 with CIs not containing 1) **(Figure 28B & Figure 30).** Anti-HSV-2 gI-specific CD8+ T cell response was not detected in any of the vaccinated groups compared to NaCl negative control **(Figure 28B).**

Results suggest that the different mutated versions of LNP-formulated SAM-HSV-2 gE/gI vector were all comparable in terms of a CD4+ and CD8+T cell response (GMRs <2-fold change with most CIs totally included in [0.5,2] and/or containing 1) **(Figure 31 & Figure 32).** As there is no response detected in anti-HSV-2 gE-specific CD4+T cell response with A248T_V340W mutant (group 5), this group seems to differ from A246W (group3) and P318I mutants (group 4) on this response (fold decrease of 2 with CI not containing 1) **(Figure 31A).**

EXAMPLE 3 - *Immuno-evaluation of several AS01-adjuvanted HSV-1 gE/gI antigens (unmutated and mutated) in CB6F1 mice*

**Objectives**

The general objective of this study was to generate immunogenicity data from different HSV-1 gE/gI proteins containing single point mutation in gE Fc binding domain to drop the avidity to human Immunoglobulin G Fc.

A comparison was carried out between the anti- HSV- IgE/gI-specific polyclonal antibody (pAb) response induced by several AS01-adjuvanted HSV-1 gE/gI antigens (unmutated or mutated) 14days post third immunization compared to NaCl control group.

14 days post third immunization, the vaccine-specific polyclonal antibody (pAb) response and the anti-HSV-1 gE/gI-specific CD4+T cell responses induced by different mutants of HSV-1 gE/gI protein were compared to NaCl control group. In addition, head-to-head comparison of all vaccine candidates in term of anti-HSV-1 gE/gI-specific IgG antibody responses and anti-HSV-1 gE- and anti-HSV-1 gI-specific CD4+ T cell responses were performed 14 days post third immunization.

Finally, the exploratory objectives were to evaluate, in different groups of mice immunized with HSV-1 gE/gI mutants, the anti-HSV-1 gE/gI-specific antibody responses after one and two immunizations, the anti-HSV-2 gE and anti-HSV-2 gI cross-reactive antibody responses after three immunizations, the functions of vaccine-specific and HSV-2 gE/gI cross reactive polyclonal antibodies after three immunizations and the levels of anti-HSV-1 gE, anti-HSV-1 gI-specific CD8+T cell responses and anti-HSV-2 gE cross-reactive T cell responses after the third immunization.

**Study design**

Female CB6F1 mice (n=6gr1-6 & n=4/gr7) were intramuscularly (i.m) immunized at days 0, 14 & 28 with 5μg of unmutated HSV-1 gE/gI (gr1) or with different mutated versions of HSV-1 gE/gI protein (gr2-6) formulated in AS01 (5μg). An additional group of mice was i.m injected with a saline solution (NaCl 150mM), following the same schedule of immunization, and used as negative control group (gr7).

Serum samples were collected at days 13, 27 & 42 post prime immunization (13PI, 13PII, 14PIII) to measure both anti-HSV-1 gE/gI-specific and anti-HSV-2 gE and anti-HSV-2 gI cross reactive IgG antibody responses. The functions of vaccine-specific and HSV-2 cross reactive antibodies were also investigated in the serum samples collected 14 days after the third immunization. Spleens were collected 14 days post third immunization (14PIII) to evaluate, ex-vivo, systemic CD4+ and CD8+ T cell responses towards HSV-1 gE, HSV-1 gI & HSV-2 gE antigens.

**Background and Scientific Rationale**

The global prevalence of genital herpes is estimated at 417 million in individuals between the ages of 15 and 49, with a disproportionate burden of disease in Africa. Herpes Simplex Virus type 1 (HSV-1) is approximately as common as Herpes Simplex Virus type 2 (HSV-2) as the cause of first time genital herpes infections in resource-rich countries. Recurrent infections are less common after HSV-1 than HSV-2 genital infections; therefore, HSV-2 remains the predominant cause of recurrent genital herpes in resource-rich countries and the burden of HSV-2 infection is even greater in resource-limited countries. Some infected individuals have severe and frequent outbreaks of genital ulcers, while others have mild or subclinical infections, yet all risk transmitting genital herpes to their intimate partners. In infected individuals, antiviral therapy reduces the frequency of recurrent genital lesions and the risk of transmitting infection to partners. However, alternative approaches to reduce the frequency of clinical and subclinical genital recurrences by vaccination are highly demanding.

Therapeutic vaccine candidates are being developed to prevent first genital HSV-2 recurrences. Previous reports showed that vaccine compositions composed of adjuvanted recombinant HSV-2 glycoprotein D (gD) could reduce 50% of episodes of genital herpes reactivation in guinea pig model and in genital herpes patients (HSV-2 positive). Herpesviruses have evolved diverse immune evasion strategies to survive in their natural hosts. HSV encodes namely the glycoproteins E (gE), that target the humoral immune system. gE can form a noncovalent heterodimer complex with glycoprotein I (gI) that functions as an immunoglobulin G (IgG) Fc receptor (FcγR). Interactions between gE and gI increase human Fc binding affinity about 100 times. Through antibody binding, the FcγR inhibits IgG Fc-mediated activities, including complement binding and antibody-dependent cellular cytotoxicity.

In context of therapeutic HSV-2, previous experiments performed in mice showed that human IgG could bind to the gE Fc binding domain of the unmutated recombinant HSV-2 gE/gI protein and mask protective epitopes implicated in HSV-2 gE-mediated immune evasion mechanism. These results suggested that a similar situation could happen in clinical studies and negatively impact the HSV-2 vaccine therapeutic efficacy. To avoid negative immunological interference in human, point mutations have been performed within the Fc binding domain of HSV-2 gE/gI protein to reduce the affinity to hIgG Fc domain and the HSV-2 gE_P317R/gI mutant construct was selected as a vaccine candidate.

As this immune evasion mechanism is used by both HSV-1 and -2 viruses, it was decided to also generate immunogenicity data with different mutated versions of recombinant HSV-1 gE/gI protein (with a strong reduction of gE Fc binding activity).

The objective of this experiment was to evaluate the immunogenicity in mice of five different HSV-1 gE/gI mutants formulated in AS01.

**Study design**

Female CB6F1 inbred mice aged 6-8 weeks old from Harlan laboratory (OlaHsd) were randomly assigned to the study groups (n=6 gr1-6 & n=4/gr7) and kept at the institutional animal facility under specified pathogen-free conditions. CB6F1 mice (gr1-6) were intramuscularly (i.m) immunized at days 0, 14 & 28 with 5μg of unmutated (gr1) or with different mutated versions of HSV-1 gE/gI proteins (gr2-6) formulated in AS01 (5μg). An additional group of mice was i.m injected with a saline solution (NaCl 150mM), following the same schedule of immunization, and used as negative control group (gr7).

Serum samples were collected at days 13, 27 & 42 post prime immunization (13PI, 13PII, 14PIII) to measure both anti-HSV-1 gE/gI-specific and anti-HSV-2 gE and anti-HSV-2 gI cross reactive IgG antibody responses. The functions of vaccine-specific and HSV-2 cross reactive antibodies were also investigated in the serum samples collected 14 days after the third immunization. Spleens were collected 14 days post third immunization (14PIII) to evaluate, ex-vivo, systemic CD4+ and CD8+ T cell responses towards HSV-1 gE, HSV-1 gI & HSV-2 gE antigens. Details for each group is described in the Table 5.

**Table 5 Summary of the study design and formulation tested**

| Group | Number of animals | Treatment | | | | | Immunization schedule (Days) | Sample collection *(organs, blood volume)* and days |
|---|---|---|---|---|---|---|---|---|
| | | Vaccine name and dose | Lot numbers | Adjuvant dose | Adjuvant lot number | Volume and route of injection | | |
| 1 | 6 | HSV-1 gE/gI (5µg) | BMP1299 | AS01 | LIQ-2019/2014 | 50µL i.m | Days 0, 14 & 28 | Days 13 & 27: Serum-partial bleeding Day 42: Serum - final bleeding + Spleen |
| 2 | 6 | HSV-1 gE_**P319R**/gI (5µg) | BMP1281 | | | | | |
| 3 | 6 | HSV-1 gE_**P321D**/gI (5µg) | BMP1282 | | | | | |
| 4 | 6 | HSV-1 gE_**R322D**/gI (5µg) | BMP1283 | | | | | |
| 5 | 6 | HSV-1 gE_**N243A_R322D**/gI (5µg) | BMP1284 | | | | | |
| 6 | 6 | HSV-1 gE_**A340G_S341G_V342G/** gI (5µg) | BMP1285 | | | | | |
| 7 | 4 | NaCl150mM | N/A | N/A | N/A | | | |

**Materials and methods**

Investigational products

The antigens used in this study were different versions of the purified recombinant gE/gI protein (ectodomain only). The material was expressed on ExpiCHO™ cells as research lot only.

AS01 is a liposome based adjuvant system (AS) containing QS-21 (a triterpene glycoside purified from the bark of Quillaja saponaria) and MPL (3-D Monophosphoryl lipid A), with liposomes as vehicles for these immunoenhancers and a buffer including NaCl as isotonic agent (variant 3).

A single human dose of the AS01b Adjuvant System (0.5 mL) contains 50μg of QS-21 and 50μg of MPL. In this study, the volume injected in mice is 1/10th of a human dose corresponding to a 5μg QS-21 and 5μg MPL per dose.

Animal model

It is well accepted that small animal models are useful tools to study the immunogenicity profile of new vaccine candidates. In order to get a general overview of the capacity of the vaccine candidates to induce T cell immune responses, CB6F1 mice (hybrid of C57B1/6 and Balb/C mice) have been used in this study.

CB6F1 mice have been shown to generate potent CD4+/CD8+ T cell and humoral immune responses following vaccination with various types of immunogens, including adjuvanted proteins and viral vectors. The profile of the vaccine-induced immune responses generated in these mice compared to expected responses in humans may nevertheless be impacted by some differences pertaining to TLR expression, HLA background and antigen presentation. However, the capacity for inducing CD4+/CD8+ T immune responses has shown comparable trends between these mice and humans.

***Immunological read-outs***

*Total anti-HSV-1 gE/gI-specific IgG binding antibodies measured by ELISA*

Quantification of the total anti- HSV-1 gE/gI-specific IgG antibodies was performed using indirect ELISA. Recombinant gE/gI heterodimer protein (BMP 1299) from HSV-1 were used as coating antigen. This protein was produced using the ExpiCHO™ expression system. The ELISA was otherwise carried out as described in Example 1.

Optical densities (OD) were captured and analysed using the SoftMaxPro GxP v5.3 software. A standard curve was generated by applying a 4-parameter logistic regression fit to the reference standard results (reference standard = 20200023 14PIII - Pool of mice 1.1 to 1.20 immunized with AS01-HSV-1 gE/gI HEK (5μg of each/dose). Antibody titer in the samples was calculated by interpolation of the standard curve. The antibody titer of the samples was obtained by averaging the values from dilutions that fell within the 20-80% dynamic range of the standard curve. Titers were expressed in EU/mL (ELISA Units per mL).

*Total anti-HSV-2 gE & gI cross-reactive IgG antibodies measured by ELISA*

Quantification of the total anti-HSV-2 gE or gI cross-reactive IgG antibodies was performed using indirect ELISA. Recombinant gE (~51kDa) (BMP 1291) or gI protein (~46kDa) (BMP 1292) from HSV-2 were used as coating antigens. These proteins were produced using the ExpiHEK293F™ expression system. The indirect ELISA was otherwise carried out as described for this method in Example 1.

*Anti-HSV-1 gE and gI-specific and **HSV-2** gE cross-reactive CD4+/CD8+ T cell responses measured by ICS assay*

The frequencies of anti-HSV-1 gE & anti-HSV-1 gI-specific and anti-HSV-2 gE cross-reactive CD4+ and CD8+ T-cells producing IL-2 and/or IFN-γ and/or TNF-α were evaluated in splenocytes collected 14 days post third immunization after ex-vivo stimulation with HSV-1 gE or gI or HSV-2 gE peptides pools.

*Isolation of splenocytes*

As described for this method in Example 1.

*Cell preparation*

Fresh splenocytes were seeded in round bottom 96-well plates at $10^6$ cells/well (100μL). The cells were then stimulated for 6 hours (37°C, 5% CO2) with anti-CD28 (BD biosciences, clone 37.51) and anti-CD49d antibodies (BD Biosciences, clone 9C10 (MFR4.B)) at 1μg/mL per well, containing 100μL of either:

- 15 mers overlapping peptides pool covering the sequence of gE protein from HSV-1 (1μg/mL per peptide per well).
- 15 mers overlapping peptides pool covering the sequence of gI protein from HSV-1 (1μg/mL per peptide per well).
- 15 mers overlapping peptides pool covering the sequence of gE protein from HSV-2 (1μg/mL per peptide per well).
- 15 mers overlapping peptides pool covering the sequence of Human β-actin protein (1μg/mL per peptide per well) (irrelevant stimulation).
- RPMI/additives medium (as negative control of the assay).
- PMA - ionomycin solution (Sigma, P8139) at working concentrations of 0,25 μg/mL and 2,5 μg/mL respectively (as positive control of the assay).

After 2 hours of ex vivo stimulation, Brefeldin A (Golgi plug ref 555029, BD Bioscience) diluted 1/200 in RPMI/additives supplemented with 5% FCS was added for 4 additional hours to inhibit cytokine secretion. Plates were then transferred at 4°C for overnight incubation.

*Intracellular Cytokine Staining*

As described for this method in Example 1.

*Cell acquisition and analysis*

As described for this method in Example 1.

*Competitive ELISA to evaluate the ability of vaccine-specific antibodies to decrease human IgG Fc binding by HSV-1 gE/gI protein*

The ability of polyclonal sera collected in different groups of mice to decrease, in-vitro, hIgG Fc binding by recombinant HSV-1 gE/gI protein was investigated by competitive ELISA. Recombinant HSV-1 gE/gI protein (BMP 1299), produced using the ExpiCHO™ expression system was used as coating antigen.

Polystyrene 96-well ELISA plate (Nunc F96 Maxisorp cat 439454) were coated with 50μL/well of HSV-1 gE/gI protein diluted at a concentration of 2μg/mL in free Calcium/Magnesium PBS buffer and incubated overnight at 4°C. After incubation, the coating solution was removed and the plates were blocked with 100μL/well of PBS supplemented with 0,1% Tween-20 + 1% BSA (blocking buffer) for 1 h at 37°C.

In another 96-well Clear V-Bottom Polypropylene microplate (Falcon, ref 353263) a two-fold serial dilution (starting dilution 1/10) in blocking buffer for each serum was prepared in 60μL/well and mixed with 60μL/well of biotinylated-hIgG antibodies (Invitrogen, ref 12000C) pre-diluted at 0,7μg/mL in blocking buffer.

Then, after 1h of incubation with blocking buffer, the blocking solution was removed from the coated plates and 100μL of the mixture containing both hIgG and mice sera was transferred in the corresponding HSV-1 gE/gI coated wells and incubated 24h at 37°C. Positive control of the assay was a pool of anti-HSV-2 gE/gI serum samples from previous studies. Negative control of the assay was a pool of irrelevant HPV serum samples diluted 1/1000 and mixed with the same concentration of hIgG as the samples.

After 24h of incubation, the plates were washed four times with PBS 0.1% Tween20 (washing buffer) and 50μL/well of Steptavidin-horsedish Peroxydase AMDEX (Amersham, ref RPN4401V) diluted 2000x were added on the wells and plates were incubated for 30min at 37°C. Plates were then washed four times with washing buffer and 50μL/well of a solution containing 75% single-component TMB Peroxidase ELISA Substrate (ref 172-1072, Bio-Rad, USA) diluted in sodium Citrate 0.1M pH5.5 buffer were added for 10min at room temperature. Enzymatic color development was stopped with 50μL/well of 0,4N Sulfuric Acid 1M ($H_2SO_4$) and the plates were read at an absorbance of 450/620nm using the Versamax ELISA reader. Optical densities (OD) were captured and fitted in curve in Excel program.

Titers were expressed as the effective dilution at which 50% (i.e. $ED_{50}$) of the signal was achieved by sample dilution.

For each plate and using a reference sample (i.e. irrelevant serum), the reference $ED_{50}$ value was estimated using the following formula:

$$ED50 = OD_{0\%} + 0.5 * (OD_{100\%} - OD_{0\%})$$

where $OD_{100\%}$ is the highest OD obtained with similar samples and $OD_{0\%}$ is the lowest achievable signal. For each plate, the former was obtained by averaging (mean) 6 replicates while the latter was set at zero.

Sample $ED_{50}$ titers were computed by way of linear interpolation between the left and right measurements closest to the $ED_{50}$ estimate within the plate. The approximation was obtained, on the untransformed OD and the logarithm base 10 transformed dilutions, with the *approx* function of the *stats* R base package.

Sample were not assigned a titer in the following cases:

◦ no measurement was available above or below the $ED_{50}$,
◦ curve crossed at least twice the $ED_{50}$ and
◦ one of the dilution step (left or right) closest to the $ED_{50}$ was missing

*Competitive ELISA to evaluate the ability of HSV-2 cross reactive antibodies to decrease human IgG Fc binding by HSV-2 gE/gIprotein*

The ability of polyclonal sera collected in different groups of mice to decrease in-vitro hIgG Fc binding by recombinant HSV-2 gE/gI protein was investigated by competitive ELISA. Recombinant HSV-2 gE/gI protein (BMP1063), produced using the ExpiHEK293F™ expression system was used as coating antigen.

Polystyrene 96-well ELISA plate (Nunc F96 Maxisorp cat 439454) were coated with 50μL/well of HSV-2 gE/gI protein diluted at a concentration of 4μg/mL in free Calcium/Magnesium PBS buffer and incubated overnight at 4°C. After incubation, the coating solution was removed and the plates were blocked with 100μL/well of PBS supplemented with 0,1% Tween-20 + 1% BSA (blocking buffer) for 1 h at 37°C.

The blocking solution was removed from the coated plates and 60μL of two serial dilution of individual mouse serum (starting dilution 1/10 in blocking buffer) was prepared into 96-well Clear V-Bottom Polypropylene microplate (Falcon, ref 353263) and mixed with 60μL/well of biotinylated-hIgG antibodies (Invitrogen, ref 12000C) pre-diluted at 0,7μg/mL in blocking buffer. The blocking buffer from HSV-2 gE/gI coated plates was removed and 100μL of the mixture was then transferred in the corresponding wells for an incubation period of 24h at 37°C. Positive control serum of the assay was a pool of HSV-2 serum samples from previous studies. Negative control serum of the assay was a pool of irrelevant HPV serum samples diluted 1/100 and mix with hIgG too.

After incubation, the plates were washed four times with PBS + 0.1% Tween20 (washing buffer) and 50μL/well of Steptavidin-horsedish Peroxydase AMDEX (Amersham, ref RPN4401V) diluted 2000x were added on the well and plates were incubated for 30min at 37°C. After incubation, plates were washed four times with washing buffer and 50μL/well of a solution containing 75% single-component TMB Peroxidase ELISA Substrate (ref 172-1072, Bio-Rad, USA) diluted in sodium Citrate 0.1M pH5.5 buffer were added for 10min at room temperature. Enzymatic color development was stopped with 50μL/well of 0,4N Sulfuric Acid 1M ($H_2SO_4$) and the plates were read at an absorbance of 450/620nm using the Versamax ELISA reader. Optical densities (OD) were captured and fitted in curve in Excel program.

Titers were expressed as the effective dilution at which 50% (i.e. $ED_{50}$) of the signal was achieved by sample dilution.

For each plate and using a reference sample (i.e. irrelevant serum), the reference $ED_{50}$ value was estimated using the following formula:

$$ED50 = OD_{0\%} + 0.5 * (OD_{100\%} - OD_{0\%})$$

where $OD_{100\%}$ is the highest OD obtained with similar samples and $OD_{0\%}$ is the lowest achievable signal. For each plate, the former was obtained by averaging (mean) 6 replicates while the latter was set at zero.

Samples ED50 titers were computed by way of linear interpolation between the left and right measurements closest to the $ED_{50}$ estimate within the plate. The approximation was obtained, on the untransformed OD and the logarithm base 10 transformed dilutions, with the *approx* function of the *stats* R base package.

Sample were not assigned a titer in the following cases:

◦ no measurement was available above or below the $ED_{50}$,
◦ curve crossed at least twice the $ED_{50}$ and
◦ one of the dilution step (left or right) closest to the $ED_{50}$ was missing

*Evaluation of the ability of HSV-1-gE/gI specific and HSV-2 gE/gI cross-reactive antibodies to bind and activate mFcγRIII after incubation with HSV-1 or HSV-2 gE/gI transfected cells (Mouse FcγRIII ADCC bioassay - Promega)*

The mouse FcyRIII Antibody Dependent Cell Cytotoxicity (ADCC) Reporter Bioassay (Cat.# M1201), developed by Promega laboratory, is a bioluminescent cell-based assay which can be used to measure the ability of antibodies to specifically bind and activate the mouse FcyRIII expressed by modified Jurkat reporter cells. The method used was as described for Example 1.

Samples titer in the mouse ADCC-like assay were computed by means of the area under the curve (AUC) as described in Huang and Pang (Assay Drug Dev Technol. 2012 10(1):88-96), with minor modifications. Briefly, log-transformed responses (i.e. RLU) were fitted, on log3-transformed sample serial dilutions, with a 5-parameter logistic model for each sample on the plate. A negative control sample (i.e. irrelevant mouse serum diluted 1/1000) was used to evaluate, in each plate, the background signal threshold estimated with its geometric mean plus 3 standard deviations (i.e. exp[mean(logRLU) + 3*sd(logRLU)]). The area below the fitted curve of each sample and above the plate background (i.e. AUC) was computed using the "integrate" function in "R" software.

*In-vitro HSV-1 neutralization assay*

An in-vitro neutralization assay was developed to detect and quantify HSV-1 specific neutralizing antibody titers in serum samples from different animal species. The method used was as described for Example 1.

*In-vitro HSV-2 neutralization assay*

The method used was as described for Example 1.

*Statistical methods*

The distribution of each response was assumed to be lognormal.

For anti- HSV-1 gE/gI-specific antibody (pAb) response, a two-way analysis of variance (ANOVA) model was fitted on $\log_{10}$ titers by including groups (all except the NaCl one), time points (Day13 (13PI), Day27 (13PII) and Day42 (14PIII)) and their interaction as fixed effects. The NaCl group was not included as no response and variability was observed. Variance-covariance model selection was based on AICC criterion and individual data plot examination.

The variance-covariance for time points was modelled via an Unstructured matrix:

$$\begin{bmatrix} \sigma_1^2 & \sigma_{21} & \sigma_{31} \\ \sigma_{21} & \sigma_2^2 & \sigma_{32} \\ \sigma_{31} & \sigma_{32} & \sigma_3^2 \end{bmatrix}$$

**UN- Unstructured**

The unstructured matrix considers different variances and estimates unique correlations for each pair of time points. The same variance-covariance matrix is modelled for each vaccine group, indicating same variance between groups.

Geometric means and their 95% CIs are derived from this model.

Despite the fact that the NaCl group was not included in the ANOVA model, the comparisons associated to the primary objective were computed as follows: geometric means and 95% CI of vaccinated groups derived from the above model were divided by titer and given to all the NaCl recipients at the last timepoint. The resulting ratios should be understood as geometric mean ratios with their corresponding 95% CI.

For head to head comparison of vaccinated groups (at the last time point) and time point comparisons (PII/PI, PIII/PII, and PIII/PI) within each group, all the geometric mean ratios and their 95% CIs were derived from the model.

For each HSV-2 gE and gI cross-reactive IgG antibody response, a one-way analysis of variance (ANOVA) model was fitted on log 10 titers by including groups (all except the NaCl one) as fixed effect. The NaCl group was not included as no response and variability was observed. For gE cross-reactive IgG antibody titers, heterogeneity of variance was detected and therefore a different variance was assumed for each group. For gI cross-reactive IgG antibody titers, no clear heterogeneity of variance was detected and therefore identical variances were assumed for the different groups. Geometric means and their 95% CIs are derived from these models. For comparison of vaccinated to NaCl groups, geometric means and 95% CI of vaccinated groups derived from the above model were divided by titer given to all the NaCl recipients. The resulting ratios should be understood as geometric mean ratios with their corresponding 95% CI. For head to head comparison of vaccinated groups, all the geometric mean ratios and their 95% CIs were derived from the model.

On each vaccine-specific or cross-reactive % of CD4+/ CD8+ T cell responses (gE or gI), a one-way analysis of variance (ANOVA) model was fitted on log10 frequencies by including groups (all groups including the NaCl group) as fixed effect. No clear heterogeneity of variance was detected and therefore identical variances were assumed for the different groups. For both % of CD4+ and CD8+ T cell responses, the NaCl threshold was based on P95 of data across stimulation in NaCl negative control group. No modelling was performed on % of HSV-1 gE-specific CD8+ T cells and on % of HSV-2 gE cross-reactive CD8+ T cells since response were below the P95 NaCl threshold for all vaccine groups. Geometric means and geometric mean ratios (with their corresponding 95% CIs) were derived from these models.

For the evaluation of the dissociation of the human IgG Fc binding by the pAbs, ED50 response was calculated for each sample. On each response (HSV-1 or HSV-2), a one-way analysis of variance (ANOVA) model was fitted on log10 values by including groups (all groups excluding the NaCl group) as fixed effect. No clear heterogeneity of variance was detected and therefore identical variances were assumed for the different groups. Geometric means and geometric mean ratios (their corresponding 95% CIs) were derived from these models.

For HSV-1-specific neutralizing antibody titers, a one-way analysis of variance (ANOVA) model awas fitted on $log_{10}$ values by including groups (all groups excluding the NaCl group) as fixed effect. No clear heterogeneity of variance was detected and therefore identical variances were assumed for the different groups. Geometric means and geometric mean ratios (their corresponding 95% CIs) were derived from these models.

**Results**

*Different versions of AS01-HSV-1 gE/gI protein can induce vaccine-specific and cross reactive IgG antibodies*

The anti-HSV-1 gE/gI-vaccine-specific and anti-HSV-2 gE or anti-HSV-2 gI cross-reactive IgG antibody responses were investigated by ELISA.

As expected, no anti-HSV-1-specific gE/gI response was observed in NaCl control group (<30 EU/ml for all mice) while all the AS01-adjuvanted HSV-1 gE/gI vaccinated-mice (gr2-6) produced a response above 2.000.000 EU/mL at the last time point. Compared to NaCl control group, high anti-HSV-1 gE/gI- vaccine-specific IgG antibody response was induced by all AS01-adjuvanted HSV-1 gE/gI proteins tested (unmutated and mutated versions) in this study at 14days post third immunization (all GMRs > 80.000) (**Figure 33 & Figure 34**).

In all groups of mice immunized with different mutated versions of HSV-1 gE/gI protein, anti-HSV-1 gE/gI-specific IgG antibody responses increased after the second immunization (day27 (13PII)) compared to the first one (day13 (13PI)), with a fold increase ranging from 68 to 145 (**Figure 35A**).

For all HSV-1 gE/gI mutant candidates, similar anti-HSV-1 gE/gI-specific antibody response was observed 14days after the third immunization (day42(14PIII)) compared to the second one (day27 (13PII)), with a fold increase close to 1 (with CIs containing 1), indicating no booster effect of the third dose (**Figure 35B**).

Altogether, results suggest very similar anti-HSV-1 gE/gI-specific IgG antibody responses between the different mutated versions of HSV-1 gE/gI mutant candidates 14days post third immunization (with GMRs close to 1-fold change and most CIs are totally included in [0.5,2]) and between the different mutated versions of HSV-1 gE/gI protein and the unmutated one (**Figure 36**).

As expected, anti-HSV-2 gE and anti-HSV-2 gI cross-reactive responses were not observed in NaCl control group (17 EU/mL for all mice). At 14 days post third immunization and compared to NaCl control group, high anti-HSV-2 gE and anti-HSV-2 gI cross-reactive IgG antibody responses were induced in all groups of mice immunized by different unmutated and different mutated versions of AS01-adjuvanted HSV-1 gE/gI protein (all GMRs > 9.000 for gE and >2.000 for gI) (**Figure 37, Figure 38**).

Several differences between HSV-1 gE/gI unmutated and mutant candidates are observed on both anti-HSV-2 gE and anti-HSV-2 gI cross-reactive antibody responses at 14days post third immunization. Indeed, both anti-HSV-2 gE and anti-HSV-2 gI cross-reactive responses induced from unmutated vaccine protein (group 1) seemed to be higher than the ones induced by P319R mutant (group 2) and N243A_R322D mutant (group 5) (fold changes ranging from 2 to 7 with CIs not containing 1). Both anti-HSV-2 HSV-2 gE and anti-HSV-2 gI responses seemed to be also higher with A340G_S341G_V342G mutant (group 6) than with P319R mutant (group 2) (GMRs of 2.9 and 2.7 with CIs not or almost not containing 1, respectively). In addition, anti-HSV-2 gE response induced by R322D mutant (group 4) seemed to be twice higher than the one induced by P319R mutant (group 2) (with CI not containing 1). Finally, anti-HSV-2 gI response induced by P321D (group 3), R322D (group 4) and A340G_S341G_V342G (group 6) mutants seemed to be higher than the one induced by N243A_R322D mutant (group 5) (fold changes ranging from 2 to 5, with CIs not containing 1). No other differences between mutants seemed to be observed (with GMRs <2-fold change and/or CIs containing 1) (**Figure 39**).

*Different versions of AS01-HSV-1 gE/gI proteins can induce functional vaccine-specific and HSV-2 gE/gI cross reactive antibody responses*

In this study, HSV-1-specific & HSV-2 gE/gI cross-reactive antibody functions were only investigated in the sera collected at 14 days post third immunization. In this regard, the ability of vaccine-induced pAbs to neutralize HSV-1 & HSV-2 viruses , to decrease in-vitro binding of human IgG Fc by HSV-1 or HSV-2 gE/gI protein and to cross-react and activate mFcγRIII expressed on modified Jurkat cells after incubation with HSV-2 or HSV-1 gE/gI positive 3T3 cells was investigated.

Consistent moderate levels of neutralizing antibody (nAb) response directed to HSV-1 VR-1789 strain was detected in all groups of mice immunized with the different versions of AS01-adjuvanted HSV-1 gE/gI protein (**Figure 40A**). These results suggest no difference in term of HSV-1 neutralizing activity between the different groups of mice (GMRs <2-fold change with all CIs containing 1). However, a trend for higher response in unmutated group compared to P319R mutant was observed (GMR P319R/unmutated=0.55, with upper limit of CI just above 1) (**Figure 40B**). In contrast, no anti-HSV-2 cross-reactive nAb response directed to HSV-2 MS strain was detected in any of the groups immunized with AS01-adjuvanted HSV-1 gE/gI proteins (**Figure 41**).

Then, the ability of sera from mice immunized with different versions of HSV-1 gE/gI protein to compete and decrease hIgG Fc binding by HSV-1 or HSV-2 gE/gI protein was assessed, in-vitro, by competitive ELISA. All HSV-1 gE/gI proteins (mutated and unmutated) elicited vaccine-specific and HSV-2 cross reactive polyclonal antibodies able to decrease hIgG Fc binding by HSV-1 or HSV-2 gE/gI protein (**Figure 42; Figure 44**).

At same serum dilution, the level of hIgG Fc binding on HSV-1 gE/gI protein was quite similar between different groups of HSV-1 gE/gI immunized mice. The calculation of ED50 shown that only R322D (group4) and A340G_S341G_V342G (group 6) mutants seemed to induce higher response compared to the unmutated one (group 1) with fold-increases of 2.2 and 2.4, respectively (and CIs not containing 1). No other differences were observed between the different groups (GMRs <2-fold change and CIs containing 1) (**Figure 43A; Figure 43B**).

At same serum dilution, a few differences between groups of HSV-1 gE/gI immunized mice seem to be observed on the level of hIgG Fc binding on HSV-2 gE/gI protein. Indeed, response induced from unmutated protein (group 1) seemed to be higher than the one induced by P319R (group 2), P321D (group 3), R322D (group 4) and N243A_R322D (group 5) mutants (fold changes ranging from 2 to 3 with CIs not containing 1). A trend for higher response with A340G_S341G_V342G mutant (group 6) than with P319R mutant (group 2) seems also observed (GMR of 1.90 and CI almost not containing 1). No other differences between groups seemed to be observed (with GMRs <2-fold change and CIs containing 1). (**Figure 45A & Figure 45B**).

Finally, the ability of HSV1-gE/gI-specific or HSV-2 gE/gI cross reactive antibodies to bind and activate in-vitro, mouse FcγRIII after incubation with HSV-1 or HSV-2 gE/gI transfected 3T3 cells, was investigated 14 days after the third immunization. Data shown on the **Figure 46, Figure 47 and Figure 74** suggested that all mice immunized with the different HSV-1 gE/gI mutant candidates could induce vaccine-specific or HSV-2 gE/gI cross reactive antibodies able to bind and activate Jurkat reporter cells expressing FcγRIII after incubation with HSV-1 or HSV-2 gE/gI transfected cells. As expected, activation of FcγRIII was not detected with sera from unvaccinated NaCl control mice (**Figure 46 and Figure 74**). All these data suggest that different mutated versions of HSV-1 gE/gI protein could induce functional vaccine-specific or HSV-2 gE/gI cross-reactive antibody response against HSV-1 or HSV-2 gE/gI protein.

*Different versions of AS01-HSV-1 gE/gIprotein induced anti-HSV-1 ge/gI-specific and anti-HSV-2 gE cross-reactive CD4+ T cell responses*

Compared to the NaCl control group, higher anti-HSV-1 gE and gI-specific and anti-HSV-2 gE cross-reactive CD4+T cell responses were detected 14 days after the third immunization in all groups of mice immunized with AS01-adjuvanted HSV-1 gE/gI proteins (unmutated and mutated versions), (**Figure 48A**) with a fold increase ranging from 11 to 63 between vaccinated and NaCl groups (CIs not containing 1) (**Figure 49A, Figure 49B & Figure 49C**). However, anti-HSV-1 gI-specific CD8+T cell response was only inconsistently detected in all vaccinated groups compared to the NaCl control group (GMRs ranging from 5 to 7 with CI not containing 1). The anti-HSV-1 gI-specific CD8+T cell response was considered as not relevant from a biological point of view (0.1 to 0.14%) (**Figure 49C & Figure 50**). No anti-HSV-1 gE-specific or anti-HSV-2 gE cross-reactive CD8+T cell responses was detected in any of vaccinated groups compared to the NaCl control group (data not shown).

Overall, results suggest very similar vaccine-specific and cross-reactive CD4+T cell responses between the different mutated versions of HSV-1 gE/gI protein and with the unmutated HSV-1 gE/gI candidate. Only P321D mutant (group3) seemed to induce higher CD4+T cell response compared to the unmutated one (group 1) with a fold increase close to 2 (GMR of 1.9 for anti-HSV-1 gE-specific CD4+T cells, GMR of 2 for HSV-2 gE cross-reactive CD4+T cells, and GMR of 1.89 for anti-HSV-1 gI-specific CD4+T cells, with CIs not containing 1) (**Figure 51A & Figure 52**). In addition, a trend for higher anti-HSV-1 gE-specific CD4+T cell response was also observed with R322D mutant (group4) compared to the unmutated protein (group 1) (GMR of 1.96 and CI not containing 1) (**Figure 51B**). Finally, slightly higher anti-HSV-1 gI-specific CD4+T cell response was observed with P321D mutant (group3) compared to R322D mutant (group4) (fold increase of almost 2 and CI not containing 1) (**Figure 52**). No other differences between mutants were observed (with GMRs <2-fold change and/or CIs containing 1).

**EXAMPLE 4 - Immuno-evaluation of several LNP-formulated SAM HSV-1 gE/gI mutants in CB6F1 mice**

**Objectives**

The objective of this study was to generate immunogenicity data from different LNP-formulated SAM-HSV-1 gE/gI constructs containing mutation(s) in gE Fc binding domain to drop the avidity to human Immunoglobulin G Fc domain.

Twenty-one days post second immunization, the vaccine-specific polyclonal antibody (pAb) response and the anti-HSV-1 gE/gI-specific CD4+T cell responses induced by different mutants of LNP-formulated SAM HSV-1 gE/gI construct were compared to NaCl control group. In addition, head-to-head comparison of all vaccine candidates in term of anti-HSV-1 gE/gI-specific IgG antibody responses and anti-HSV-1 gE- and anti-HSV-1 gI-specific CD4+ T cell responses were performed 21 days post second immunization.

Finally, the exploratory objectives were to evaluate, in different groups of mice immunized with different mutated versions of LNP-formulated SAM HSV-1 gE/gI construct, the anti-HSV-1 gE/gI-specific antibody responses after one immunization, the anti-HSV-2 gE and anti-HSV-2gI cross-reactive antibody responses after two immunization, the functions of vaccine-specific and HSV-2 gE/gI cross-reactive polyclonal antibodies after two immunization and the levels of anti-HSV-1 gE-specific and anti-HSV-1 gI-specific CD8+T cell responses and anti-HSV-2 gE cross-reactive T cell response after the second immunization.

**Overview of the study design**

Female CB6F1 inbred mice aged 6-8 weeks old from Harlan laboratory (OlaHsd) were randomly assigned to the study groups (n=6 gr1-5; n=6 gr6) and kept at the institutional animal facility under specified pathogen-free conditions. CB6F1 mice (gr1-5) were intramuscularly (i.m) immunized at days 0 & 28 with 1μg of different versions of SAM-HSV-1 gE/gI mutants formulated in Lipid nanoparticles (LNP). An additional group of mice was i.m injected with a saline solution (NaCl150mM) following the same schedule of immunization and used as negative control group (gr6).

Serum samples were collected at days 28 & 49 post prime immunization (28PI, 21PII) to measure both anti-HSV-1 gE/gI-specific and cross reactive anti-HSV-2 gE and anti-HSV-2 gI IgG antibody responses. The functions of vaccine-specific and anti-HSV-1 gE/gI cross-reactive antibodies were also investigated in the serum samples collected 21 days after the second immunization. Spleens were collected 21 days post second immunization (21PII) to evaluate, ex-vivo, systemic CD4+ and CD8+ T cell responses towards HSV-1 gE, HSV-1 gI & HSV-2 gE antigens. Details for each group is described in the Table 6. A total of 36 animals was used for the whole study.

**Table 6 Summary of the study design and formulation tested**

| Group | Number of animals | Treatment | | | | |
|---|---|---|---|---|---|---|
| | | Vaccine name and dose | Lot numbers | Volume and route of injection | Immunization schedule (Days) | Sample collection *(organs, blood volume)* and days |
| 1 | 6 | LNP/SAM HSV-1 gE_P319R/gI (1μg) | 1700-29-1203 | 50μL i.m | Days 0 & 28 | Day 28: Serum - partial bleeding Day 49: Serum - final bleeding + Spleen |
| 2 | 6 | LNP/SAM HSV-1 gE_P321 D/gI (1μg) | 1700-29-1204 | | | |
| 3 | 6 | LNP/SAM HSV-1 gE_R322D/gI (1μg) | 1700-29-1204 | | | |
| 4 | 6 | LNP/SAM HSV-1 gE_N243A_ R322D /gI (1μg) | 1700-29-1205 | | | |
| 5 | 6 | LNP/SAM HSV-1 gE_A340G_S341 G_V342G/gI (1μg) | 1700-29-1206 | | | |
| 6 | 6 | NaCl150mM | N/A | | | |

**Materials and methods**

***Investigational products***

Different mutated versions of SAM HSV-1 gE/gI vector were generated by In vitro Transcription (IVT) according to established protocols. Preparation of LNP with SAM followed established methods as follows. In this method, LNP-SAM is prepared by rapidly mixing ethanolic solutions of lipids with aqueous buffers that contain SAM. The rapid mixing results in a supersaturation of hydrophobic components that have ionically paired with SAM.

The SAM/lipid complexes condensate and precipitate through nucleation mediated precipitation, yielding small and narrowly disperse nanoparticles. Following this mixing step, the lipid nanoparticles mature to entrap the RNA and then are transferred to a final Tris/sucrose storage buffer through a buffer exchange step. The LNP solutions are then characterized for size, lipid content, RNA entrapment, final SAM concentration, and in vitro potency. The materials can be frozen at -80C for over 6 months after the addition of 5% (wt/v) sucrose.

***Immunological read-outs***

*Total anti-HSV-1 ge/gI-specific IgG antibodies measured by ELISA*

This was performed as described for Example 3.

*Total anti-HSV-2 gE & anti-HSV-2 gI cross-reactive IgG antibodies measured by ELISA*

This was performed as described for Example 3.

*Anti-HSV-1 gE and anti-HSV-1 gI-specific & anti-HSV-2 gE cross-reactive CD4+/CD8+ T cell responses measured by ICS assay*

This was performed as described for Example 3.

*Competitive ELISA to evaluate the ability of vaccine-specific antibodies to decrease human IgG Fc binding by gE/gIprotein*

This was performed as described for Example 3.

*Competitive ELISA to evaluate the ability o/ HSV-1 cross reactive antibodies to decrease human IgG Fc binding by HSV-2 gE/gI protein*

This was performed as described for Example 3.

*Evaluation of the ability ofHSV-2 cross-reactive antibodies to bind and activate mFcγRIII after incubation with HSV-2 gE/gI transfected cells (Mouse FcγRIII ADCC bioassay - Promega)*

This was performed as described for Example 3.

*In-vitro HSV-1 neutralization assay*

This was performed as described for Example 3.

*In-vitro HSV-2 MS neutralization assay*

This was performed as described for Example 3.

*Statistical methods*

The distribution of each response was assumed to be lognormal.

For anti- HSV-1 gE/gI-specific polyclonal antibody (pAb) response, a two-way analysis of variance (ANOVA) model is fitted on $\log_{10}$ titers by including groups (all except the NaCl one), time points (Day28 (28PI) and Day49 (21PII)) and their interaction as fixed effects. The NaCl group was not included as no response and variability was observed. Variance-covariance model selection was based on AICC criterion and individual data plot examination.

The variance-covariance for time points was modelled via a Compound Symmetry matrix:

$$\begin{bmatrix} \boldsymbol{\sigma}^2 + \boldsymbol{\sigma}_1 & \boldsymbol{\sigma}_1 \\ \boldsymbol{\sigma}_1 & \boldsymbol{\sigma}^2 + \boldsymbol{\sigma}_1 \end{bmatrix}$$

**CS- Compound Symmetry**

The compound symmetry considers same variance and same correlation between timepoints. The same variance-covariance matrix was modelled for each vaccine group, indicating same variance between groups.

Geometric means and their 95% CIs are derived from this model.

Despite the fact that the NaCl group was not included in the ANOVA model, the comparisons associated to the primary objective were computed as follows: geometric means and 95% CI of vaccinated groups derived from the above model were divided by titer given to all the NaCl recipients at the last timepoint. The resulting ratios should be understood as geometric mean ratios with their corresponding 95% CI.

For head to head comparison of vaccinated groups (at the last time point) and time point comparison (PII/PI) within each group, all the geometric mean ratios and their 95% CIs were derived from the model.

For each HSV-2 cross-reactive IgG antibody response (gE or gI), a one-way analysis of variance (ANOVA) model was fitted on $\log_{10}$ titers by including groups (all except the NaCl one) as fixed effect. The NaCl group was not included as no response and variability was observed. No clear heterogeneity of variance was detected and therefore identical variances were assumed for the different groups. Geometric means and their 95% CIs are derived from these models. For comparison of vaccinated to NaCl groups, geometric means and 95% CI of vaccinated groups derived from the above model were divided by titer given to all the NaCl recipients. The resulting ratios should be understood as geometric mean ratios with their corresponding 95% CI. For head to head comparison of vaccinated groups, all the geometric mean ratios and their 95% CIs were derived from the model.

On each vaccine-specific or cross-reactive % of CD4+/ CD8+ T cell responses (gE-, or gI-specific), a one-way analysis of variance (ANOVA) model was fitted on log10 frequencies by including groups (all groups including the NaCl group) as fixed effect. No clear heterogeneity of variance was detected for % of HSV-2 gE cross-reactive CD4+ T cell response and therefore identical variances were assumed for the different groups. For other responses, different variances were modeled for the different groups. For both % of CD4+ and CD8+ T cell responses, the NaCl threshold is based on P95 of data across stimulation in NaCl negative control group. No modelling was performed on % of HSV-1 gE-specific or HSV-2 gE cross-reactive CD8+ T cells since most of the response were below the P95 NaCl threshold for all vaccine groups. Geometric means and geometric mean ratios (with their corresponding 95% CIs) were derived from these models.

For the evaluation of the dissociation of the human IgG Fc binding by the pAbs (HSV-1 or HSV-2), ED50 response was calculated for each sample. On each response (HSV-1 or HSV-2), a one-way analysis of variance (ANOVA) model was fitted on log10 values by including groups (all groups excluding the NaCl group) as fixed effect. No clear heterogeneity of variance was detected and therefore identical variances were assumed for the different groups. Geometric means and geometric mean ratios (their corresponding 95% CIs) were derived from these models.

For HSV-1-specific neutralizing antibody titers, a one-way analysis of variance (ANOVA) model was fitted on log10 values by including groups (all groups excluding the NaCl group) as fixed effect. No clear heterogeneity of variance was detected and therefore identical variances were assumed for the different groups. Geometric means and geometric mean ratios (their corresponding 95% CIs) were derived from this model.

**Results**

***Different LNP/SAM HSV-1 gE/gI mutants induced vaccine-specific and anti-HSV-2 gE and anti-HSV-2 gI cross-reactive IgG antibody responses***

The level of anti-HSV-1 gE/gI-specific IgG antibody response was investigated by ELISA. Note that one sample in NaCl group (sample 6.2) was not evaluated 21 days after the second immunization due to lack of serum availability.

Twenty-one day post the second immunization, all LNP/SAM-HSV-1 gE/gI vaccinated groups developed strong anti-HSV-1 gE/gI-specific antibody response compared to the NaCl control group (response above 600.000 EU/mL; all GMRs> 18.000) (**Figure 53 & Figure 54**). As expected, no anti-HSV-1 gE/gI-specific response was observed in the NaCl control group (<40 EU/ml for all mice). In all groups of mice immunized with different mutated versions of LNP-formulated SAM-HSV-1 gE/gI vector, anti-HSV-1 gE/gI-specific IgG antibody responses increased after the second immunization (day49 (21PII)) compared to the first immunisation (day28 (28PI)), with a fold increase ranging from 12 to 16 (**Figure 55**).

Finally, head to head comparison of all vaccine-immunized groups of mice suggests the induction of a similar anti-HSV-1 gE/gI-specific IgG antibody response between the different mutated versions of LNP-formulated SAM-HSV-1 gE/gI vector 21 days post second immunization (with GMRs close to 1-fold change and most CIs are totally included in [0.5,2] and contain 1) (**Figure 56**).

As expected, anti-HSV-2 gE and anti-HSV-2 gI cross-reactive responses were not observed in NaCl control group (<30 EU/mL for all mice). At 21 days post second immunization and compared to NaCl control group, high anti-HSV-2 gE and anti-HSV-2 gI cross-reactive IgG antibody responses were induced in all groups of mice immunized with different mutated versions of LNP-formulated SAM-HSV-1 gE/gI vector (all GMRs > 2.000 for gE and >600 for gI) (**Figure 57, Figure 58**). In overall, head to head comparison of vaccine-immunized groups of mice suggests similar anti-HSV-2 gE or anti-HSV-2 gI cross-reactive IgG antibody responses 21days post second immunization (with most GMRs <2-fold change and/or CIs containing 1). Only A340G_S341G_V342G mutant (group 5) seemed to induce higher gE response compared to R322D mutant (group 3) with a fold increase of 2.47 (CI not containing 1) (**Figure 59**).

***Different mutated versions of LNP/SAM HSV-1 gE/gI vector can induce functional HSV-1 gE/gI specific and HSV-2 gE/gI cross-reactive antibody response***

In this study, HSV-1-specific and HSV-2 cross-reactive antibody functions were only investigated in the sera collected at 21days post second immunization. In this regard, the ability of polyclonal Abs to neutralize HSV-1 or HSV-2 viruses, to decrease, in-vitro, binding of human IgG Fc by HSV-1 or HSV-2 gE/gI protein and to bind and activate mFcyRIII expressed on modified Jurkat cells after incubation with HSV-2 gE/gI positive 3T3 cells was investigated for each group of mice.

Note that one sample in NaCl group (sample 6.2) was not evaluated in HSV-1/HSV-2 neutralization assays and in hIgG competitive ELISA on HSV-1/HSV-2 gE/gI protein due to lack of serum availability. In addition, one additional sample in LNP/SAM-HSV-1 gE_P321D/gI group (sample 2.3) was also not evaluated in HSV-2 neutralization assay and hIgG competitive ELISA on HSV-1/HSV-2 gE/gI protein for the same reason.

Low but consistent vaccine-specific neutralizing antibody (nAb) response directed to HSV-1 VR-1789 strain was detected in all groups of mice immunized with different mutated versions of LNP-formulated SAM-HSV-1 gE/gI vector (**Figure 60A**). No difference in the intensity of the response induced by the different candidates was detected after group comparisons (GMRs close to 1 with all CIs containing 1) (**Figure 60B**). However, no cross-reactive nAb response directed to HSV-2 MS strain was detected in any vaccinated groups of mice (**Figure 61**).

Then, the ability of sera from mice immunized with different LNP-formulated SAM-HSV-1 gE/gI mutants to compete and decrease, in-vitro, hIgG Fc binding by HSV-1 or HSV-2 gE/gI protein was assessed by competitive ELISA. All LNP/SAM HSV-1 gE/gI mutants elicited vaccine-specific polyclonal antibody response able to decrease hIgG Fc binding by HSV-1 or HSV-2 gE/gI protein.

No clear difference in the dissociation curve of hIgG Fc binding by HSV-1 gE/gI protein was observed with the polyclonal sera collected in mice immunized with the different LNP/SAM HSV-1 gE/gI mutants (GMRs close to 1 with all CIs containing 1), which suggest that all candidates induce vaccine specific functional antibodies able to inhibit hIgG Fc binding by HSV-1 gE/gI protein (**Figure 62 & Figure 63**). In addition, no significant difference in the dissociation curve of hIgG Fc binding by HSV-2 gE/gI protein was observed with polyclonal sera collected in mice immunized with the different LNP/SAM HSV-1 gE/gI mutants (with most GMRs <2-fold change and/or CIs containing 1). Only A340G_S341G_V342G mutant (group 5) seemed to induce higher cross reactive HSV-2 hIgG FC binding response compared to N243A_R322D mutant (group 4) with a fold increase of 2.18 (CI not containing 1). A trend for higher response with A340G_S341G_V342G mutant (group 5) than with R322D mutant (group 3) seems also observed (GMR of 1.86 and CI almost not containing 1) (**Figure 64, Figure 65**).

Finally, the ability of vaccine-specific pAbs to bind and activate, in-vitro, mouse FcγRIII after incubation with HSV-2 gE/gI transfected 3T3 cells, was investigated 21days after the second immunization. Data shown in **Figure 66** suggested that all mice immunized with the different LNP-SAM-HSV-1gE/gI mutant candidates could induce HSV-2 cross reactive antibodies able to specifically bind and activate Jurkat reporter cells expressing FcγRIII after incubation with HSV-2 gE/gI transfected cells. As expected, activation of FcγRIII was not detected with sera from unvaccinated NaCl control mice (Figure 66).

Altogether, these results suggest that all mutated versions of LNP-SAM HSV-1 gE/gI can induce functional vaccine-specific and cross-reactive polyclonal antibody response 21 days after the second immunization in mice.

***Different mutated versions of LNP/SAM HSV-1 gE/gI vector induced vaccine specific and cross-reactive T cell responses***

Compared to the NaCl control group, higher anti-HSV-1 gE & anti-HSV-1 gI specific and HSV-2 gE cross-reactive CD4+T cell responses were detected 21 days after the second immunization in all groups of mice immunized with different mutated versions of LNP-formulated SAM-HSV-1 gE/gI vector (**Figure 67A**). Note that only moderate level of anti-HSV-1 gE-specific CD4+T cell response was observed (about 0.4%, with GMRs over NaCl around 25) (**Figure 67A; Figure 68A**). Cross-reactive HSV-2 gE CD4+T cell response was also detected in all vaccinated groups compared to the NaCl control group (GMRs ranging from 5 to 14 with CI not containing 1), but this response was low (about 0.15%) (**Figure 67A; Figure 68B**). Compared to the NaCl control group, high anti-HSV-1 gI-specific CD4+/CD8+T cell responses were detected in all groups of mice immunized with different mutated versions of LNP-formulated SAM HSV-1 gE/gI vector (about 1% for anti-HSV-1 gI-CD4+ T cells and 3% for anti-HSV-1 gI-CD8+ T cells, with GMRs over NaCl around 100) (**Figure 67A; Figure 67B; Figure 68C; Figure 68D**). However, compared to NaCl group, there is no clear evidence that either anti-HSV-1 gE-specific or anti-HSV-2 gE cross-reactive CD8+T cell responses were detected in any of vaccine groups (most of the vaccinated animals had a response below the P95 NaCl threshold) (**Figure 67B**).

A head to head comparison of different LNP/SAM HSV-1 gE/gI mutants have suggested similar vaccine-specific and anti-HSV-2 gE cross reactive CD4+T cell responses and anti-HSV-1 gI-specific CD4+/CD8+ T cell responses. Only N243A_R322D mutant (group4) seemed to induce higher anti-HSV-2 gE cross-reactive CD4+T cell response compared to other mutants (**Figure 69B**). However, this difference seemed to be mainly triggered by one extremely high responder in this group 4 (0.82%). No differences in the intensity of vaccine-specific CD4+ and CD8+T cell responses was observed between the different mutants (GMRs <2-fold change with most CIs totally included in [0.5,2] and/or containing 1) (**Figure 69A; Figure 70A; Figure 70B**).

## Claims

1. An HSV2 Fc receptor or an immunogenic fragment or variant thereof for use in inducing a cross reactive immune response against HSV1 when administered to a subject.

2. An HSV1 Fc receptor or an immunogenic fragment or variant thereof for use in inducing a cross reactive immune response against HSV2 when administered to a subject.

3. The HSV1 or HSV2 Fc receptor or immunogenic fragment or variant thereof for use according to claim 1 or 2 wherein the Fc receptor or immunogenic fragment or variant thereof is administered to a subject for use in treating a subject infected with HSV or for preventing HSV infection in a subject, optionally wherein the Fc receptor or immunogenic fragment or variant thereof is administered to a subject for use in treating a subject infected with HSV1 and/or HSV2 or for preventing HSV1 and/or HSV2 infection in a subject.

4. The HSV1 or HSV2 Fc receptor or immunogenic fragment or variant thereof for use according to any previous claim wherein the cross-reactive immune response comprises a functional cross-reactive immune response.

5. The HSV1 or HSV2 Fc receptor or immunogenic fragment or variant thereof for use according to claim 4 wherein the functional cross-reactive immune response comprises a cross serotype cytotoxic antibody response, a cross reactive T cell response and/or generation of antibodies that can inhibit the immune evasion activity of HSV, optionally wherein the antibodies that can inhibit the immune evasion activity of HSV, inhibit binding of human IgG to the HSV Fc receptor.

6. The HSV1 or HSV2 Fc receptor or immunogenic fragment or variant thereof for use according to any one of claims 1 or 3 to 5 wherein said Fc receptor or immunogenic fragment or variant thereof is from a HSV2 gE2 ectodomain.

7. The HSV1 or HSV2 Fc receptor or immunogenic fragment or variant thereof for use according to any one of claims 2 to 5 wherein said Fc receptor or immunogenic fragment or variant thereof is from a HSV1 gE1 ectodomain.

8. The HSV1 or HSV2 Fc receptor or immunogenic fragment or variant thereof for use according to any one of the preceding claims wherein said Fc receptor or immunogenic fragment or variant thereof is part of a heterodimer with a binding partner from HSV or a fragment thereof.

9. The HSV1 or HSV2 Fc receptor or immunogenic fragment or variant thereof for use according to any one of claims 1, 3 to 6 or 8 wherein said Fc receptor or immunogenic fragment or variant thereof is a HSV2 gE2 ectodomain having the amino acid sequence shown at SEQ ID NO: 7, or a variant thereof which is at least 90% identical thereto.

10. The HSV1 or HSV2 Fc receptor or immunogenic fragment or variant thereof for use according to claim 6 wherein said Fc receptor or immunogenic fragment or variant thereof is part of a heterodimer with a binding partner from HSV or a fragment thereof, preferably wherein i) the Fc receptor is HSV2 gE2 or an immunogenic fragment thereof, and the binding partner is HSV2 gI2 or a fragment thereof and/or ii) wherein said binding partner or fragment thereof is a HSV2 gI2 ectodomain

11. The HSV1 or HSV2 Fc receptor or immunogenic fragment or variant thereof for use according to claim 7 wherein said Fc receptor or immunogenic fragment or variant thereof is part of a heterodimer with a binding partner from HSV or a fragment thereof, preferably wherein i) the Fc receptor is HSV1 gE1 or an immunogenic fragment thereof, and the binding partner is HSV1 gI1 or a fragment thereof and/or ii) said binding partner or fragment thereof is a HSV1 gI1 ectodomain.

12. The HSV1 or HSV2 Fc receptor or immunogenic fragment or variant thereof for use according to claim 10 wherein said binding partner or fragment thereof is a HSV2 gI2 ectodomain having the amino acid sequence shown at SEQ ID NO: 8, or a variant thereof which is at least 90% identical thereto.

**13.** The HSV1 or HSV2 Fc receptor or immunogenic fragment or variant thereof for use according to any preceding claim wherein: -

i) said Fc receptor or immunogenic fragment or variant thereof is administered to the subject together with an adjuvant, preferably an adjuvant comprising a TLR4 agonist and an immunologically active saponin, more preferably an adjuvant comprising 3D-MPL and QS21 in a liposomal formulation,
ii) said use does not comprise administration of an immunodominant viral antigen to the subject, in particular when the Fc receptor is HSV2 gE2 or HSV1 gE1, the Fc receptor or immunogenic fragment thereof is not administered to the subject together with HSV2 gD2 or HSV1 gD1 (respectively), or a fragment thereof comprising immunodominant regions and/or .
iii) said Fc receptor is administered to the subject together with HSV2 gC2 or an immunodominant fragment thereof, or HSV1 gC1 or an immunogenic fragment thereof.

**14.** The HSV1 or HSV2 Fc receptor or immunogenic fragment or variant thereof for use according to any one of claims 1-8 or 10-13 wherein the Fc receptor or immunogenic fragment or variant thereof is a recombinant Fc receptor or immunogenic fragment or variant thereof and the ability of said Fc receptor or immunogenic fragment or variant thereof to bind to a human antibody Fc domain is reduced or abolished compared to the corresponding native viral Fc receptor.

**15.** The HSV1 or HSV2 Fc receptor or immunogenic fragment or variant thereof for use according to any one of claims 1, 3-6, 8-10 or 12, wherein:

(i) the Fc receptor or immunogenic fragment or variant thereof is a recombinant Fc receptor or immunogenic fragment or variant thereof;
(ii) the ability of said Fc receptor or immunogenic fragment or variant thereof to bind to a human antibody Fc domain is reduced or abolished compared to the corresponding native viral Fc receptor;
(iii) said recombinant Fc receptor or immunogenic fragment or variant thereof is a HSV2 gE2 or immunogenic fragment thereof and
(iv) said HSV2 gE2 or immunogenic fragment thereof comprises a mutation or a combination of mutations with respect to the sequence shown in SEQ ID NO: 1 selected from 289_insert ADIGL; 338_insert ARAA; H245K; P317R; P319R; P319G; P319K; H245A_P319R; H245A P319G; H245AP319K; H245A P319T; P319D; S338D; R320D; N241A_R320D; A248K_V340M; P318Y; A248K_V340R; A248T_V340W; A248K_V340W; A246W_R320G; A246W_P317K; A246W_R320D; A246W_R320T; V340W; A248G_V340W; H245G_R320D; P318D; A246W_P317F; P319G_V340W; A248T_V340M; P317K_V340W; V340F; V340D; H245A_R320D; P317F_V340W; A246W_P317S; H245S_R320D; R314G_P318D; A248T; P318S; P317K; P317S_V340W; H245D; R314P_V340W; R314L_318D; P319L_V340W; P317F; P318D_S338G; R314G_V340W; P317K_S338H; R314L_V340W; P318R; P318Q; P317F_S338G; R314G P318I; H245G P319G; P317L; P318I; A248T F322A; H245E; P318T; P318R_S338G; P318D_S338H; P317F_S338H; A248T V340R; A248T_F322I; H245A_R320G; P318R S338H; H245S_R320G; P317K S338G; A248T F322P; V340R; R314LP318R; H245S R320T; R314G P318R; R320E; H245G_R320G; H245A_R320T; A246W; P318I_S338G; P317K V340M; P317I; R320H; R314P P318I; P318I_S338H; P317F V340M; H245A P319G; H245A_P319L; R320P; H245G R320T; R314L V340R; P319G V340R; R314G F322I; R314L P318I; R320A; R314N; P317F_V340R; P318D_S338L; A248G_V340R; R314E; R314P_P318D; H245S_P319G; V340Q; A248K_F322I; R320G; H245S_P319L; R314F; P319L; P317K_S338L; P319L_V340M; P317G; R320S; R320Q; R314P_V340R; V340A; H245G_P319L; R320T; R314P P318R; A248G F322I; R320N; P317N; R314D; R314Y; R314P_F322I; P319G V340M; P317S_V340R; R314V; P317R_P319D; P317R_R320D; P319D_R320D; Δ319_Δ320; P317G_P318G_Δ319 Δ320; P318E_ Δ319_Δ320; P318G_Δ319_Δ320; P318K_Δ319_Δ320; P317R_P318E_Δ319_320; P317R_P318G_Δ319_Δ320 and P317G_P318K_Δ319_Δ320.

**16.** The HSV1 or HSV2 Fc receptor or immunogenic fragment or variant thereof for use according to any one of claims 2-5, 7, 8, 11 or 13 wherein:

(i) the Fc receptor or immunogenic fragment or variant thereof is a recombinant Fc receptor or immunogenic fragment or variant thereof;
(ii) the ability of said Fc receptor or immunogenic fragment or variant thereof to bind to a human antibody Fc domain is reduced or abolished compared to the corresponding native viral Fc receptor;
(iii) said recombinant Fc receptor or immunogenic fragment or variant thereof is a HSV1 gE2 or immunogenic fragment thereof and

(iv) said HSV1 gE2 or immunogenic fragment thereof comprises a mutation or a combination of mutations with respect to the sequence shown in SEQ ID NO: 3 selected from H247A, H247K, P319R, P321A, P321R, P321G, P321K, P321T, A339G, P321D, P321S, A340D, N243A and R322D, H247A/P321A, H247A/P321R, H247A/P321G, H247A/P321K, H247A/P321T, N243A/R322D, N243A/P321D, H247G/P319G, P319G/P321G, A340G/S341G/V342G.

**17.** A pharmaceutical composition comprising a HSV2 Fc receptor or an immunogenic fragment or variant thereof, a binding partner from HSV2 or a fragment thereof, and a pharmaceutically acceptable carrier, for use in inducing a cross reactive immune response against HSV1 when administered to a subject.

**18.** A pharmaceutical composition comprising a HSV1 Fc receptor or an immunogenic fragment or variant thereof, a binding partner from HSV1 or a fragment thereof, and a pharmaceutically acceptable carrier, for use in inducing a cross reactive immune response against HSV2 when administered to a subject.

**19.** A method of treating a herpes virus infection or herpes virus related disease in a subject in need thereof comprising administering an immunologically effective amount of a HSV2 Fc receptor or immunogenic fragment or variant thereof to the subject wherein the HSV2 Fc receptor or immunogenic fragment or variant thereof can induce a cross reactive immune response against HSV1 when administered to a subject.

**20.** A method of treating a herpes virus infection or herpes virus related disease in a subject in need thereof comprising administering an immunologically effective amount of a HSV1 Fc receptor or immunogenic fragment or variant thereof to the subject wherein the HSV1 Fc receptor or immunogenic fragment or variant thereof can induce a cross reactive immune response against HSV2 when administered to a subject.

FIG. 1A

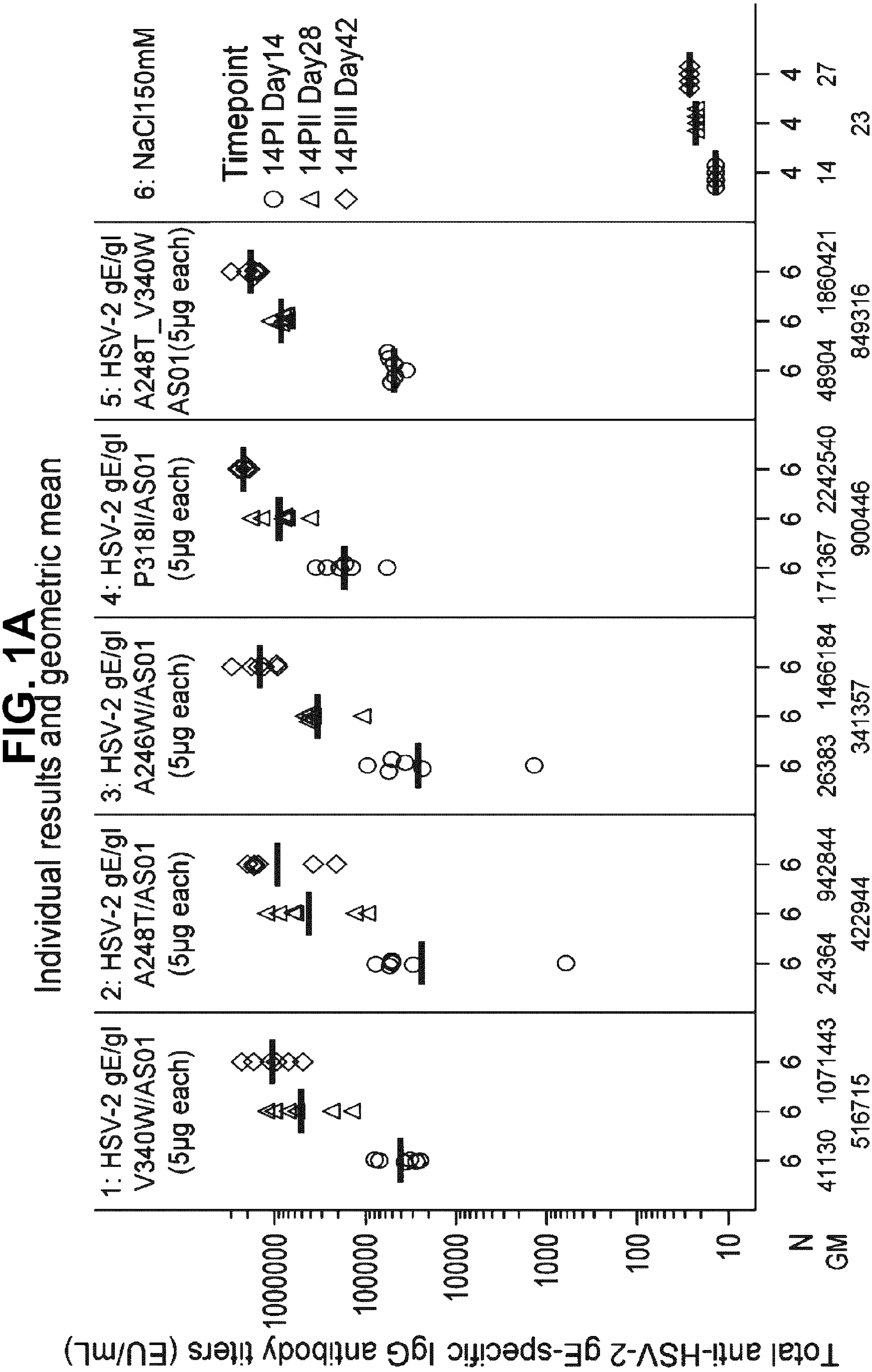
Individual results and geometric mean
Total anti-HSV-2 gE-specific IgG antibody titers (EU/mL)
1000000
100000
10000
1000
100
10
N
GM
1: HSV-2 gE/gI V340W/AS01 (5µg each)
2: HSV-2 gE/gI A248T/AS01 (5µg each)
3: HSV-2 gE/gI A246W/AS01 (5µg each)
4: HSV-2 gE/gI P318I/AS01 (5µg each)
5: HSV-2 gE/gI A248T_V340W AS01(5µg each)
6: NaCl150mM
Timepoint
14PI Day14
14PII Day28
14PIII Day42
6 6 6 6 6 6 6 6 6 6 6 6 6 6 6 4 4 4
41130 516715 1071443 24364 422944 942844 26383 341357 1466184 171367 900446 2242540 48904 849316 1860421 14 23 27

FIG. 1B

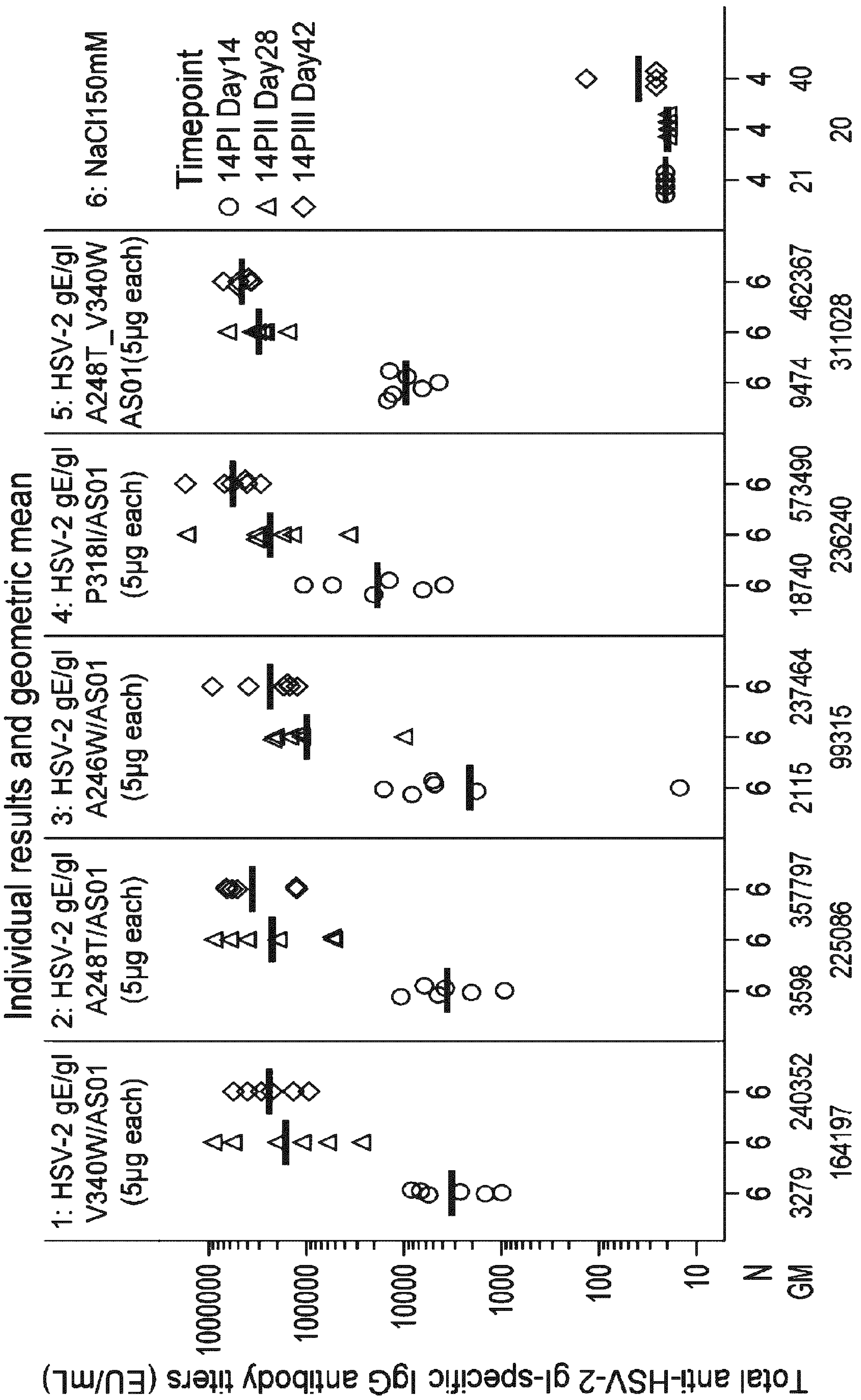
Individual results and geometric mean
Total anti-HSV-2 gI-specific IgG antibody titers (EU/mL)
1000000
100000
10000
1000
100
10
N
GM
1: HSV-2 gE/gI V340W/AS01 (5µg each)
2: HSV-2 gE/gI A248T/AS01 (5µg each)
3: HSV-2 gE/gI A246W/AS01 (5µg each)
4: HSV-2 gE/gI P318I/AS01 (5µg each)
5: HSV-2 gE/gI A248T_V340W AS01(5µg each)
6: NaCl150mM
Timepoint
14PI Day14
14PII Day28
14PIII Day42
6 6 6
3279 164197 240352
6 6 6
3598 225086 357797
6 6 6
2115 99315 237464
6 6 6
18740 236240 573490
6 6 6
9474 311028 462367
4 4 4
21 20 40

# FIG. 2A

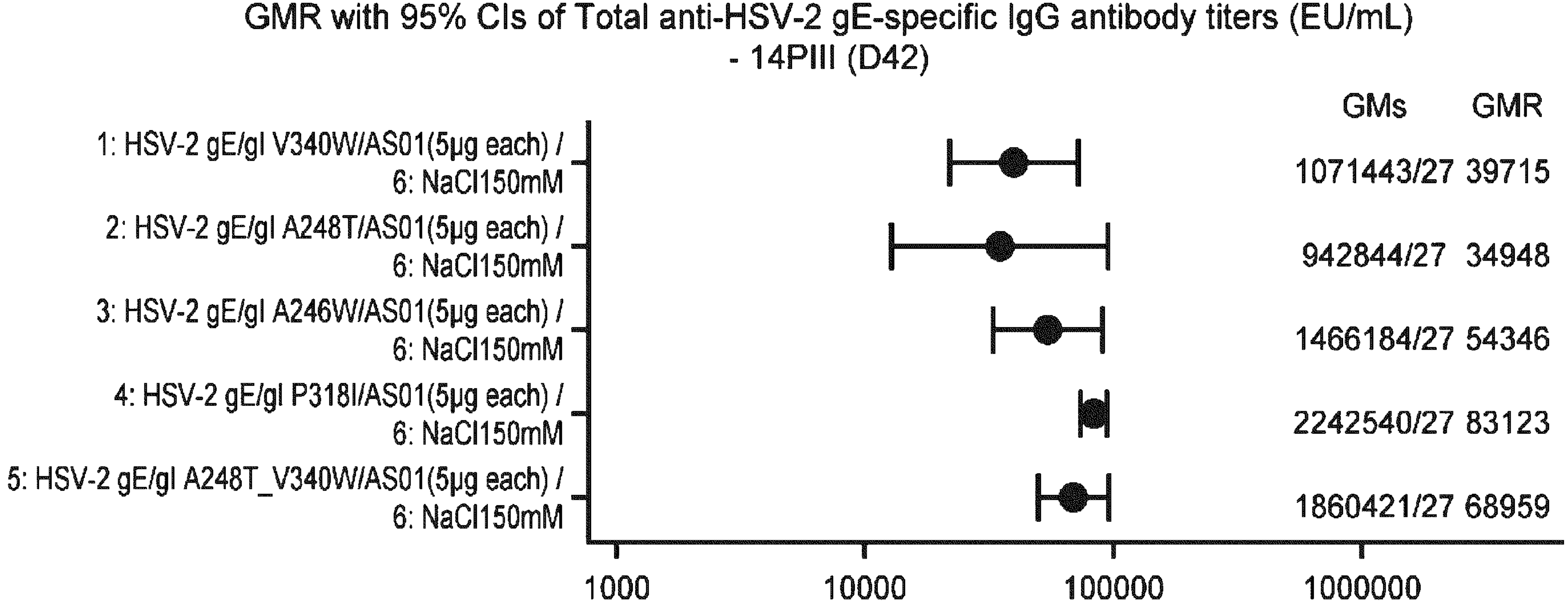
GMR with 95% CIs of Total anti-HSV-2 gE-specific IgG antibody titers (EU/mL)
- 14PIII (D42)
GMs GMR
1: HSV-2 gE/gI V340W/AS01(5µg each) / 6: NaCl150mM
1071443/27 39715
2: HSV-2 gE/gI A248T/AS01(5µg each) / 6: NaCl150mM
942844/27 34948
3: HSV-2 gE/gI A246W/AS01(5µg each) / 6: NaCl150mM
1466184/27 54346
4: HSV-2 gE/gI P318I/AS01(5µg each) / 6: NaCl150mM
2242540/27 83123
5: HSV-2 gE/gI A248T_V340W/AS01(5µg each) / 6: NaCl150mM
1860421/27 68959
1000
10000
100000
1000000

FIG. 2B

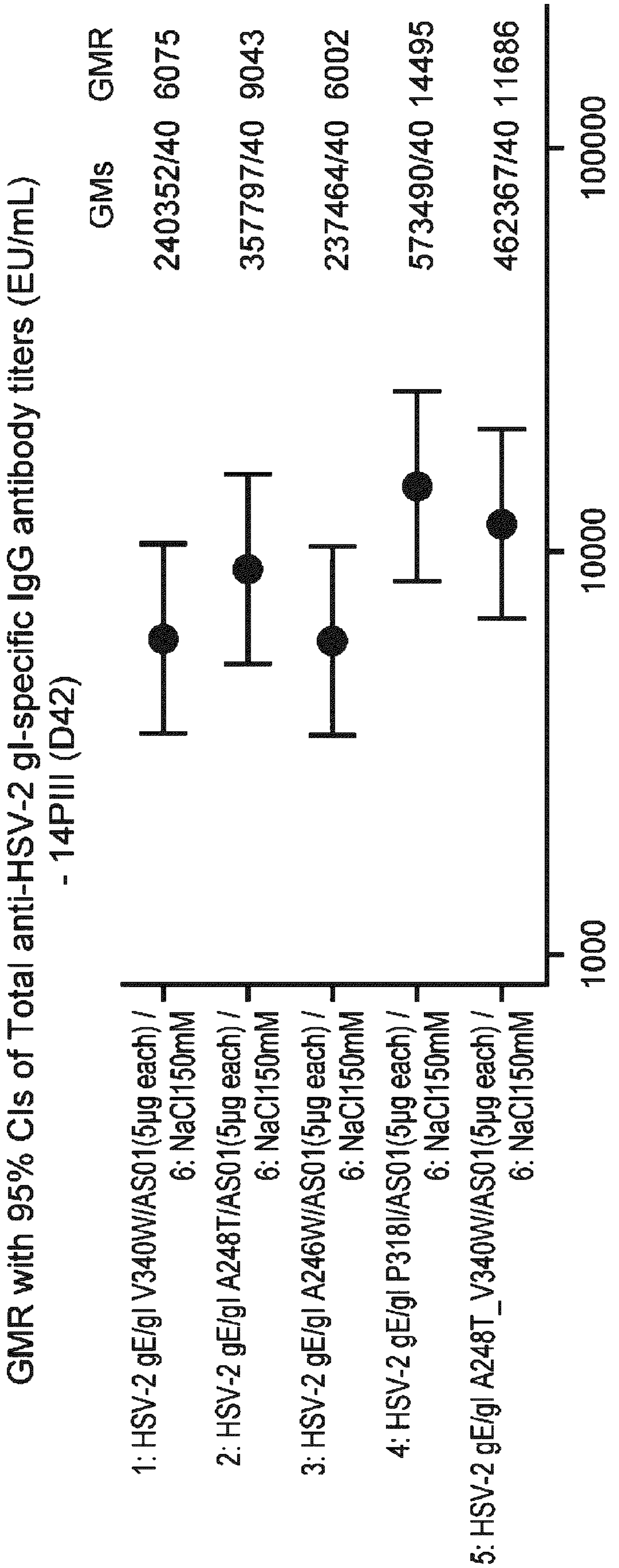
GMR with 95% CIs of Total anti-HSV-2 gI-specific IgG antibody titers (EU/mL)
- 14PIII (D42)
GMs
GMR
240352/40 6075
357797/40 9043
237464/40 6002
573490/40 14495
462367/40 11686
1: HSV-2 gE/gI V340W/AS01(5µg each) / 6: NaCl150mM
2: HSV-2 gE/gI A248T/AS01(5µg each) / 6: NaCl150mM
3: HSV-2 gE/gI A246W/AS01(5µg each) / 6: NaCl150mM
4: HSV-2 gE/gI P318I/AS01(5µg each) / 6: NaCl150mM
5: HSV-2 gE/gI A248T_V340W/AS01(5µg each) / 6: NaCl150mM
1000
10000
100000

FIG. 3A

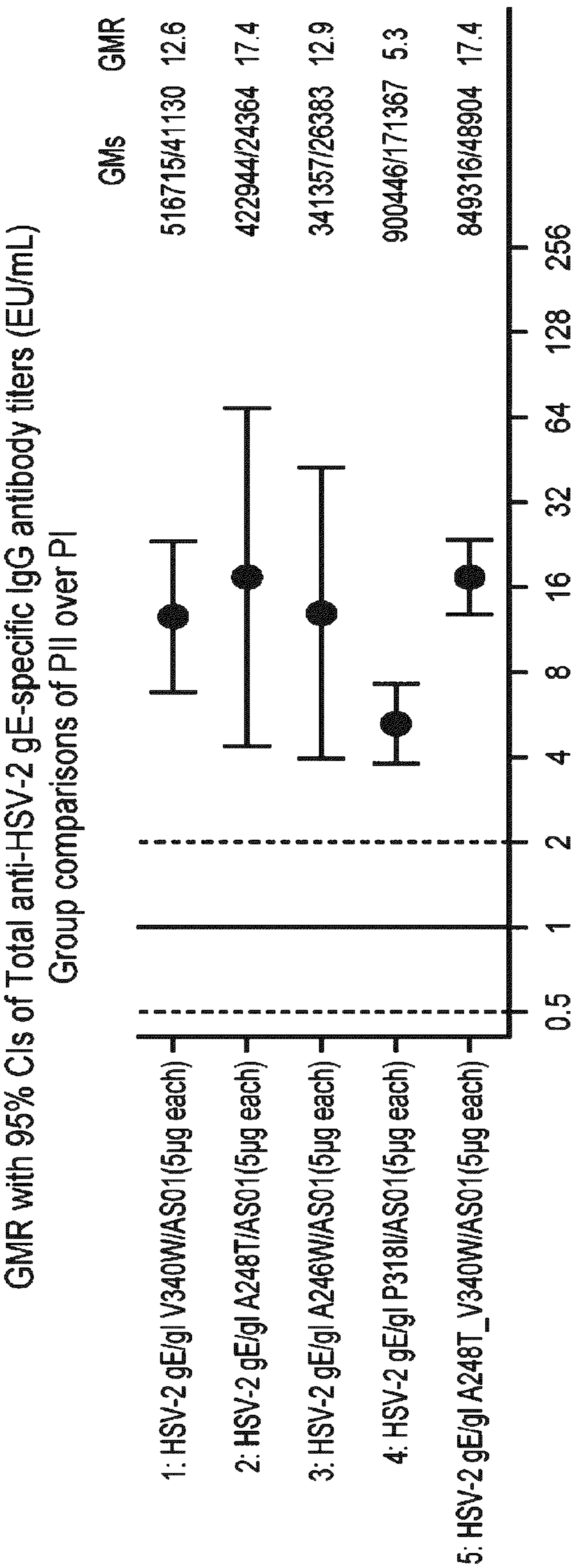
GMR with 95% CIs of Total anti-HSV-2 gE-specific IgG antibody titers (EU/mL)
Group comparisons of PII over PI
GMs
GMR
1: HSV-2 gE/gI V340W/AS01(5µg each)
516715/41130
12.6
2: HSV-2 gE/gI A248T/AS01(5µg each)
422944/24364
17.4
3: HSV-2 gE/gI A246W/AS01(5µg each)
341357/26383
12.9
4: HSV-2 gE/gI P318I/AS01(5µg each)
900446/171367
5.3
5: HSV-2 gE/gI A248T_V340W/AS01(5µg each)
849316/48904
17.4
0.5
1
2
4
8
16
32
64
128
256

FIG. 3B

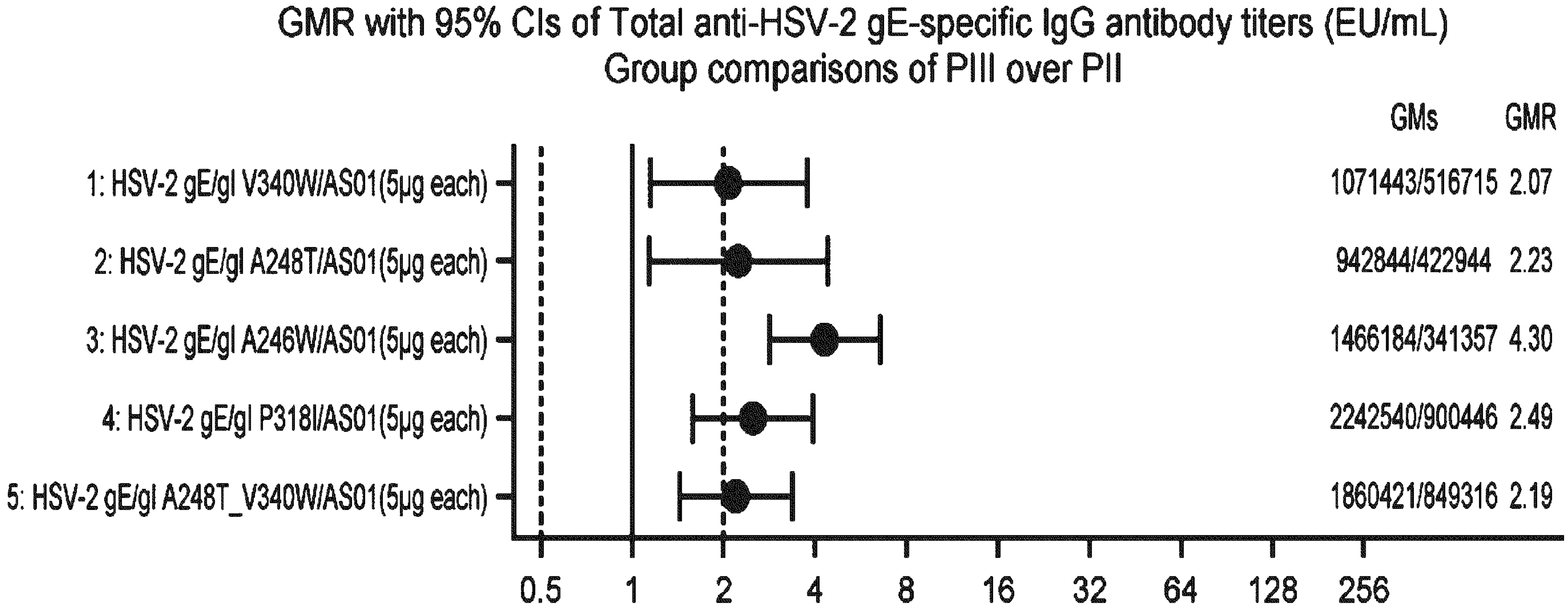
GMR with 95% CIs of Total anti-HSV-2 gE-specific IgG antibody titers (EU/mL)
Group comparisons of PIII over PII
GMs
GMR
1: HSV-2 gE/gI V340W/AS01(5µg each)
2: HSV-2 gE/gI A248T/AS01(5µg each)
3: HSV-2 gE/gI A246W/AS01(5µg each)
4: HSV-2 gE/gI P318I/AS01(5µg each)
5: HSV-2 gE/gI A248T_V340W/AS01(5µg each)
1071443/516715 2.07
942844/422944 2.23
1466184/341357 4.30
2242540/900446 2.49
1860421/849316 2.19
0.5
1
2
4
8
16
32
64
128
256

FIG. 3C

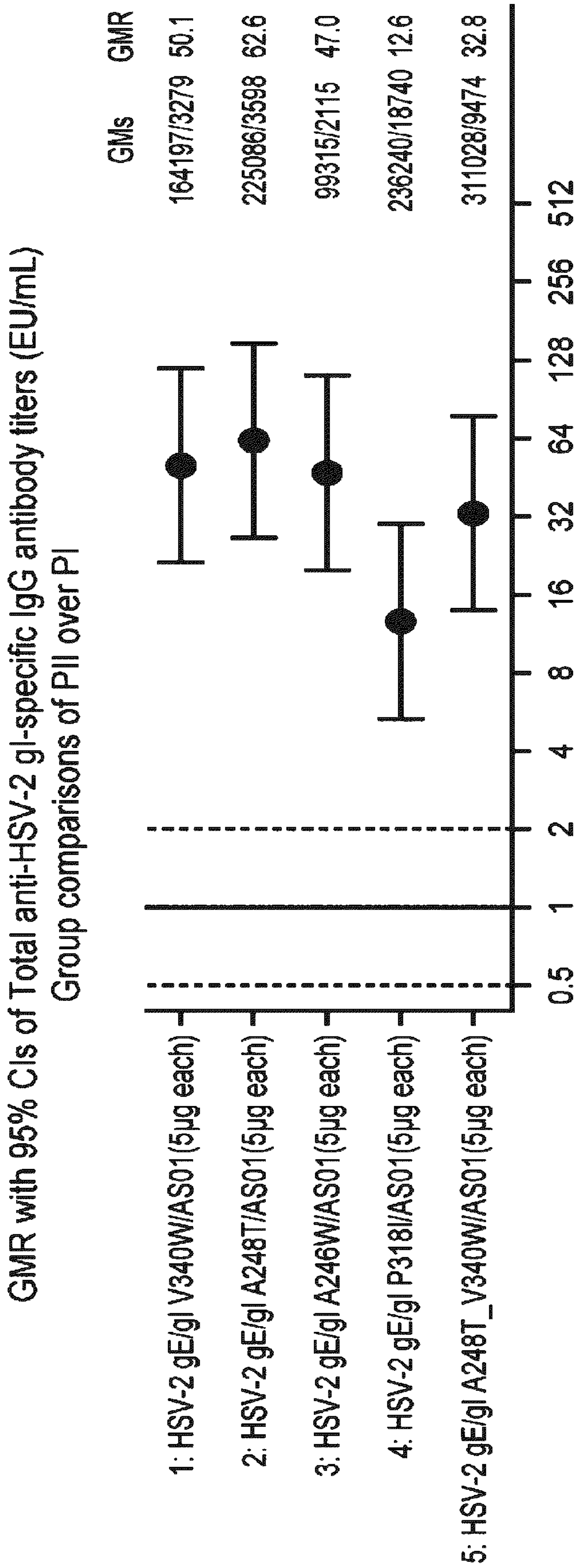
GMR with 95% CIs of Total anti-HSV-2 gI-specific IgG antibody titers (EU/mL)
Group comparisons of PII over PI
GMs
GMR
1: HSV-2 gE/gI V340W/AS01(5µg each)
164197/3279
50.1
2: HSV-2 gE/gI A248T/AS01(5µg each)
225086/3598
62.6
3: HSV-2 gE/gI A246W/AS01(5µg each)
99315/2115
47.0
4: HSV-2 gE/gI P318I/AS01(5µg each)
236240/18740
12.6
5: HSV-2 gE/gI A248T_V340W/AS01(5µg each)
311028/9474
32.8
0.5
1
2
4
8
16
32
64
128
256
512

# FIG. 3D

GMR with 95% CIs of Total anti-HSV-2 gI-specific IgG antibody titers (EU/mL)
Group comparisons of PIII over PII

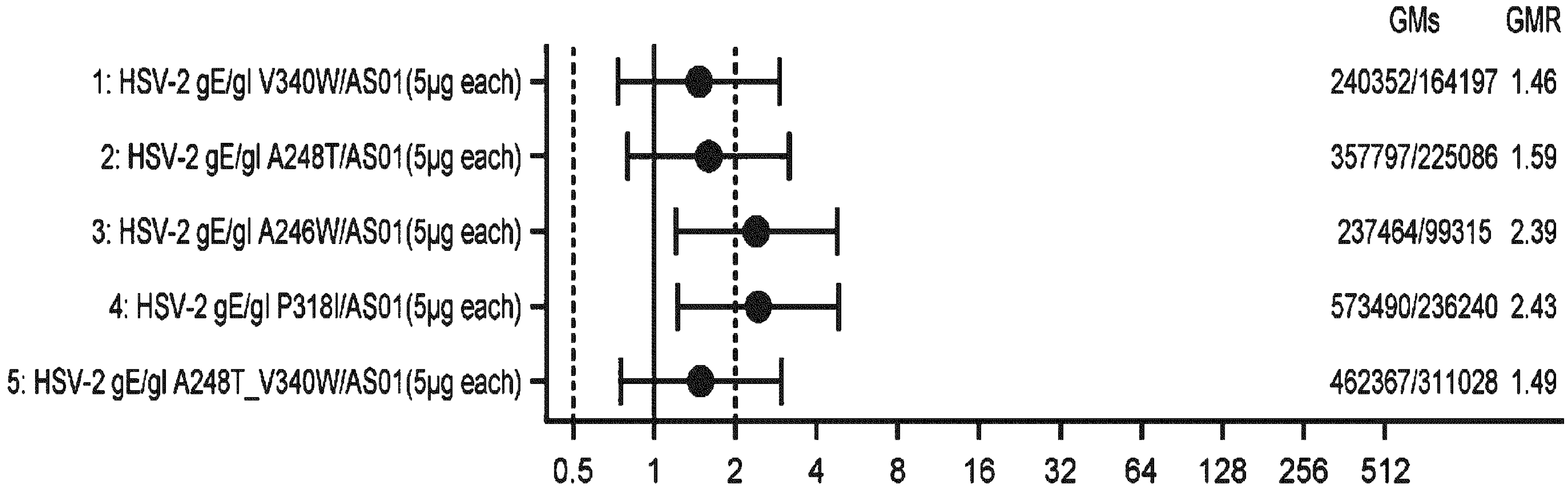

FIG. 4A

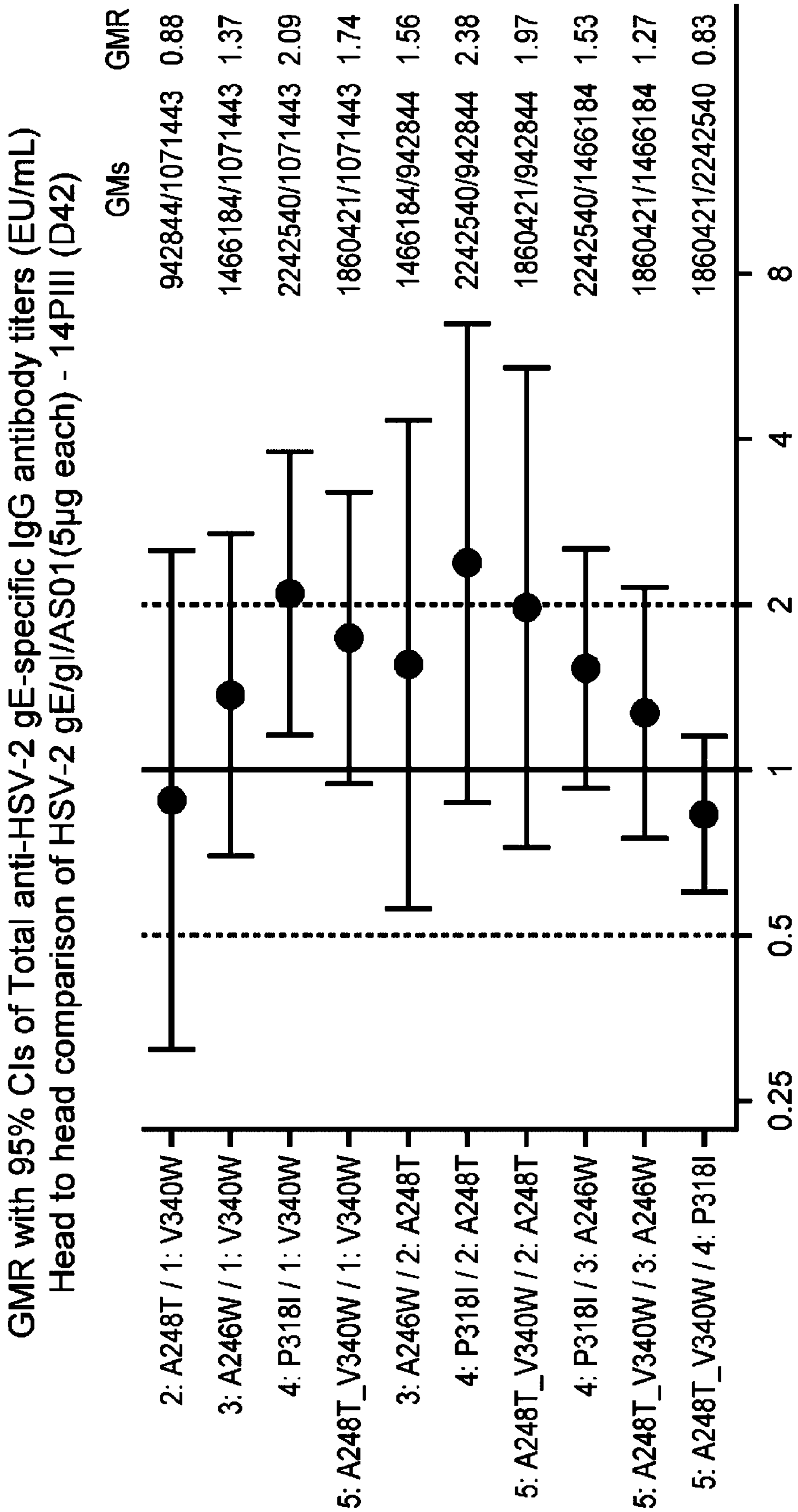
GMR with 95% CIs of Total anti-HSV-2 gE-specific IgG antibody titers (EU/mL)
Head to head comparison of HSV-2 gE/gI/AS01(5µg each) - 14PIII (D42)
GMs
GMR
2: A248T / 1: V340W
942844/1071443
0.88
3: A246W / 1: V340W
1466184/1071443
1.37
4: P318I / 1: V340W
2242540/1071443
2.09
5: A248T_V340W / 1: V340W
1860421/1071443
1.74
3: A246W / 2: A248T
1466184/942844
1.56
4: P318I / 2: A248T
2242540/942844
2.38
5: A248T_V340W / 2: A248T
1860421/942844
1.97
4: P318I / 3: A246W
2242540/1466184
1.53
5: A248T_V340W / 3: A246W
1860421/1466184
1.27
5: A248T_V340W / 4: P318I
1860421/2242540
0.83
0.25
0.5
1
2
4
8

FIG. 4B

GMR with 95% CIs of Total anti-HSV-2 gI-specific IgG antibody titers (EU/mL)
Head to head comparison of HSV-2 gE/gI/AS01(5µg each) - 14PIII (D42)

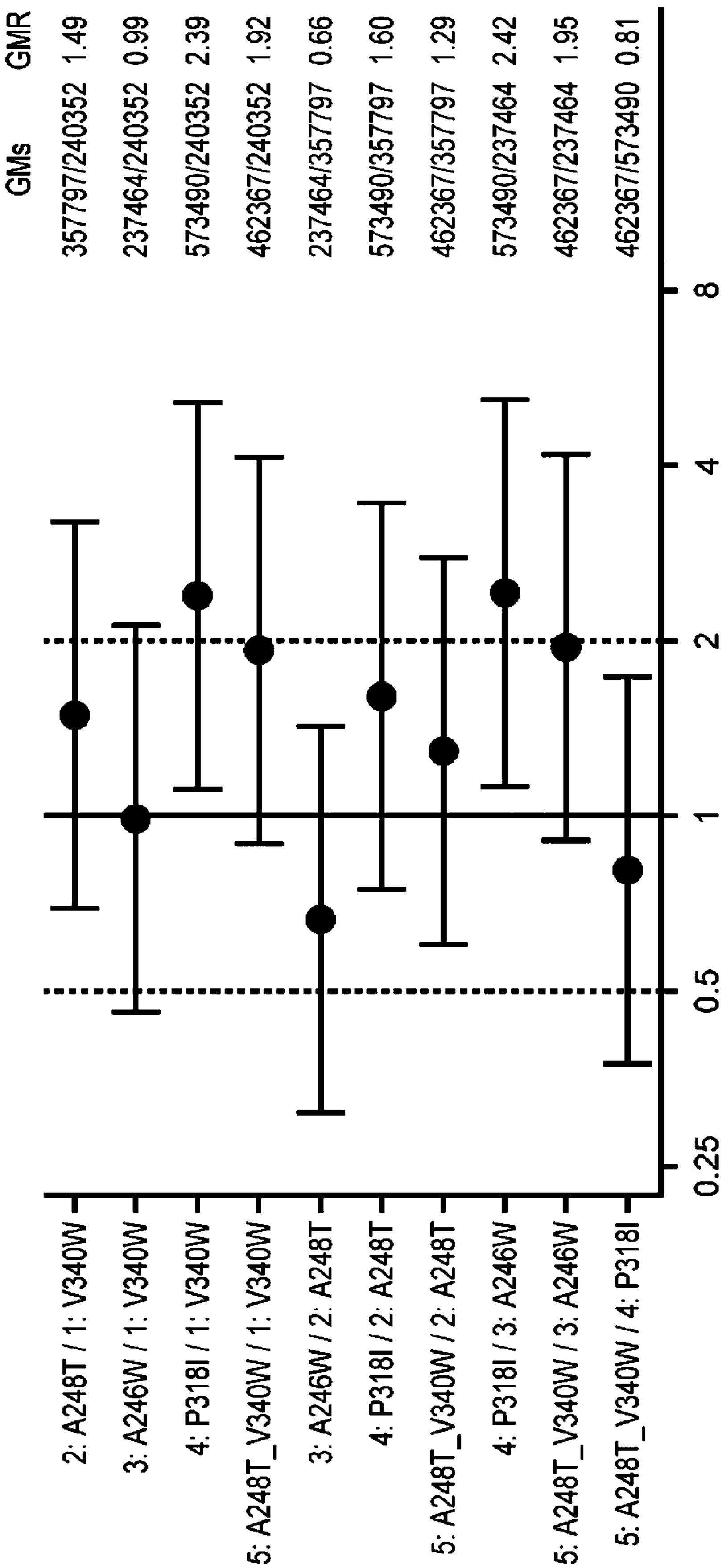

FIG. 5

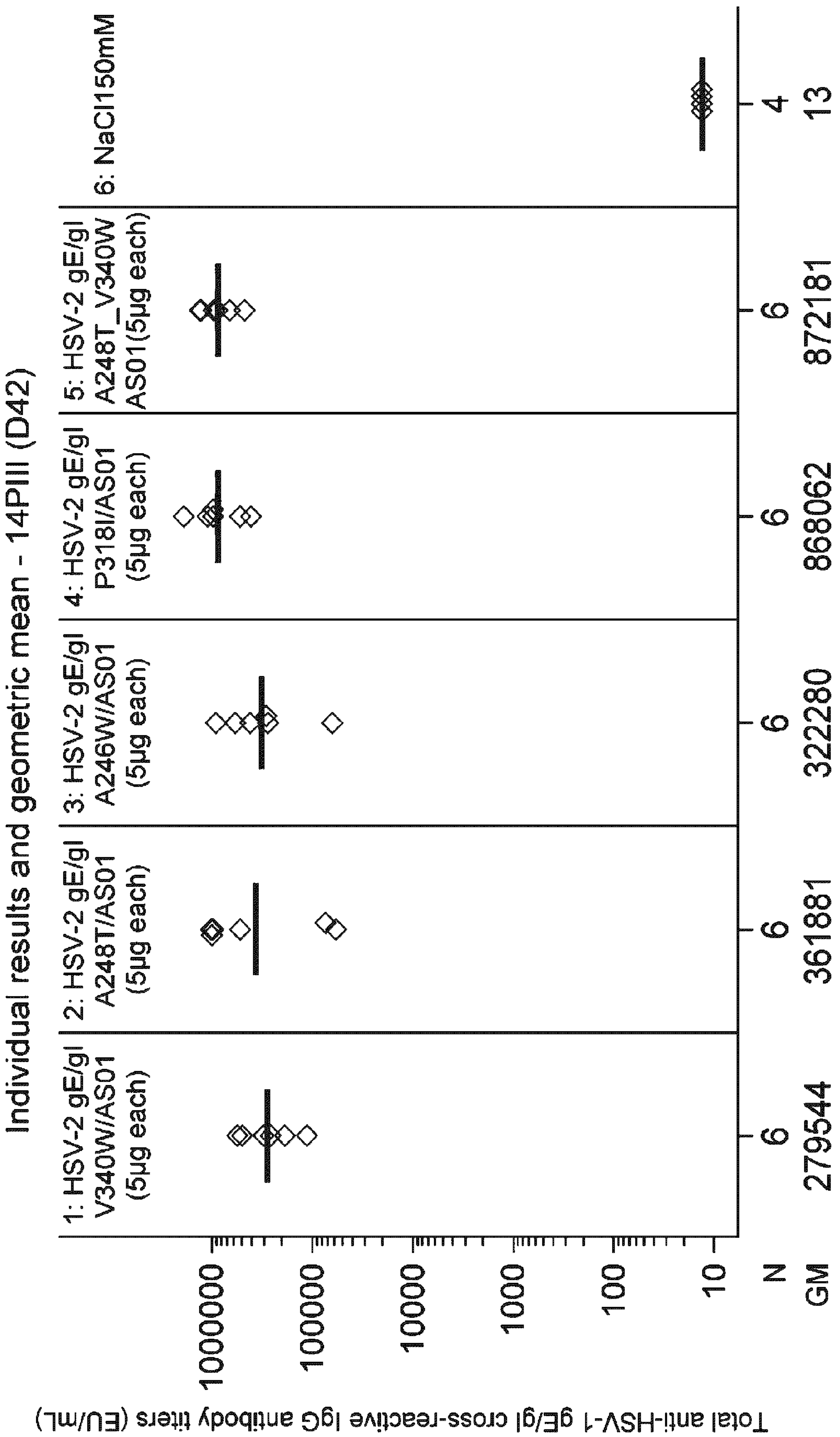
Individual results and geometric mean - 14PIII (D42)
1: HSV-2 gE/gI V340W/AS01 (5µg each)
2: HSV-2 gE/gI A248T/AS01 (5µg each)
3: HSV-2 gE/gI A246W/AS01 (5µg each)
4: HSV-2 gE/gI P318I/AS01 (5µg each)
5: HSV-2 gE/gI A248T_V340W AS01(5µg each)
6: NaCl150mM
1000000
100000
10000
1000
100
10
N
GM
6
279544
6
361881
6
322280
6
868062
6
872181
4
13
Total anti-HSV-1 gE/gI cross-reactive IgG antibody titers (EU/mL)

FIG. 6

GMR with 95% CIs of Total anti-HSV-1 gE/gI cross-reactive IgG antibody titers (EU/mL) - 14PIII (D42)

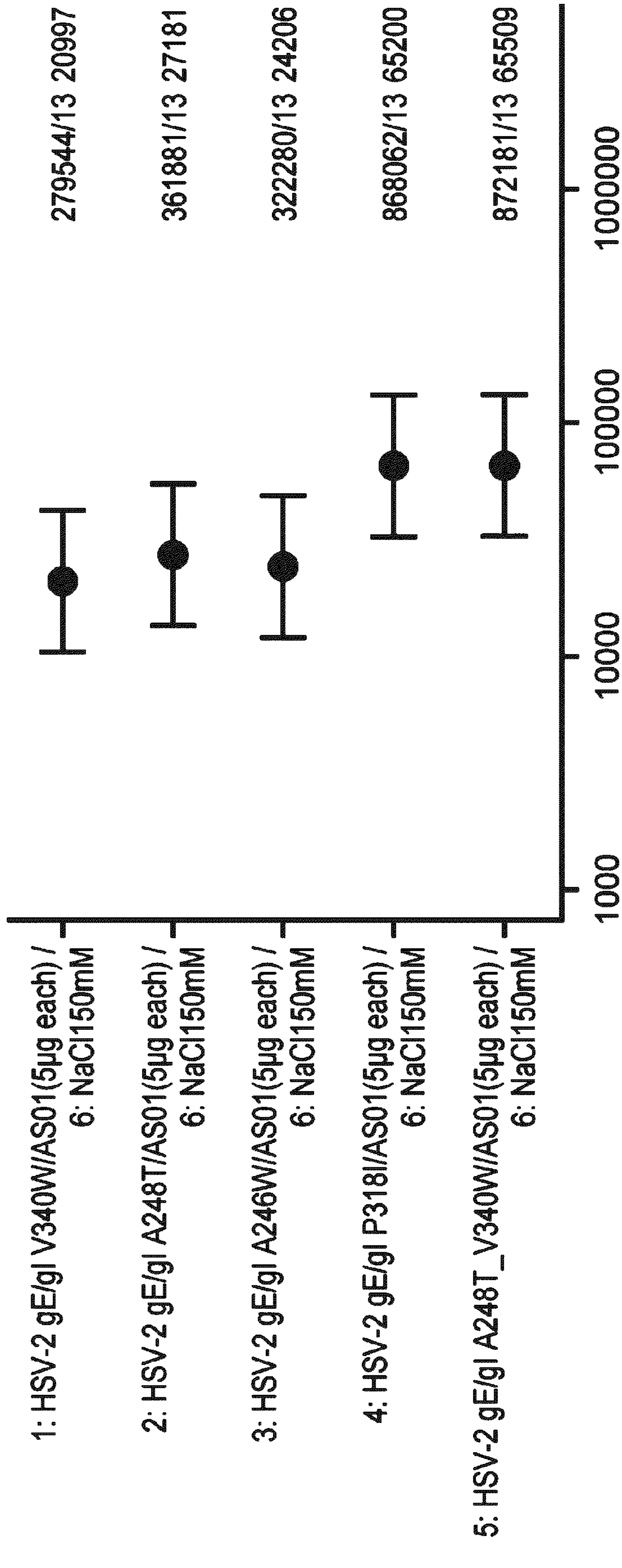

# FIG. 7

GMR with 95% CIs of Total anti-HSV-1 gE/gI cross-reactive IgG antibody titers (EU/mL)
Head to head comparison of HSV-2 gE/gI/AS01(5µg each) - 14PIII (D42)

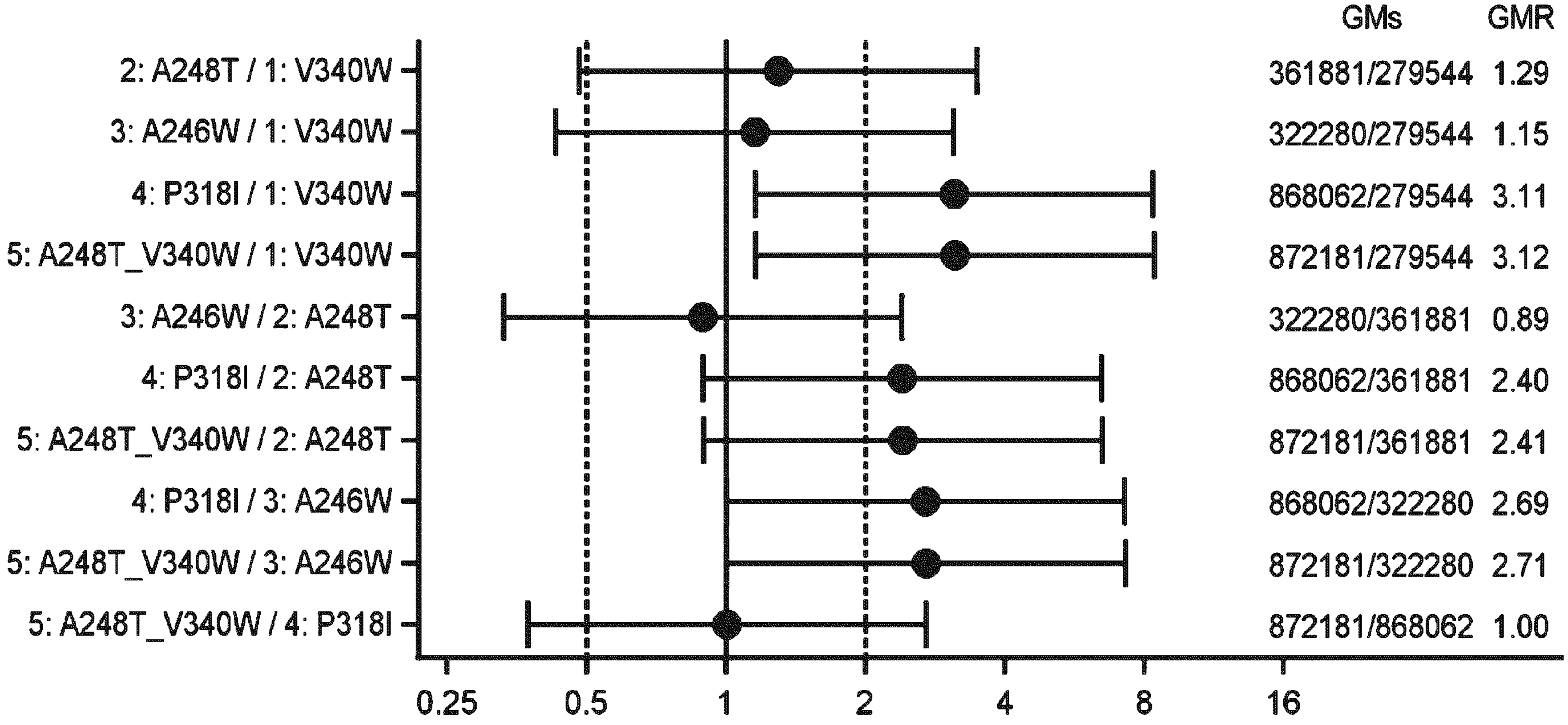

# FIG. 8A

**HSV-2-specific neutralizing antibody response induced 14 days after third immunization with different AS01-adjuvanted HSV-2 gE/gI mutant candidates**

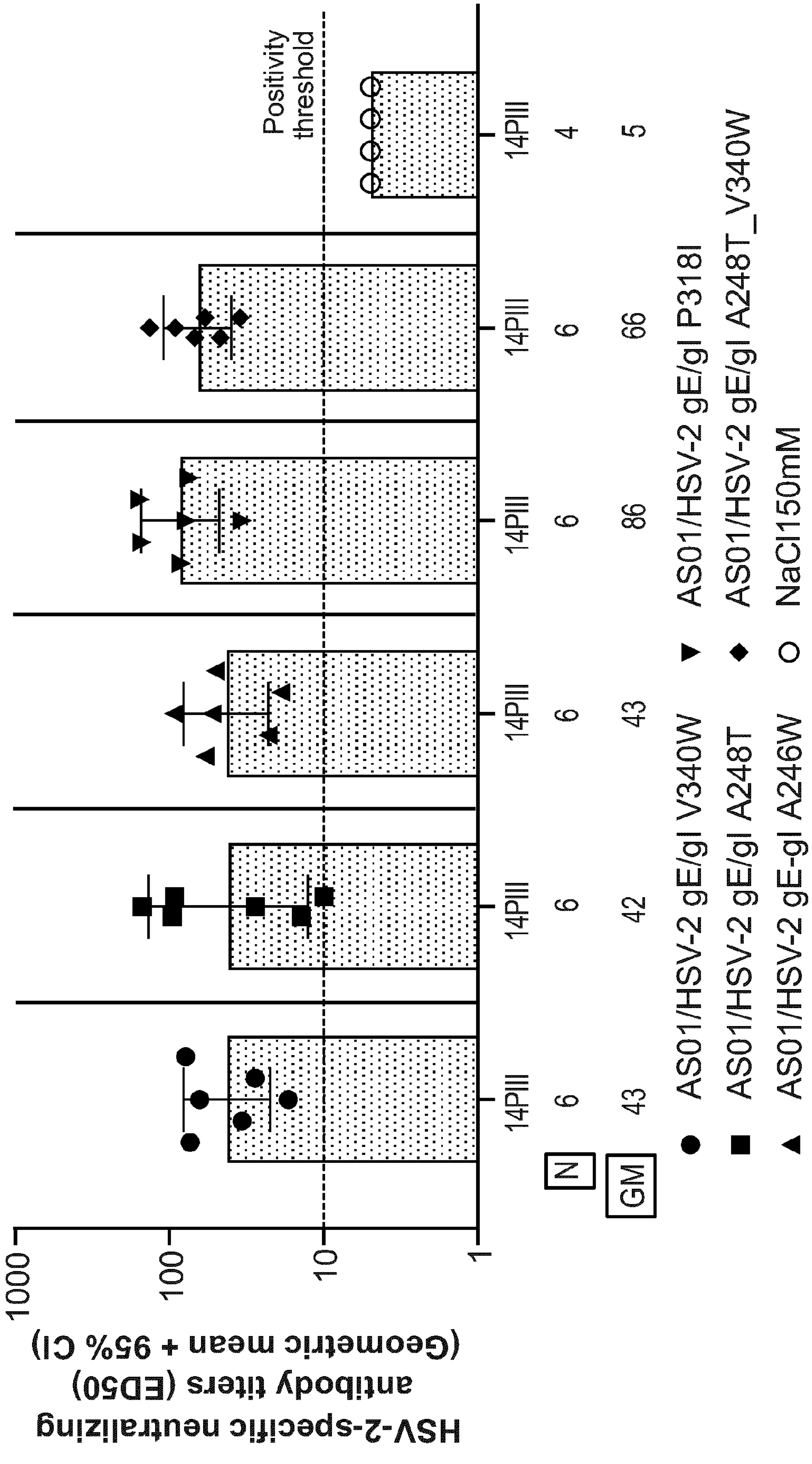

FIG. 8B

GMR with 95% CI of HSV-2-specific neutralizing antibody titers (ED50)
Head to head comparison of HSV-2 gE/gI/AS01(5µg each) - 14PIII (D42)

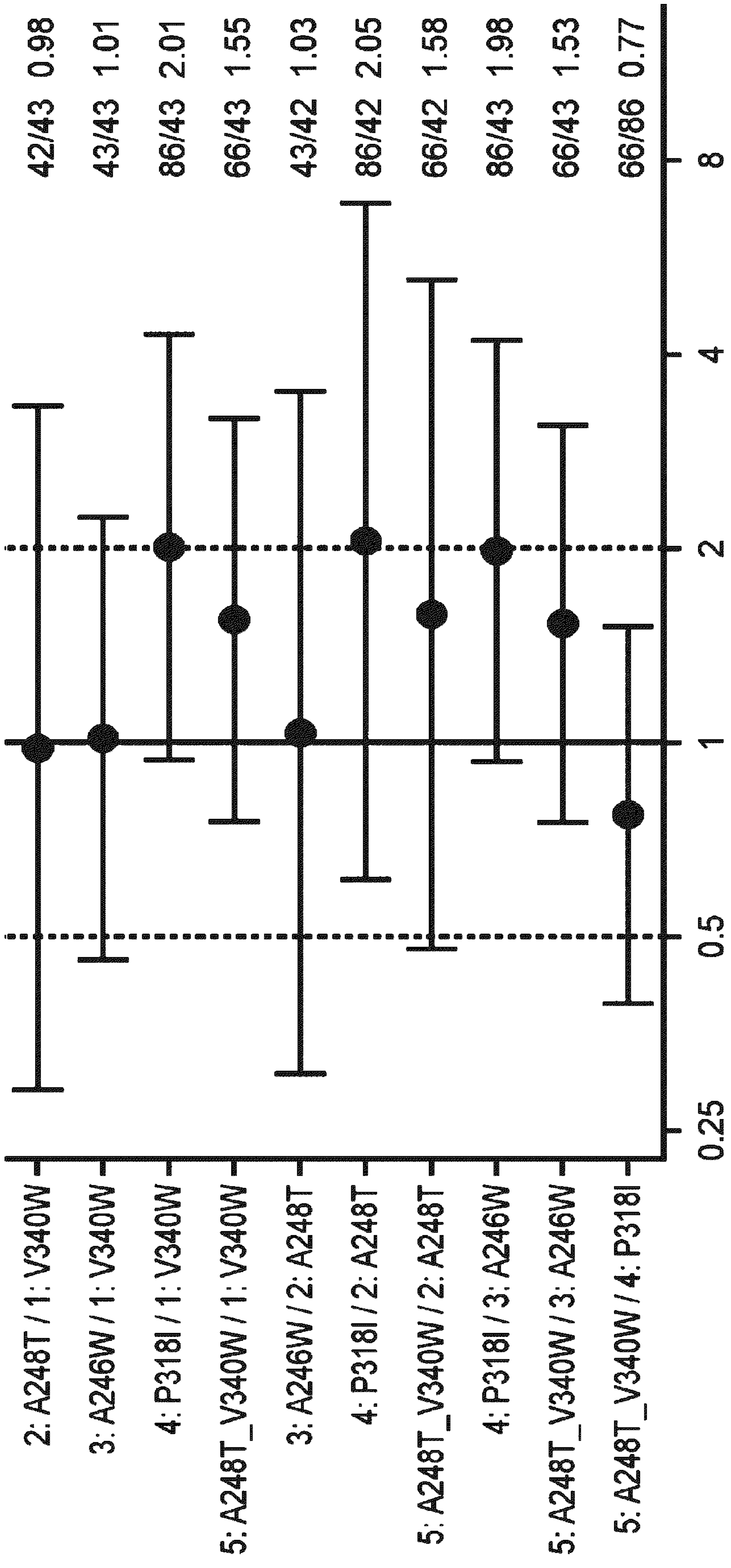

# FIG. 9A

**HSV-1-cross-reactive neutralizing antibody response induced 14 days after third immunization with different AS01-adjuvanted HSV-2 gE/gI mutant candidates**

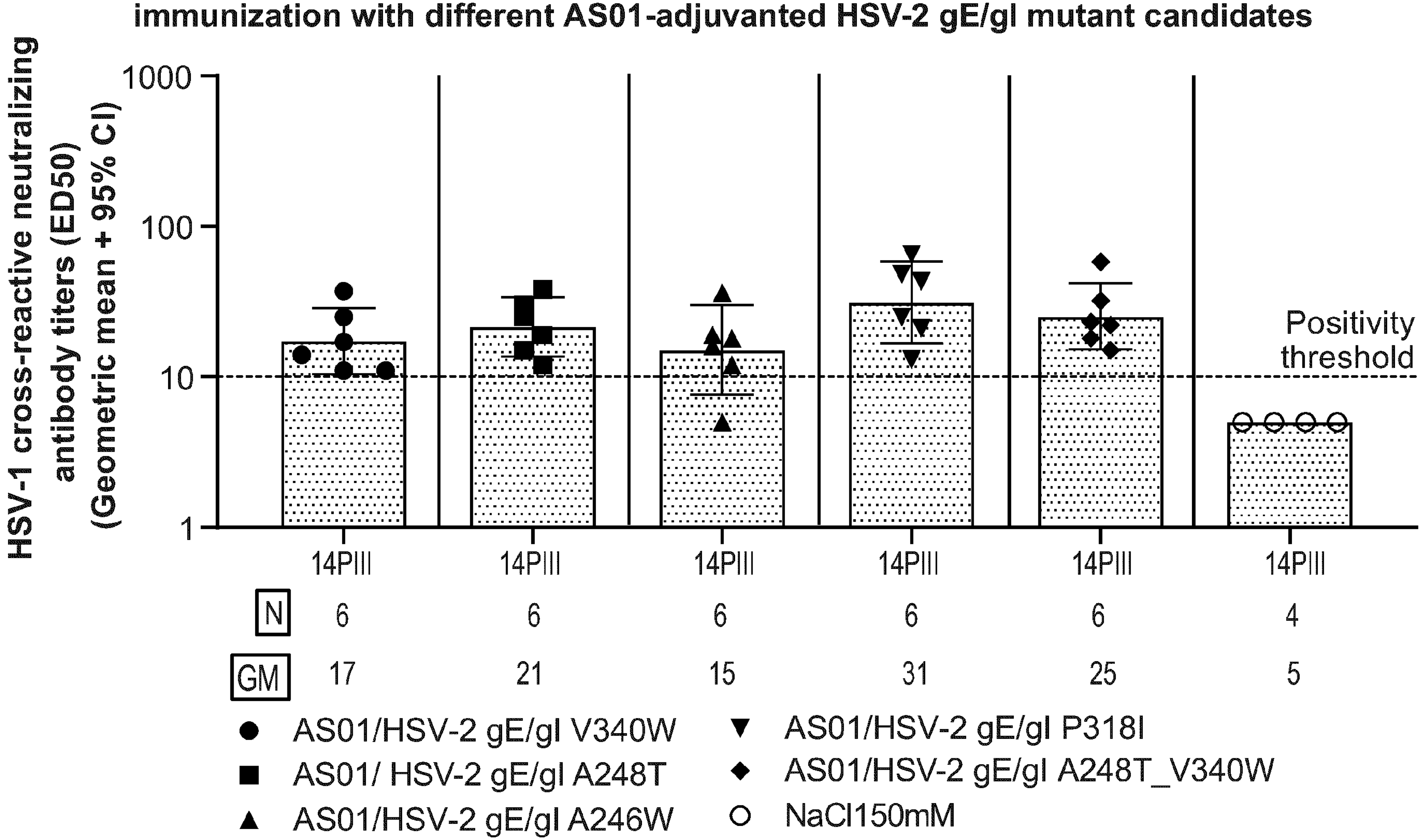

# FIG. 9B

GMR with 95% CI of HSV-1 cross-reactive neutralizing antibody titers (ED50)
Head to head comparison of HSV-2 gE/gI/AS01(5µg each) groups - 14PIII (Day42)

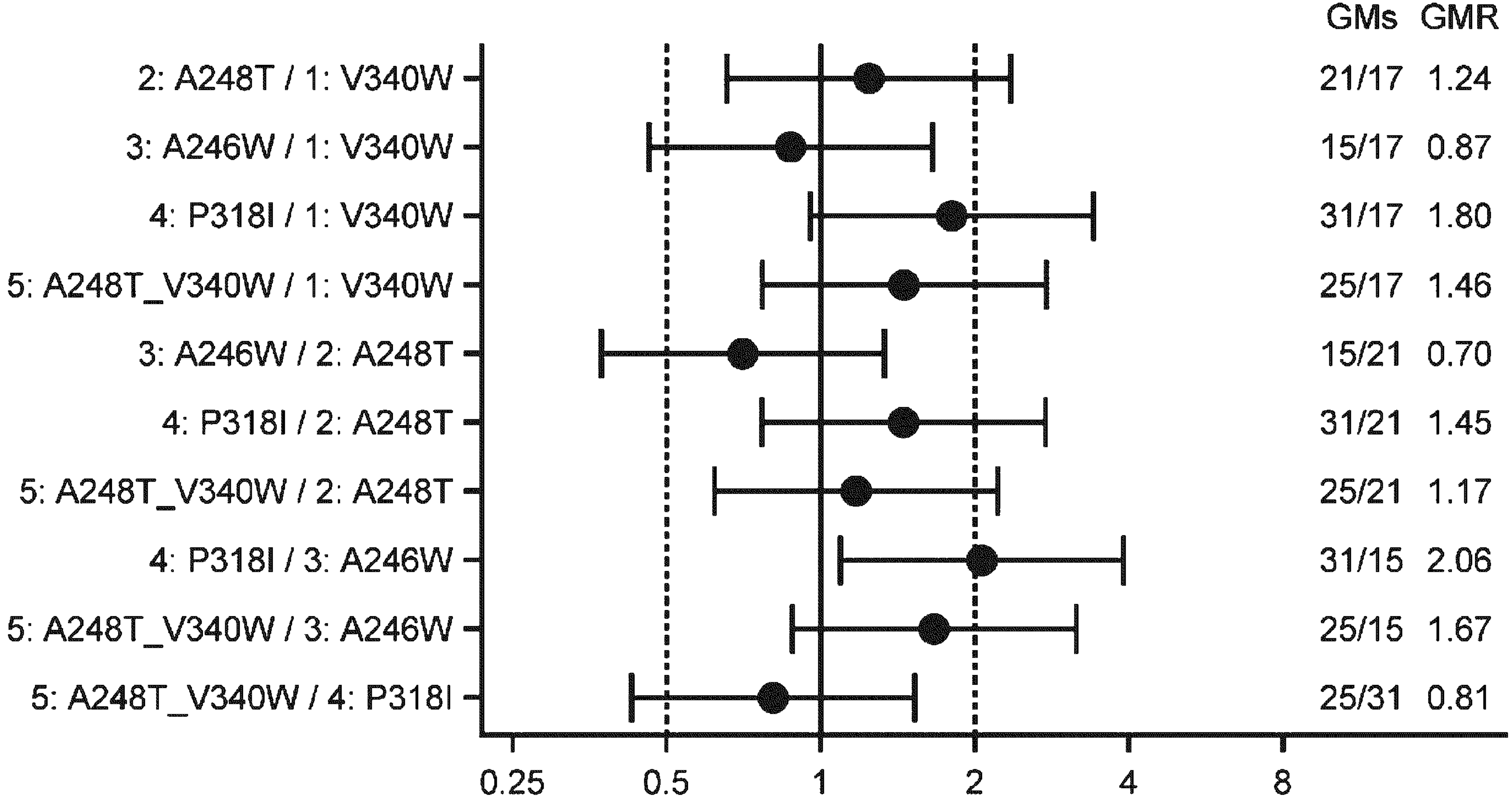

FIG. 10A

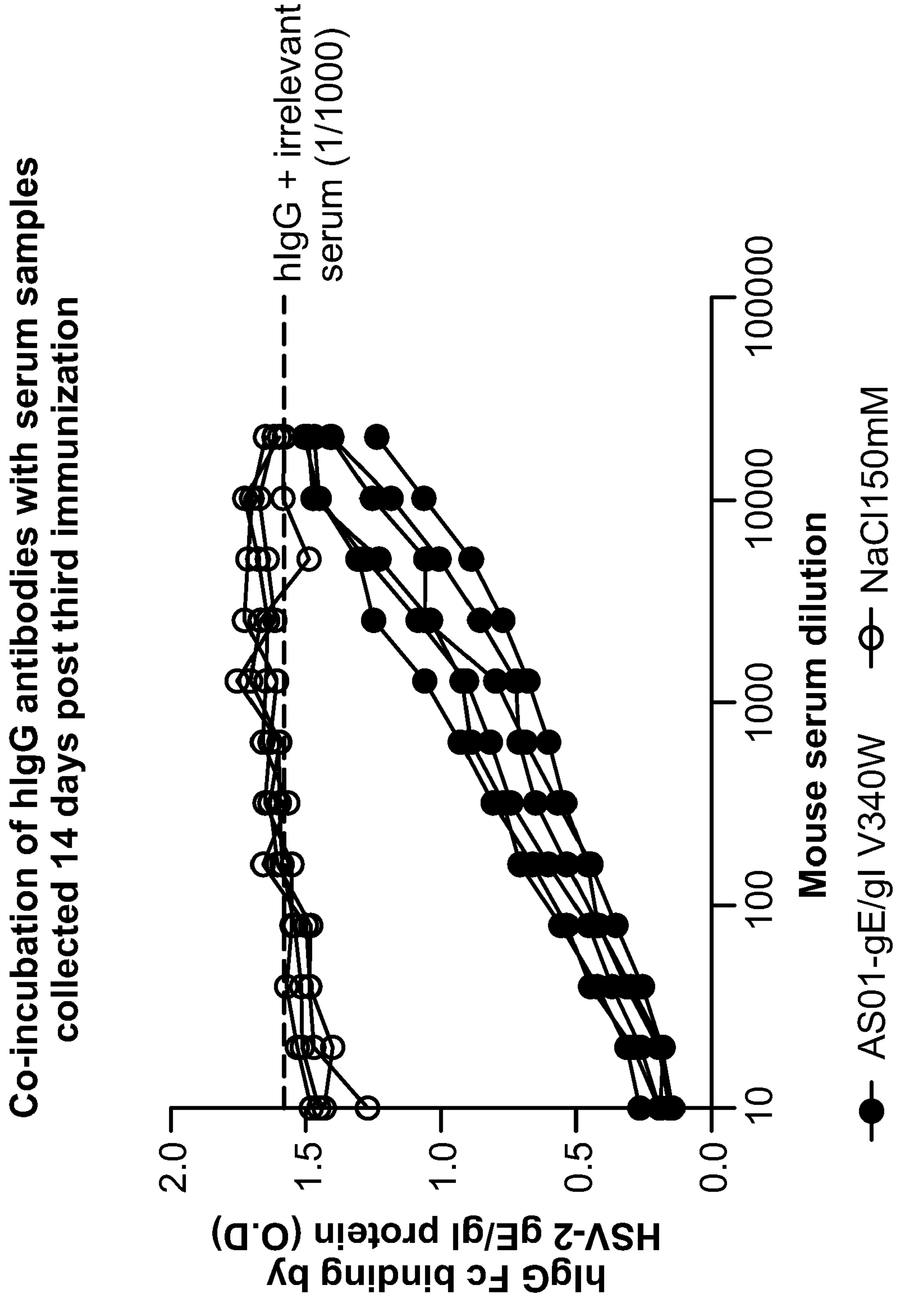
Co-incubation of hIgG antibodies with serum samples collected 14 days post third immunization
hIgG Fc binding by HSV-2 gE/gI protein (O.D)
2.0
1.5
1.0
0.5
0.0
10
100
1000
10000
100000
Mouse serum dilution
hIgG + irrelevant serum (1/1000)
AS01-gE/gI V340W
NaCl150mM

# FIG. 10B

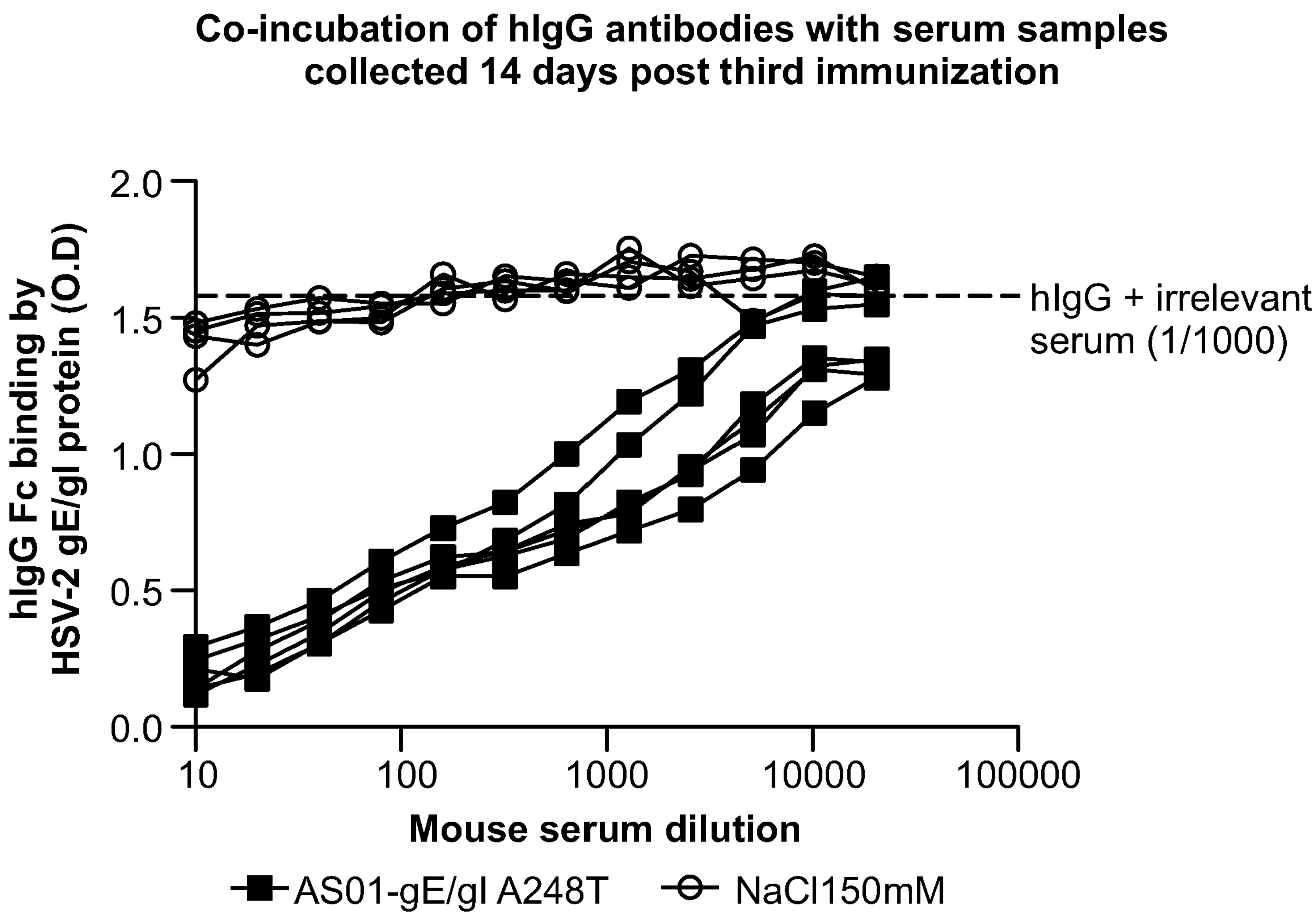
Co-incubation of hIgG antibodies with serum samples collected 14 days post third immunization
hIgG Fc binding by HSV-2 gE/gI protein (O.D)
2.0
1.5
1.0
0.5
0.0
10
100
1000
10000
100000
Mouse serum dilution
hIgG + irrelevant serum (1/1000)
AS01-gE/gI A248T
NaCl150mM

FIG. 10C

Co-incubation of hIgG antibodies with serum samples collected 14 days post third immunization

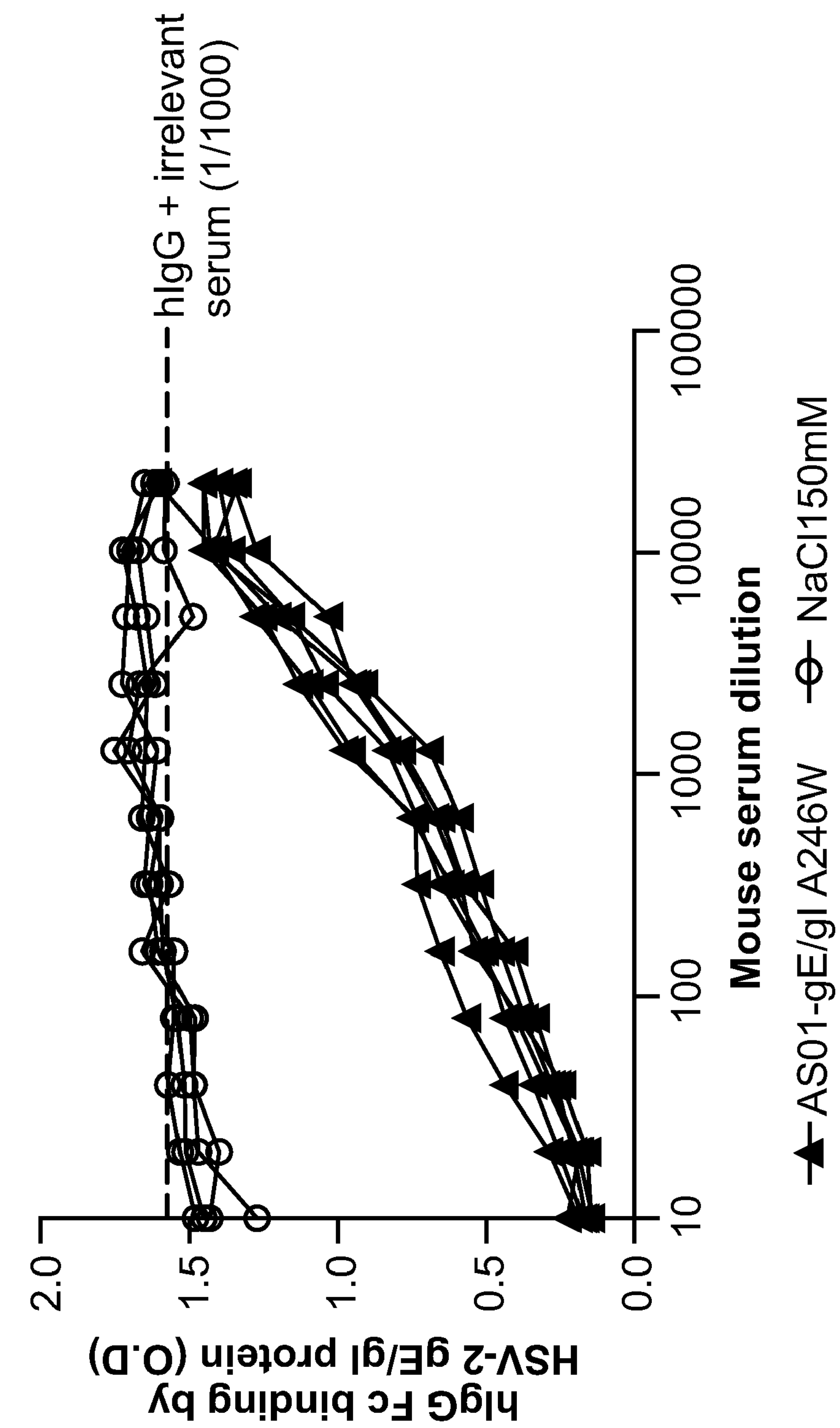

FIG. 10D

Co-incubation of hIgG antibodies with serum samples collected 14 days post third immunization

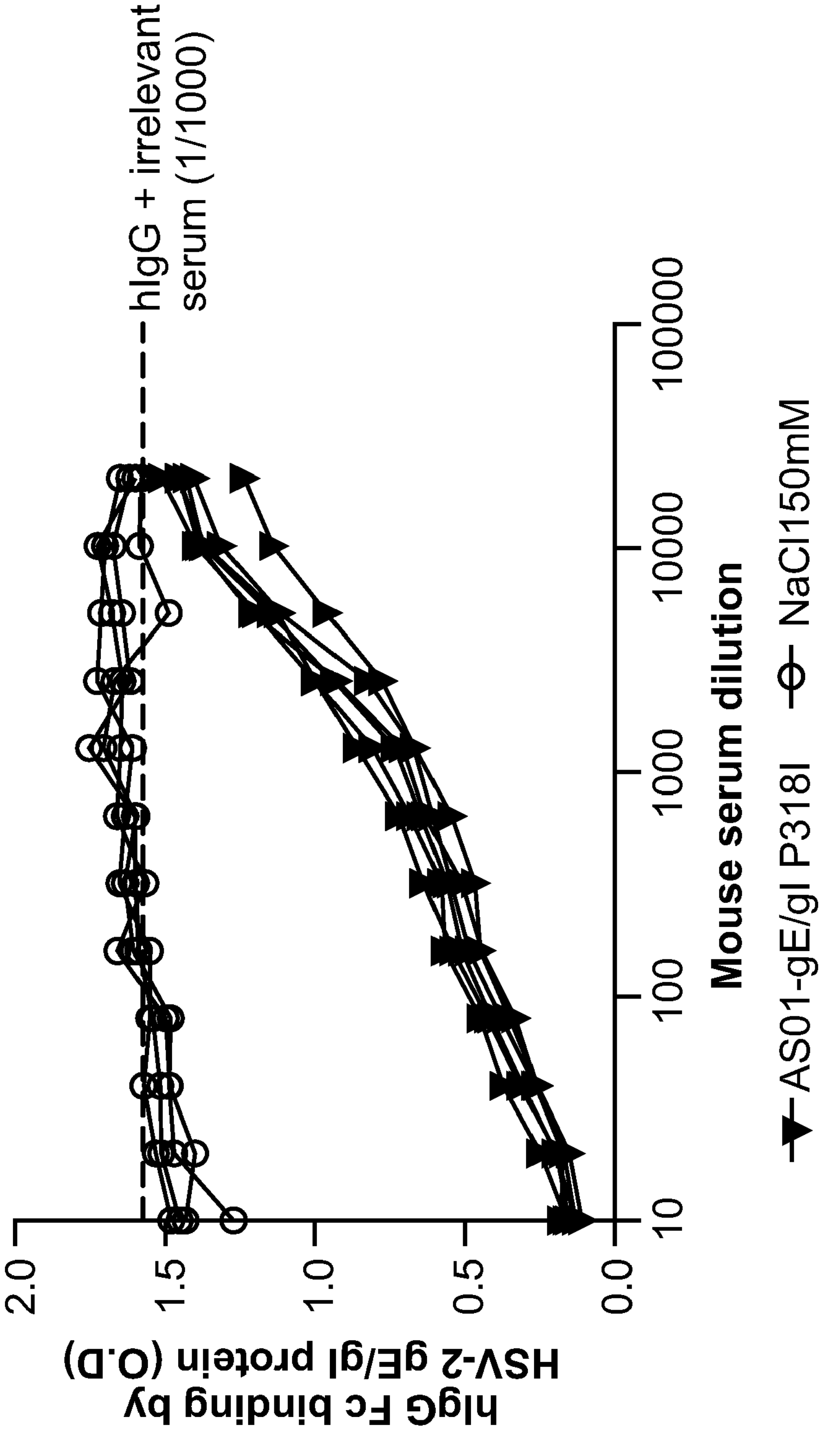

# FIG. 10E

**Co-incubation of hIgG antibodies with serum samples collected 14 days post third immunization**

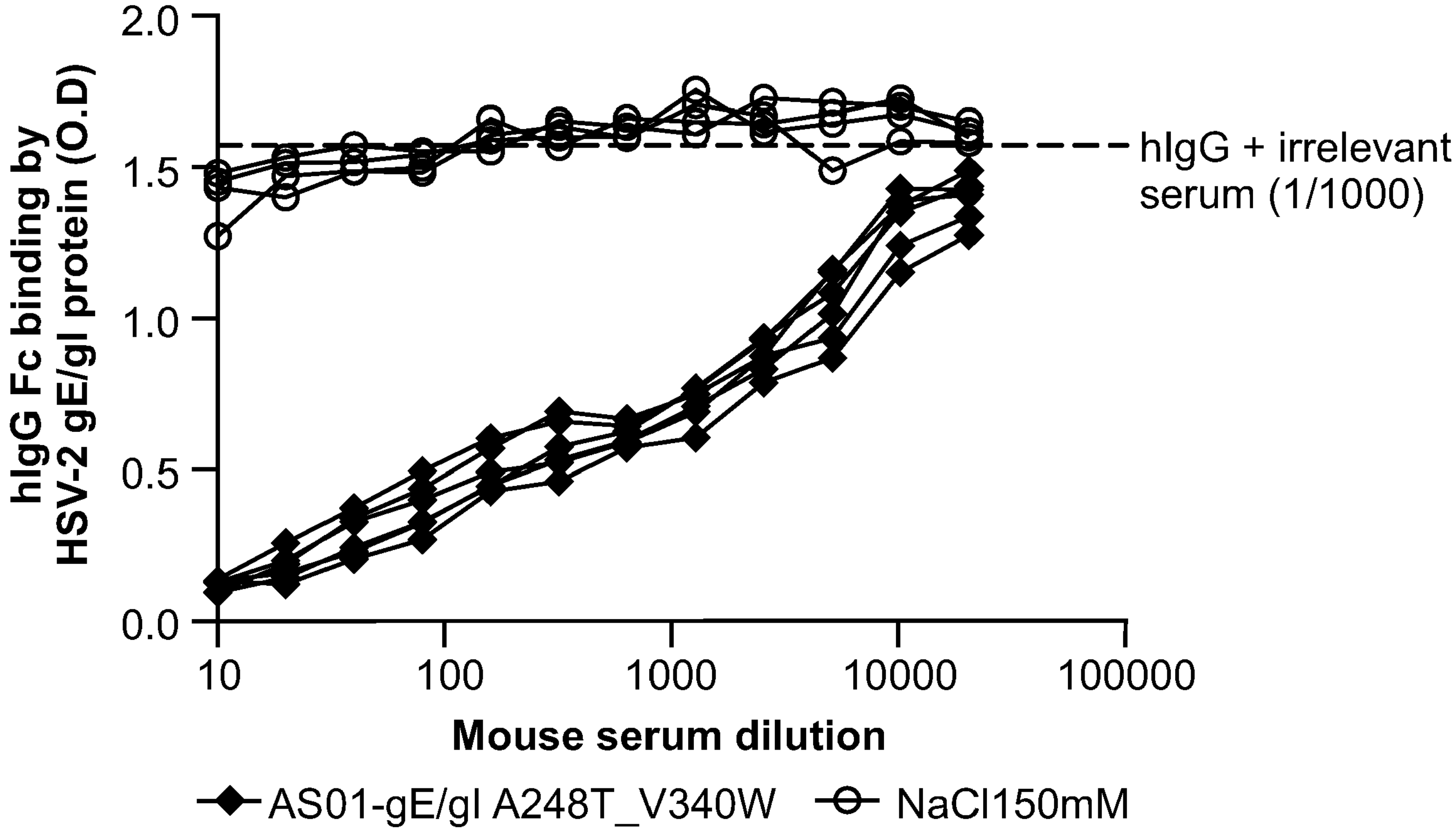

FIG. 11A

HigG Fc binding after co-incubation of higG antibodies with serum samples collected 14 days post third immunization

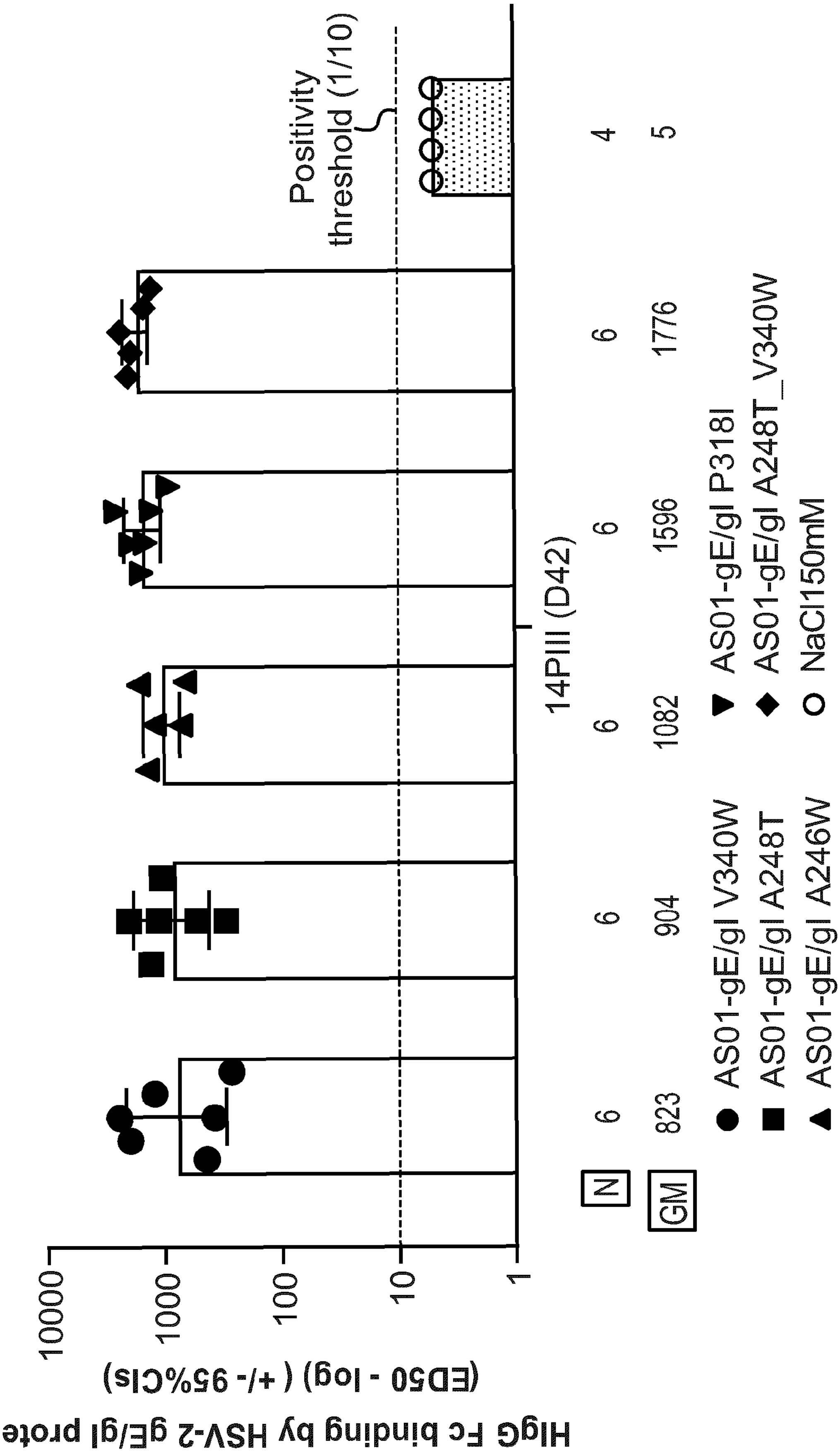

# FIG. 11B

**Inhibition of higG Fc binding activity by gE/gl protein (ED50)**
**GMR with 95% CI: Head to head comparison of HSV-2 gE/gl/AS01 (5µg each) groups**

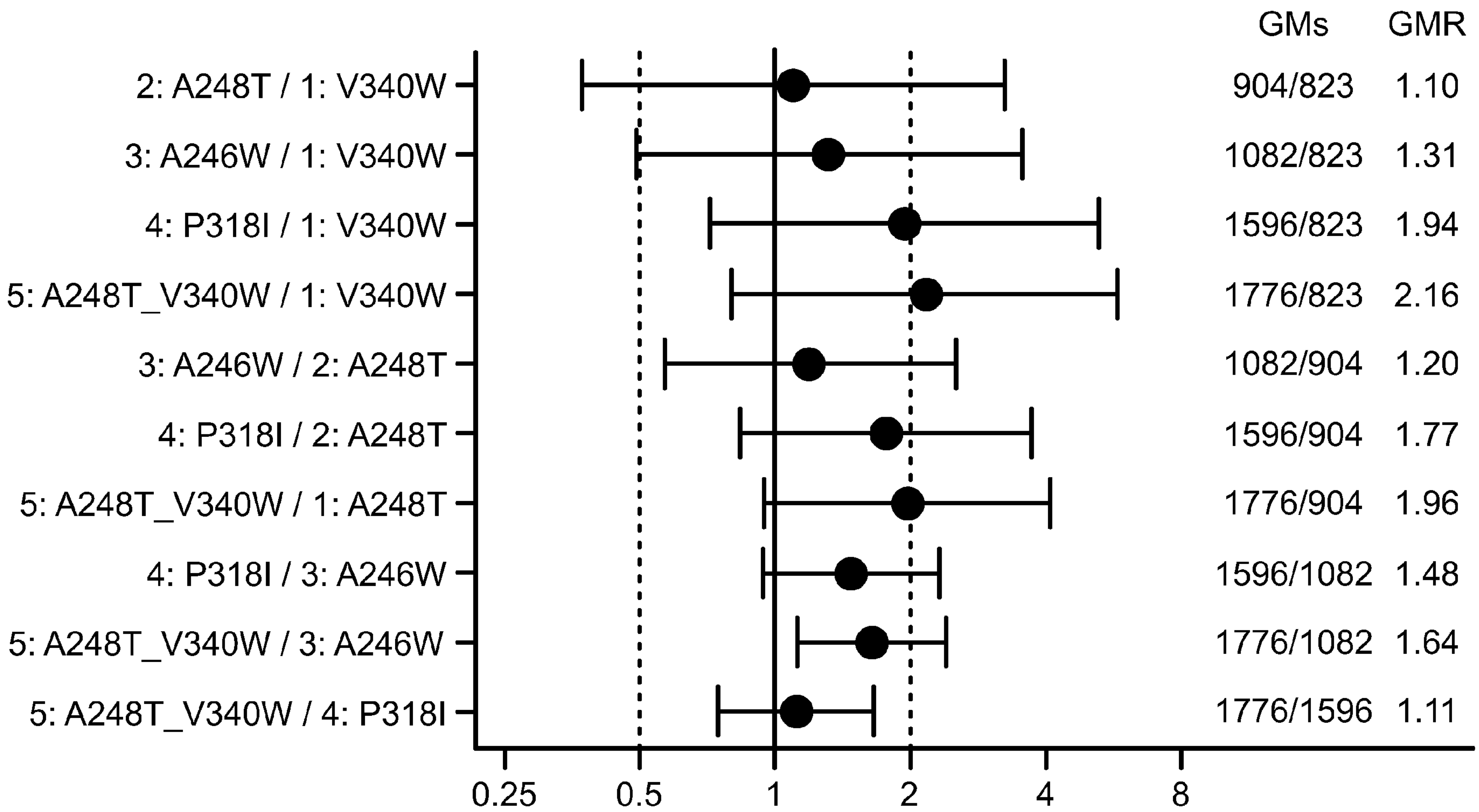

# FIG. 12A

**mFcγRIII binding activity detected in serum samples collected 14days post third immunization with AS01-adjuvanted HSV-2 gE/gI V340W mutant candidate**

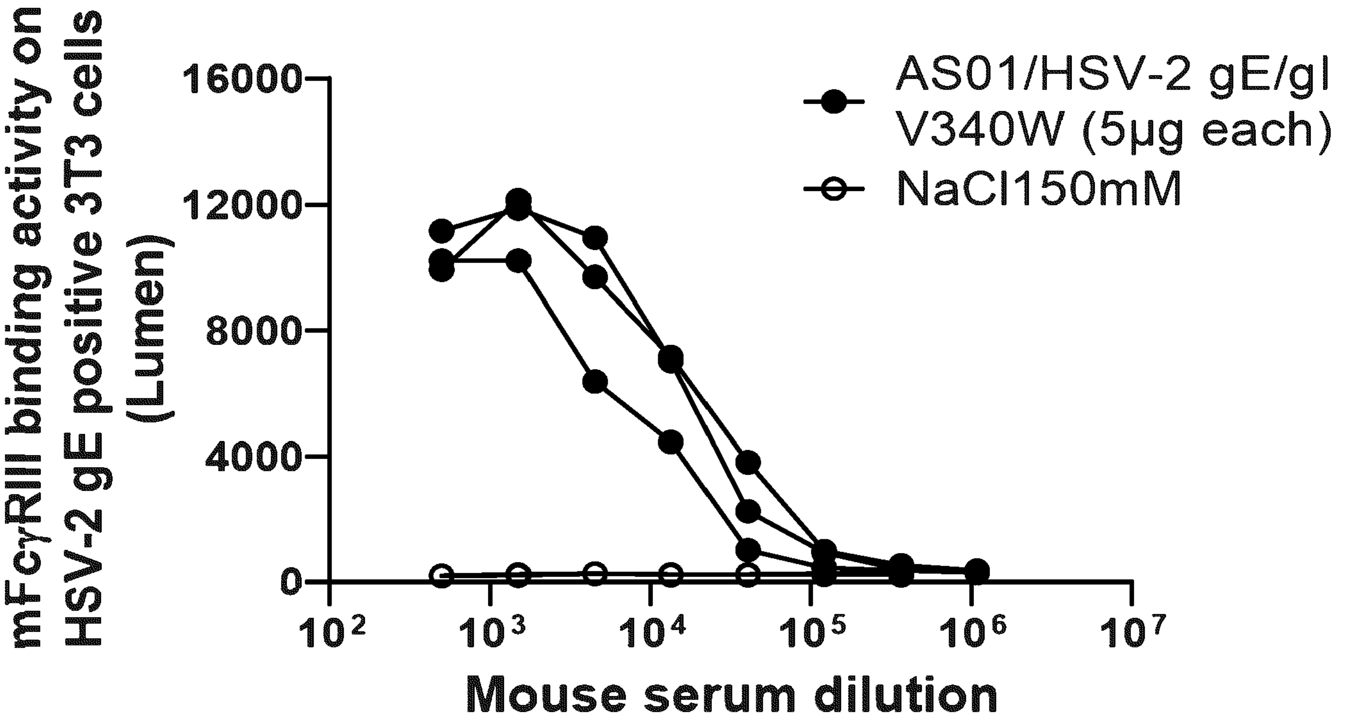

# FIG. 12B

**mFcγRIII binding activity detected in serum samples collected 14days post third immunization with AS01-adjuvanted HSV-2 gE/gI A248T mutant candidate**

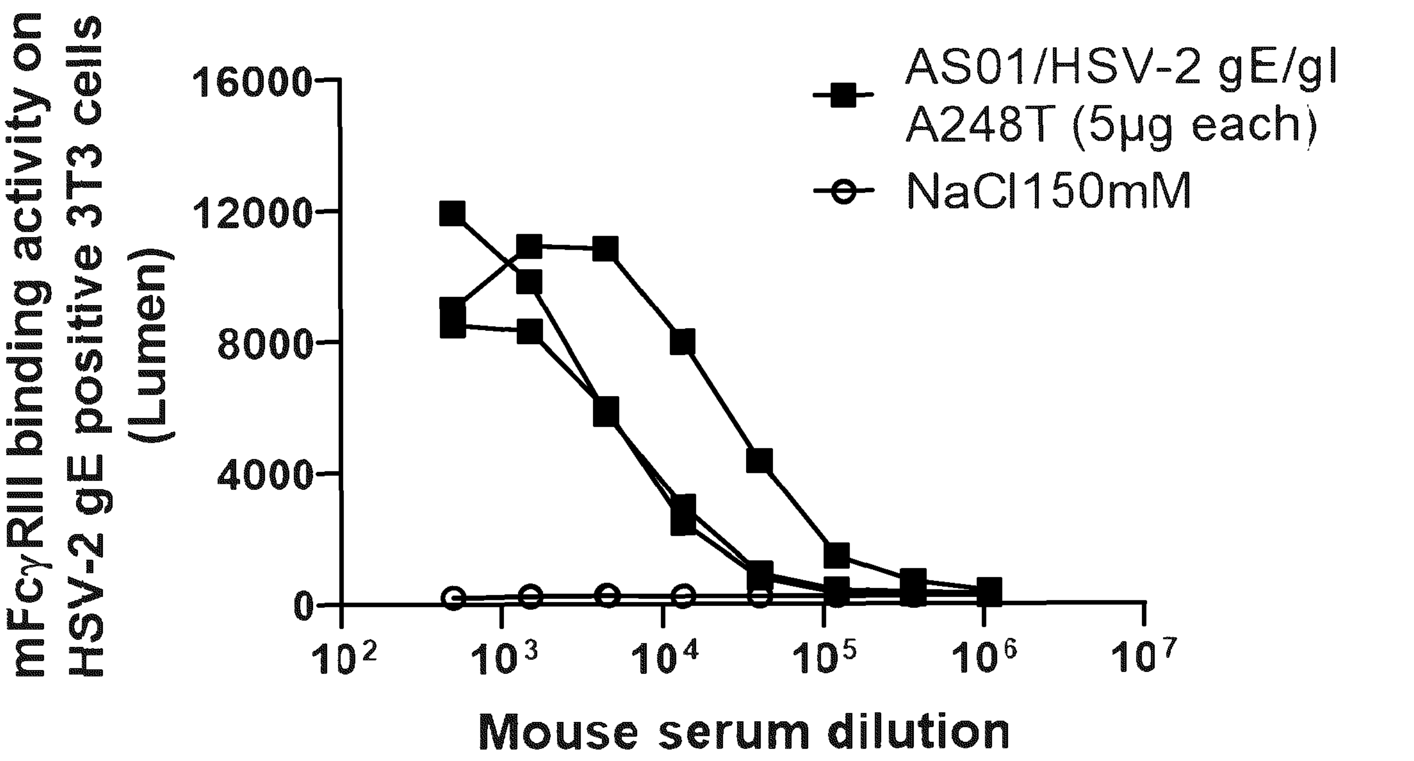

# FIG. 12C

**mFcγRIII binding activity detected in serum samples collected 14days post third immunization with AS01-adjuvanted HSV-2 gE/gI A246W mutant candidate**

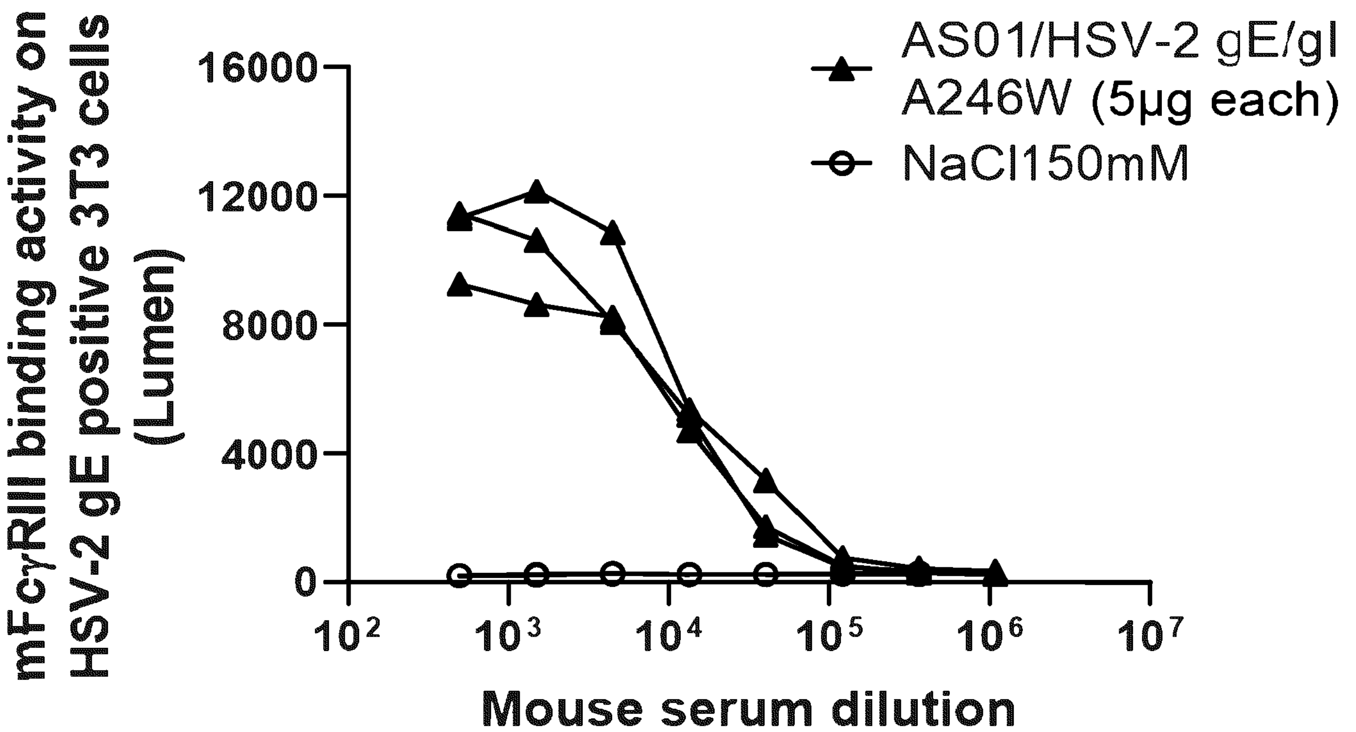

# FIG. 12D

**mFcγRIII binding activity detected in serum samples collected 14 days post third immunization with AS01-adjuvanted HSV-2 gE/gI P318I mutant candidate**

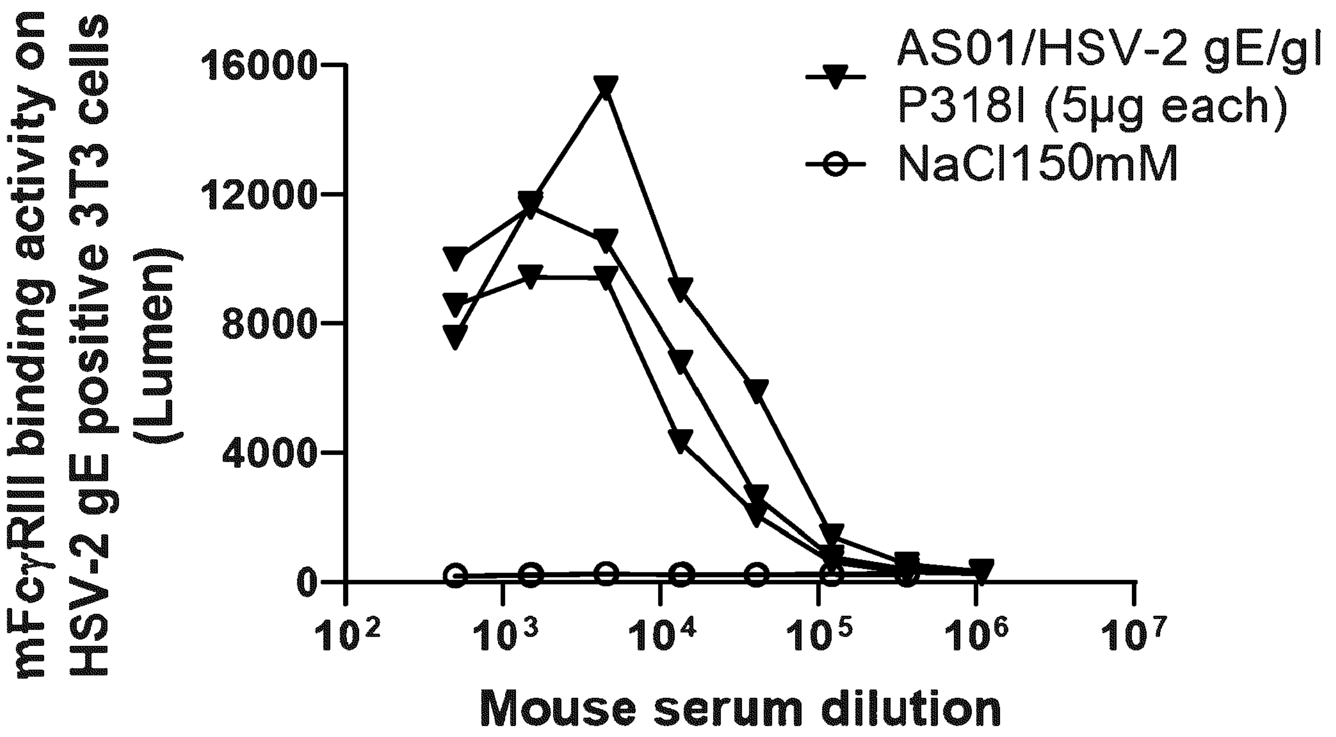

# FIG. 12E

**mFcγRIII binding activity detected in serum samples collected 14 days post third immunization with AS01-adjuvanted HSV-2 gE/gI A248T_V340W mutant candidate**

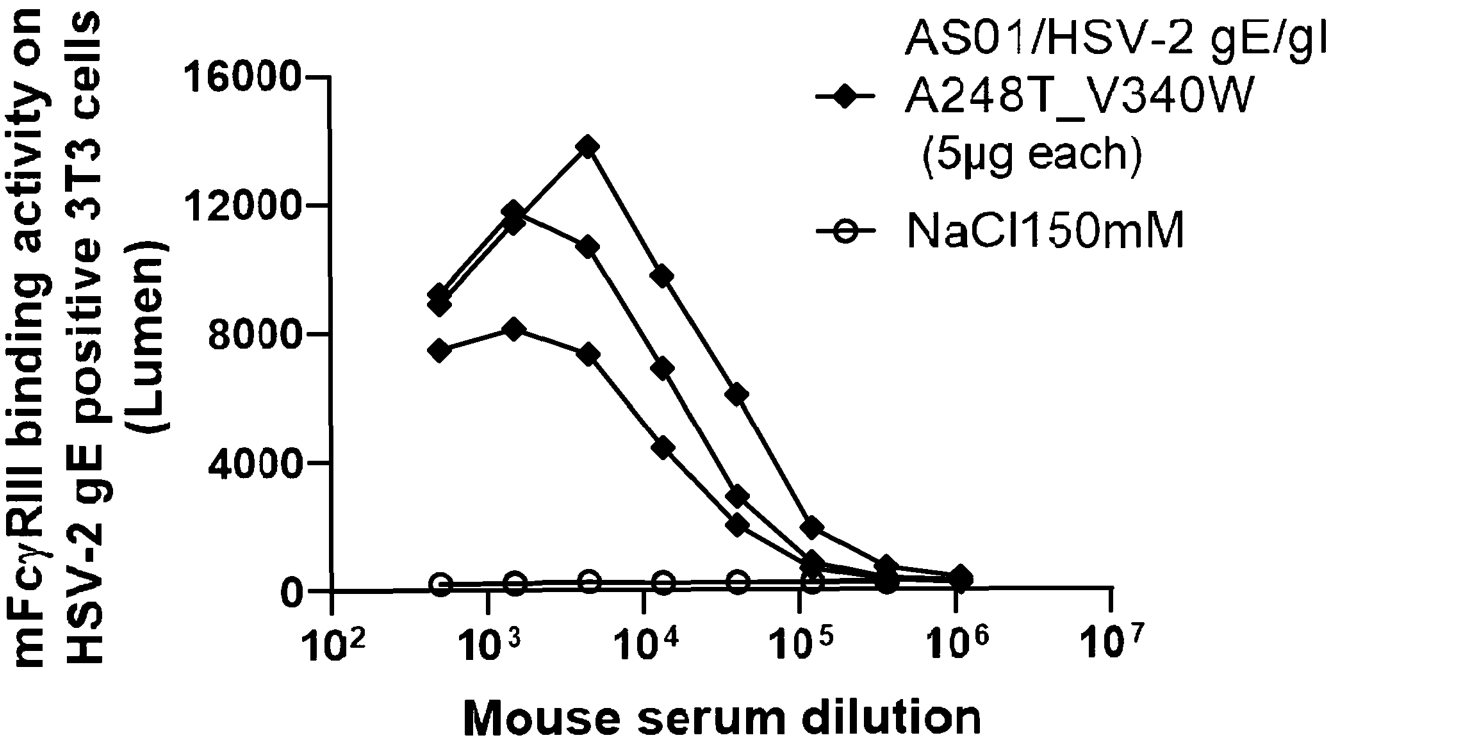

# FIG. 12F

**mFcγRIII binding activity detected in serum samples collected 14 days post third immunization with different mutated versions of AS01-adjuvanted HSV-2 gE/gI protein**

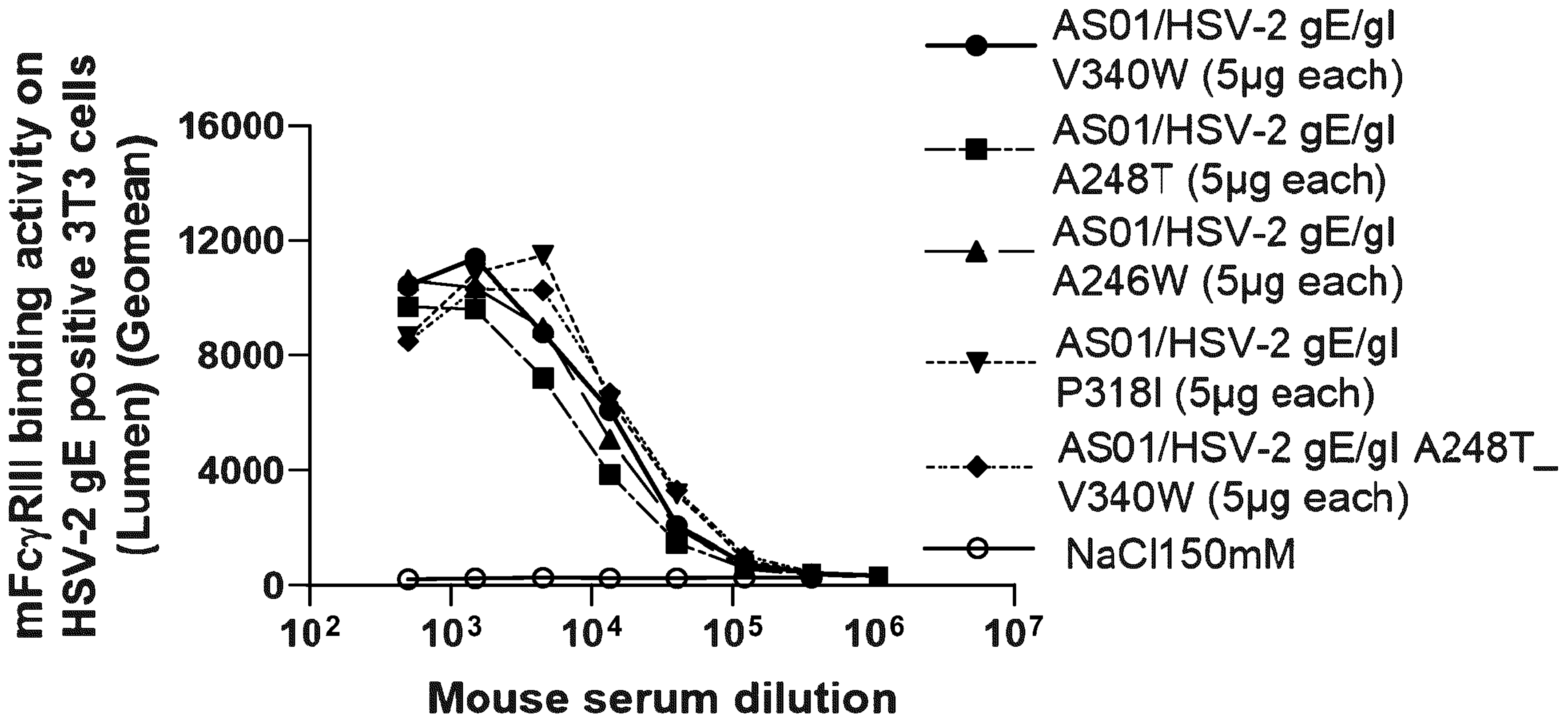

FIG. 13A

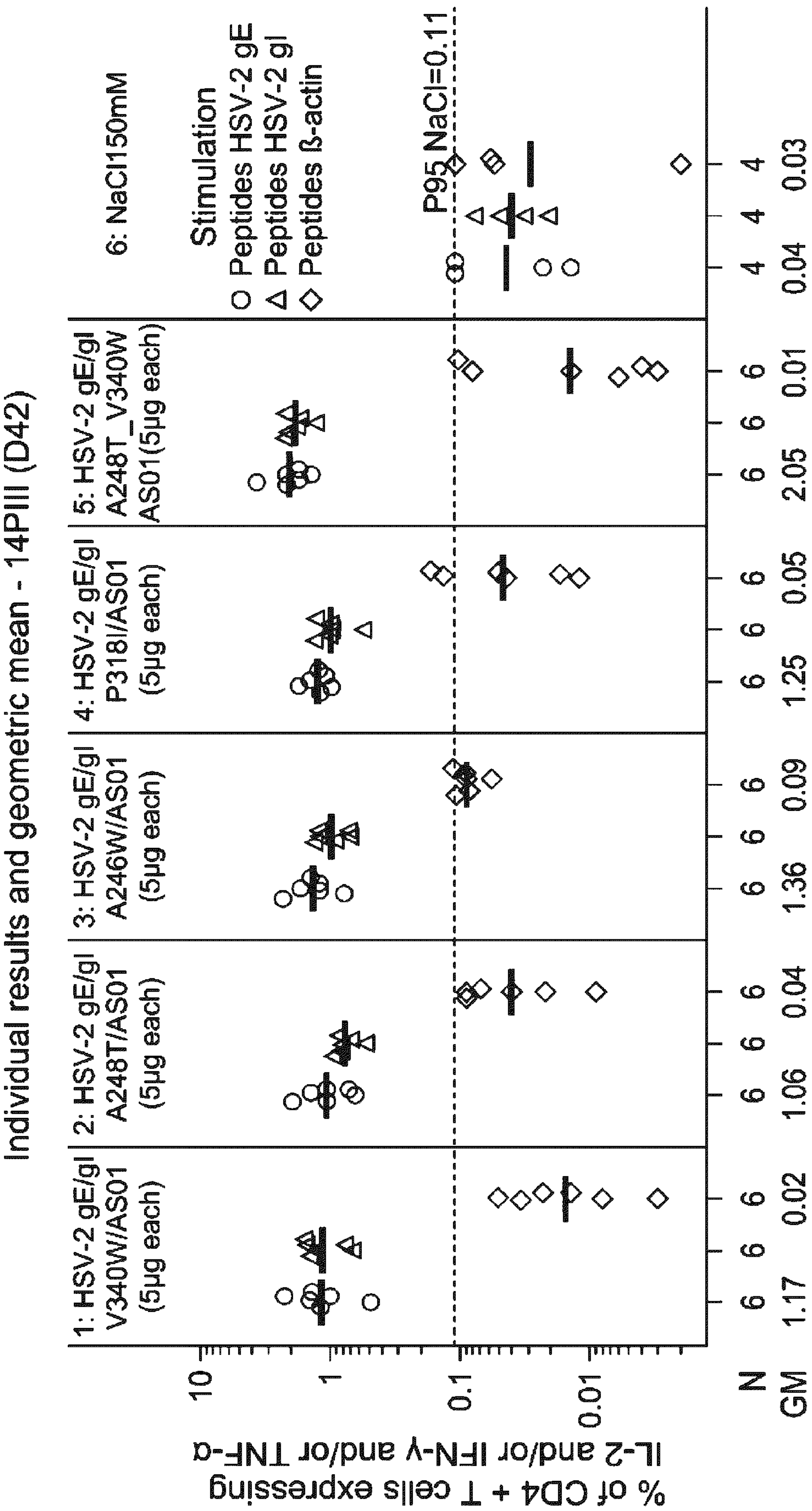
Individual results and geometric mean - 14PIII (D42)
1: HSV-2 gE/gI V340W/AS01 (5µg each)
2: HSV-2 gE/gI A248T/AS01 (5µg each)
3: HSV-2 gE/gI A246W/AS01 (5µg each)
4: HSV-2 gE/gI P318I/AS01 (5µg each)
5: HSV-2 gE/gI A248T_V340W AS01(5µg each)
6: NaCl150mM
Stimulation
Peptides HSV-2 gE
Peptides HSV-2 gI
Peptides ß-actin
P95 NaCl=0.11
% of CD4 + T cells expressing IL-2 and/or IFN-γ and/or TNF-α
10
1
0.1
0.01
N
GM
6 6 6 6 6 6 6 6 6 6 6 6 6 6 6 4 4 4
1.17 1.15 0.02 1.06 0.77 0.04 1.36 0.99 0.09 1.25 0.99 0.05 2.05 1.87 0.01 0.04 0.04 0.03

# FIG. 13B

Individual results and geometric mean - 14PIII (D42)

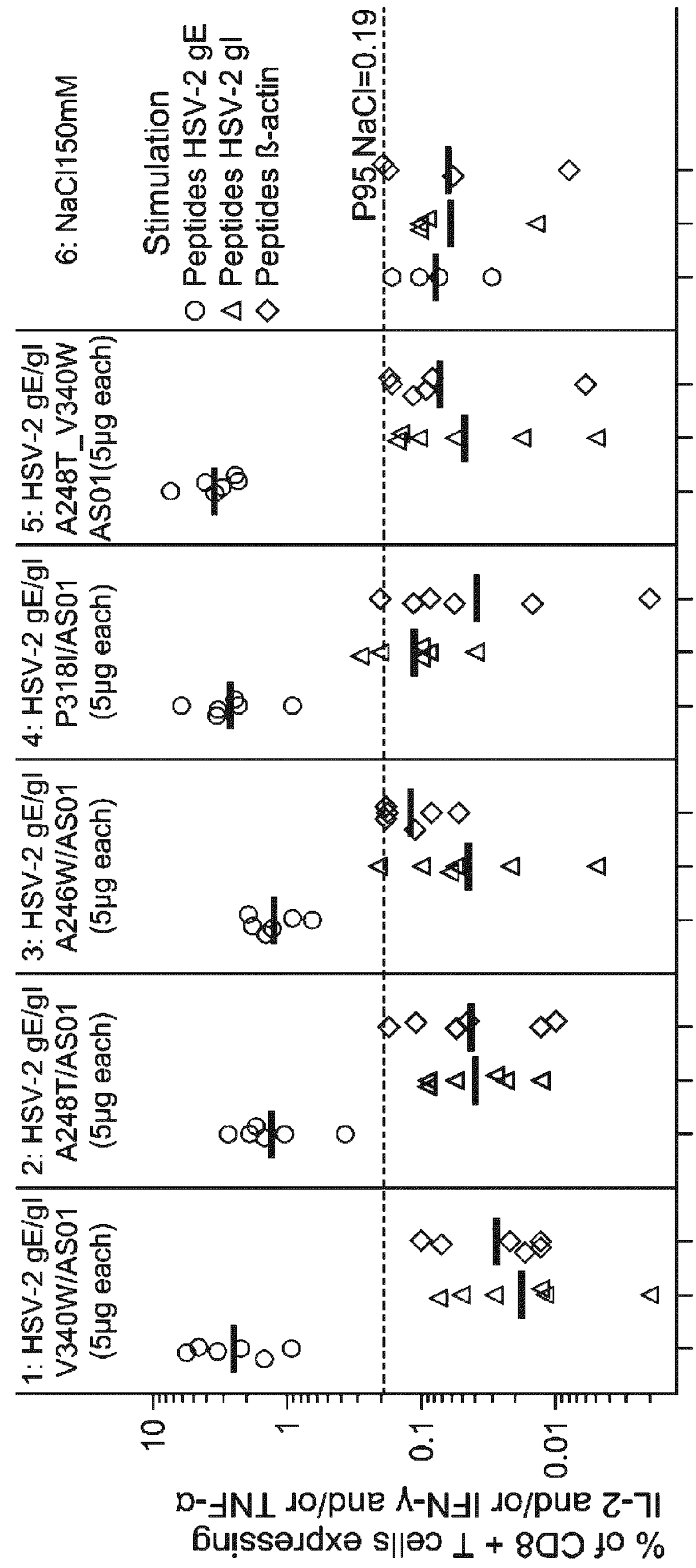

FIG. 14A

Geometric mean ratios with 95% CIs of % of HSV-2 gE-specific CD4+ T cells

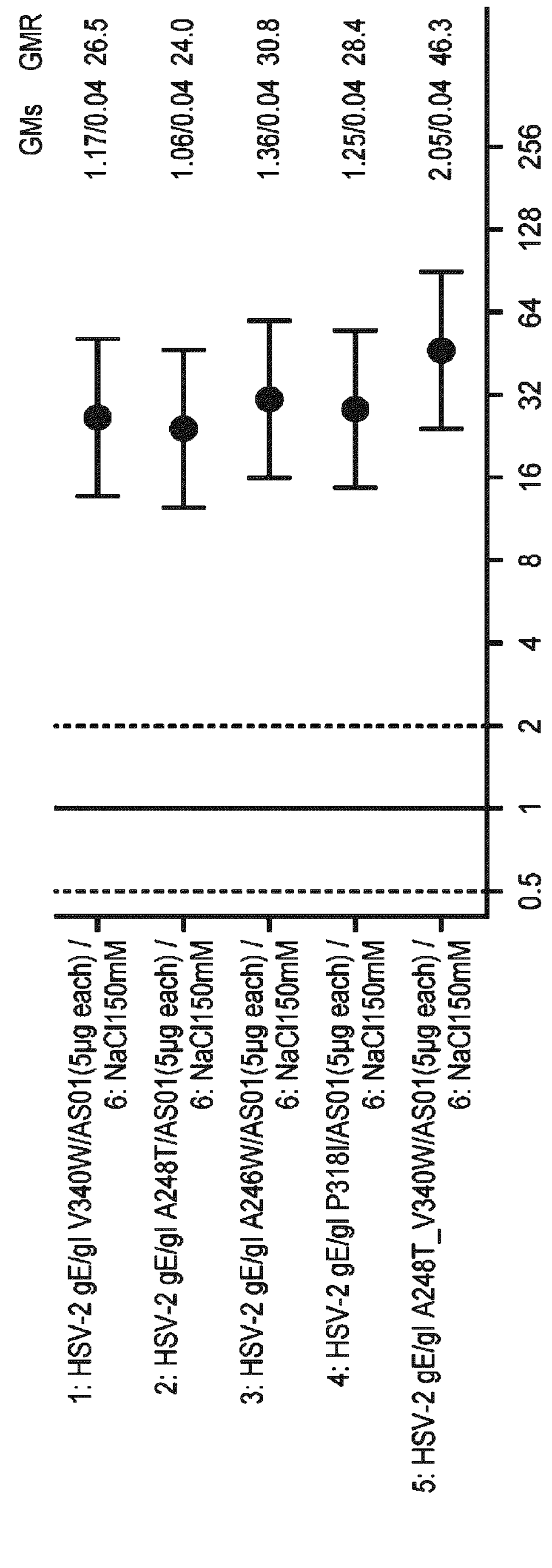

# FIG. 14B

Geometric mean ratios with 95% CIs of % of HSV-2 gI-specific CD4+ T cells

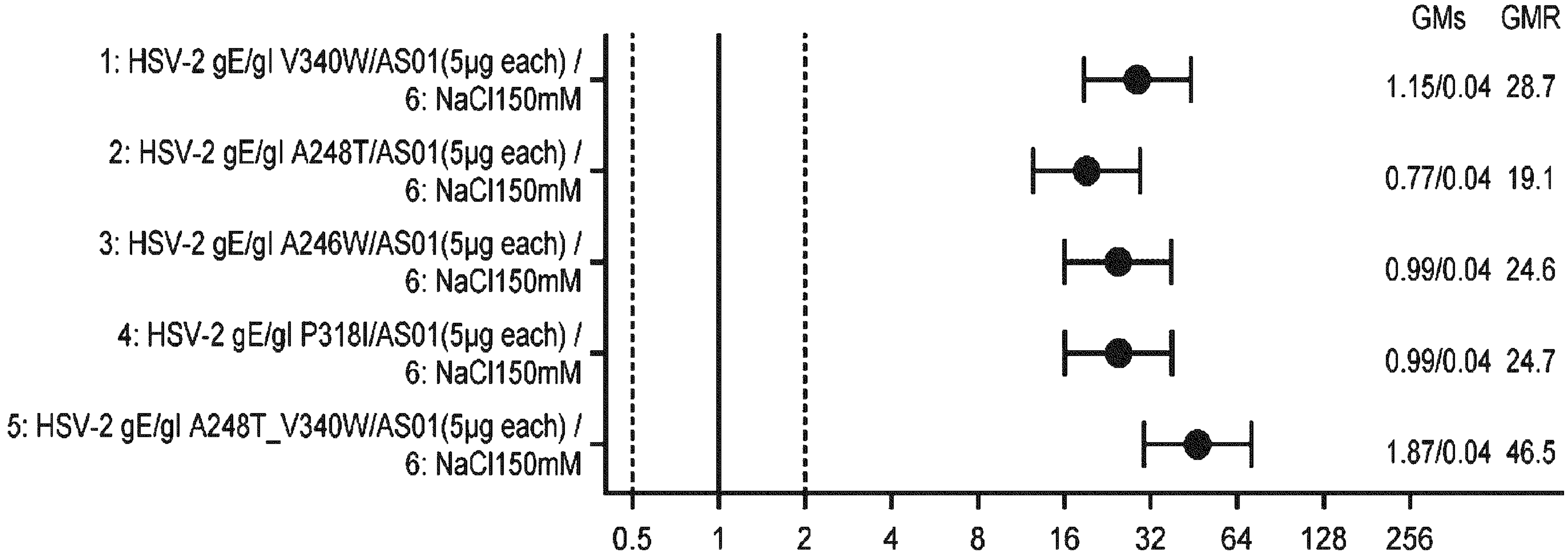

FIG. 14C

Geometric mean ratios with 95% CIs of % of HSV-2 gE-specific CD8+ T cells

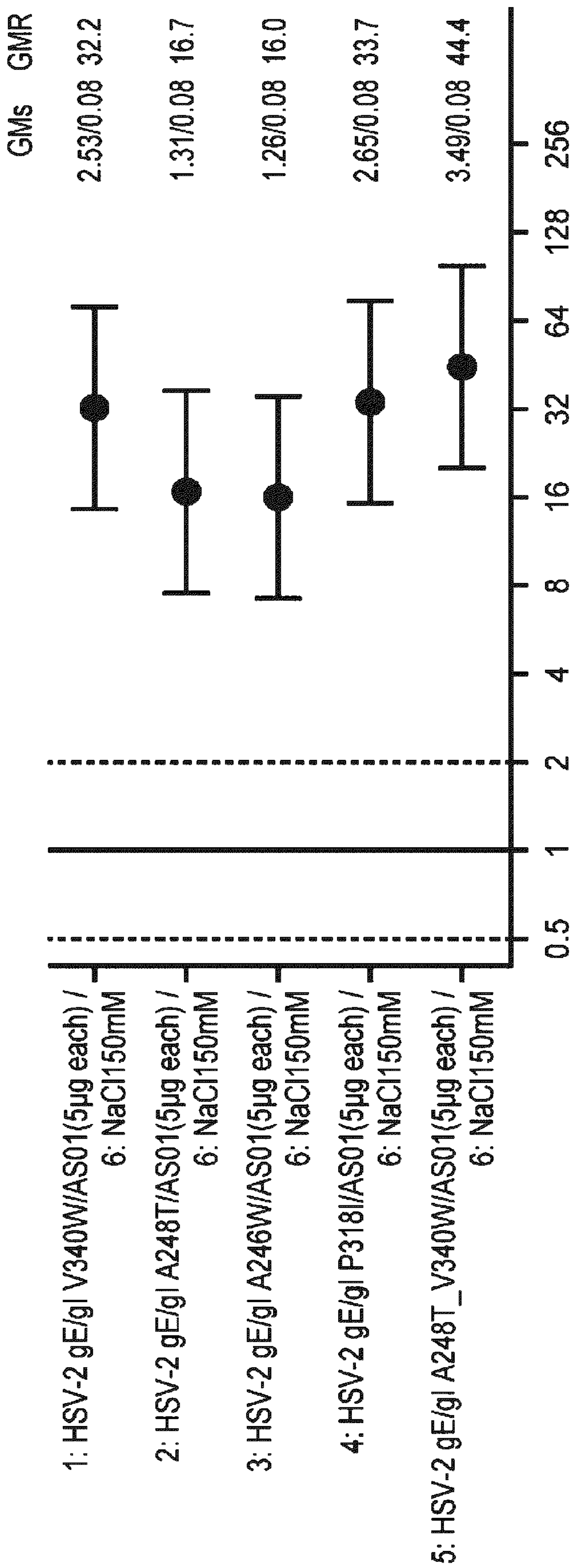

# FIG. 15A

Geometric mean ratios with 95% CIs of % of HSV-2 gE-specific CD4+ T cells
Head to head comparison of HSV-2 gE/gI/AS01(5µg each) groups

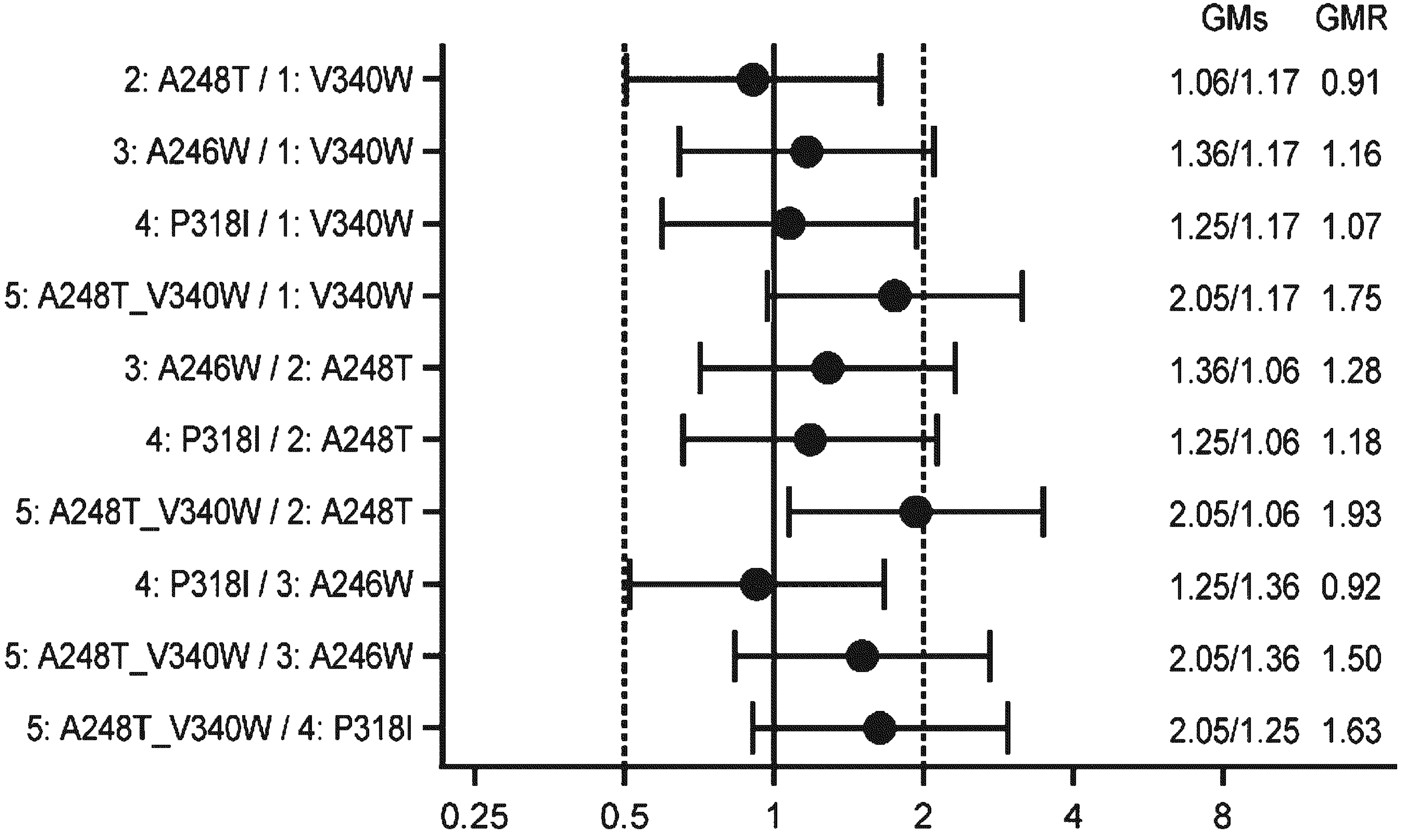

# FIG. 15B

Geometric mean ratios with 95% CIs of % of HSV-2 gI-specific CD4+ T cells
Head to head comparison of HSV-2 gE/gI/AS01(5μg each) groups

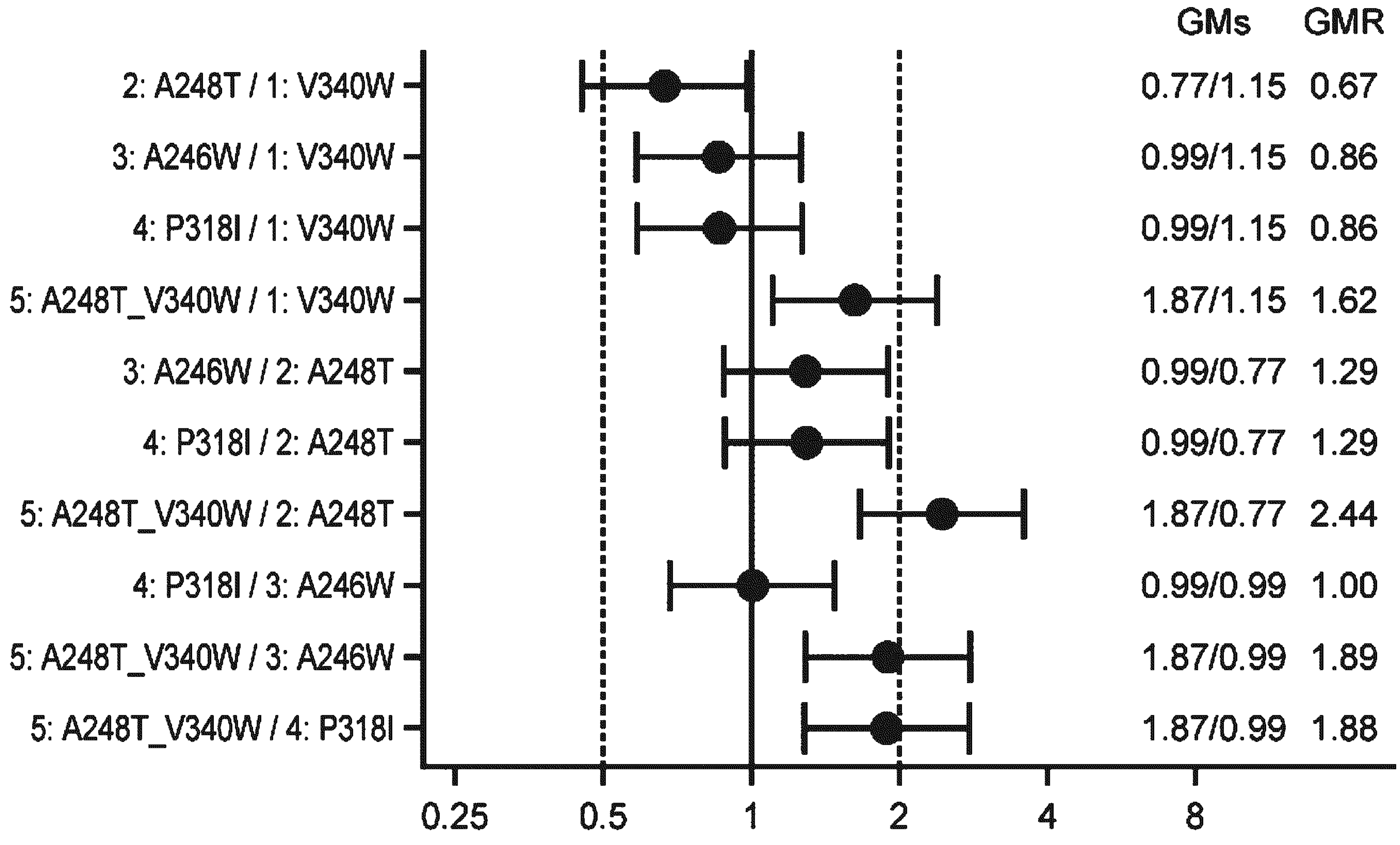

FIG. 15C

Geometric mean ratios with 95% CIs of % of HSV-2 gE-specific CD4+ T cells
Head to head comparison of HSV-2 gE/gI/AS01(5µg each) groups

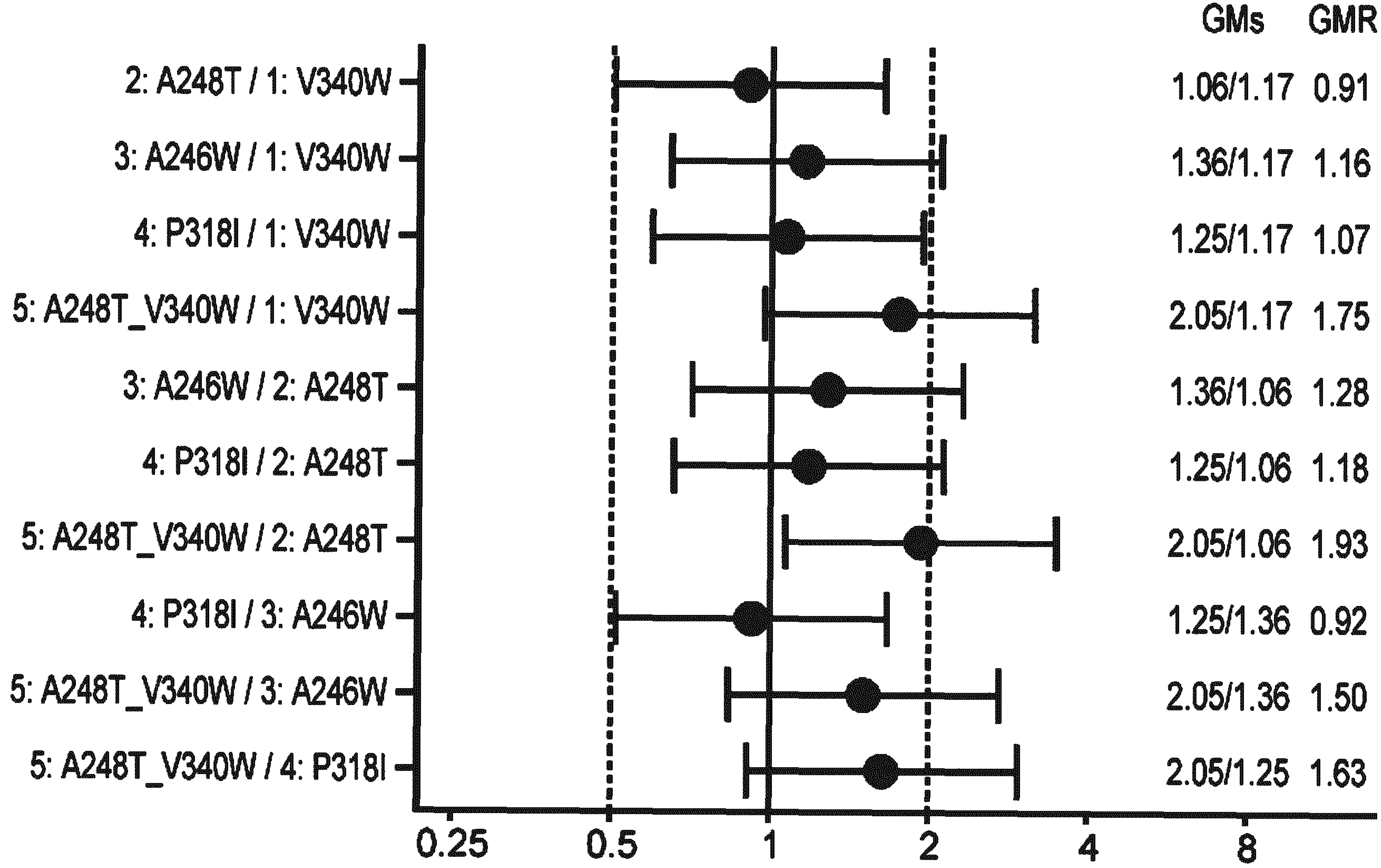

FIG. 16A

Individual results and geometric mean

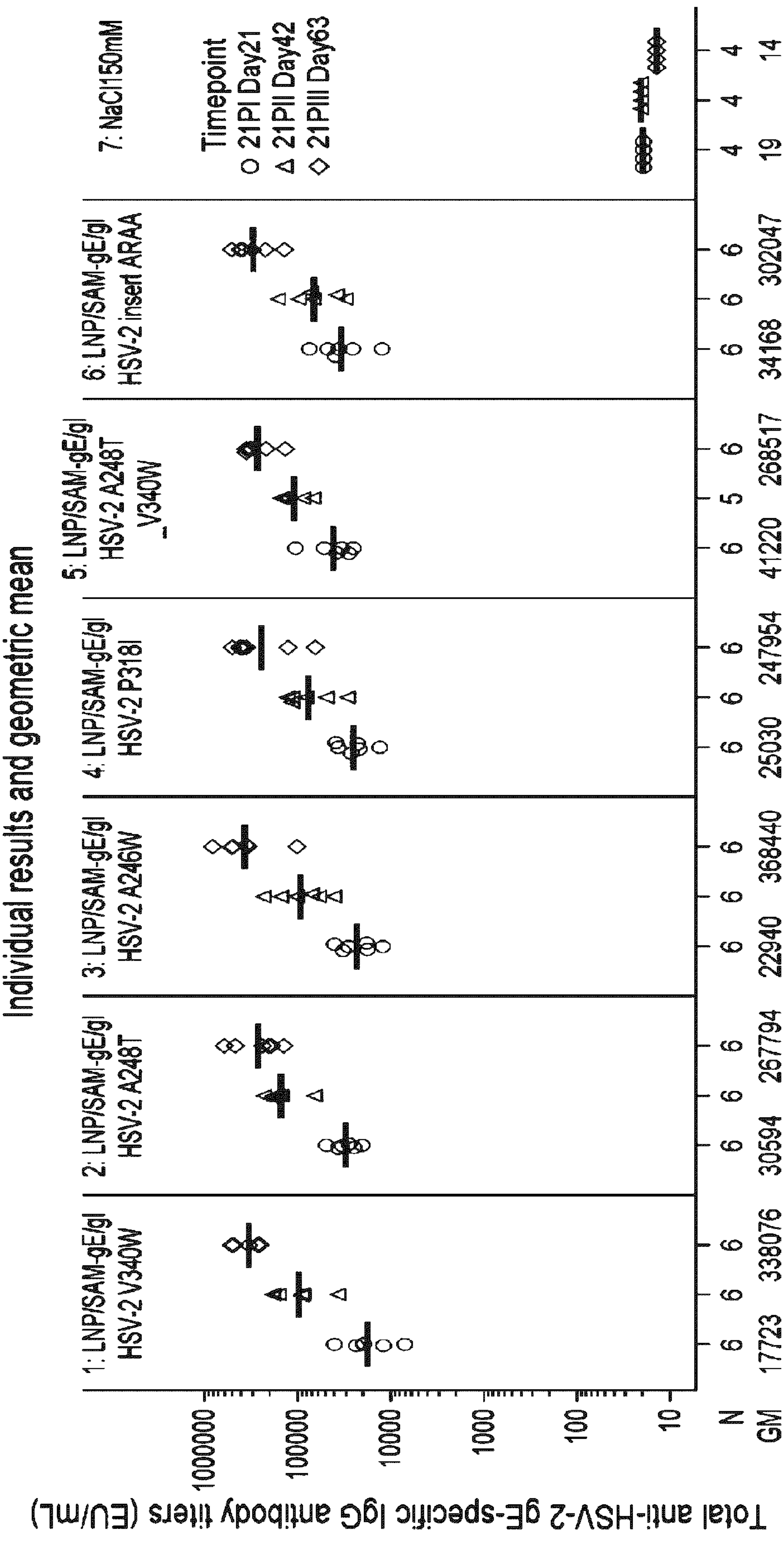

FIG. 16B

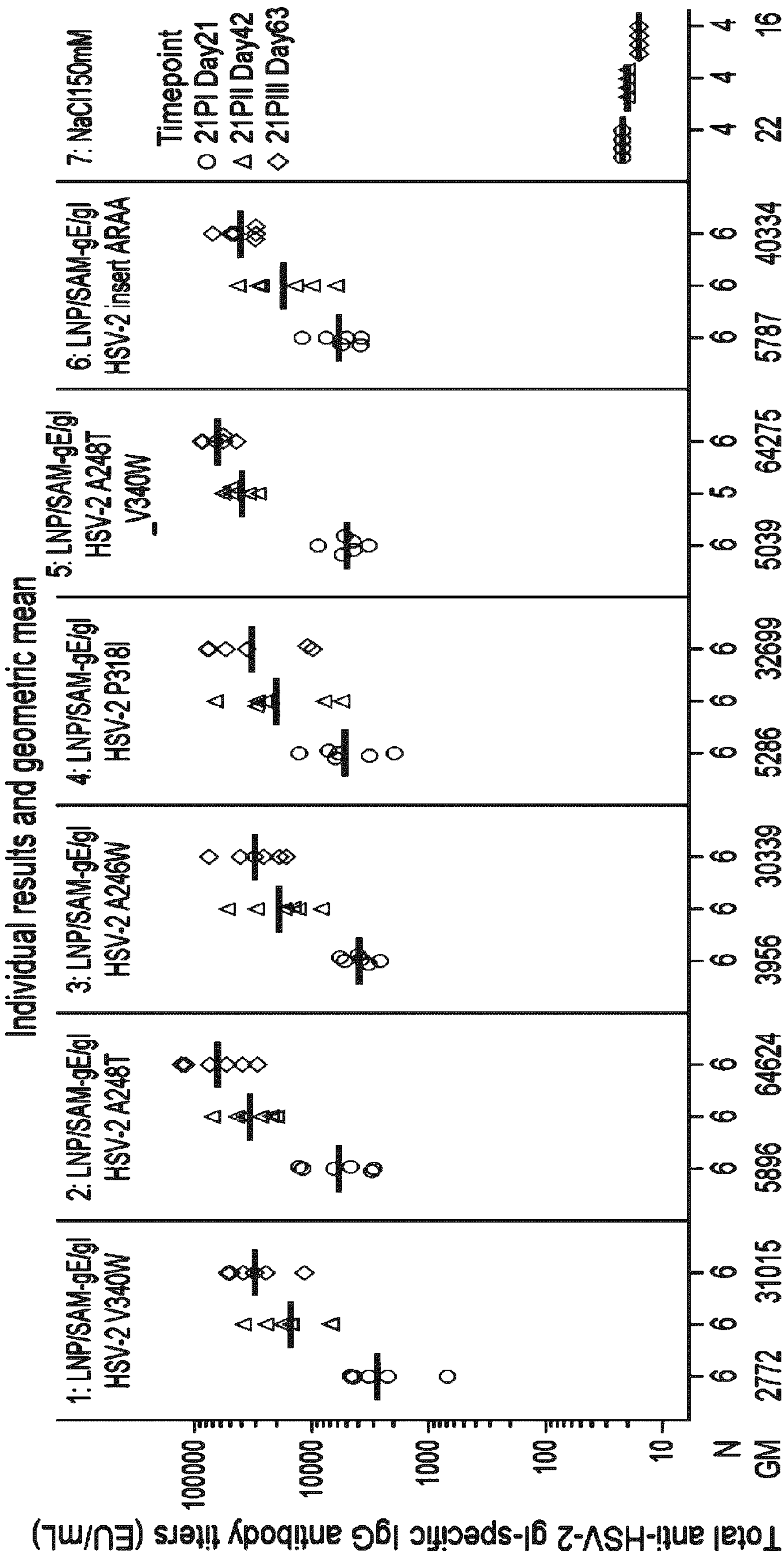
Individual results and geometric mean
Total anti-HSV-2 gI-specific IgG antibody titers (EU/mL)
100000
10000
1000
100
10
N
GM
1: LNP/SAM-gE/gI HSV-2 V340W
2: LNP/SAM-gE/gI HSV-2 A248T
3: LNP/SAM-gE/gI HSV-2 A246W
4: LNP/SAM-gE/gI HSV-2 P318I
5: LNP/SAM-gE/gI HSV-2 A248T _V340W
6: LNP/SAM-gE/gI HSV-2 insert ARAA
7: NaCl150mM
Timepoint
21PI Day21
21PII Day42
21PIII Day63
6 6 6
2772 15217 31015
6 6 6
5896 33704 64624
6 6 6
3956 18799 30339
6 6 6
5286 20076 32699
6 5 6
5039 39871 64275
6 6 6
5787 17477 40334
4 4 4
22 20 16

# FIG.17A

GMR with 95% CIs of Total anti-HSV-2 gE-specific IgG antibody titers (EU/mL) - 21PIII (D63)

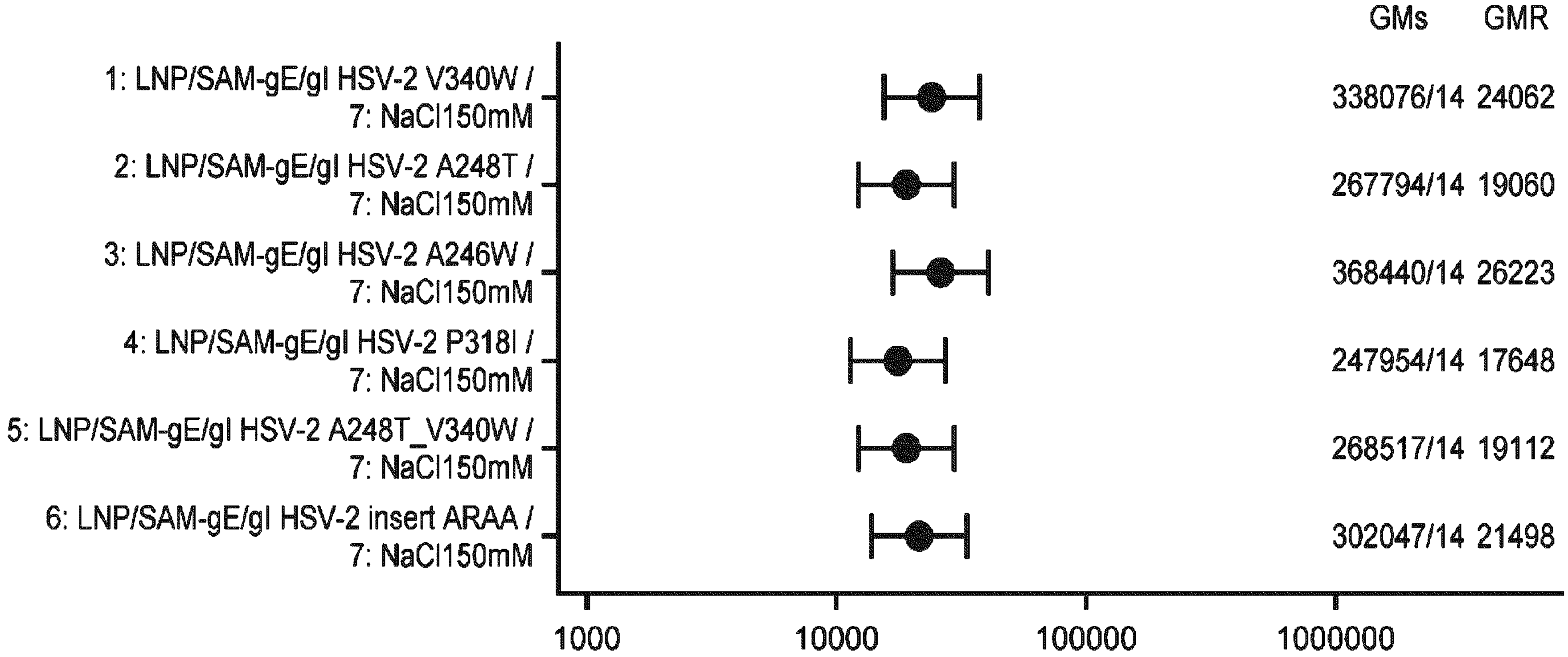

FIG.17B

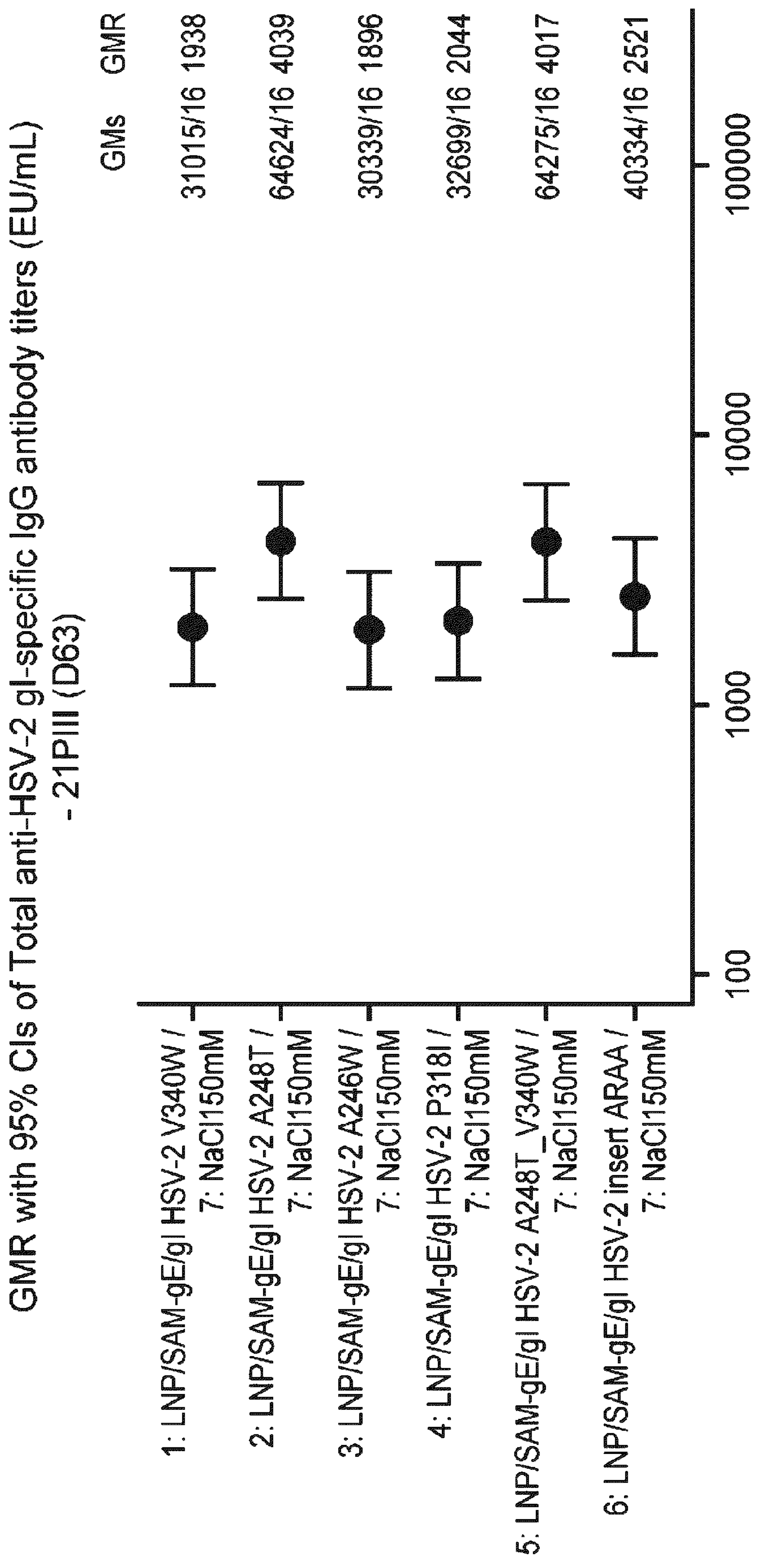
GMR with 95% CIs of Total anti-HSV-2 gI-specific IgG antibody titers (EU/mL) - 21PIII (D63)
GMs GMR
1: LNP/SAM-gE/gI HSV-2 V340W / 7: NaCl150mM
31015/16 1938
2: LNP/SAM-gE/gI HSV-2 A248T / 7: NaCl150mM
64624/16 4039
3: LNP/SAM-gE/gI HSV-2 A246W / 7: NaCl150mM
30339/16 1896
4: LNP/SAM-gE/gI HSV-2 P318I / 7: NaCl150mM
32699/16 2044
5: LNP/SAM-gE/gI HSV-2 A248T_V340W / 7: NaCl150mM
64275/16 4017
6: LNP/SAM-gE/gI HSV-2 insert ARAA / 7: NaCl150mM
40334/16 2521
100
1000
10000
100000

# FIG.18A

GMR with 95% CIs of Total anti-HSV-2 gE-specific IgG antibody titers (EU/mL)
Group comparisons of PII over PI

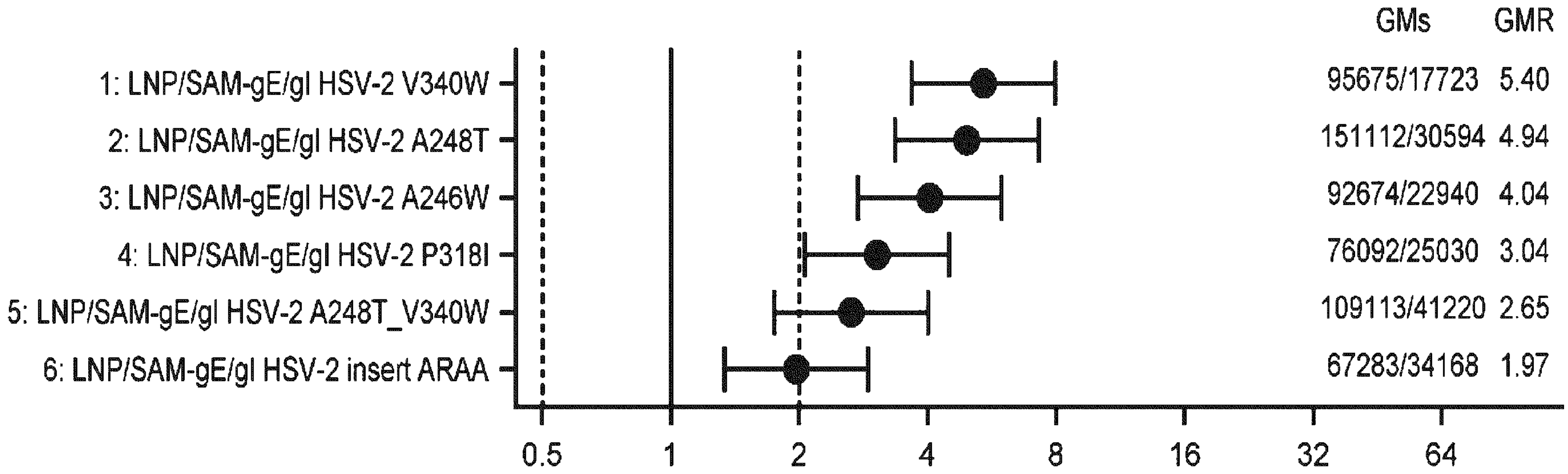

# FIG.18B

GMR with 95% CIs of Total anti-HSV-2 gE-specific IgG antibody titers (EU/mL)
Group comparisons of PIII over PII

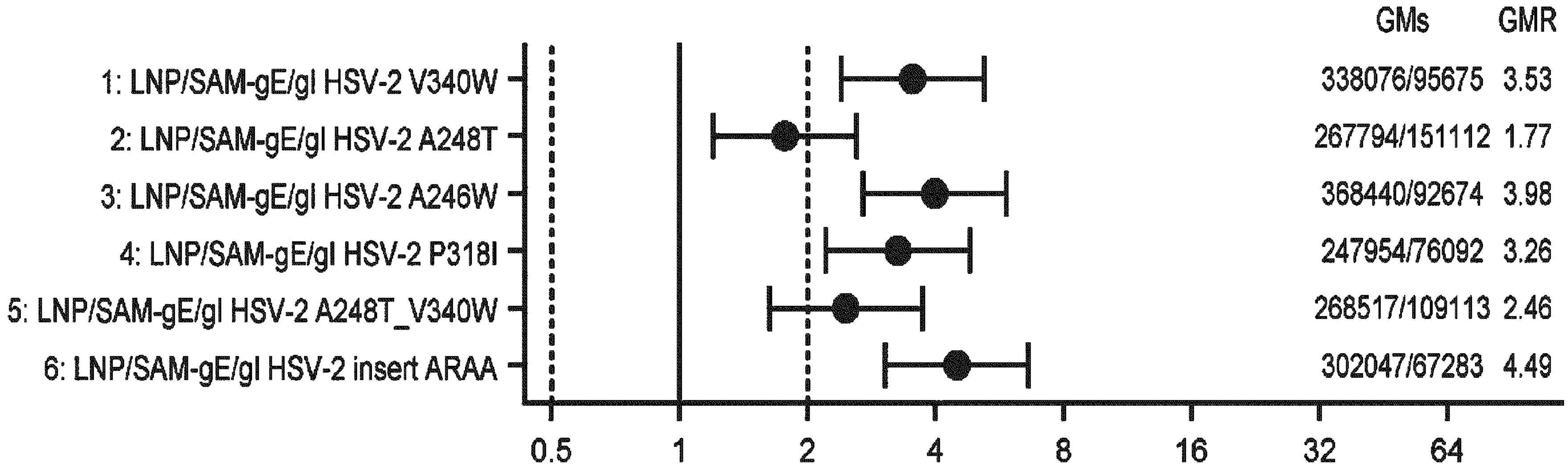

# FIG.18C

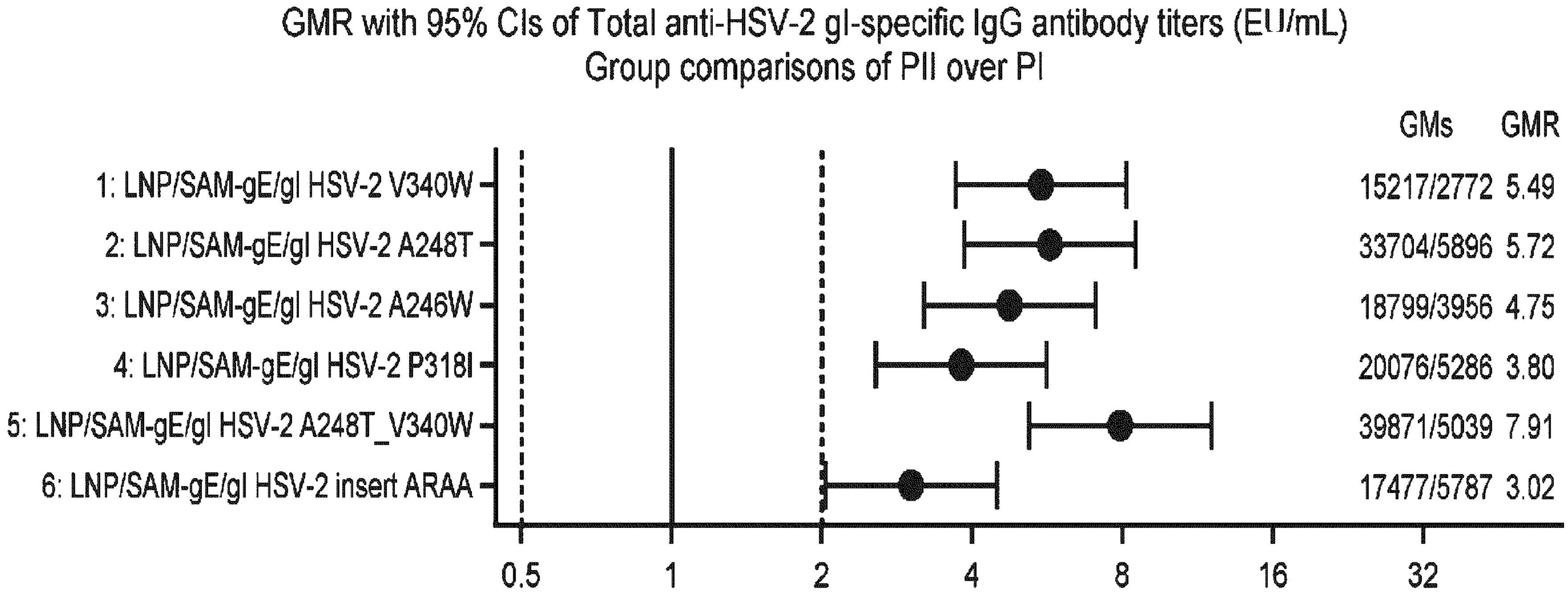
GMR with 95% CIs of Total anti-HSV-2 gI-specific IgG antibody titers (EU/mL)
Group comparisons of PII over PI
GMs
GMR
1: LNP/SAM-gE/gI HSV-2 V340W
2: LNP/SAM-gE/gI HSV-2 A248T
3: LNP/SAM-gE/gI HSV-2 A246W
4: LNP/SAM-gE/gI HSV-2 P318I
5: LNP/SAM-gE/gI HSV-2 A248T_V340W
6: LNP/SAM-gE/gI HSV-2 insert ARAA
15217/2772 5.49
33704/5896 5.72
18799/3956 4.75
20076/5286 3.80
39871/5039 7.91
17477/5787 3.02
0.5
1
2
4
8
16
32

# FIG.18D

GMR with 95% CIs of Total anti-HSV-2 gI-specific IgG antibody titers (EU/mL)
Group comparisons of PIII over PII

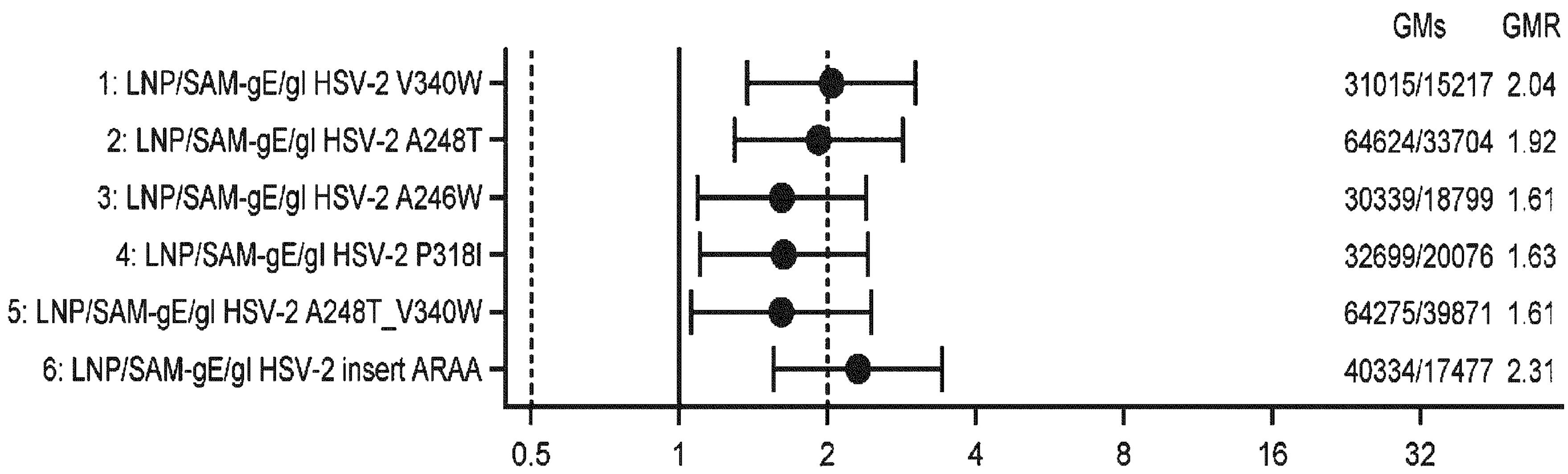

**FIG. 19A** GMR with 95% CIs of Total anti-HSV-2 gE-specific IgG antibody titers (EU/mL)
Head to head comparison of LNP/SAM-gE/gI HSV-2 - 21PIII (D63)

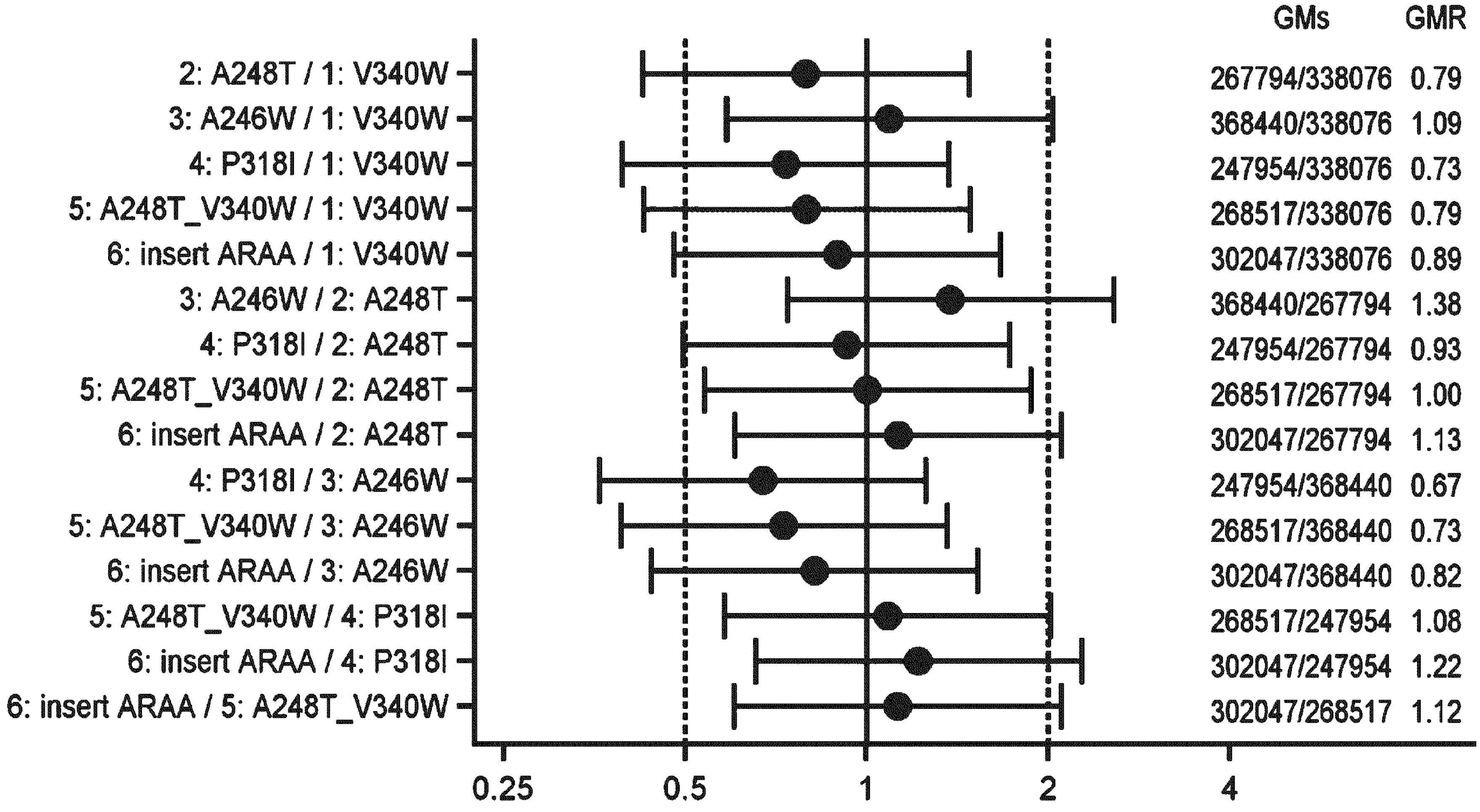

**FIG. 19B** GMR with 95% CIs of Total anti-HSV-2 gI-specific IgG antibody titers (EU/mL)
Head to head comparison of LNP/SAM-gE/gI HSV-2 - 21PIII (D63)

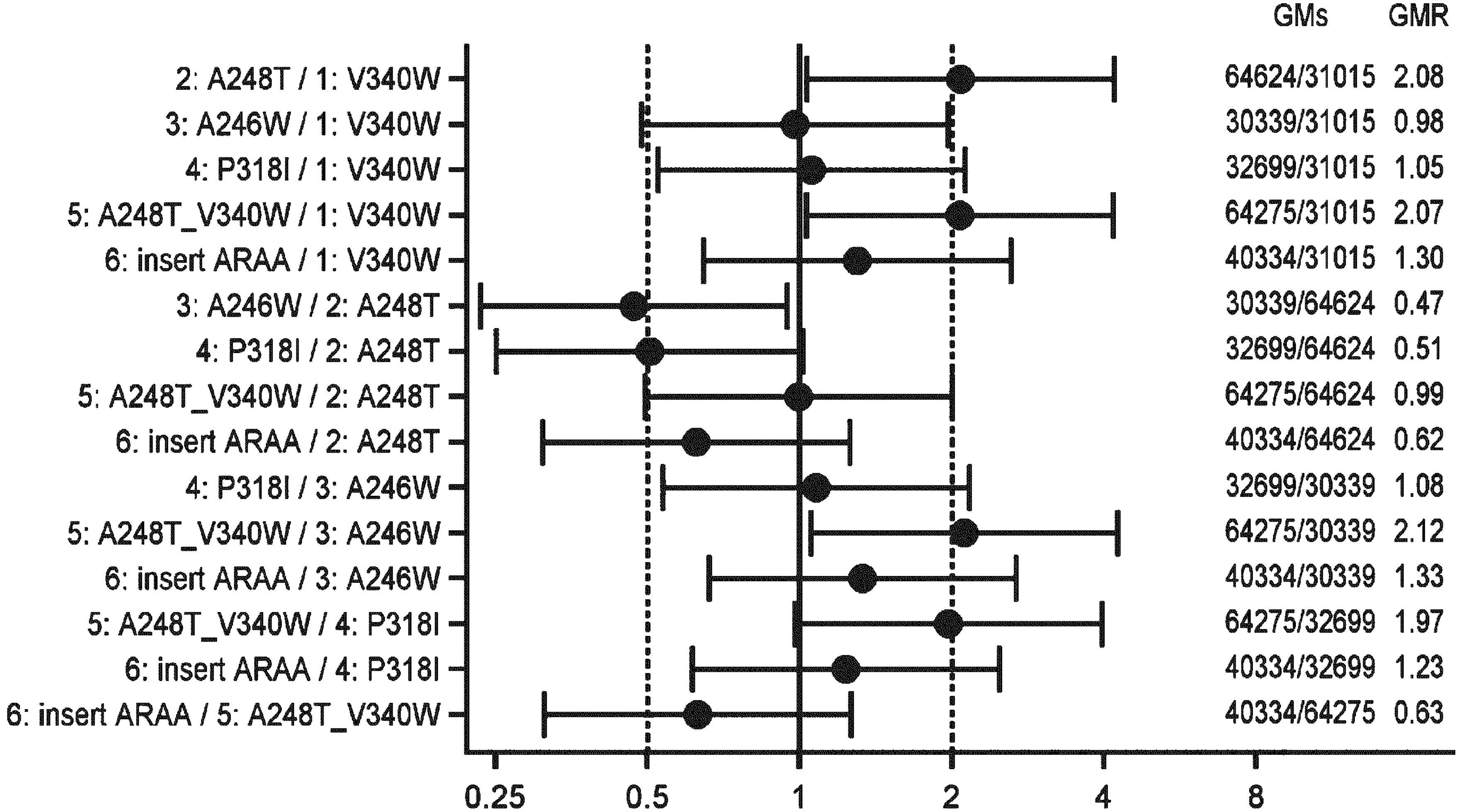

# FIG. 20

Individual results and geometric mean - 21PIII (D63)

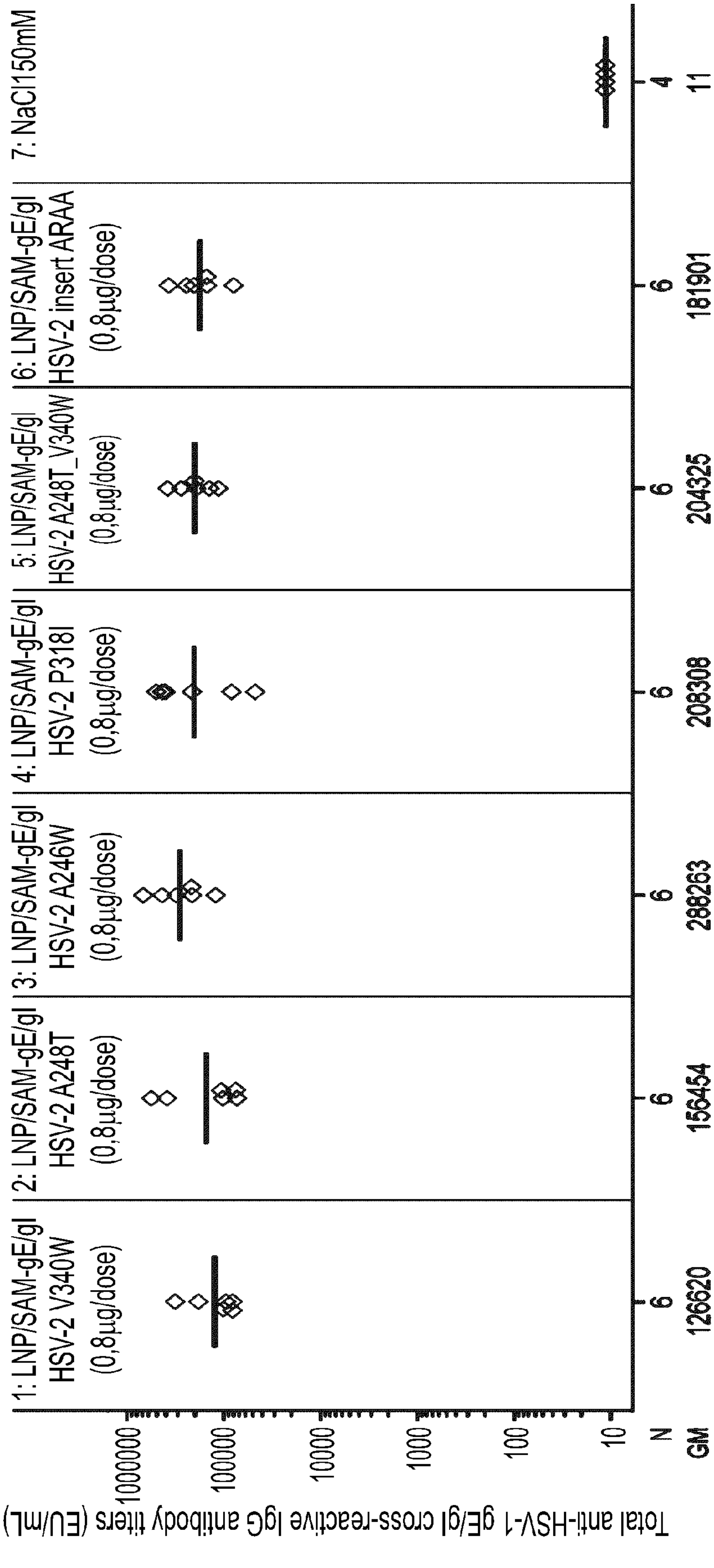

# FIG. 21

GMR with 95% CIs of Total anti-HSV-1 gE/gI cross-reactive IgG antibody titers (EU/mL) - 21PIII (D63)

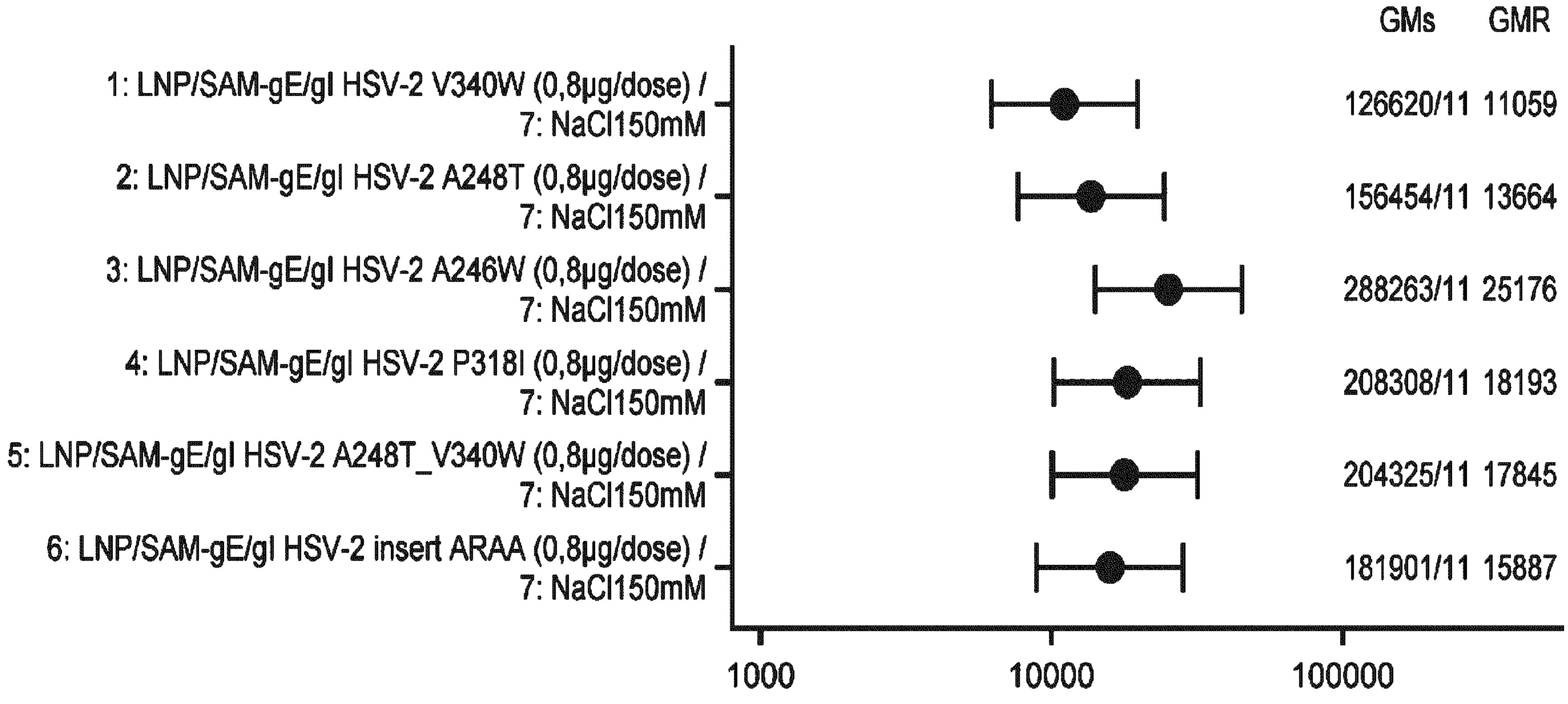

**FIG. 22** GMR with 95% CIs of Total anti-HSV-1 gE/gI cross-reactive IgG antibody titers (EU/mL)
Head to head comparison of LNP/SAM-gE/gI HSV-2 - 21PIII (D63)

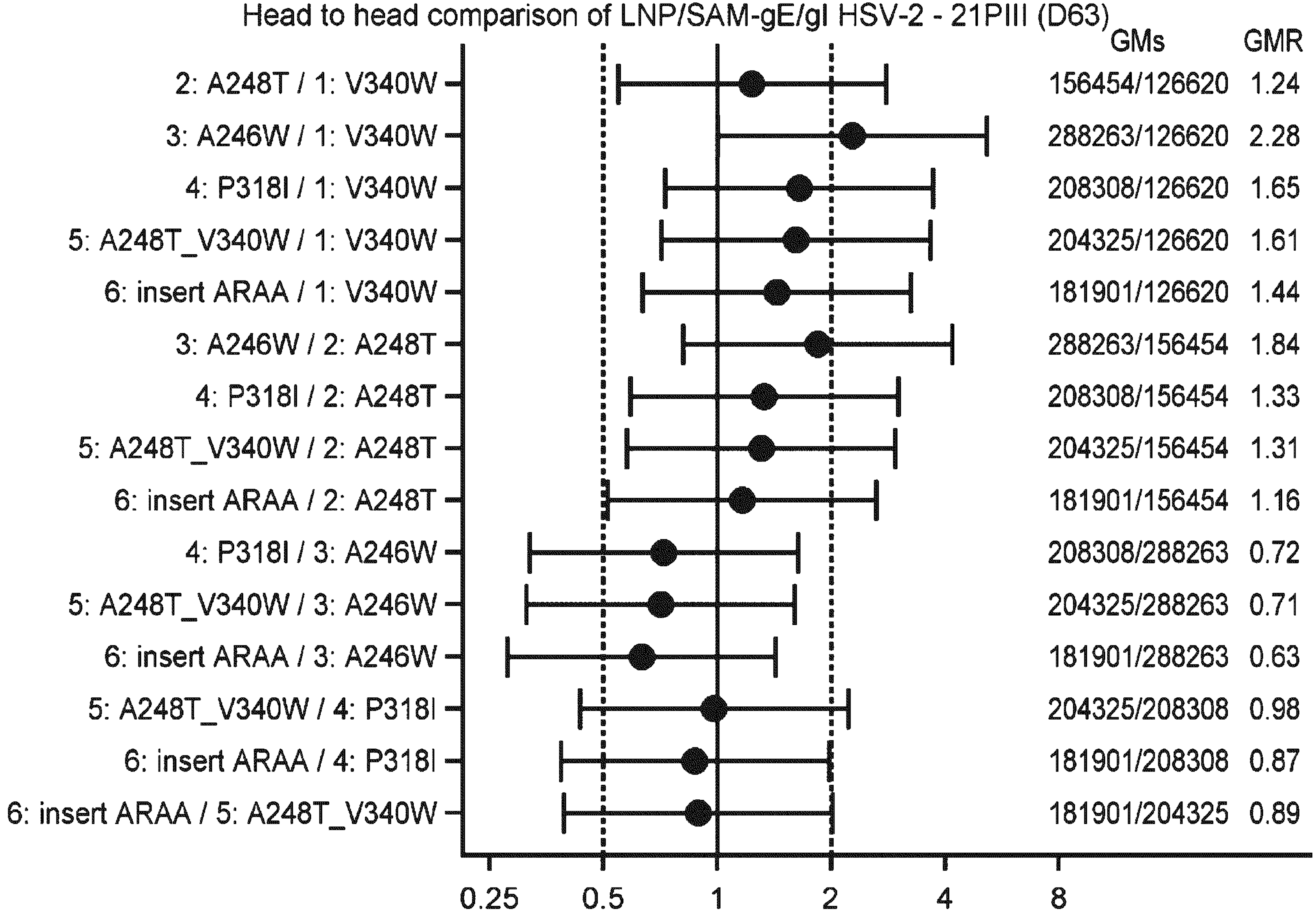

# FIG. 23A

**HSV-2 specific neutralizing antibody response induced 21 days after the third immunization with different mutated versions of LNP-SAM HSV-2 gE/gI vector**

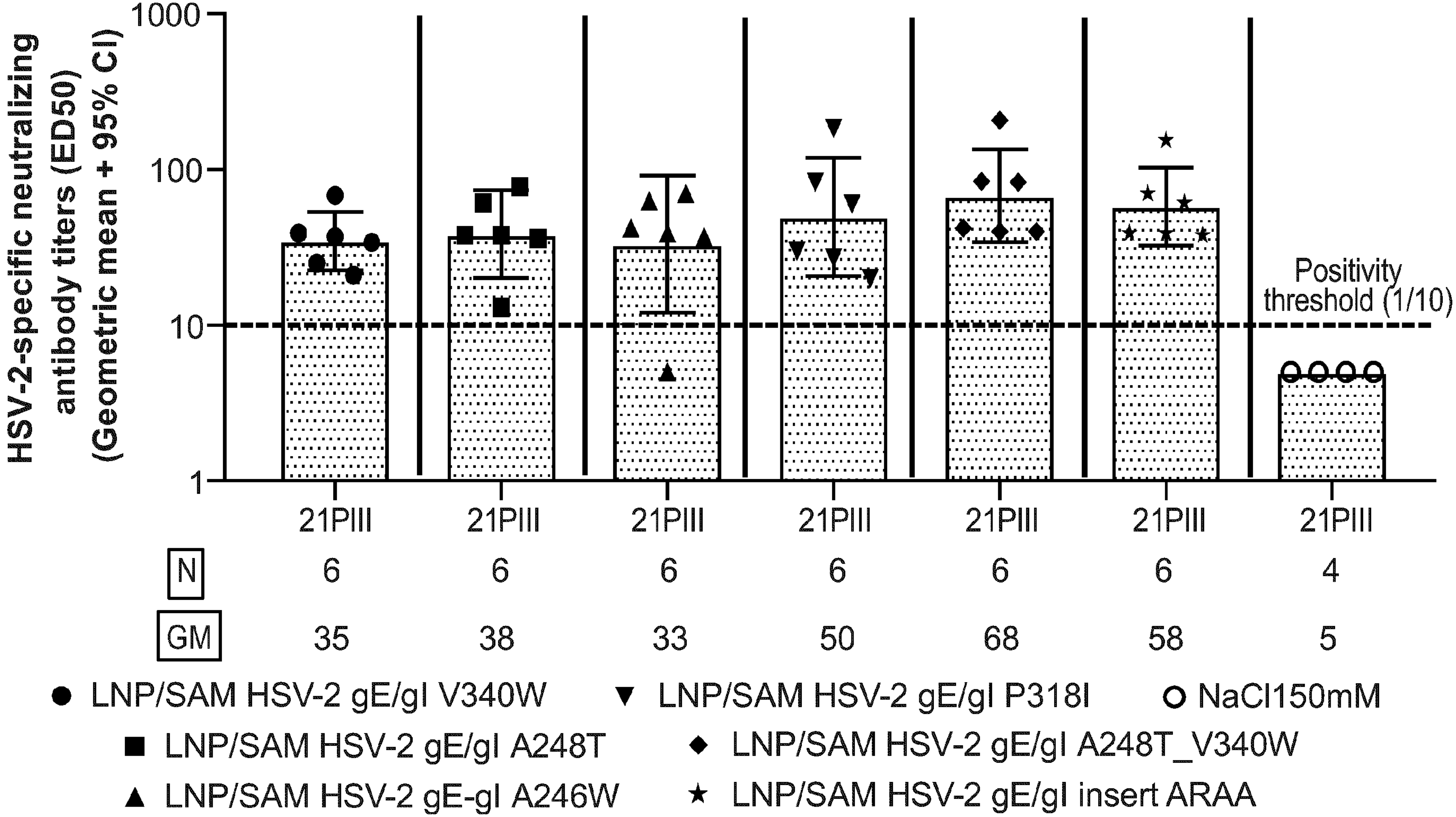

FIG. 23B

GMR with 95% CIs of HSV-2-specific neutralizing antibody titers
Head to head comparison of LNP/SAM-gE/gI HSV-2 - 21PIII (D63)

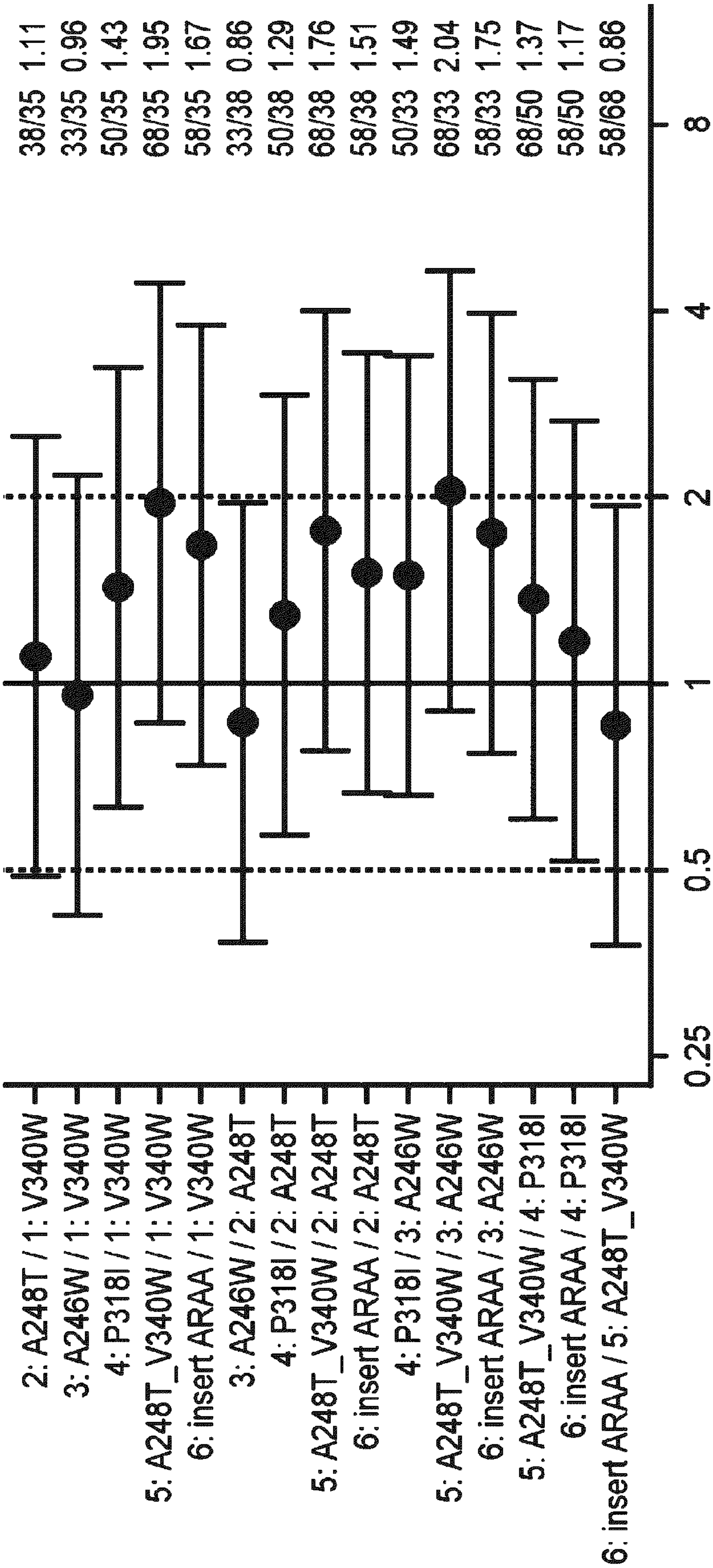

# FIG. 24A

**HSV-1 cross-reactive neutralizing antibody response induced 21 days after the third immunization with different mutated versions of LNP-SAM HSV-2 gE/gI vector**

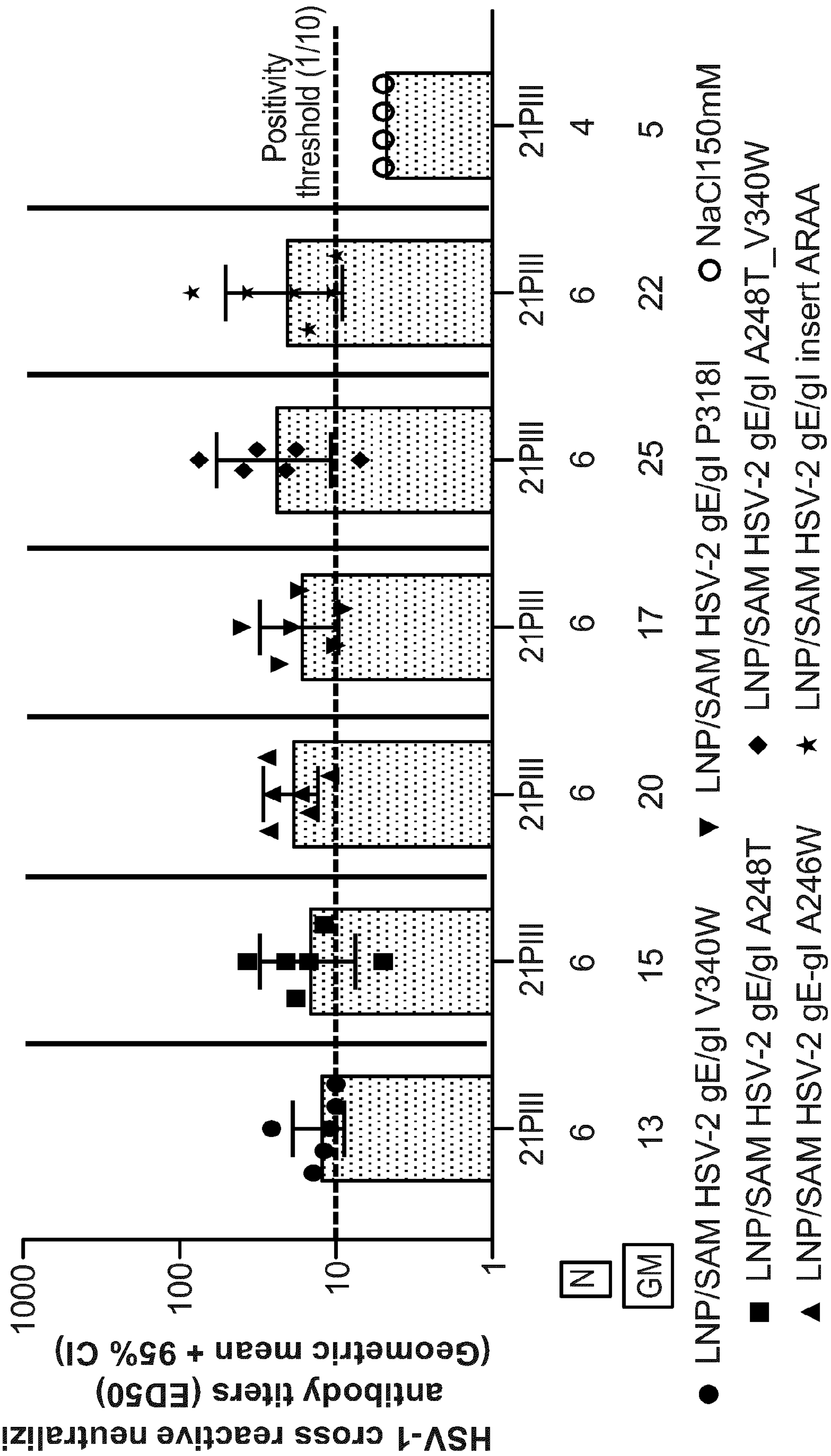

FIG. 24B

GMR with 95% CI of HSV-1 cross-reactive neutralizing antibody titers (ED50)
Head to head comparison of LNP/SAM HSV-2 gE/gI groups - 21PIII (Day63)

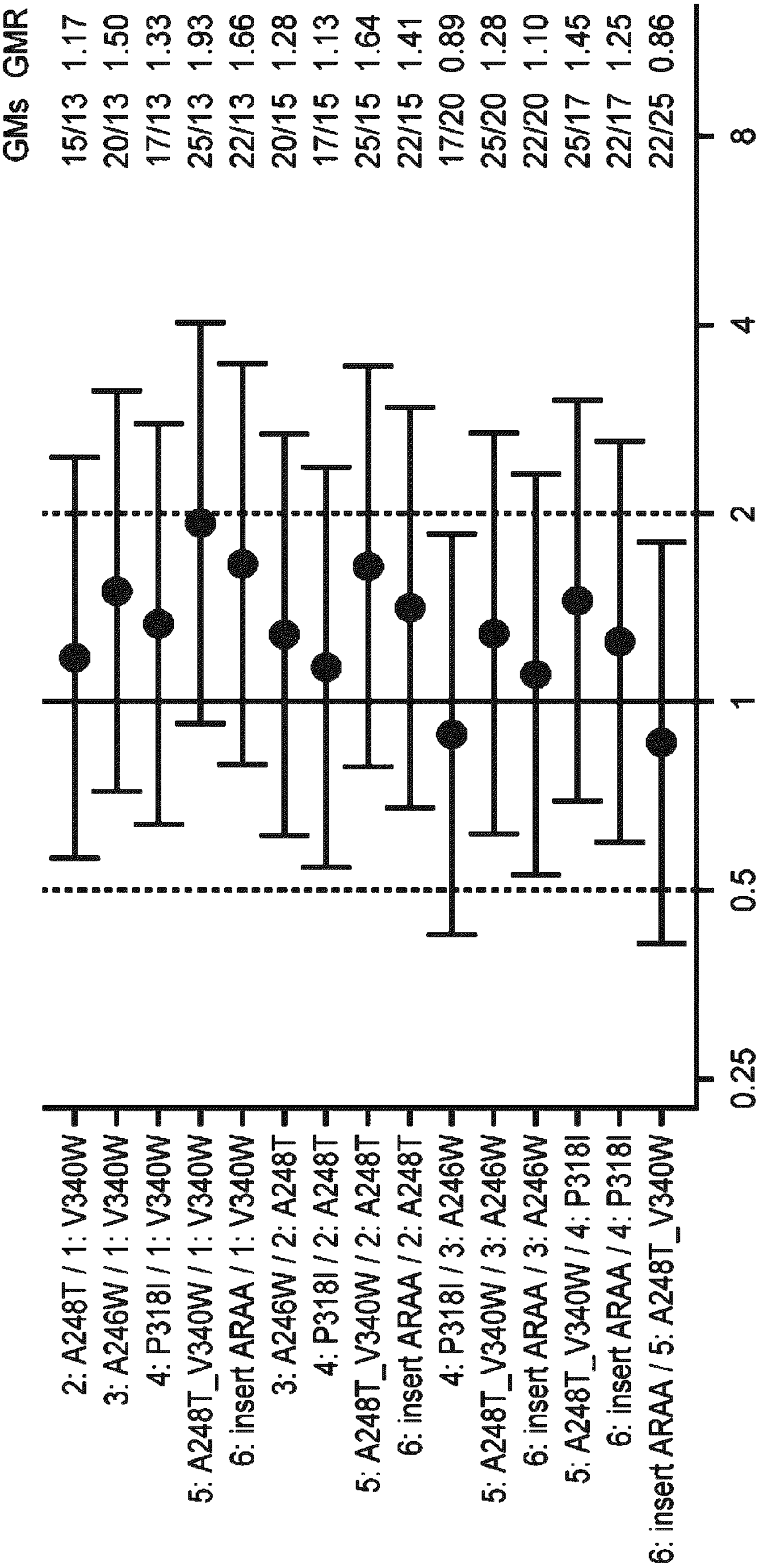

# FIG. 25A

**Co-incubation of hIgG with serum samples collected 21 days post third immunization with LNP-SAM HSV-2 gE/gI V340W**

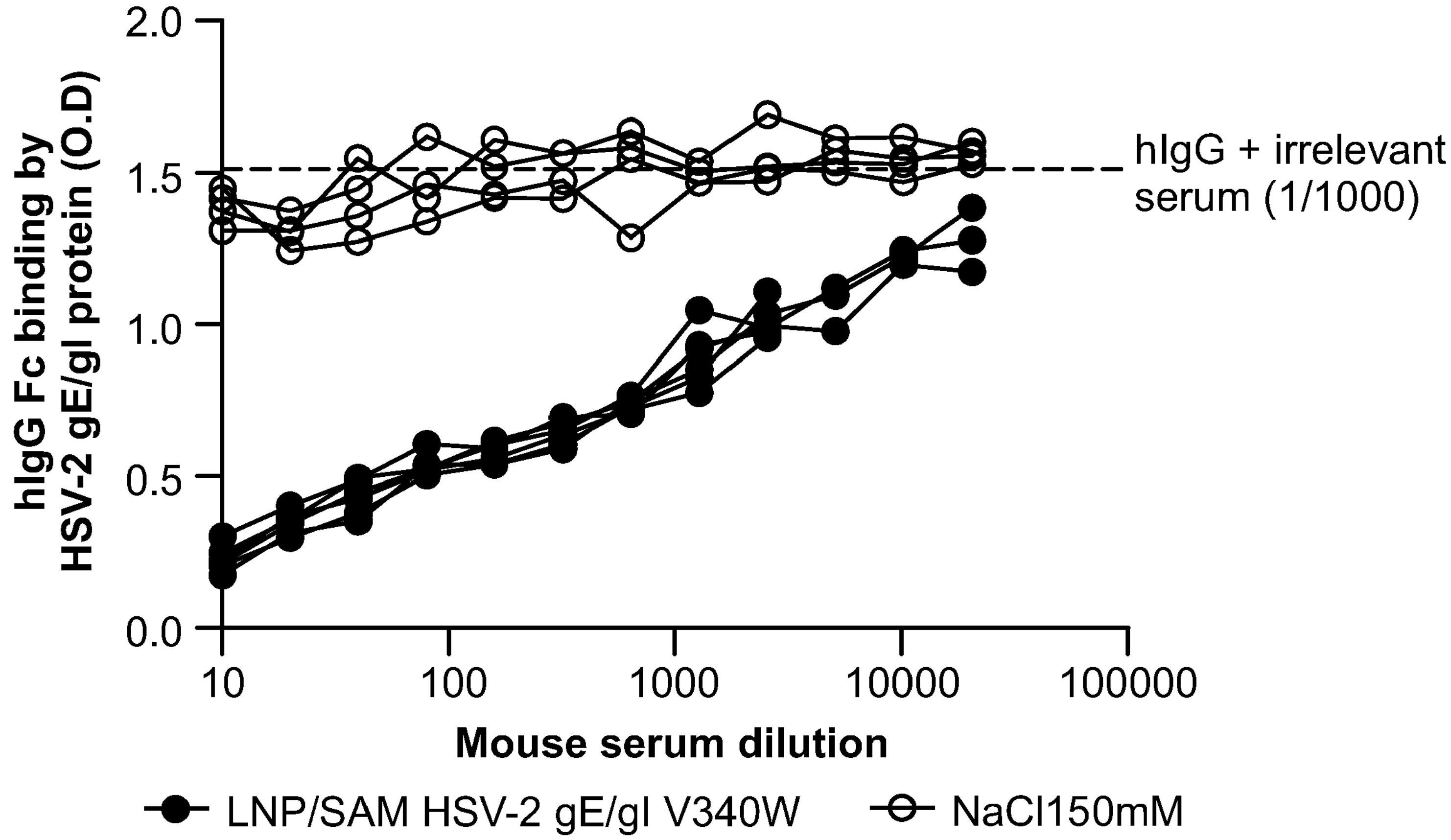

# FIG. 25B

**Co-incubation of hIgG with serum samples collected 21 days post third immunization with LNP-SAM HSV-2 gE/gI A248T**

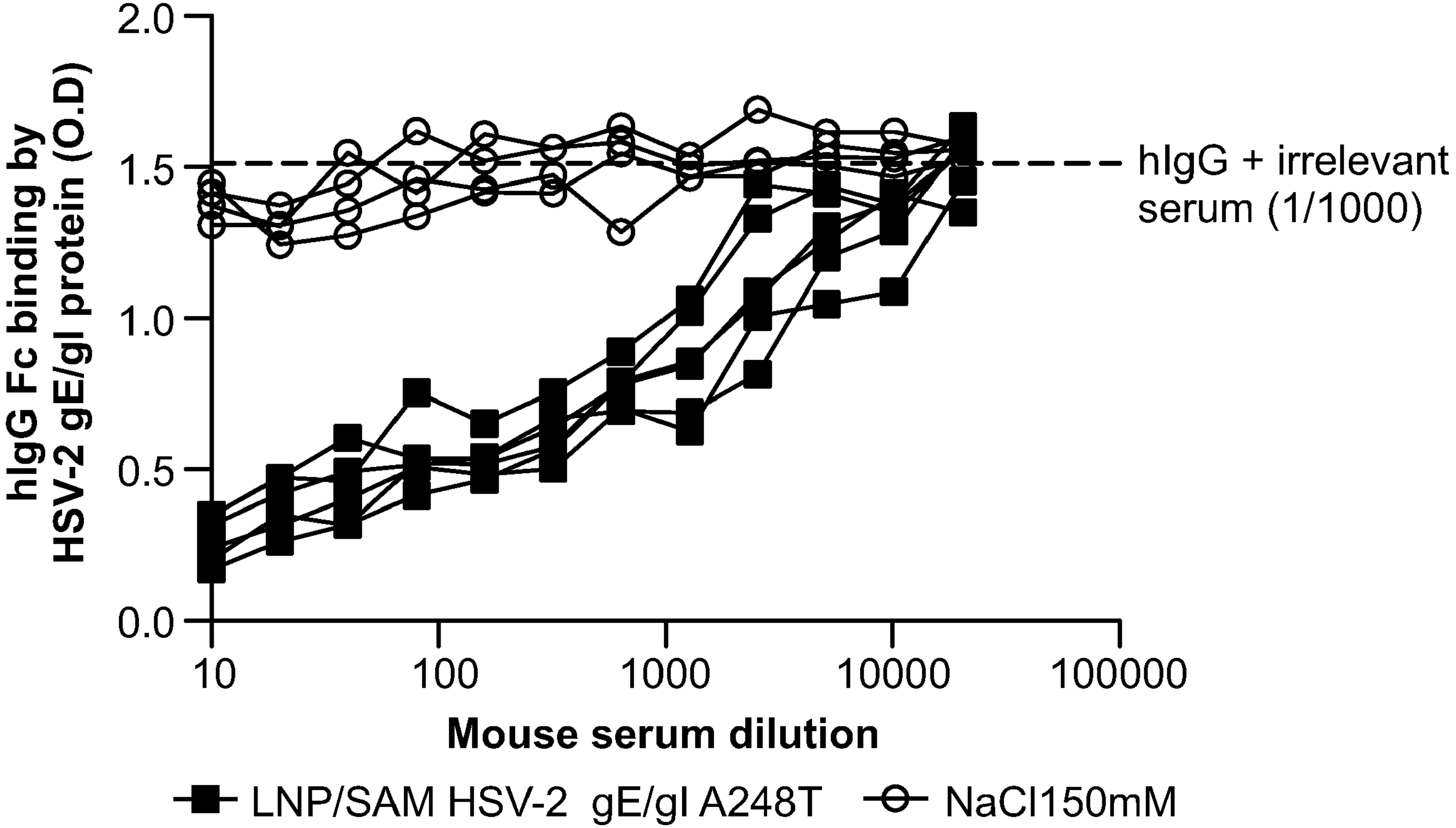

# FIG. 25C

**Co-incubation of hIgG with serum samples collected 21 days post third immunization with LNP-SAM HSV-2 gE/gI A246W**

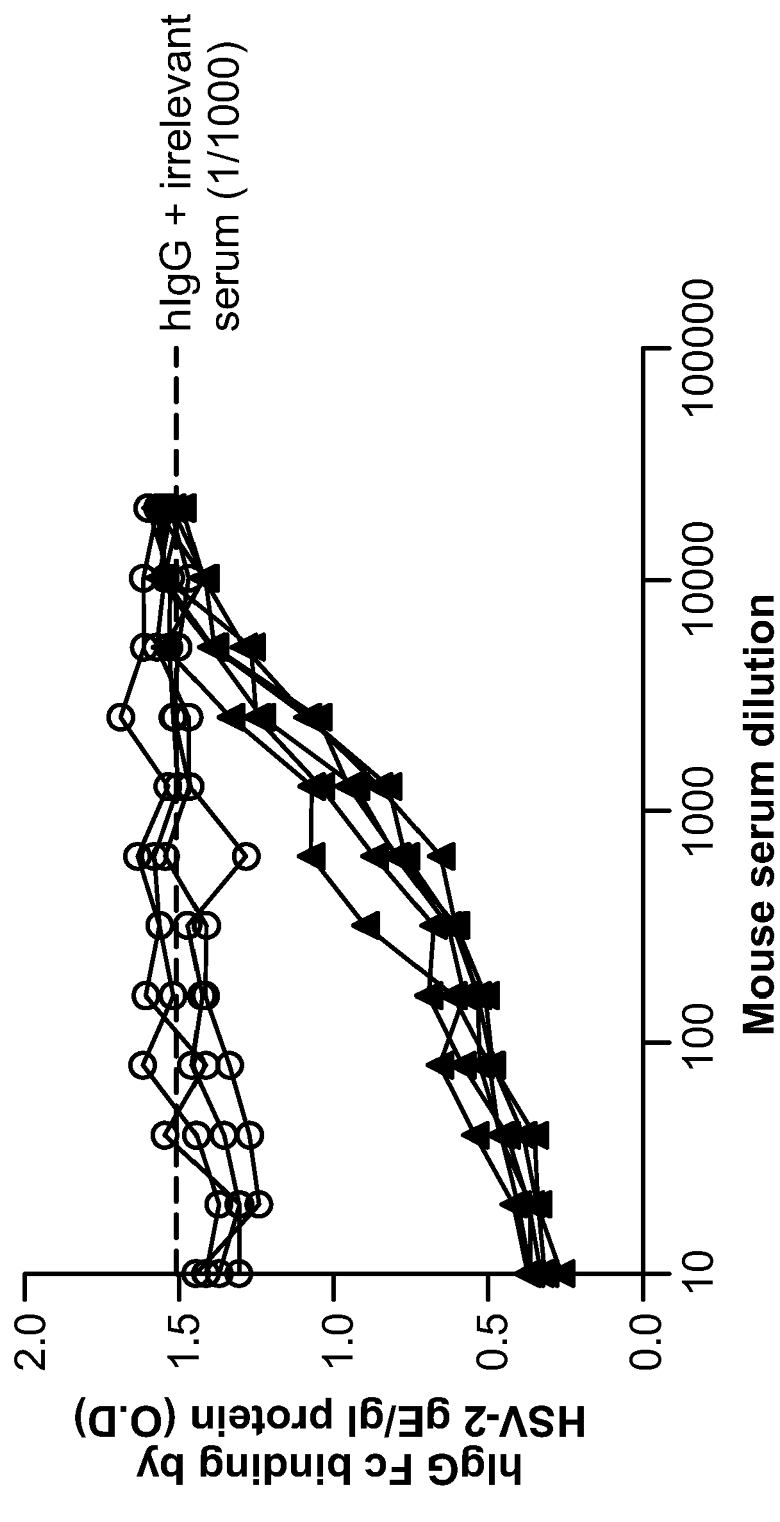


LNP-SAM HSV-2 gE/gI A246W
NaCl150mM

# FIG. 25D

**Co-incubation of hIgG with serum samples collected 21 days post third immunization with LNP-SAM HSV-2 gE/gI P318I**

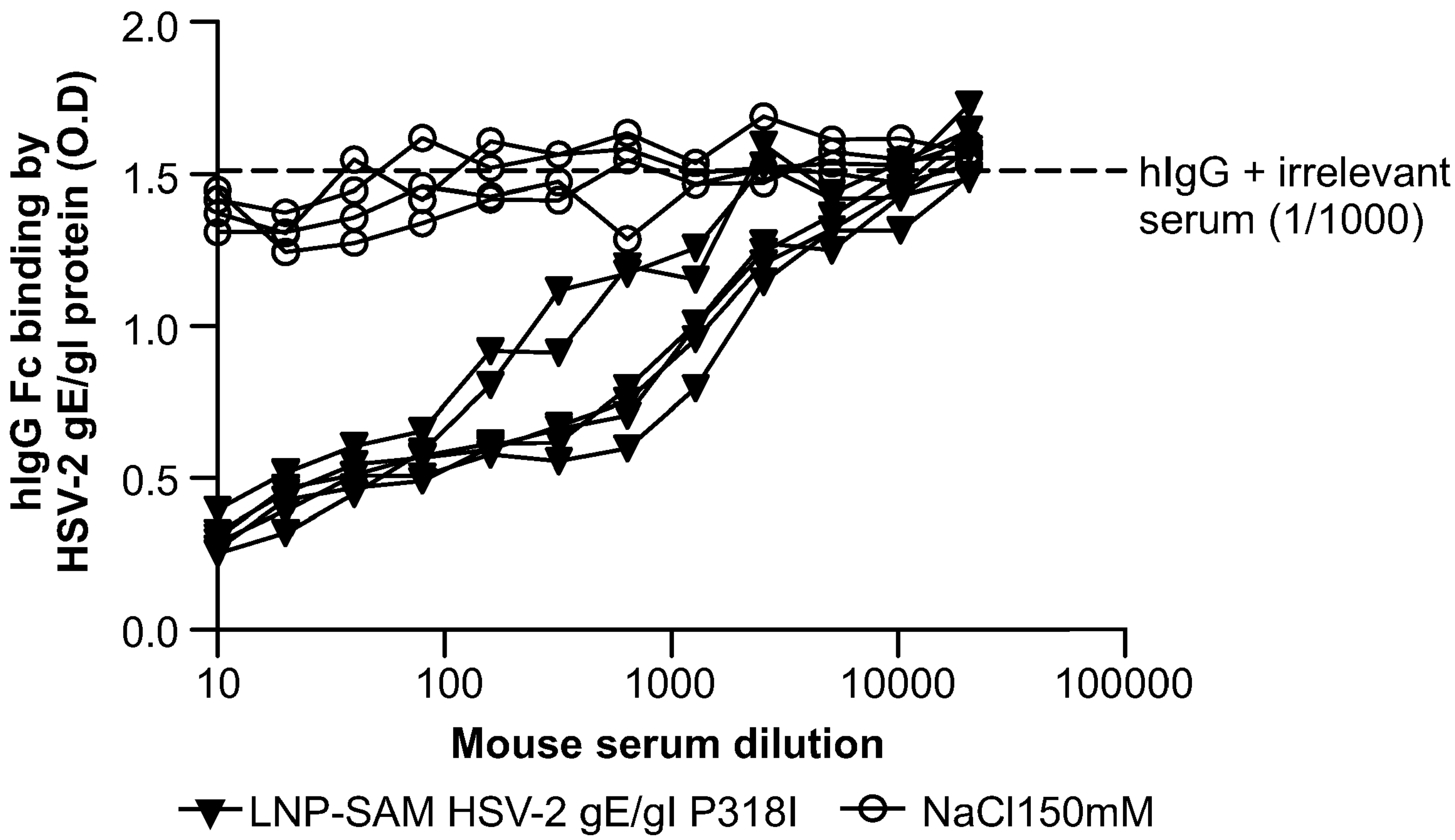

# FIG. 25E

**Co-incubation of hIgG with serum samples collected 21 days post third immunization with LNP-SAM HSV-2 gE/gI A248T_V340W**

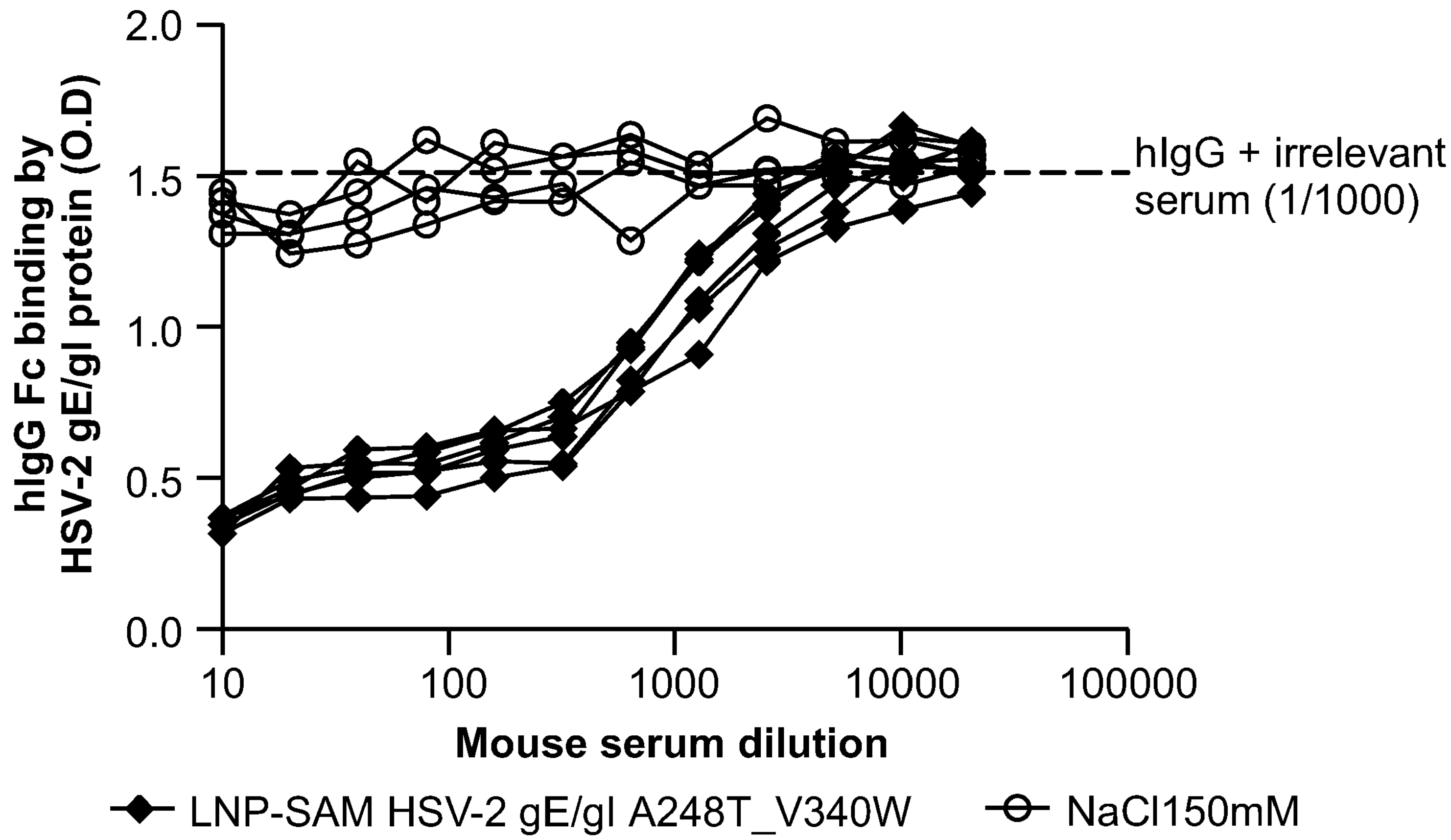

# FIG. 25F

**Co-incubation of hIgG with serum samples collected 21 days post third immunization with LNP-SAM HSV-2 gE/gI insert ARAA candidate**

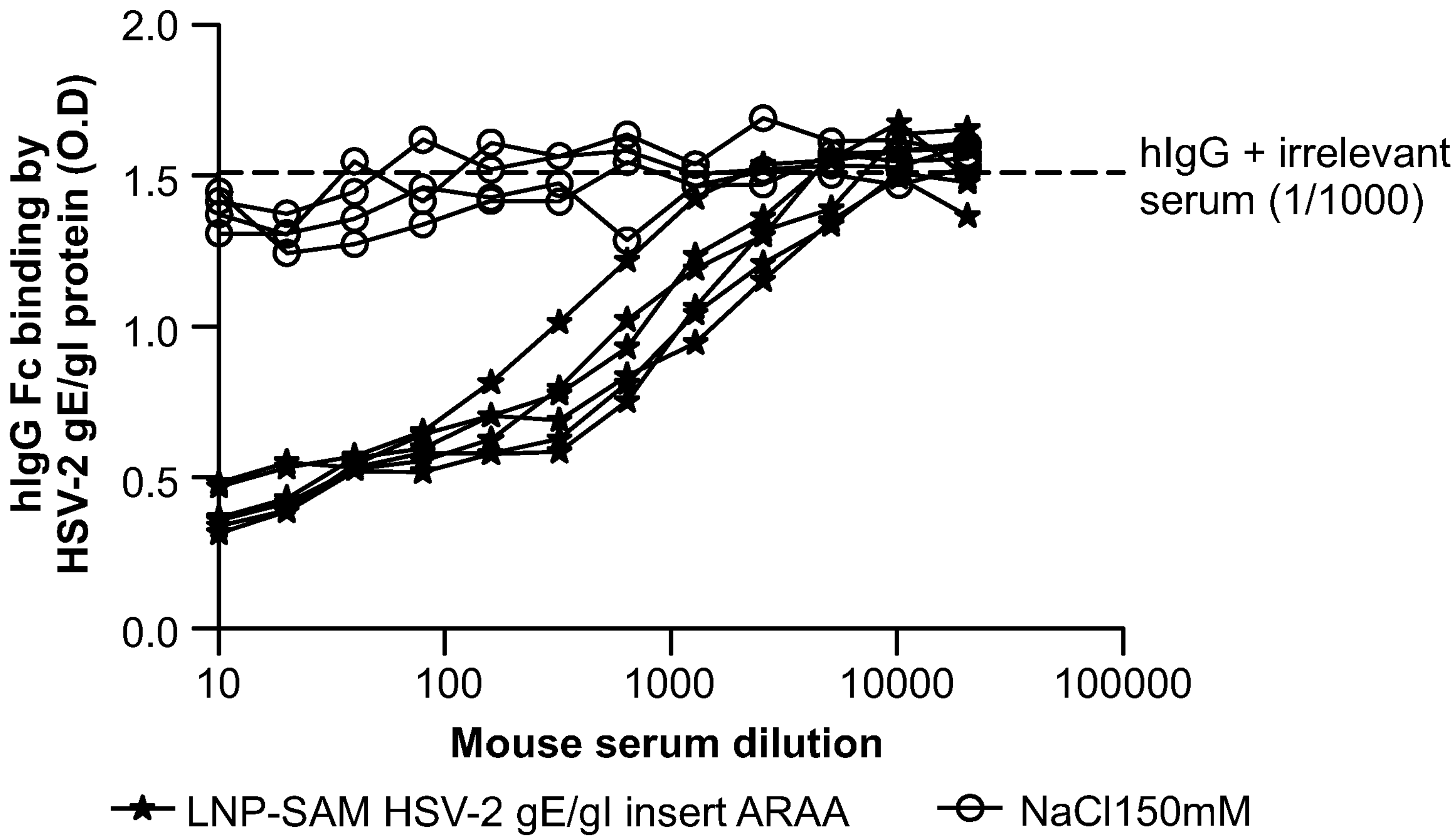

# FIG. 26

**hIgG Fc binding after co-incubation of hIgG antibodies with serum samples collected 21 days post third immunization**

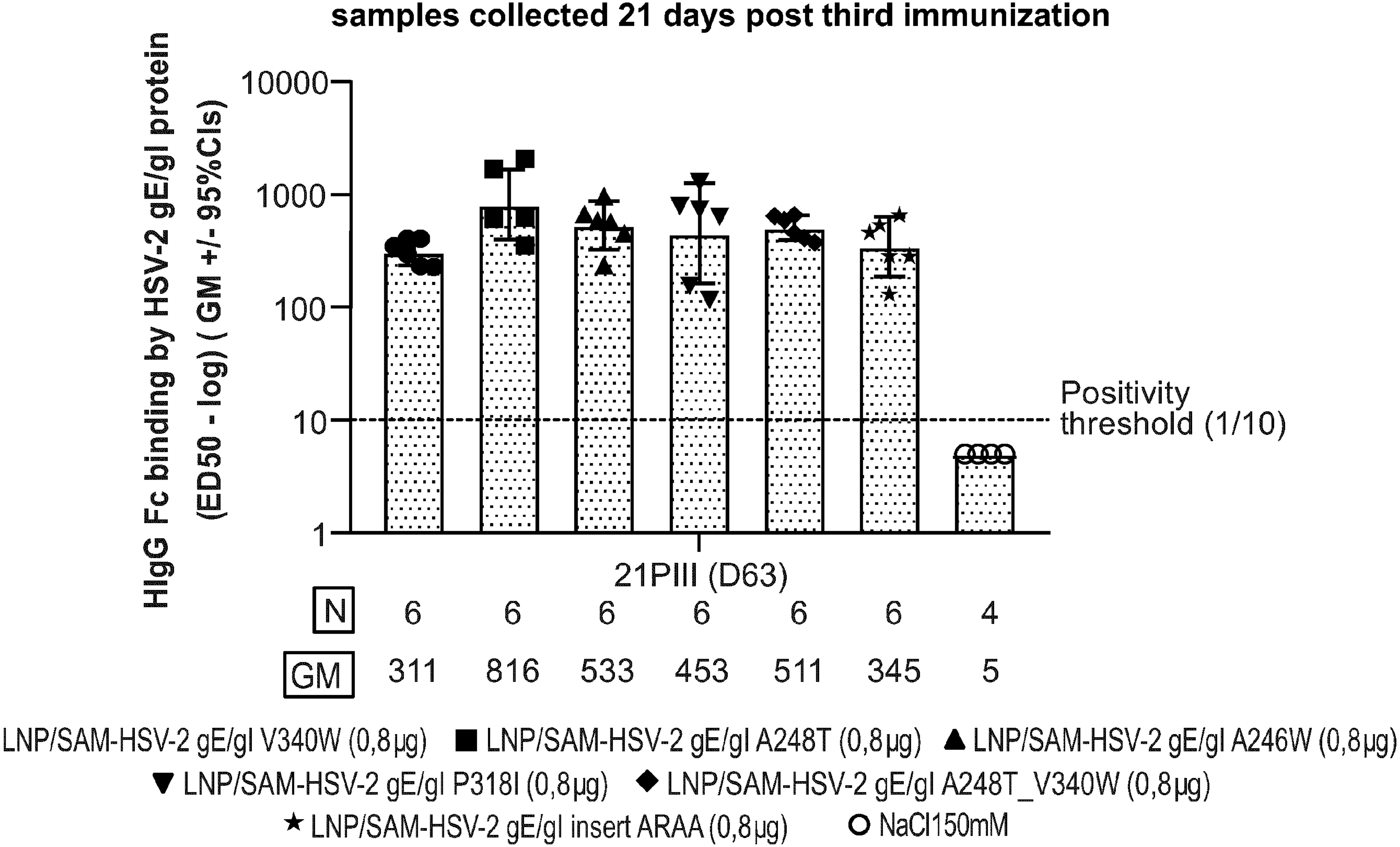

# FIG. 27A

**mFcγRIII binding activity detected in serum samples collected 21days post third immunization with LNP-formulated SAM-HSV-2 gE/gI V340W mutant candidate**

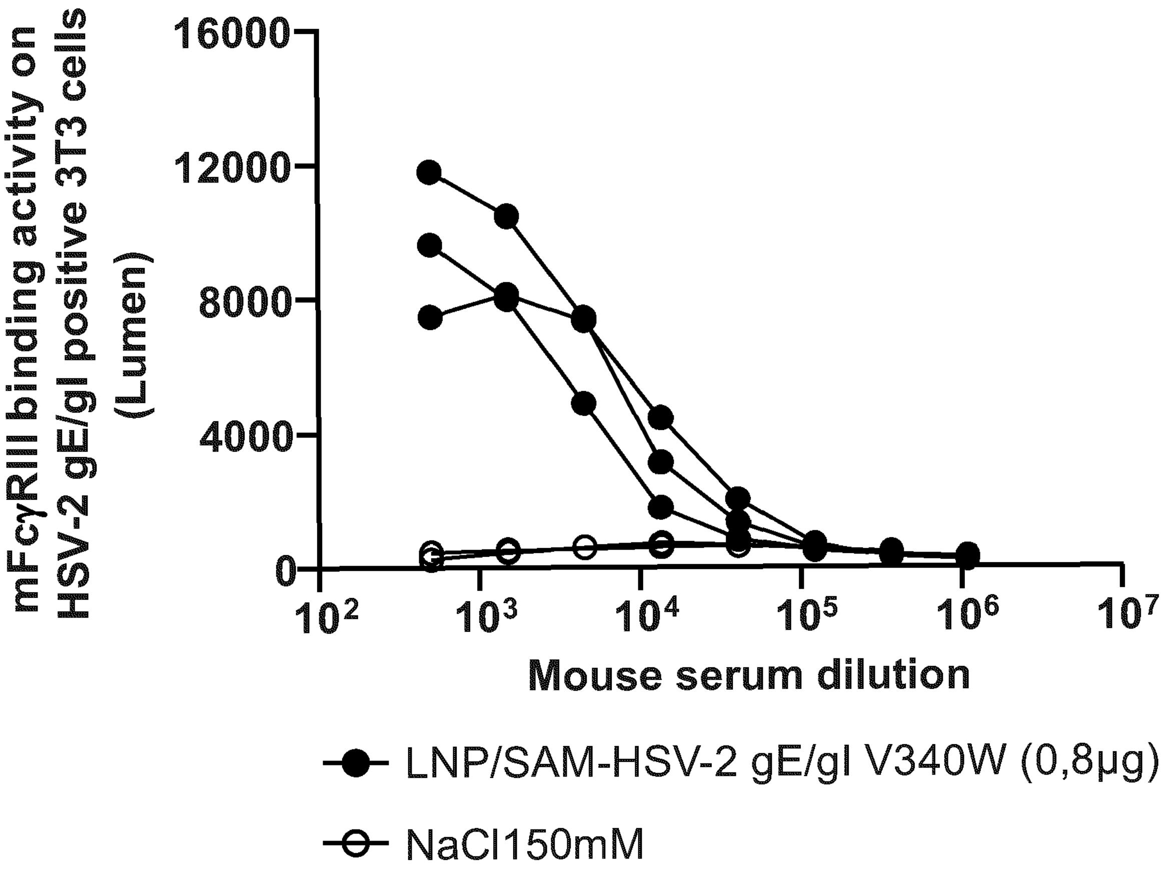

# FIG. 27B

**mFcγRIII binding activity detected in serum samples collected 21days post third immunization with LNP-formulated SAM-HSV-2 gE/gI A248T mutant candidate**

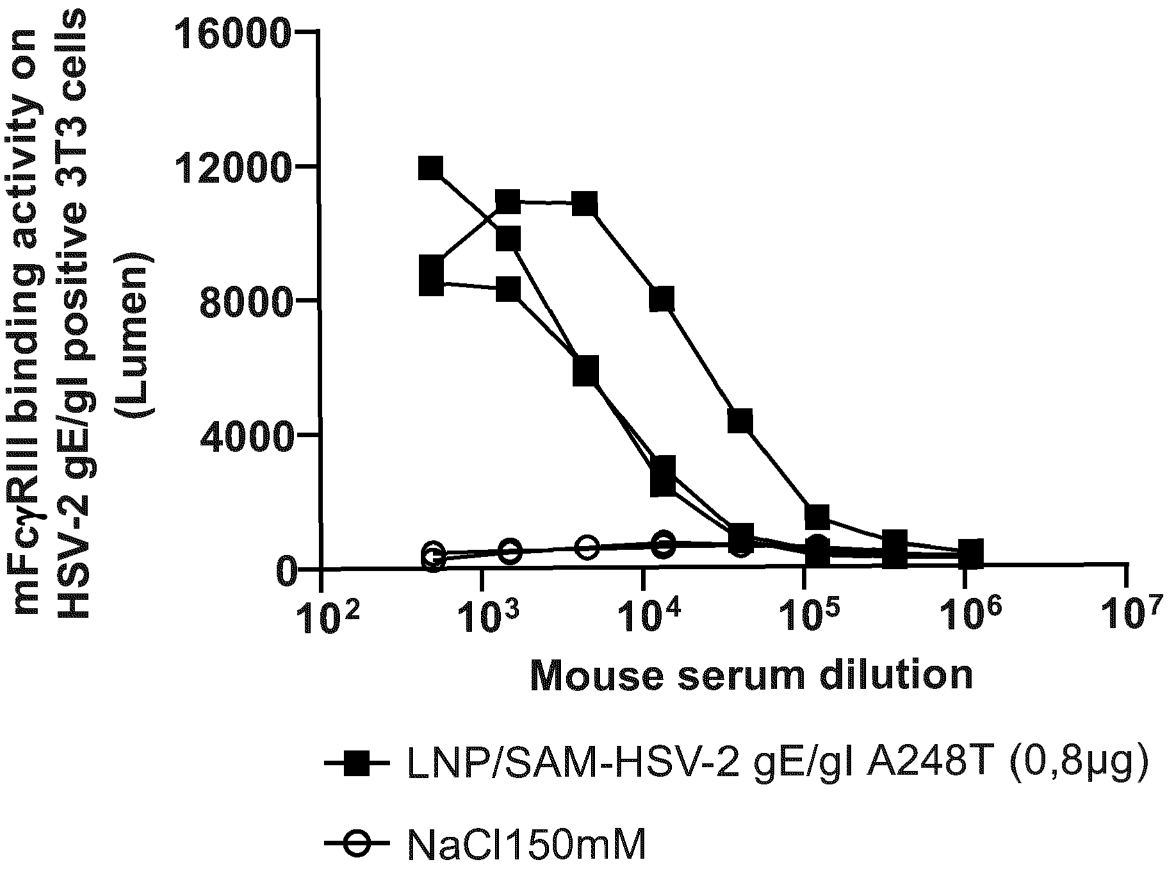

# FIG. 27C

**mFcγRIII binding activity detected in serum samples collected 21days post third immunization with LNP-formulated SAM-HSV-2 gE/gI A246W mutant candidate**

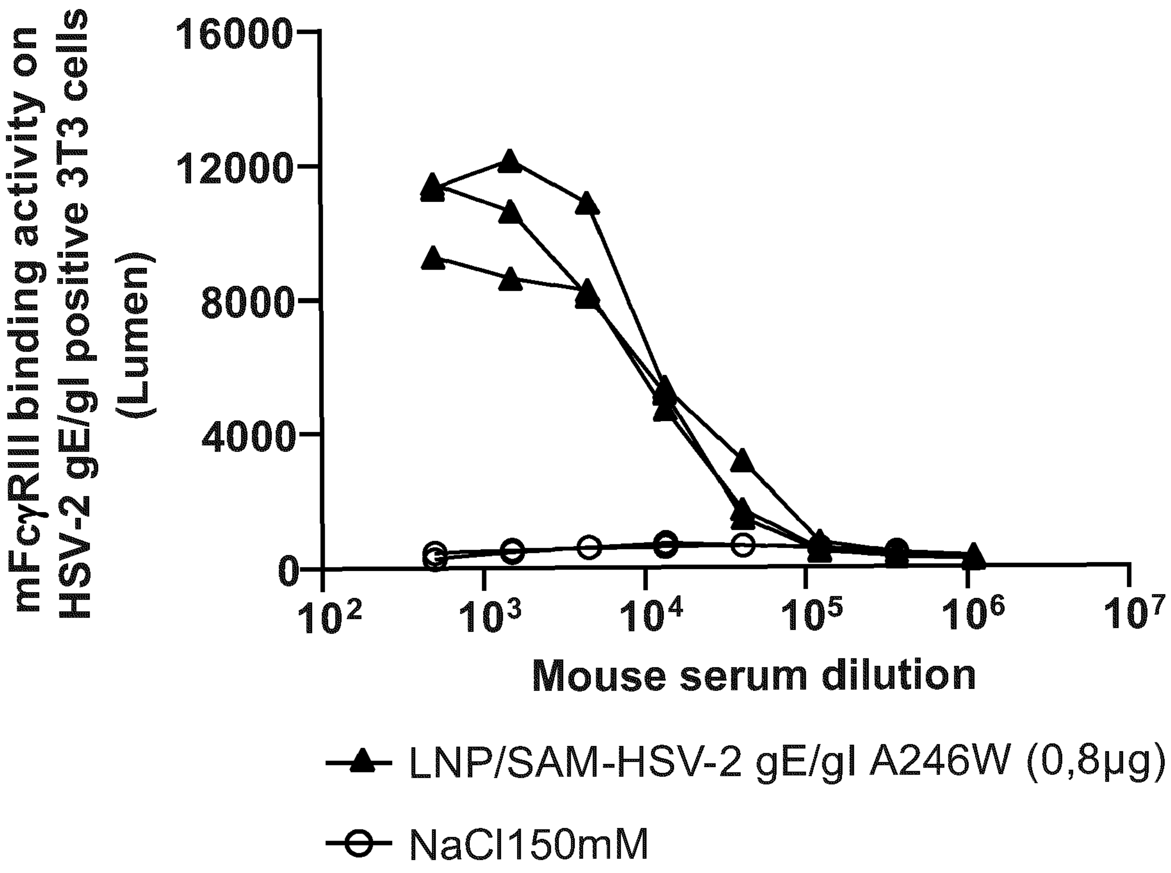

# FIG. 27D

**mFcγRIII binding activity detected in serum samples collected 21days post third immunization with LNP-formulated SAM-HSV-2 gE/gI P318I mutant candidate**

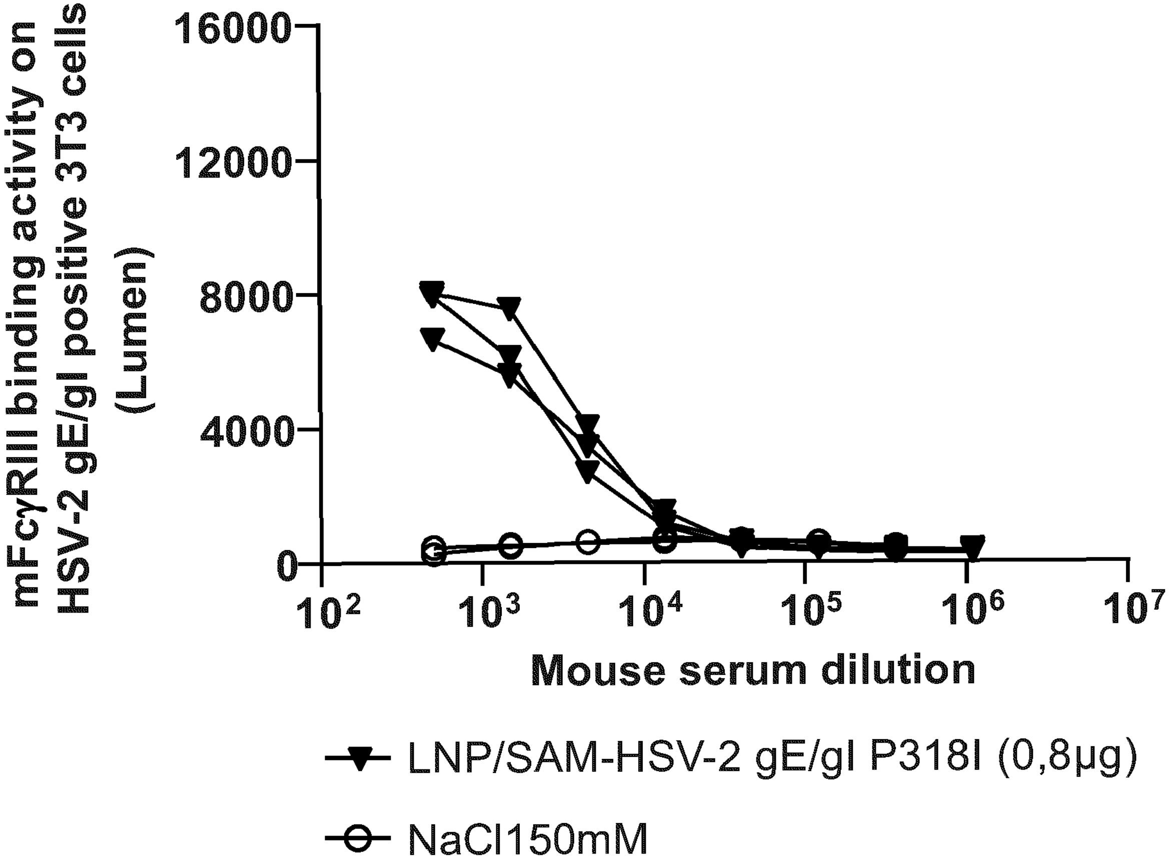

# FIG. 27E

**mFcγRIII binding activity detected in serum samples collected 21days post third immunization with LNP-formulated SAM-HSV-2 gE/gI A248T_V340W mutant candidate**

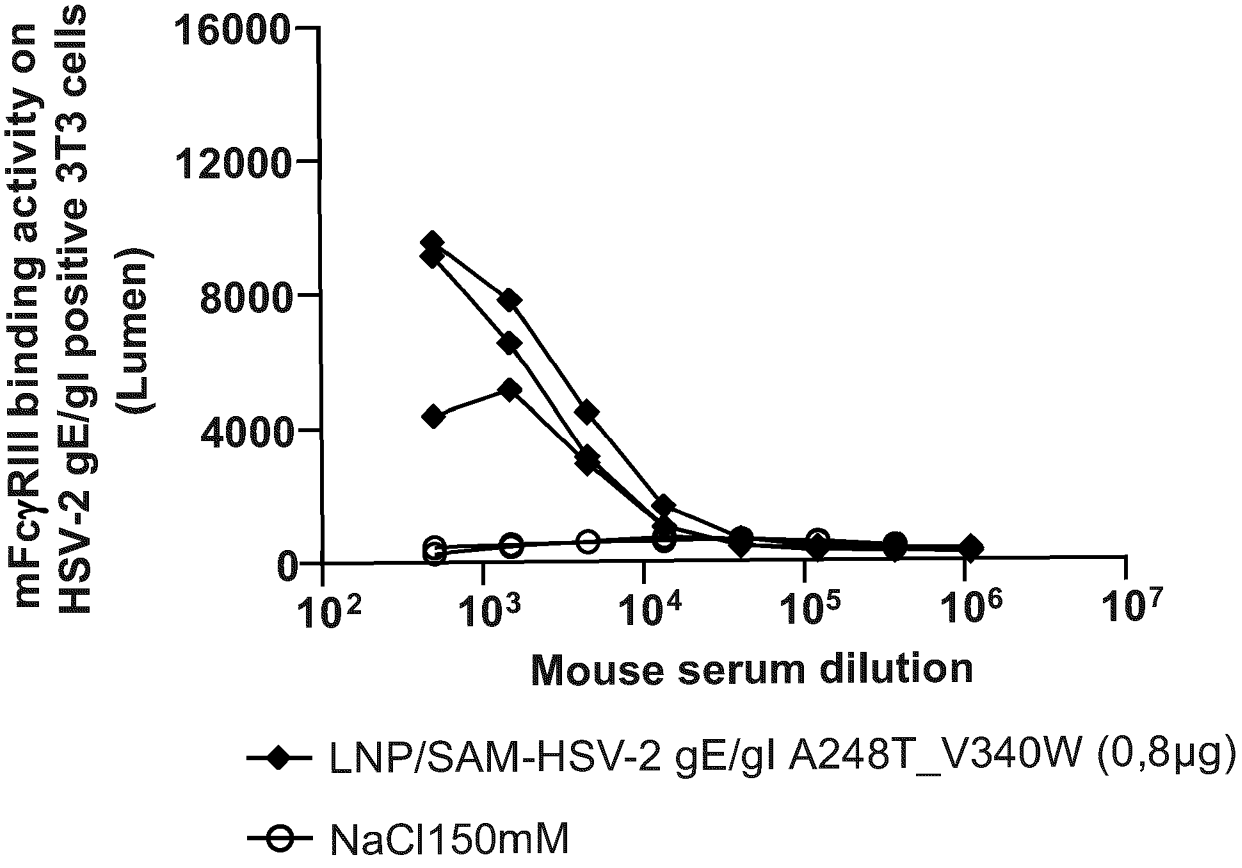

# FIG. 27F

**mFcγRIII binding activity detected in serum samples collected 21days post third immunization with LNP-formulated SAM-HSV-2 gE/gI insert ARAA mutant candidate**

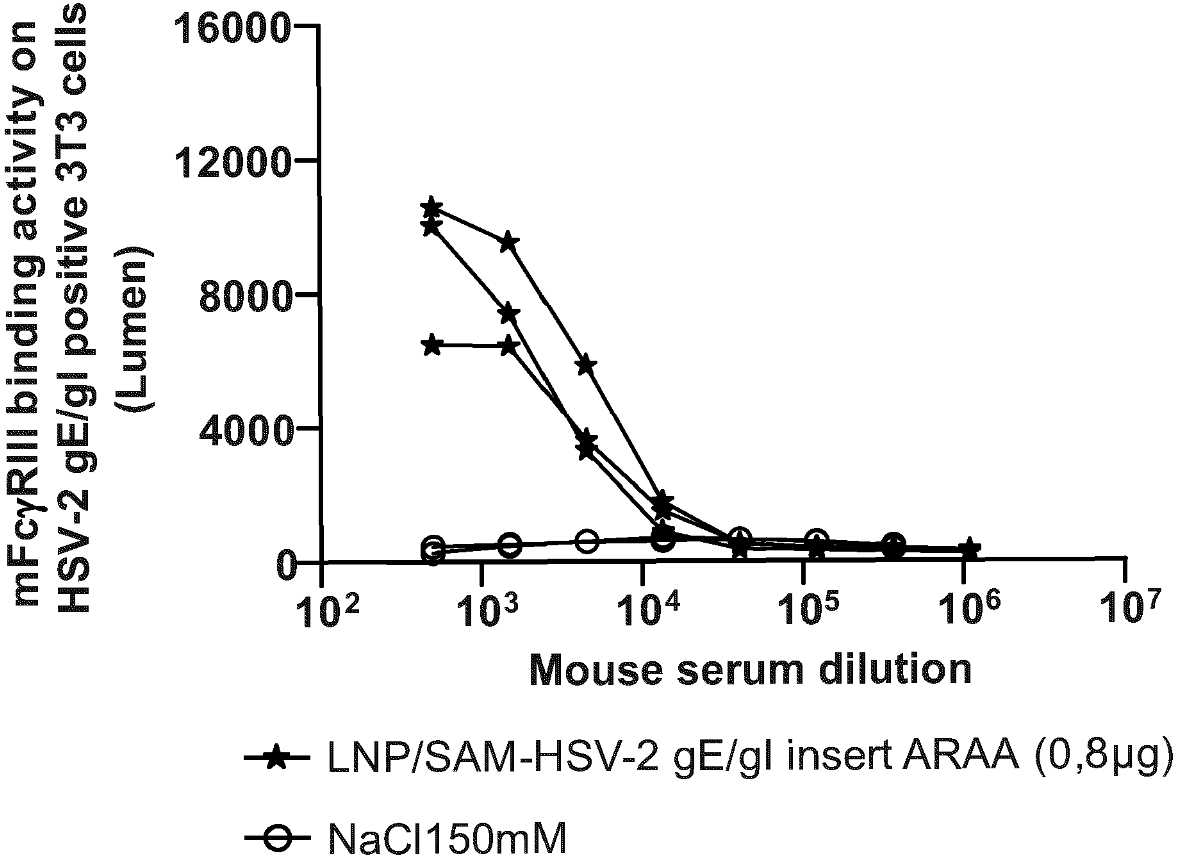

# FIG. 27G

**mFcγRIII binding activity detected in serum samples collected 21days post third immunization with different versions of LNP-formulated SAM-HSV-2 gE/gI protein**

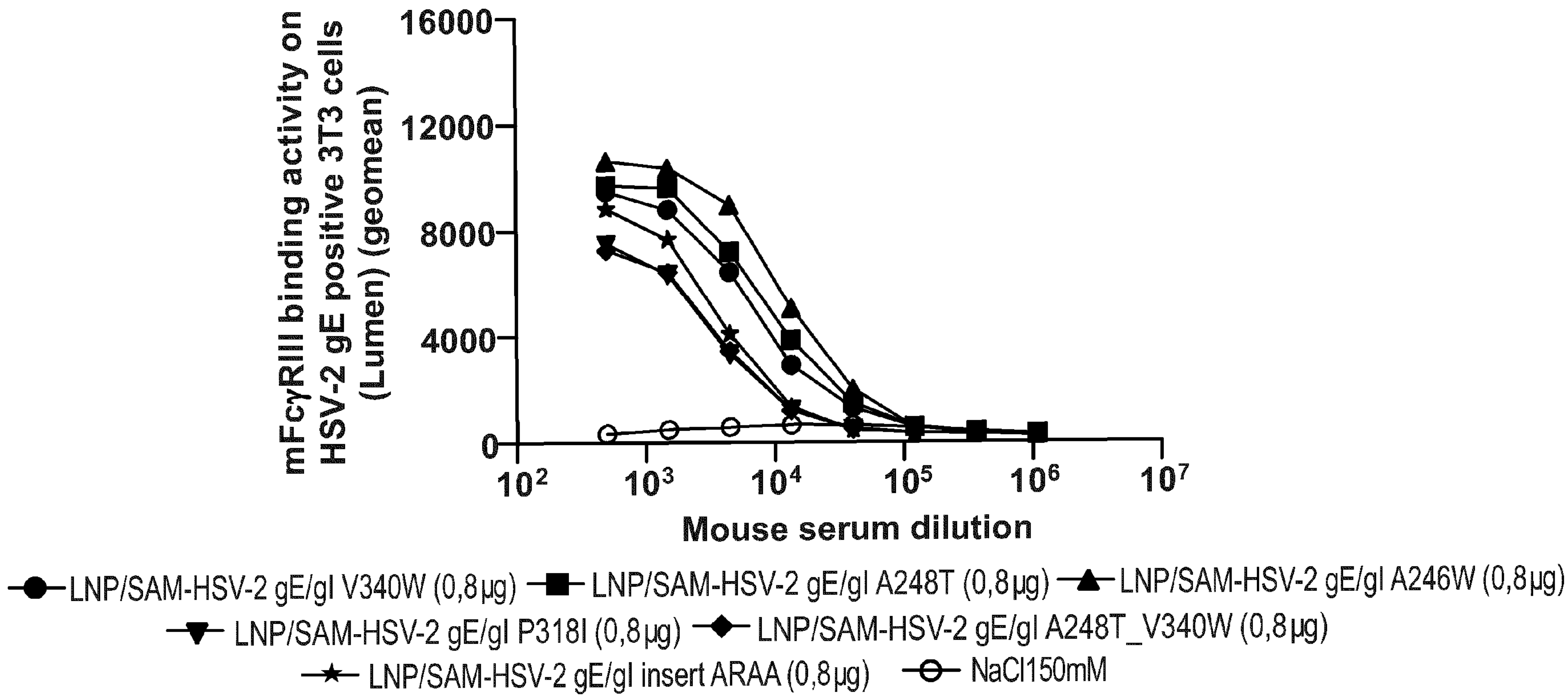

FIG. 28A

Individual results and geometric mean - 21PIII (D63)

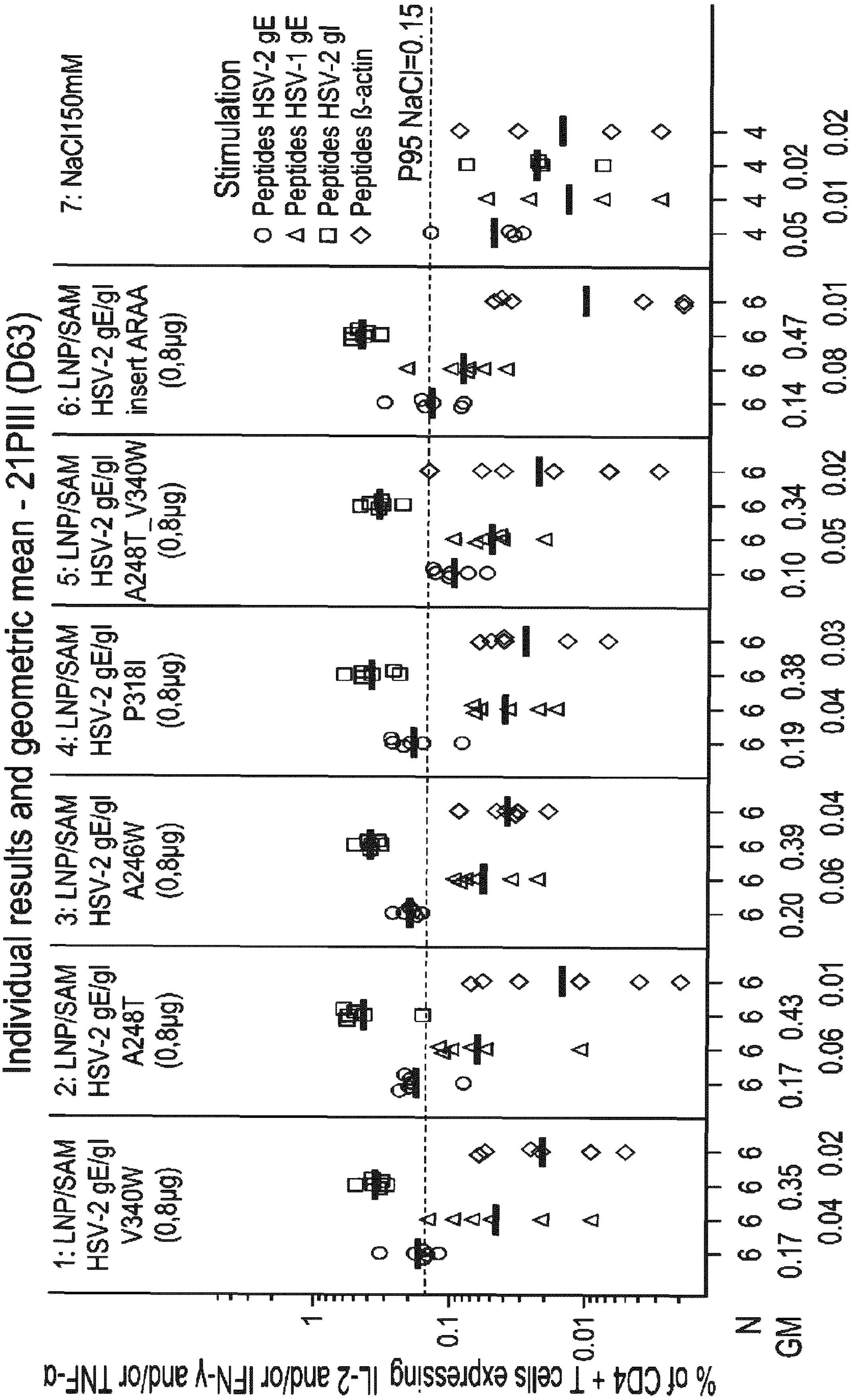

# FIG. 28B

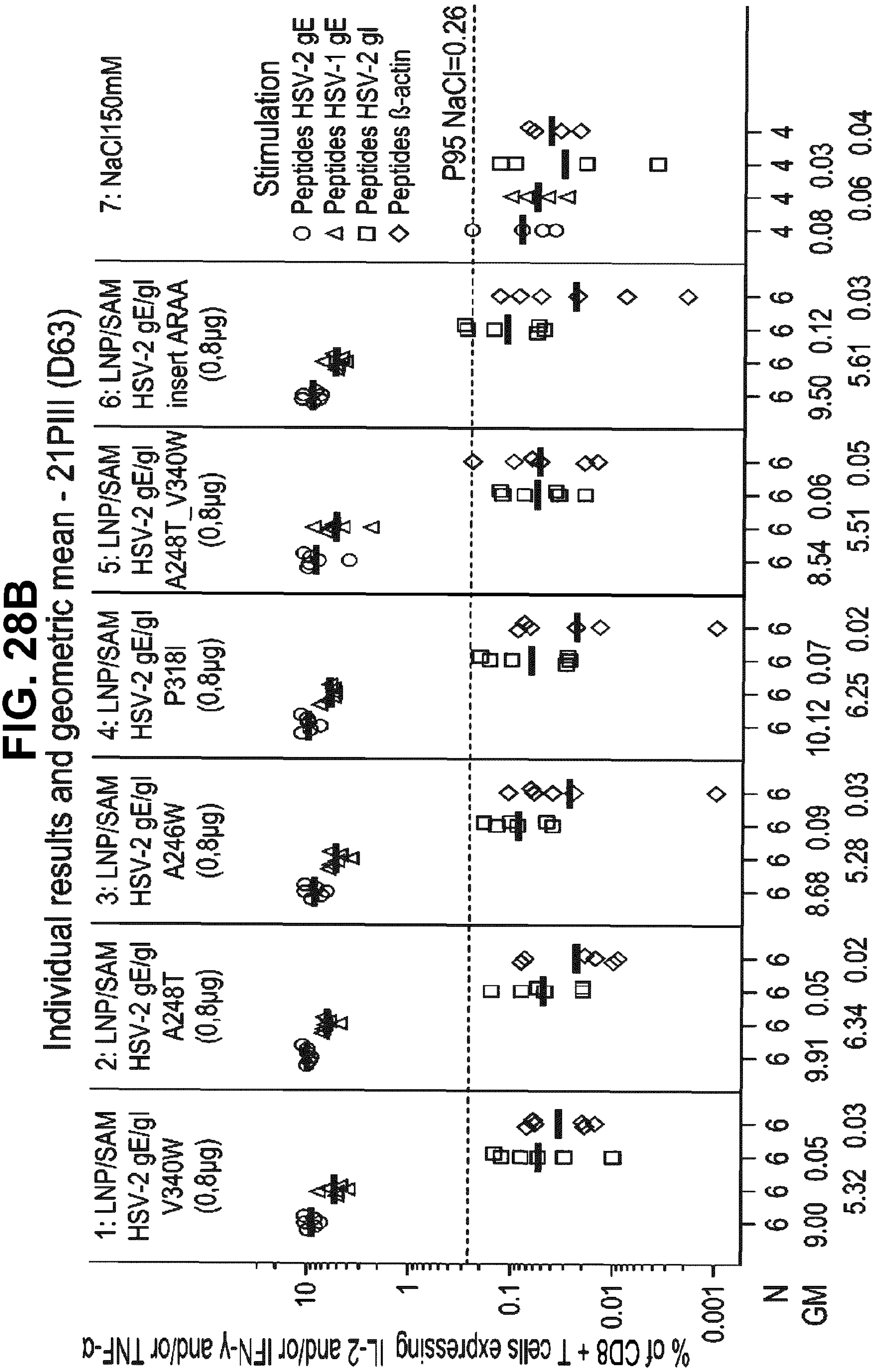
Individual results and geometric mean - 21PIII (D63)
1: LNP/SAM HSV-2 gE/gI V340W (0,8µg)
2: LNP/SAM HSV-2 gE/gI A248T (0,8µg)
3: LNP/SAM HSV-2 gE/gI A246W (0,8µg)
4: LNP/SAM HSV-2 gE/gI P318I (0,8µg)
5: LNP/SAM HSV-2 gE/gI A248T_V340W (0,8µg)
6: LNP/SAM HSV-2 gE/gI insert ARAA (0,8µg)
7: NaCl150mM
Stimulation
Peptides HSV-2 gE
Peptides HSV-1 gE
Peptides HSV-2 gI
Peptides ß-actin
P95 NaCl=0.26
% of CD8 + T cells expressing IL-2 and/or IFN-γ and/or TNF-α
10
1
0.1
0.01
0.001
N
GM
6 6 6 6
9.00 0.05
5.32 0.03
6 6 6 6
9.91 0.05
6.34 0.02
6 6 6 6
8.68 0.09
5.28 0.03
6 6 6 6
10.12 0.07
6.25 0.02
6 6 6 6
8.54 0.06
5.51 0.05
6 6 6 6
9.50 0.12
5.61 0.03
4 4 4 4
0.08 0.03
0.06 0.04

FIG. 29A

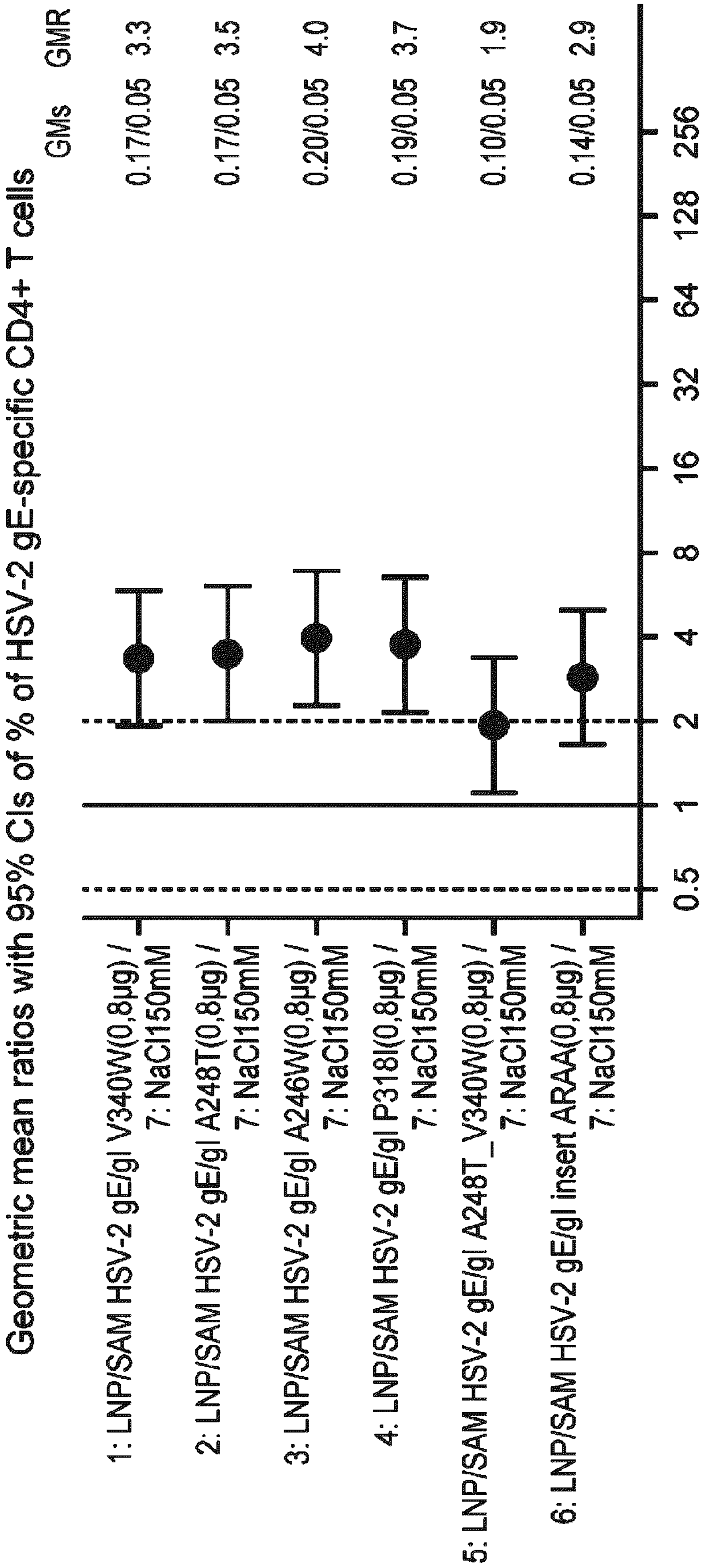
Geometric mean ratios with 95% CIs of % of HSV-2 gE-specific CD4+ T cells
GMs
GMR
1: LNP/SAM HSV-2 gE/gI V340W(0,8µg) / 7: NaCl150mM
0.17/0.05 3.3
2: LNP/SAM HSV-2 gE/gI A248T(0,8µg) / 7: NaCl150mM
0.17/0.05 3.5
3: LNP/SAM HSV-2 gE/gI A246W(0,8µg) / 7: NaCl150mM
0.20/0.05 4.0
4: LNP/SAM HSV-2 gE/gI P318I(0,8µg) / 7: NaCl150mM
0.19/0.05 3.7
5: LNP/SAM HSV-2 gE/gI A248T_ V340W(0,8µg) / 7: NaCl150mM
0.10/0.05 1.9
6: LNP/SAM HSV-2 gE/gI insert ARAA(0,8µg) / 7: NaCl150mM
0.14/0.05 2.9
0.5
1
2
4
8
16
32
64
128
256

FIG. 29B

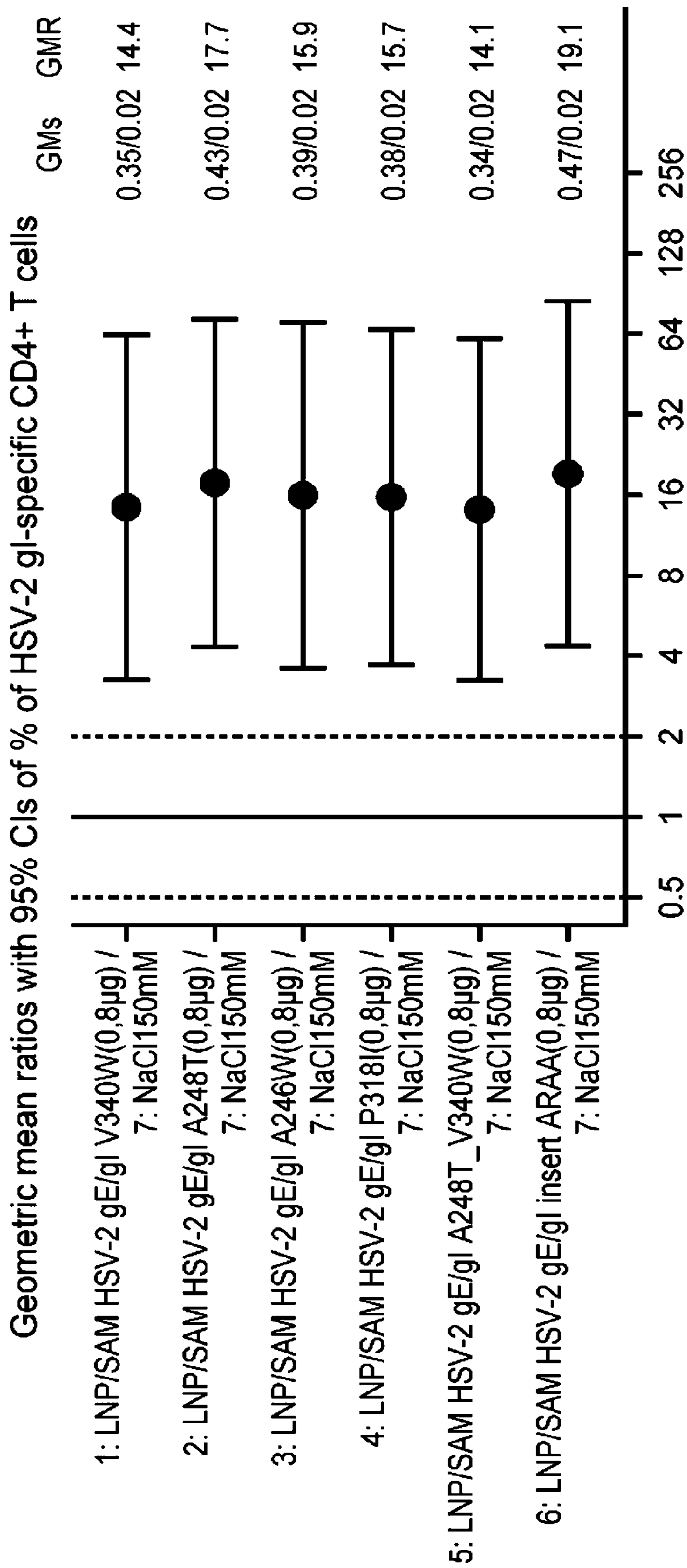
Geometric mean ratios with 95% CIs of % of HSV-2 gI-specific CD4+ T cells
GMs
GMR
1: LNP/SAM HSV-2 gE/gI V340W(0,8µg) / 7: NaCl150mM
0.35/0.02 14.4
2: LNP/SAM HSV-2 gE/gI A248T(0,8µg) / 7: NaCl150mM
0.43/0.02 17.7
3: LNP/SAM HSV-2 gE/gI A246W(0,8µg) / 7: NaCl150mM
0.39/0.02 15.9
4: LNP/SAM HSV-2 gE/gI P318I(0,8µg) / 7: NaCl150mM
0.38/0.02 15.7
5: LNP/SAM HSV-2 gE/gI A248T_V340W(0,8µg) / 7: NaCl150mM
0.34/0.02 14.1
6: LNP/SAM HSV-2 gE/gI insert ARAA(0,8µg) / 7: NaCl150mM
0.47/0.02 19.1
0.5
1
2
4
8
16
32
64
128
256

# FIG. 30A

Geometric mean ratios with 95% CIs of % of HSV-2 gE-specific CD8+ T cells

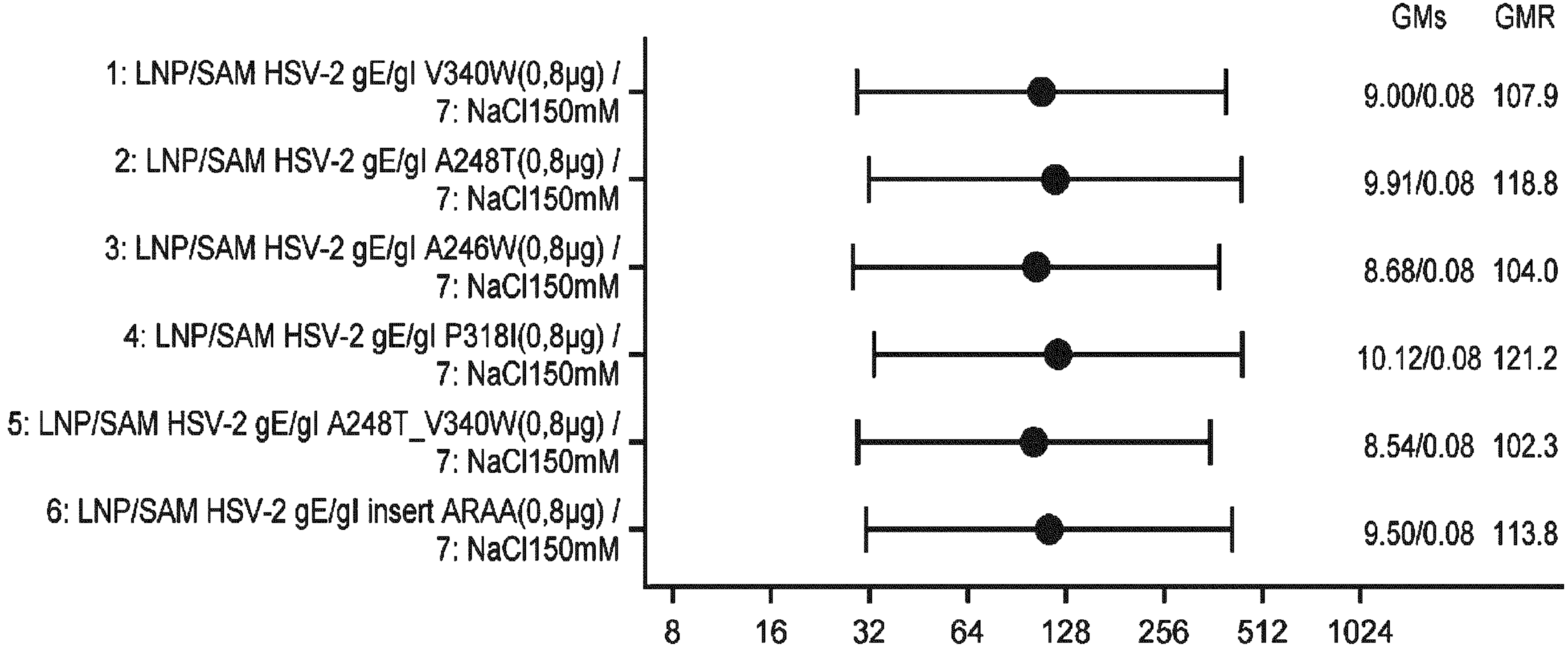

FIG. 30B

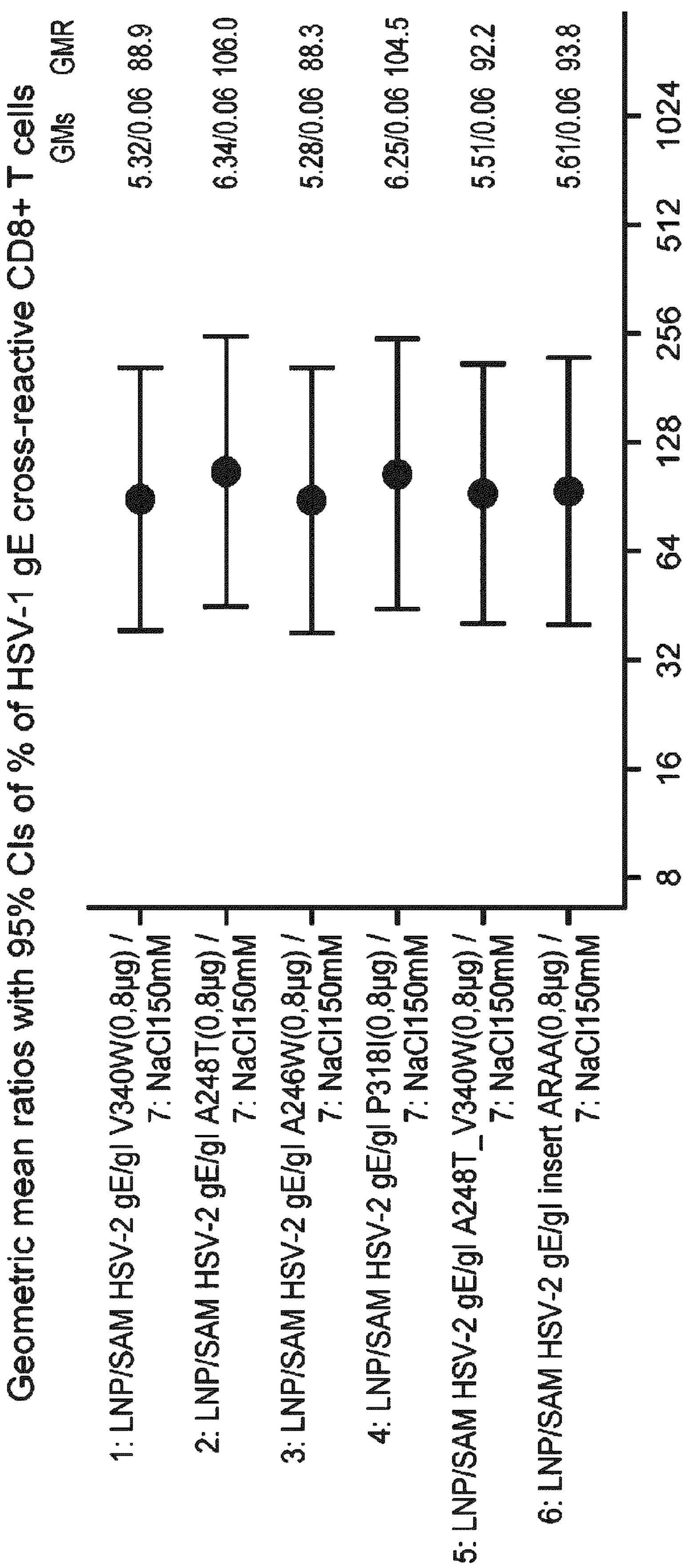
Geometric mean ratios with 95% CIs of % of HSV-1 gE cross-reactive CD8+ T cells
GMs GMR
1: LNP/SAM HSV-2 gE/gI V340W(0,8µg) / 7: NaCl150mM
5.32/0.06 88.9
2: LNP/SAM HSV-2 gE/gI A248T(0,8µg) / 7: NaCl150mM
6.34/0.06 106.0
3: LNP/SAM HSV-2 gE/gI A246W(0,8µg) / 7: NaCl150mM
5.28/0.06 88.3
4: LNP/SAM HSV-2 gE/gI P318I(0,8µg) / 7: NaCl150mM
6.25/0.06 104.5
5: LNP/SAM HSV-2 gE/gI A248T_V340W(0,8µg) / 7: NaCl150mM
5.51/0.06 92.2
6: LNP/SAM HSV-2 gE/gI insert ARAA(0,8µg) / 7: NaCl150mM
5.61/0.06 93.8
8
16
32
64
128
256
512
1024

# FIG. 31A

Geometric mean ratios with 95% CIs of % of HSV-2 gE-specific CD4+ T cells
Head to head comparison of LNP/SAM HSV-2 gE/gI(0,8µg) groups

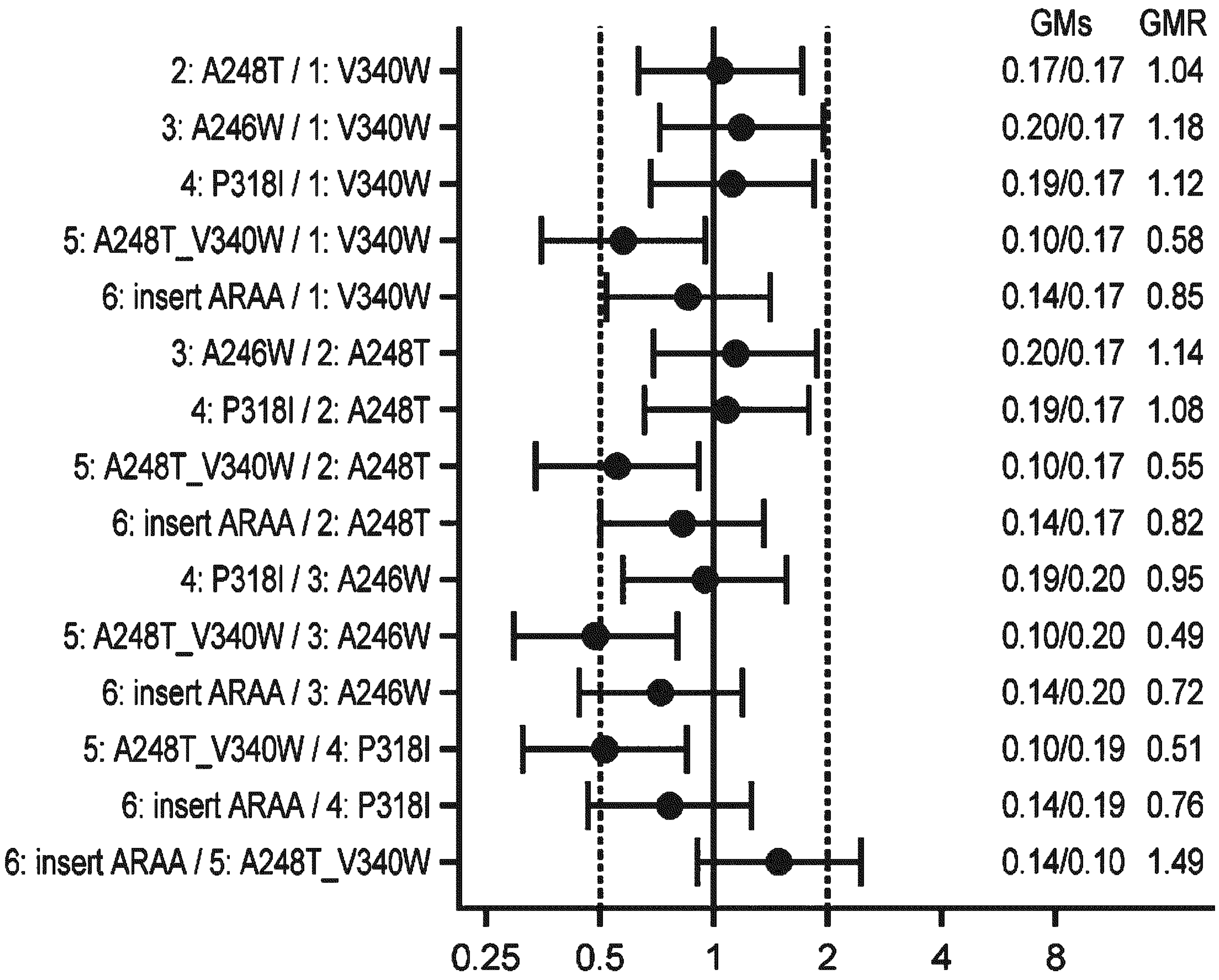

FIG. 31B Geometric mean ratios with 95% CIs of % of HSV-2 gI-specific CD4+ T cells
Head to head comparison of LNP/SAM HSV-2 gE/gI(0,8µg) groups

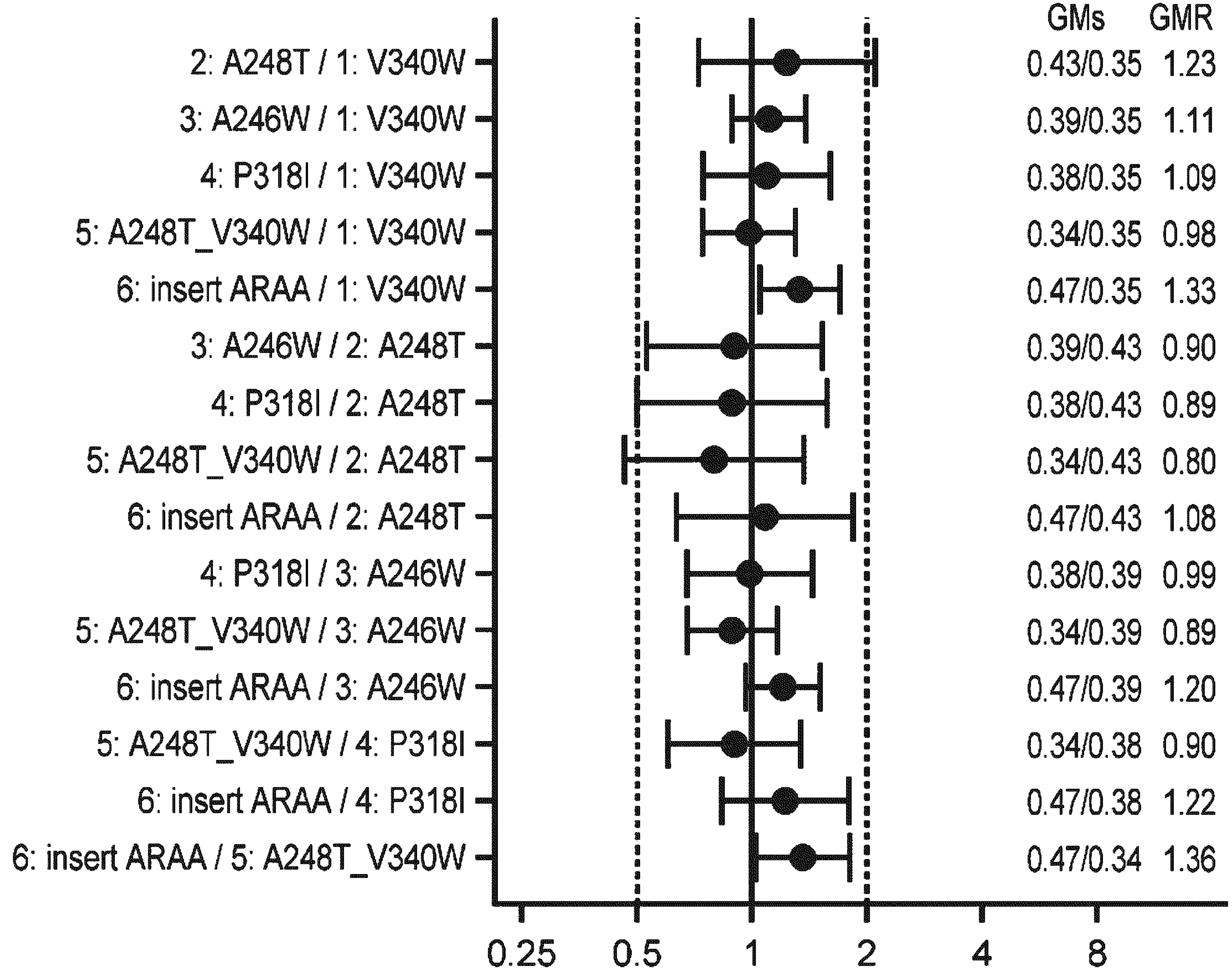

**FIG. 32A** Geometric mean ratios with 95% CIs of % of HSV-2 gE-specific CD8+ T cells
Head to head comparison of LNP/SAM HSV-2 gE/gI(0,8µg) groups

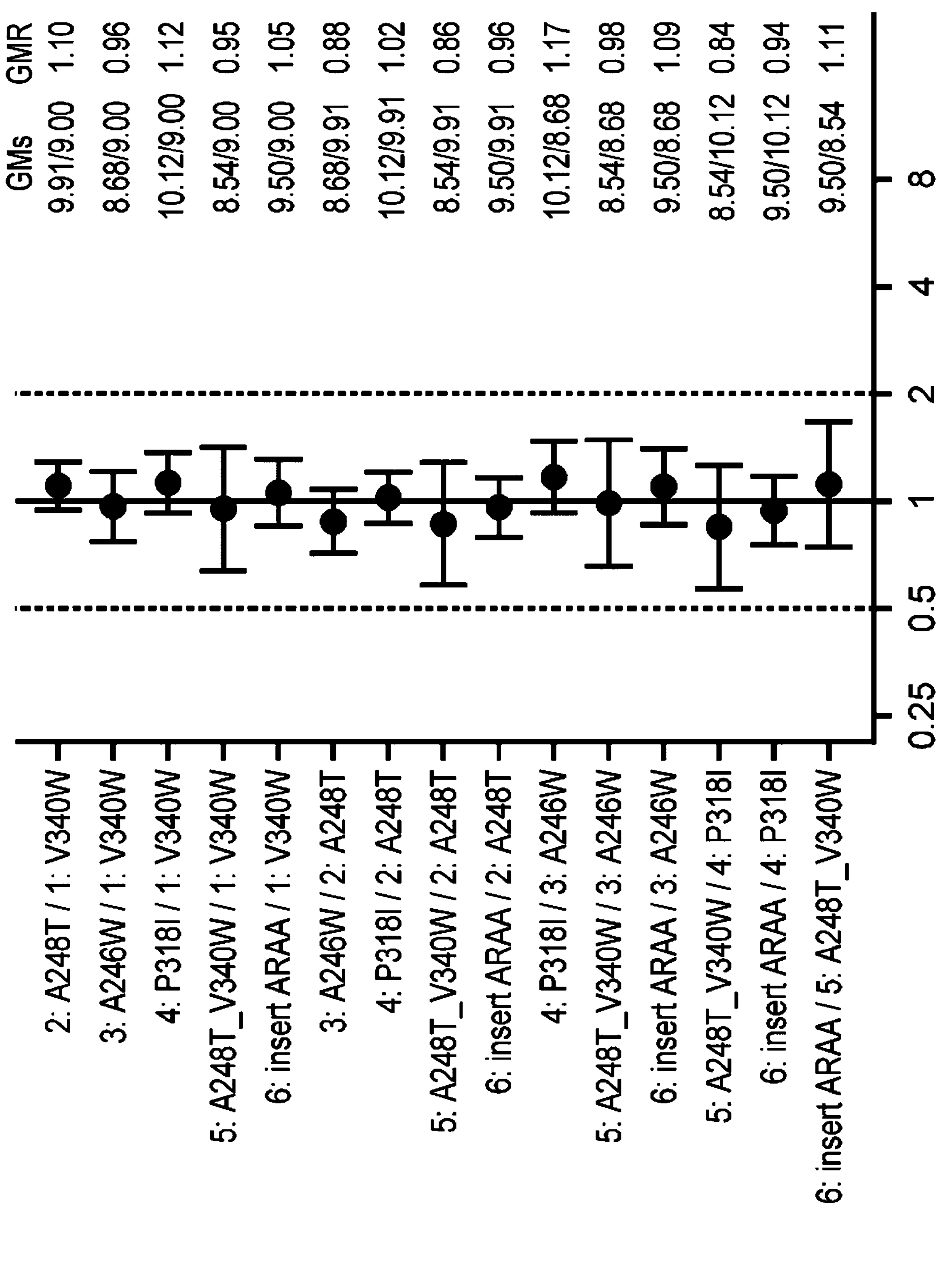

FIG. 32B Geometric mean ratios with 95% CIs of % of HSV-1 gE cross-reactive CD8+ T cells
Head to head comparison of LNP/SAM HSV-2 gE/gI(0,8µg) groups

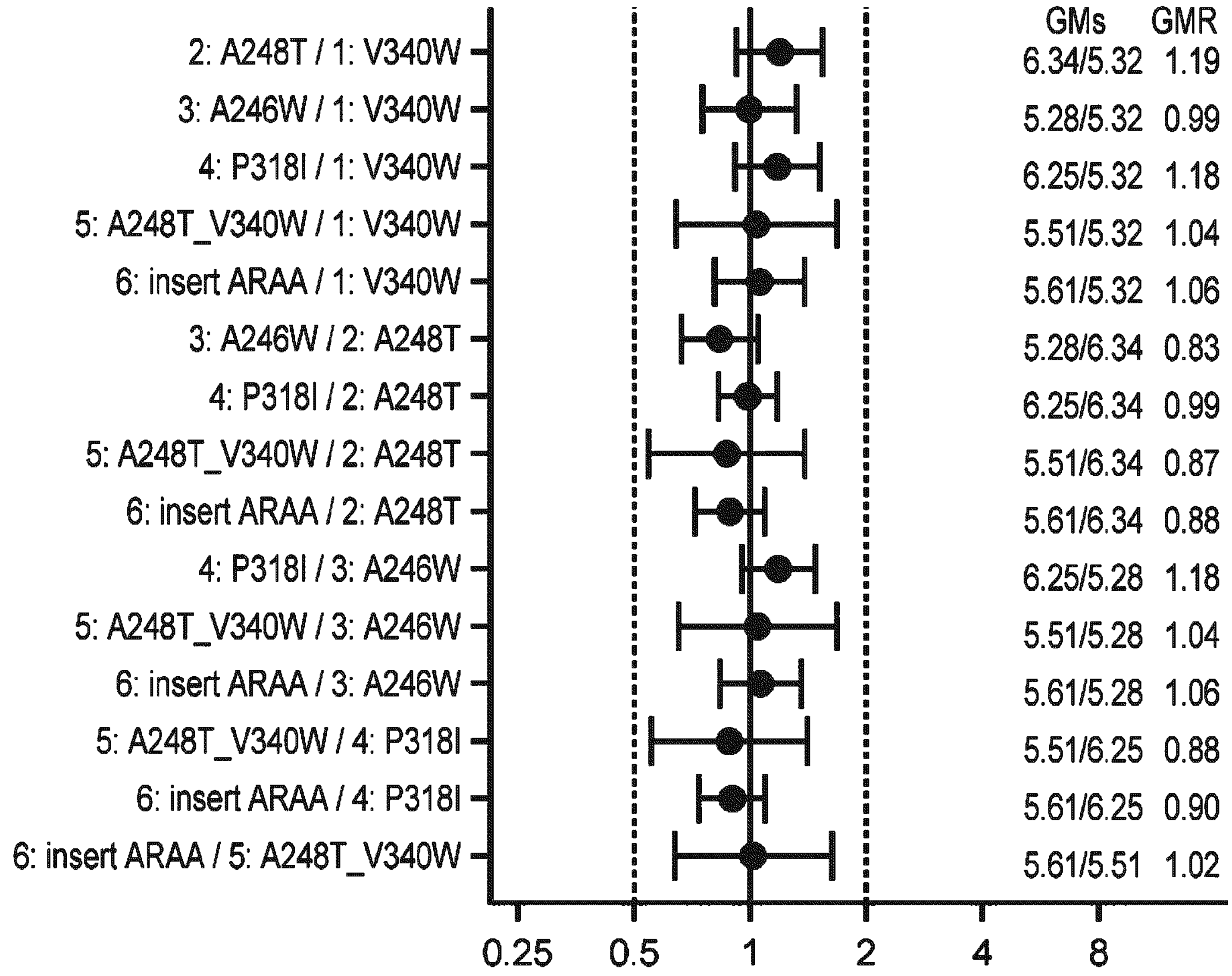

# FIG. 33

Individual results and geometric mean

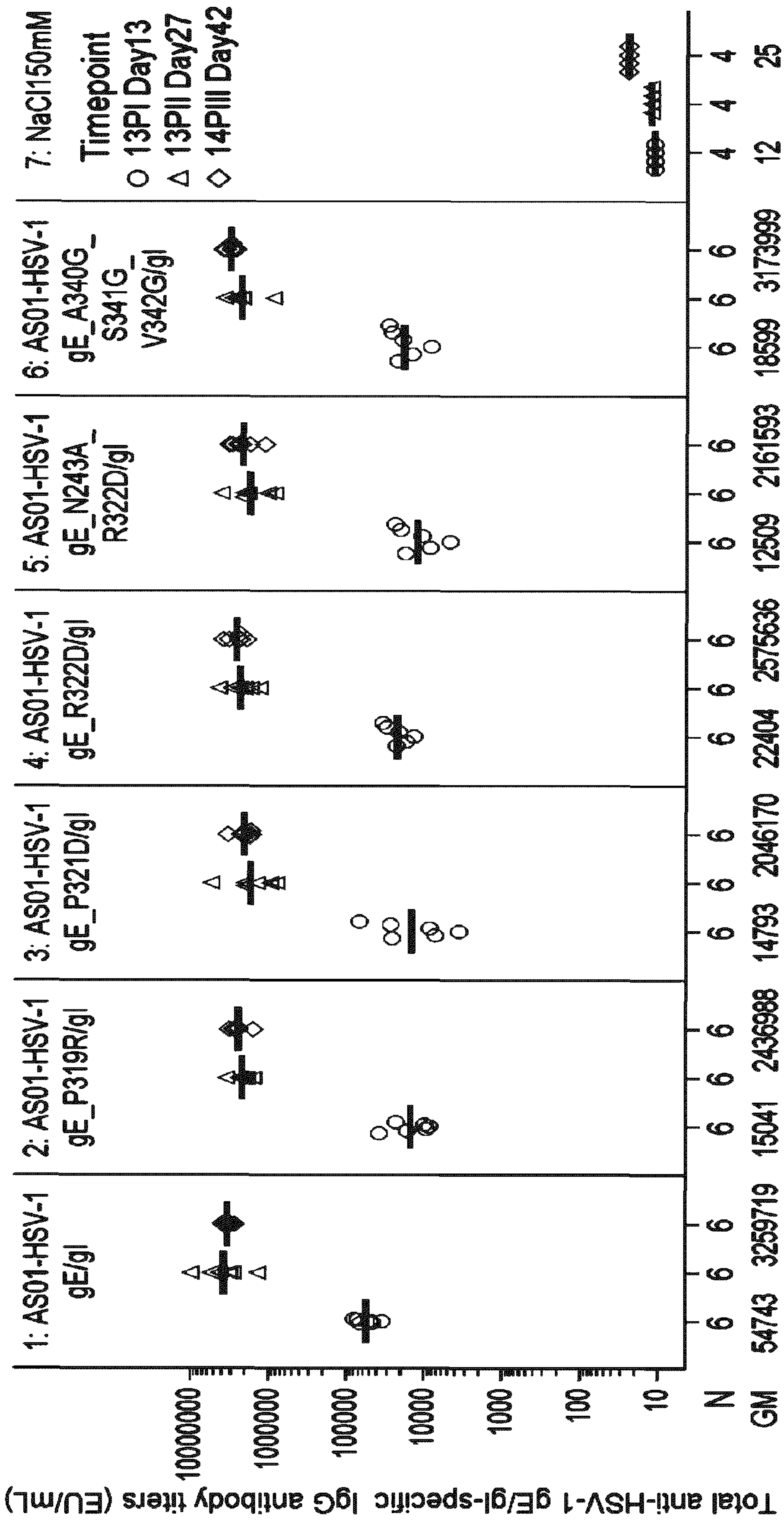

FIG. 34

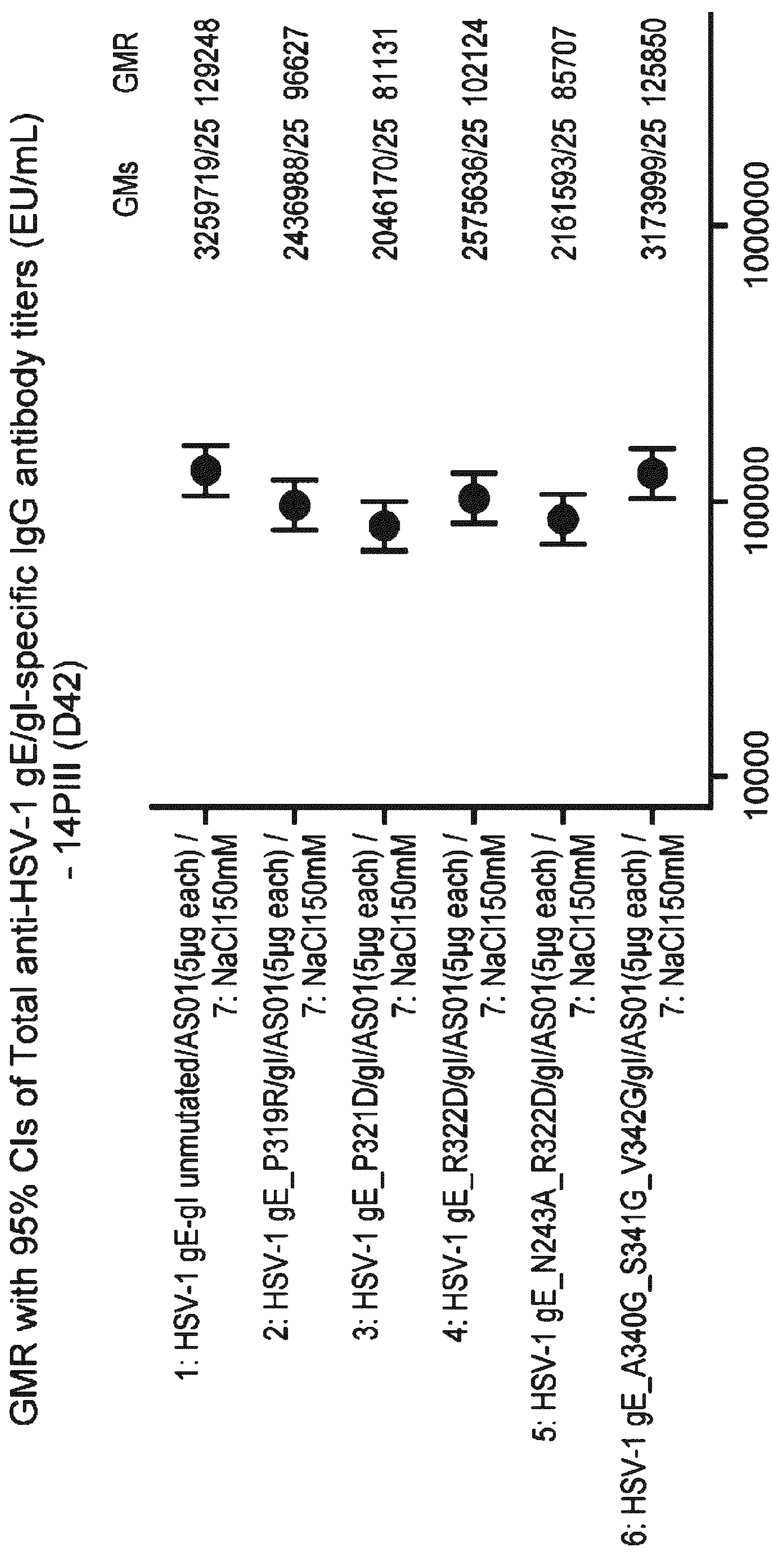
GMR with 95% CIs of Total anti-HSV-1 gE/gI-specific IgG antibody titers (EU/mL)
- 14PIII (D42)
GMs
GMR
1: HSV-1 gE-gI unmutated/AS01(5µg each) / 7: NaCl150mM
3259719/25 129248
2: HSV-1 gE_P319R/gI/AS01(5µg each) / 7: NaCl150mM
2436988/25 96627
3: HSV-1 gE_P321D/gI/AS01(5µg each) / 7: NaCl150mM
2046170/25 81131
4: HSV-1 gE_R322D/gI/AS01(5µg each) / 7: NaCl150mM
2575636/25 102124
5: HSV-1 gE_N243A_R322D/gI/AS01(5µg each) / 7: NaCl150mM
2161593/25 85707
6: HSV-1 gE_A340G_S341G_V342G/gI/AS01(5µg each) / 7: NaCl150mM
3173999/25 125850
10000
100000
1000000

# FIG. 35A

GMR with 95% CIs of Total anti-HSV-1 gE/gI-specific IgG antibody titers (EU/mL)

Group comparisons of PII over PI

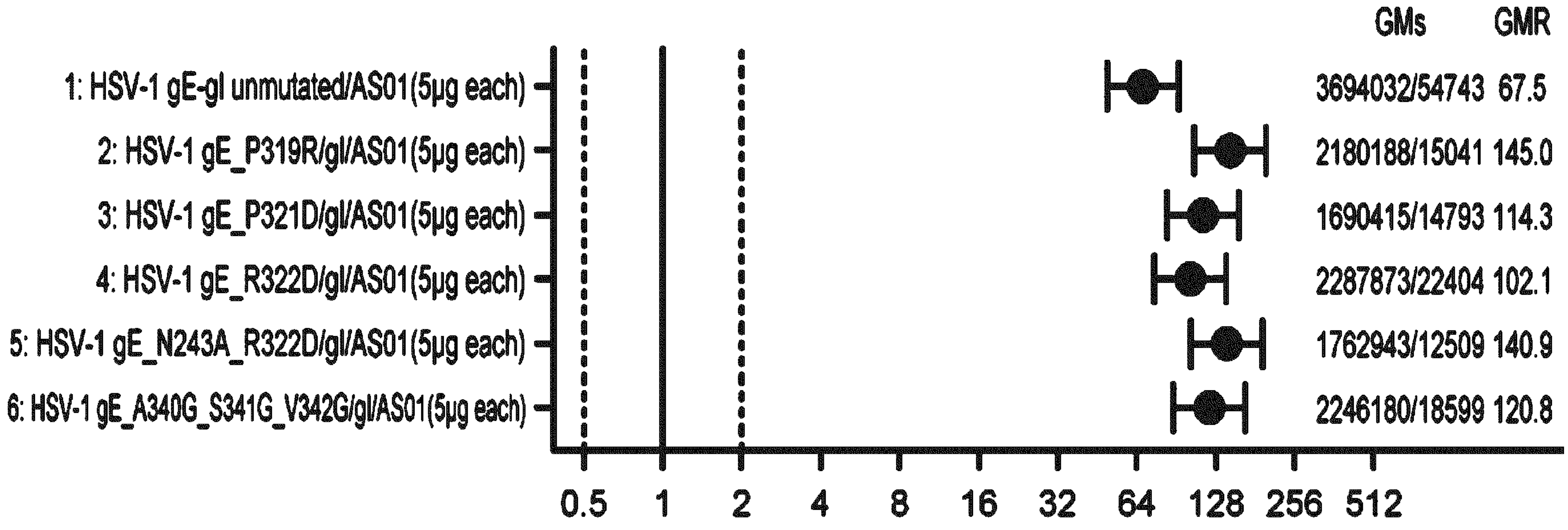

FIG. 35B

GMR with 95% CIs of Total anti-HSV-1 gE/gI-specific IgG antibody titers (EU/mL)

Group comparisons of PIII over PII

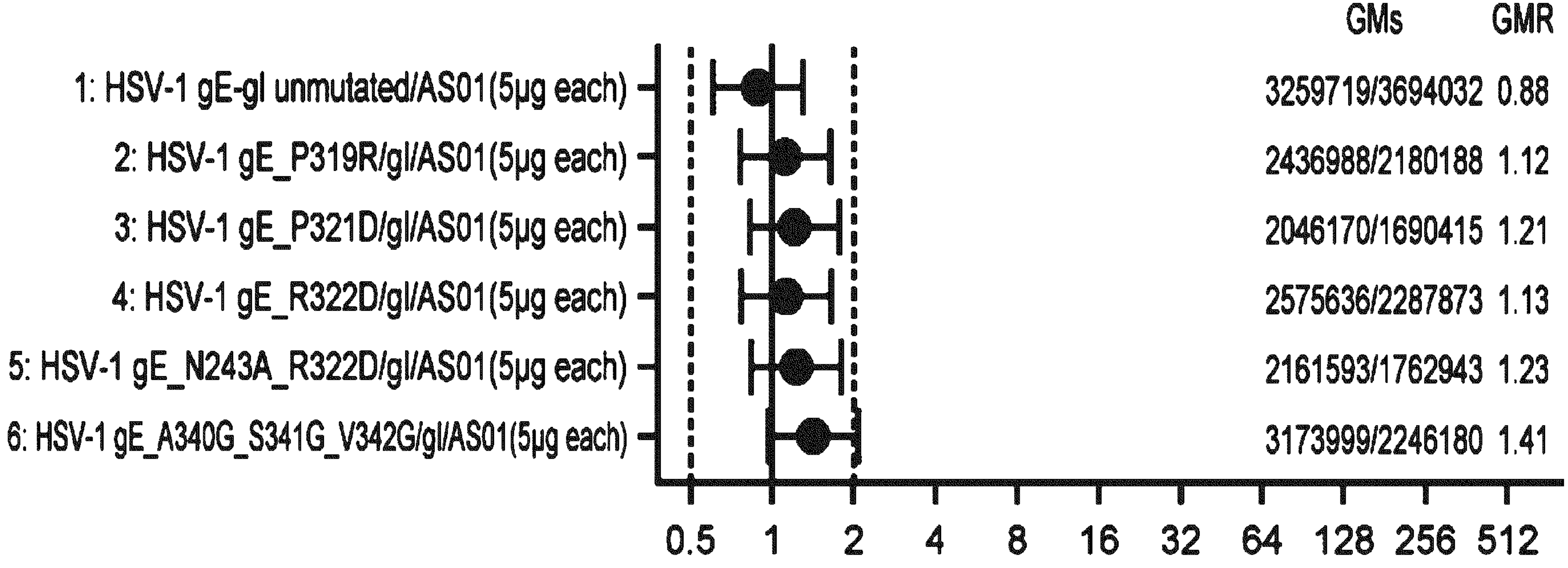

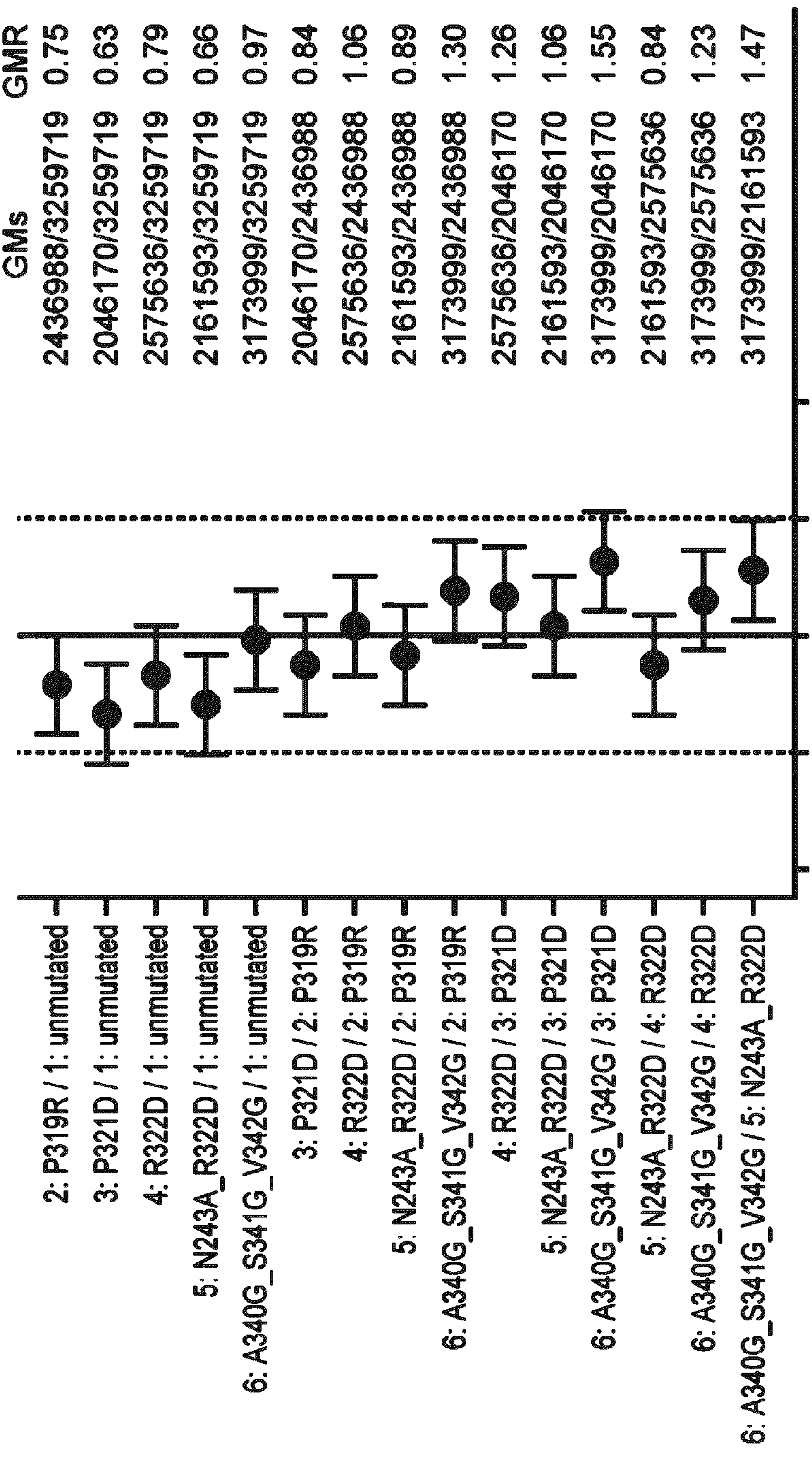


**FIG. 36** GMR with 95% CIs of Total anti-HSV-1 gE/gI-specific IgG antibody titers (EU/mL)
Head to head comparison of HSV-1 gE/gI/AS01(5µg each) - 14PIII (D42)

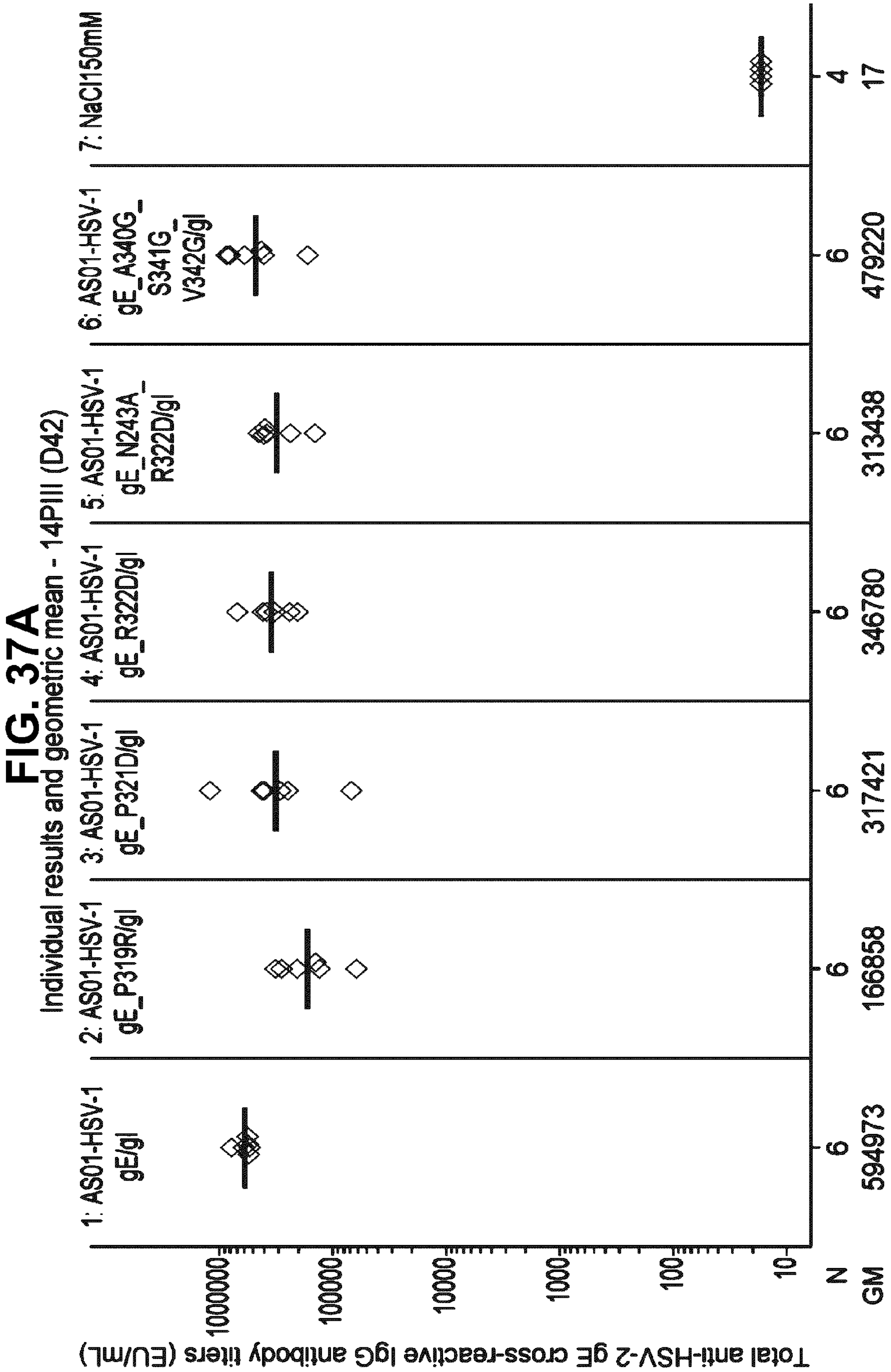
Individual results and geometric mean - 14PIII (D42)
Total anti-HSV-2 gE cross-reactive IgG antibody titers (EU/mL)
1000000
100000
10000
1000
100
10
1: AS01-HSV-1 gE/gI
2: AS01-HSV-1 gE_P319R/gI
3: AS01-HSV-1 gE_P321D/gI
4: AS01-HSV-1 gE_R322D/gI
5: AS01-HSV-1 gE_N243A_R322D/gI
6: AS01-HSV-1 gE_A340G_S341G_V342G/gI
7: NaCl150mM
N
GM
6
594973
6
166858
6
317421
6
346780
6
313438
6
479220
4
17

FIG. 37A

FIG. 37B

Individual results and geometric mean - 14PIII (D42)

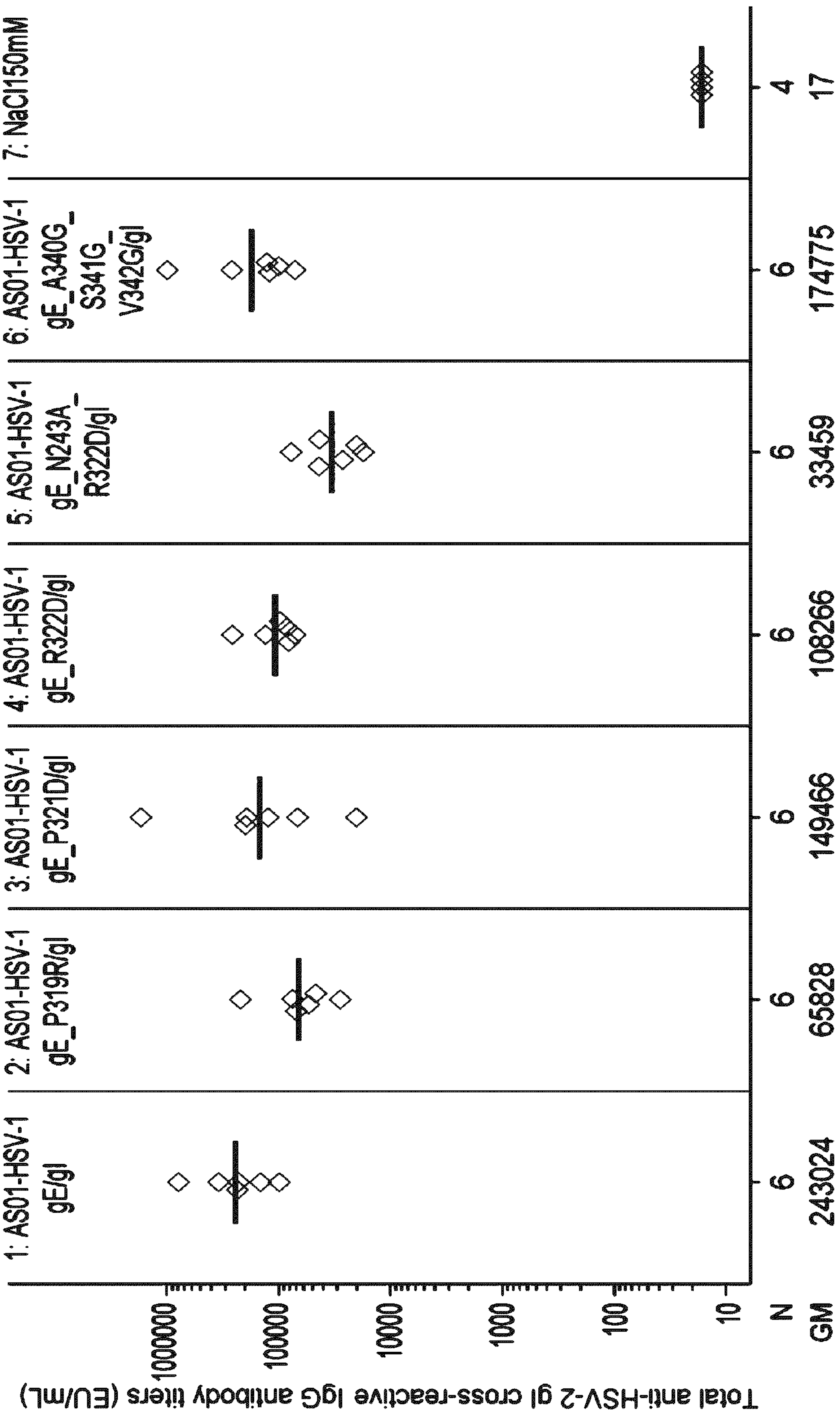

# FIG. 38A

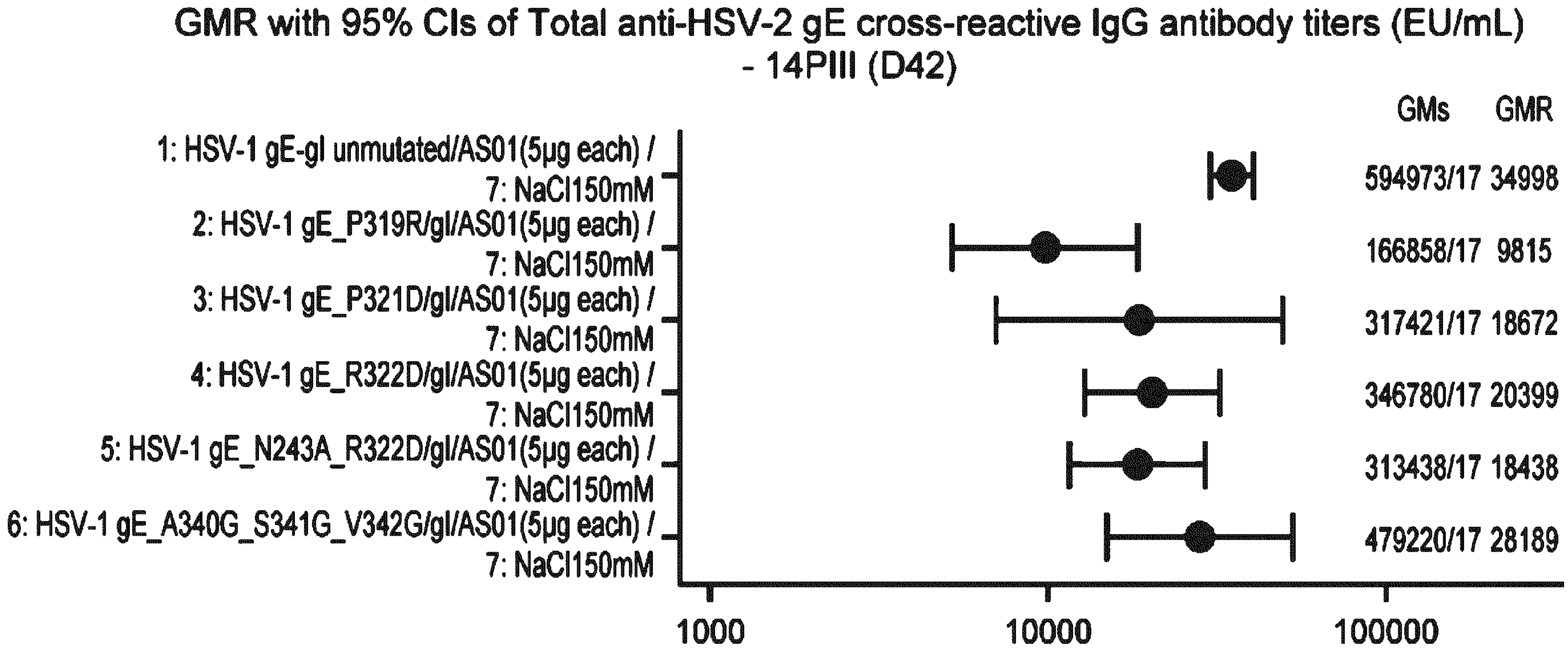
GMR with 95% CIs of Total anti-HSV-2 gE cross-reactive IgG antibody titers (EU/mL)
- 14PIII (D42)
GMs GMR
1: HSV-1 gE-gI unmutated/AS01(5µg each) /
7: NaCl150mM
594973/17 34998
2: HSV-1 gE_P319R/gI/AS01(5µg each) /
7: NaCl150mM
166858/17 9815
3: HSV-1 gE_P321D/gI/AS01(5µg each) /
7: NaCl150mM
317421/17 18672
4: HSV-1 gE_R322D/gI/AS01(5µg each) /
7: NaCl150mM
346780/17 20399
5: HSV-1 gE_N243A_R322D/gI/AS01(5µg each) /
7: NaCl150mM
313438/17 18438
6: HSV-1 gE_A340G_S341G_V342G/gI/AS01(5µg each) /
7: NaCl150mM
479220/17 28189
1000
10000
100000

FIG. 38B

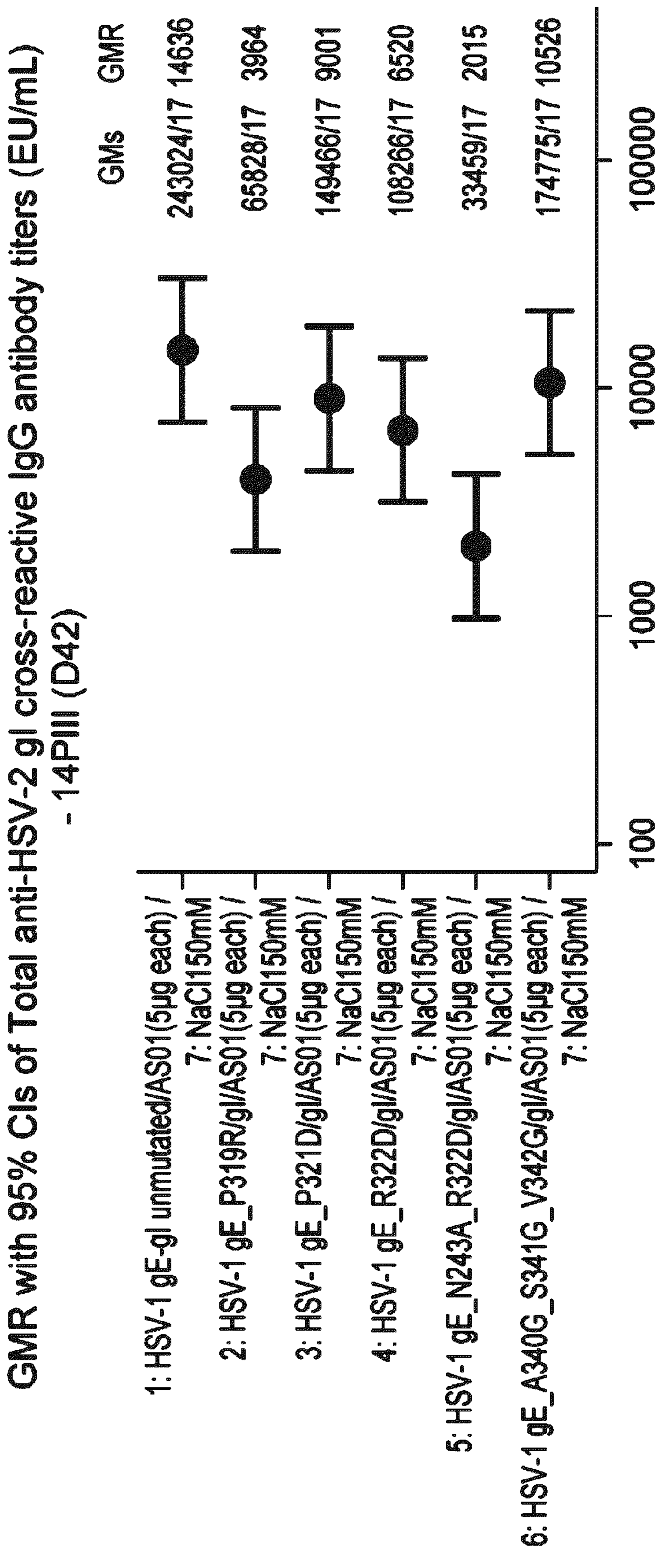

GMR with 95% CIs of Total anti-HSV-2 gI cross-reactive IgG antibody titers (EU/mL)
- 14PIII (D42)
GMs GMR
1: HSV-1 gE-gI unmutated/AS01(5µg each) / 7: NaCl150mM
243024/17 14636
2: HSV-1 gE_P319R/gI/AS01(5µg each) / 7: NaCl150mM
65828/17 3964
3: HSV-1 gE_P321D/gI/AS01(5µg each) / 7: NaCl150mM
149466/17 9001
4: HSV-1 gE_R322D/gI/AS01(5µg each) / 7: NaCl150mM
108266/17 6520
5: HSV-1 gE_N243A_R322D/gI/AS01(5µg each) / 7: NaCl150mM
33459/17 2015
6: HSV-1 gE_A340G_S341G_V342G/gI/AS01(5µg each) / 7: NaCl150mM
174775/17 10526
100
1000
10000
100000

**FIG. 39A** GMR with 95% CIs of Total anti-HSV-2 gE cross-reactive IgG antibody titers (EU/mL)
Head to head comparison of HSV-1 gE/gI/AS01(5µg each) - 14PIII (D42)

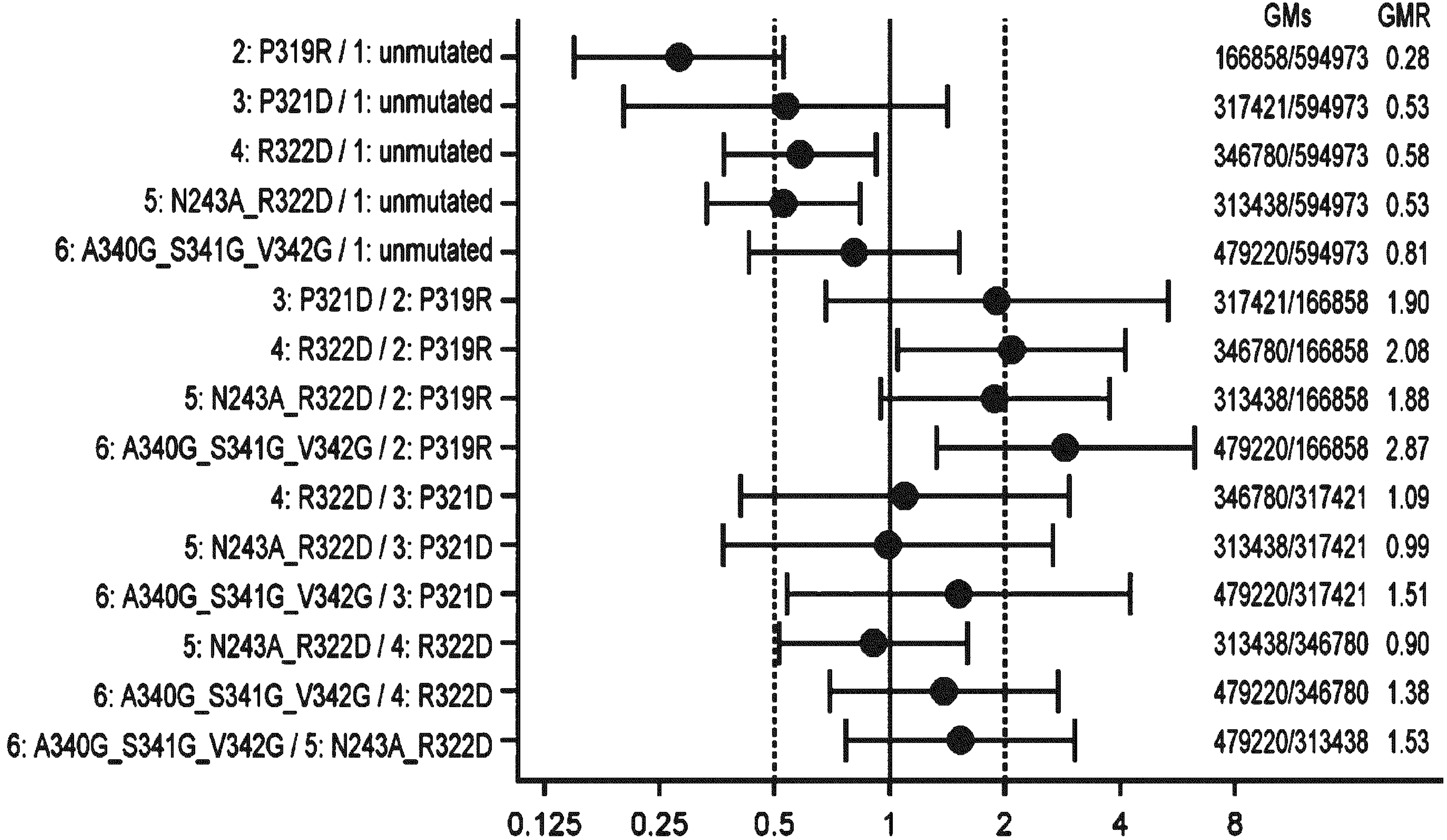

**FIG. 39B** GMR with 95% CIs of Total anti-HSV-2 gI cross-reactive IgG antibody titers (EU/mL)
Head to head comparison of HSV-1 gE/gI/AS01(5µg each) - 14PIII (D42)

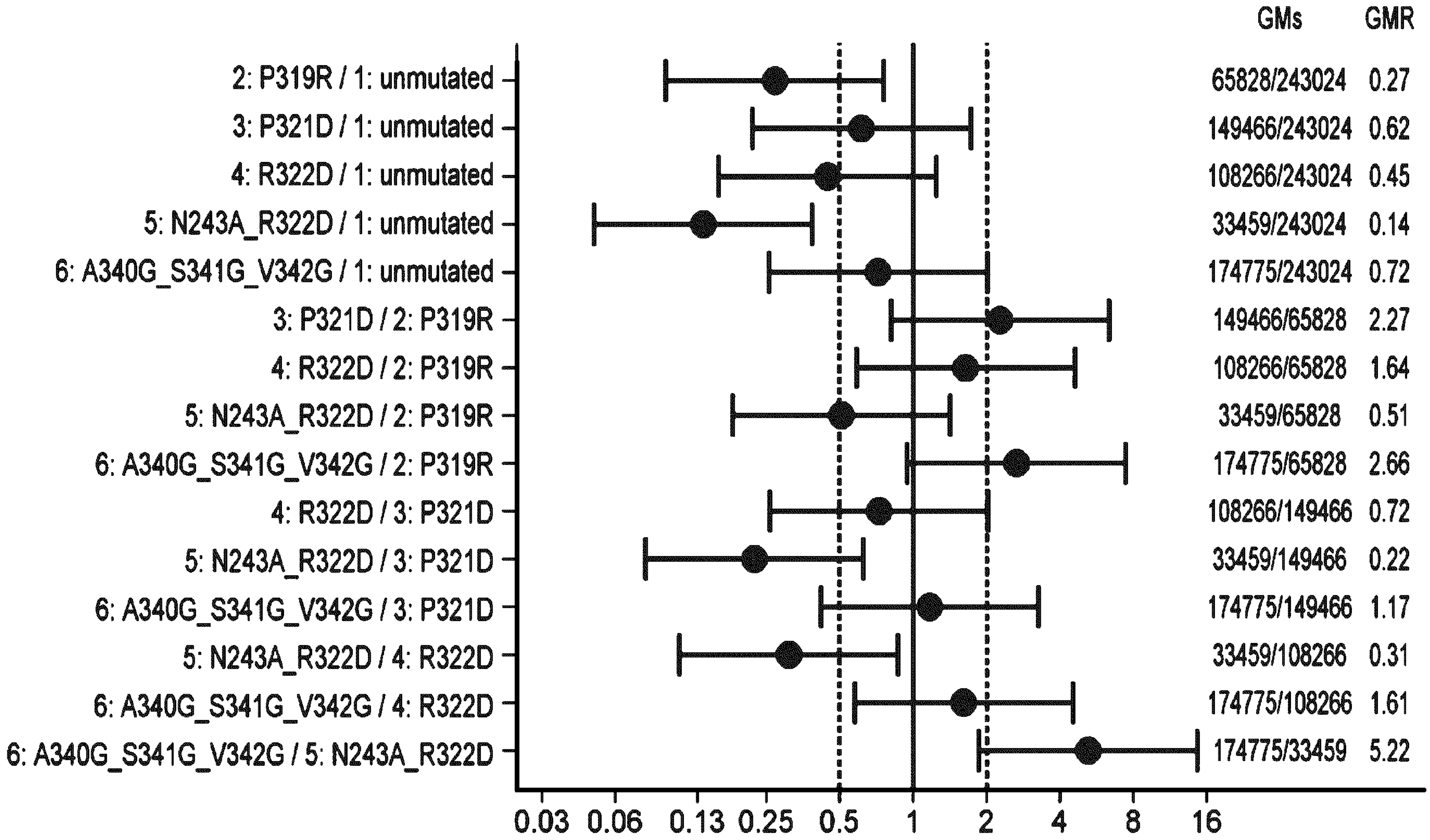

# FIG. 40A

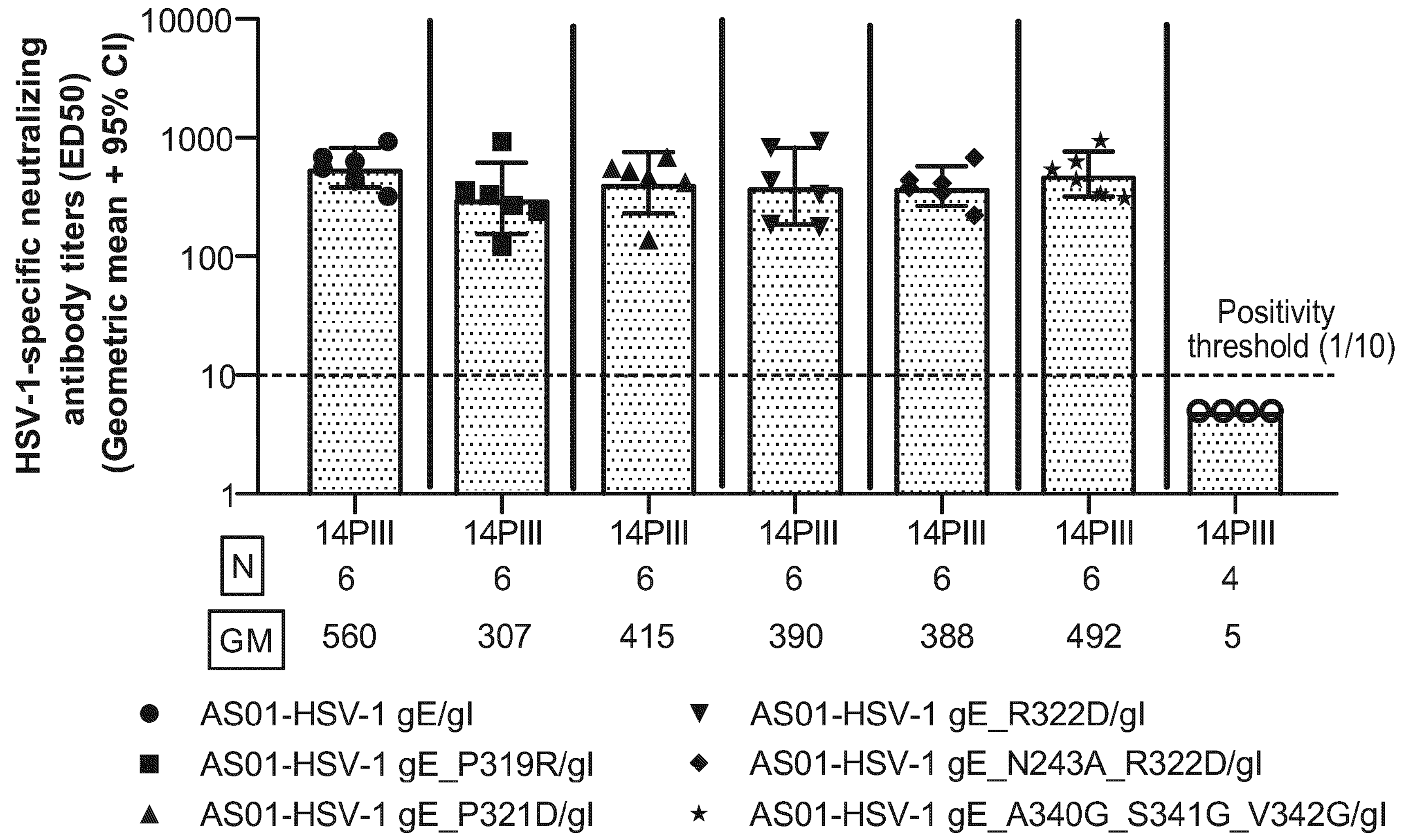
HSV-1-specific neutralizing antibody response induced 14 days after third immunization with different AS01-adjuvanted HSV-1 gE/gI candidates
HSV-1-specific neutralizing antibody titers (ED50) (Geometric mean + 95% CI)
10000
1000
100
10
1
Positivity threshold (1/10)
14PIII
N
6
6
6
6
6
6
4
GM
560
307
415
390
388
492
5
AS01-HSV-1 gE/gI
AS01-HSV-1 gE_P319R/gI
AS01-HSV-1 gE_P321D/gI
AS01-HSV-1 gE_R322D/gI
AS01-HSV-1 gE_N243A_R322D/gI
AS01-HSV-1 gE_A340G_S341G_V342G/gI

**FIG. 40B** GMR with 95% CIs of HSV-1-specific neutralizing antibody titers
Head to head comparison of HSV-1 gE/gI/AS01(5µg each) - 14PIII(D42)

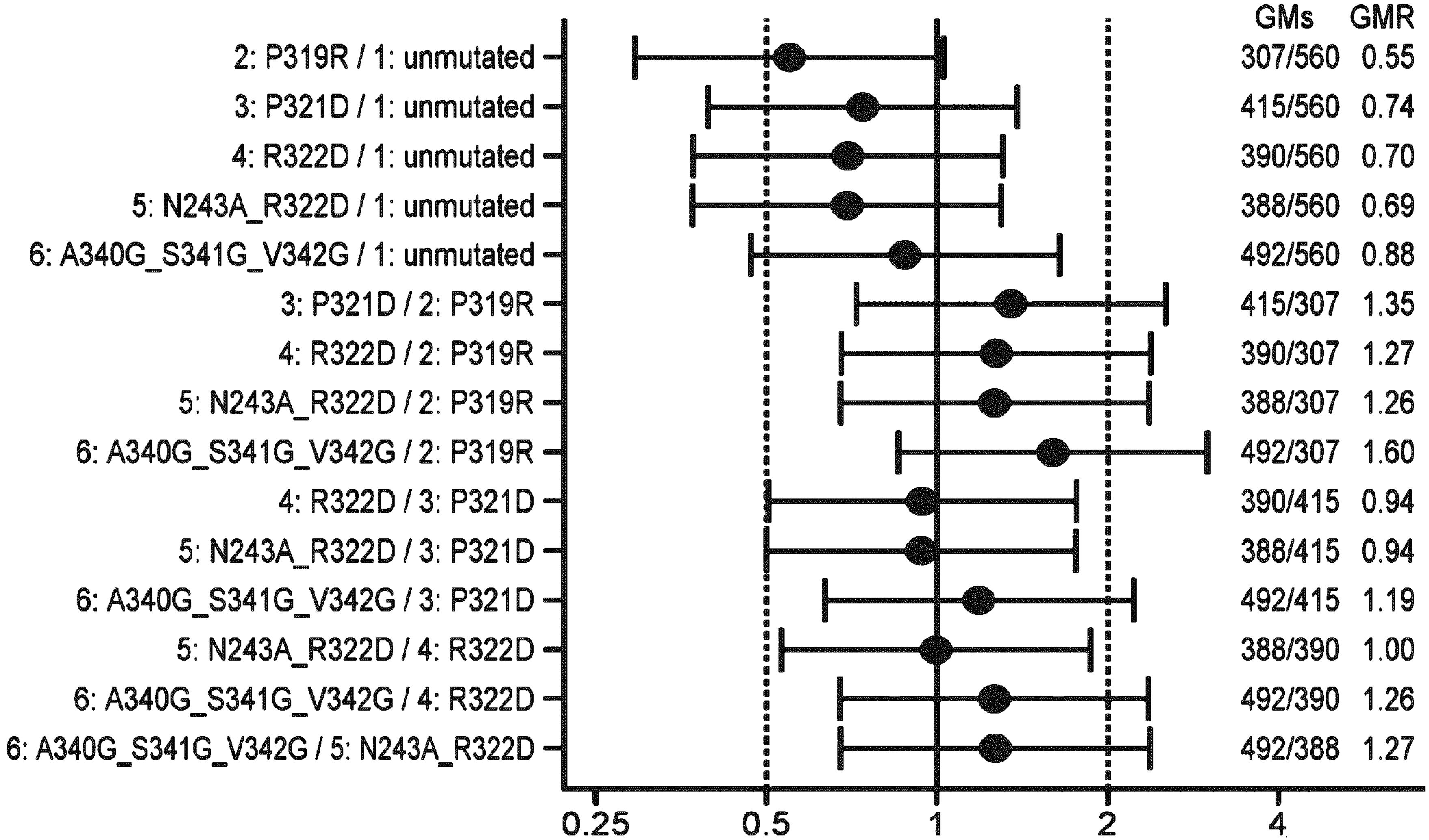

# FIG. 41

**HSV-2-cross-reactive neutralizing antibody response induced 14 days after third immunization with different AS01-adjuvanted HSV-1 gE/gI candidates**

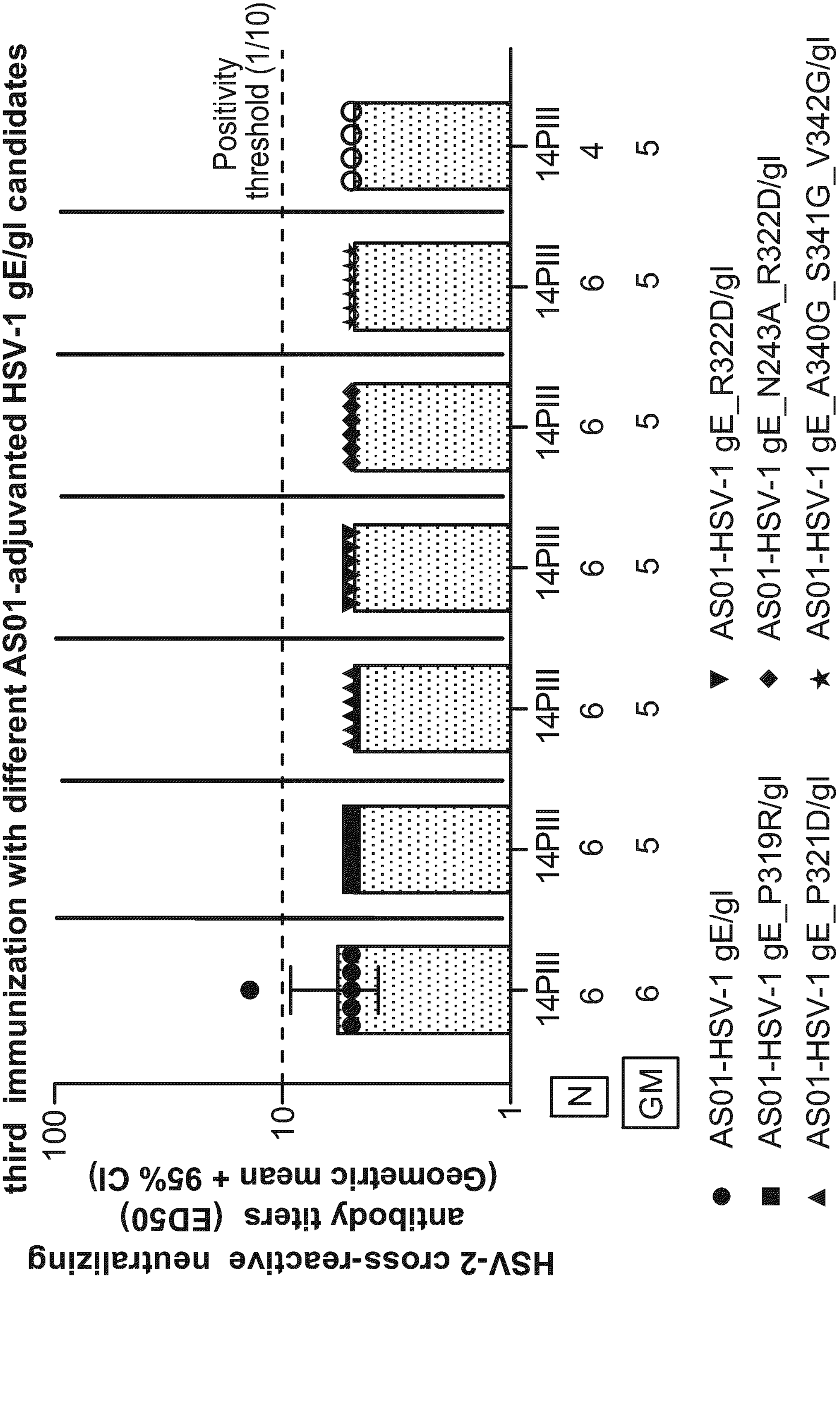

# FIG. 42A

**Co-incubation of hIgG antibodies with serum samples collected 14 days post third immunization**

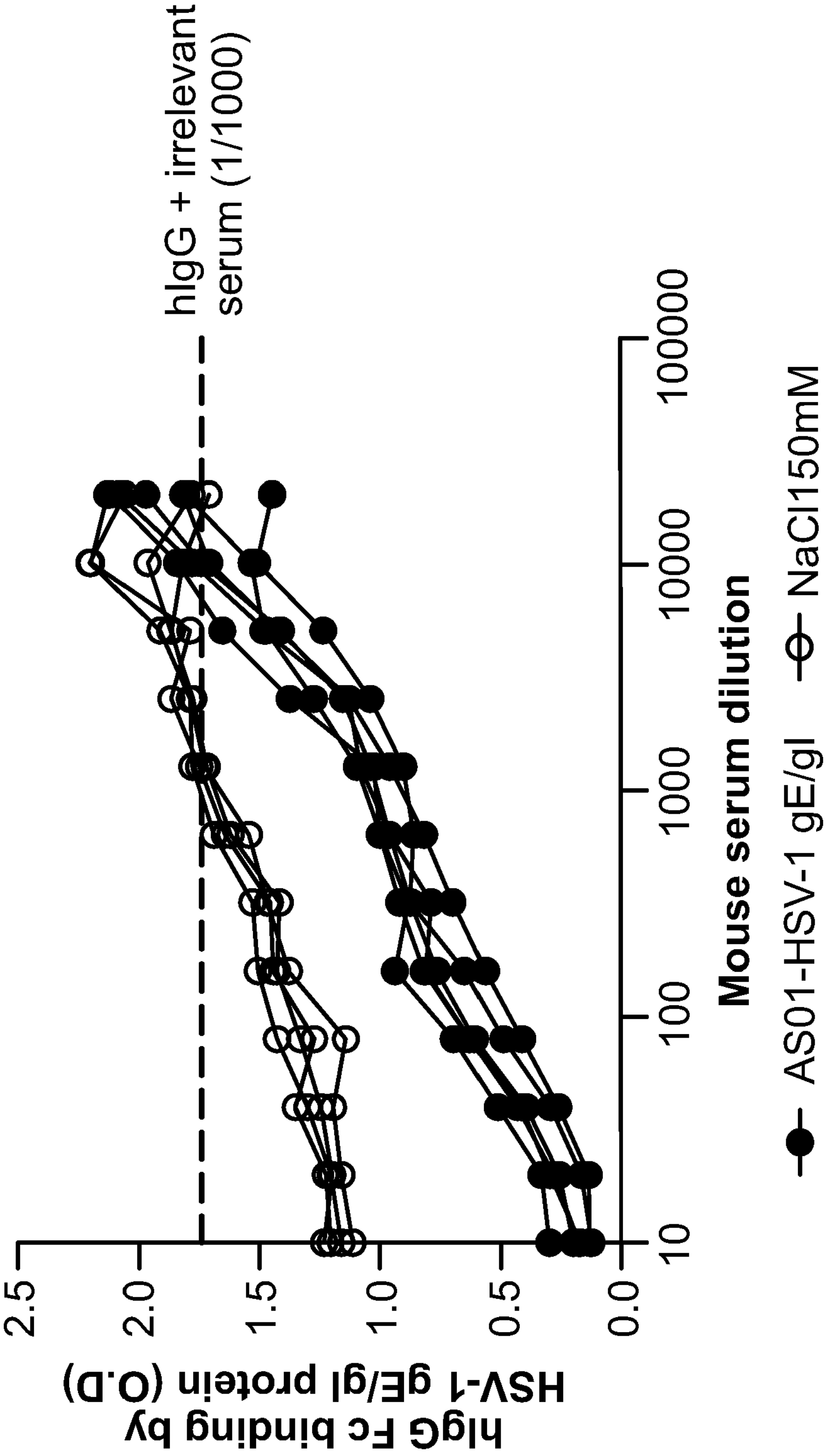

FIG. 42B

**Co-incubation of hIgG antibodies with serum samples collected 14 days post third immunization**

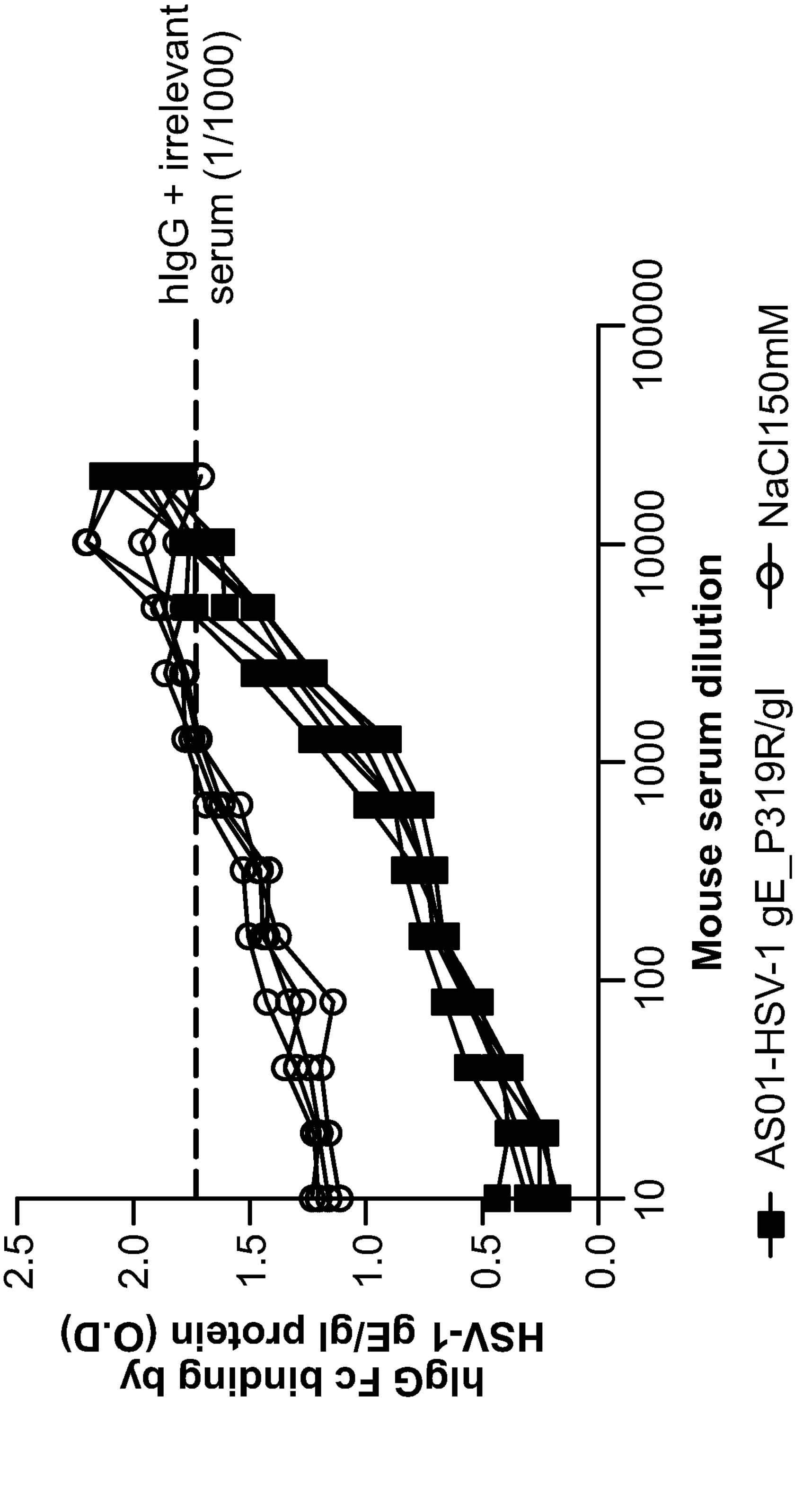

# FIG. 42C

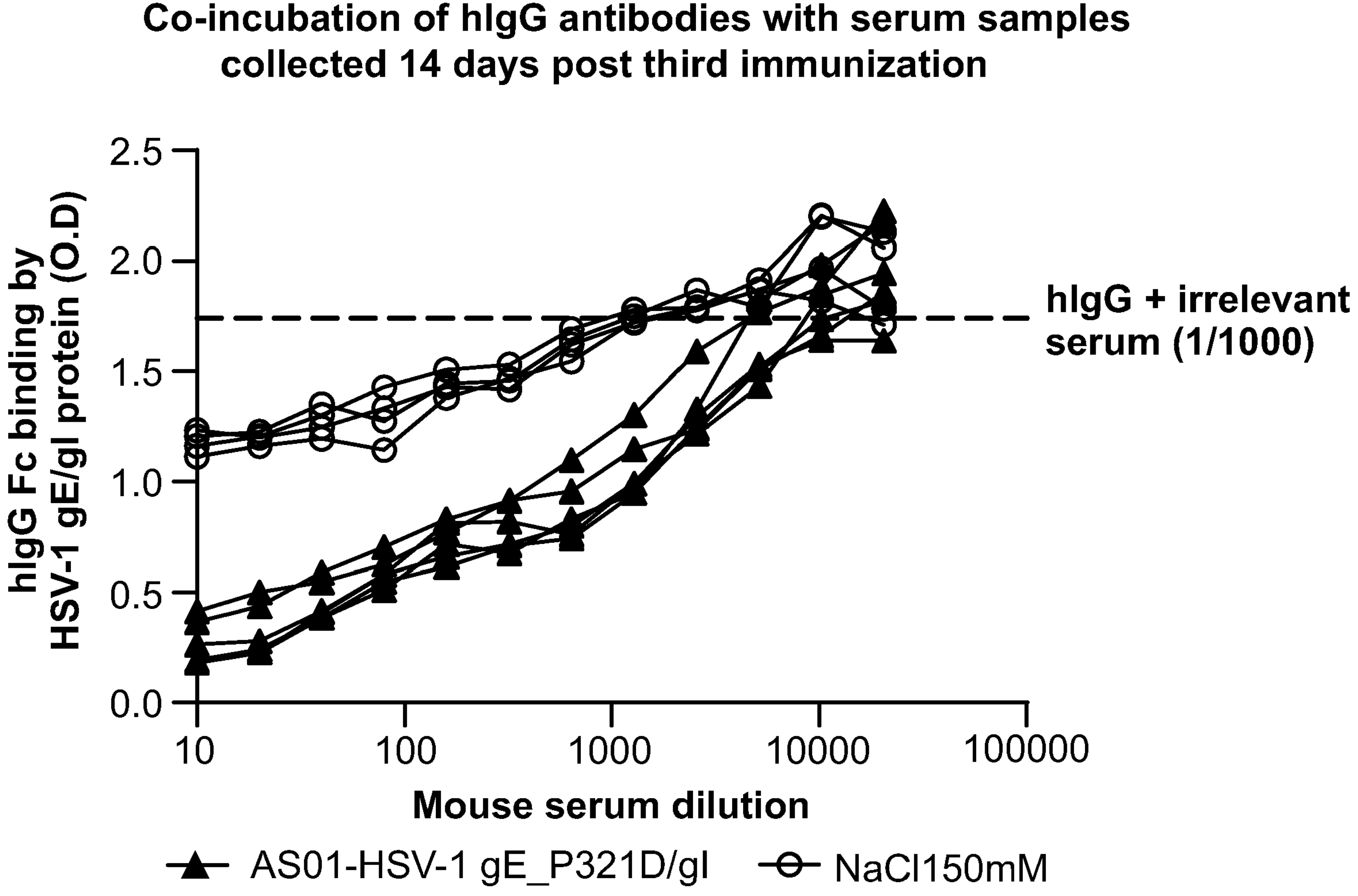
Co-incubation of hIgG antibodies with serum samples
collected 14 days post third immunization
hIgG Fc binding by HSV-1 gE/gI protein (O.D)
2.5
2.0
1.5
1.0
0.5
0.0
10
100
1000
10000
100000
Mouse serum dilution
hIgG + irrelevant serum (1/1000)
AS01-HSV-1 gE_P321D/gI
NaCl150mM

# FIG. 42D

**Co-incubation of hIgG antibodies with serum samples collected 14 days post third immunization**

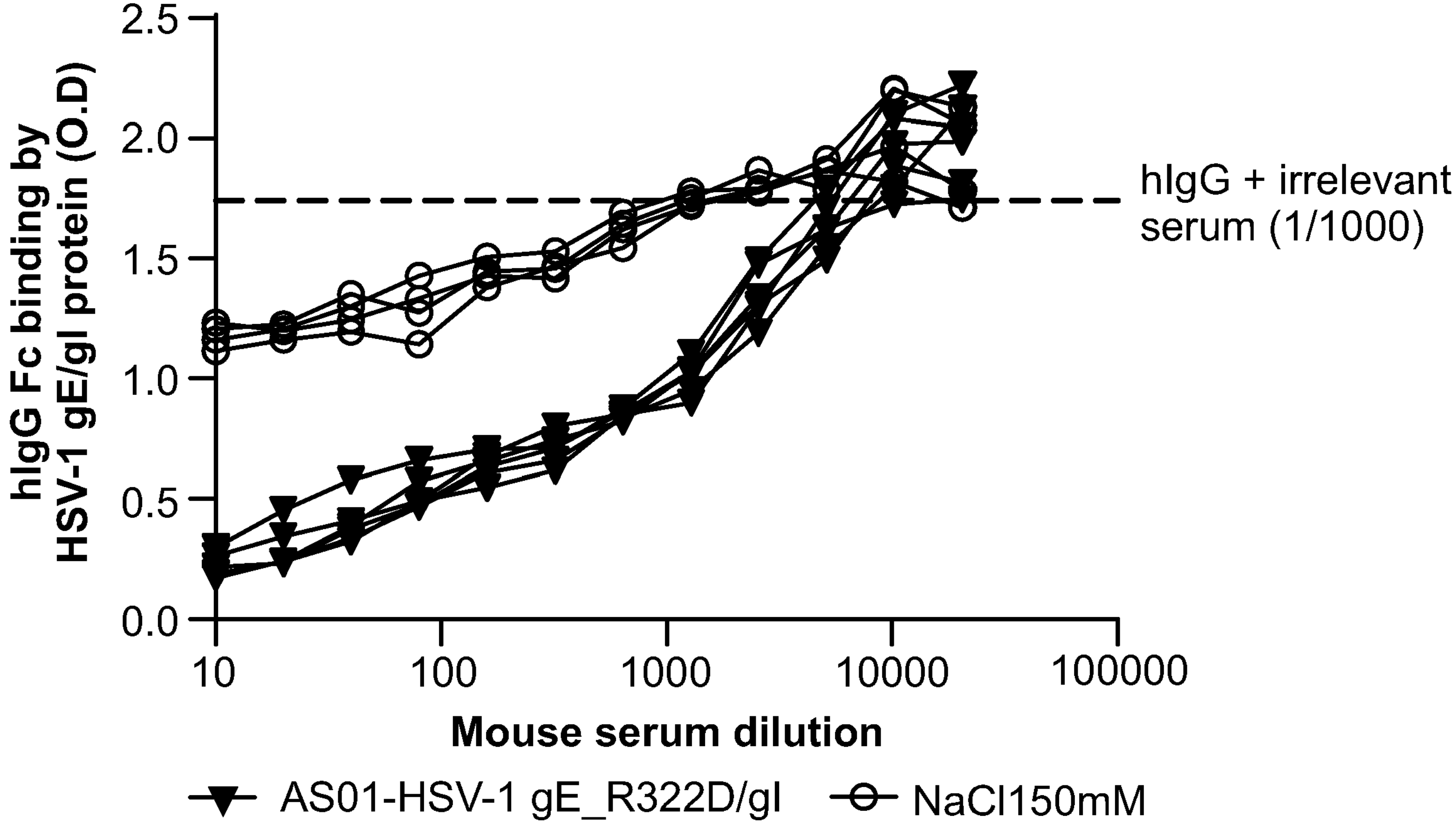

FIG. 42E

Co-incubation of hIgG antibodies with serum samples collected 14 days post third immunization

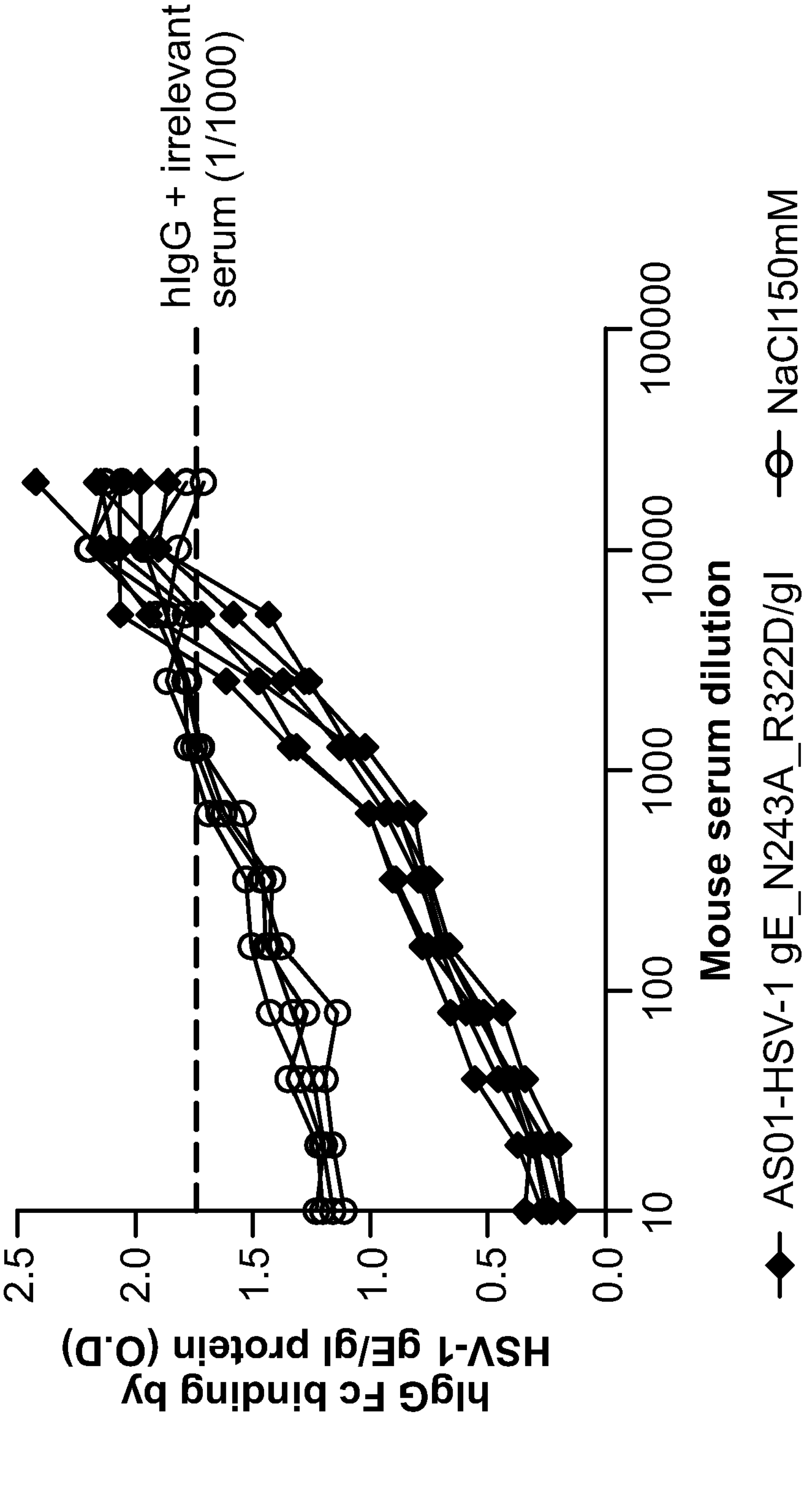

# FIG. 42F

**Co-incubation of hIgG antibodies with serum samples collected 14 days post third immunization**

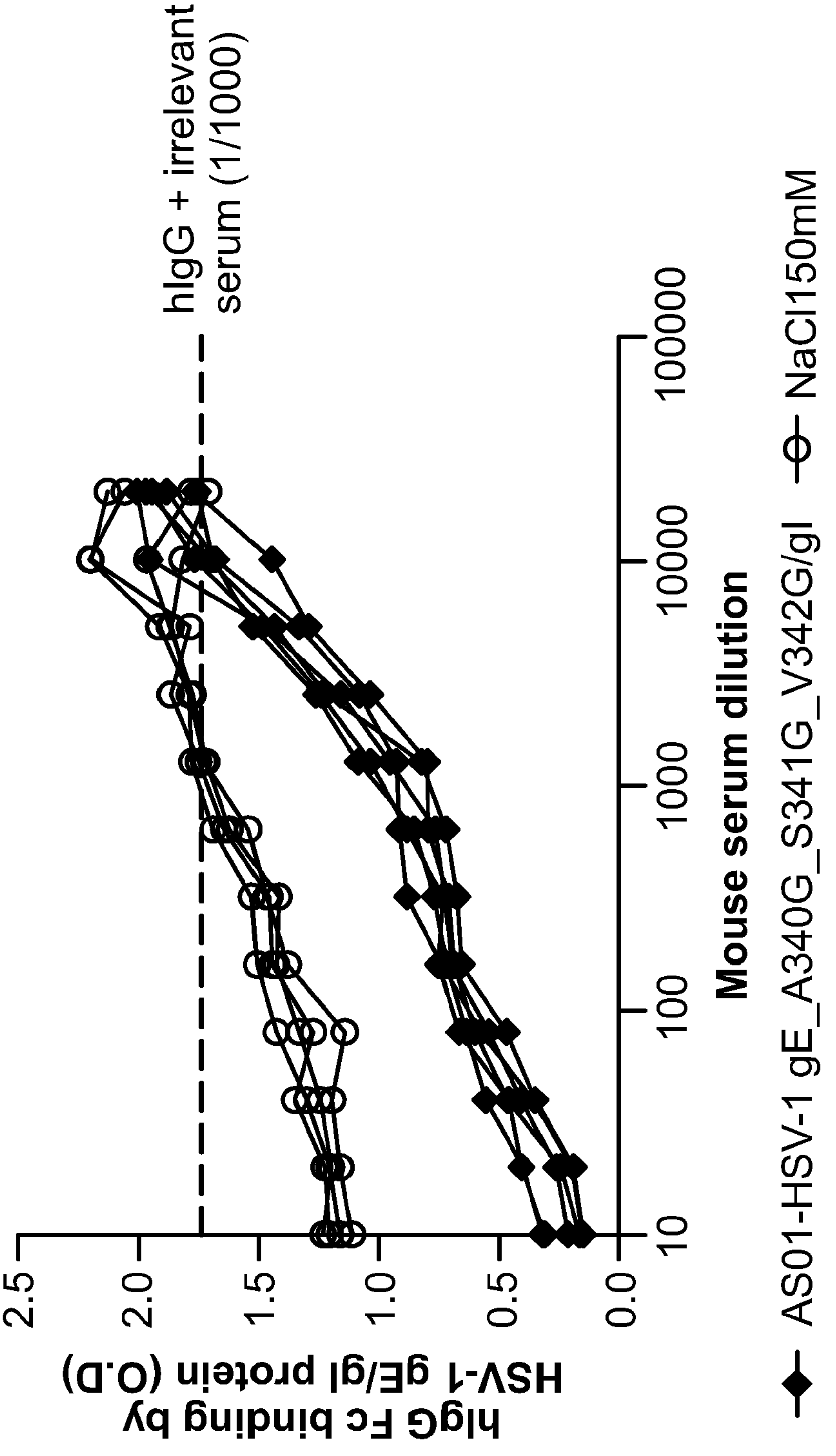

FIG. 43A

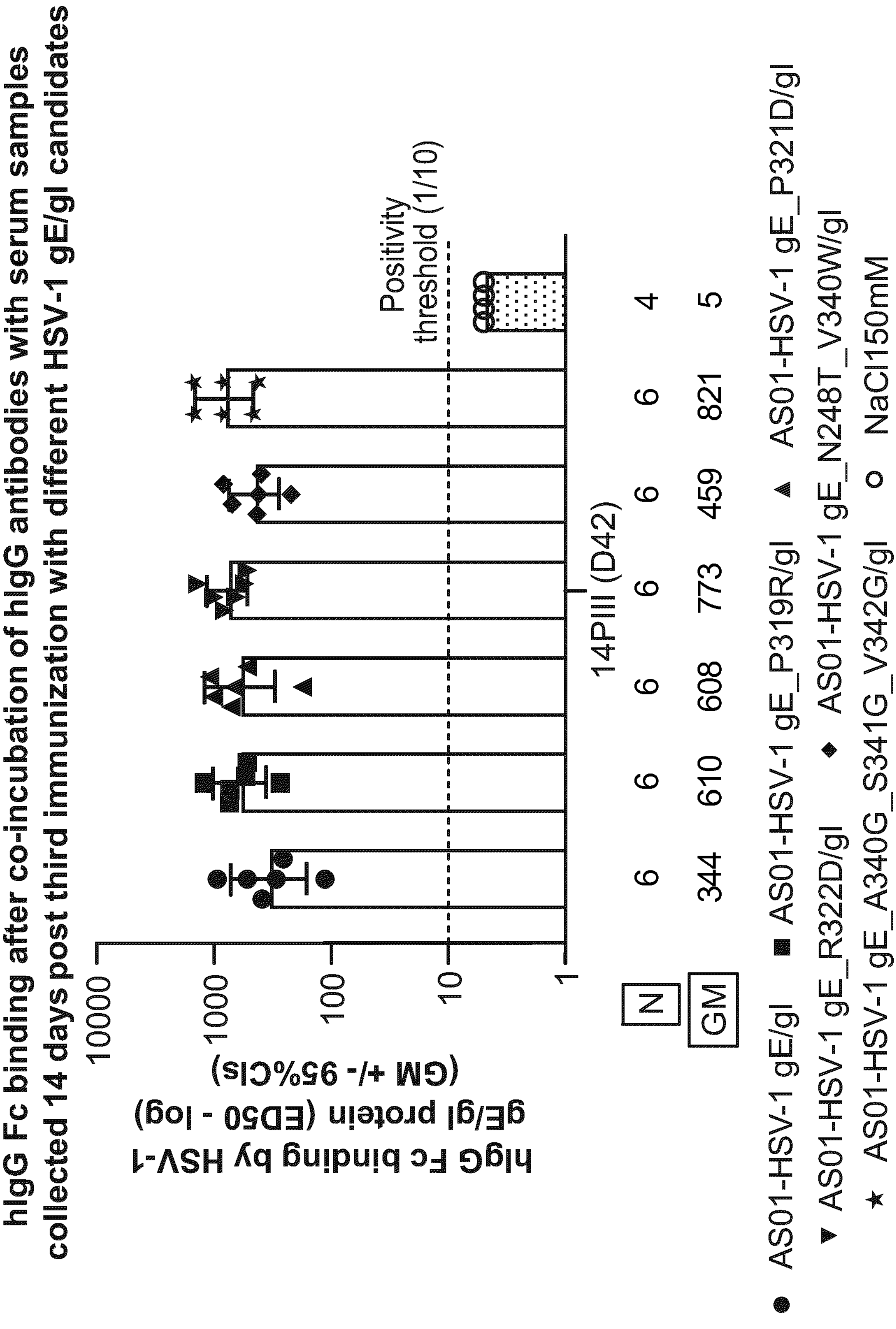

hIgG Fc binding after co-incubation of hIgG antibodies with serum samples collected 14 days post third immunization with different HSV-1 gE/gI candidates
hIgG Fc binding by HSV-1 gE/gI protein (ED50 - log) (GM +/- 95%CIs)
10000
1000
100
10
1
Positivity threshold (1/10)
14PIII (D42)
N
6 6 6 6 6 6 4
GM
344 610 608 773 459 821 5
AS01-HSV-1 gE/gI
AS01-HSV-1 gE_P319R/gI
AS01-HSV-1 gE_P321D/gI
AS01-HSV-1 gE_R322D/gI
AS01-HSV-1 gE_N248T_V340W/gI
AS01-HSV-1 gE_A340G_S341G_V342G/gI
NaCl150mM

# FIG. 43B

GMR with 95% CIs of Inhibition of hIgG Fc binding by HSV-1 gE/gI antigen (ED50)
Head to head comparison of HSV-1 gE/gI/AS01(5µg each) - 14PIII (D42)

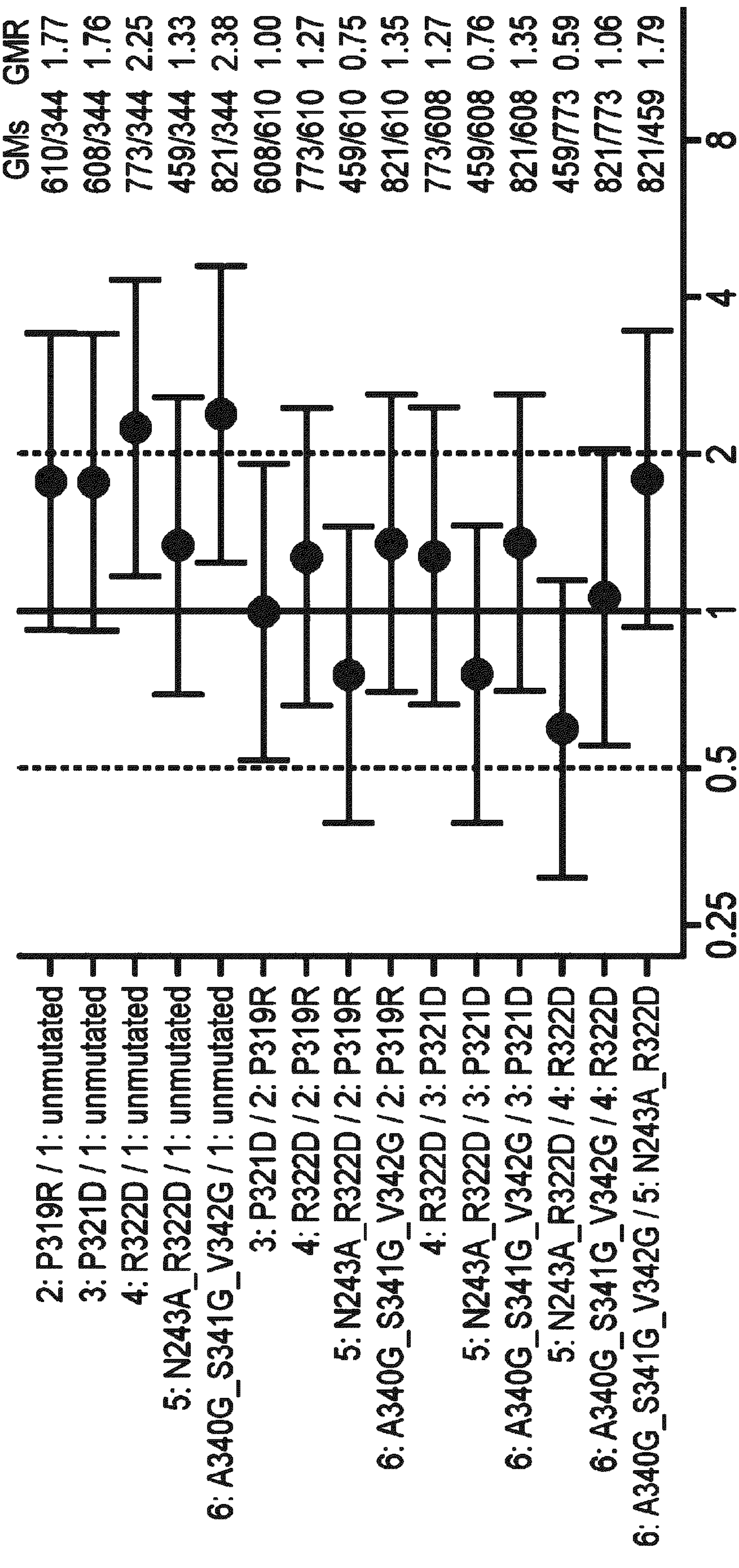

# FIG. 44A

**Co-incubation of hIgG antibodies with serum samples collected 14 days post third immunization**

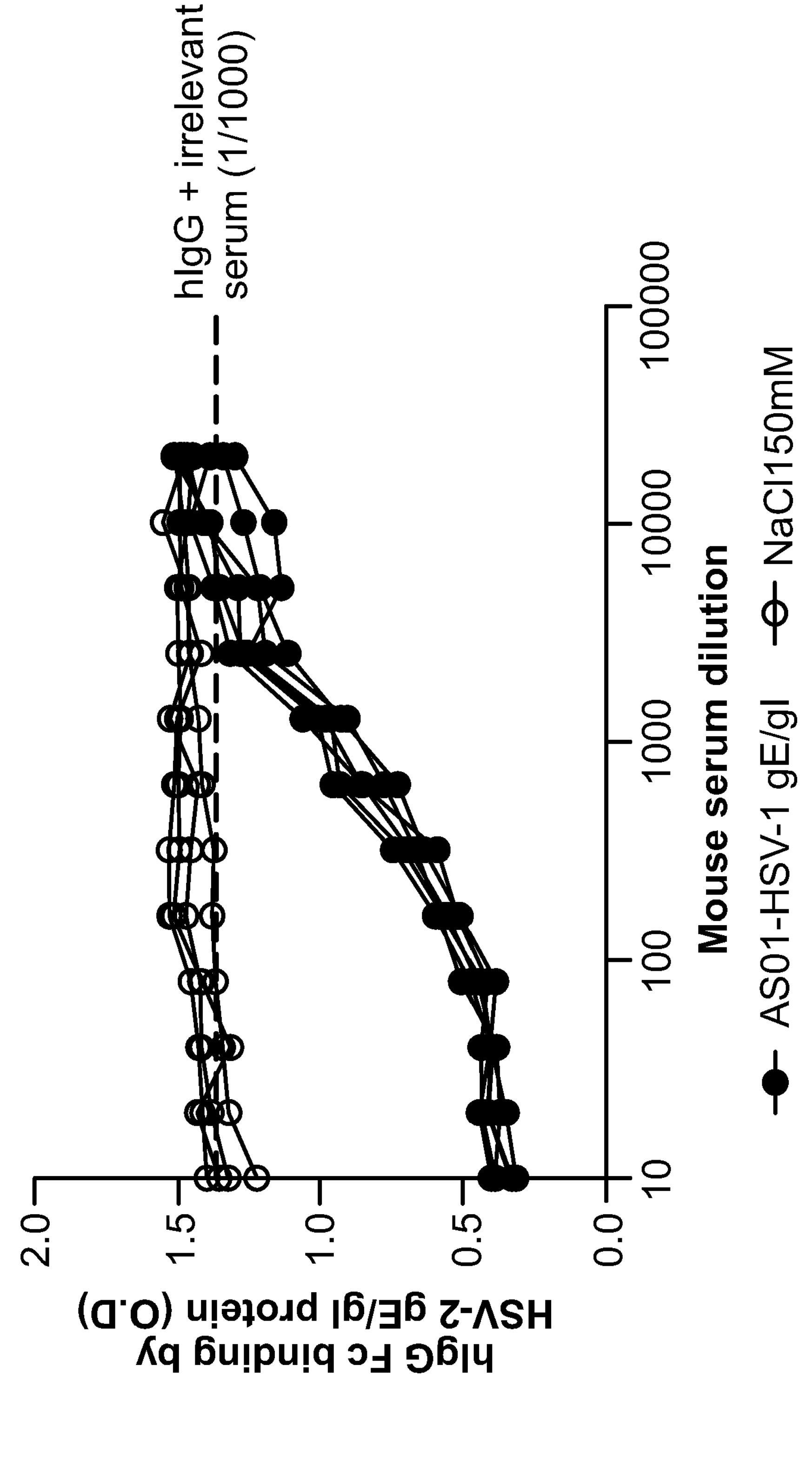

# FIG. 44B

**Co-incubation of hIgG antibodies with serum samples collected 14 days post third immunization**

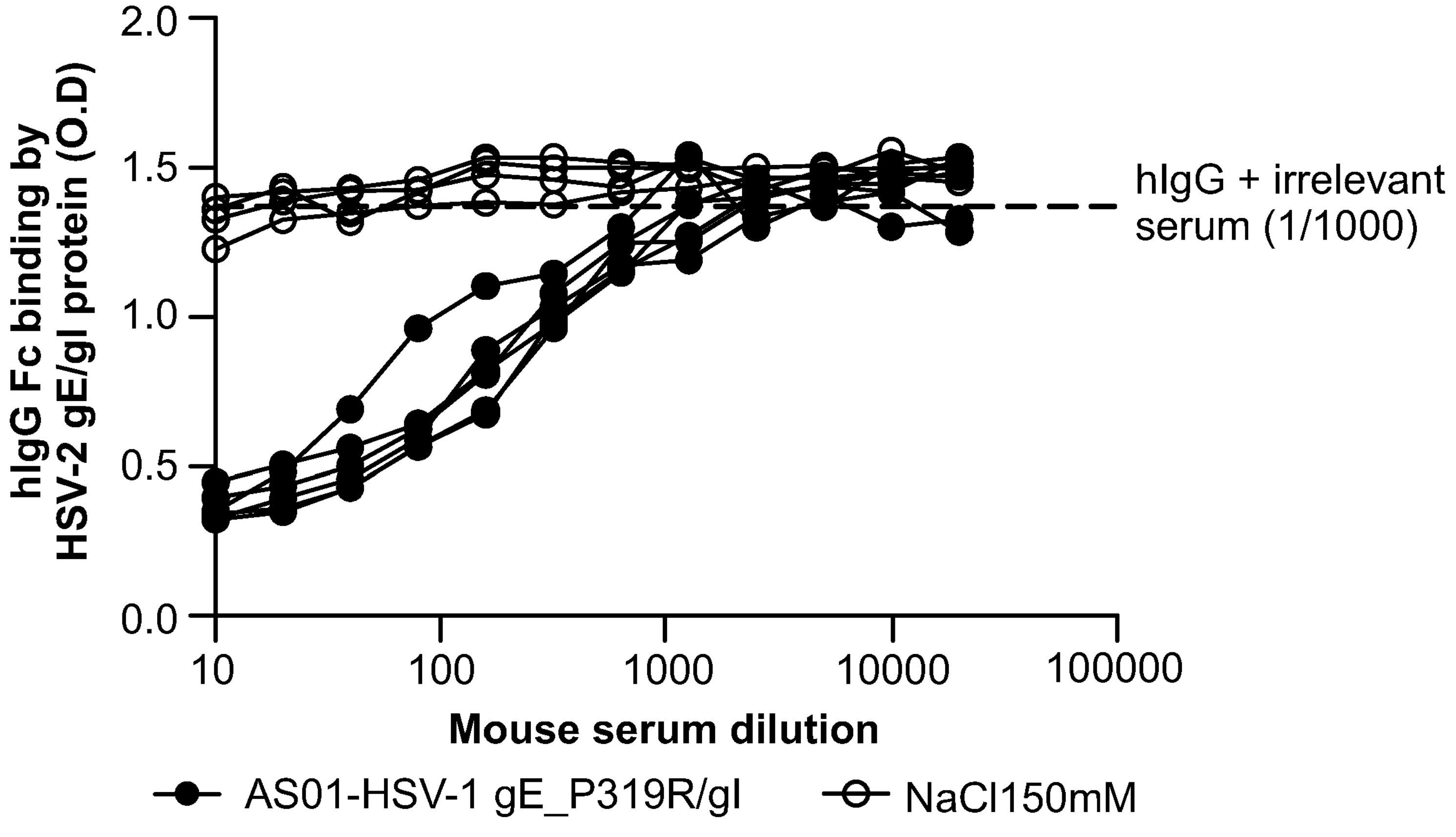

FIG. 44C

**Co-incubation of hIgG antibodies with serum samples collected 14 days post third immunization**

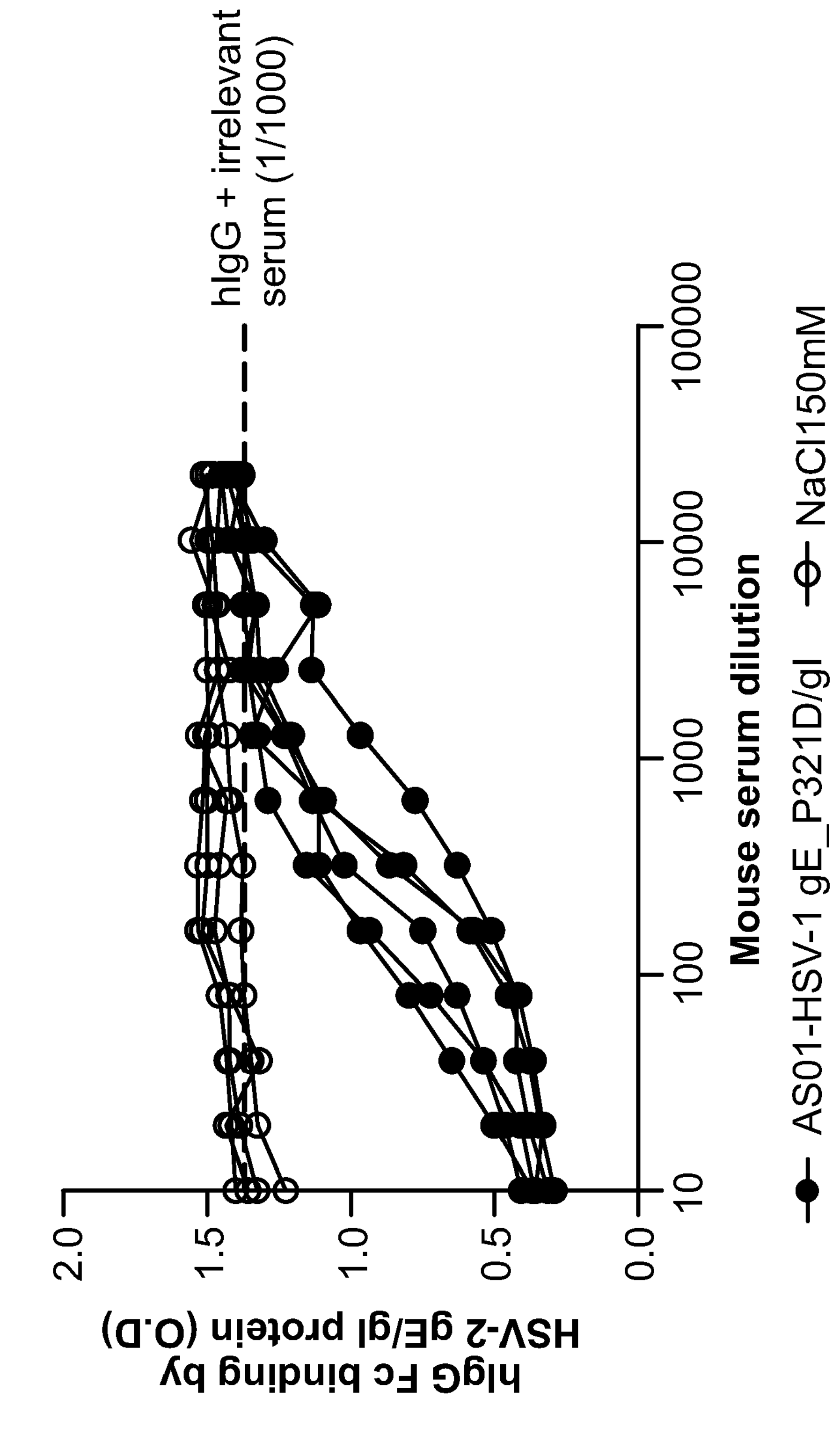

FIG. 44D

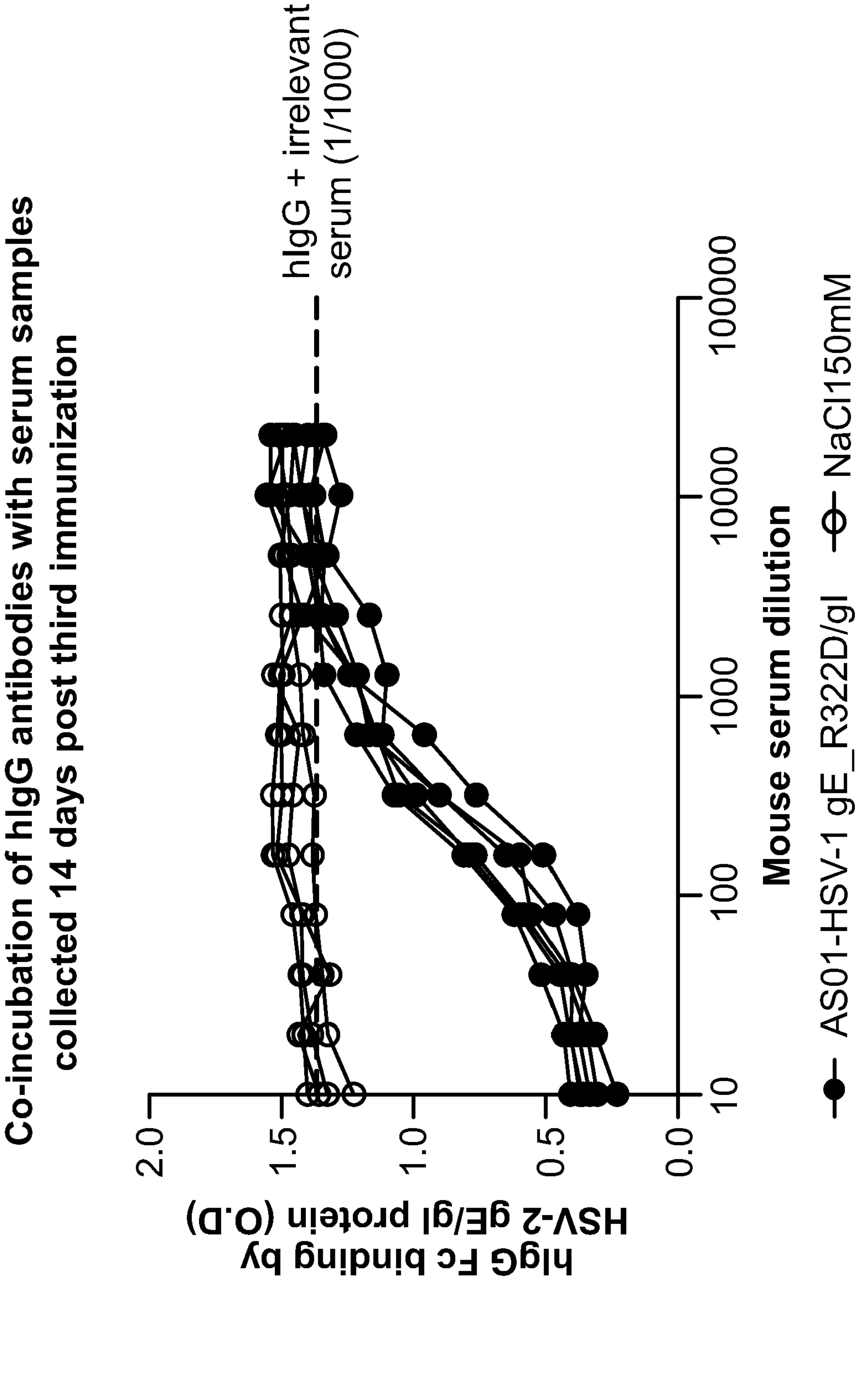
Co-incubation of hIgG antibodies with serum samples collected 14 days post third immunization
hIgG Fc binding by HSV-2 gE/gI protein (O.D)
2.0
1.5
1.0
0.5
0.0
10
100
1000
10000
100000
Mouse serum dilution
hIgG + irrelevant serum (1/1000)
AS01-HSV-1 gE_R322D/gI
NaCl150mM

# FIG. 44E

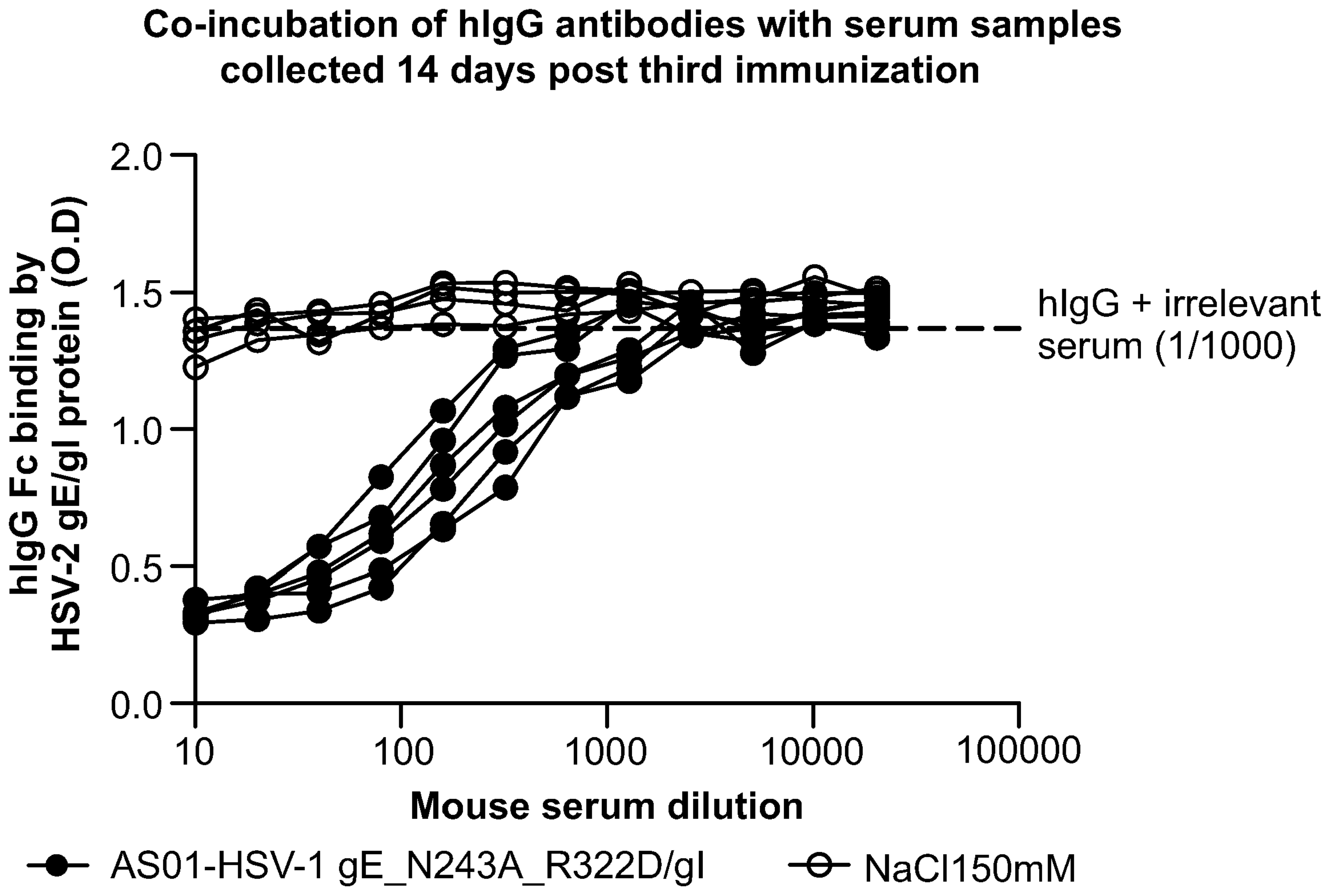

Co-incubation of hIgG antibodies with serum samples collected 14 days post third immunization
hIgG Fc binding by HSV-2 gE/gI protein (O.D)
2.0
1.5
1.0
0.5
0.0
10
100
1000
10000
100000
Mouse serum dilution
hIgG + irrelevant serum (1/1000)
AS01-HSV-1 gE_N243A_R322D/gI
NaCl150mM

# FIG. 44F

**Co-incubation of hIgG antibodies with serum samples collected 14 days post third immunization**

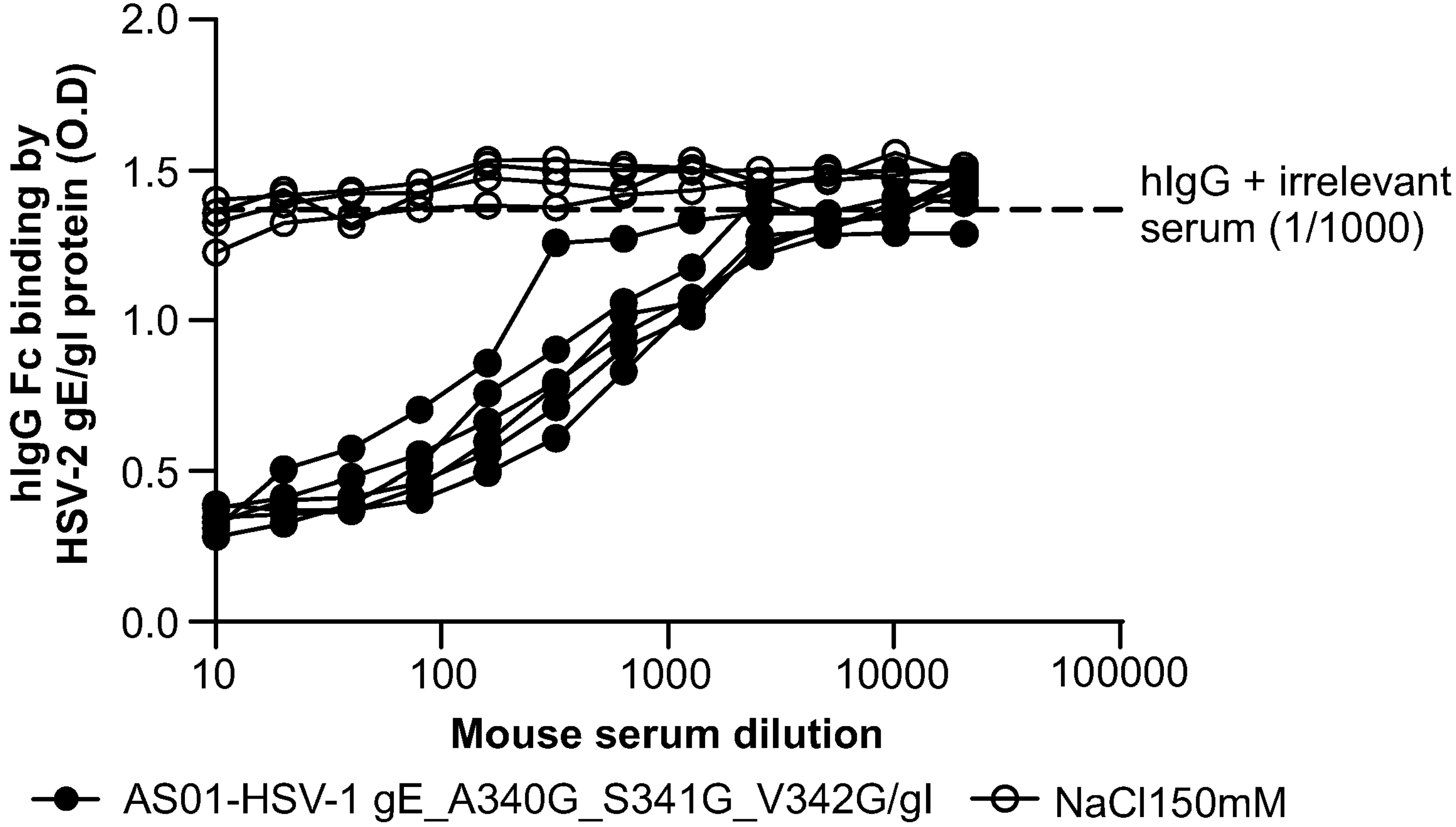

# FIG. 45A

**hIgG Fc binding on HSV-2 gE/gI protein after co-incubation of hIgG antibodies with serum samples collected 14 days post third immunization with different HSV-1 gE/gI candidates**

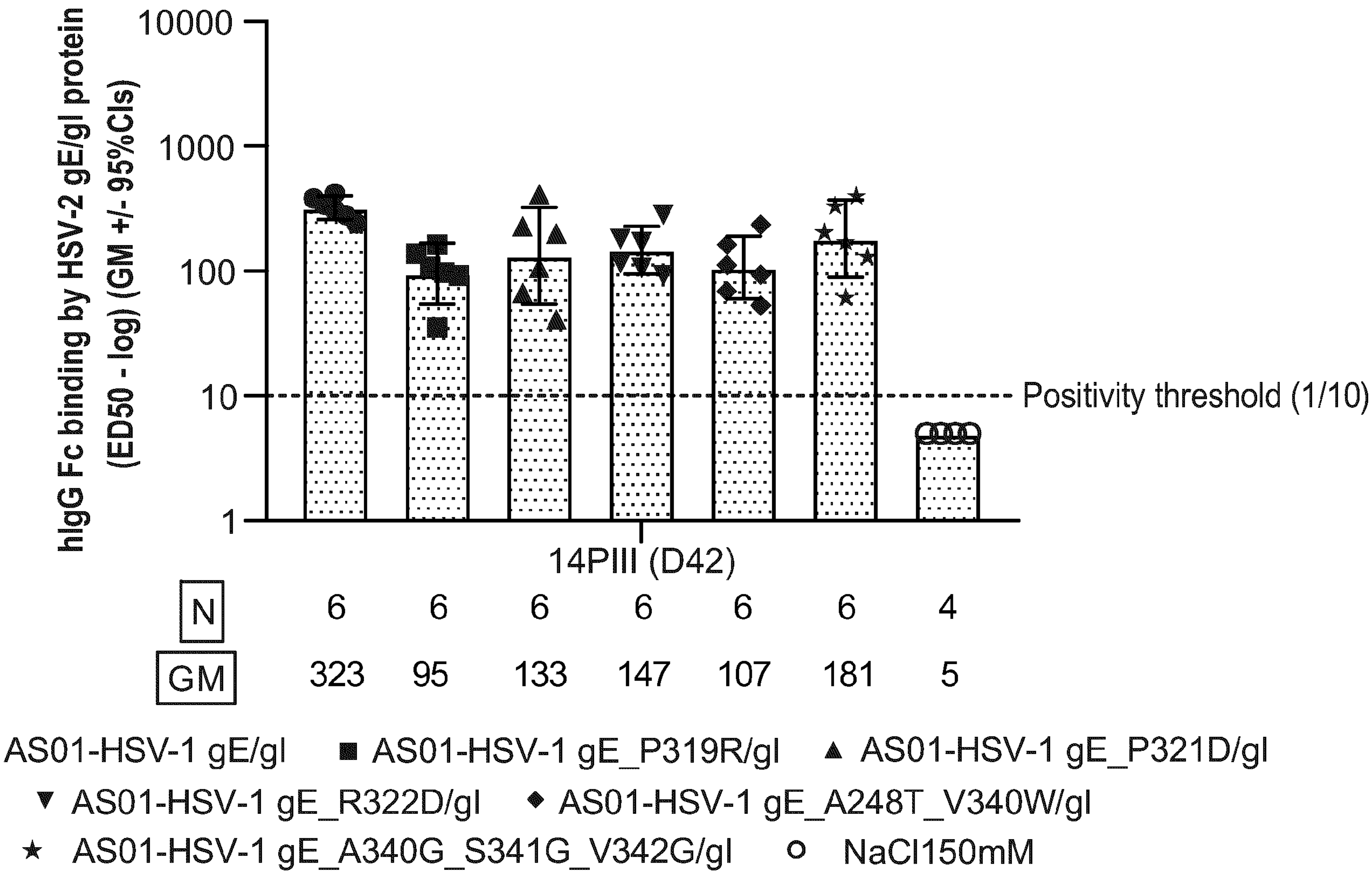

FIG. 45B

GMR with 95% CI of Inhibition of hIgG Fc binding by HSV-2 gE/gI antigen (ED50)
Head to head comparison of HSV-1 gE/gI/AS01(5µg each) groups - 14PIII (Day42)

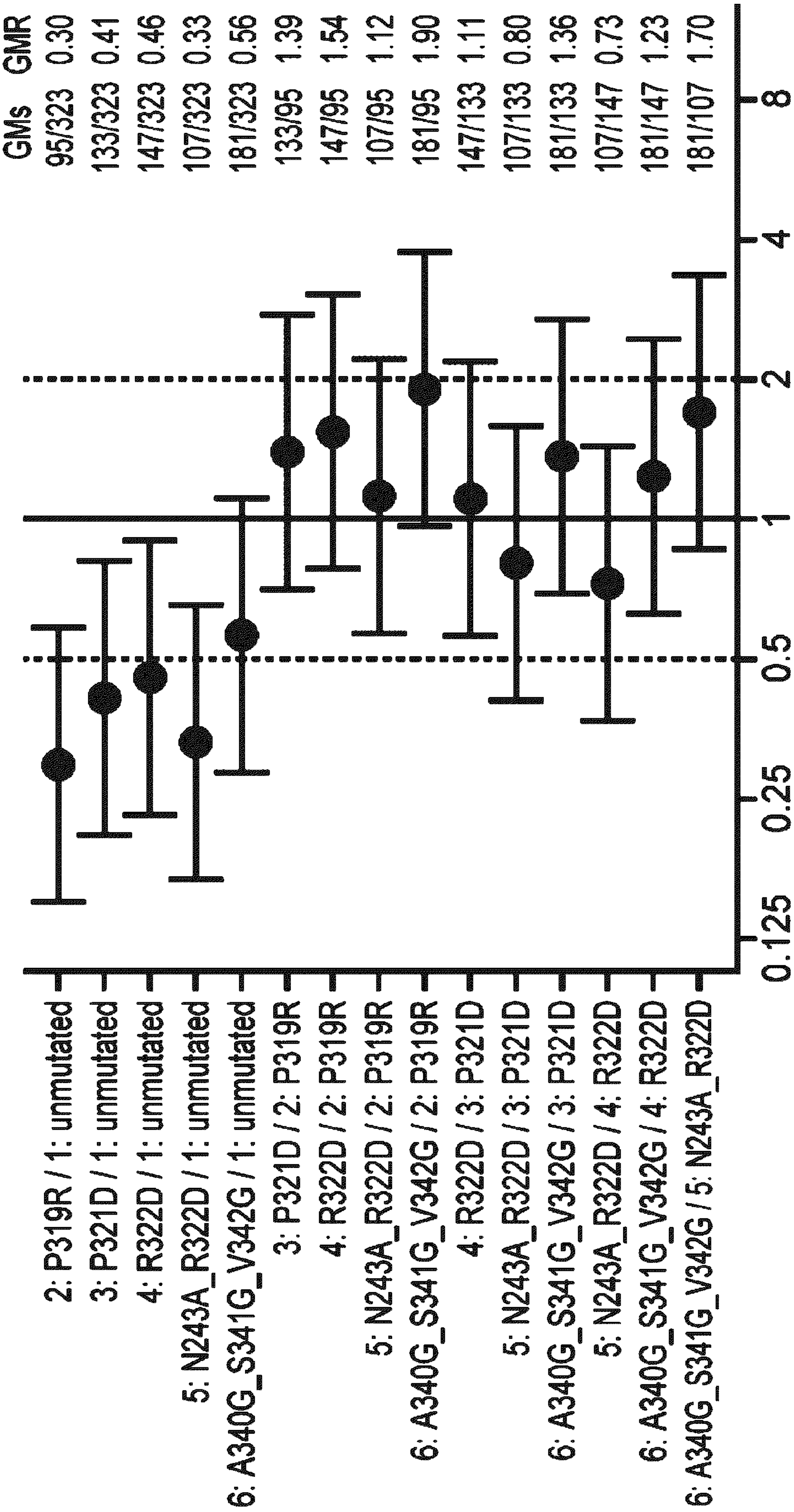

# FIG. 46A

**HSV-2 cross-reactive mFcγRIII binding activity detected in serum samples collected 14 days post third immunization with AS01 adjuvanted HSV-1 gE/gI unmutated protein**

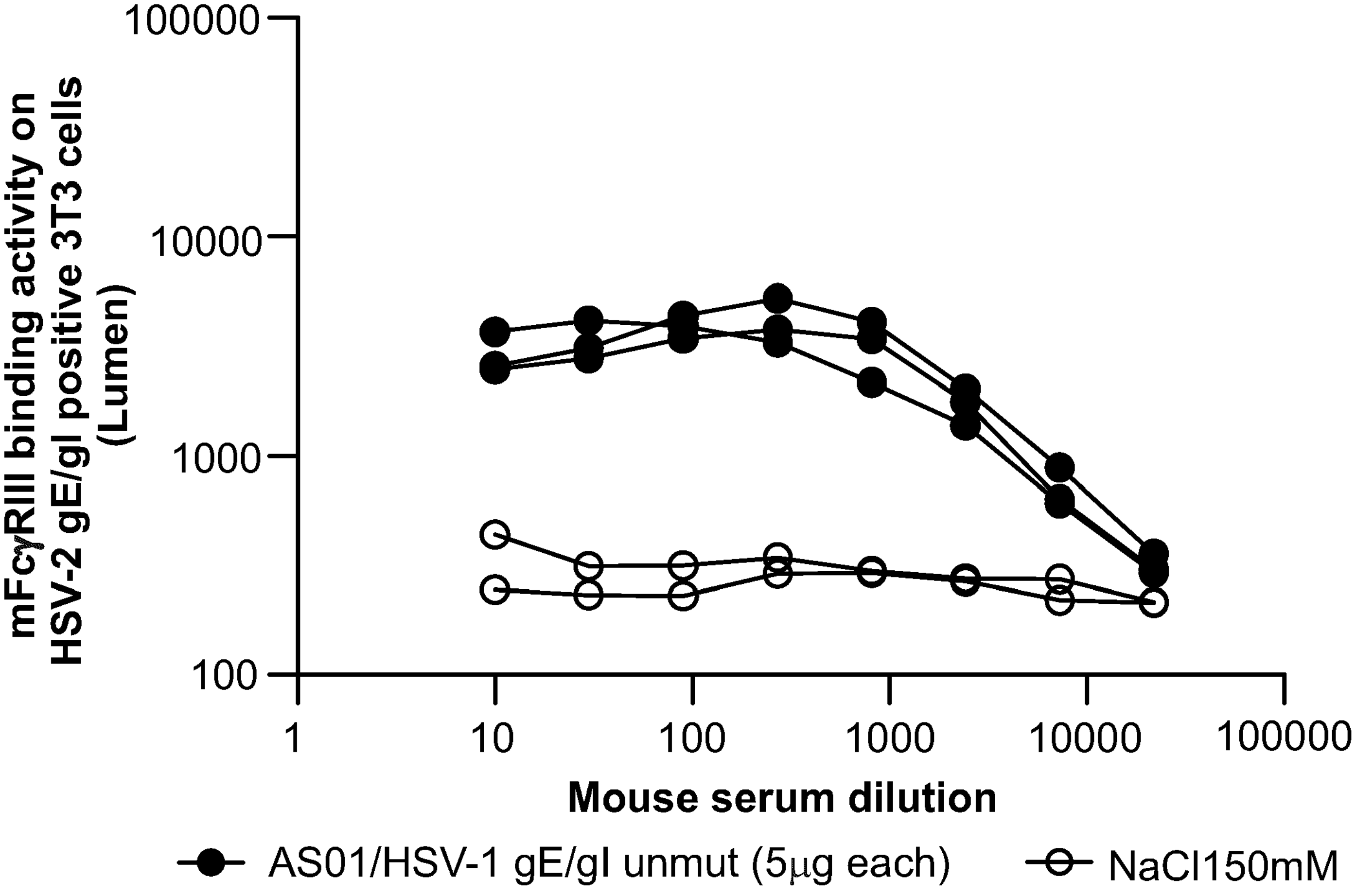

# FIG. 46B

**HSV-2 cross-reactive mFcγRIII binding activity detected in serum samples collected 14 days post third immunization with AS01-adjuvanted HSV-1 gE_P319R/gI mutant protein**

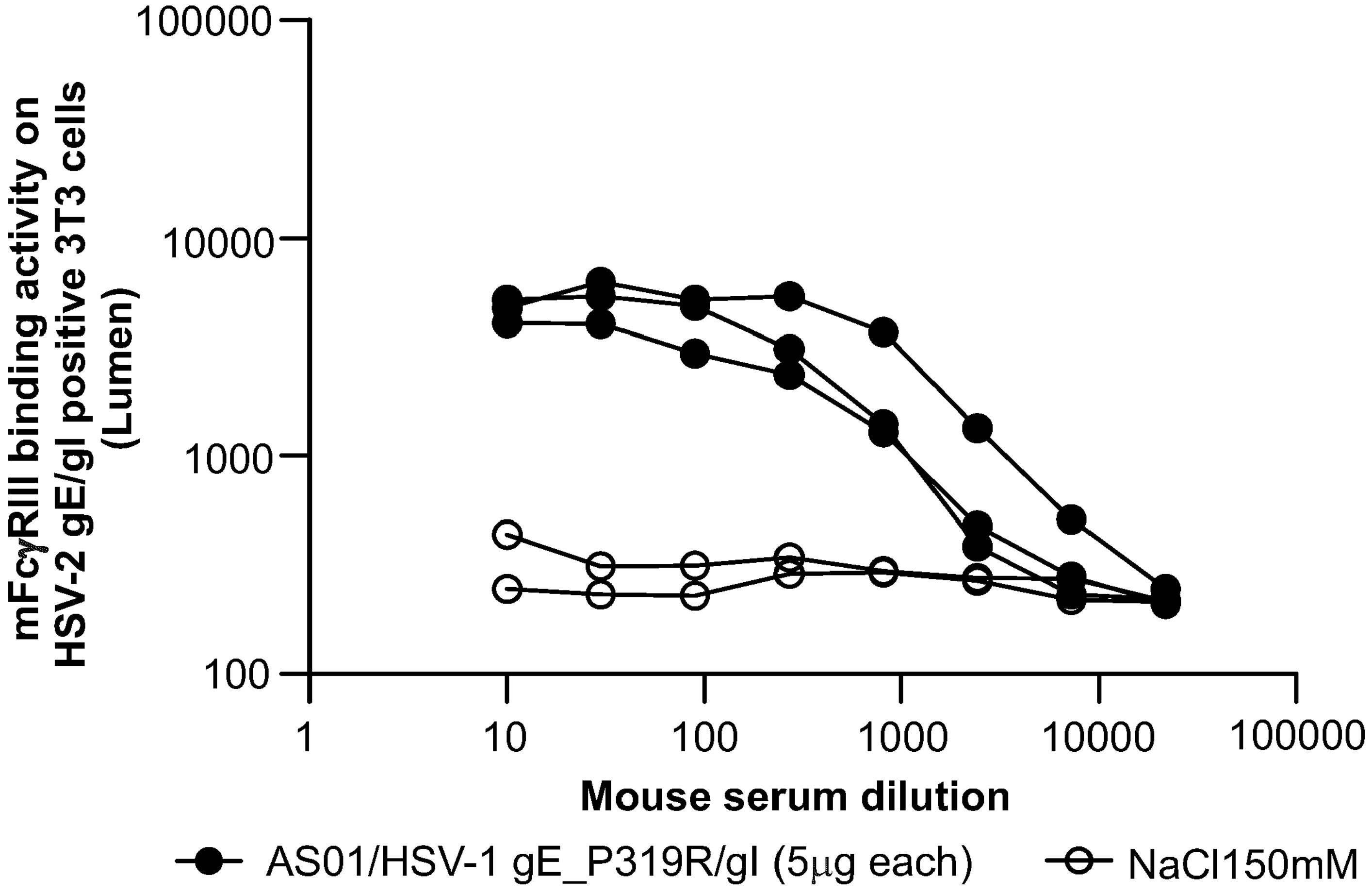

# FIG. 46C

**HSV-2 cross-reactive mFcγRIII binding activity detected in serum samples collected 14 days post third immunization with AS01-adjuvanted HSV-1 gE_P321D/gI mutant protein**

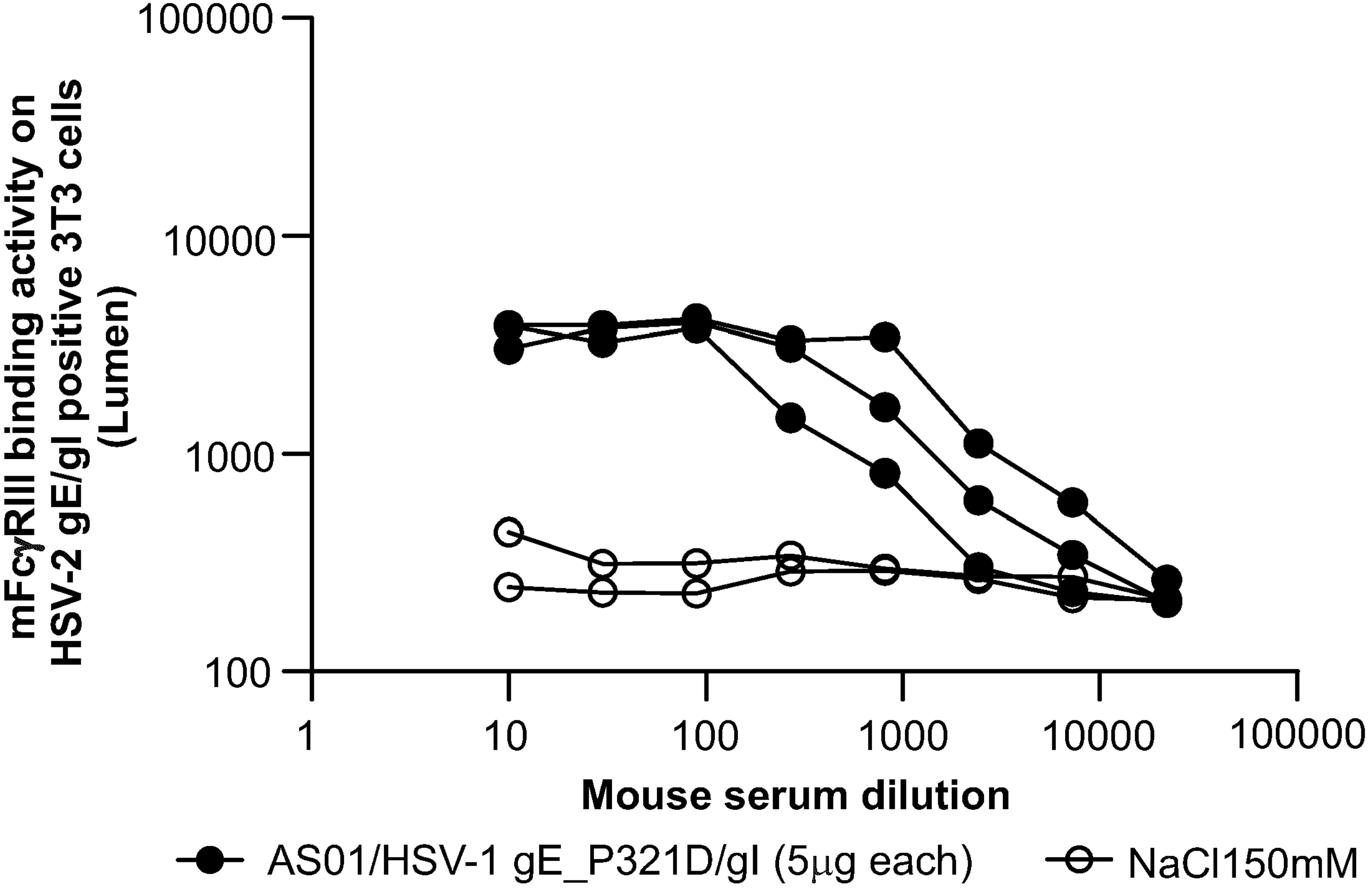

# FIG. 46D

HSV-2 cross-reactive mFcγRIII binding activity detected in serum samples collected 14 days post third immunization with AS01-adjuvanted HSV-1 gE_P322D/gI mutant protein

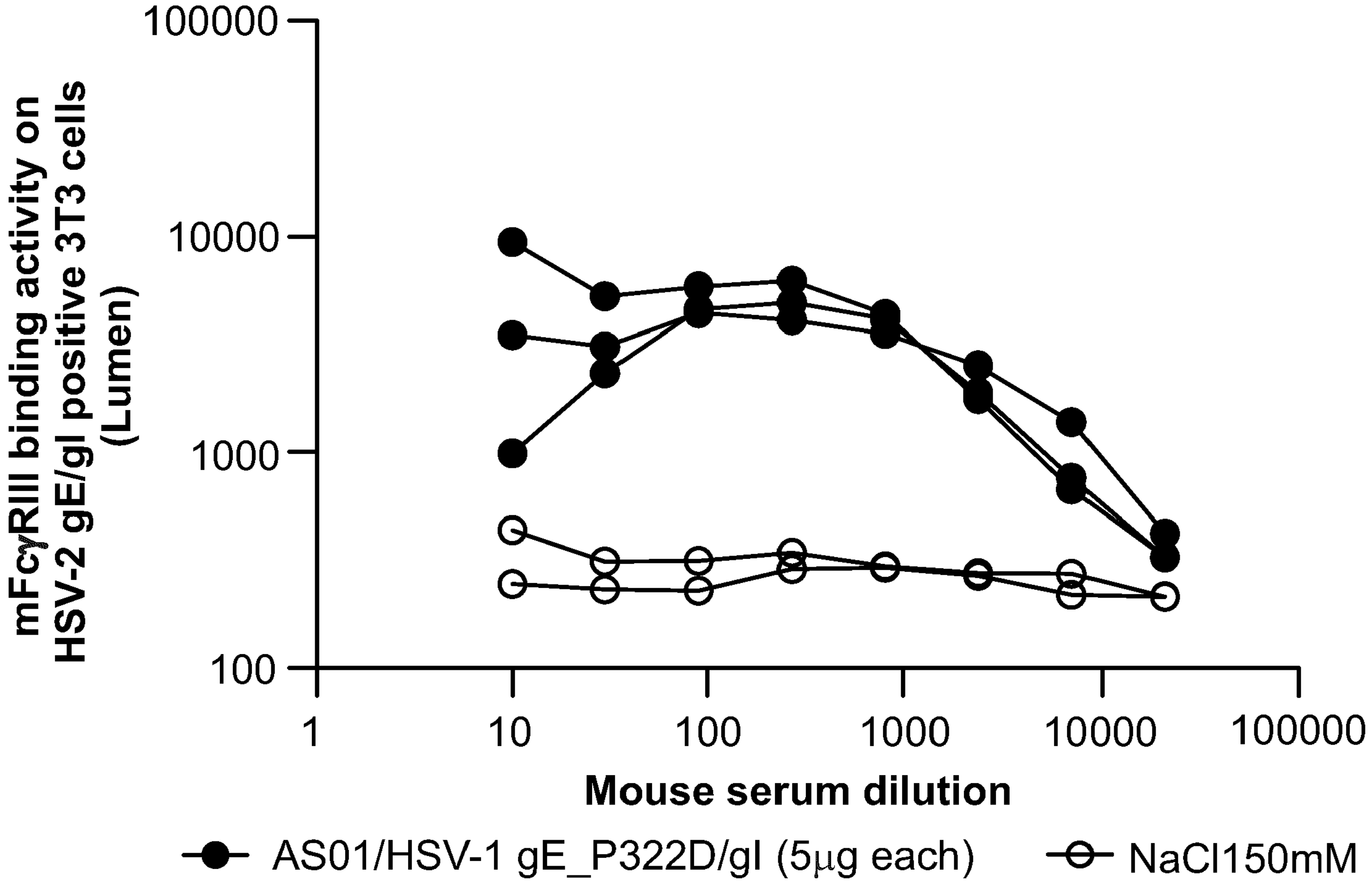

# FIG. 46E

**HSV-2 cross-reactive mFcγRIII binding activity detected in serum samples collected 14 days post third immunization with AS01-adjuvanted HSV-1 gE_N243A_R322D/gI mutant protein**

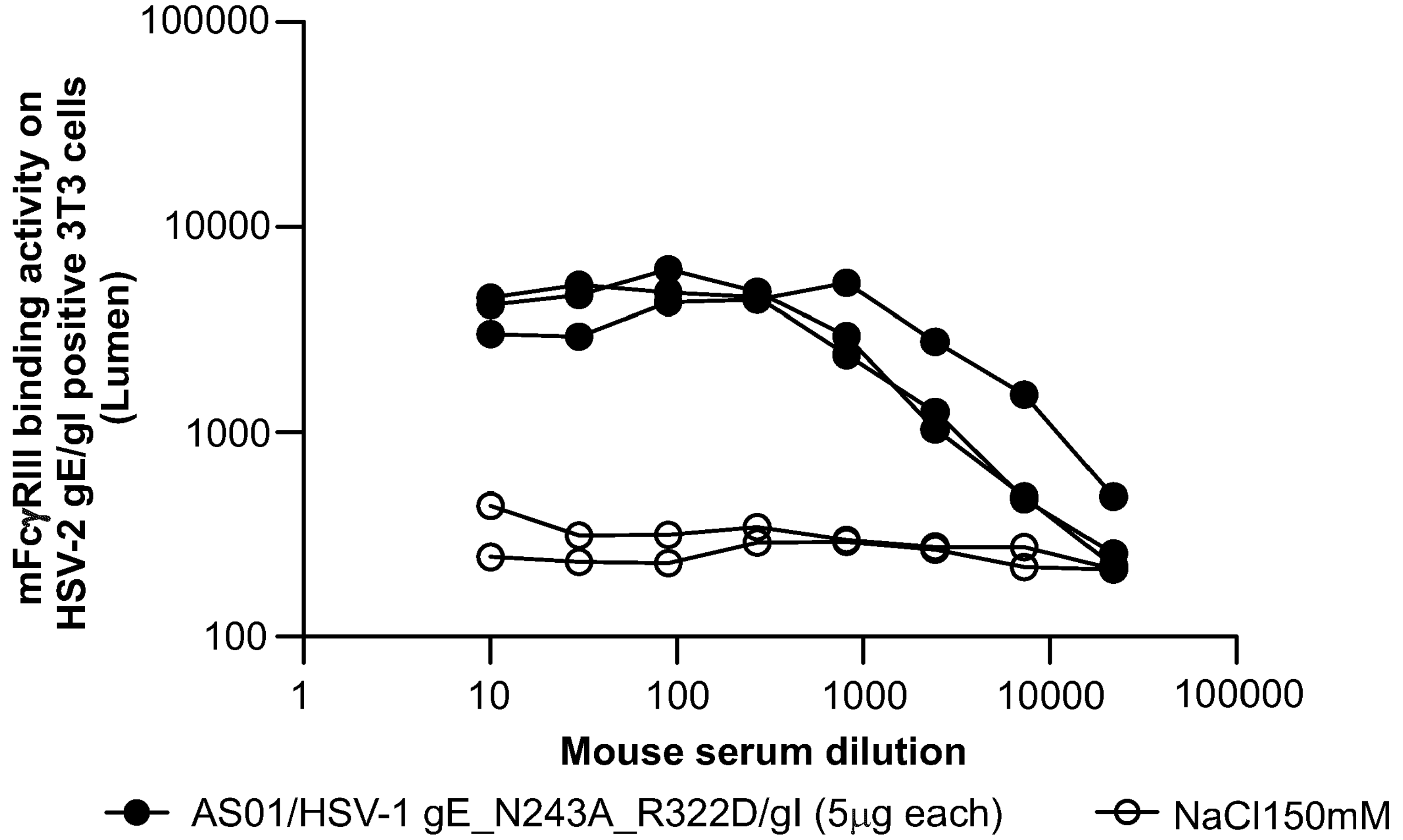

# FIG. 46F

**HSV-2 cross-reactive mFcγRIII binding activity detected in serum samples collected 14 days post third immunization with AS01-adjuvanted HSV-1 gE_A340G_S341G_V342G/gI mutant protein**

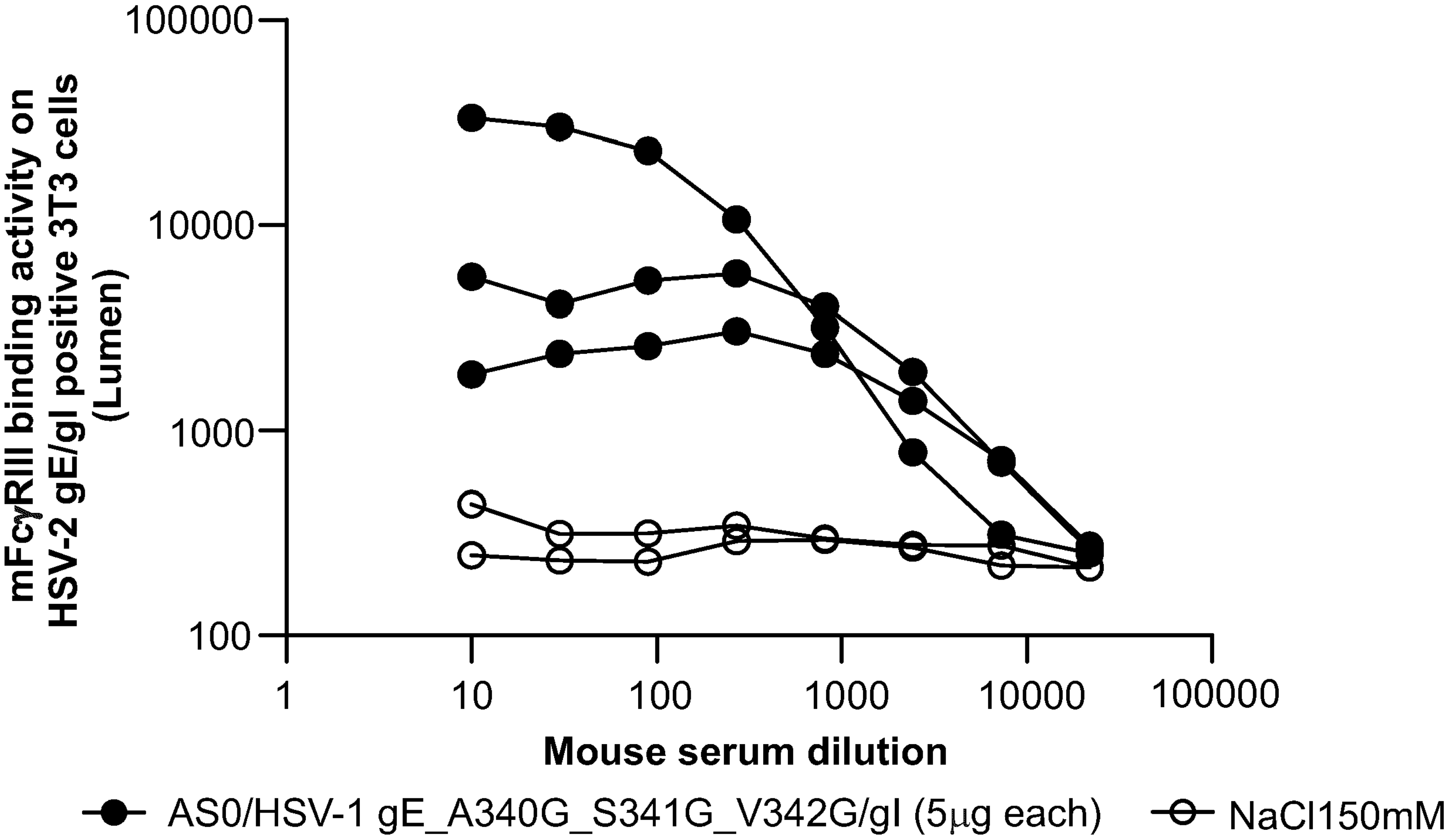

# FIG. 47

## Comparison of the ability of HSV-1-specific antibodies to cross-bind and activate mFcγRIII after incubation with HSV-2 gE/gI transfected cells

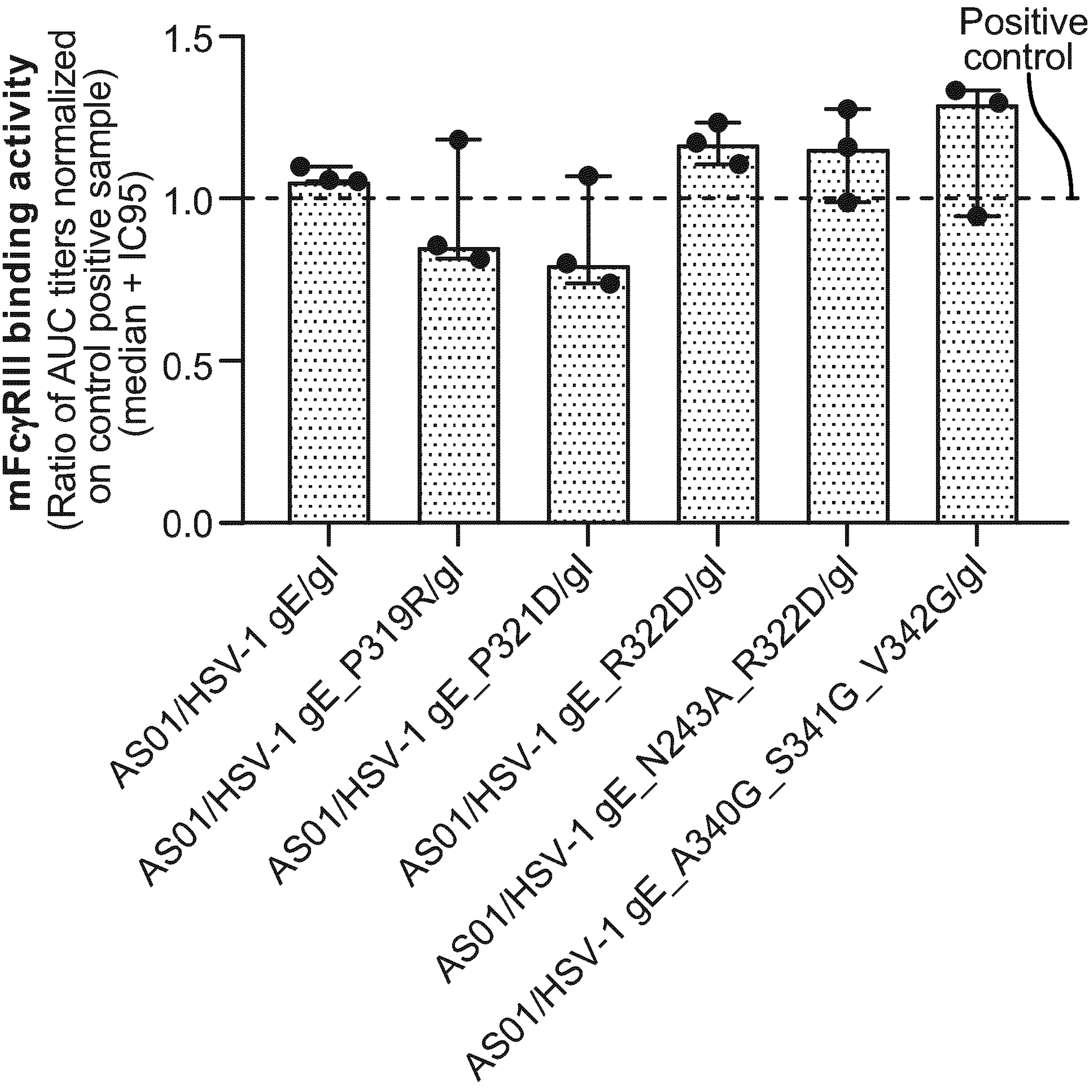

FIG. 48A

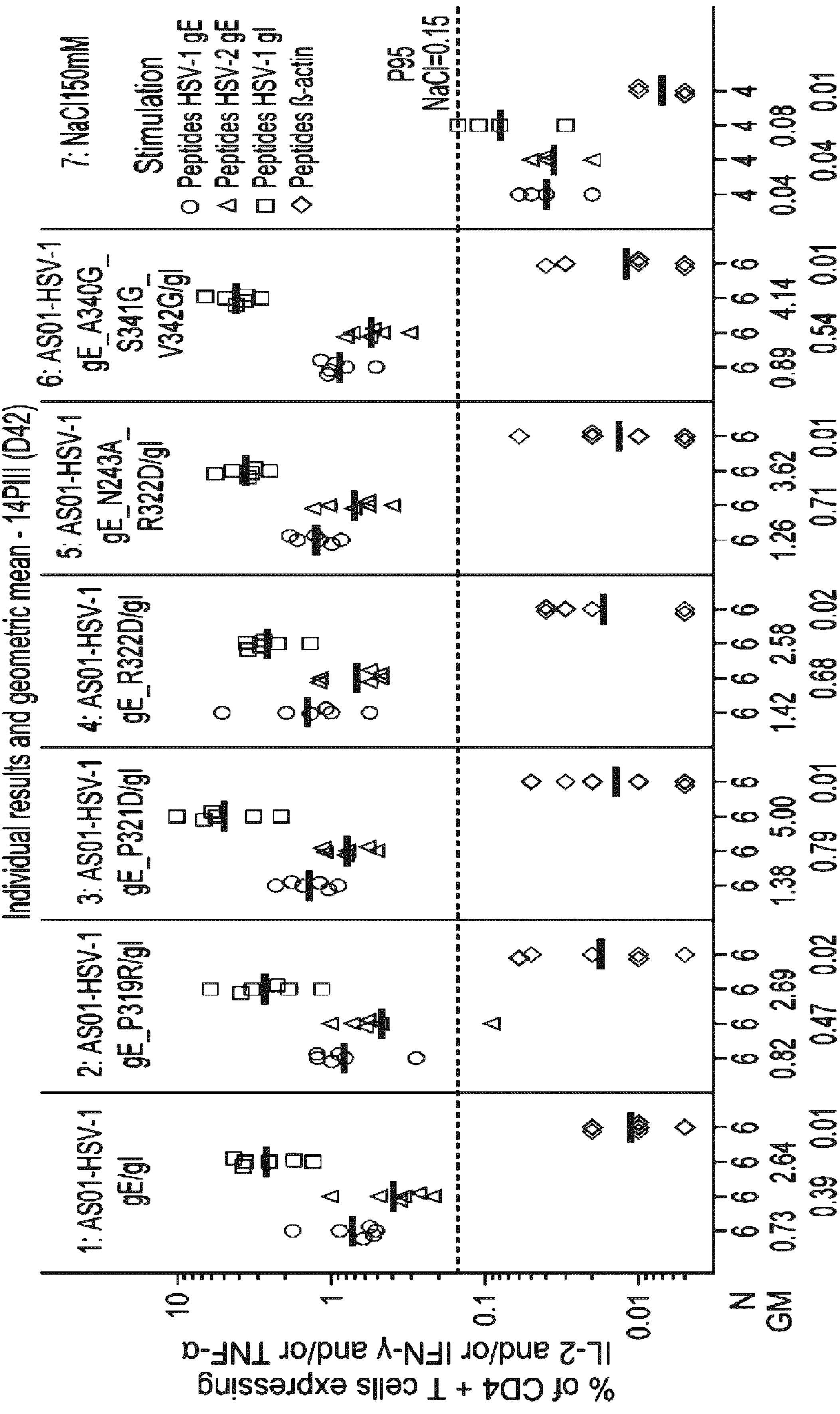
Individual results and geometric mean - 14PIII (D42)
% of CD4 + T cells expressing
IL-2 and/or IFN-γ and/or TNF-α
10
1
0.1
0.01
N
GM
1: AS01-HSV-1 gE/gI
2: AS01-HSV-1 gE_P319R/gI
3: AS01-HSV-1 gE_P321D/gI
4: AS01-HSV-1 gE_R322D/gI
5: AS01-HSV-1 gE_N243A_R322D/gI
6: AS01-HSV-1 gE_A340G_S341G_V342G/gI
7: NaCl150mM
Stimulation
Peptides HSV-1 gE
Peptides HSV-2 gE
Peptides HSV-1 gI
Peptides ß-actin
P95
NaCl=0.15
6 6 6 6
0.73 0.39 2.64 0.01
6 6 6 6
0.82 0.47 2.69 0.02
6 6 6 6
1.38 0.79 5.00 0.01
6 6 6 6
1.42 0.68 2.58 0.02
6 6 6 6
1.26 0.71 3.62 0.01
6 6 6 6
0.89 0.54 4.14 0.01
4 4 4 4
0.04 0.04 0.08 0.01

# FIG. 48B

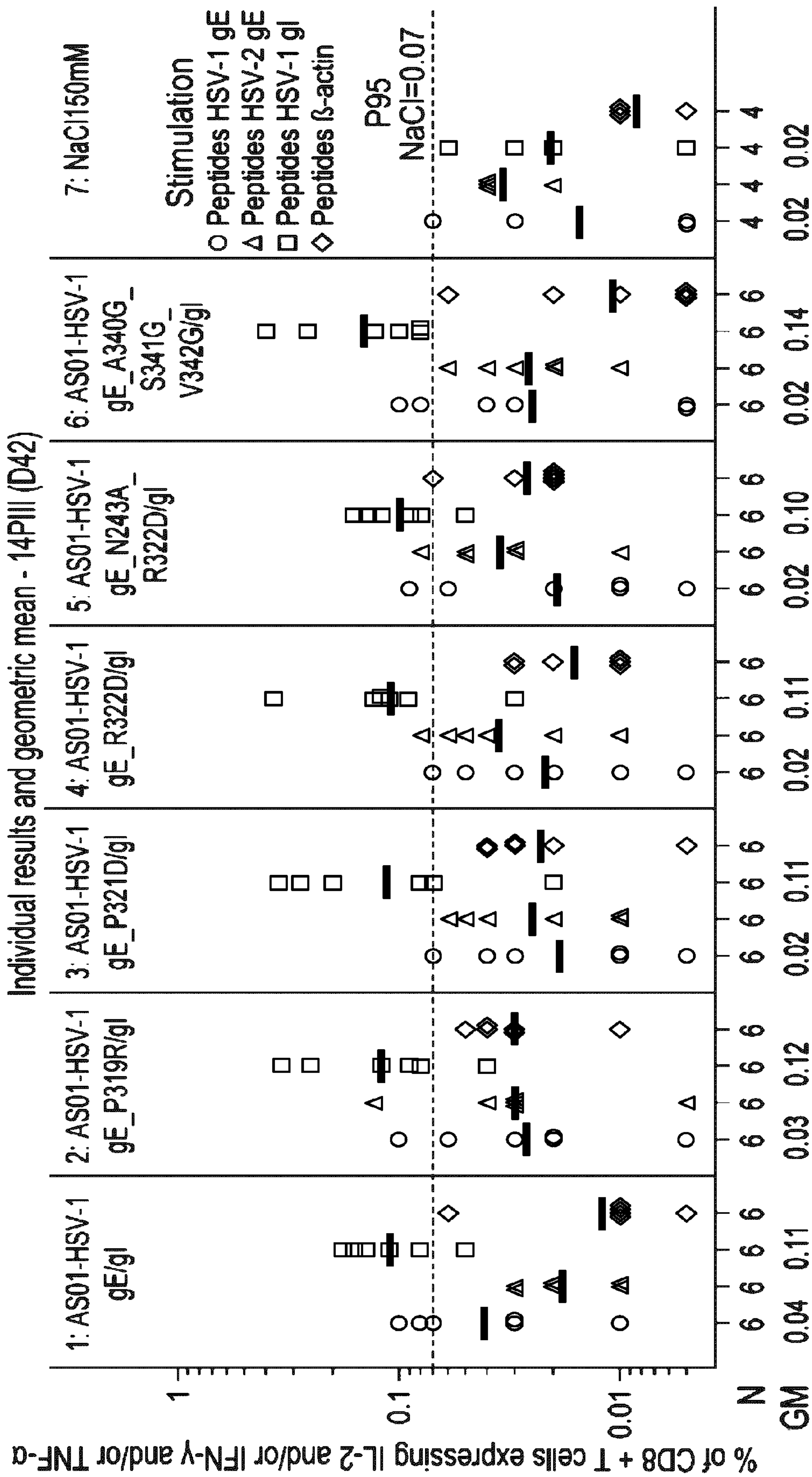

Individual results and geometric mean - 14PIII (D42)
% of CD8 + T cells expressing IL-2 and/or IFN-γ and/or TNF-α
1
0.1
0.01
1: AS01-HSV-1 gE/gI
2: AS01-HSV-1 gE_P319R/gI
3: AS01-HSV-1 gE_P321D/gI
4: AS01-HSV-1 gE_R322D/gI
5: AS01-HSV-1 gE_N243A_R322D/gI
6: AS01-HSV-1 gE_A340G_S341G_V342G/gI
7: NaCl150mM
Stimulation
Peptides HSV-1 gE
Peptides HSV-2 gE
Peptides HSV-1 gI
Peptides ß-actin
P95
NaCl=0.07
N
GM
6 6 6 6
0.04 0.02 0.11 0.01
6 6 6 6
0.03 0.03 0.12 0.03
6 6 6 6
0.02 0.02 0.11 0.02
6 6 6 6
0.02 0.04 0.11 0.02
6 6 6 6
0.02 0.03 0.10 0.03
6 6 6 6
0.02 0.03 0.14 0.01
4 4 4 4
0.02 0.03 0.02 0.01

# FIG. 49A

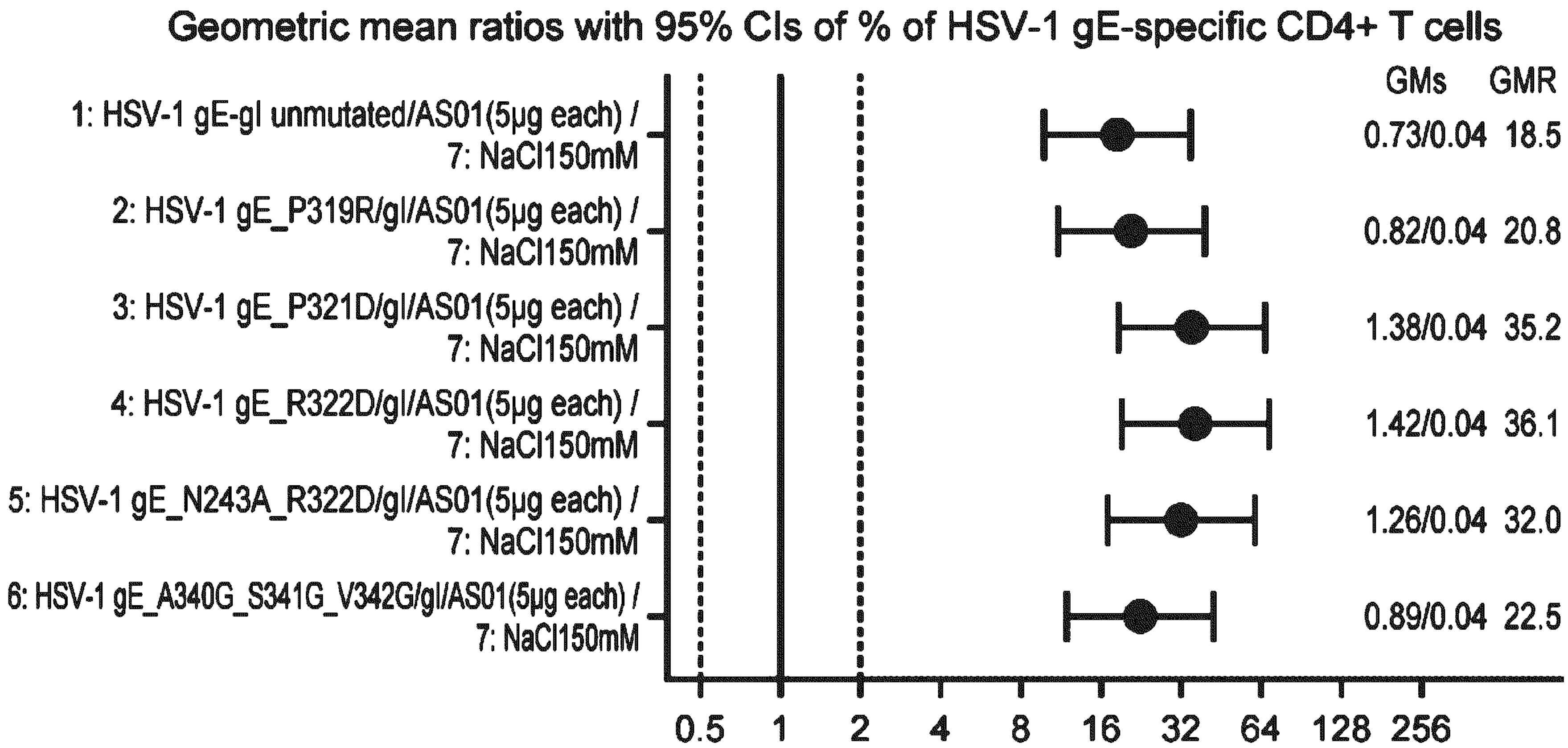
Geometric mean ratios with 95% CIs of % of HSV-1 gE-specific CD4+ T cells
GMs GMR
1: HSV-1 gE-gI unmutated/AS01(5µg each) / 7: NaCl150mM
0.73/0.04 18.5
2: HSV-1 gE_P319R/gI/AS01(5µg each) / 7: NaCl150mM
0.82/0.04 20.8
3: HSV-1 gE_P321D/gI/AS01(5µg each) / 7: NaCl150mM
1.38/0.04 35.2
4: HSV-1 gE_R322D/gI/AS01(5µg each) / 7: NaCl150mM
1.42/0.04 36.1
5: HSV-1 gE_N243A_R322D/gI/AS01(5µg each) / 7: NaCl150mM
1.26/0.04 32.0
6: HSV-1 gE_A340G_S341G_V342G/gI/AS01(5µg each) / 7: NaCl150mM
0.89/0.04 22.5
0.5
1
2
4
8
16
32
64
128
256

# FIG. 49B

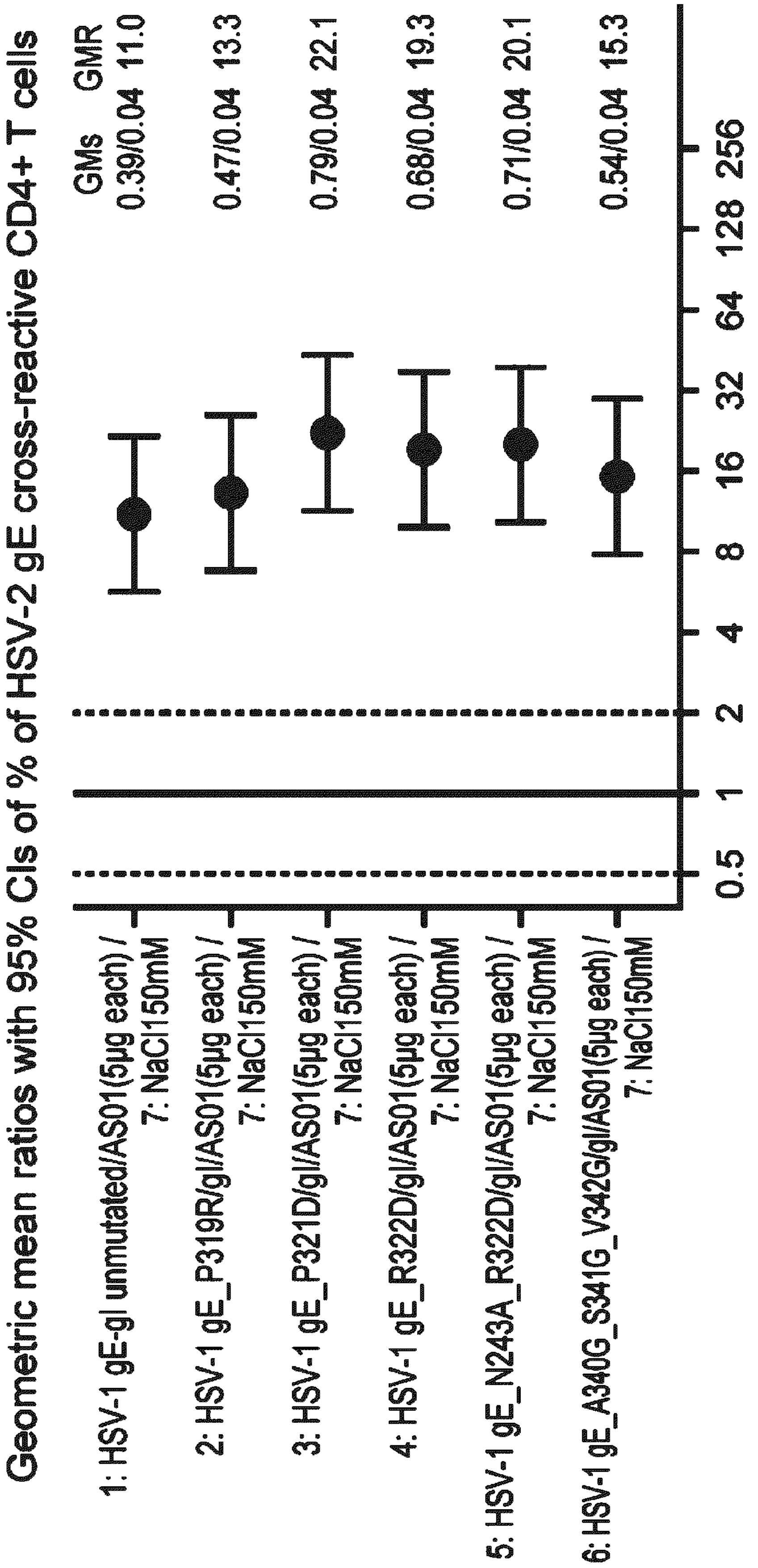
Geometric mean ratios with 95% CIs of % of HSV-2 gE cross-reactive CD4+ T cells
GMs GMR
1: HSV-1 gE-gI unmutated/AS01(5µg each) / 7: NaCl 150mM
0.39/0.04 11.0
2: HSV-1 gE_P319R/gI/AS01(5µg each) / 7: NaCl 150mM
0.47/0.04 13.3
3: HSV-1 gE_P321D/gI/AS01(5µg each) / 7: NaCl 150mM
0.79/0.04 22.1
4: HSV-1 gE_R322D/gI/AS01(5µg each) / 7: NaCl 150mM
0.68/0.04 19.3
5: HSV-1 gE_N243A_R322D/gI/AS01(5µg each) / 7: NaCl 150mM
0.71/0.04 20.1
6: HSV-1 gE_A340G_S341G_V342G/gI/AS01(5µg each) / 7: NaCl 150mM
0.54/0.04 15.3
0.5
1
2
4
8
16
32
64
128
256

FIG. 49C

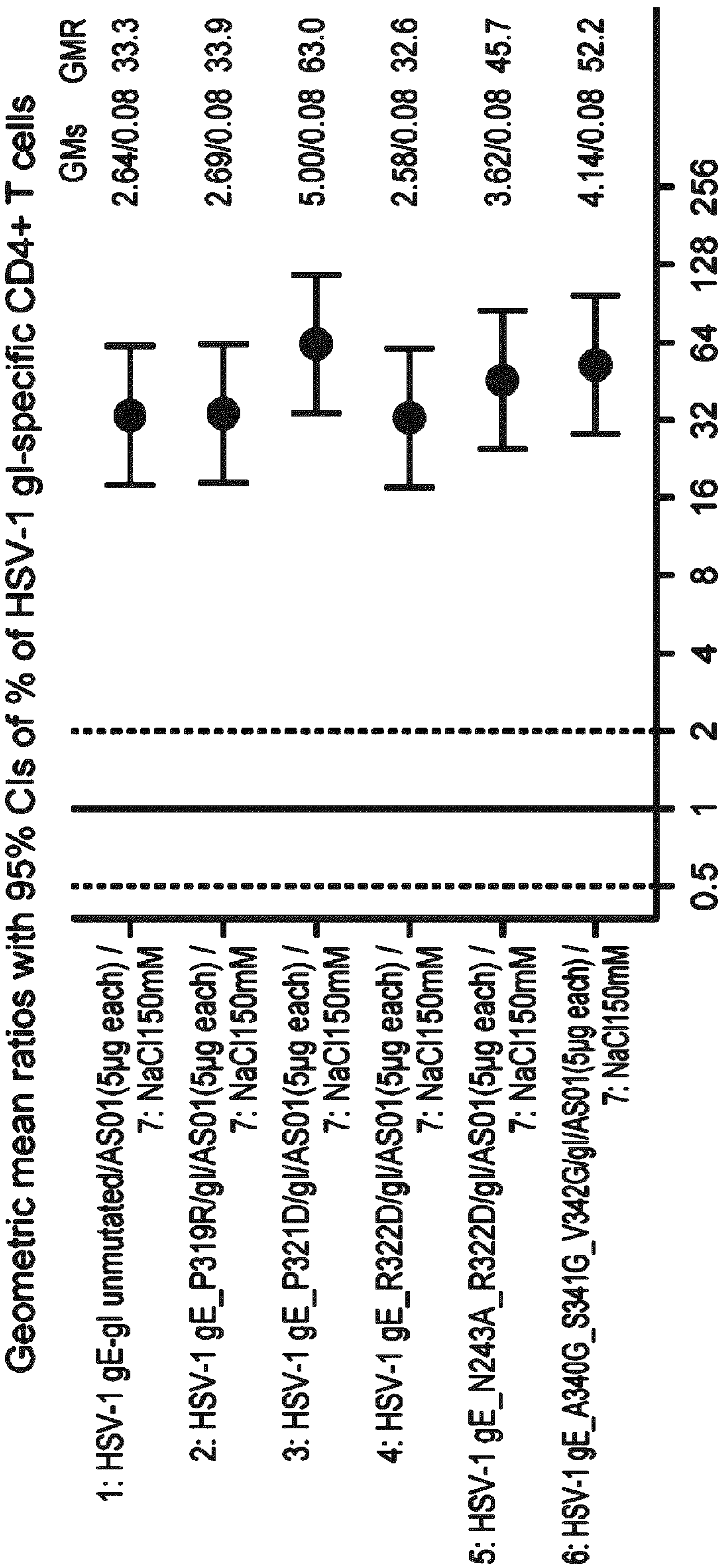
Geometric mean ratios with 95% CIs of % of HSV-1 gI-specific CD4+ T cells
GMs GMR
1: HSV-1 gE-gI unmutated/AS01(5µg each) / 7: NaCl150mM 2.64/0.08 33.3
2: HSV-1 gE_P319R/gI/AS01(5µg each) / 7: NaCl150mM 2.69/0.08 33.9
3: HSV-1 gE_P321D/gI/AS01(5µg each) / 7: NaCl150mM 5.00/0.08 63.0
4: HSV-1 gE_R322D/gI/AS01(5µg each) / 7: NaCl150mM 2.58/0.08 32.6
5: HSV-1 gE_N243A_R322D/gI/AS01(5µg each) / 7: NaCl150mM 3.62/0.08 45.7
6: HSV-1 gE_A340G_S341G_V342G/gI/AS01(5µg each) / 7: NaCl150mM 4.14/0.08 52.2
0.5
1
2
4
8
16
32
64
128
256

FIG. 50

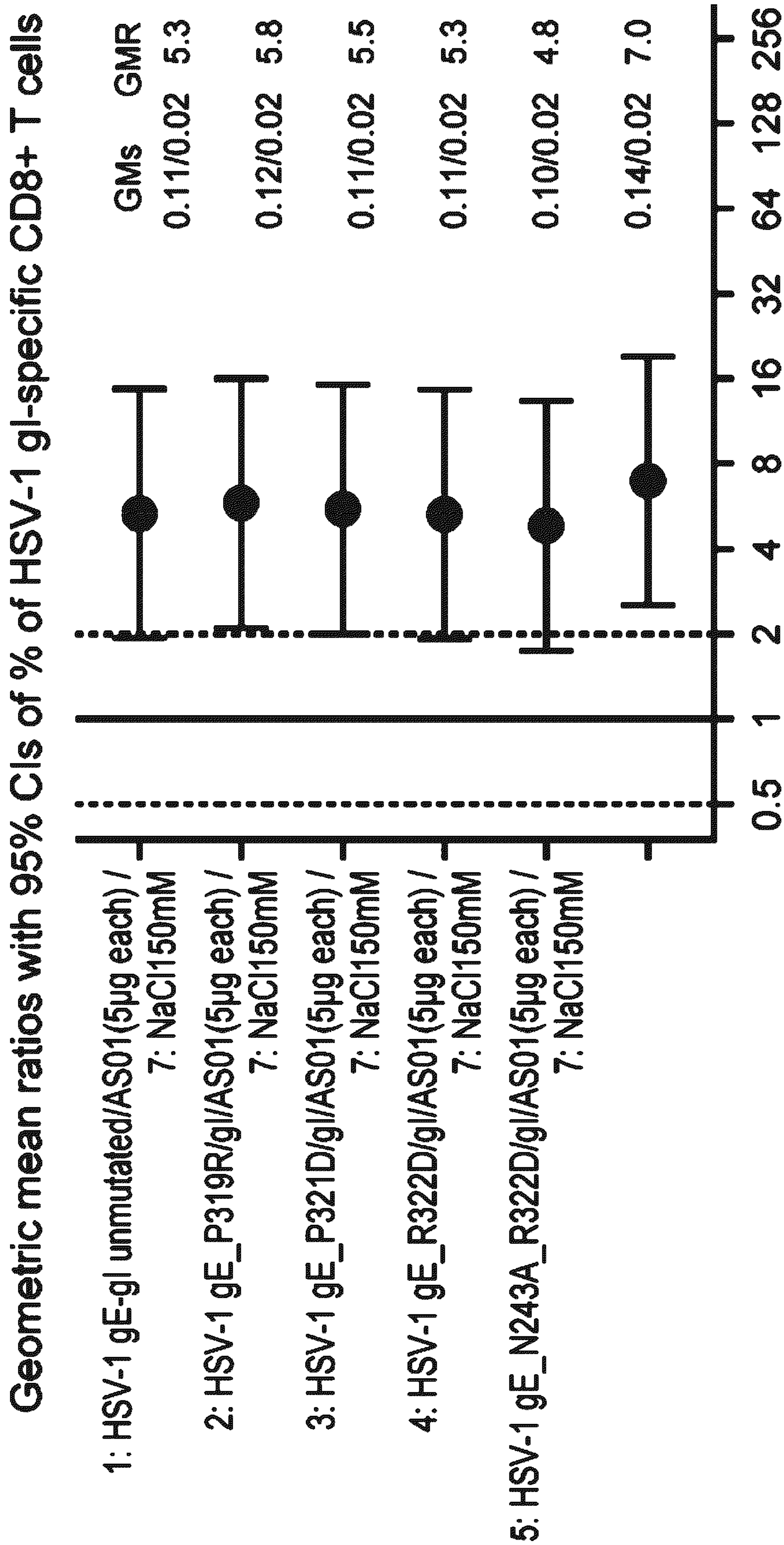
Geometric mean ratios with 95% CIs of % of HSV-1 gI-specific CD8+ T cells
GMs GMR
0.11/0.02 5.3
0.12/0.02 5.8
0.11/0.02 5.5
0.11/0.02 5.3
0.10/0.02 4.8
0.14/0.02 7.0
1: HSV-1 gE-gI unmutated/AS01(5µg each) / 7: NaCl150mM
2: HSV-1 gE_P319R/gI/AS01(5µg each) / 7: NaCl150mM
3: HSV-1 gE_P321D/gI/AS01(5µg each) / 7: NaCl150mM
4: HSV-1 gE_R322D/gI/AS01(5µg each) / 7: NaCl150mM
5: HSV-1 gE_N243A_R322D/gI/AS01(5µg each) / 7: NaCl150mM
0.5
1
2
4
8
16
32
64
128
256

FIG. 51A Geometric mean ratios with 95% CIs of % of HSV-1 gE-specific CD4+ T cells
Head to head comparison of HSV-1 gE/gI/AS01(5µg each) groups

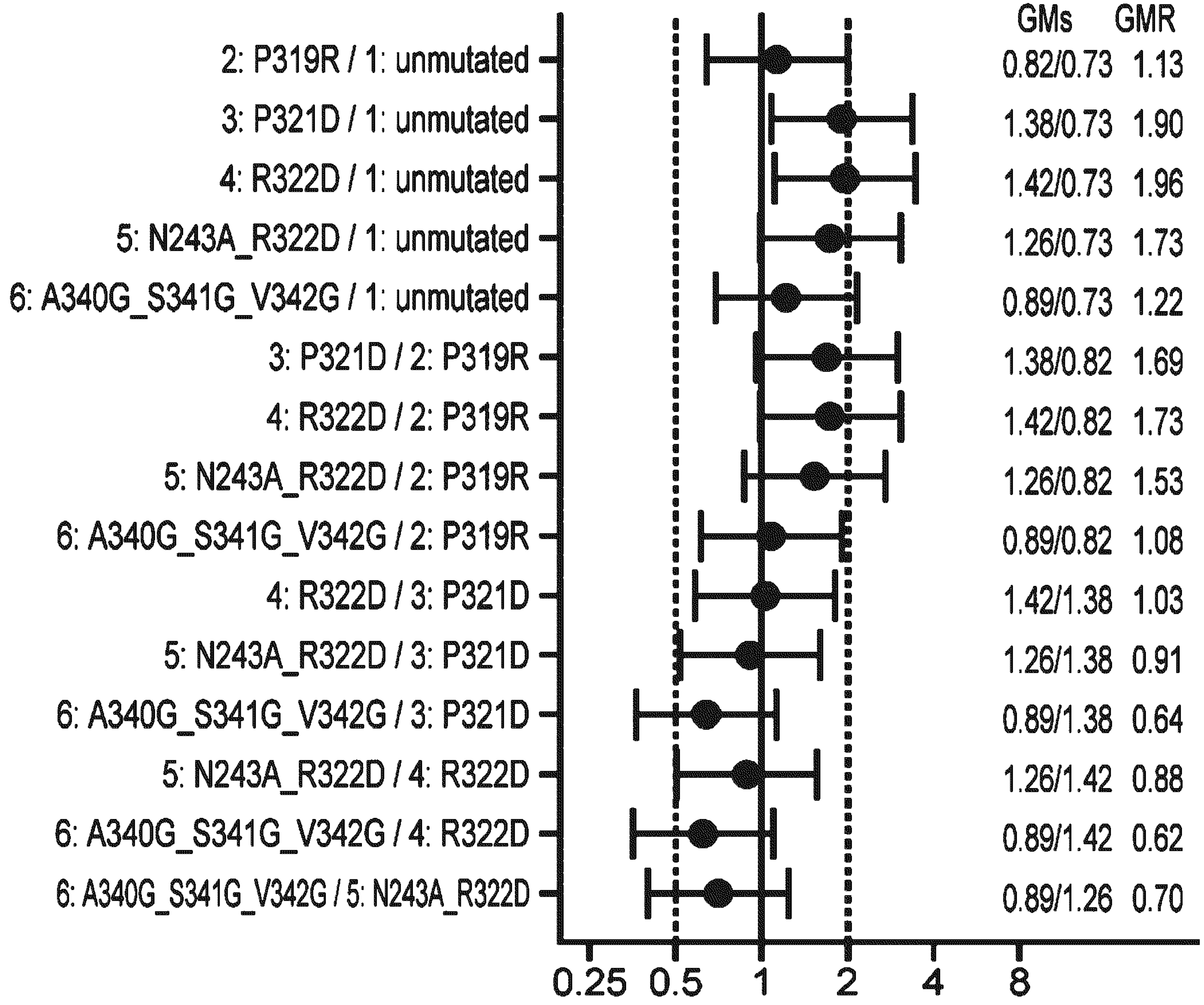

FIG. 51B Geometric mean ratios with 95% CIs of % of HSV-2 gE cross-reactive CD4+ T cells
Head to head comparison of HSV-1 gE/gI/AS01(5µg each) groups

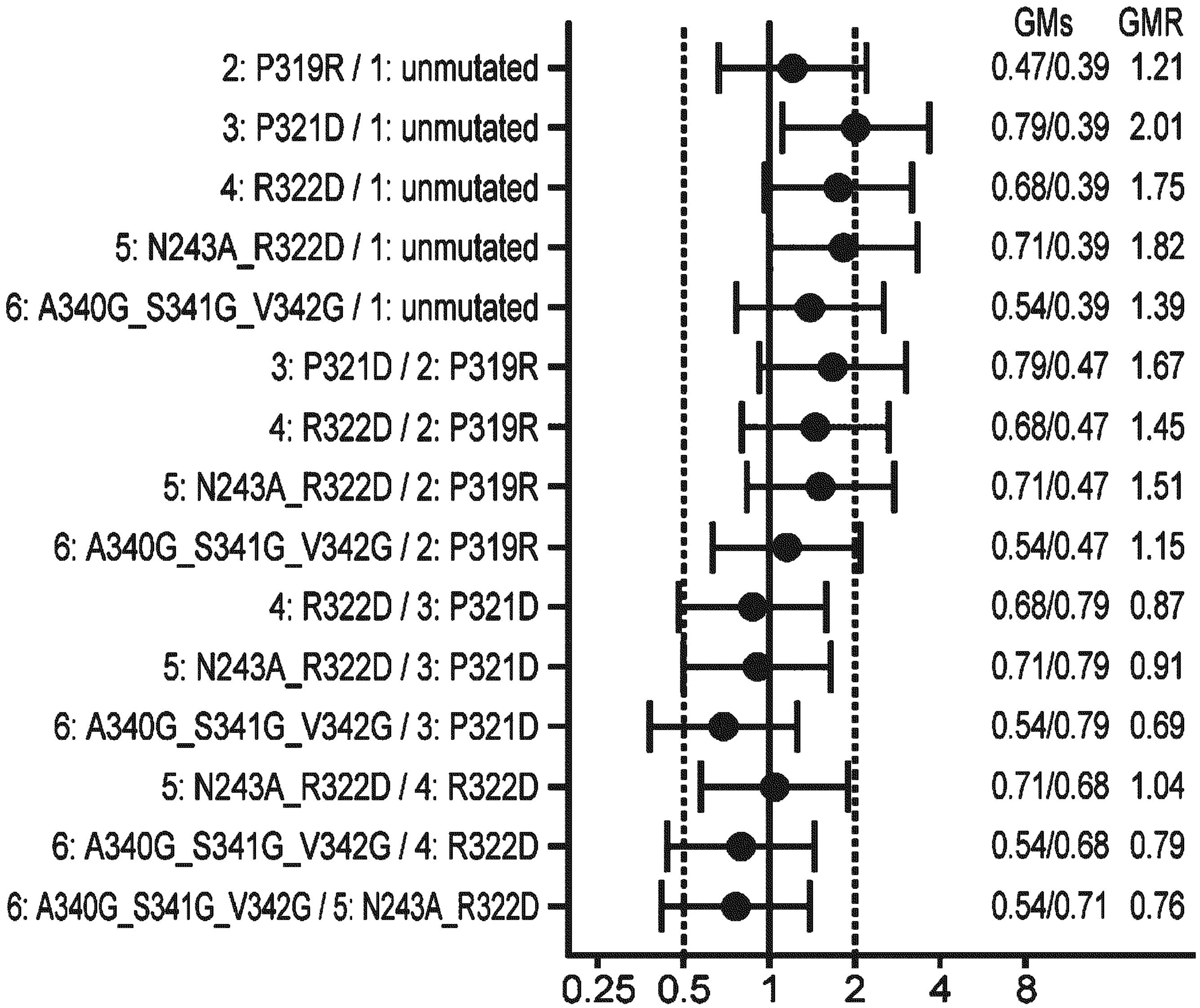

**FIG. 52** Geometric mean ratios with 95% CIs of % of HSV-1 gI-specific CD4+ T cells
Head to head comparison of HSV-1 gE/gI/AS01(5µg each) groups

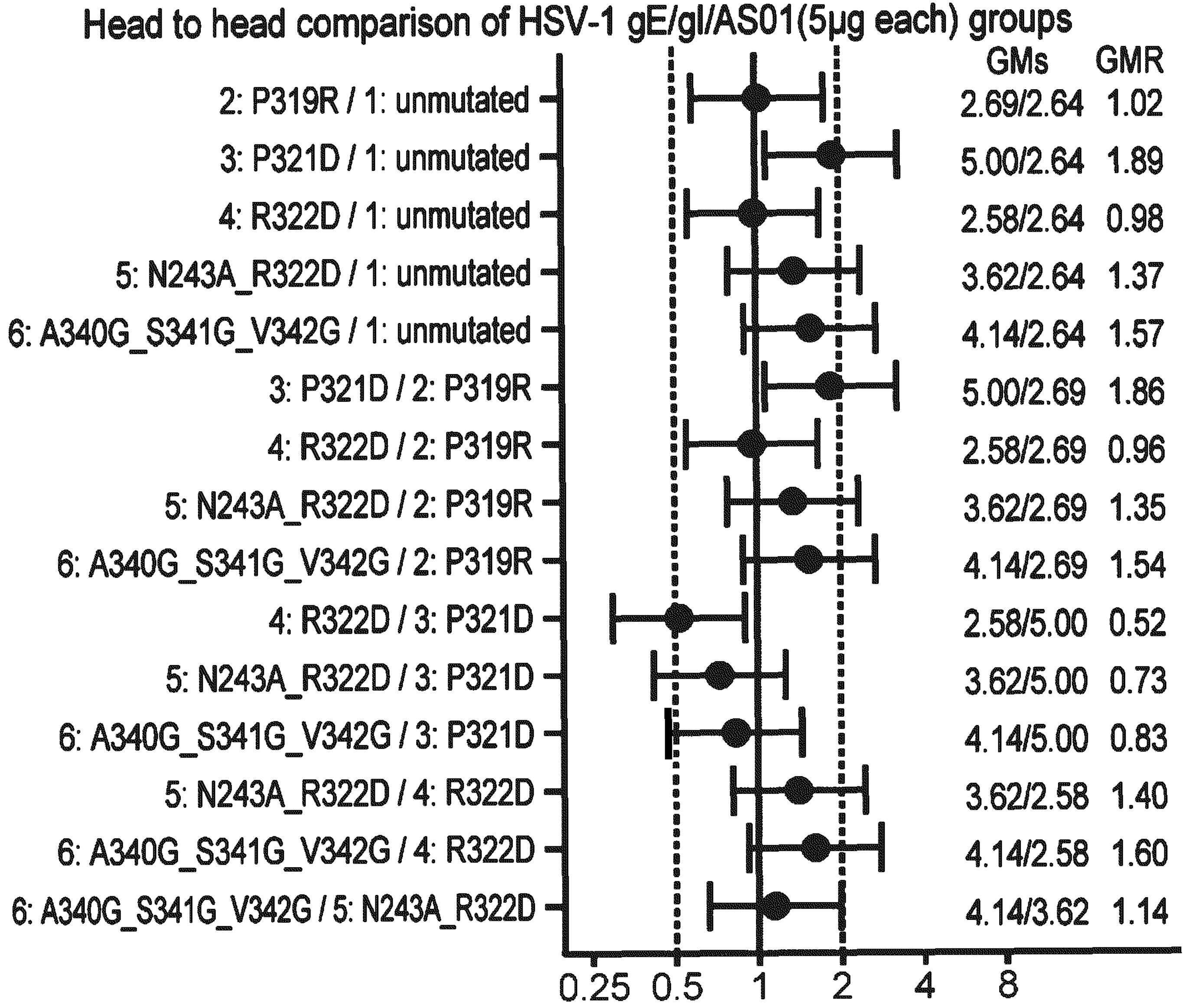

# FIG. 53

Individual results and geometric mean

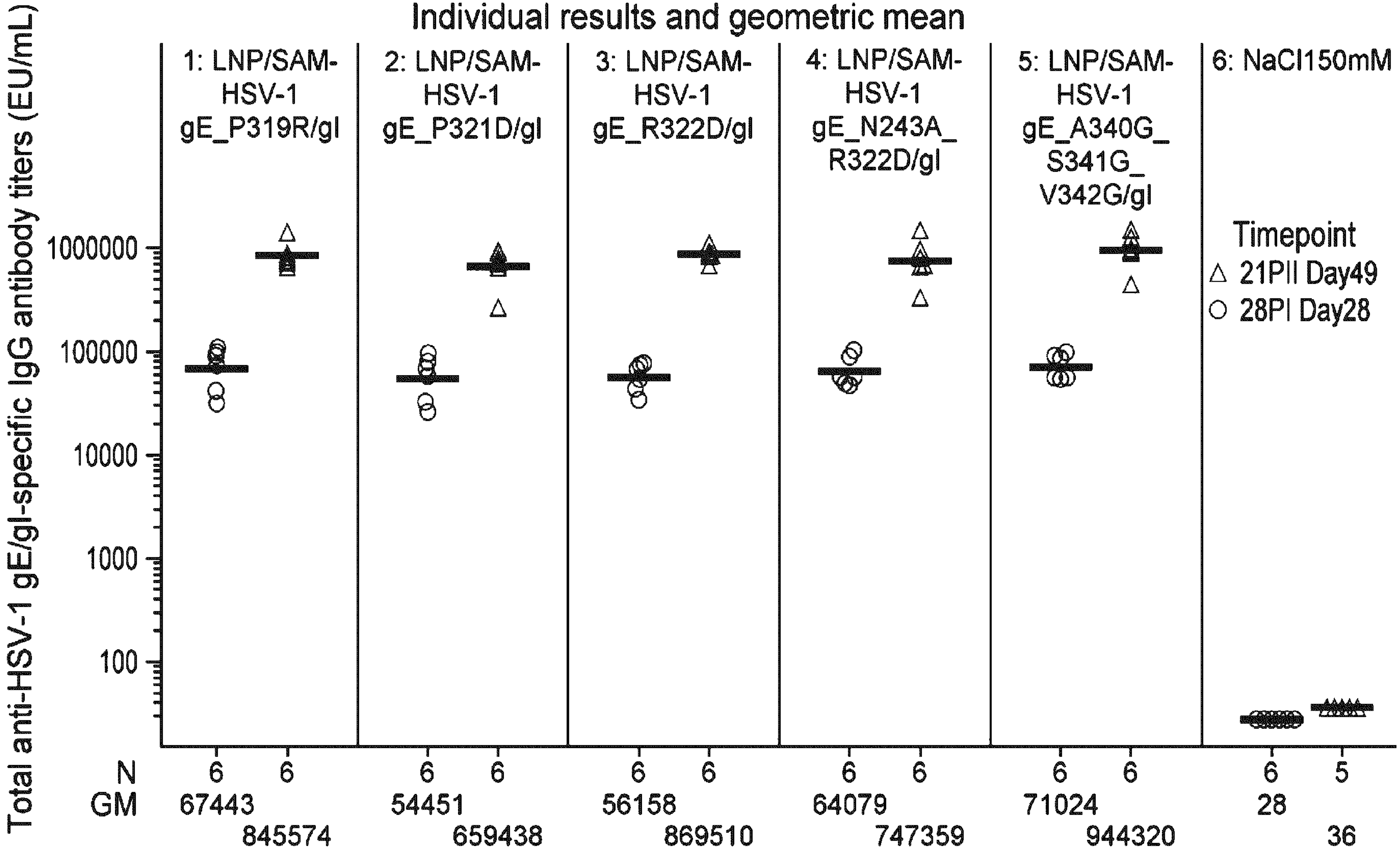

FIG. 54

GMR with 95% CIs of Total anti-HSV-1 gE/gI-specific IgG antibody titers (EU/mL) - 21PII(D49)

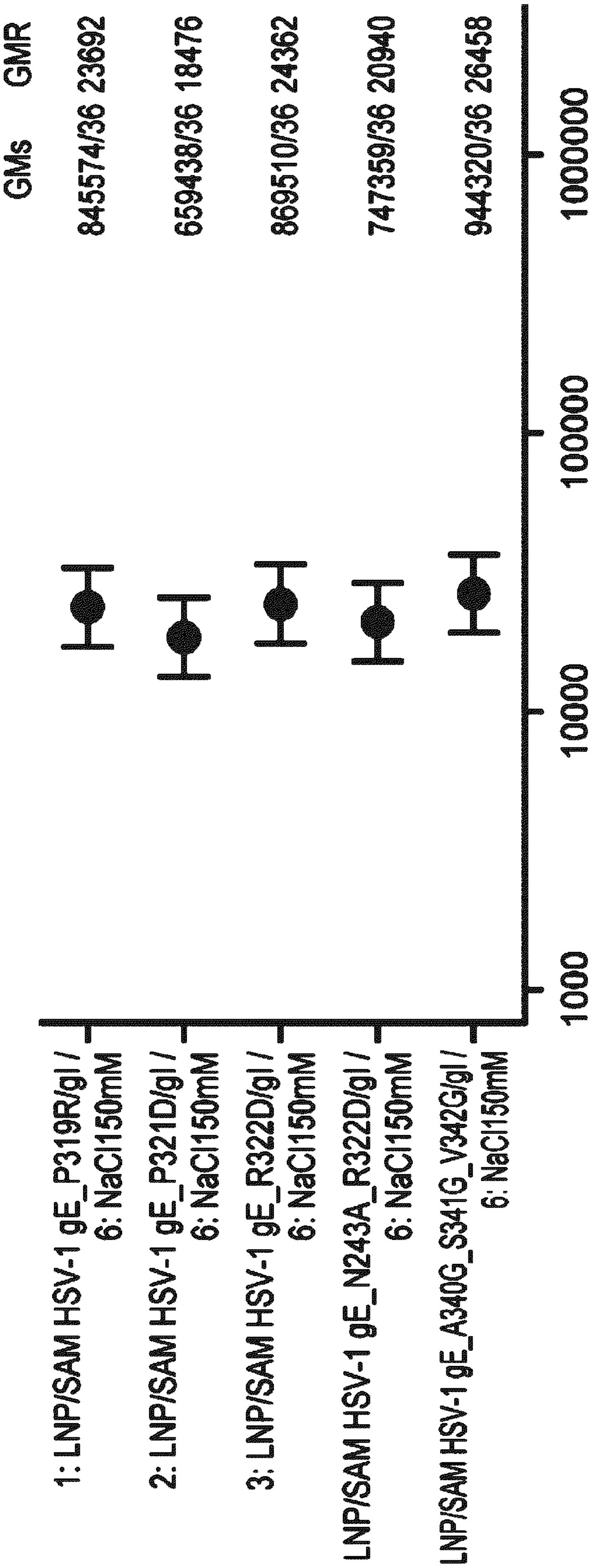

FIG. 55

GMR with 95% CIs of Total anti-HSV-1 gE/gI-specific IgG antibody titers (EU/mL)
Group comparisons of PII over PI

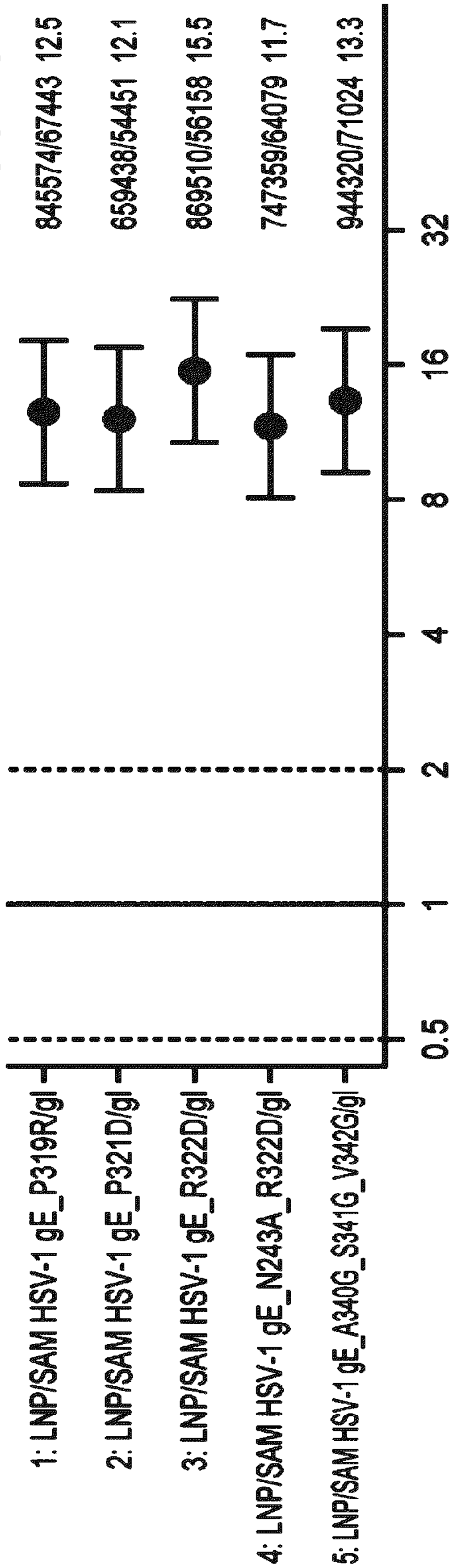

**FIG. 56** GMR with 95% CIs of Total anti-HSV-1 gE/gI-specific IgG antibody titers (EU/mL)
Head to head comparison of LNP/SAM-gE/gI HSV-1 - 21PII(D49)

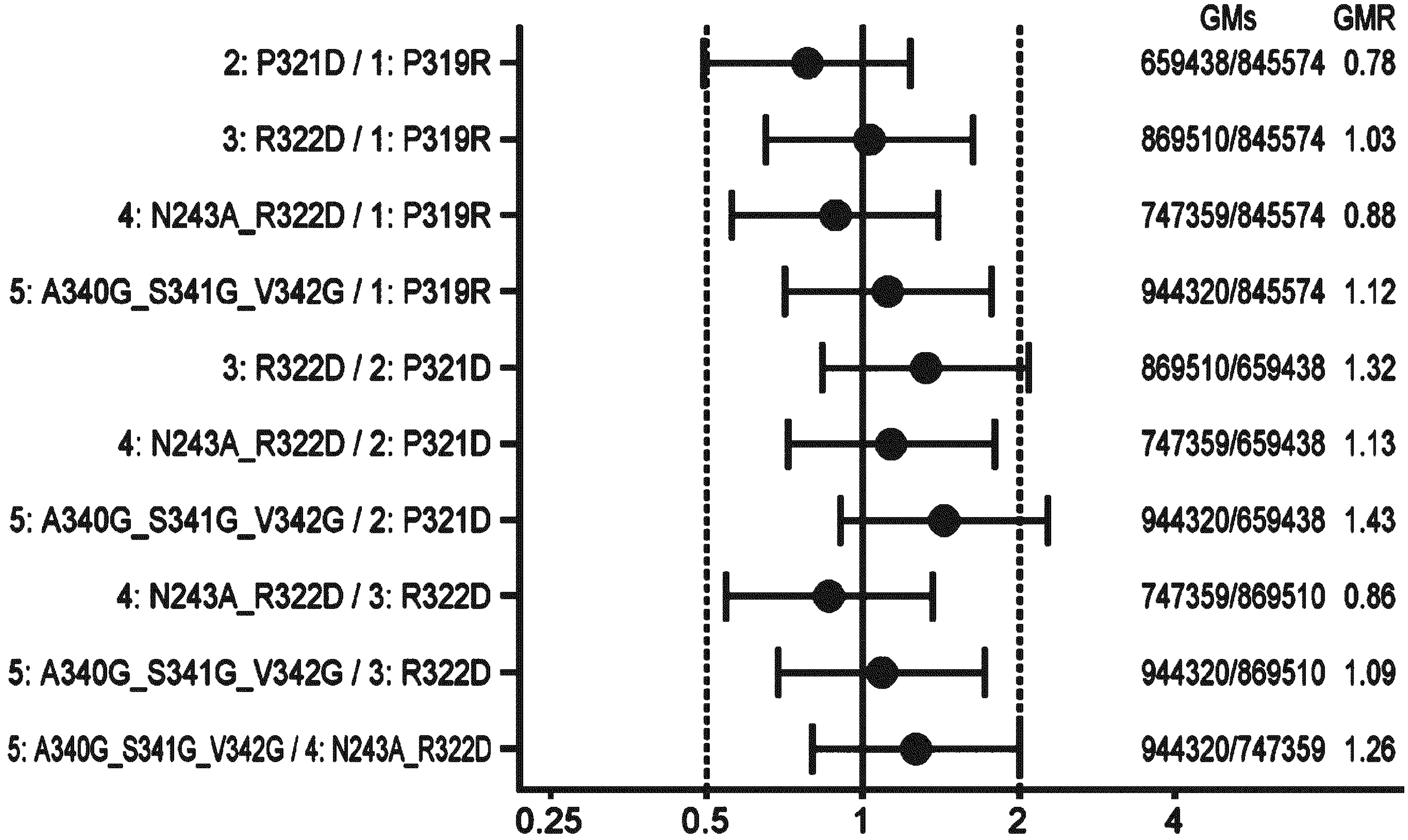

FIG. 57A

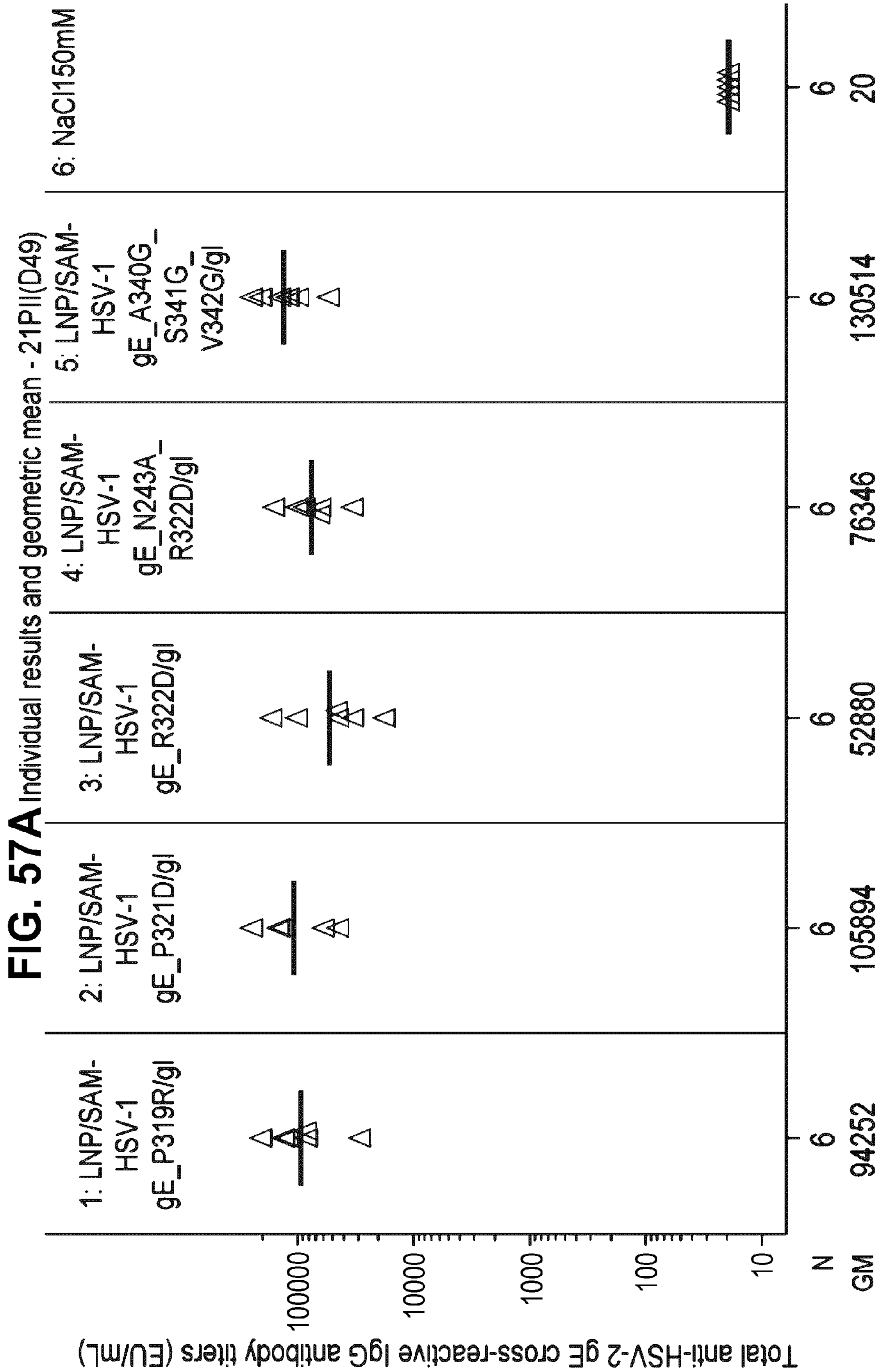
Individual results and geometric mean - 21PII(D49)
1: LNP/SAM-HSV-1 gE_P319R/gI
2: LNP/SAM-HSV-1 gE_P321D/gI
3: LNP/SAM-HSV-1 gE_R322D/gI
4: LNP/SAM-HSV-1 gE_N243A_R322D/gI
5: LNP/SAM-HSV-1 gE_A340G_S341G_V342G/gI
6: NaCl150mM
Total anti-HSV-2 gE cross-reactive IgG antibody titers (EU/mL)
100000
10000
1000
100
10
N
GM
6
94252
6
105894
6
52880
6
76346
6
130514
6
20

**FIG. 57B** Individual results and geometric mean - 21PII(D49)

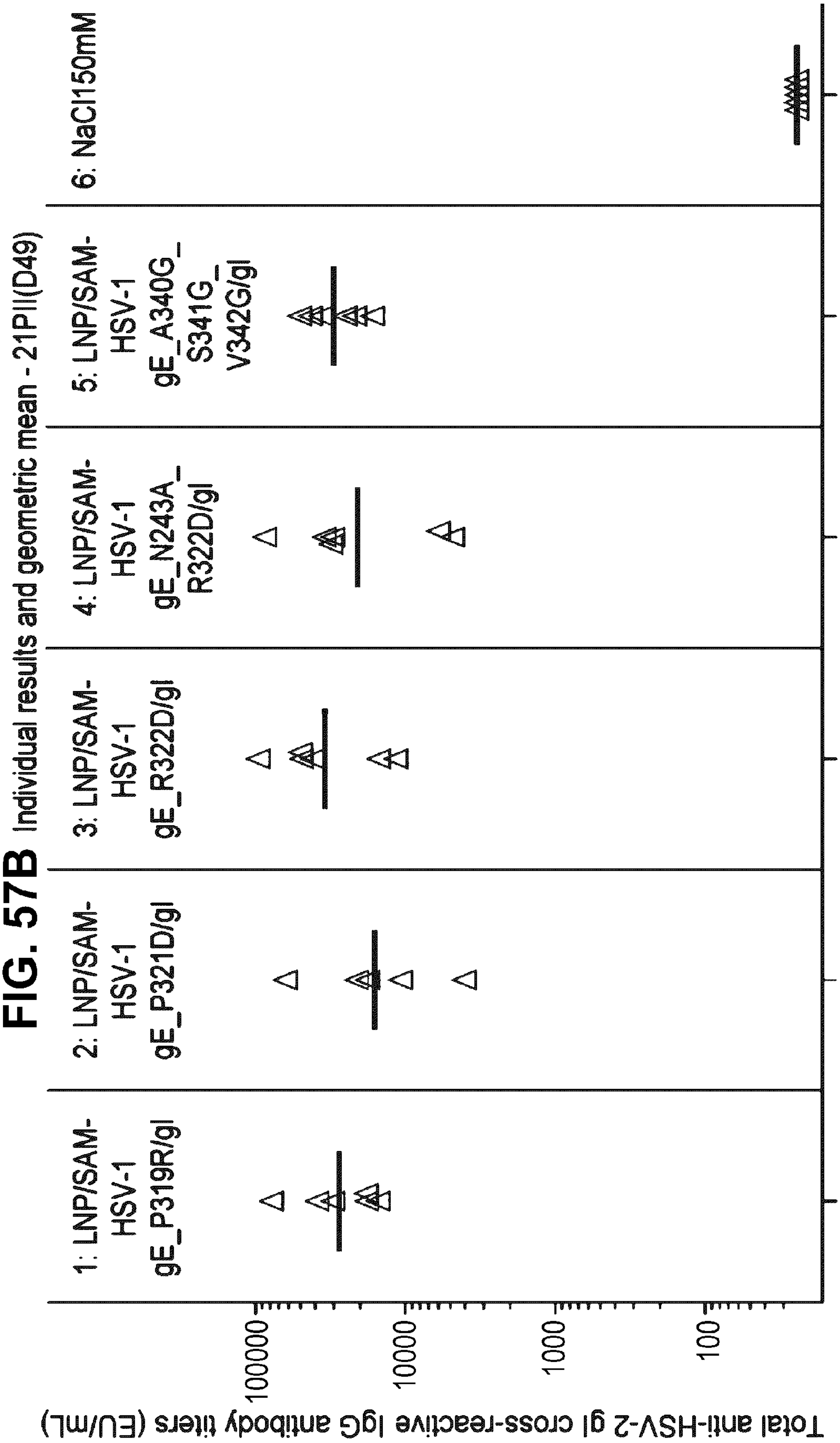

# FIG. 58A

GMR with 95% CIs of Total anti-HSV-2 gE cross-reactive IgG antibody titers (EU/mL) over NaCl - 21PII(D49)

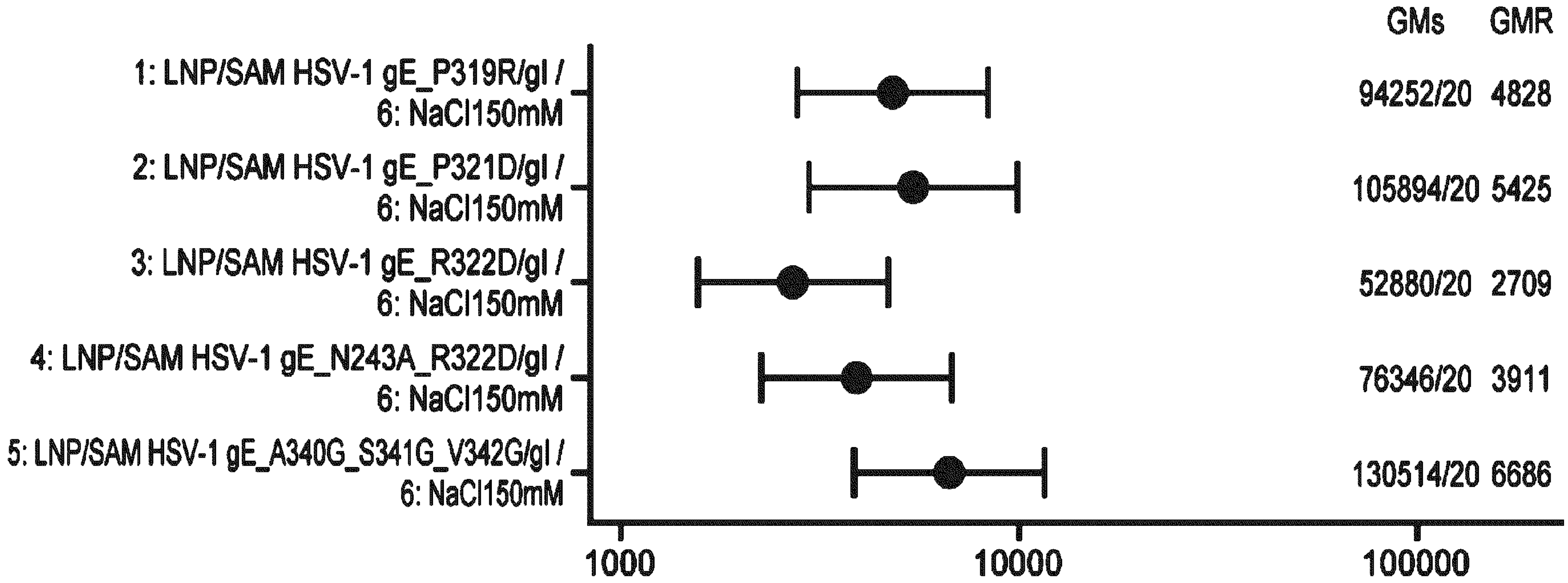

# FIG. 58B

GMR with 95% CIs of Total anti-HSV-2 gI cross-reactive IgG antibody titers (EU/mL) over NaCl - 21PII(D49)

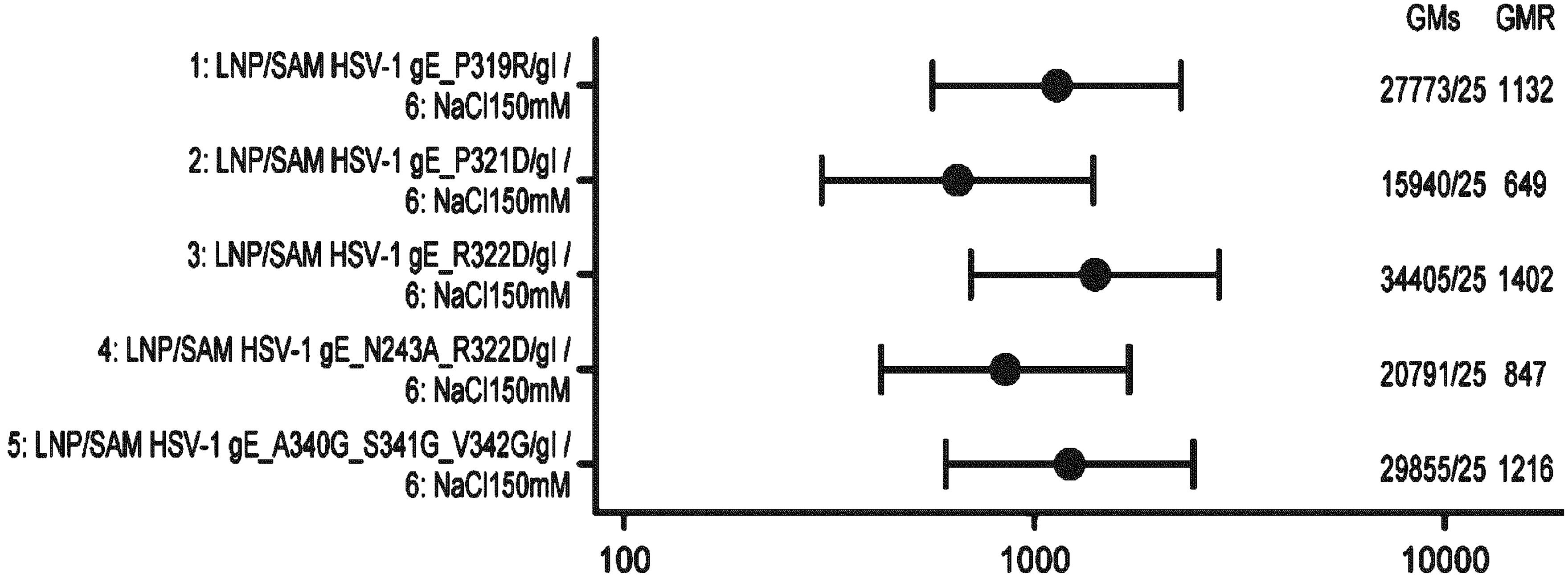

FIG. 59A

GMR with 95% CIs of Total anti-HSV-2 gE cross-reactive IgG antibody titers (EU/mL)
Head to head comparison of LNP/SAM-gE/gI HSV-1 - 21PII(D49)

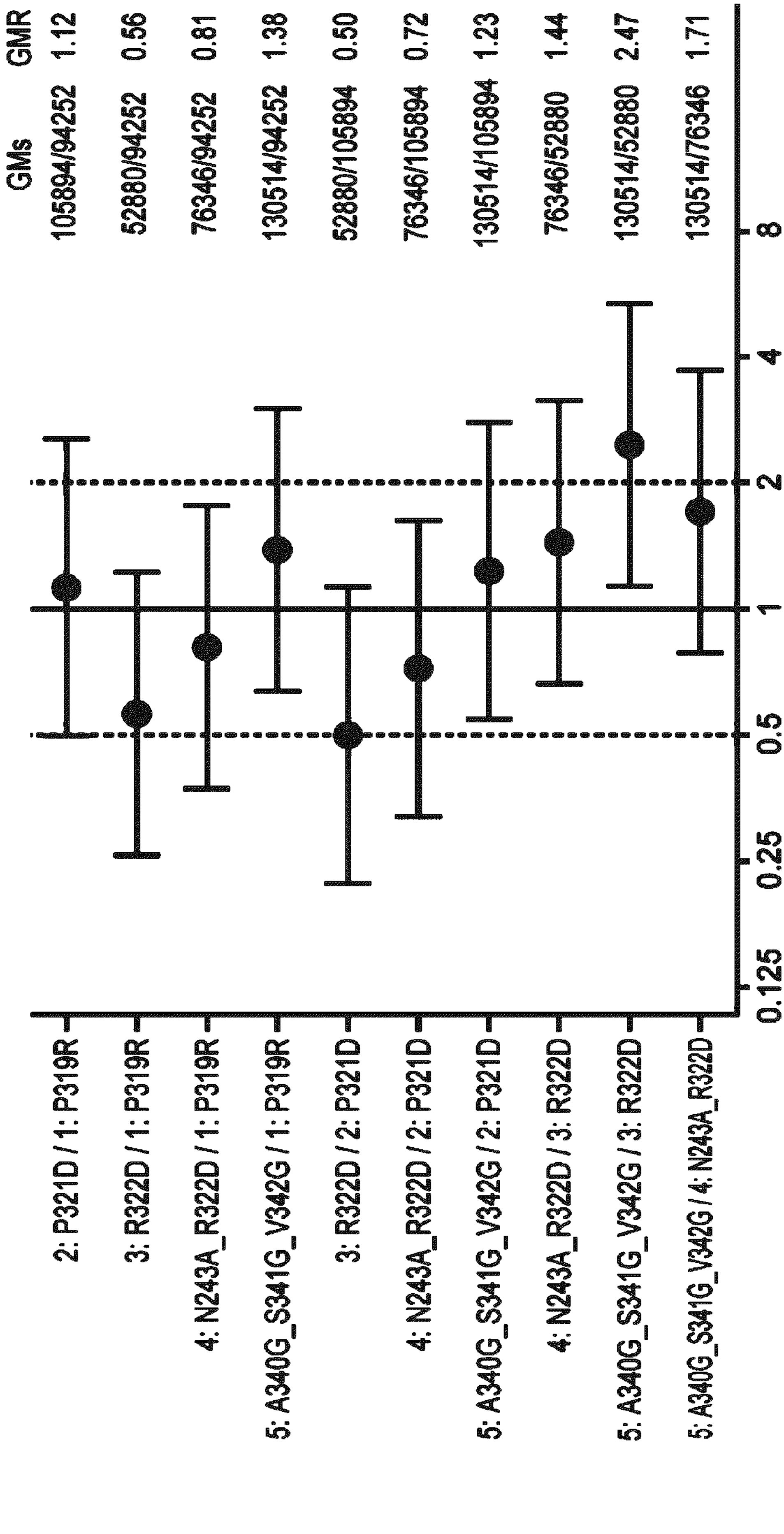

# FIG. 59B

GMR with 95% CIs of Total anti-HSV-2 gI cross-reactive IgG antibody titers (EU/mL)
Head to head comparison of LNP/SAM-gE/gI HSV-1 - 21PII(D49)

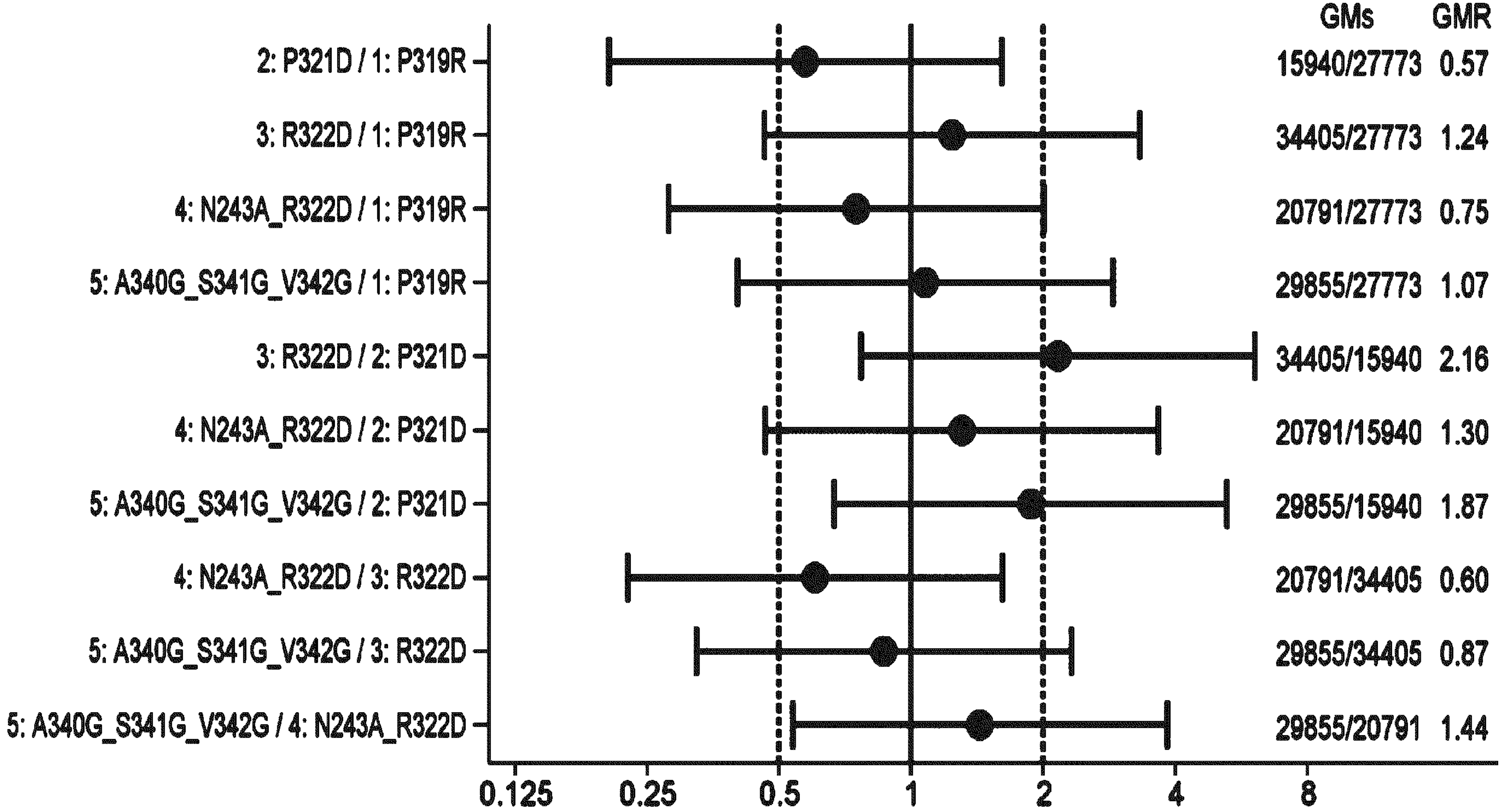

FIG. 60A

HSV-1-specific neutralizing antibody response induced 21days after second immunization with different mutated versions of LNP-formulated HSV-1 gE/gI vector

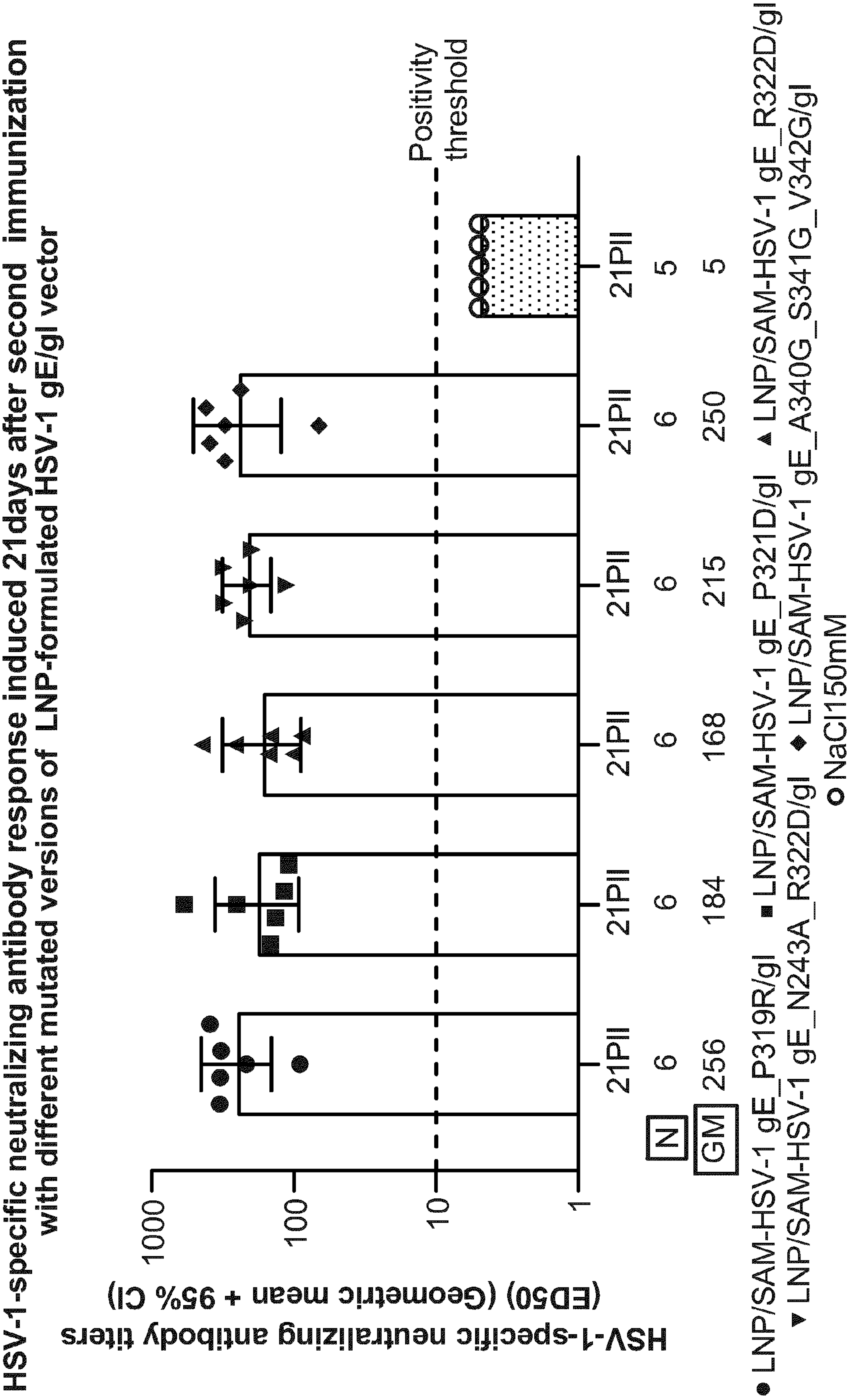

FIG. 60B

GMR with 95% CIs of HSV-1-specific neutralizing antibody titers
Head to head comparison of LNP/SAM-gE/gI HSV-1 - 21PII(D49)

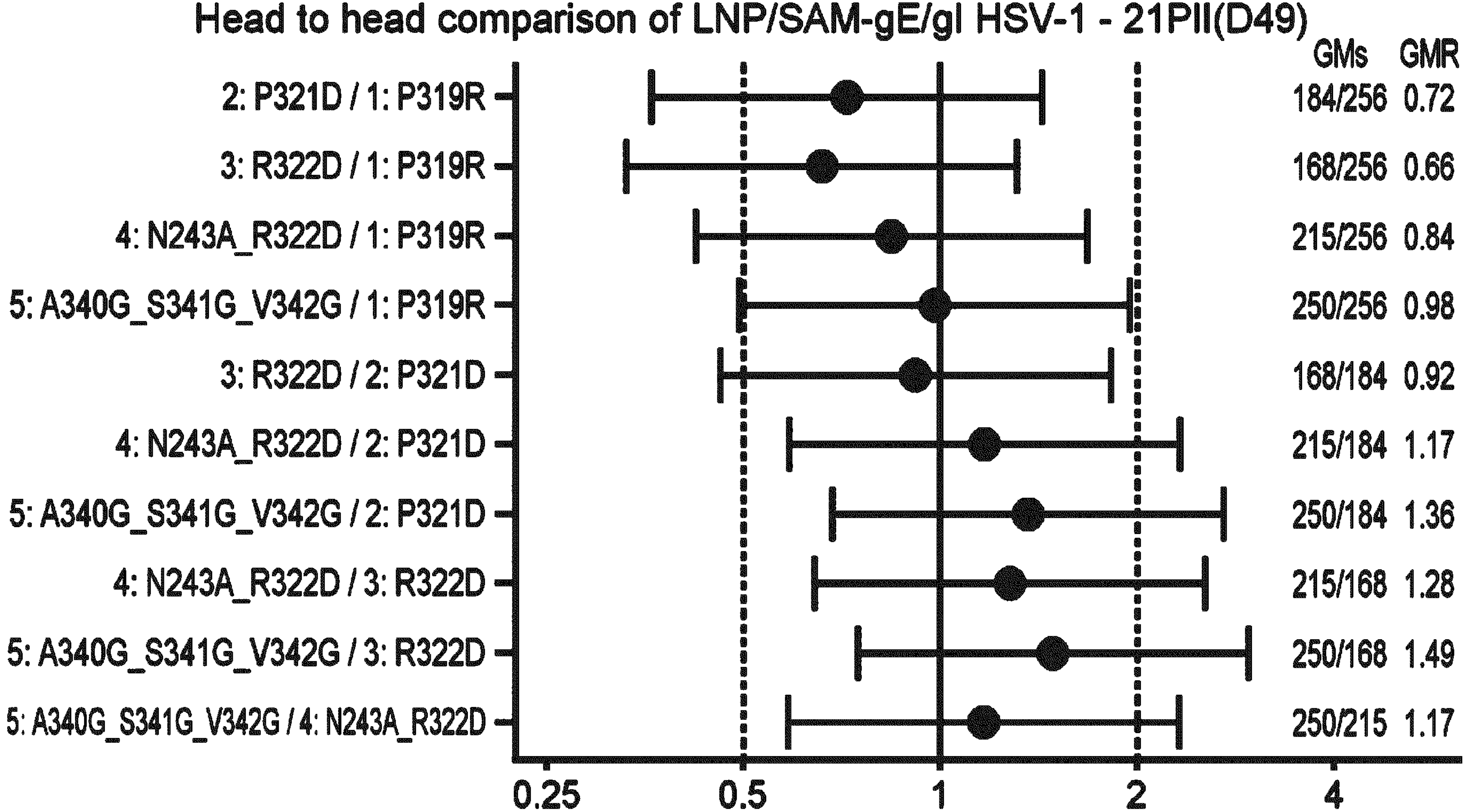

# FIG. 61

**HSV-2 cross-reactive neutralizing antibody response induced 21 days after second immunization with different mutated versions of LNP-formulated HSV-1 gE/gI vector**

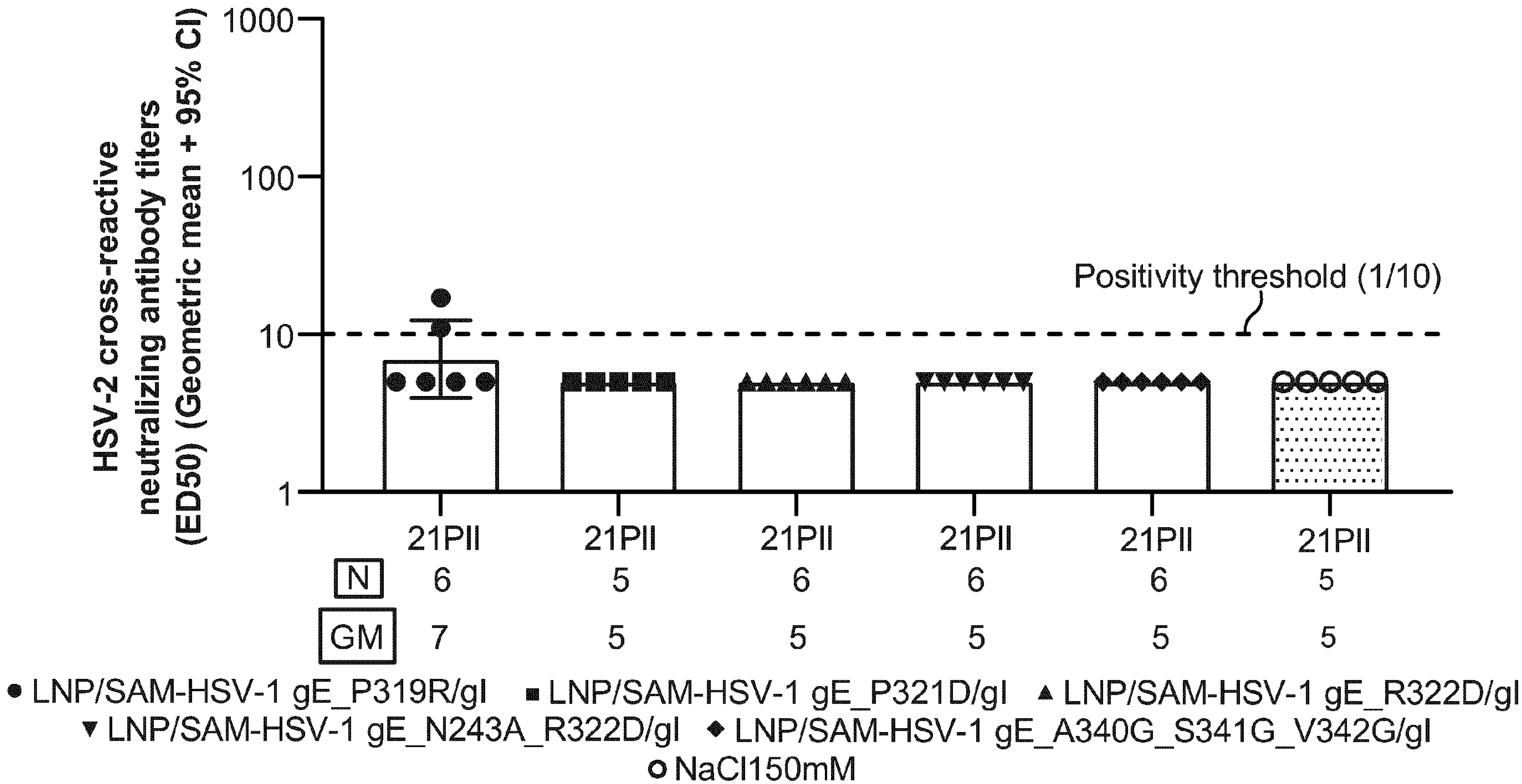

# FIG. 62A

**Co-incubation of hIgG antibodies with serum samples collected 21 days post second immunization with LNP-formulated SAM- HSV-1 gE_P319R/gI mutant candidate**

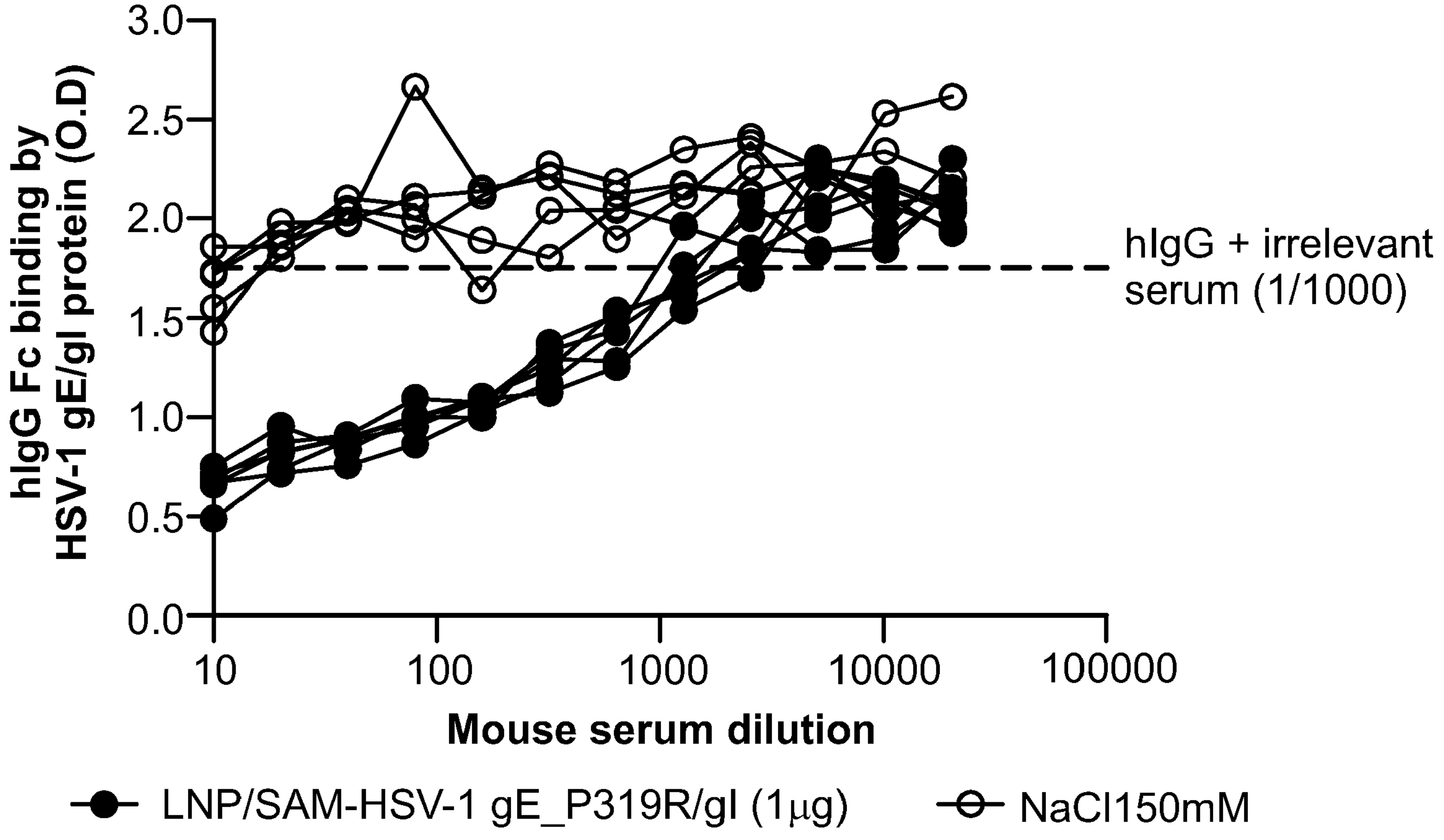

# FIG. 62B

**Co-incubation of hIgG antibodies with serum samples collected 21 days post second immunization with LNP-formulated SAM- HSV-1 gE_P321D/gI mutant candidate**

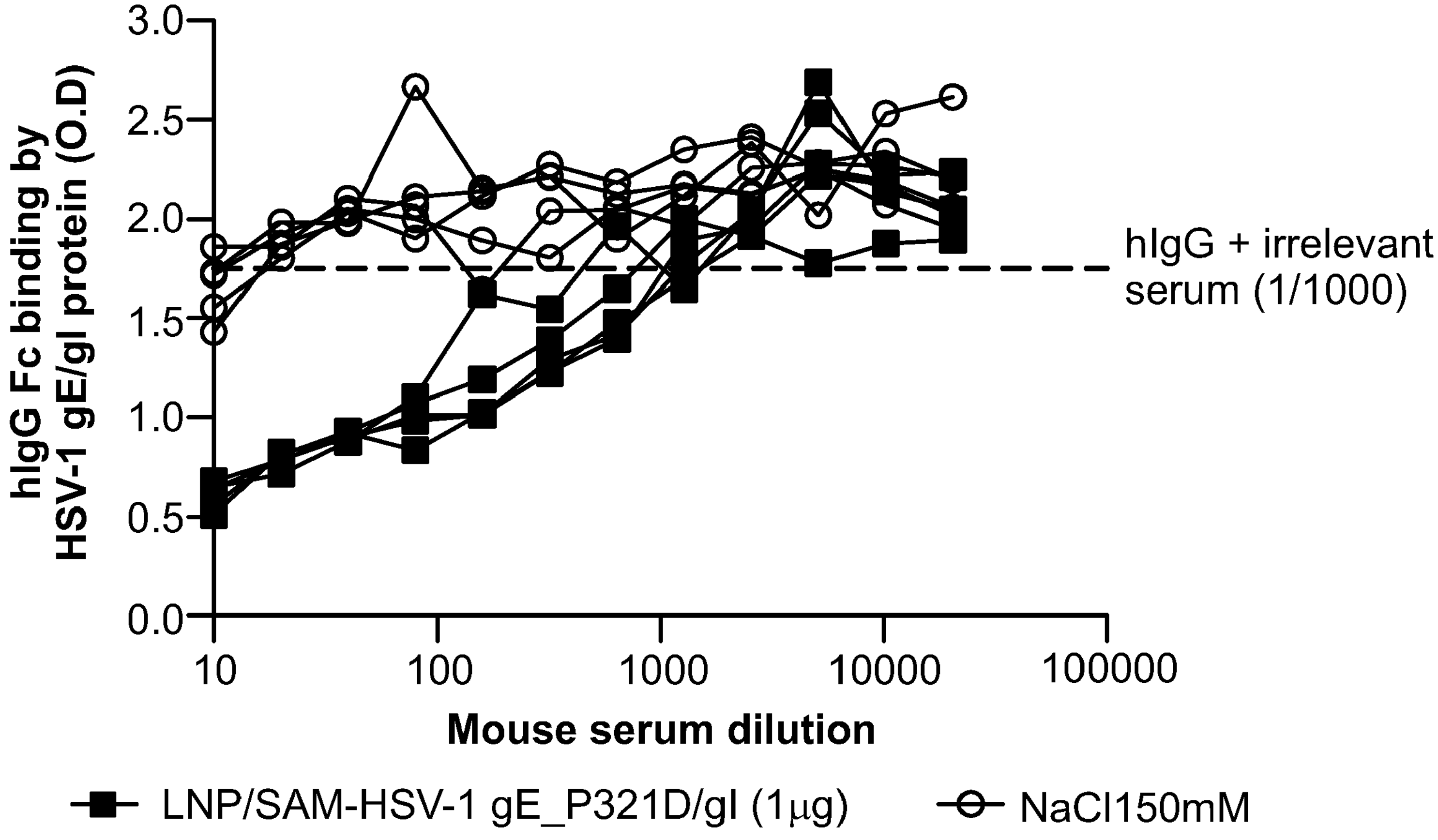

# FIG. 62C

**Co-incubation of hIgG antibodies with serum samples collected 21 days post second immunization with LNP-formulated SAM- HSV-1 gE_R322D/gI mutant candidate**

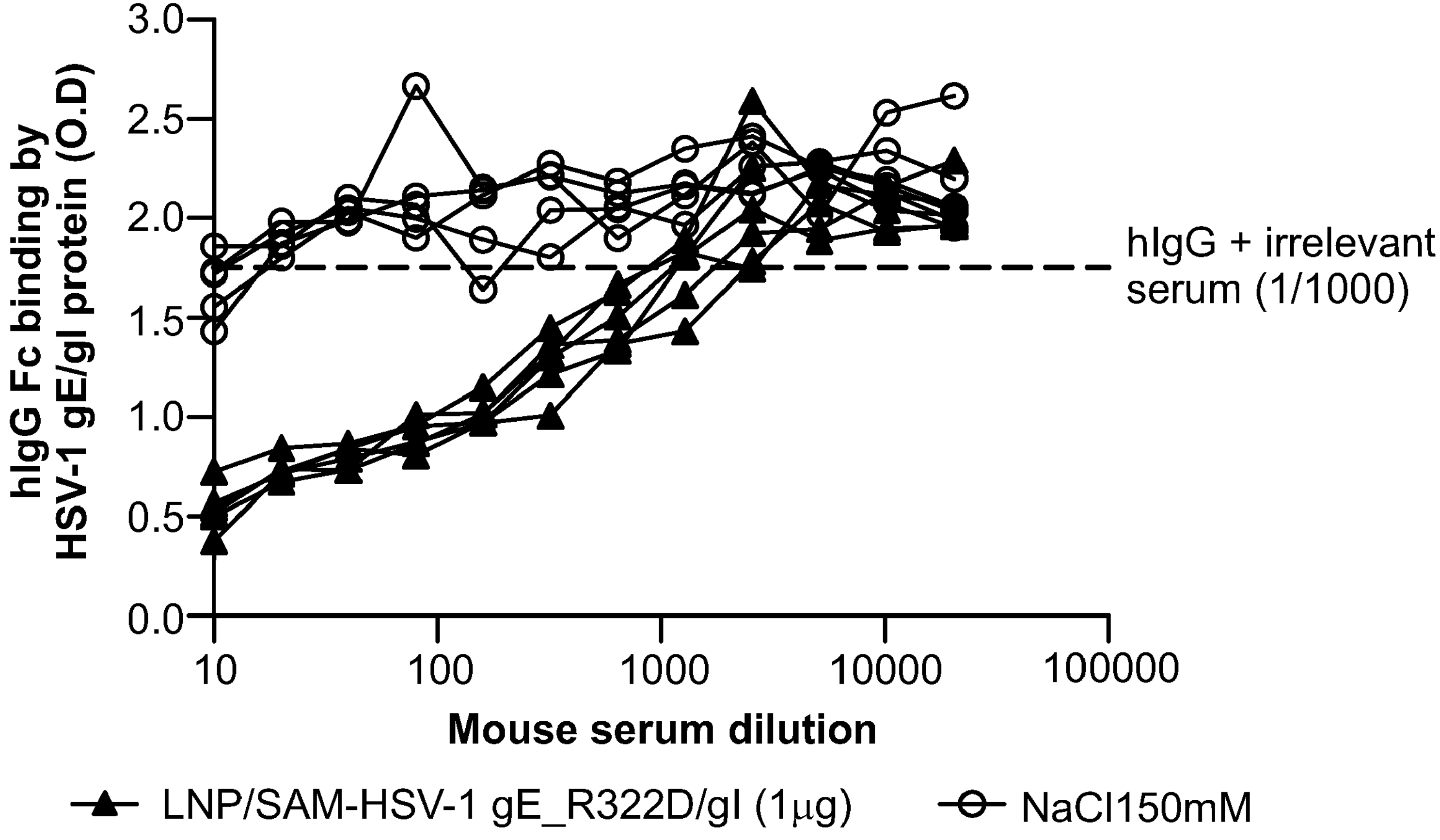

# FIG. 62D

**Co-incubation of hIgG antibodies with serum samples collected 21 days post second immunization with LNP-formulated SAM-HSV-1 gE_N243A_R322D/gI mutant candidate**

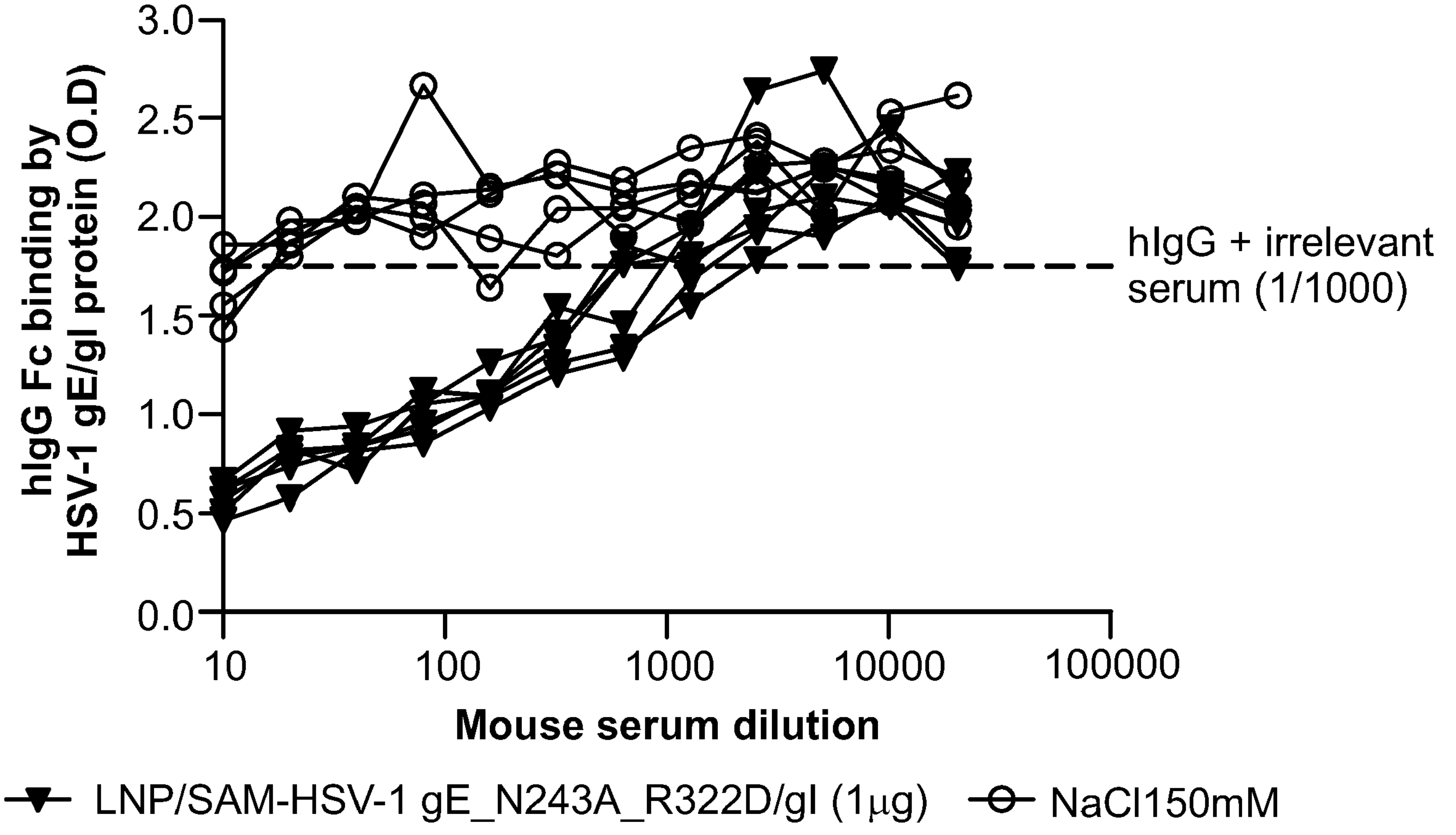

# FIG. 62E

**Co-incubation of hIgG antibodies with serum samples collected 21 days post second immunization with LNP-formulated SAM-HSV-1 gE_A340G_S341G_V342G/gI mutant candidate**

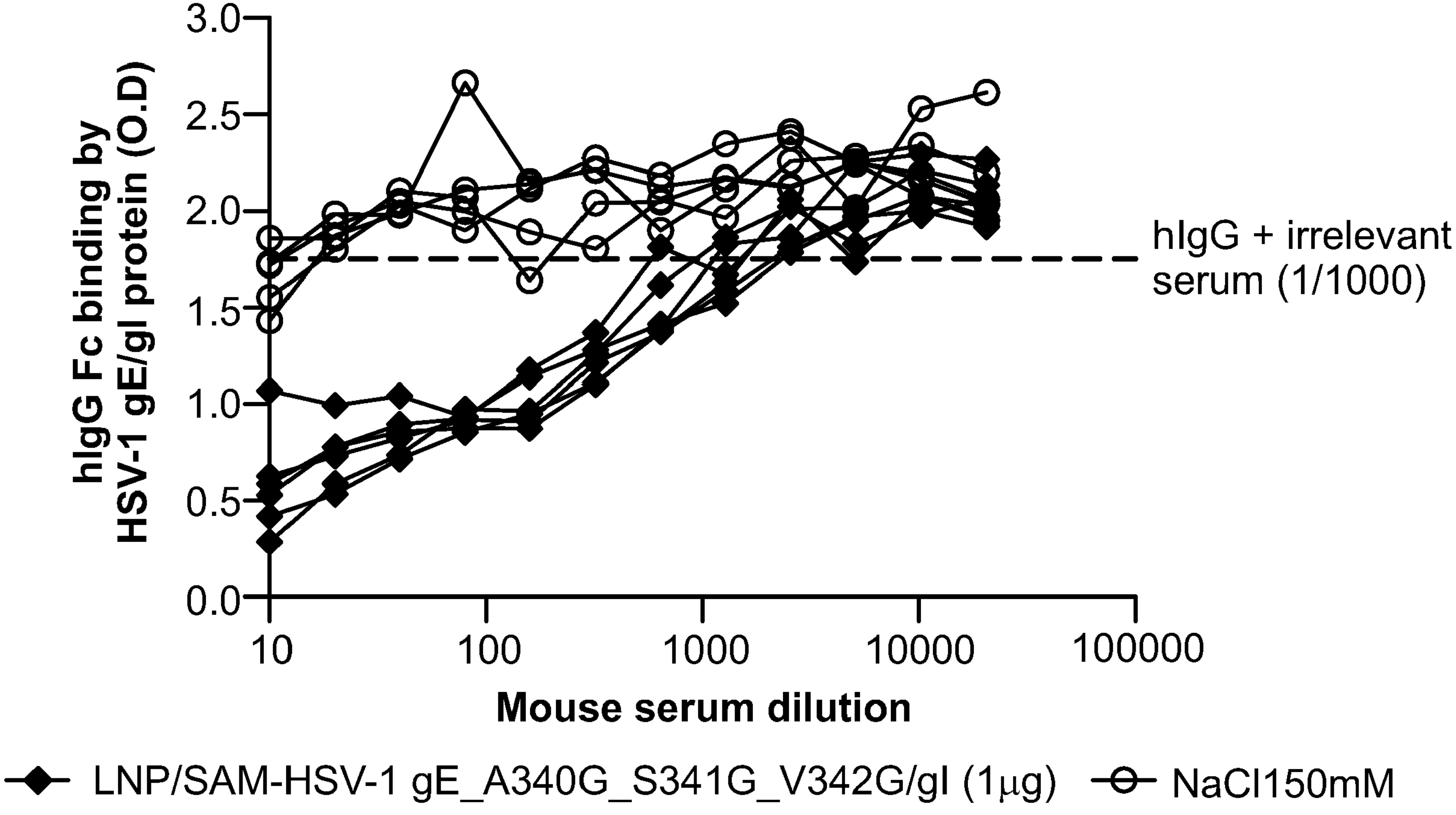

# FIG. 63A

**hIgG Fc binding after co-incubation of hIgG antibodies with serum samples collected 21 days post second immunization**

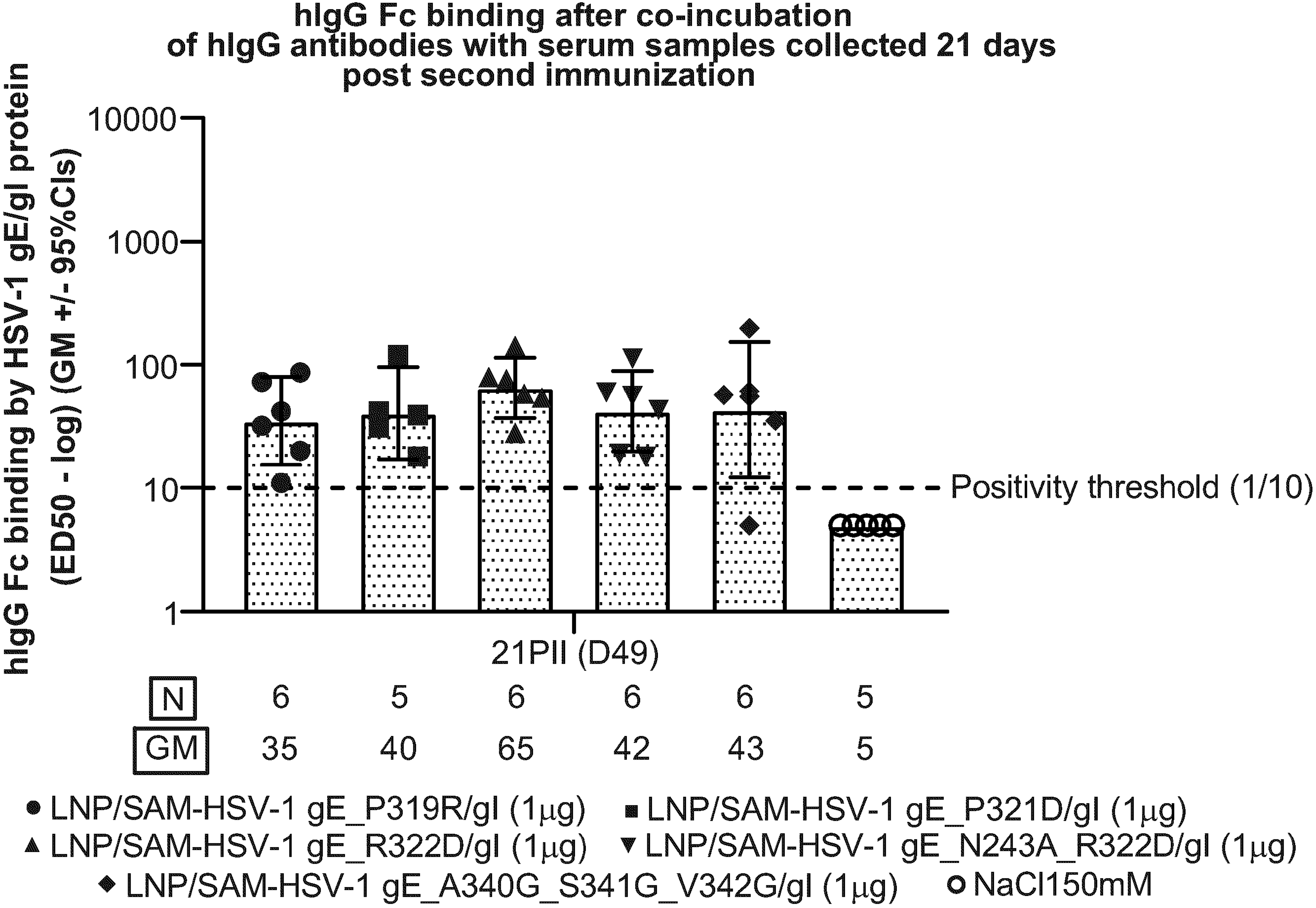

FIG. 63B

GMR with 95% CIs of Inhibition of hIgG Fc binding by HSV-1 gE/gI antigen (ED50)
Head to head comparison of LNP/SAM-gE/gI HSV-1 - 21PII(D49)

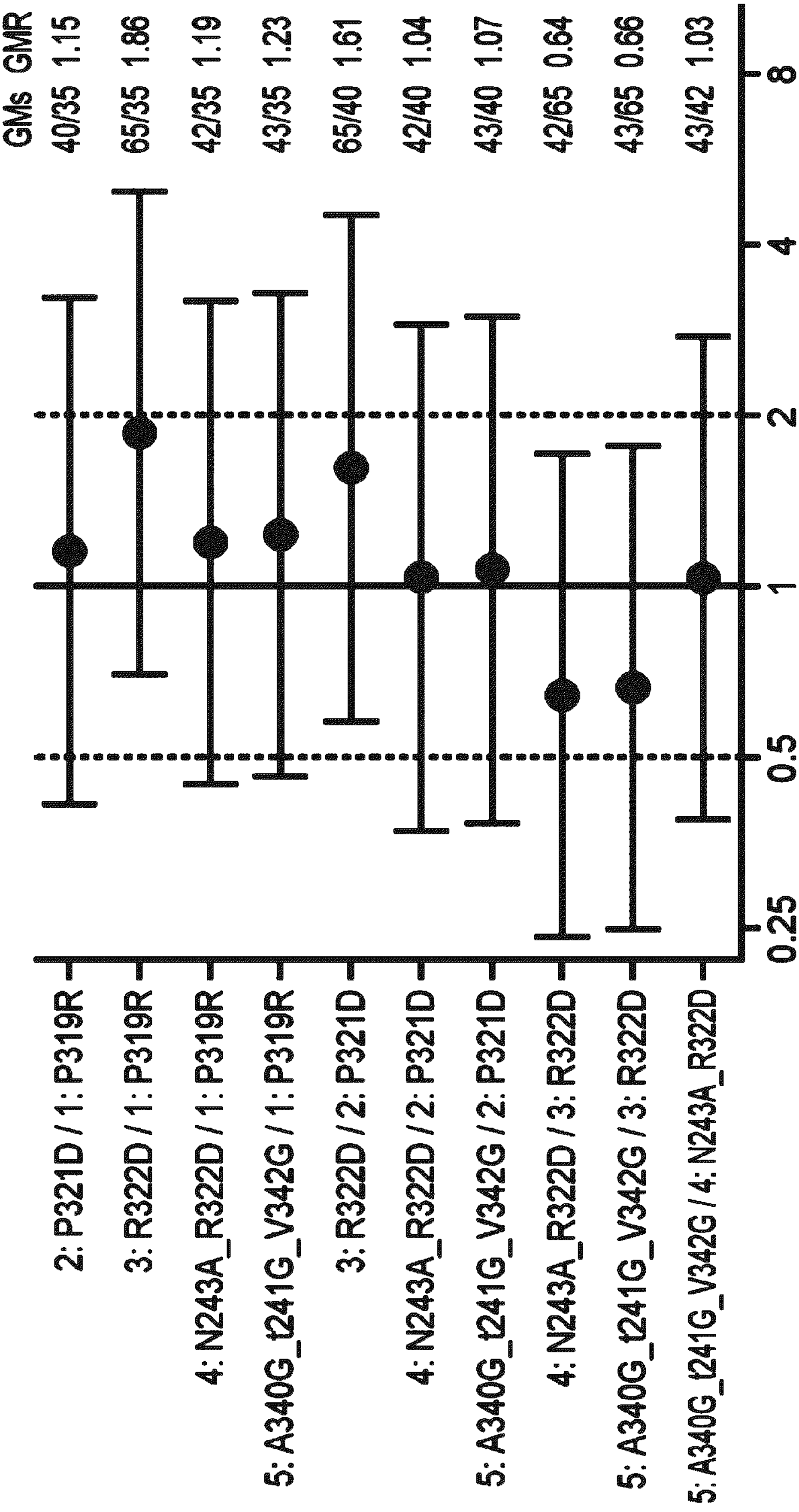

# FIG. 64A

**Co-incubation of hIgG antibodies with serum samples collected 21 days post second immunization with LNP-formulated SAM-HSV-1 gE_P319R/gI mutant candidate**

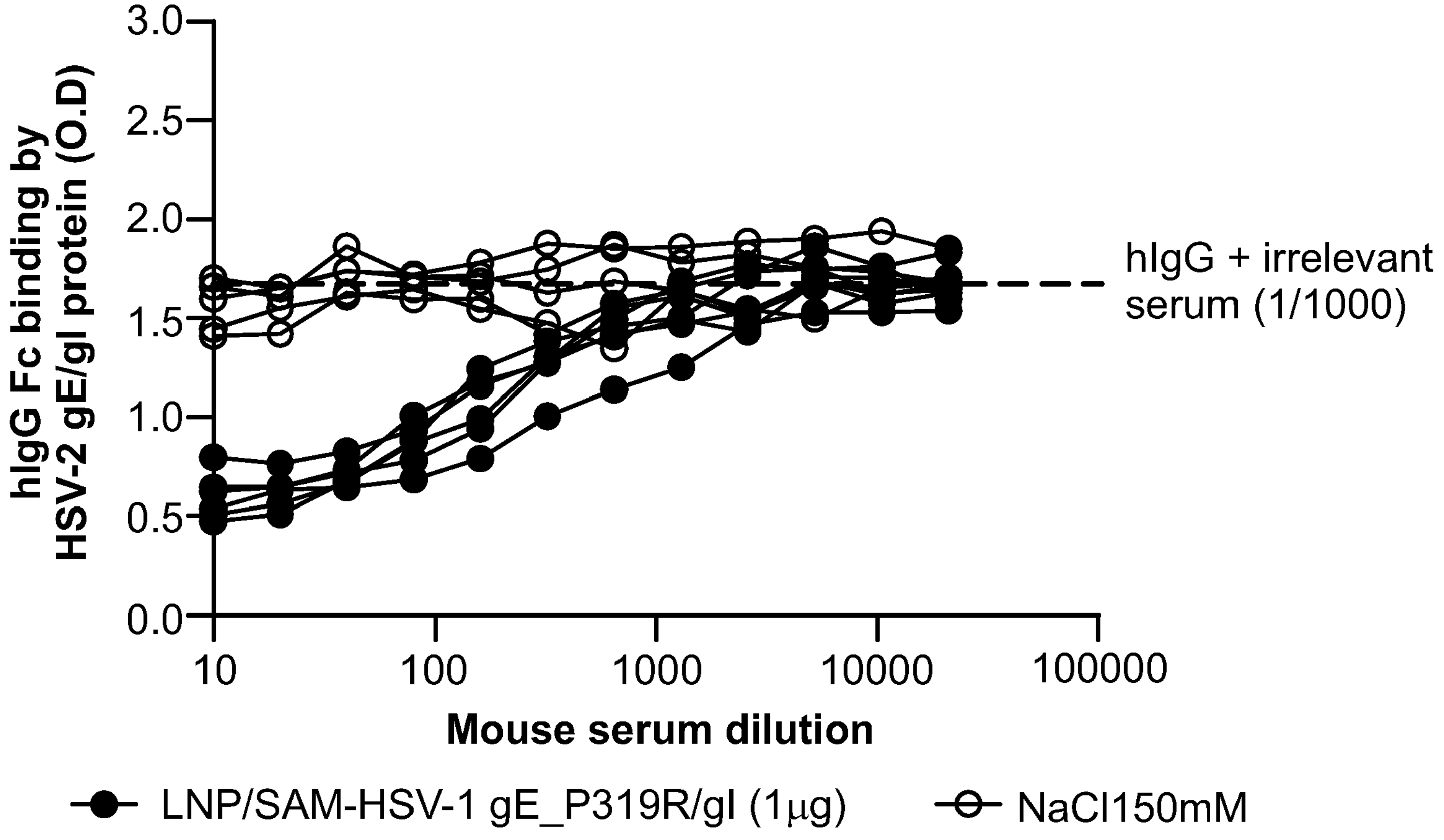

# FIG. 64B

**Co-incubation of hIgG antibodies with serum samples collected 21 days post second immunization with LNP-formulated SAM-HSV-1 gE_P321D/gI mutant candidate**

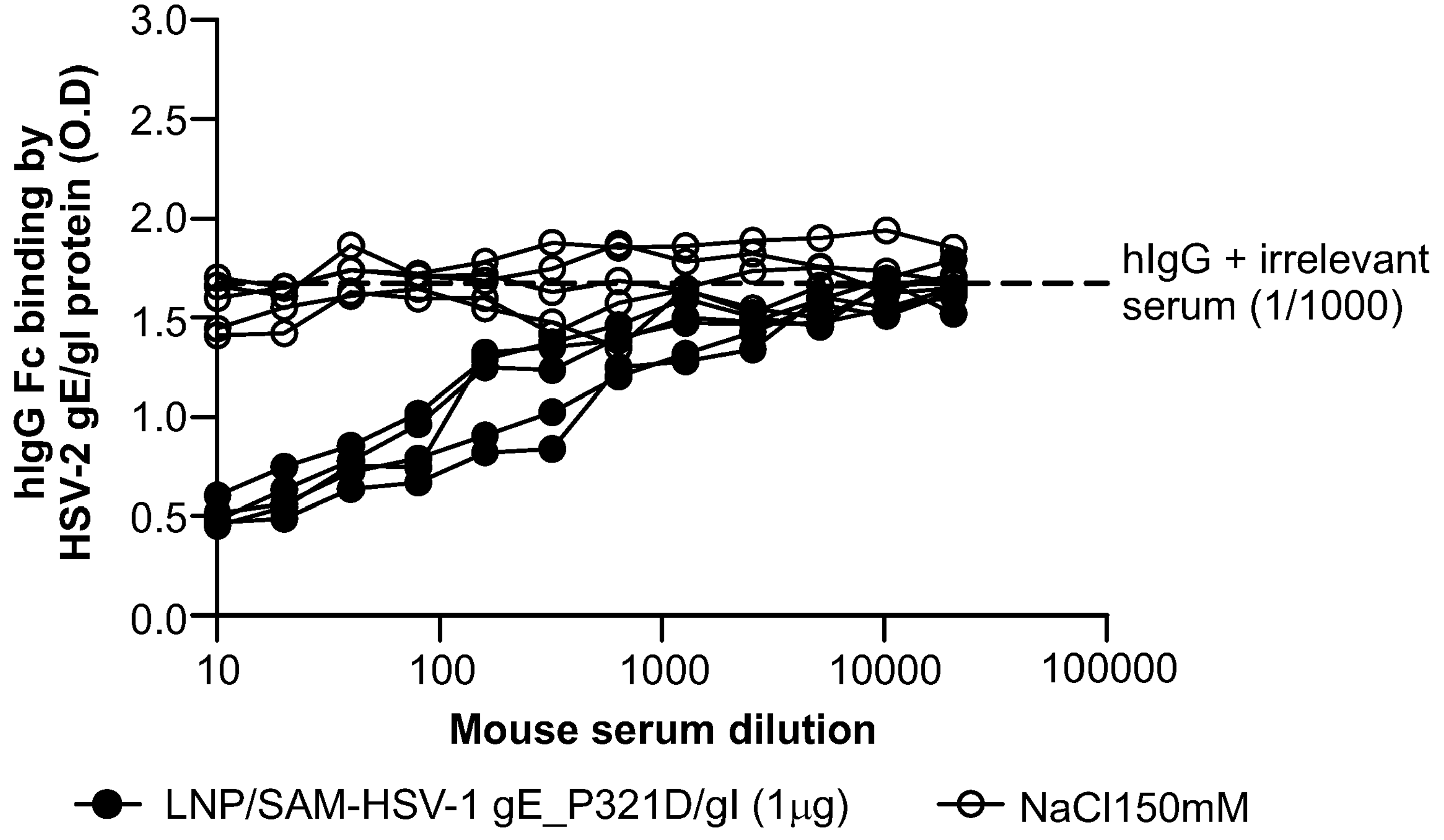

# FIG. 64C

**Co-incubation of hIgG antibodies with serum samples collected 21 days post second immunization with LNP-formulated SAM-HSV-1 gE_R322D/gI mutant candidate**

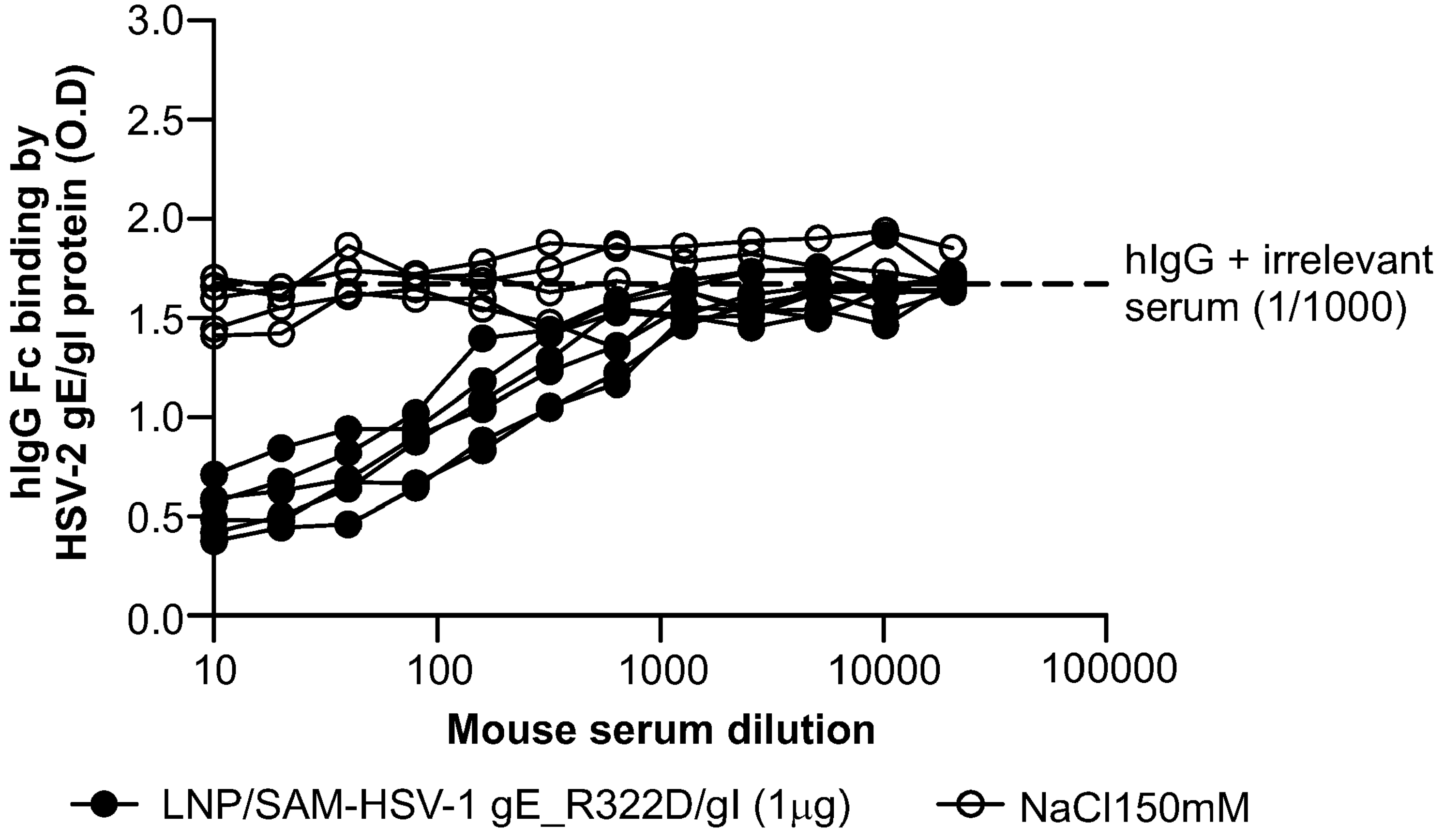

# FIG. 64D

**Co-incubation of hIgG antibodies with serum samples collected 21 days post second immunization with LNP-formulated SAM-HSV-1 gE_N243A_R322D/gI mutant candidate**

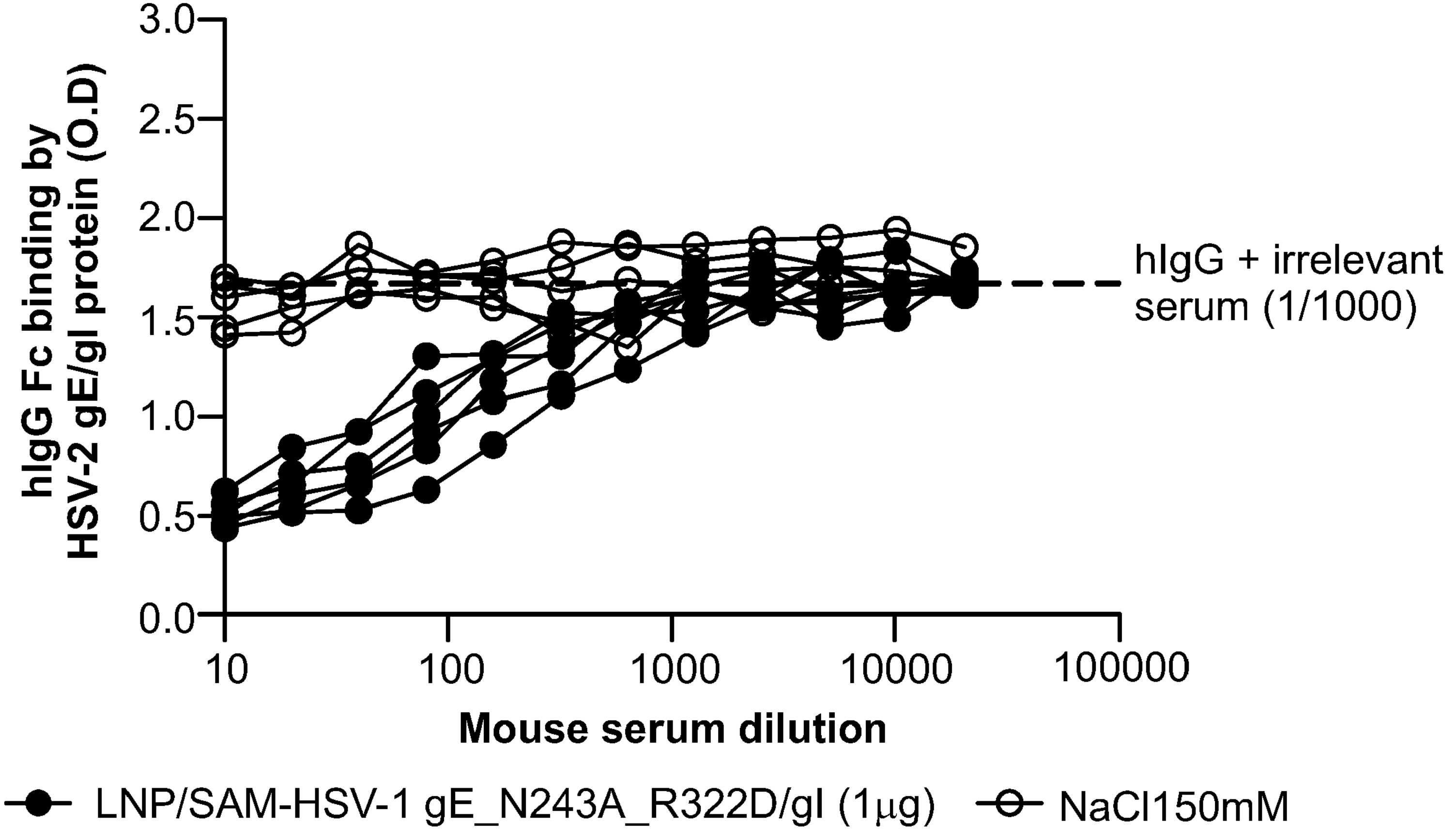

# FIG. 64E

**Co-incubation of hIgG antibodies with serum samples collected 21 days post second immunization with LNP-formulated SAM-HSV-1 gE_A340G_S341G_V342G/gI mutant candidate**

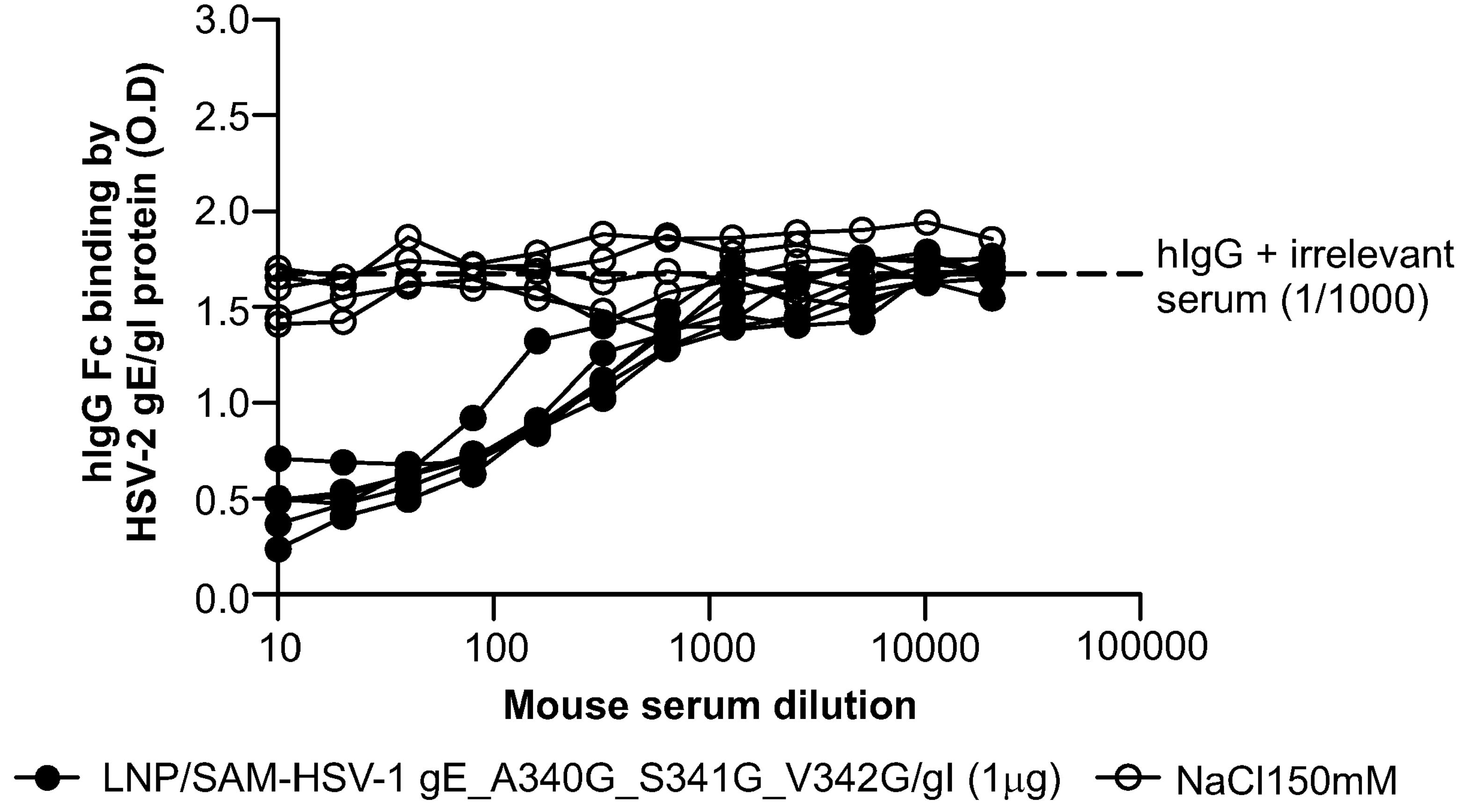

# FIG. 65A

**hIgG Fc binding on HSV-2 gE/gI protein after co-incubation of hIgG antibodies with serum samples collected 21 days post second immunization**

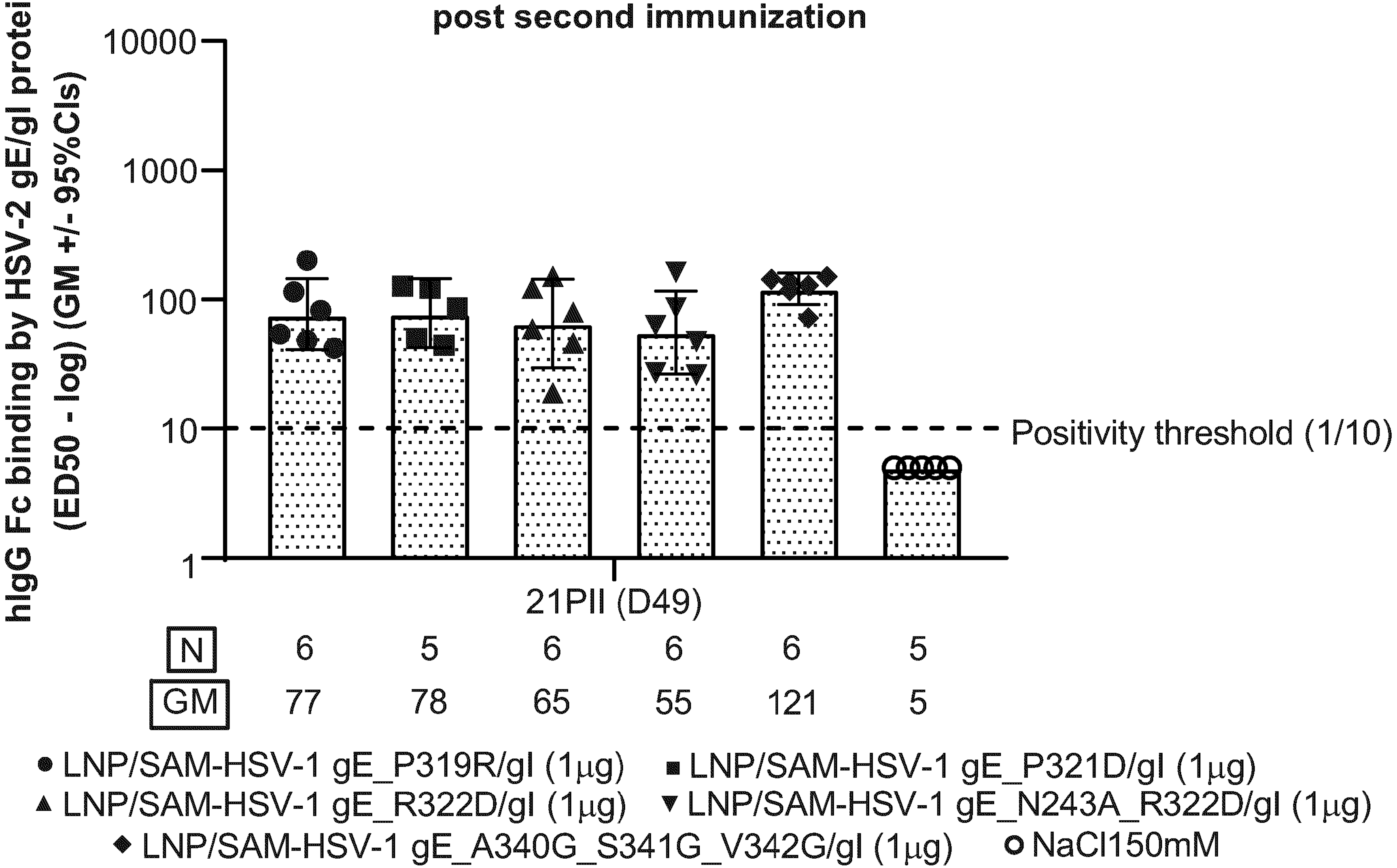

FIG. 65B

GMR with 95% CI of Inhibition of hIgG Fc binding by HSV-2 gE/gI antigen (ED50)
Head to head comparison of LNP/SAM HSV-1 gE/gI groups - 21PII (Day49)

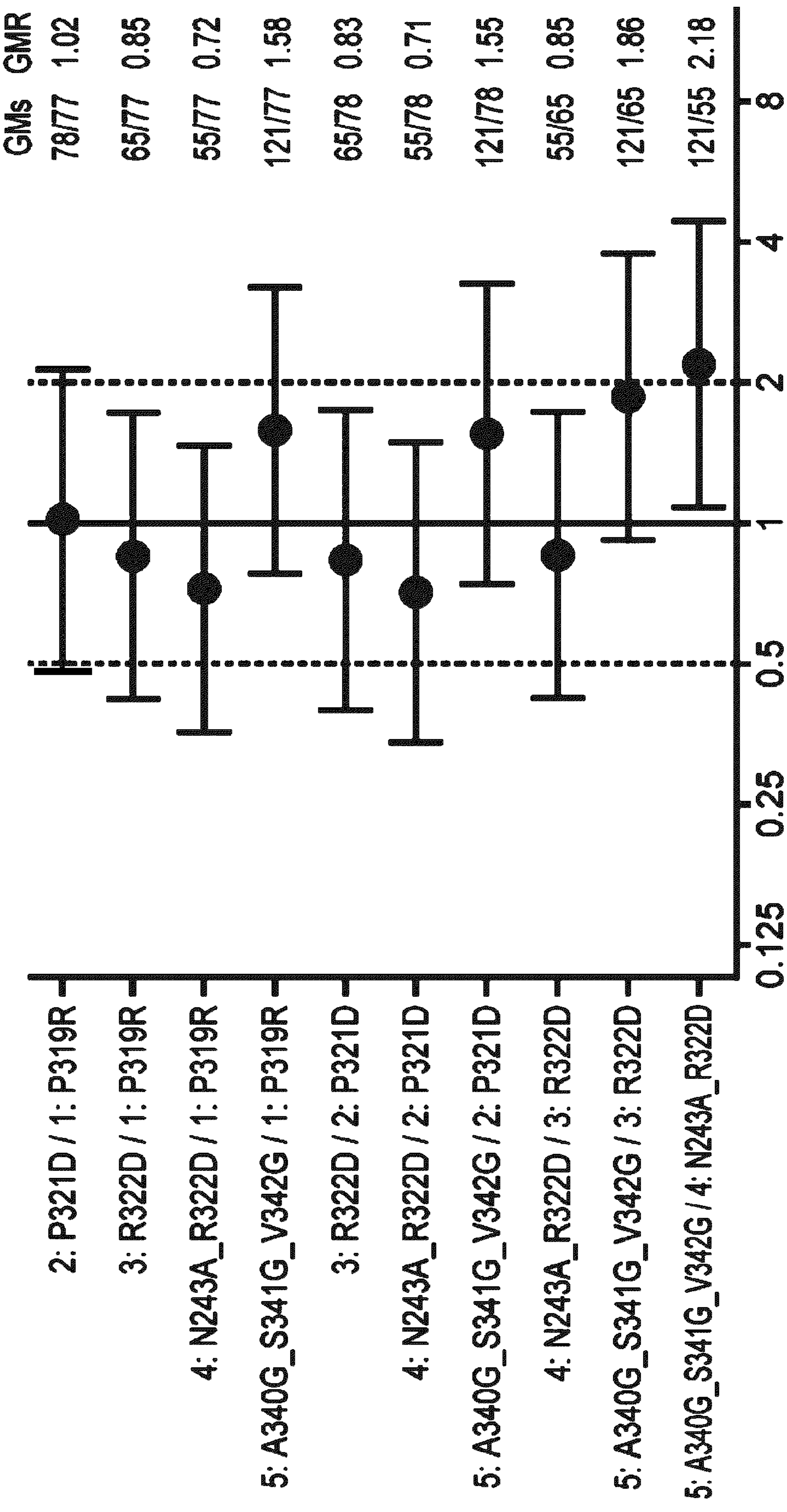

# FIG. 66A

**HSV-2 cross-reactive mFcγRIII binding activity detected in serum samples collected 21 days post second immunization with LNP-formulated SAM-HSV-1 gE_P319R/gI mutant candidate**

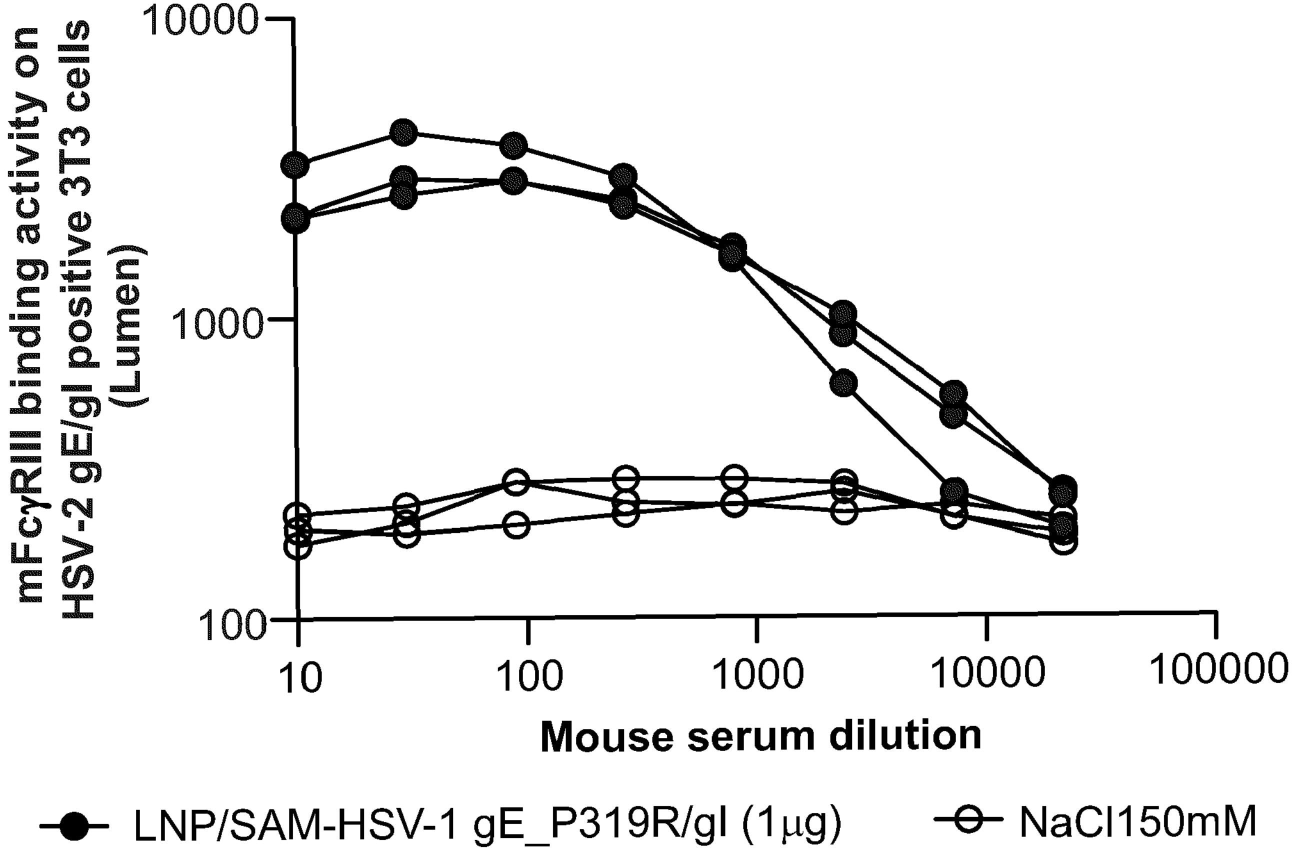

# FIG. 66B

**HSV-2 cross-reactive mFcγRIII binding activity detected in serum samples collected 21 days post second immunization with LNP-formulated SAM-HSV-1 gE_P321D/gI mutant candidate**

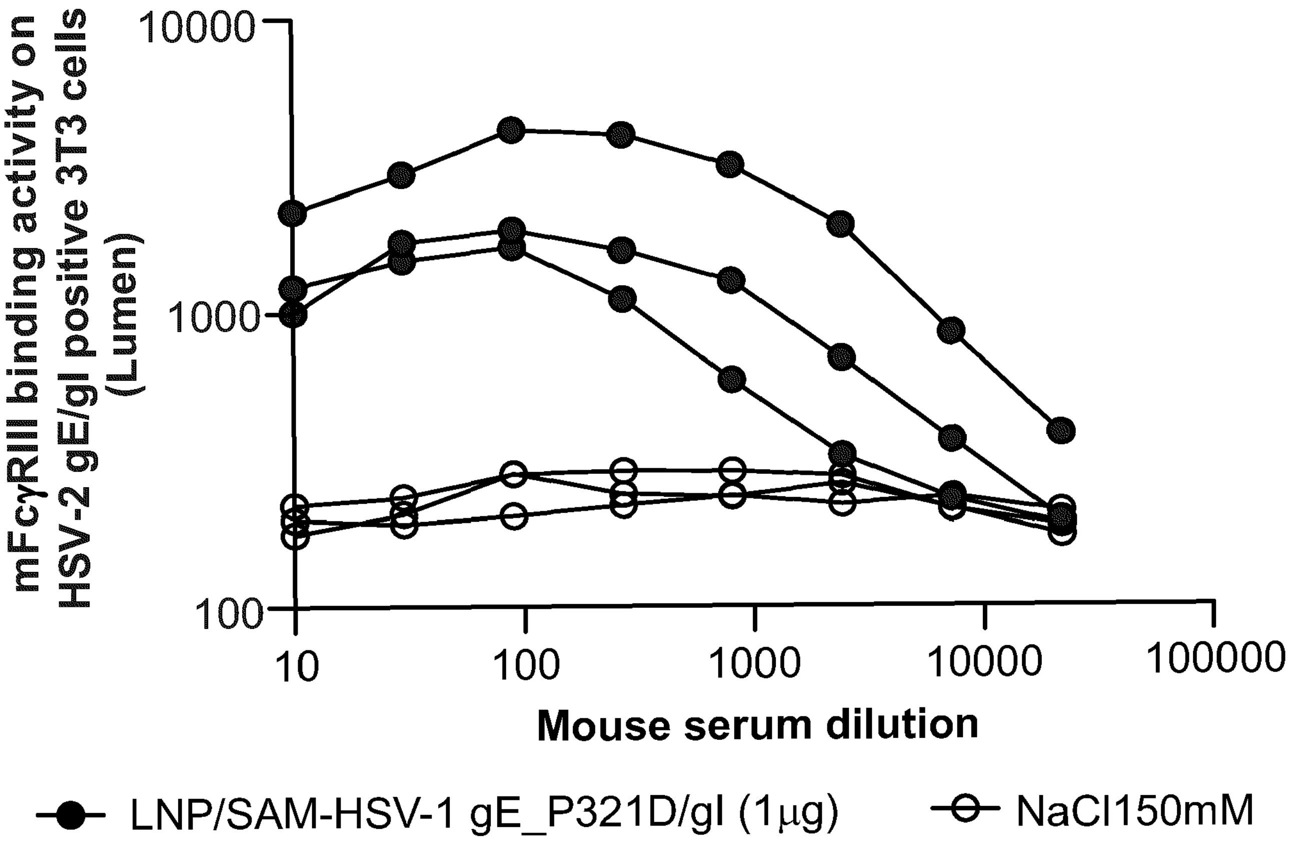

# FIG. 66C

**HSV-2 cross-reactive mFcγRIII binding activity detected in serum samples collected 21 days post second immunization with LNP-formulated SAM-HSV-1 gE_R322D/gI mutant candidate**

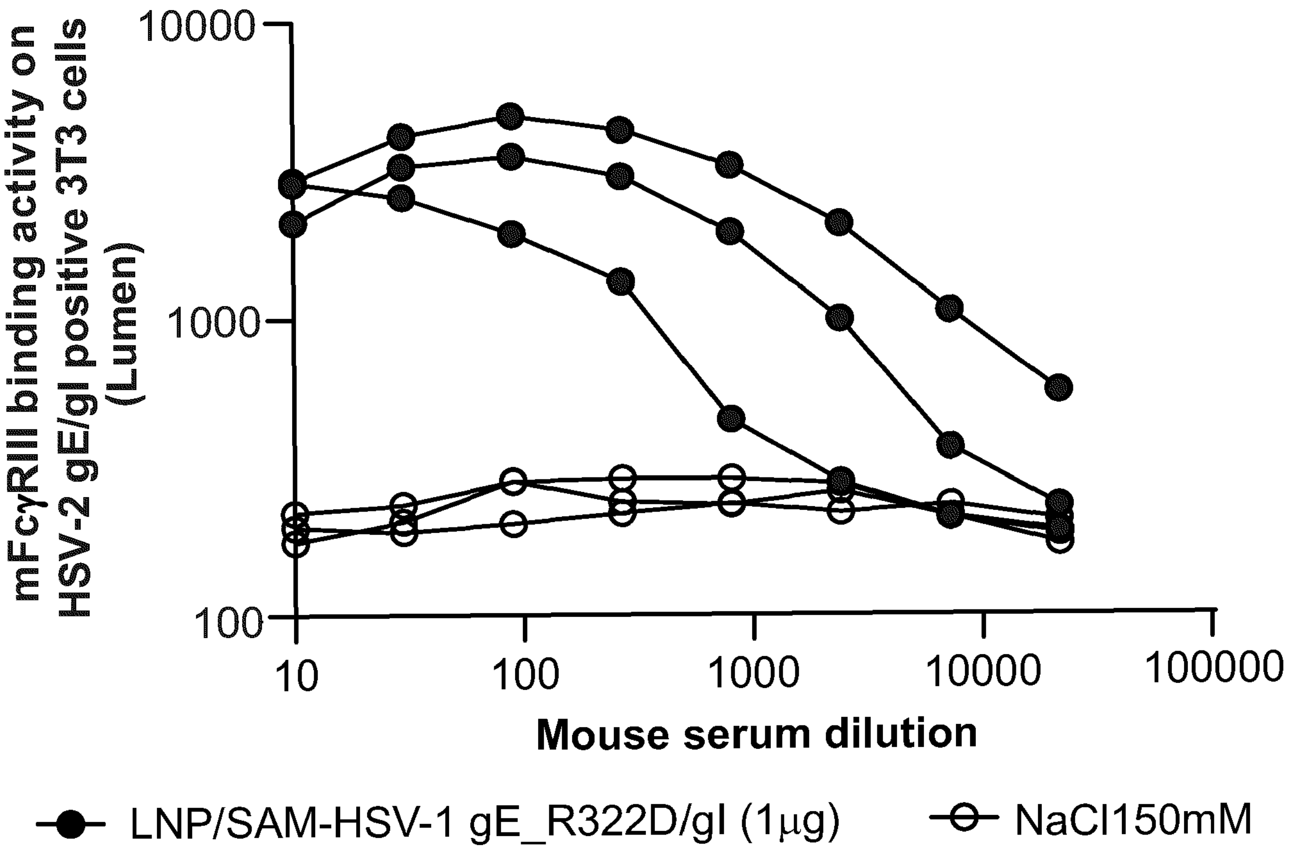

# FIG. 66D

**HSV-2 cross-reactive mFcγRIII binding activity detected in serum samples collected 21 days post second immunization with LNP-formulated SAM-HSV-1 gE_N243A_R322D/gI mutant candidate**

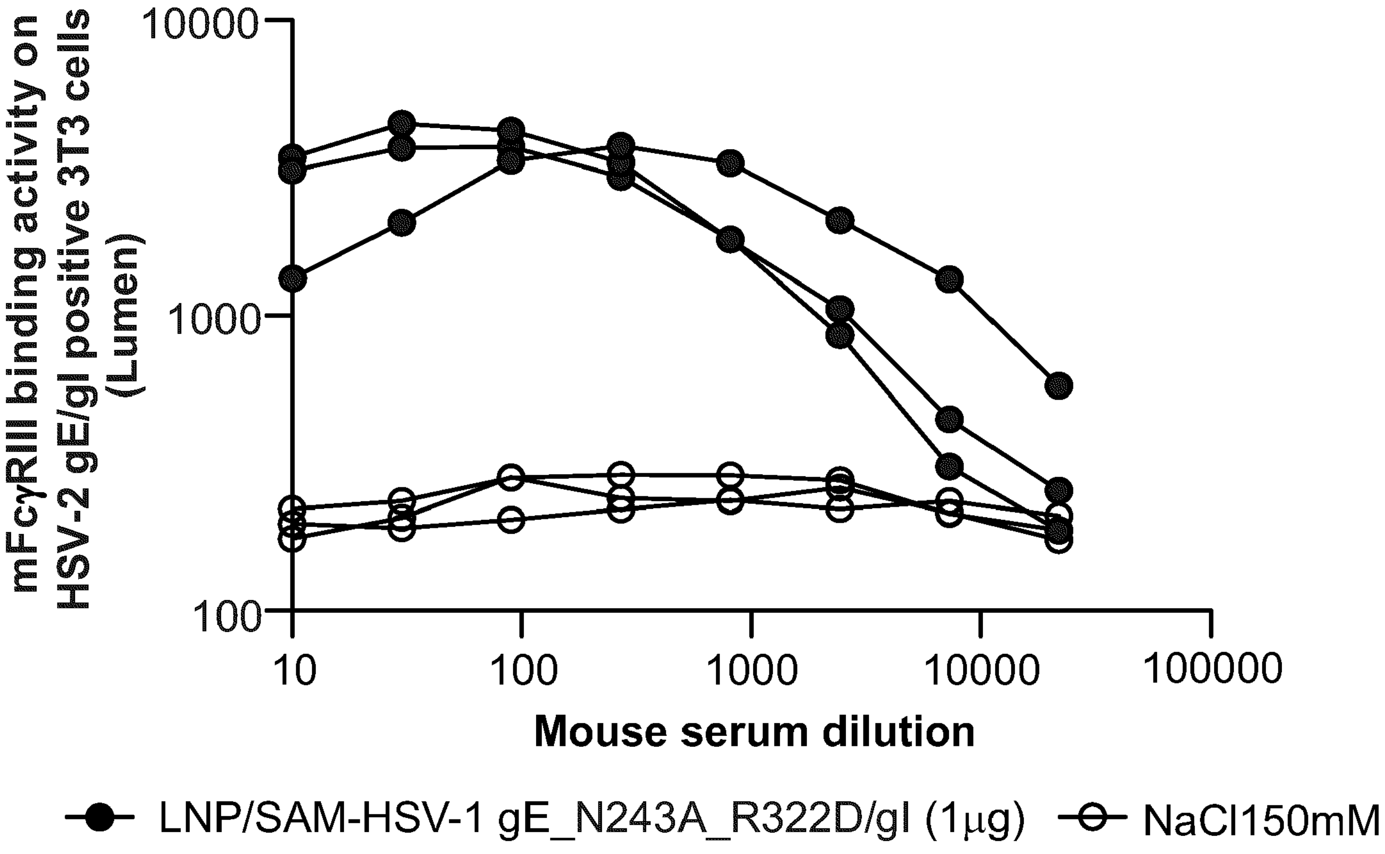

# FIG. 66E

**HSV-2 cross-reactive mFcγRIII binding activity detected in serum samples collected 21 days post second immunization with LNP-formulated SAM-HSV-1 gE_A340G_S341G_V342G/gI mutant candidate**

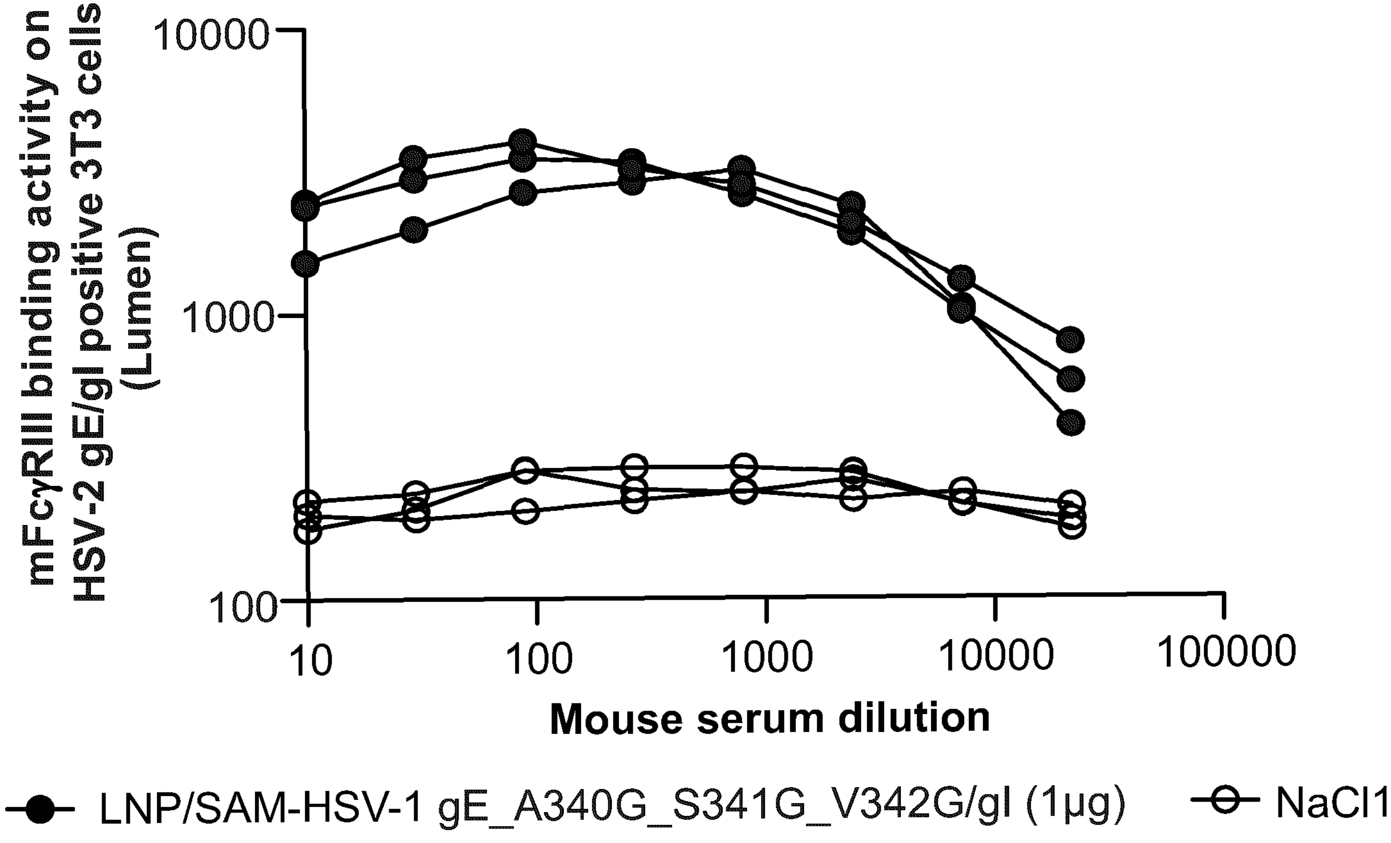

FIG. 67A

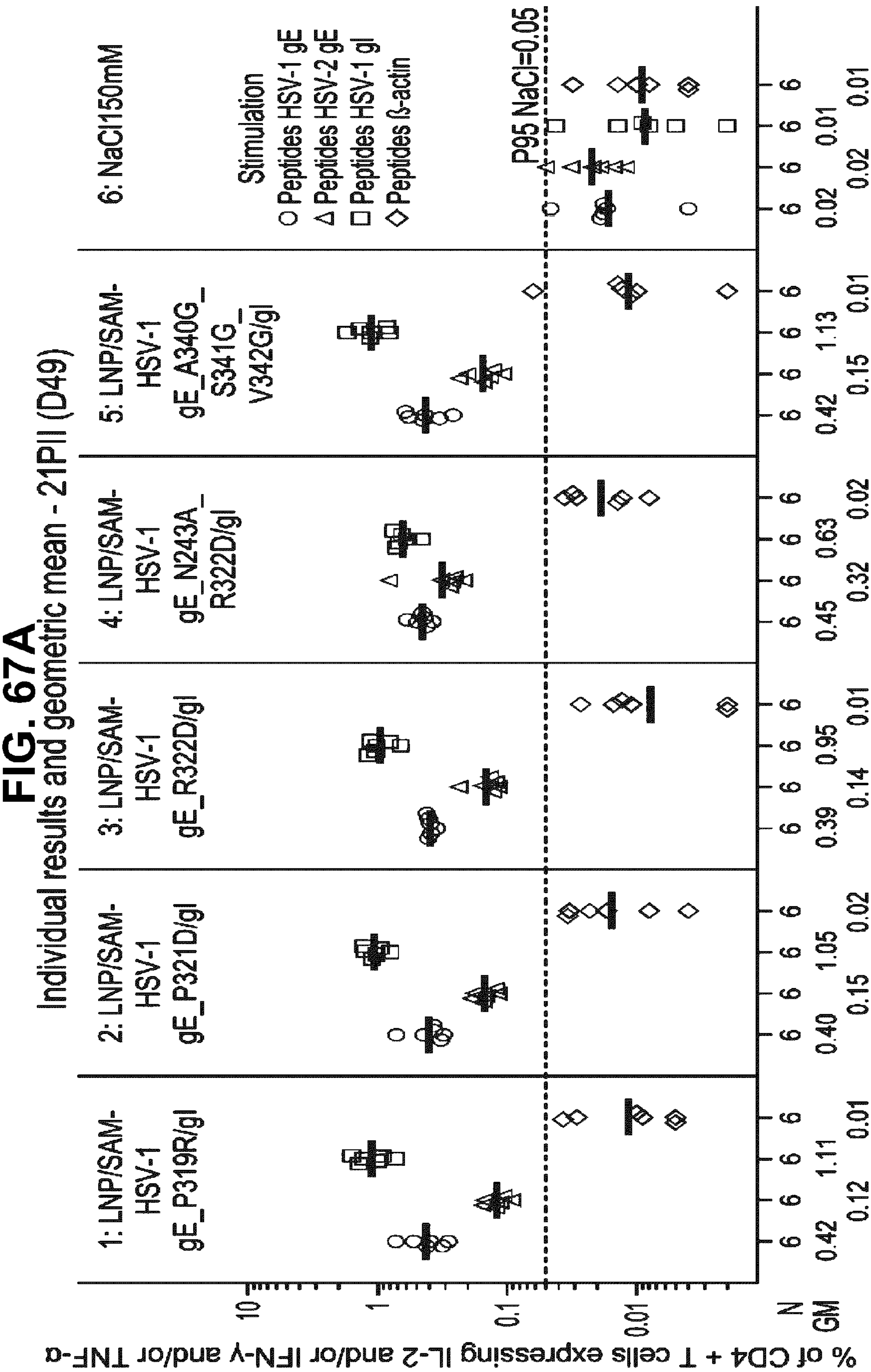
Individual results and geometric mean - 21PII (D49)
1: LNP/SAM-HSV-1 gE_P319R/gI
2: LNP/SAM-HSV-1 gE_P321D/gI
3: LNP/SAM-HSV-1 gE_R322D/gI
4: LNP/SAM-HSV-1 gE_N243A_R322D/gI
5: LNP/SAM-HSV-1 gE_A340G_S341G_V342G/gI
6: NaCl150mM
Stimulation
Peptides HSV-1 gE
Peptides HSV-2 gE
Peptides HSV-1 gI
Peptides ß-actin
P95 NaCl=0.05
% of CD4 + T cells expressing IL-2 and/or IFN-γ and/or TNF-α
10
1
0.1
0.01
N
GM
6 6 6 6
0.42 0.12 1.11 0.01
6 6 6 6
0.40 0.15 1.05 0.02
6 6 6 6
0.39 0.14 0.95 0.01
6 6 6 6
0.45 0.32 0.63 0.02
6 6 6 6
0.42 0.15 1.13 0.01
6 6 6 6
0.02 0.02 0.01 0.01

# FIG. 67B

Individual results and geometric mean - 21PII (D49)

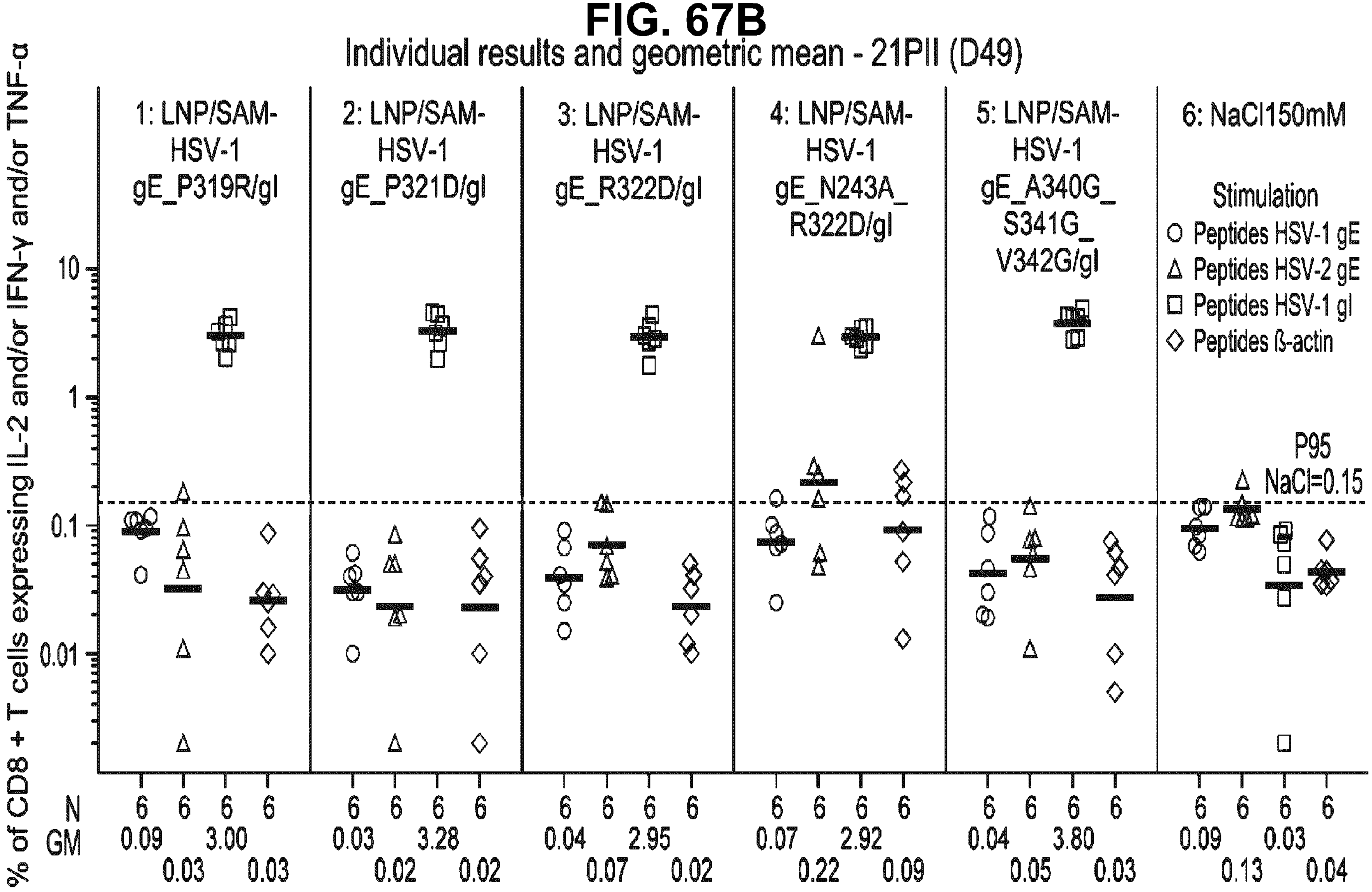

FIG. 68A

Geometric mean ratios with 95% CIs of % of HSV-1 gE-specific CD4+ T cells

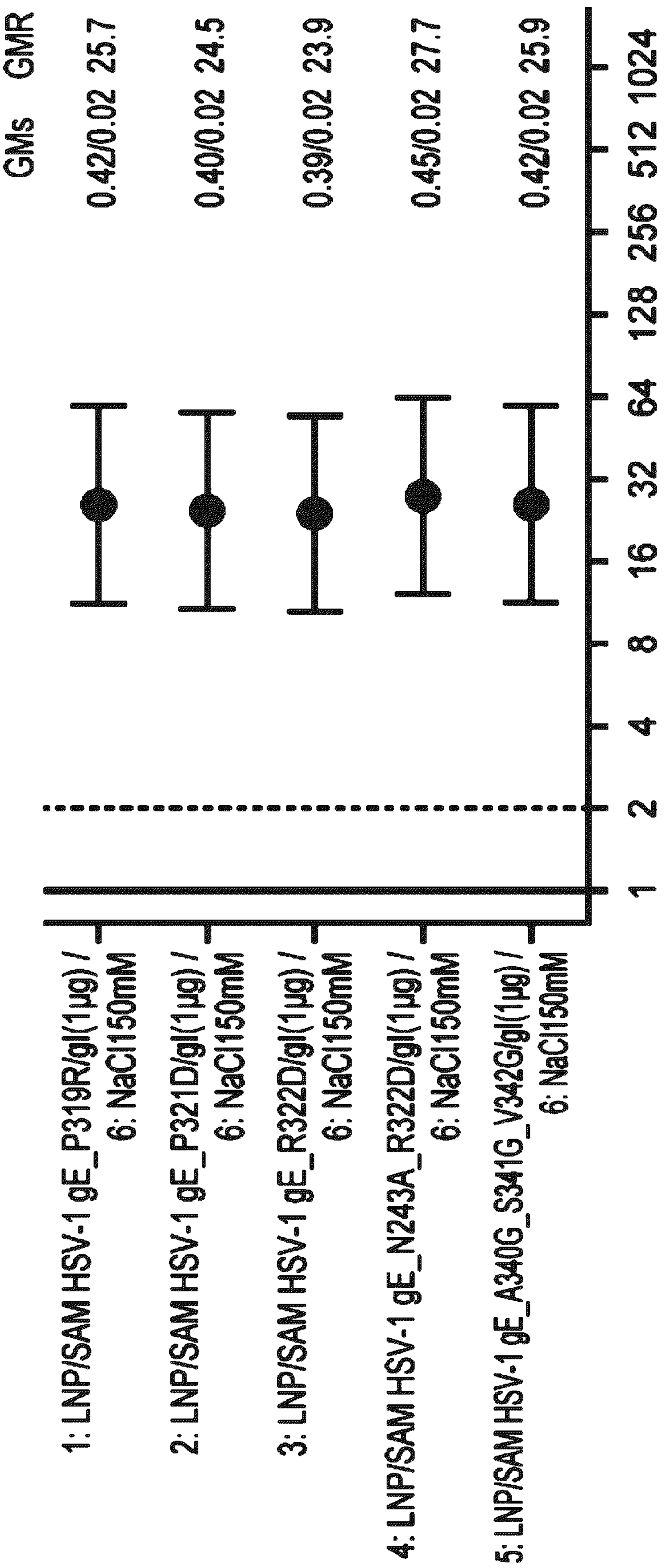

# FIG. 68B

Geometric mean ratios with 95% CIs of % of HSV-2 gE cross-reactive CD4+ T cells

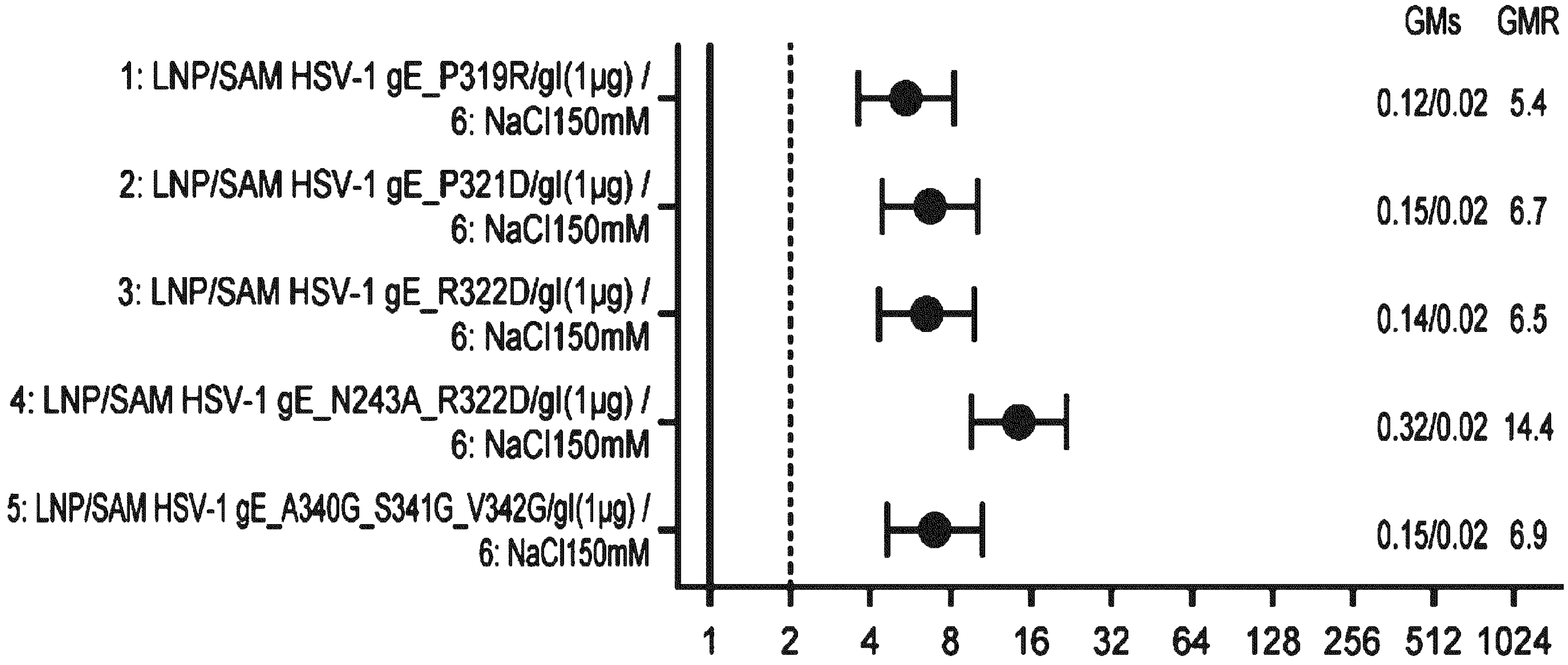

# FIG. 68C

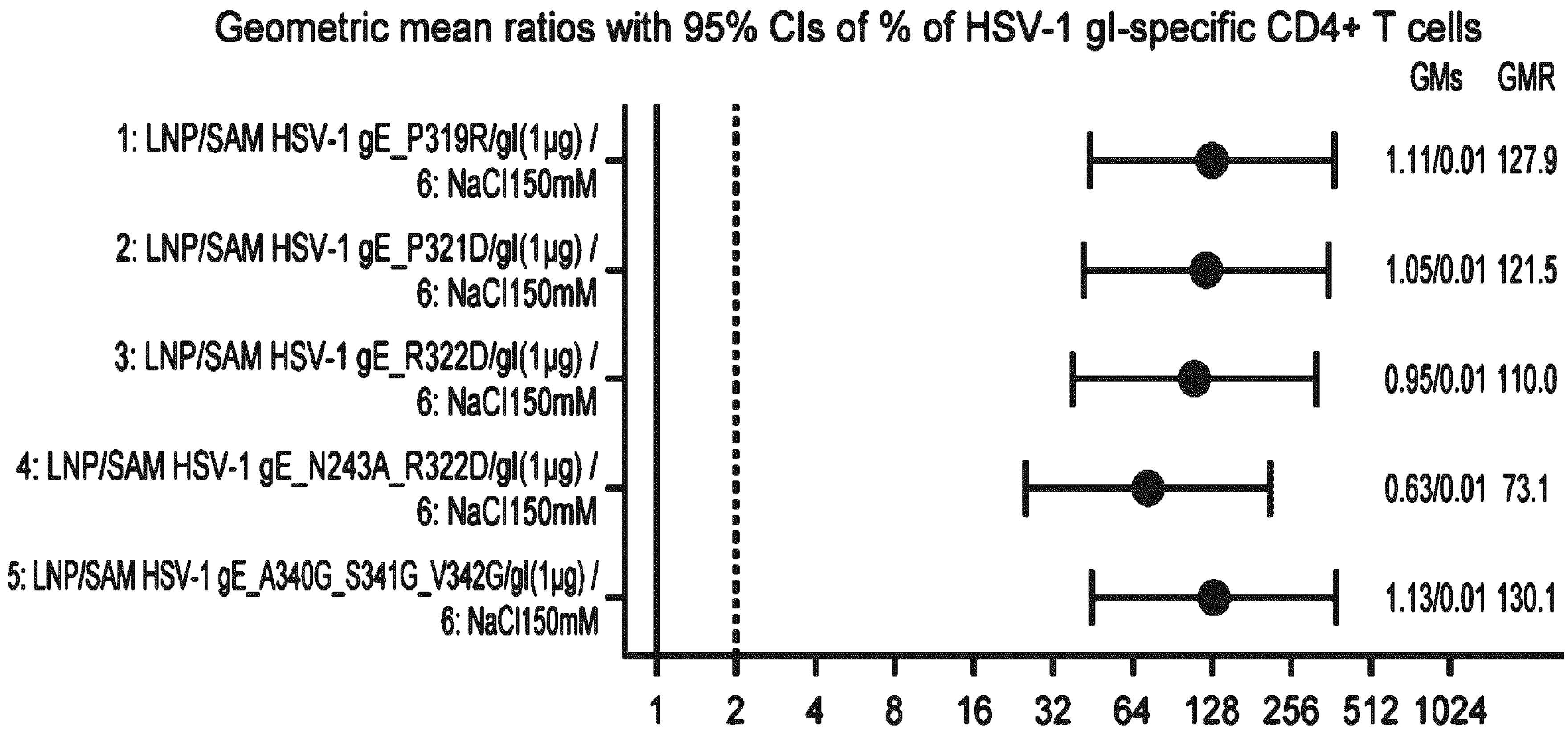
Geometric mean ratios with 95% CIs of % of HSV-1 gI-specific CD4+ T cells
GMs GMR
1: LNP/SAM HSV-1 gE_P319R/gI(1μg) / 6: NaCl150mM
1.11/0.01 127.9
2: LNP/SAM HSV-1 gE_P321D/gI(1μg) / 6: NaCl150mM
1.05/0.01 121.5
3: LNP/SAM HSV-1 gE_R322D/gI(1μg) / 6: NaCl150mM
0.95/0.01 110.0
4: LNP/SAM HSV-1 gE_N243A_R322D/gI(1μg) / 6: NaCl150mM
0.63/0.01 73.1
5: LNP/SAM HSV-1 gE_A340G_S341G_V342G/gI(1μg) / 6: NaCl150mM
1.13/0.01 130.1
1 2 4 8 16 32 64 128 256 512 1024

# FIG. 68D

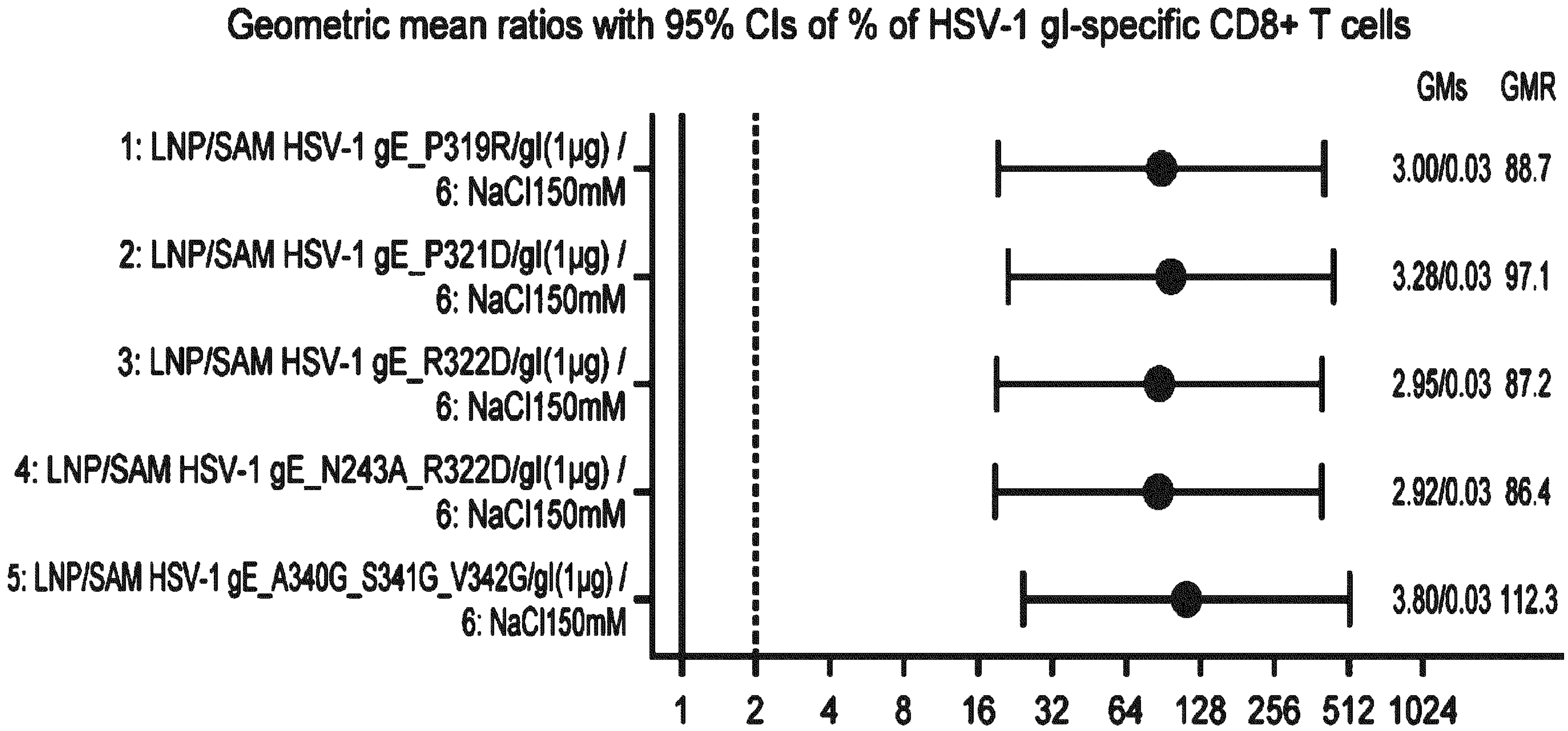
Geometric mean ratios with 95% CIs of % of HSV-1 gI-specific CD8+ T cells
GMs GMR
1: LNP/SAM HSV-1 gE_P319R/gI(1μg) / 6: NaCl150mM
3.00/0.03 88.7
2: LNP/SAM HSV-1 gE_P321D/gI(1μg) / 6: NaCl150mM
3.28/0.03 97.1
3: LNP/SAM HSV-1 gE_R322D/gI(1μg) / 6: NaCl150mM
2.95/0.03 87.2
4: LNP/SAM HSV-1 gE_N243A_R322D/gI(1μg) / 6: NaCl150mM
2.92/0.03 86.4
5: LNP/SAM HSV-1 gE_A340G_S341G_V342G/gI(1μg) / 6: NaCl150mM
3.80/0.03 112.3
1 2 4 8 16 32 64 128 256 512 1024

FIG. 69A Geometric mean ratios with 95% CIs of % of HSV-1 gE-specific CD4+ T cells Head to head comparison of LNP/SAM HSV-1 gE/gI(1µg) groups

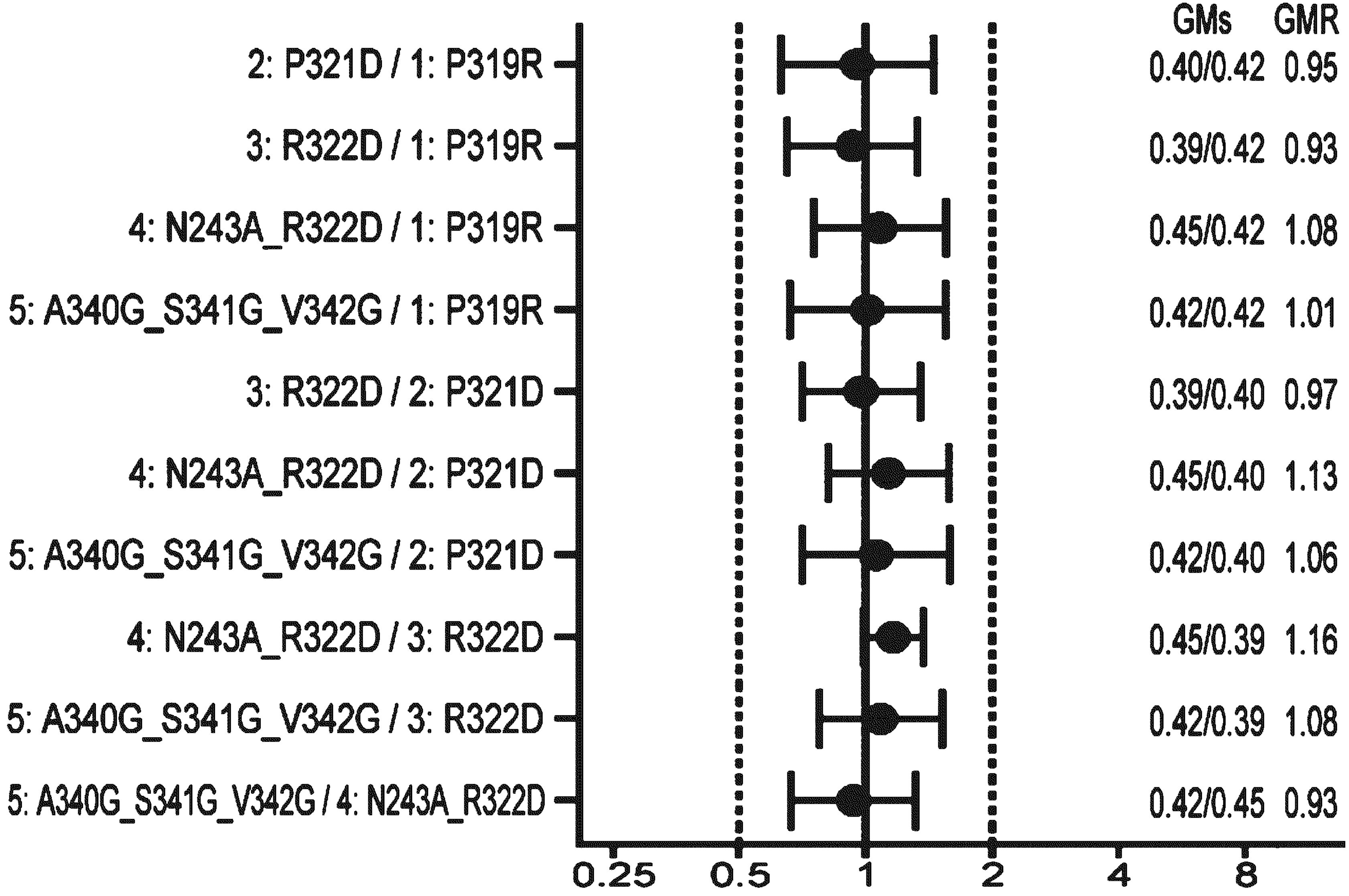

**FIG. 69B** Geometric mean ratios with 95% CIs of % of HSV-2 gE cross-reactive CD4+ T cells Head to head comparison of LNP/SAM HSV-1 gE/gI(1µg) groups

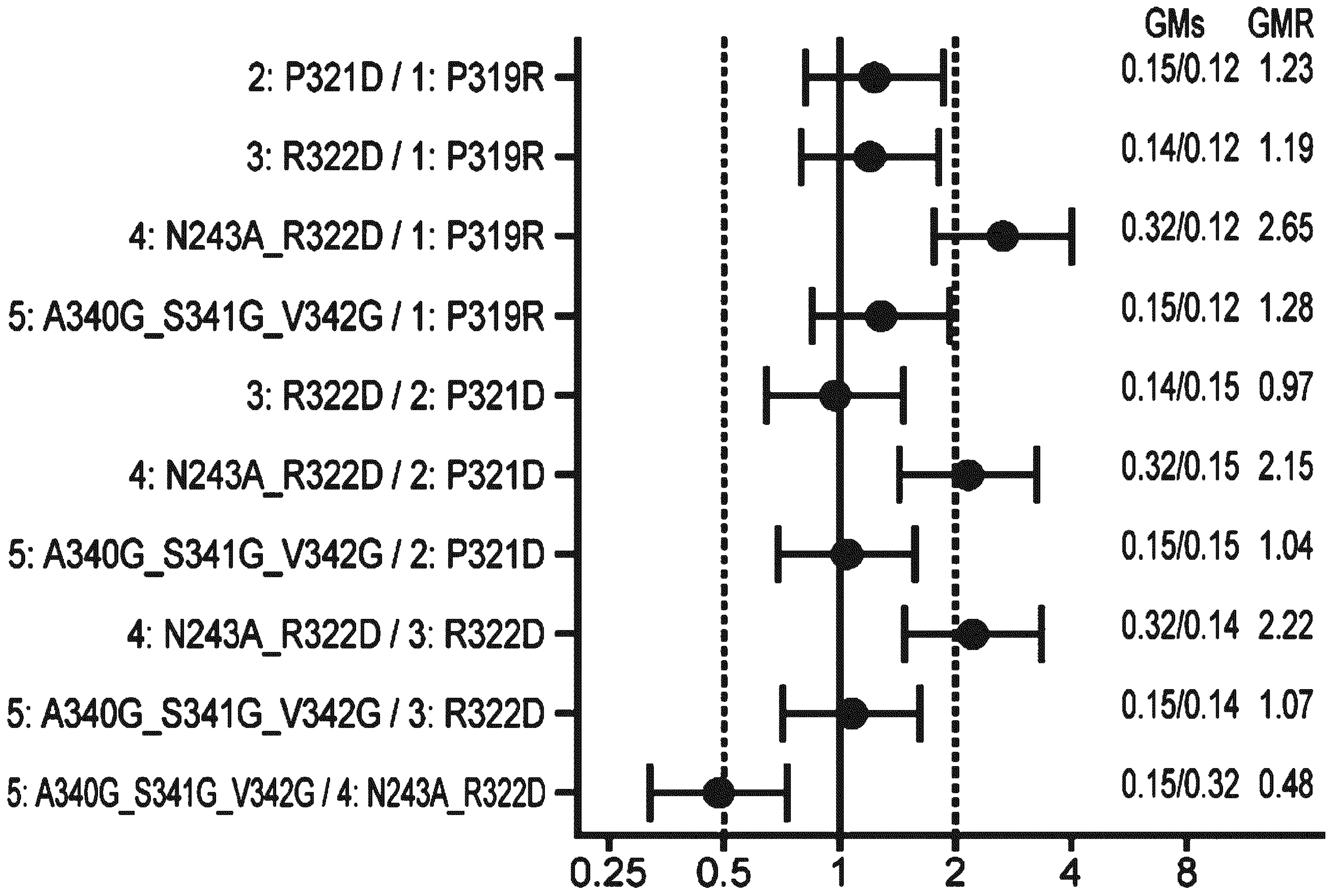

**FIG. 70A** Geometric mean ratios with 95% CIs of % of HSV-1 gI-specific CD4+ T cells Head to head comparison of LNP/SAM HSV-1 gE/gI(1µg) groups

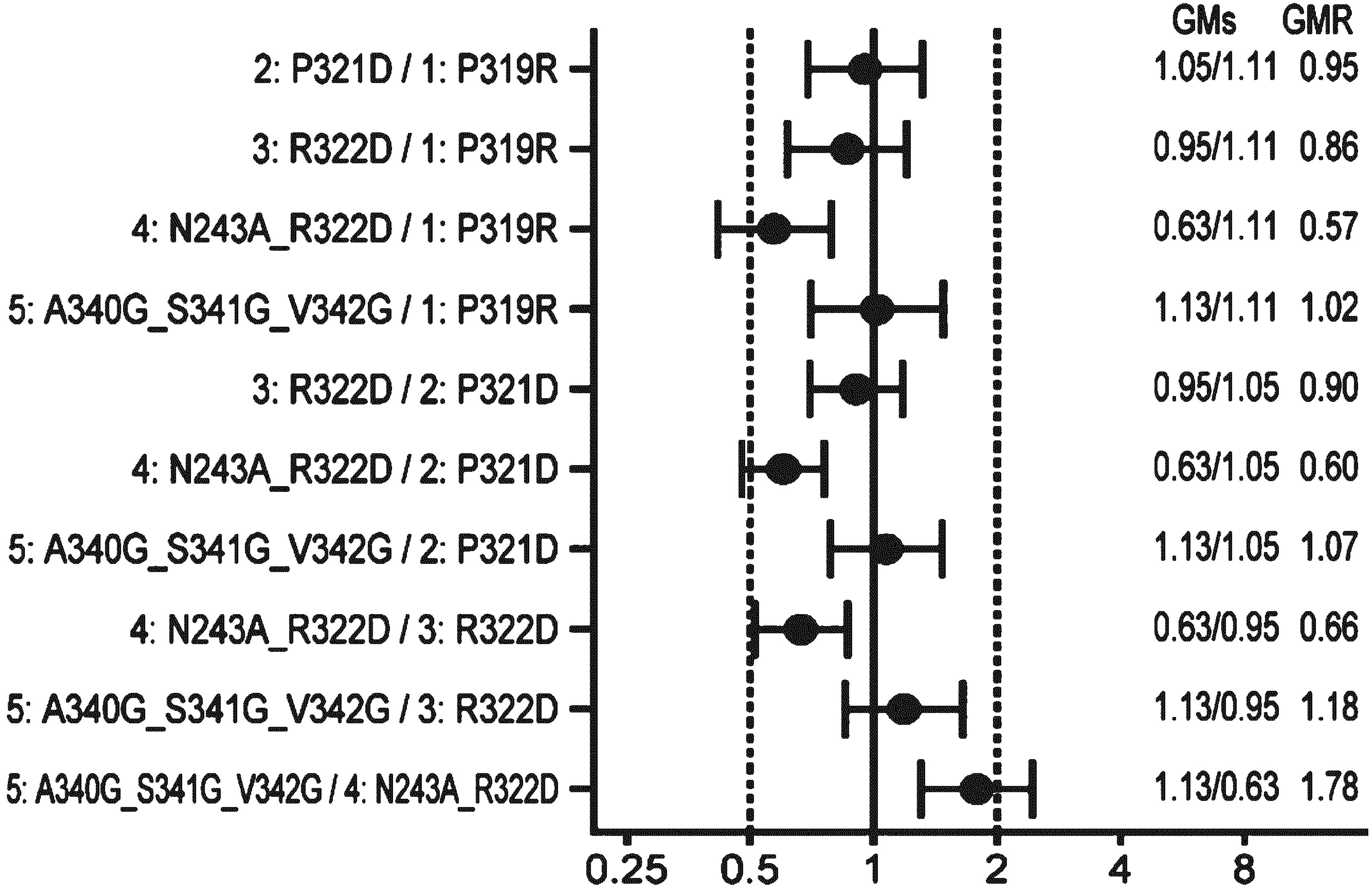

**FIG. 70B** Geometric mean ratios with 95% CIs of % of HSV-1 gI-specific CD8+ T cells Head to head comparison of LNP/SAM HSV-1 gE/gI(1μg) groups

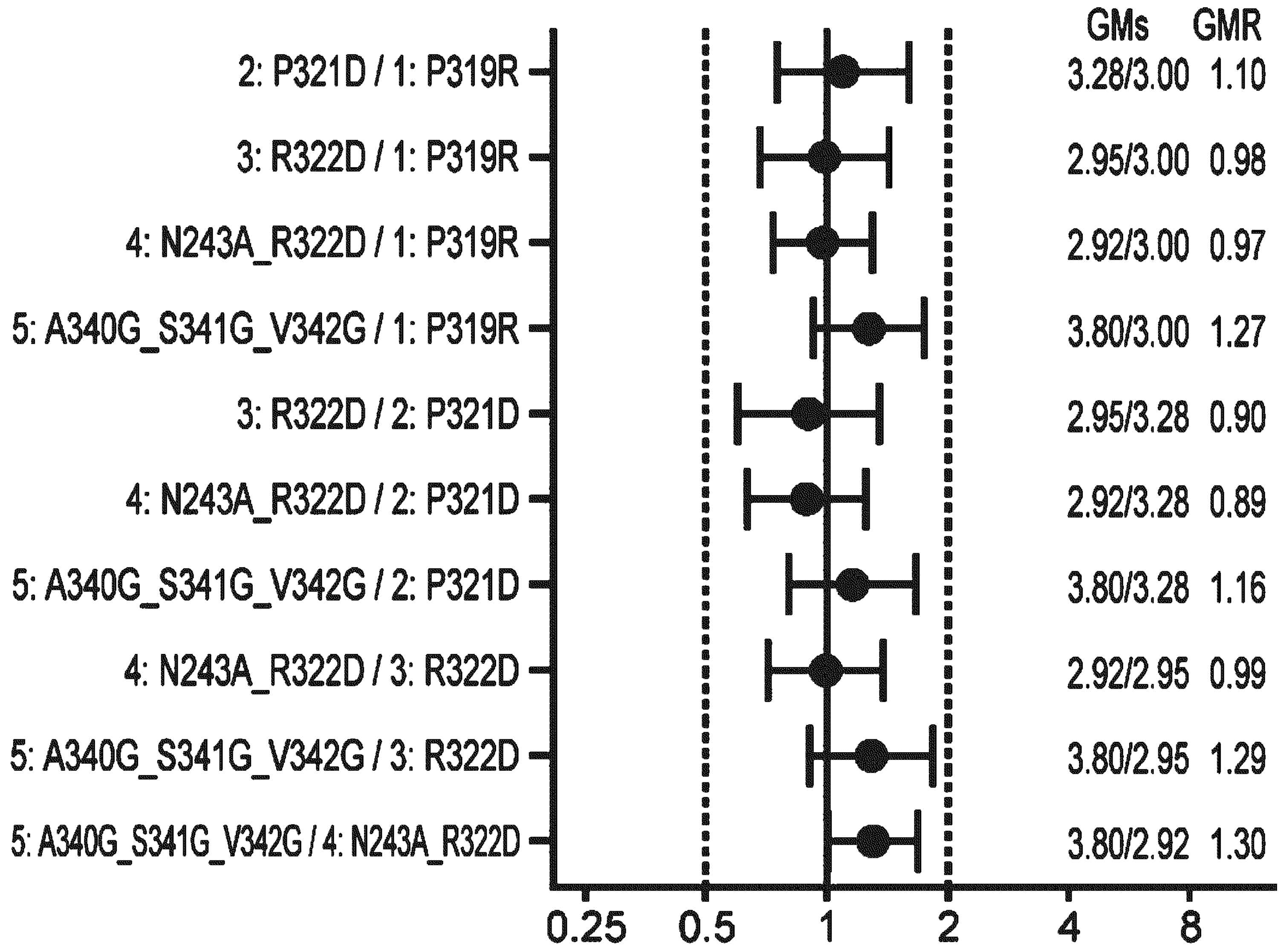

# FIG. 71A

**Co-incubation of hIgG antibodies with serum samples collected 14 days post third immunization**

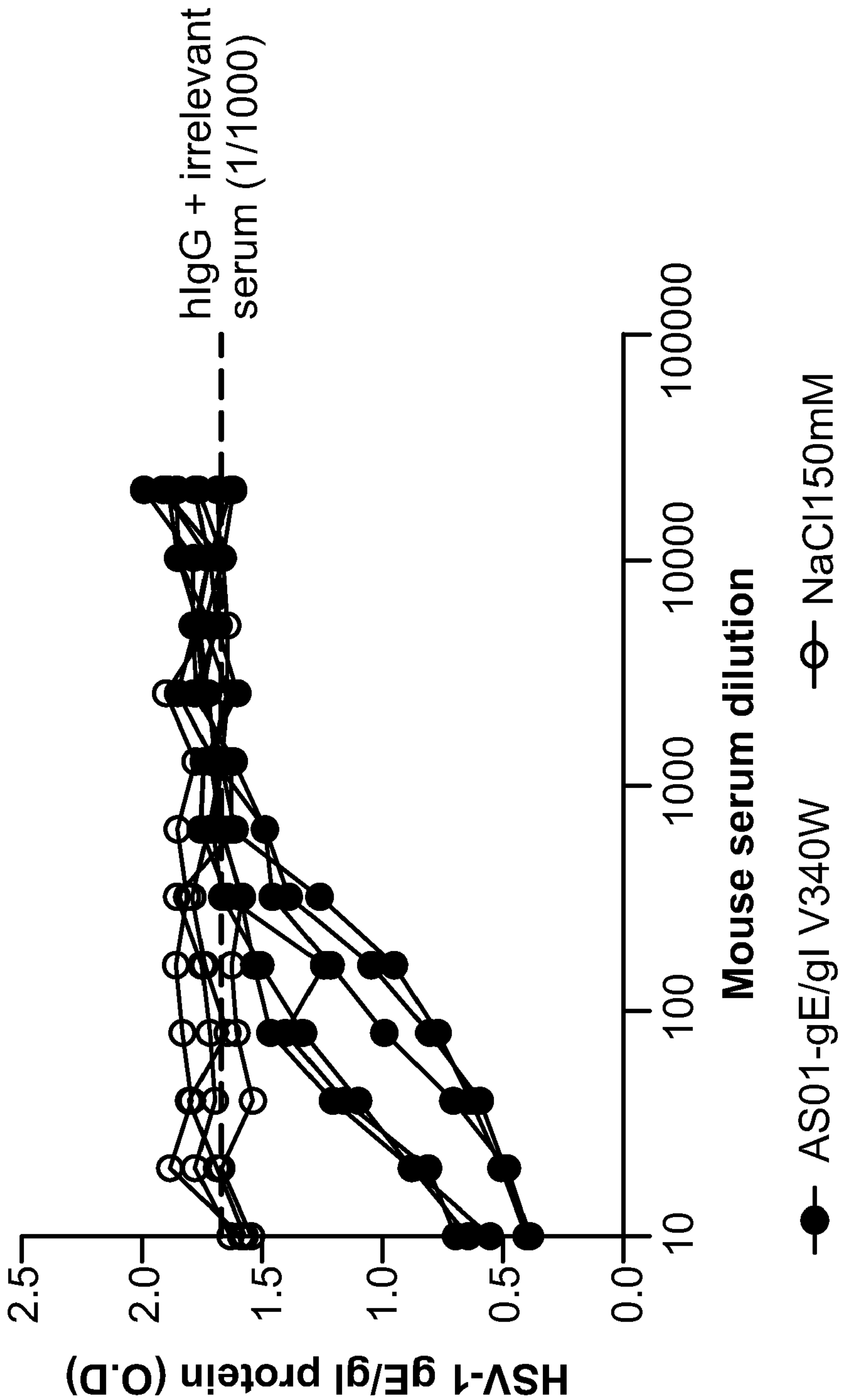

FIG. 71B

Co-incubation of hIgG antibodies with serum samples collected 14 days post third immunization

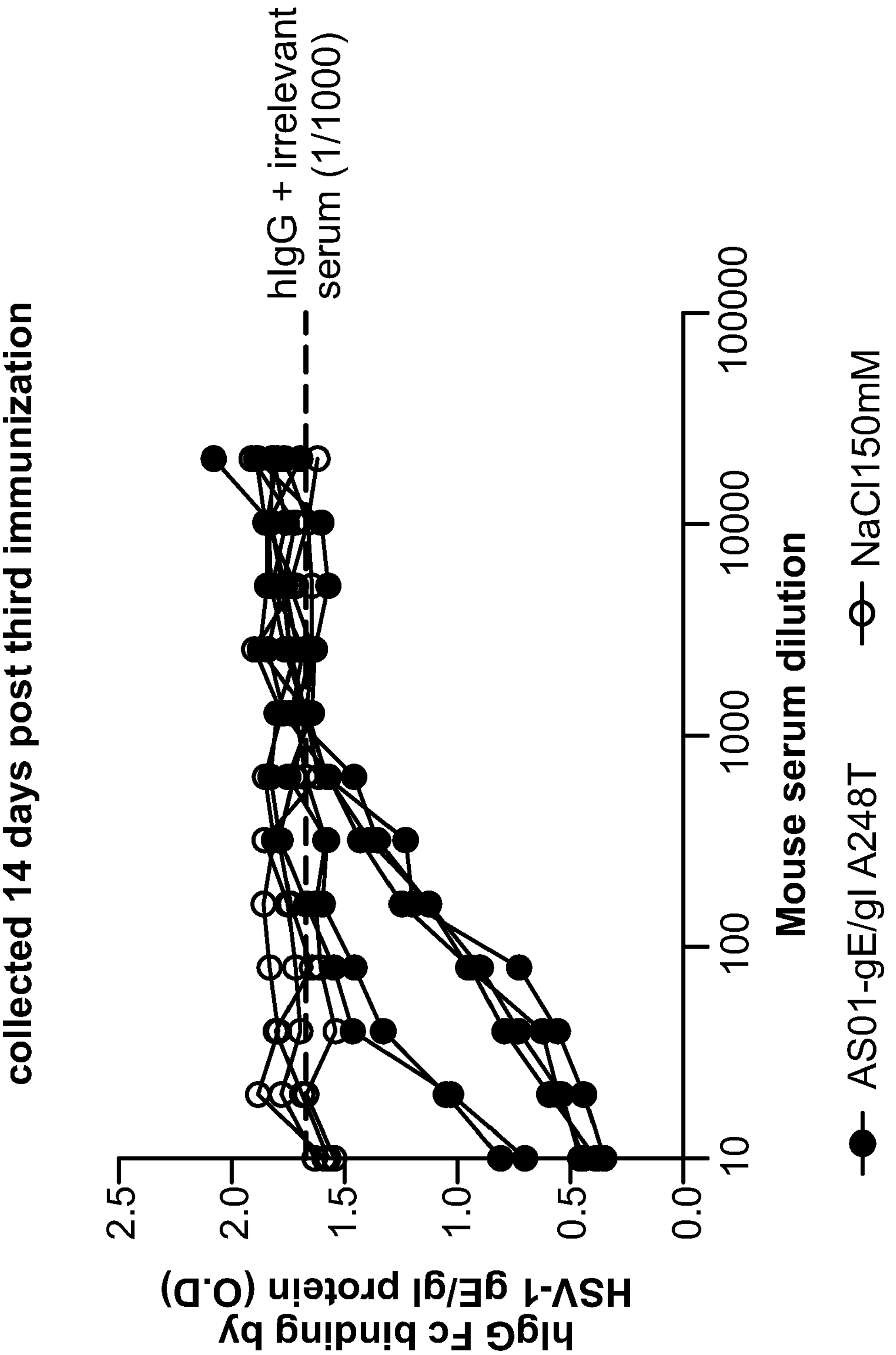

# FIG. 71C

**Co-incubation of hIgG antibodies with serum samples collected 14 days post third immunization**

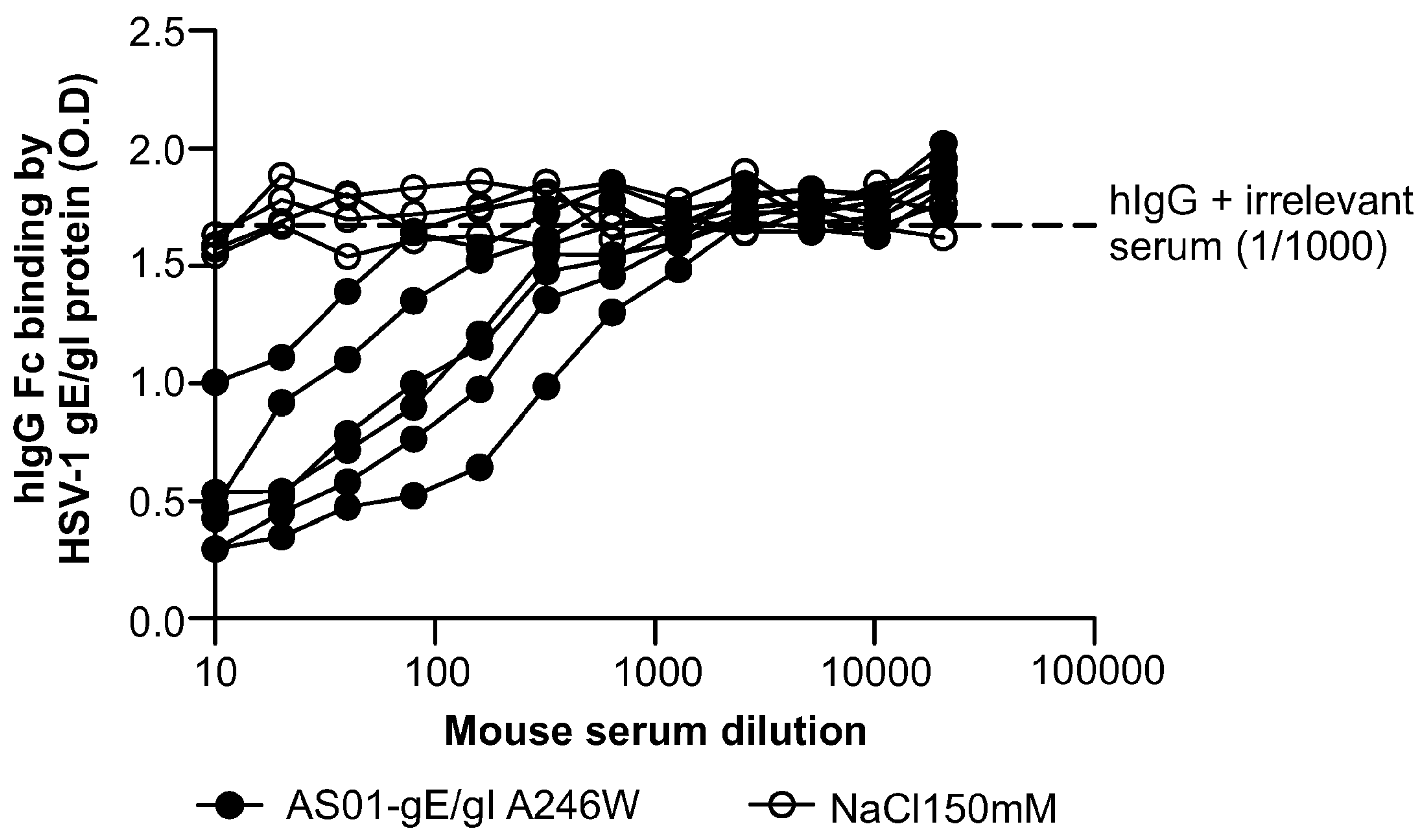

FIG. 71D

Co-incubation of hIgG antibodies with serum samples collected 14 days post third immunization

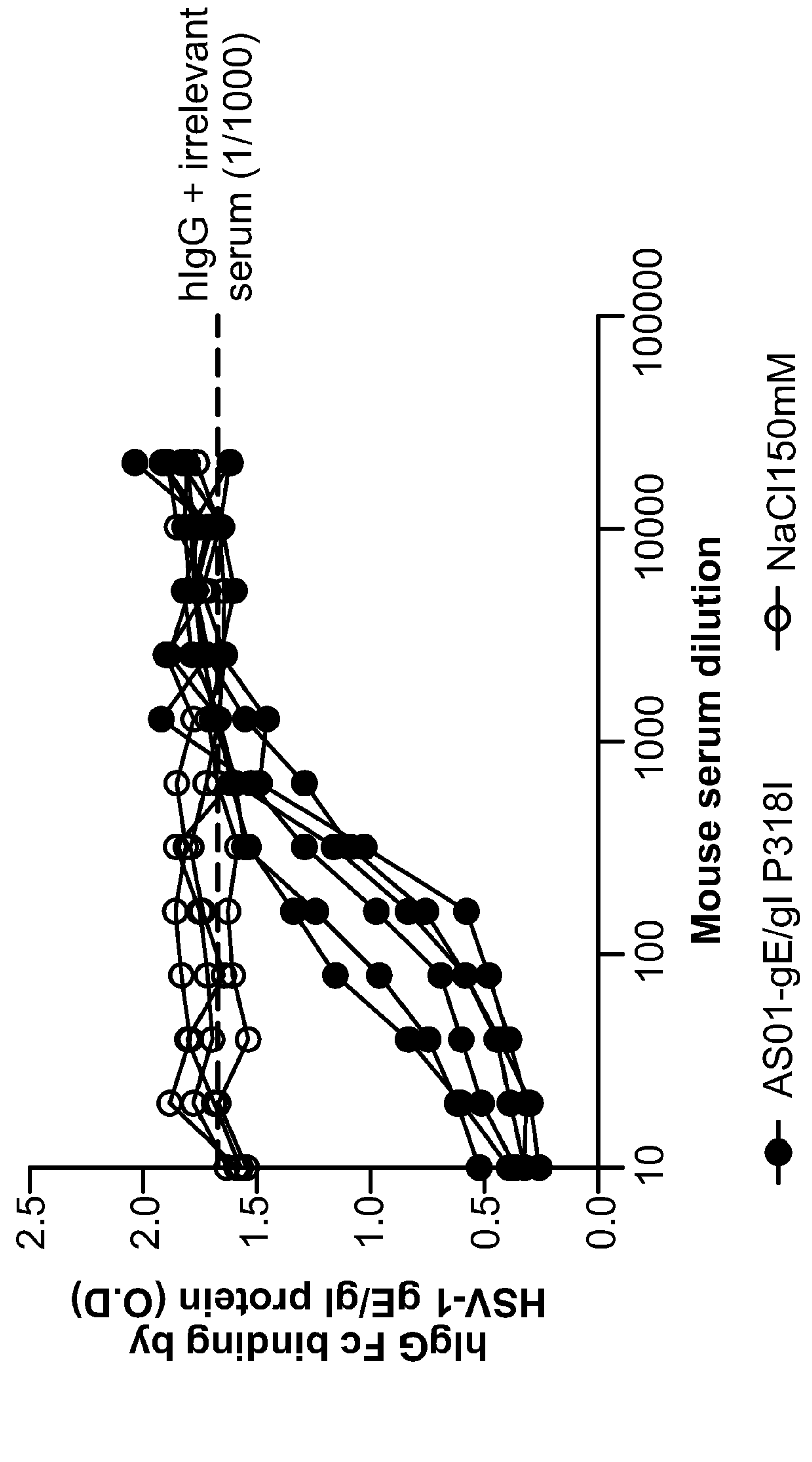

FIG. 71E

Co-incubation of hIgG antibodies with serum samples collected 14 days post third immunization

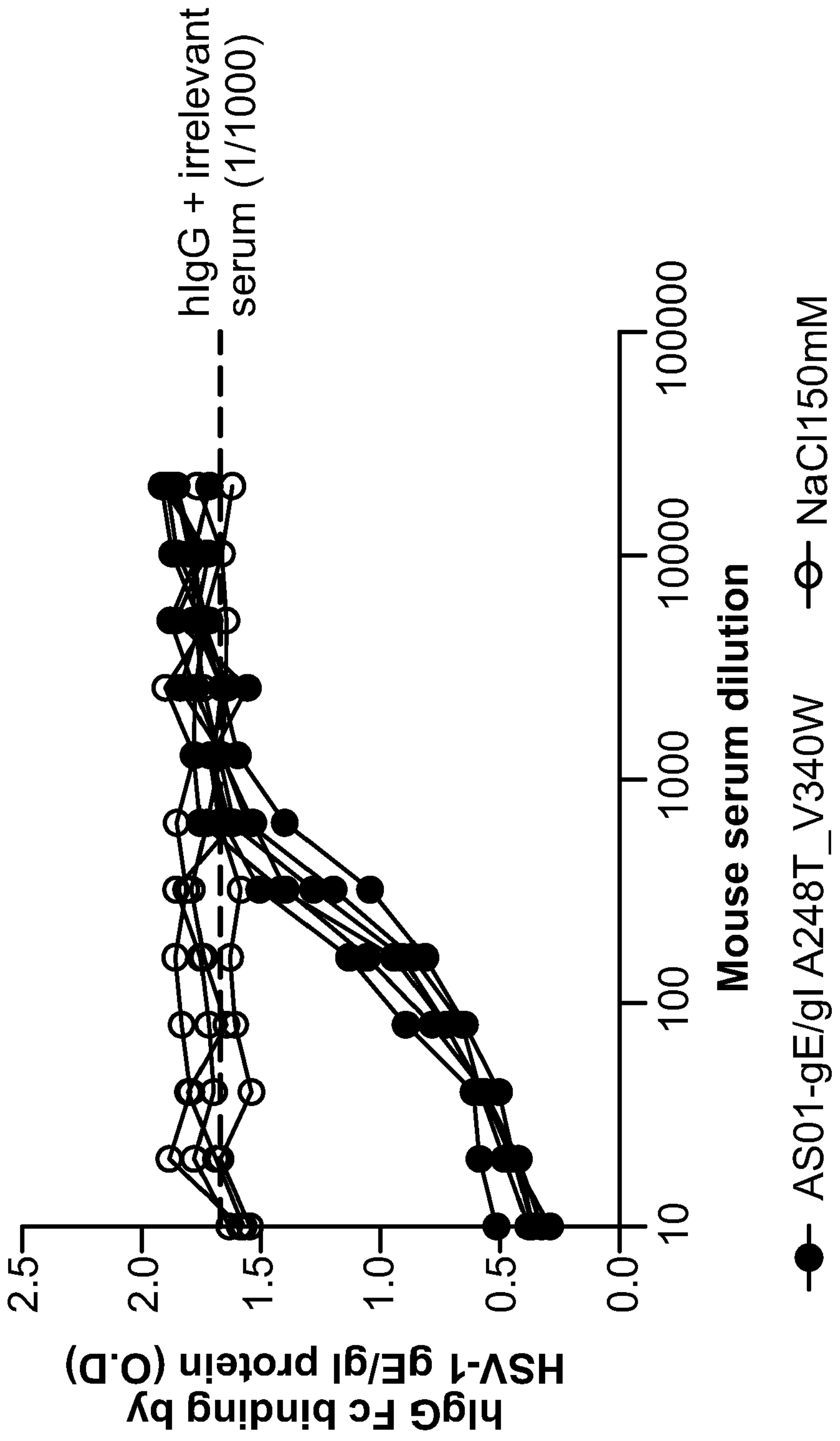

FIG. 72A

Serum collected 14 days post third immunization

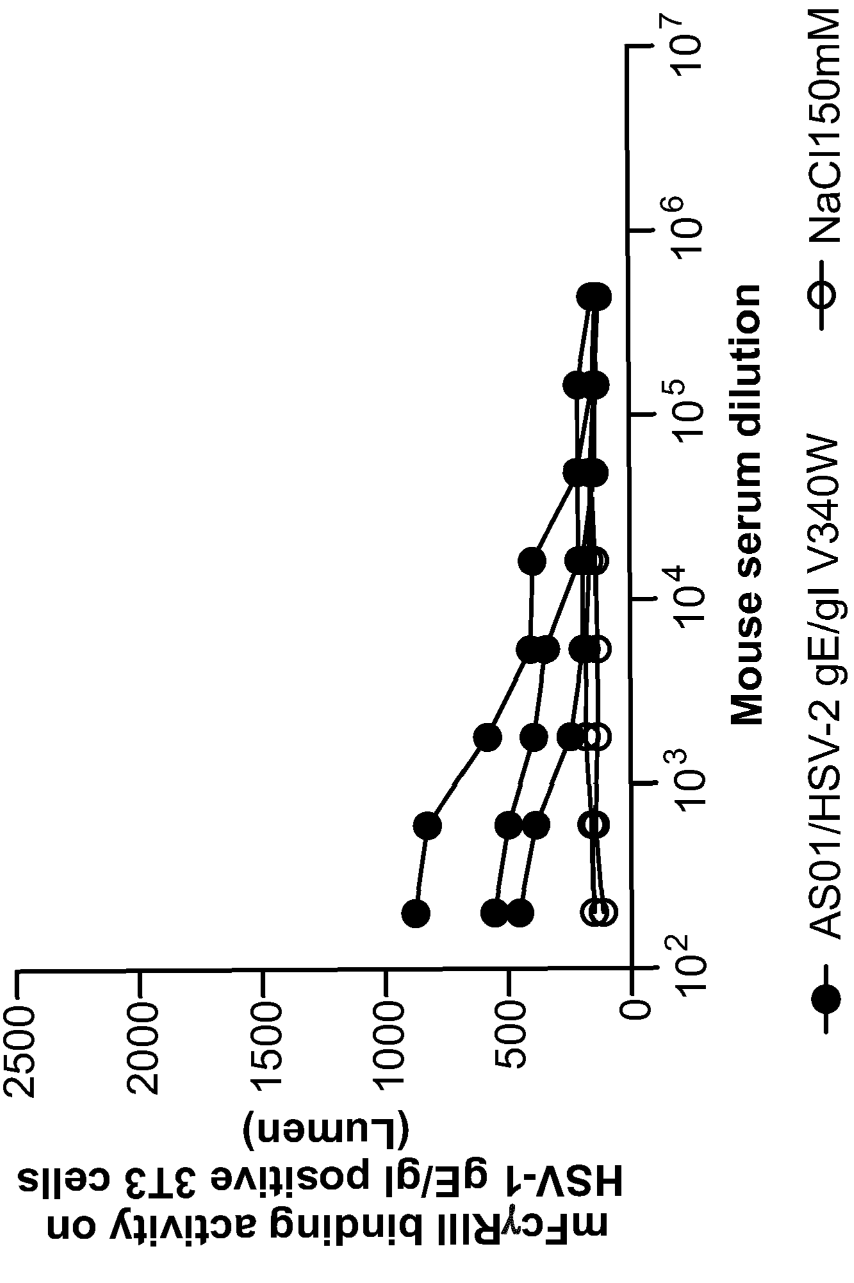

FIG. 72B

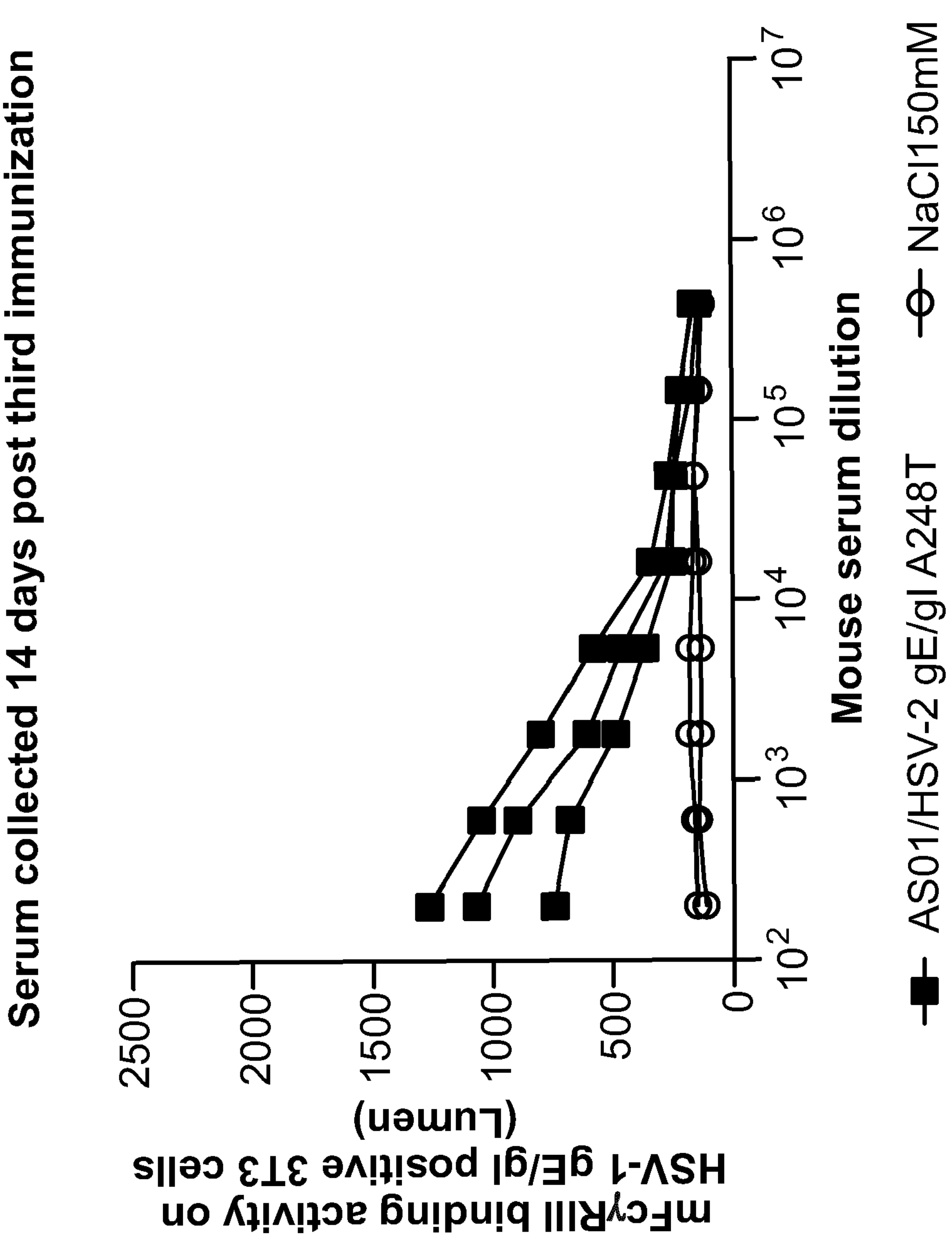
Serum collected 14 days post third immunization
mFcγRIII binding activity on HSV-1 gE/gI positive 3T3 cells (Lumen)
2500
2000
1500
1000
500
0
$10^2$
$10^3$
$10^4$
$10^5$
$10^6$
$10^7$
Mouse serum dilution
AS01/HSV-2 gE/gI A248T
NaCl150mM

FIG. 72C

Serum collected 14 days post third immunization

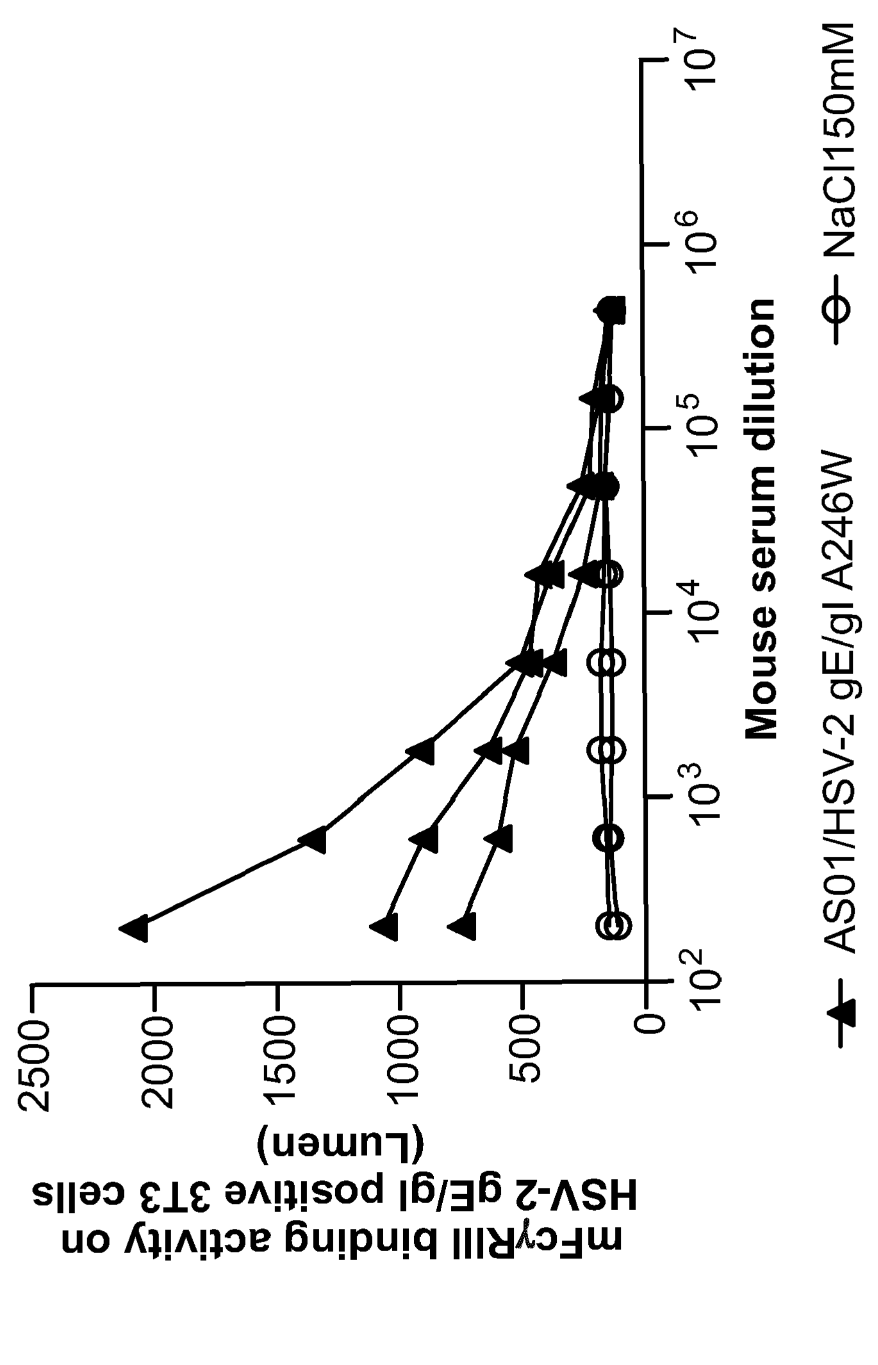

# FIG. 72D

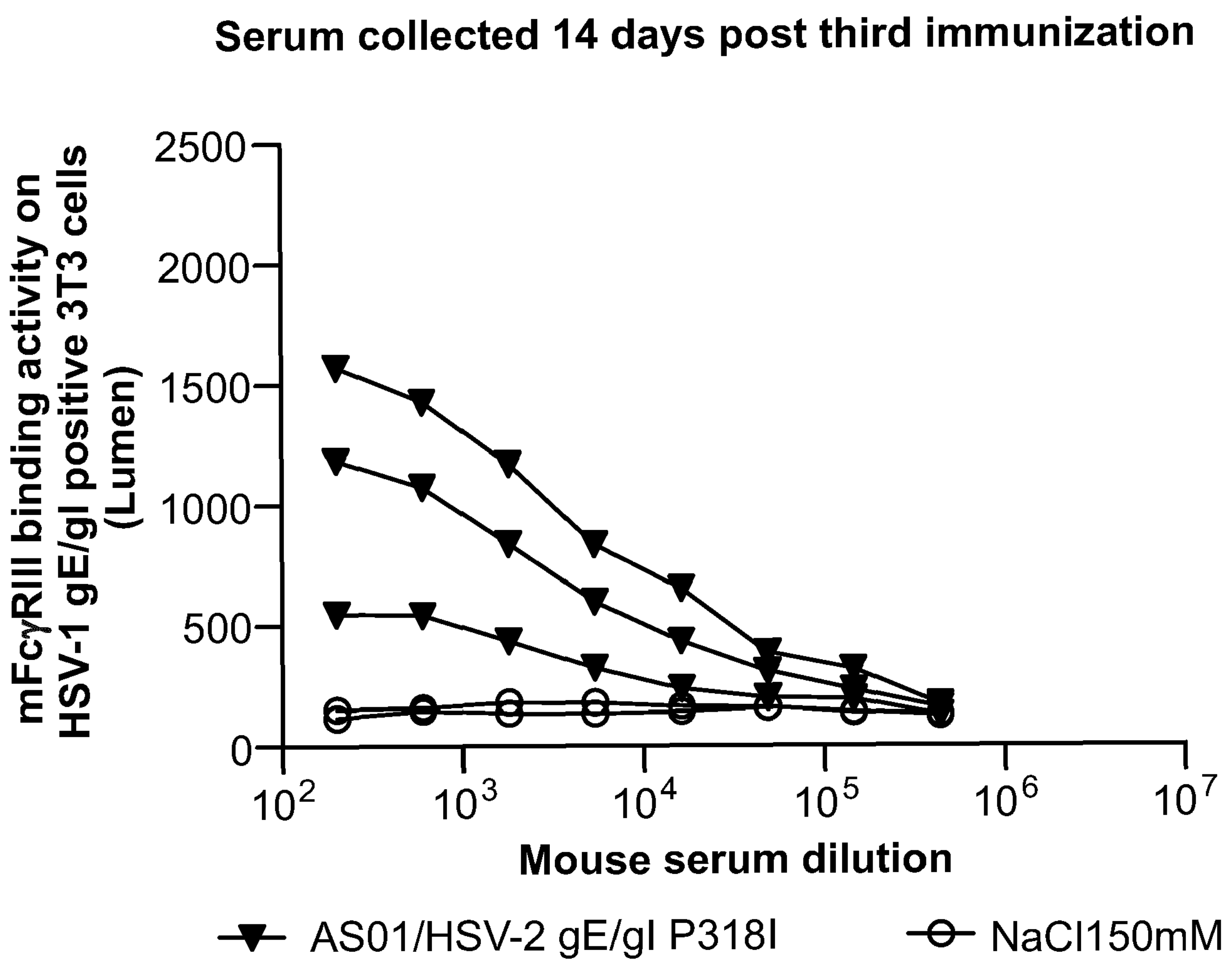
Serum collected 14 days post third immunization
mFcγRIII binding activity on HSV-1 gE/gI positive 3T3 cells (Lumen)
2500
2000
1500
1000
500
0
10^2
10^3
10^4
10^5
10^6
10^7
Mouse serum dilution
AS01/HSV-2 gE/gI P318I
NaCl150mM

FIG. 72E

Serum collected 14 days post third immunization

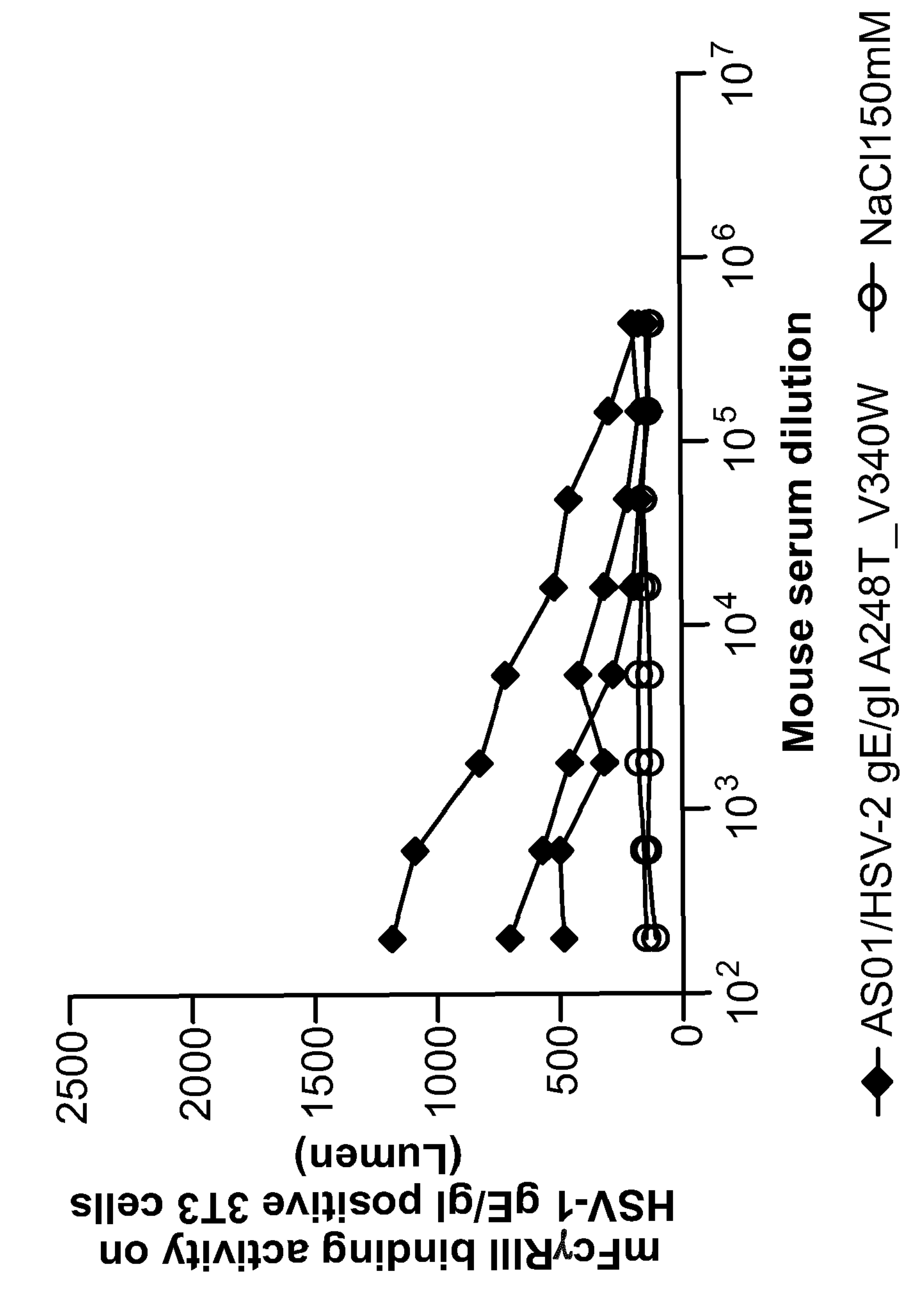

# FIG. 72F

**Serum samples collected 14 days post third immunization**

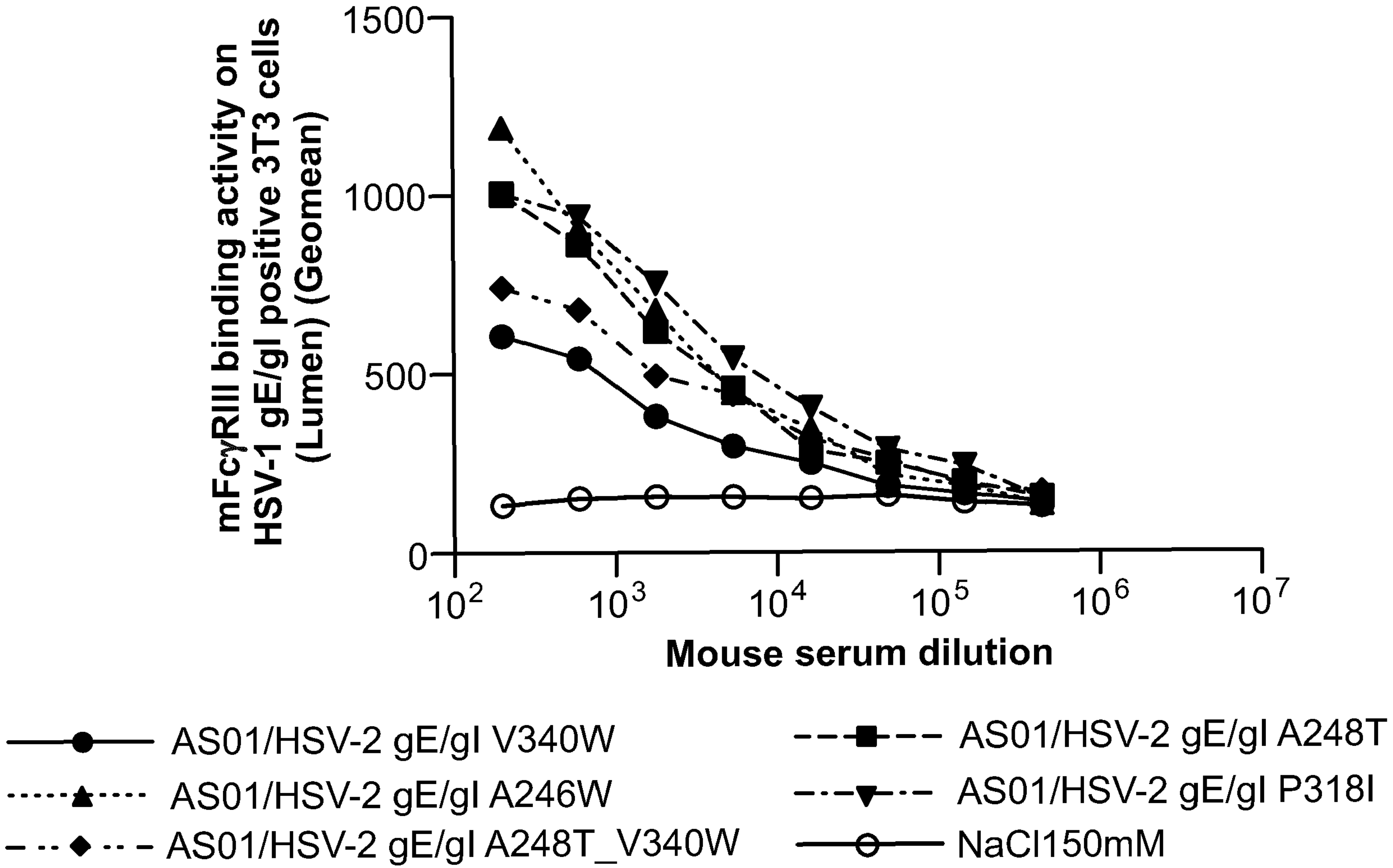

# FIG. 73A

**Co-incubation of hIgG with serum samples collected 21 days post third immunization with LNP-SAM HSV-2 gE/gI V340W**

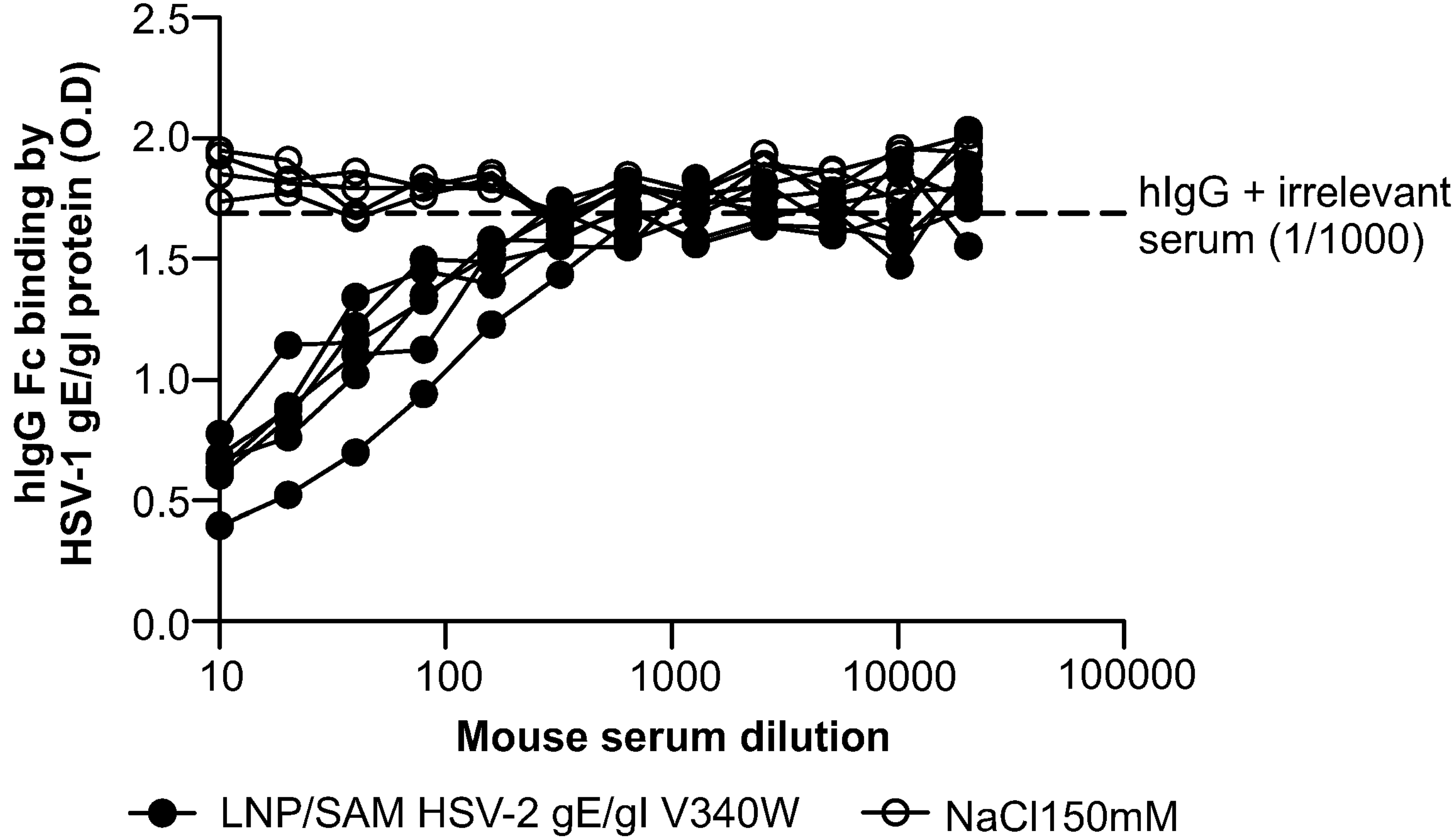

# FIG. 73B

**Co-incubation of hIgG with serum samples collected 21 days post third immunization with LNP-SAM HSV-2 gE/gI A248T**

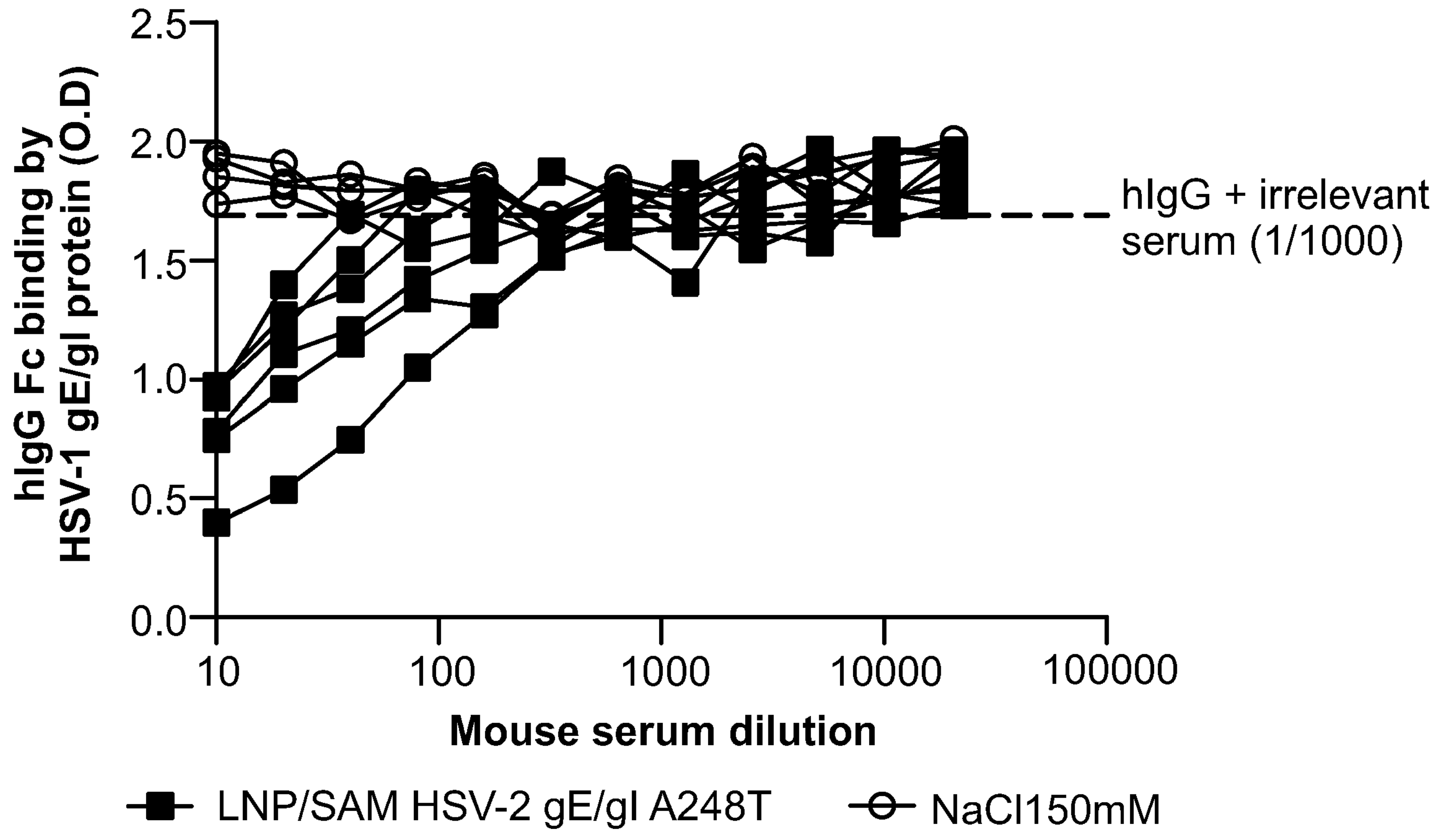

FIG. 73C

**Co-incubation of hIgG with serum samples collected 21 days post third immunization with LNP-SAM HSV-2 gE/gI A246W**

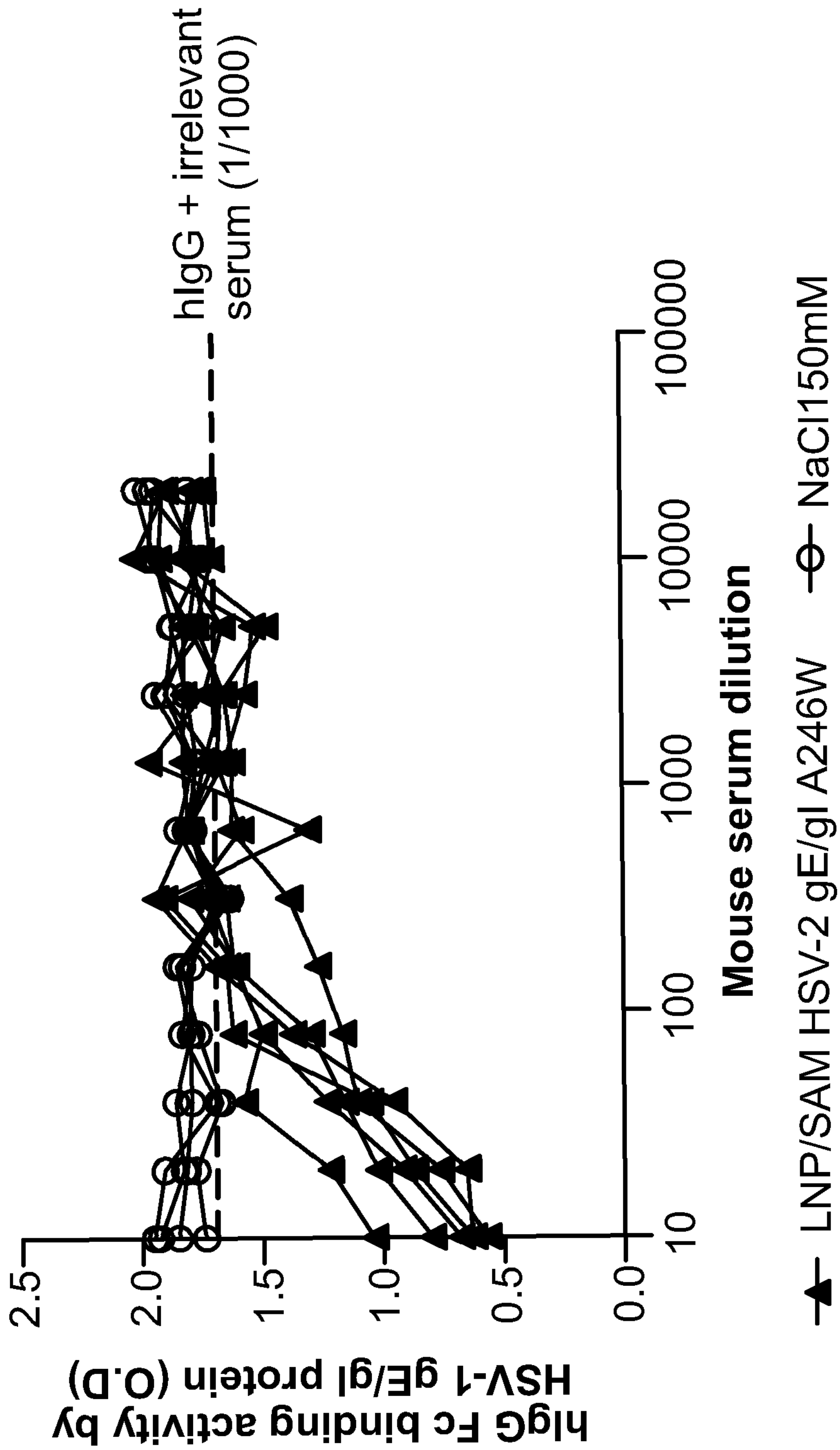

# FIG. 73D

**Co-incubation of hIgG with serum samples collected 21 days post third immunization with LNP-SAM HSV-2 gE/gI P318I**

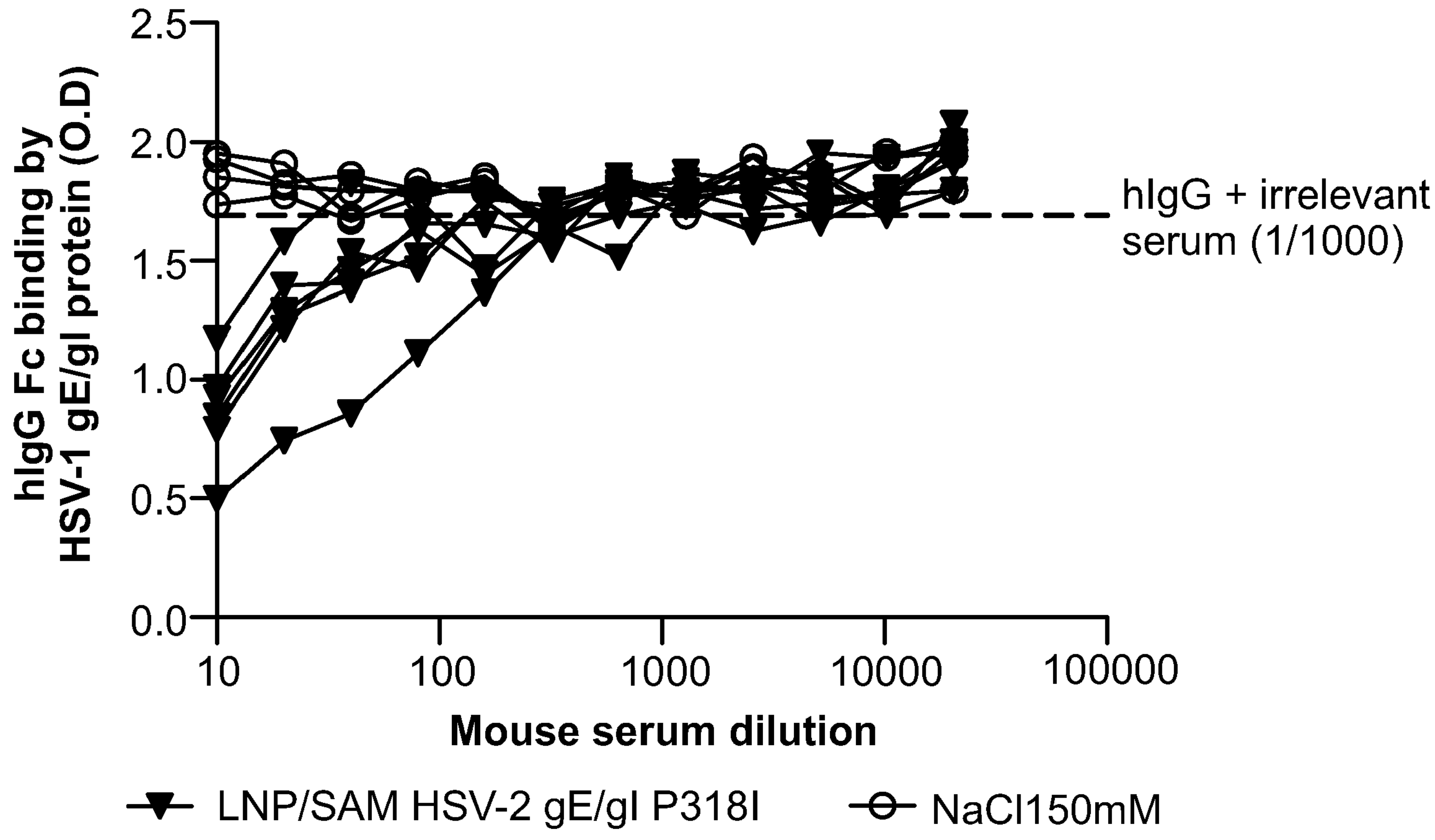

# FIG. 73E

**Co-incubation of hIgG with serum samples collected 21 days post third immunization with LNP-SAM HSV-2 gE/gI A248T_V340W**

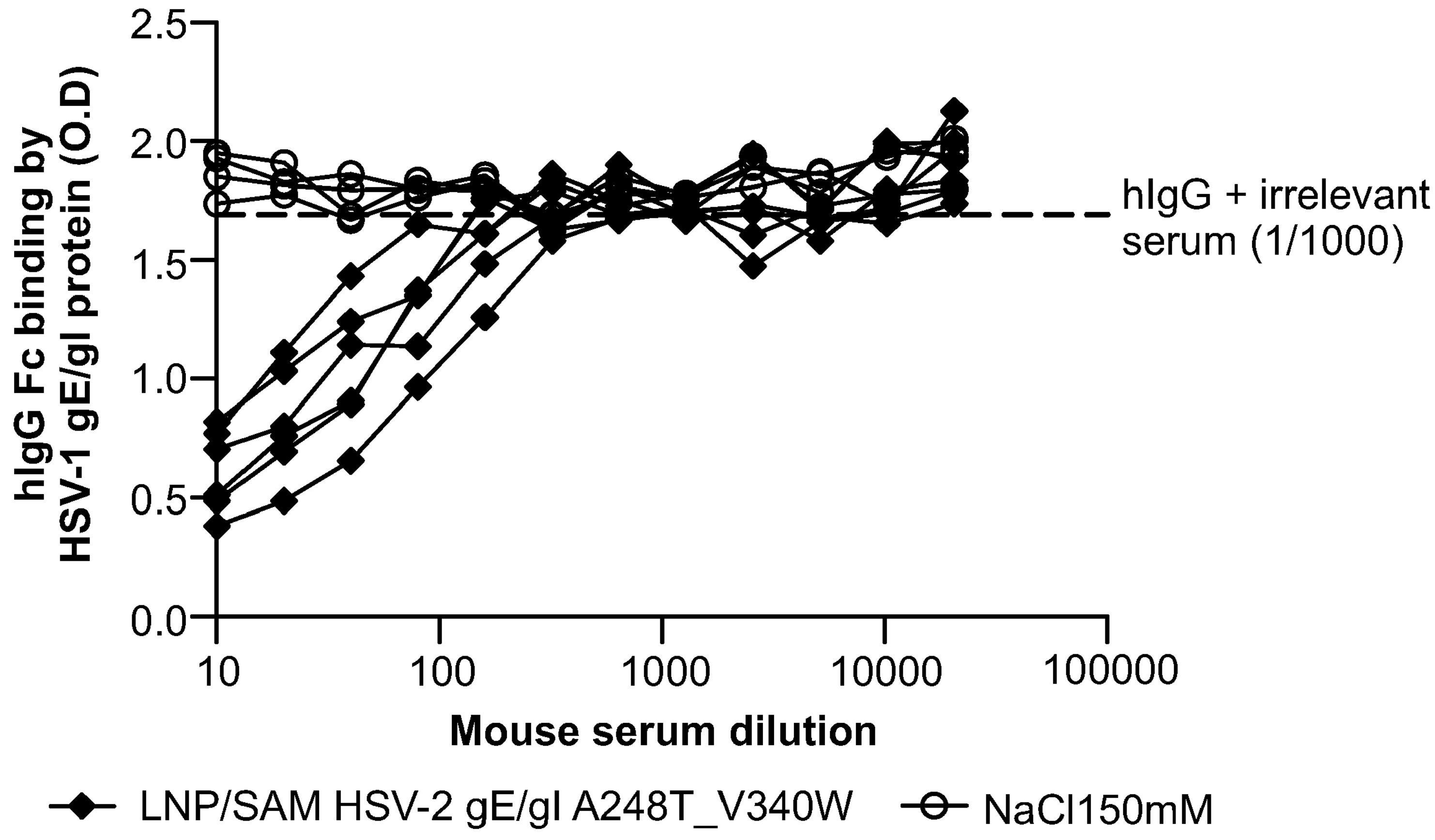

# FIG. 73F

**Co-incubation of hIgG with serum samples collected 21 days post third immunization with LNP-SAM HSV-2 gE/gI insert ARAA candidate**

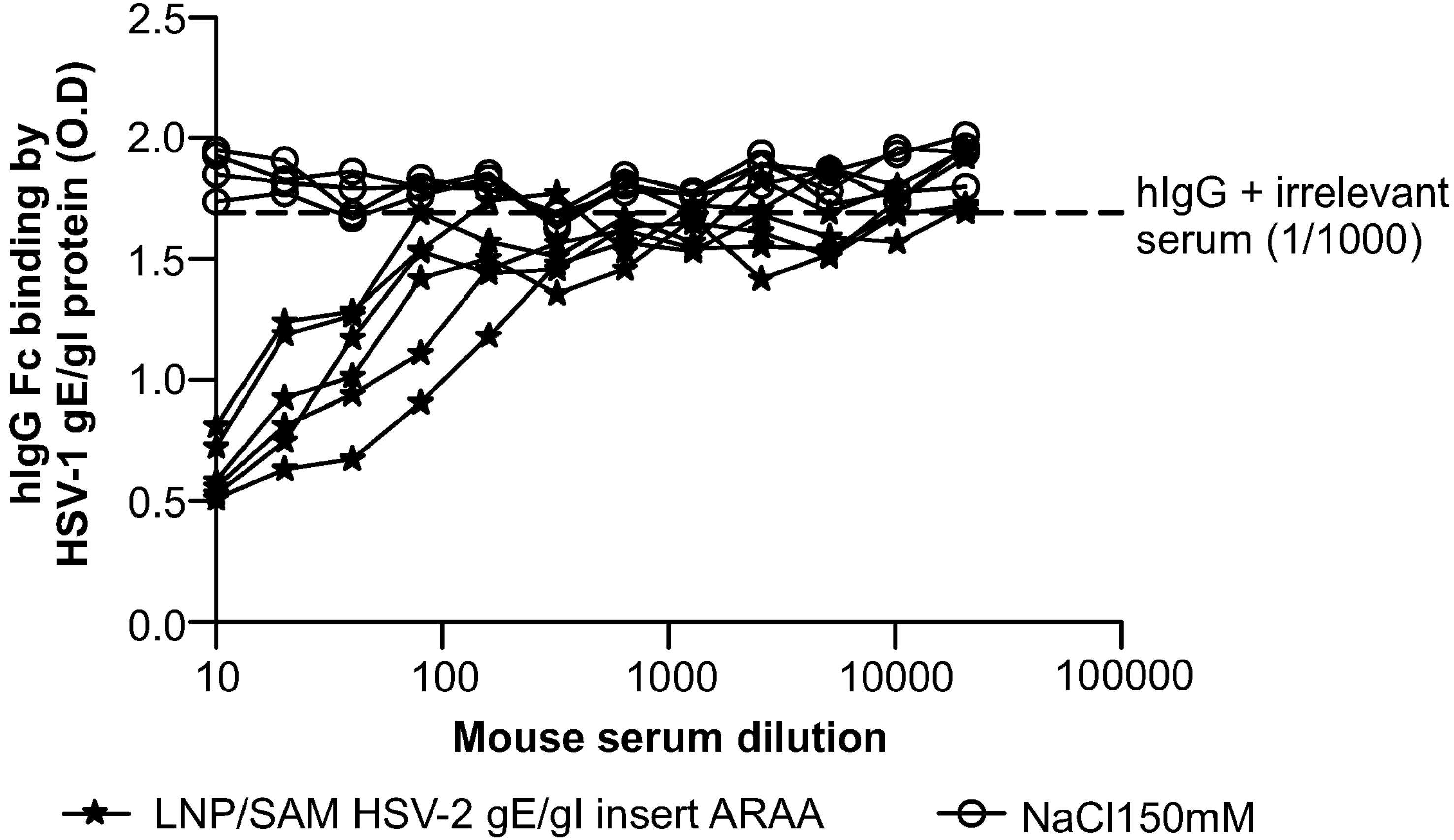

FIG. 74A

Serum collected 14 days post third immunization

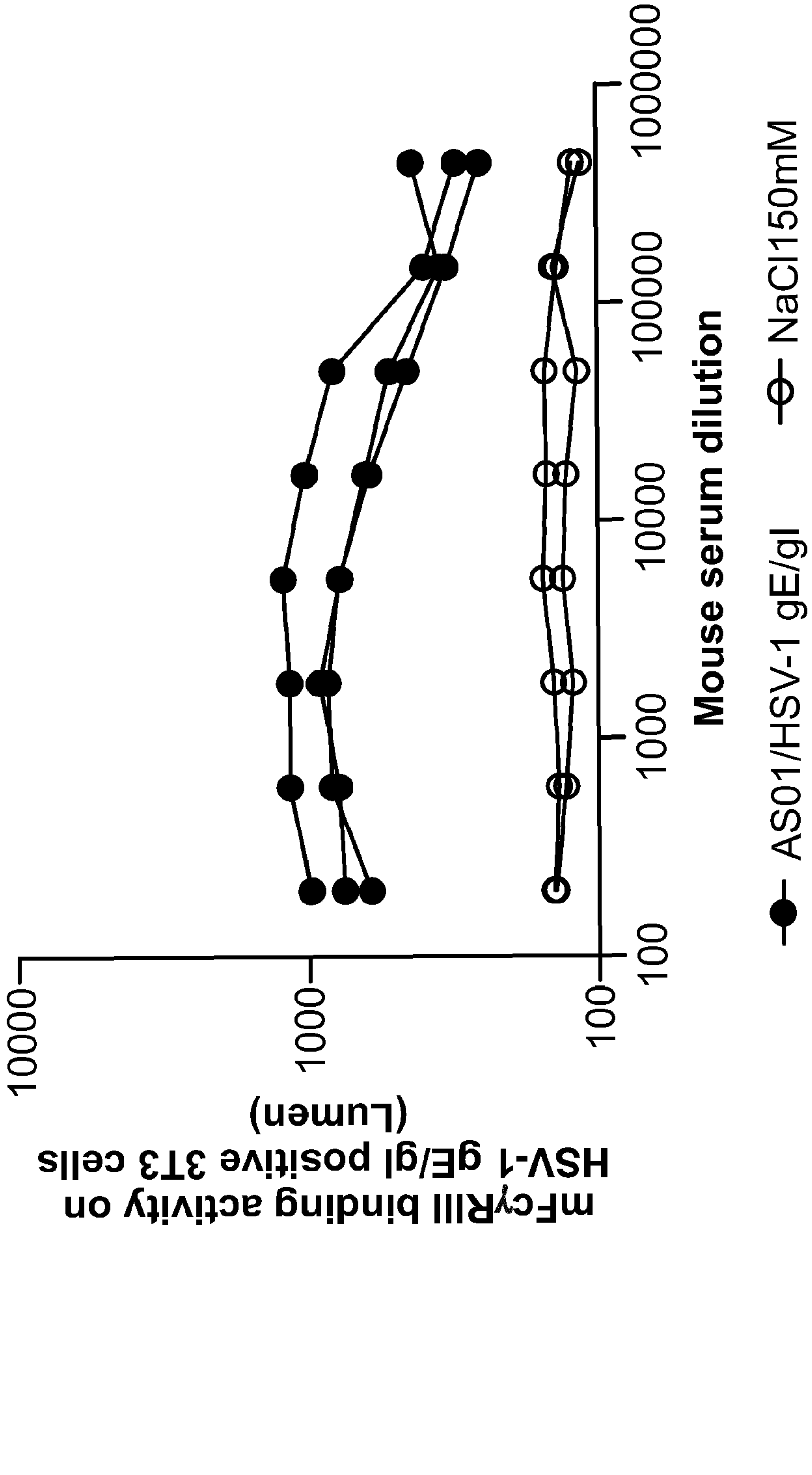

FIG. 74B

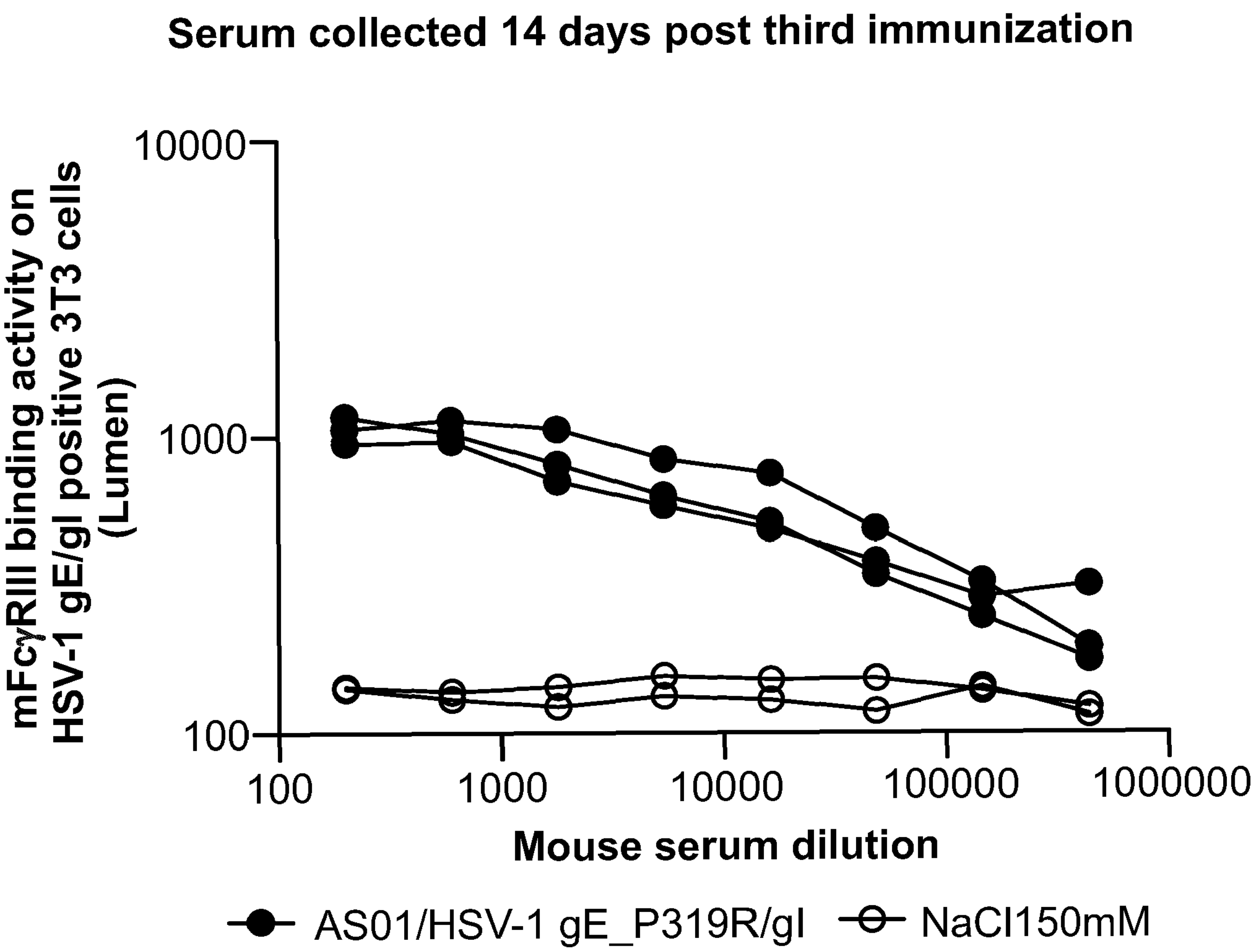
Serum collected 14 days post third immunization
mFcγRIII binding activity on HSV-1 gE/gI positive 3T3 cells (Lumen)
10000
1000
100
100
1000
10000
100000
1000000
Mouse serum dilution
AS01/HSV-1 gE_P319R/gI
NaCl150mM

FIG. 74C

Serum collected 14 days post third immunization

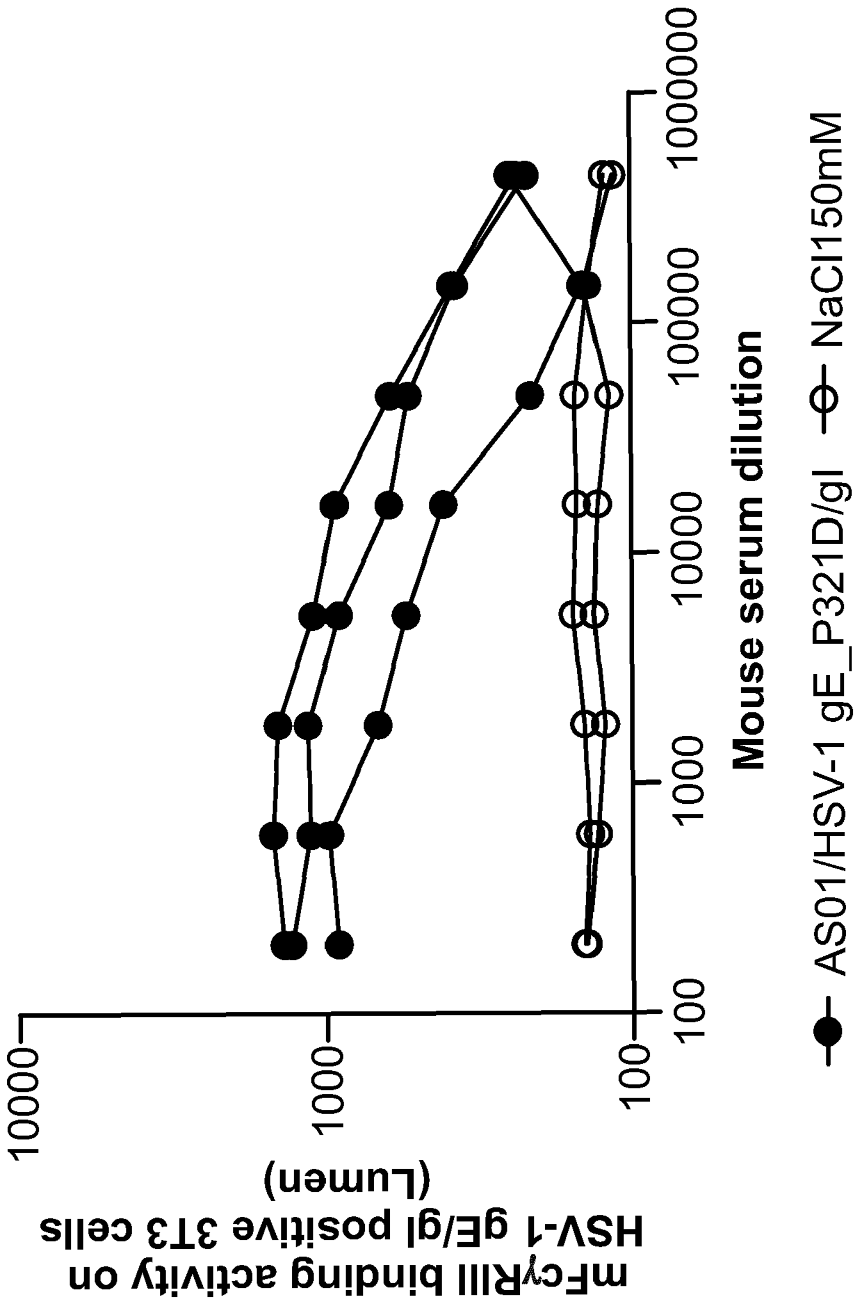

# FIG. 74D

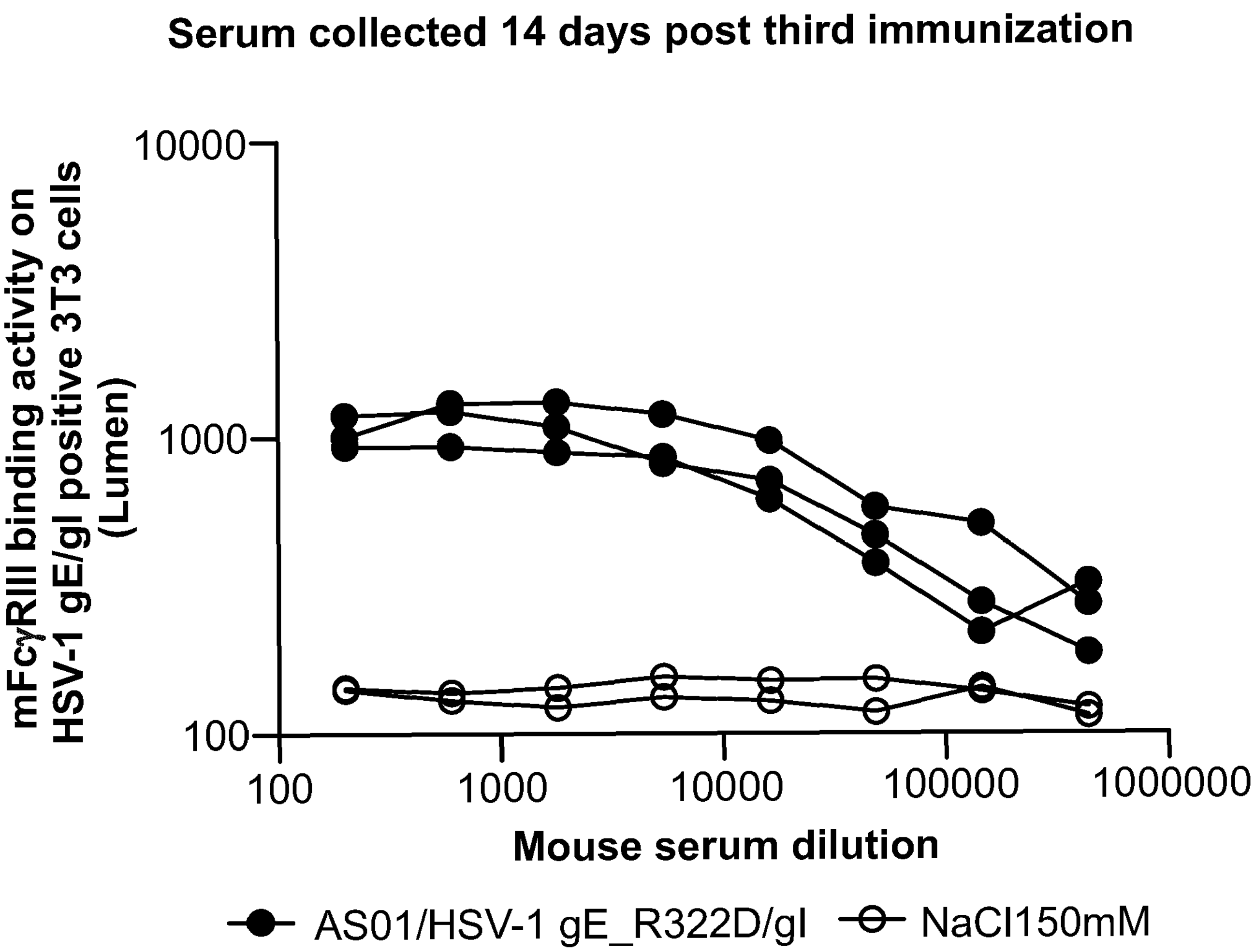
Serum collected 14 days post third immunization
mFcγRIII binding activity on HSV-1 gE/gI positive 3T3 cells (Lumen)
10000
1000
100
100
1000
10000
100000
1000000
Mouse serum dilution
AS01/HSV-1 gE_R322D/gI
NaCl150mM

FIG. 74E

Serum collected 14 days post third immunization

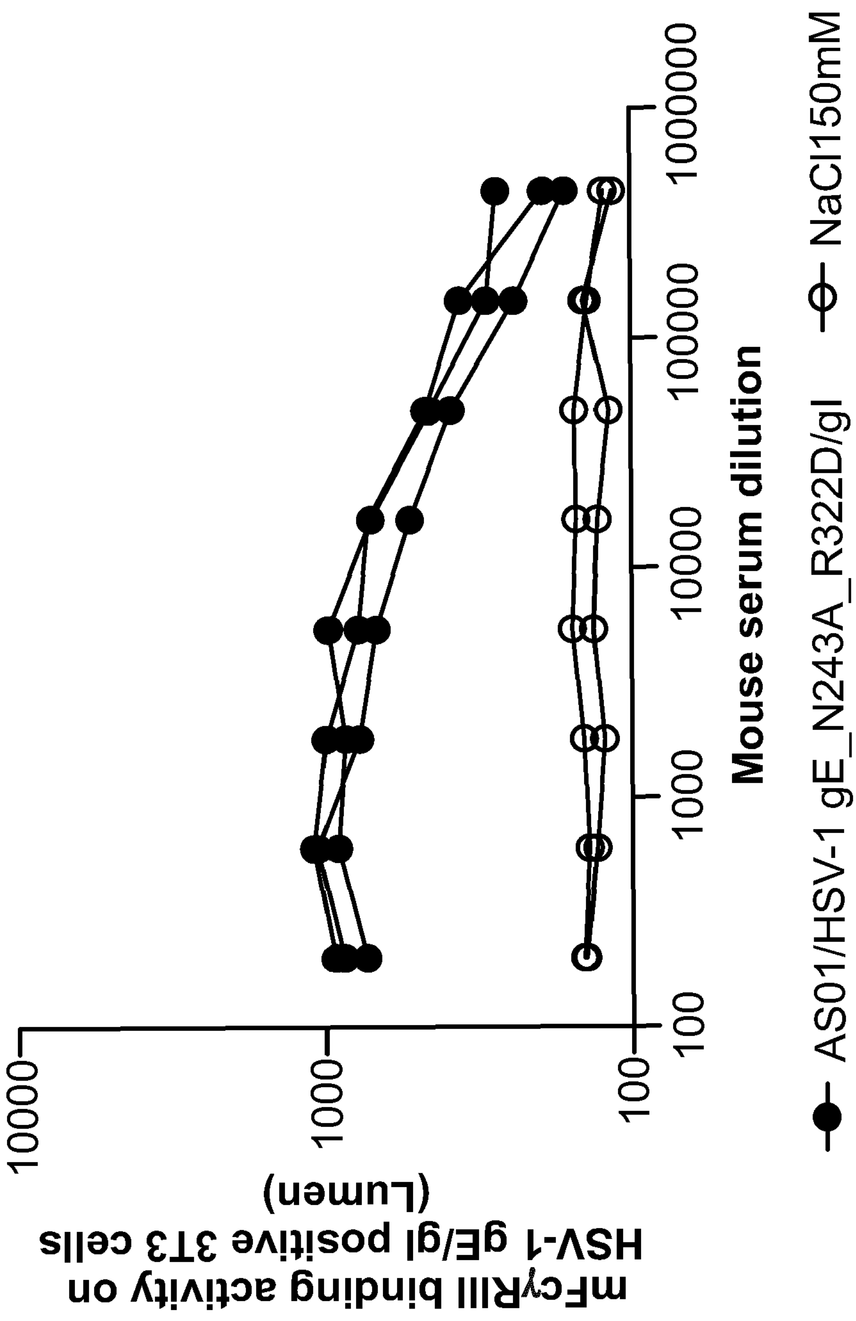

FIG. 74F

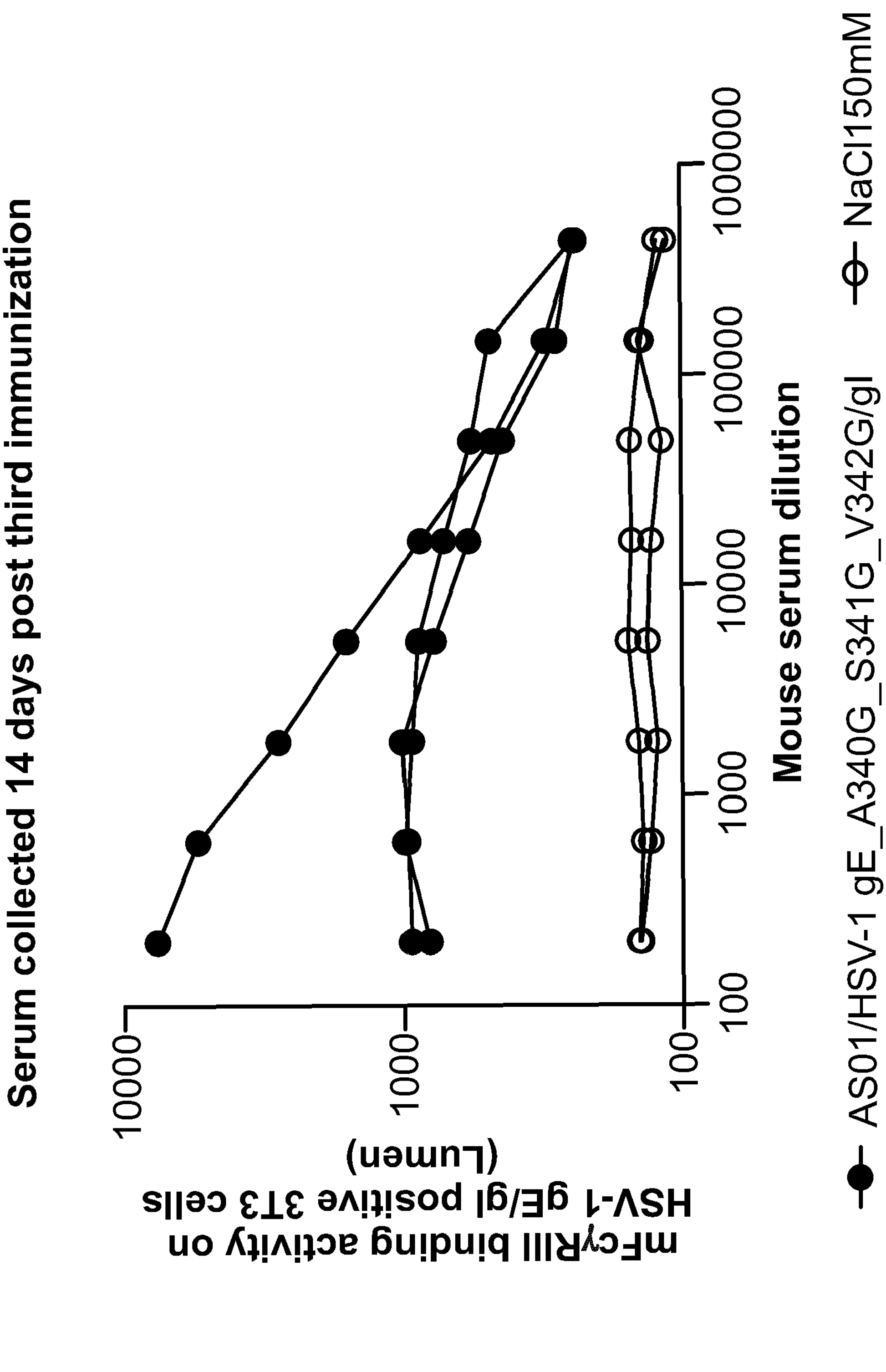
Serum collected 14 days post third immunization
mFcγRIII binding activity on HSV-1 gE/gI positive 3T3 cells (Lumen)
10000
1000
100
100
1000
10000
100000
1000000
Mouse serum dilution
AS01/HSV-1 gE_A340G_S341G_V342G/gI
NaCl150mM

FIG. 74G

Serum collected 14 days post third immunization

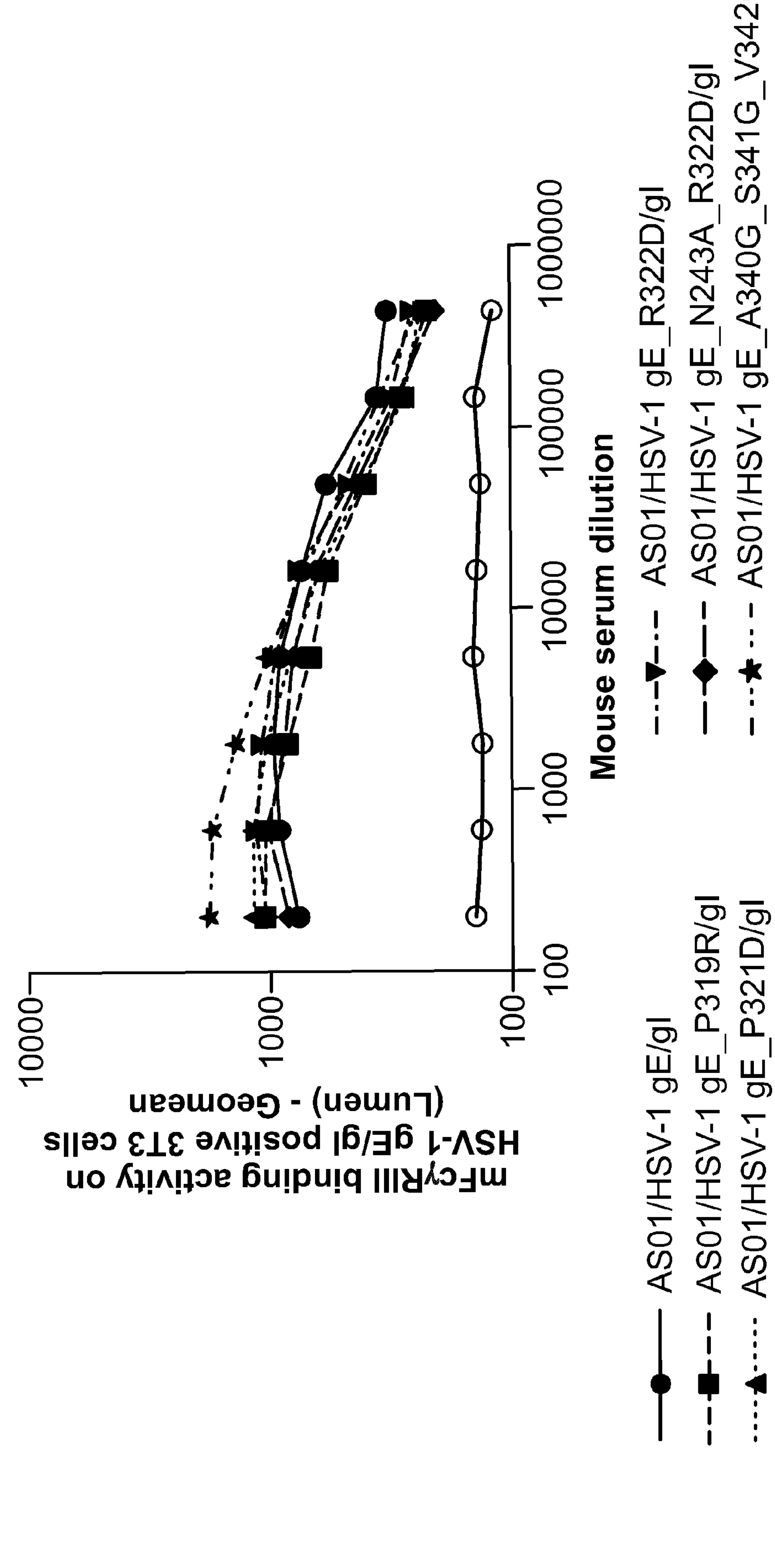

FIG. 75

```
HSV2  1     MARGAGLVFFVGVWVVSCLAAAPRTSWKRVTSGEDVVLLPAPAGPEERTR  50
            | |||  . | .|| ||||||  |:|||:||. |||| |||||  ||  |  
HSV1  1     MDRGAVVGFLLGVCVVSCLAGTPKTSWRRVSVGEDVSLLPAP.GPTGRGP  49

HSV2  51    AHKLLWAAEPLDACGPLRPSWVALWPPRRVLETVVDAACMRAPEPLAIAY  100
              ||||| |||| |||| ||||.| ||:.| |||||||||||| |||.||
HSV1  50    TQKLLWAVEPLDGCGPLHPSWVSLMPPKQVPETVVDAACMRAPVPLAMAY  99

HSV2  101   SPPFPAGDEGLYSELAWRDRVAVVNESLVIYGALETDSGLYTLSVVGLSD  150
            .|| |.   || .:  |.:| |||| ||||:|  |||||||||||  : |
HSV1  100   APPAPSATGGLRTDFVWQERAAVVNRSLVIHGVRETDSGLYTLSVGDIKD  149

HSV2  151   EARQVASVVLVVEPAPVPTP..TPDDYDEED.DAGVSER...TPVSVPPP  194
             |||||||||||:|||||||  || ||||:| | |  |   || |  |  
HSV1  150   PARQVASVVLVVQPAPVPTPPPTPADYDEDDNDEGEDESLAGTPASGTPR  199

HSV2  195   TPPRRPPVAPPTHPRVIPEVSHVRGVTVHMETPEAILFAPGETFGTNVSI  244
             ||  ||  | . |  ||||||||||| |||||||||.||||| |||||
HSV1  200   LPP..PPAPPRSWPSA.PEVSHVRGVTVRMETPEAILFSPGETFSTNVSI  246

HSV2  245   HAIAHDDGPYAMDVVWMRFDVPSSCAEMRIYEACLYHPQLPECLSPADAP  294
            |||||||  |.|||||:|||||.|||||||||.|||||||||||||||||
HSV1  247   HAIAHDDQTYSMDVVWLRFDVPTSCAEMRIYESCLYHPQLPECLSPADAP  296
```

```
HSV2  295  CAVSSWAYRLAVRSYAGCSRTTPPPRCFAEARMEPVPGLAWLASTVNLEF  344
           || |.|  ||||||||||||| ||||| ||| ||||||||| |..|||||
HSV1  297  CAASTWTSRLAVRSYAGCSRTNPPPRCSAEAHMEPVPGLAWQAASVNLEF  346

HSV2  345  QHASPQHAGLYLCVVYVDDHIHAWGHMTISTAAQYRNAVVEQHLPQRQPE  394
           . |||||.|||||||||.||||||||.||||||||||||||| ||||  :
HSV1  347  RDASPQHSGLYLCVVYVNDHIHAWGHITISTAAQYRNAVVEQPLPQRGAD  396

HSV2  395  PVEPTRPHVRAPPPAPSARGPLRLGAVLGAALLLAALGLSAWACMTCWRR  444
             ||| ||| ||| ||   | ||||||:||||||.||||| |||||||||
HSV1  397  LAEPTHPHVGAPPHAPPTHGALRLGAVMGAALLLSALGLSVWACMTCWRR  446

HSV2  445  RSWRAVKSRASATGPTYIRVADSELYADWSSDSEGERDGSLWQDPPERPD  494
           |.|||||||||  |||||||||||||||||||||||||   |  ||||||
HSV1  447  RAWRAVKSRASGKGPTYIRVADSELYADWSSDSEGERDQVPWLAPPERPD  496

HSV2  495  SPSTNGSGFEILSPTAPSVYPHSEGRKSRRPLTTFGSGSPGRRHSQASYS  544
           ||||||||||||||||||||| |:| .||| ||||||| | ||:|||| |
HSV1  497  SPSTNGSGFEILSPTAPSVYPRSDGHQSRRQLTTFGSGRPDRRYSQASDS  546

HSV2  545  SVLW  548
           || |
HSV1  547  SVFW  550
```

FIG. 75(contd)

**FIG. 76**

```
                  .         .         .         .         .
HSV2   1 MPGRSLQGLAILGLWVCATGLVVRGPTVSLVSDSLVDAGAVGPQGFVEED 50
         || | |||| :.||||||| ||||||||||||.| |||||.|| | ||||
HSV1   1 MPCRPLQGLVLVGLWVCATSLVVRGPTVSLVSNSFVDAGALGPDGVVEED 50
                  .         .         .         .         .

HSV2  51 LRVFGELHFVGAQVPHTNYYDGIIELFHYPLGNHCPRVVHVVTLTACPRR 100
         | : ||| ||| ||||| |||| :||.|||:|. |||||||||.||||||
HSV1  51 LLILGELRFVGDQVPHTTYYDGGVELWHYPMGHKCPRVVHVVTVTACPRR 100
                  .         .         .         .         .

HSV2 101 PAVAFTLCRSTHHAHSPAYPTLELGLARQPLLRVRTATRDYAGLYVLRVW 150
         ||||| |||.|   |||||||||| ||.||||||. |||||||.||||||
HSV1 101 PAVAFALCRATDSTHSPAYPTLELNLAQQPLLRVQRATRDYAGVYVLRVW 150
                  .         .         .         .         .

HSV2 151 VGSATNASLFVLGVALSANGTFVYNGSDYGSCDPAQLPFSAPRLGPSSVY 200
         || | ||||||||.|:.| ||  |||| ||||||  || ||||| |.|||
HSV1 151 VGDAPNASLFVLGMAIAAEGTLAYNGSAYGSCDPKLLPSSAPRLAPASVY 200
                  .         .         .         .         .

HSV2 201 TPGASRPTPPRTTTS.PSS....PRDPTPAPGDTGTPAPASGERAPP...    242
          |  .. . | |||| ||.    |    |||  . || |    :  |
HSV1 201 QPAPNQASTPSTTTSTPSTTIPAPSTTIPAPQASTTPFPTGDPKPQPPGV 250
                  .         .         .         .         .

HSV2 243 ......NSTRSASESRHRLTVAQVIQIAIPASIIAFVFLGSCICFIHRCQ 286
               |.||.  :||: ||| |:||||||||||| ||||||||||||||
HSV1 251 NHEPPSNATRATRDSRYALTVTQIIQIAIPASIIALVFLGSCICFIHRCQ 300
                  .         .         .         .         .

HSV2 287 RRYRRPRGQIYNPG...GVSCAVNEAAMARLGAELRSHPNTPPKPRRRSS 333
         ||||| |  ||.|    |:||||||||||||||||:|||.|||| ||| |
HSV1 301 RRYRRSRRPIYSPQMPTGISCAVNEAAMARLGAELKSHPSTPPKSRRR.S 349
                  .         .         .         .         .

HSV2 334 SSTTMPSLTSIAEESEPGPVVLLSVSPRPRSGPTAPQEV.....       372
         | | |||||.|||||||     |   | |   || |
HSV1 350 SRTPMPSLTAIAEESEPAGAAGL...PTPPVDPTTPTPTPPLLV       390
```

# FIG. 77

```
                      *        20         *
AKC59449.1 : MARGAGLVFFVGVWVVSCLAAAPRTSWKRVTSGEDVVL : 38
AKC42830.1 : MARGAGLVFFVGVWVVSCLAAAPRTSWKRVTSGEDVVL : 38
ABU45436.1 : MARGAGLVFFVGVWVVSCLAAAPRTSWKRVTSGEDVVL : 38
ABU45439.1 : MARGAGLVFFVGVWVVSCLAAAPRTSWKRVTSGEDVVL : 38
ABU45437.1 : MARGAGLVFFVGVWVVSCLAAAPRTSWKRVTSGEDVVL : 38
ABU45438.1 : MARGAGLVFFVGVWVVSCLAAAPRTSWKRVTSGEDVVL : 38
AMB66104.1 : MARGAGLVFFVGVWVVSCLAAAPRTSWKRVTSGEDVVL : 38
AMB66173.1 : MARGAGLVFFVGVWVVSCLAAAPRTSWKRVTSGEDVVV : 38
AMB66246.1 : MARGAGLVFFVGVWVVSCLAAAPRTSWKRVTSGEDVVL : 38
AKC59520.1 : MARGAGLVFFVGVWVVSCLAAAPRTSWKRVTSGEDVVL : 38
AKC59591.1 : MARGAGLVFFVGVWVVSCLAAAPRTSWKRVTSGEDVVL : 38
AKC59307.1 : MARGAGLVFFVGVWVVSCLAAAPRTSWKRVTSGEDVVL : 38
AMB66465.1 : MARGAGLVFFVGVWVVSCLAAAPRTSWKRVTSGEDVVL : 38
AKC59378.1 : MARGAGLVFFVGVWVVSCLAAAPRTSWKRVTSGEDVVL : 38
AEV91407.1 : MARGAGLVFFVGVWVVSCLAAAPRTSWKRVTSGEDVVL : 38
CAB06715.1 : MARGAGLVFFVGVWVVSCLAAAPRTSWKRVTSGEDVVL : 38
YP_0091372 : MARGAGLVFFVGVWVVSCLAAAPRTSWKRVTSGEDVVL : 38
ABW83306.1 : MARGAGLVFFVGVWVVSCLAAAPRTSWKRVTSGEDVVL : 38
ABW83324.1 : MARGAGLVFFVGVWVVSCLAAAPRTSWKRVTSGEDVVL : 38
ABW83308.1 : MARGAGLVFFVGVWVVSCLAAAPRTSWKRVTSGEDVVL : 38
ABW83310.1 : MARGAGLVFFVGVWVVSCLGAAPRTSWKRVTSGEDVVL : 38
ABW83312.1 : MARGAGLVFFVGVWVVSCLAAAPRTSWKRVTSGEDVVL : 38
ABW83314.1 : MARGAGLVFFVGVWVVSCLAAAPRTSWKRVTSGEDVVL : 38
ABW83316.1 : MARGAGLVFFVGVWVVSCLAAAPRTSWKRVTSGEDVVL : 38
ABW83318.1 : MARGAGLVFFVGVWVVSCLAAAPRTSWKRVTSGEDVVL : 38
ABW83320.1 : MARGAGLVFFVGVWVVSCLAAAPRTSWKRVTSGEDVVL : 38
ABW83322.1 : MARGAGLVFFVGVWVVSCLAAAPRTSWKRVTSGEDVVL : 38
ABW83398.1 : MARGAGLVFFVGVWVVSCLAAAPRTSWKRVTSGEDVVL : 38
ABW83380.1 : MARGAGLVFFVGVWVVSCLAAAPRTSWKRVTSGEDVVL : 38
ABW83396.1 : MARGAGLVFFVGVWVVSCLAAAPRTSWKRVTSGEDVVL : 38
ABW83382.1 : MARGAGLVFFVGVWVVSCLAAAPRTSWKRVTSGEDVVL : 38
ABW83384.1 : MARGAGLVFFVGVWVVSCLAAAPRTSWKRVTSGEDVVL : 38
ABW83394.1 : MARGAGLVFFVGVWVVSCLAAAPRTSWKRVTSGEDVVL : 38
ABW83386.1 : MARGAGLVFFVGVWVVSCLAAAPRTSWKRVTSGEDVVL : 38
ABW83388.1 : MARGAGLVFFVGVWVVSCLAAAPRTSWKRVTSGEDVVL : 38
ABW83390.1 : MARGAGLVFFVGVWVVSCLAAAPRTSWKRVTSGEDVVL : 38
ABW83392.1 : MARGAGLVFFVGVWVVSCLAAAPRTSWKRVTSGEDVVL : 38
ABW83400.1 : MARGAGLVFFVGVWVVSCLAAAPRTSWKRVTSGEDVVL : 38
AHG54732.1 : MARGAGLVFFVGVWVVSCLAAAPRTSWKRVTSGEDVVL : 38
ABW83342.1 : MARGAGLVFFVGVWVVSCLAAAPRTSWKRVTSGEDVVL : 38
ABW83340.1 : MARGAGLVFFVGVWVVSCLAAAPRTSWKRVTSGEDVVL : 38
ABW83346.1 : MARGAGLVFFVGVWVVSCLAAAPRTSWKRVTSGEDVVL : 38
ABW83348.1 : MARGAGLVFFVGVWVVSCLAAAPRTSWKRVTSGEDVVL : 38
ABW83326.1 : MARGAGLVFFVGVWVVSCLAAAPRTSWKRVTSGEDVVL : 38
ABW83350.1 : MARGAGLVFFVGVWVVSCLAAAPRTSWKRVTSGEDVVL : 38
ABW83352.1 : MARGAGLVFFVGVWVVSCLAAAPRTSWKRVTSGEDVVL : 38
ABW83336.1 : MARGAGLVFFVGVWVVSCLAAAPRTSWKRVTSGEDVVL : 38
ABW83334.1 : MARGAGLVFFVGVWVVSCLAAAPRTSWKRVTSGEDVVL : 38
ABW83354.1 : MARGAGLVFFVGVWVVSCLAAAPRTSWKRVTSGEDVVL : 38
ABW83338.1 : MARGAGLVFFVGVWVVSCLAAAPRTSWKRVTSGEDVVL : 38

                   40         *        60         *
AKC59449.1 : LPAPAGPEERTRAHKLLWAAEPLDACGPLRPSWVALWP : 76
AKC42830.1 : LPAPAGPEERTRAHKLLWAAEPLDACGPLRPSWVALWP : 76
ABU45436.1 : LPAPAGPEERTRAHKLLWAAEPLDACGPLRPSWVALWP : 76
ABU45439.1 : LPAPAGPEERTRAHKLLWAAEPLDACGPLRPSWVALWP : 76
ABU45437.1 : LPAPAGPEERTRAHKLLWAAEPLDACGPLRPSWVALWP : 76
```

FIG. 77(contd)

```
ABU45438.1 : LPAPAGPEERTRAHKLLWAAEPLDACGPLRPSWVALWP :  76
AMB66104.1 : LPAP---AERTRAHKLLWAAEPLDACGPLRPSWVALWP :  73
AMB66173.1 : LPAPAX---XXRAHKLLWAAEPXDACGPLRPSWXALWP :  73
AMB66246.1 : LPAPAGPEERTRAHKLLWAAEPLDACGPLRPSWVALWP :  76
AKC59520.1 : LPAPAGPEERTRAHKLLWAAEPLDACGPLRPSWVALWP :  76
AKC59591.1 : LPAPAGPEERTRAHKLLWAAEPLDACGPLRPSWVALWP :  76
AKC59307.1 : LPAPAGPEERTRAHKLLWAAEPLDACGPLRPSWVALWP :  76
AMB66465.1 : LPAPAGPEERTRAHKLLWAAEPLDACGPLRPSWVALWP :  76
AKC59378.1 : LPAPAGPEERTRAHKLLWAAEPLDACGPLRPSWVALWP :  76
AEV91407.1 : LPAP---AERTRAHKLLWAAEPLDACGPLRPSWVALWP :  73
CAB06715.1 : LPAP---AERTRAHKLLWAAEPLDACGPLRPSWVALWP :  73
YP_0091372 : LPAP---AERTRAHKLLWAAEPLDACGPLRPSWVALWP :  73
ABW83306.1 : LPAPAGPEERTRAHKLLWAAEPLDACGPLRPSWVALWP :  76
ABW83324.1 : LPAPAGPEERTRAHKLLWAAEPLDACGPLRPSWVALWP :  76
ABW83308.1 : LPAPAGPEERTRAHKLLWAAEPLDACGPLRPSWVALWP :  76
ABW83310.1 : LPAPAGPEERTRAHKLLWAAEPLDACGPLRPSWVALWP :  76
ABW83312.1 : LPAPAGPEERTRAHKLLWAAEPLDACGPLRPSWVALWP :  76
ABW83314.1 : LPAPAGPEERTRAHKLLWAAEPLDACGPLRPSWVALWP :  76
ABW83316.1 : LPAPAGPEERTRAHKLLWAAEPLDACGPLRPSWVALWP :  76
ABW83318.1 : LPAPAGPEERTRAHKLLWAAEPLDACGPLRPSWVALWP :  76
ABW83320.1 : LPAPAGPEERTRAHKLLWAAEPLDACGPLRPSWVALWP :  76
ABW83322.1 : LPAPAGPEERTRAHKLLWAAEPLDACGPLRPSWVALWP :  76
ABW83398.1 : LPAPAGPEERTRAHKLLWAAEPLDACGPLRPSWVALWP :  76
ABW83380.1 : LPAPAGPEERTRAHKLLWAAEPLDACGPLRPSWVALWP :  76
ABW83396.1 : LPAP---AERTRAHKLLWAAEPLDACGPLRPSWVALWP :  73
ABW83382.1 : LPAPAGPEERTRAHKLLWAAEPLDACGPLRPSWVALWP :  76
ABW83384.1 : LPAPAGPEERTRAHKLLWAAEPLDACGPLRPSWVALWP :  76
ABW83394.1 : LPAP---AERTRAHKLLWAAEPLDACGPLRPSWVALWP :  73
ABW83386.1 : LPAPAGPEERTRAHKLLWAAEPLDACGPLRPSWVALWP :  76
ABW83388.1 : LPAPAGPEERTRAHKLLWAAEPLDACGPLRPSWVALWP :  76
ABW83390.1 : LPAPAGPEERTRVHKLLWAAEPLDACGPLRPSWVALWP :  76
ABW83392.1 : LPAPAGPEERTRAHKLLWAAEPLDACGPLRPSWVALWP :  76
ABW83400.1 : LPAPAGPEERTRAHKLLWAAEPLDACGPLRPSWVALWP :  76
AHG54732.1 : LPAPAGPEERTRAHKLLWAAEPLDACGPLRPSWVALWP :  76
ABW83342.1 : LPAPAGPEERTRAHKLLWAAEPLDACGPLRPSWVALWP :  76
ABW83340.1 : LPAPAGPEERTRAHKLLWAAEPLDACGPLRPSWVALWP :  76
ABW83346.1 : LPAPAGPEERTRAHKLLWAAEPLDACGPLRPSWVALWP :  76
ABW83348.1 : LPAPAGPEERTRTHKLLWAAEPLDACGPLRPSWVALWP :  76
ABW83326.1 : LPAPAGPEERTRAHKLLWAAEPLDACGPLRPSWVALWP :  76
ABW83350.1 : LPAPAGPEERTRAHKLLWAAEPLDACGPLRPSWVALWP :  76
ABW83352.1 : LPAPAGPEERTRAHKLLWAAEPLDACGPLRPSWVALWP :  76
ABW83336.1 : LPAPTGPEERTRAHKLLWAAEPLDACGPLRPSWVALWP :  76
ABW83334.1 : LPAPTGPEERTRAHKLLWAAEPLDACGPLRPSWVALWP :  76
ABW83354.1 : LPAPAGPEERTRAHKLLWAAEPLDACGPLRPSWVALWP :  76
ABW83338.1 : LPAPAGPEERTRAHKLLWAAEPLDACGPLRPSWVALWP :  76

                      80         *        100         *
AKC59449.1 : PRRVLETVVDAACMRAPEPLAIAYSPPFPAGDEGLYSE : 114
AKC42830.1 : PRRVLETVVDAACMRAPEPLAIAYSPPFPAGDEGLYSE : 114
ABU45436.1 : PRRVLETVVDAACMRAPEPLAIAYSPPFPAGDEGLYSE : 114
ABU45439.1 : PRRVLETVVDAACMRAPEPLAIAYSPPFPAGDEGLYSE : 114
ABU45437.1 : PRRVLETVVDAACMRAPEPLAIAYSPPFPAGDEGLYSE : 114
ABU45438.1 : PRRVLETVVDAACMRAPEPLAIAYSPPFPAGDEGLYSE : 114
AMB66104.1 : PRRVLETVVDAACMRAPEPLAIAYSPPFPAGDEGLXSE : 111
AMB66173.1 : PRRVLETXXXXXXXXXXXXXXXXXXXXXXXXXXXXXXX : 111
AMB66246.1 : PRRVLETVVDAACMRAPEPLAIAYSPPFPAGDEGLYSE : 114
AKC59520.1 : PRRVLETVVDAACMRAPEPLAIAYSPPFPAGDEGLYSE : 114
AKC59591.1 : PRRVLETVVDAACMRAPEPLAIAYSPPFPAGDEGLYSE : 114
AKC59307.1 : PRRVLETVVDAACMRAPEPLAIAYSPPFPAGDEGLYSE : 114
AMB66465.1 : PRRVLETVVDAACMRAPEPLAIAYSPPFPAGDEGLYSE : 114
```

FIG. 77(contd)

```
AKC59378.1 : PRRVLETVVDAACMRAPEPLAIAYSPPFPAGDEGLYSE : 114
AEV91407.1 : PRRVLETVVDAACMRAPEPLAIAYSPPFPAGDEGLYSE : 111
CAB06715.1 : PRRVLETVVDAACMRAPEPLAIAYSPPFPAGDEGLYSE : 111
YP_0091372 : PRRVLETVVDAACMRAPEPLAIAYSPPFPAGDEGLYSE : 111
ABW83306.1 : PRRVLETVVDAACMRAPEPLAIAYSPPFPAGDEGLYSE : 114
ABW83324.1 : PRRVLETVVDAACMRAPEPLAIAYSPPFPAGDEGLYSE : 114
ABW83308.1 : PRRVLETVVDAACMRAPEPLAIAYSPPFPAGDEGLYSE : 114
ABW83310.1 : PRRVLETVVDAACMRAPEPLAIAYSPPFPAGDEGLYSE : 114
ABW83312.1 : PRRVLETVVDAACMRAPEPLAIAYSPPFPAGDEGLYSE : 114
ABW83314.1 : PRRVLETVVDAACMRAPEPLAIAYSPPFPAGDEGLYSE : 114
ABW83316.1 : PRRVLETVVDAACMRAPEPLAIAYSPPFPAGDEGLYSE : 114
ABW83318.1 : PRRVLETVVDAACMRAPEPLAIAYSPPFPAGDEGLYSE : 114
ABW83320.1 : PRRVLETVVDAACMRAPEPLAIAYSPPFPAGDEGLYSE : 114
ABW83322.1 : PRRVLETVVDAACMRAPEPLAIAYSPPFPAGDEGLYSE : 114
ABW83398.1 : PRRVLETVVDAACMRAPEPLAIAYSPPFPAGDEGLYSE : 114
ABW83380.1 : PRRVLETVVDAACMRAPEPLAIAYSPPFPAGDEGLYSE : 114
ABW83396.1 : PRRVLETVVDAACMRAPEPLAIAYSPPFPAGDEGLYSE : 111
ABW83382.1 : PRRVLETVVDAACMRAPEPLAIAYSPPFPAGDEGLYSE : 114
ABW83384.1 : PRRVLETVVDAACMRAPEPLAIAYSPPFPAGDEGLYSE : 114
ABW83394.1 : PRRVLETVVDAACMRAPEPLAIAYSPPFPAGDEGLYSE : 111
ABW83386.1 : PRRVLETVVDAACMRAPEPLAIAYSPPFPAGDEGLYSE : 114
ABW83388.1 : PRRVLETVVDAACMRAPEPLAIAYSPPFPAGDEGLYSE : 114
ABW83390.1 : PRRVLETVVDAACMRAPEPLAIAYSPPFPAGDEGLYSE : 114
ABW83392.1 : PRRVLETVVDAACMRAPEPLAIAYSPPFPAGDEGLYSE : 114
ABW83400.1 : PRRVLETVVDAACMRAPEPLAIAYSPPFPAGDEGLYSE : 114
AHG54732.1 : PRRVLETVVDAACMRAPEPLAIAYSPPFPAGDEGLYSE : 114
ABW83342.1 : PRRVLETVVDAACMRAPEPLAIAYSPPFPAGDEGLYSE : 114
ABW83340.1 : PRRVLETVVDAACMRAPEPLAIAYSPPFPAGDEGLYSE : 114
ABW83346.1 : PRRVLETVVDAACMRAPEPLAIAYSPPFPAGDEGLYSE : 114
ABW83348.1 : PRRVLETVVDAACMRAPEPLAIAYSPPFPAGDEGLYSE : 114
ABW83326.1 : PRRVLETVVDAACMRAPEPLAIAYSPPFPAGDEGLYSE : 114
ABW83350.1 : PRRVLETVVDAACMRAPEPLAIAYSPPFPAGDEGLYSE : 114
ABW83352.1 : PRRVLETVVDAACMRAPEPLAIAYSPPFPAGDEGLYSE : 114
ABW83336.1 : PRRVLETVVDAACMRAPEPLAIAYSPPFPAGDEGLYSE : 114
ABW83334.1 : PRRVLETVVDAACMRAPEPLAIAYSPPFPAGDEGLYSE : 114
ABW83354.1 : PRRVLETVVDAACMRAPEPLAIAYSPPFPAGDEGLYSE : 114
ABW83338.1 : PRRVLETVVDAACMRAPEPLAIAYSPPFPAGDEGLYSE : 114


                      120         *         140         *
AKC59449.1 : LAWRDRVAVVNESLVIYGALETDSGLYTLSVVGLSDEA : 152
AKC42830.1 : LAWRDRVAVVNESLVIYGALETDSGLYTLSVVGLSDEA : 152
ABU45436.1 : LAWRDRVAVVNESLVIYGALETDSGLYTLSVVGLSDEA : 152
ABU45439.1 : LAWRDRVAVVNESLVIYGALETDSGLYTLSVVGLSDEA : 152
ABU45437.1 : LAWRDRVAVVNESLVIYGALETDSGLYTLSVVGLSDEA : 152
ABU45438.1 : LAWRDRVAVVNESLVIYGALETDSGLYTLSVVGLSDEA : 152
AMB66104.1 : LAWRDRVAVVNESLVIYGALETDSGLYTLSVVGLXXXX : 149
AMB66173.1 : XXXXXXXXXXXXXXXXXXXXXXXXXXXXXXXXXXXXXX : 149
AMB66246.1 : LAWRDRVAVVNESLVIYGALETDSGLYTLSVVGLSDEA : 152
AKC59520.1 : LAWRDRVAVVNESLVIYGALETDSGLYTLSVVGLSDKA : 152
AKC59591.1 : LAWRDRVAVVNESLVIYGALETDSGLYTLSVVGLSDEA : 152
AKC59307.1 : LAWRDRVAVVNESLVIYGALETDSGLYTLSVVGLSDKA : 152
AMB66465.1 : LAWRDXVAVVNESLVIYGALETDSGLXXXXXXXXXXXX : 152
AKC59378.1 : LAWRDRVAVVNESLVIYGALETDSGLYTLSVVGLSDEA : 152
AEV91407.1 : LAWRDRVAVVNESLVIYGALETDSGLYTLSVVGLSDEA : 149
CAB06715.1 : LAWRDRVAVVNESLVIYGALETDSGLYTLSVVGLSDEA : 149
YP_0091372 : LAWRDRVAVVNESLVIYGALETDSGLYTLSVVGLSDEA : 149
ABW83306.1 : LAWRDRVAVVNESLVIYGALETDSGLYTLSVVGLSDEA : 152
ABW83324.1 : LAWRDRVAVVNESLVIYGALETDSGLYTLSVVGLSDEA : 152
ABW83308.1 : LAWRDRVAVVNESLVIYGALETDSGLYTLSVVGLSDEA : 152
ABW83310.1 : LAWRDRVAVVNESLVIYGALETDSGLYTLSVVGLSDEA : 152
```

FIG. 77(contd)

```
ABW83312.1 : LAWRDRVAVVNESLVIYGALETDSGLYTLSVVGLSDEA : 152
ABW83314.1 : LAWRDRVAVVNESLVIYGALETDSGLYTLSVVGLSDEA : 152
ABW83316.1 : LAWRDRVAVVNESLVIYGALETDSGLYTLSVVGLSDEA : 152
ABW83318.1 : LAWRDRVAVVNESLVIYGALETDSGLYTLSVVGLSDEA : 152
ABW83320.1 : LAWRDRVAVVNESLVIYGALETDSGLYTLSVVGLSDEA : 152
ABW83322.1 : LAWRDRVAVVNESLVIYGALDTDSGLYTLSVVGLSDEA : 152
ABW83398.1 : LAWRDRVAVVNESLVIYGALETDSGLYTLSVVGLSDEA : 152
ABW83380.1 : LAWRDRVAVVNESLVIYGARETDSGLYTLSVVGLSDEA : 152
ABW83396.1 : LAWRDRVAVVNESLVIYGALETDSGLYTLSVVGLSDEA : 149
ABW83382.1 : LAWRDRVAVVNESLVIYGALETDSGLYTLSVVGLSDEA : 152
ABW83384.1 : LAWRDRVAVVNESLVIYGALETDSGLYTLSVVGLSDEA : 152
ABW83394.1 : LAWRDRVAVVNESLVIYGALETDSGLYTLSVVGLSDEA : 149
ABW83386.1 : LAWRDRVAVVNESLVIYGALETDSGLYTLSVVGLSDEA : 152
ABW83388.1 : LAWRDRVAVVNESLVIYGALETDSGLYTLSVVGLSDEA : 152
ABW83390.1 : LAWRDRVAVVNESLVIYGALETDSGLYTLSVVGLSDEA : 152
ABW83392.1 : LAWRDRVAVVNESLVIYGALETDSGLYTLSVVGLSDEA : 152
ABW83400.1 : LAWRDRVAVVNESLVIYGALETDSGLYTLSVVGLSDEA : 152
AHG54732.1 : LAWRDRVAVVNESLVIYGALETDSGLYTLSVVGLSDEA : 152
ABW83342.1 : LAWRDRVAVVNESLVIYGARETDSGLYTLSVVGLSDEA : 152
ABW83340.1 : LAWRDRVAVVNESLVIYGARETDSGLYTLSVVGLSDEA : 152
ABW83346.1 : LAWRDRVAVVNESLVIYGARETDSGLYTLSVVGLSDEA : 152
ABW83348.1 : LAWRDRVAVVNESLVIYGALETDSGLYTLSVVGLSDEA : 152
ABW83326.1 : LAWRDRVAVVNESLVIYGALETDSGLYTLSVVGLSDEA : 152
ABW83350.1 : LAWRDRVAVVNESLVIYGALETDSGLYTLSVVGLSDEA : 152
ABW83352.1 : LAWRDRVAVVNESLVIYGALETDSGLYTLSVVGLSDEA : 152
ABW83336.1 : LACRDRVAVVNESLVIYGARETDSGLYTLSVVGLSDEA : 152
ABW83334.1 : LACRDRVAVVNESLVIYGARETDSGLYTLSVVGLSDEA : 152
ABW83354.1 : LAWRDRVAVVNESLVIYGARETDSGLYTLSVVGLSDEA : 152
ABW83338.1 : LAWRDRVAVVNESLVIYGARETDSGLYTLSVVGLSDEA : 152

                      160         *        180        *
AKC59449.1 : RQVASVVLVVEPAPVPTPTPDDYDEEDDAGVSERTPVS : 190
AKC42830.1 : RQVASVVLVVEPAPVPTPTPDDYDEEDDAGVSERTPVS : 190
ABU45436.1 : RQVASVVLVVEPAPVPTPTPDDYDEEDDAGVSERTPVS : 190
ABU45439.1 : RQVASVVLVVEPAPVPTPTPDDYDEEDDAGVSERTPVS : 190
ABU45437.1 : RQVASVVLVVEPAPVPTPTPDDYDEEDDAGVSERTPVS : 190
ABU45438.1 : RQVASVVLVVEPAPVPTPTPDDYDEEDDAGVSERTPVS : 190
AMB66104.1 : XXXXXXXXXXXXXXXXXXXXXXXXXXXXXXXXXXXXXX : 187
AMB66173.1 : XXXXXXXXXXXXXXXXXXXXXXXXXXXXXXXXXXXXXX : 187
AMB66246.1 : RQVASVVLVVEPAPVPTPTPDDYDEEDDXXXXXXXXXX : 190
AKC59520.1 : RQVASVVLVVEPAPVPTPTPDDYDEEDDAGVSERTPVS : 190
AKC59591.1 : RQVASVVLVVEPAPVPTPTPDDYDEEDDAGVSERTPVS : 190
AKC59307.1 : RQVASVVLVVEPAPVPTPTPDDYDEEDDAGVSERTPVS : 190
AMB66465.1 : XXXXXXXXXXXXXXXXXXXXXXXXXXXXXXXXXXXXXX : 190
AKC59378.1 : RQVASVVLVVEPAPVPTPTPDDYDEEDDAGVSERTPVS : 190
AEV91407.1 : RQVASVVLVVEPAPVPTPTPDDYDEEDDAGVSERTPVS : 187
CAB06715.1 : RQVASVVLVVEPAPVPTPTPDDYDEEDDAGVTNARRSA : 187
YP_0091372 : RQVASVVLVVEPAPVPTPTPDDYDEEDDAGVSERTPVS : 187
ABW83306.1 : RQVASVVLVVEPAPVPTPTPDDYDEEDDAGVSERTPVS : 190
ABW83324.1 : RQVASVVLVVEPAPVPTPTPDDYDEEDDAGVSERTPVS : 190
ABW83308.1 : RQVASVVLVVEPAPVPTPTPDDYDEEDDAGVSERTPVS : 190
ABW83310.1 : RQVASVVLVVEPAPVPTPTPDDYDEEDDAGVSERTPVS : 190
ABW83312.1 : RQVASVVLVVEPAPVPTPTPDDYDEEDDAGVSERTPVS : 190
ABW83314.1 : RQVASVVLVVEPAPVPTPTPDDYDEEDDAGVSERTPVS : 190
ABW83316.1 : RQVASVVLVVEPAPVPTPTPDDYDEEDDAGVSERTPVS : 190
ABW83318.1 : RQVASVVLVVEPAPVPTPTPDDYDEEDDAGVSERTPVS : 190
ABW83320.1 : RQVASVVLVVEPAPVPTPTPDDYDEEDDAGVSERTPVS : 190
ABW83322.1 : RQVASVVLVVEPAPVPTPTPDDYDEEDDAGVSERTPVS : 190
ABW83398.1 : RQVASVVLVVEPAPVPTPTPDDYDEEDDAGVSERTPVS : 190
ABW83380.1 : RQVASVVLVVEPAPVPTPTPDDYDEEDDAGVSERTPVS : 190
```

FIG. 77(contd)

```
ABW83396.1 : RQVASVVLVVEPAPVPTPTPDDYDEEDDAGVSERTPVS : 187
ABW83382.1 : RQVASVVLVVEPAPVPTPTPDDYDEEDDAGVSERTPVS : 190
ABW83384.1 : RQVASVVLVVEPAPVPTPTPDDYDEEDDAGVSERTPVS : 190
ABW83394.1 : RQVASVVLVVEPAPVPTPTPDDYDEEDDAGVSERTPVS : 187
ABW83386.1 : RQVASVVLVVEPAPVPTPTPDDYDEEDDAGVSERTPVS : 190
ABW83388.1 : RQVASVVLVVEPAPVPTPTPDDYDEEDDAGVSERTPVS : 190
ABW83390.1 : RQVASVVLVVEPAPVPTPTPDDYDEEDDAGVSERTPVS : 190
ABW83392.1 : RQVASVVLVVEPAPVPTPTPDDYDEEDDAGVSERTPVS : 190
ABW83400.1 : RQVASVVLVVEPAPVPTPTPDDYDEEDDAGVSERTLVS : 190
AHG54732.1 : RQVASVVLVVEPAPVPTPTPDDYDEEDDAGVSERTPVS : 190
ABW83342.1 : RQVASVVLVVEPAPVPTPTPDDYDEEDDAGVSERTPVS : 190
ABW83340.1 : RQVASVVLVVEPAPVPTPTPDDYDEEDDAGVSERTPVS : 190
ABW83346.1 : RQVASVVLVVEPAPVPTPTPDDYDEEDDAGVSERTPVS : 190
ABW83348.1 : RQVASVVLVVEPAPVPTPTPDDYDEEDDAGVSERTPVS : 190
ABW83326.1 : RQVASVVLVVEPAPVPTPTPDDYDEEDDAGVSERTPVS : 190
ABW83350.1 : RQVASVVLVVEPAPVPTPTPDDYDEEDDAGVSERTPVS : 190
ABW83352.1 : RQVASVVLVVEPAPVPTPTPDDYDEEDDAGVSERTPVS : 190
ABW83336.1 : RQVASVVLVVEPAPVPTPTPDDYDEEDDAGVSERTPVS : 190
ABW83334.1 : RQVASVVLVVEPAPVPTPTPDDYDEEDDAGVSERTPVS : 190
ABW83354.1 : RQVASVVLVVEPAPVPTPTPDDYDEEDDAGVSERTPVS : 190
ABW83338.1 : RQVASVVLVVEPAPVPTPTPDDYDEEDDAGVSERTPVS : 190

                                   200         *        220
AKC59449.1 : VPPATPPRRPPVAPPTHPRVIPEVSHVRGVTVHMETPE : 228
AKC42830.1 : VPPPTPPRRPPVAPPTHPRVIPEVSHVRGVTVHMETPE : 228
ABU45436.1 : VPPPTPPRRPPVAPPTHPRVIPEVSHVRGVTVHMETPE : 228
ABU45439.1 : VPPPTPPRRPPVAPPTHPRVIPEVSHVRGVTVHMETPE : 228
ABU45437.1 : VPPPTPPRRPPVAPPTHPRVIPEVSHVRGVTVHMETPE : 228
ABU45438.1 : VPPPTPPRRPPVAPPTHPRVIPEVSHVRGVTVHMETPE : 228
AMB66104.1 : XXXXXXXXRPPVAPPTHPRVIPEVSHVRGVTVHMETPE : 225
AMB66173.1 : XXXXXXXXXXXXXXXXXXXVIPEVSHVRGVTVHMETPE : 225
AMB66246.1 : XXXXXXXXXXXXXXPPTHPRVIPEVSHVRGVTVHMETPE : 228
AKC59520.1 : VPPPTPPRRPPVAPPTHPRVIPEVSHVRGVTVHMETPE : 228
AKC59591.1 : VPPPTPPRRPPVAPPTHPRVIPEVSHVRGVTVHMETPE : 228
AKC59307.1 : VPPPTPPRRPPVAPPTHPRVIPEVSHVRGVTVHMETPE : 228
AMB66465.1 : XXXXXXXXRPPVAPPTHPRVIPEVSHVRGVTVHMETPE : 228
AKC59378.1 : VPPPTPPRRPPVAPPTHPRVIPEVSHVRGVTVHMETPE : 228
AEV91407.1 : VPPPTPPRRPPVAPPTHPRVIPEVSHVRGVTVHMETPE : 225
CAB06715.1 : FPPQPPPRRPPVAPPTHPRVIPEVSHVRGVTVHMETLE : 225
YP_0091372 : VPPPTPPRRPPVAPPTHPRVIPEVSHVRGVTVHMETPE : 225
ABW83306.1 : VPPPTPPRRPPVAPPTHPRVIPEVSHVRGVTVHMETPE : 228
ABW83324.1 : VPPPTPPRRPPVAPPTHPRVIPEVSHVRGVTVHMETPE : 228
ABW83308.1 : VPPATPPRRPPVAPPTHPRVIPEVSHVRGVTVHMETPE : 228
ABW83310.1 : VPPPTPPRRPPVAPPTHPRVIPEVSHVRGVTVHMETPE : 228
ABW83312.1 : VPPPTPPRRPPVAPPTHPRVIPEVSHVRGVTVHMETPE : 228
ABW83314.1 : VPPPTPPRRPPVAPPTHPRVIPEVSHVRGVTVHMETPE : 228
ABW83316.1 : VPPPTPPRRPPVAPPTHPRVIPEVSHVRGVTVHMETPE : 228
ABW83318.1 : VPPPTPPRRPPVAPPTHPRVIPEVSHVRGVTVHMETPE : 228
ABW83320.1 : VPPPTPPRRPPVAPPTHPRVIPEVSHVRGVTVHMETPE : 228
ABW83322.1 : VPPPTPPRRPPVAPPTHPRVIPEVSHVRGVTVHMETPE : 228
ABW83398.1 : VPPPTPPRRPPVAPPTHPRVIPEVSHVRGVTVHMETPE : 228
ABW83380.1 : VPPPTPPRRPPVAPPTHPRVIPEVSHVRGVTVHMETPE : 228
ABW83396.1 : VPPPTPPRRPPVAPPTHPRVIPEVSHVRGVTVHMETPE : 225
ABW83382.1 : VPPPTPPRRPPVAPPTHPRVIPEVSHVRGVTVHMETPE : 228
ABW83384.1 : VPPPTPPRRPPVAPPTHPRVIPEVSHVRGVTVHMETPE : 228
ABW83394.1 : VPPPTPPRRPPVAPPTHPRVIPEVSHVRGVTVHMETPE : 225
ABW83386.1 : VPPPTPPRRPPVAPPTHPRVIPEVSHVRGVTVHMETPE : 228
ABW83388.1 : VPPPTPPRRPPVAPPTHPRVIPEVSHVRGVTVHMETPE : 228
ABW83390.1 : VPPPTPPRRPPVAPPTHPRVIPEVSHVRGVTVHMETPE : 228
ABW83392.1 : VPPPTPPRRPPVAPPTHPRVIPEVSHVRGVTVHMETPE : 228
```

FIG. 77(contd)

```
ABW83400.1 : VPPPTPPRRPPVAPPTHPRVIPEVSHVRGVTVHMETPE : 228
AHG54732.1 : VPPPTPPRRPPVAPPTHPRVIPEVSHVRGVTVHMETPE : 228
ABW83342.1 : VPPTTPPRRPPVAPPTHPRVIPEVSHVRGVTVHMETPE : 228
ABW83340.1 : VPPTTPPRRPPVAPPTHPRVIPEVSHVRGVTVHMETPE : 228
ABW83346.1 : VPPPTPPRRPPVAPQTHPRVIPEVSHVRGVTVHMETPE : 228
ABW83348.1 : VPPATPPRRPPVAPPTHPRVIPEVSHVRGVTVHMETPE : 228
ABW83326.1 : VPPPTPPRRPPVAPPTHPRVIPEVSHVRGVTVHMETPE : 228
ABW83350.1 : VPPPTPPRRPPVAPPTHPRVIPEVSHVRGVTVHMETPE : 228
ABW83352.1 : VPPPTPPRRPPVAPPTHPRVIPEVSHVRGVTVHMETPE : 228
ABW83336.1 : VPPPTPPRRPPVAPQTHPRVIPEVSHVRGVTVHMETPE : 228
ABW83334.1 : VPPPTPPRRPPVAPQTHPRVIPEVSHVRGVTVHMETPE : 228
ABW83354.1 : VPPPTPPRRPPVAPQTHPRVIPEVSHVRGVTVHMETPE : 228
ABW83338.1 : VPPTTPPRRPPVAPPTHPRVIPEVSHVRGVTVHMETPE : 228

                     *        240         *        260
AKC59449.1 : AILFAPGETFGTNVSIHAIAHDDGPYAMDVVWMRFDVP : 266
AKC42830.1 : AILFAPGETFGTNVSIHAIAHDDGPYAMDVVWMRFDVP : 266
ABU45436.1 : AILFAPGETFGTNVSIHAIAHDDGPYAMDVVWMRFDVP : 266
ABU45439.1 : AILFAPGETFGTNVSIHAIAHDDGPYAMDVVWMRFDVP : 266
ABU45437.1 : AILFAPGETFGTNVSIHAIAHDDGPYAMDVVWMRFDVP : 266
ABU45438.1 : AILFAPGETFGTNVSIHAIAHDDGPYAMDVVWMRFDVP : 266
AMB66104.1 : AILFAPGETFGTNVSIHAIAHDDGPYAMDVVWMRFDVP : 263
AMB66173.1 : AILFAPGETFGTNVSIHAIAHDDGPYAMDVVWMRFDVP : 263
AMB66246.1 : AILFAPGETFGTNVSIHAIAHDDGPYAMDVVWMRFDVP : 266
AKC59520.1 : AILFAPGETFGTNVSIHAIAHDDGPYAMDVVWMRFDVP : 266
AKC59591.1 : AILFAPGETFGTNVSIHAIAHDDGPYAMDVVWMRFDVP : 266
AKC59307.1 : AILFAPGETFGTNVSIHAIAHDDGPYAMDVVWMRFDVP : 266
AMB66465.1 : AILFAPGETFGTNVSIHAIAHDDGPYAMDVVWMRFDVP : 266
AKC59378.1 : AILFAPGETFGTNVSIHAIAHDDGPYAMDVVWMRFDVP : 266
AEV91407.1 : AILFAPGETFGTNVSIHAIAHDDGPYAMDVVWMRFDVP : 263
CAB06715.1 : AILFAPGETFGTNVSIHAIAHDDGPYAMDVVWMRFDVP : 263
YP_0091372 : AILFAPGETFGTNVSIHAIAHDDGPYAMDVVWMRFDVP : 263
ABW83306.1 : AILFAPGETFGTNVSIHAIAHDDGPYAMDVVWMRFDVP : 266
ABW83324.1 : AILFAPGETFGTNVSIHAIAHDDGPYAMDVVWMRFDVP : 266
ABW83308.1 : AILFAPGETFGTNVSIHAIAHDDGPYAMDVVWMRFDVP : 266
ABW83310.1 : AILFAPGETFGTNVSIHAIAHDDGPYAMDVVWMRFDVP : 266
ABW83312.1 : AILFAPGETFGTNVSIHAIAHDDGPYAMDVVWMRFDVP : 266
ABW83314.1 : AILFAPGETFGTNVSIHAIAHDDGPYAMDVVWMRFDVP : 266
ABW83316.1 : AILFAPGETFGTNVSIHAIAHDDGPYAMDVVWMRFDVP : 266
ABW83318.1 : AILFAPGETFGTNVSIHAIAHDDGPYAMDVVWMRFDVP : 266
ABW83320.1 : AILFAPGETFGTNVSIHAIAHDDGPYAMDVVWMRFDVP : 266
ABW83322.1 : AILFAPGETFGTNVSIHAIAHDDGPYAMDVVWMRFDVP : 266
ABW83398.1 : AILFAPGETFGTNVSIHAIAHDDGPYAMDVVWMRFDVP : 266
ABW83380.1 : AILFAPGETFGTNVSIHAIAHDDGPYAMDVVWMRFDVP : 266
ABW83396.1 : AILFAPGETFGTNVSIHAIAHDDGPYAMDVVWMRFDVP : 263
ABW83382.1 : AILFAPGETFGTNVSIHAIAHDDGPYAMDVVWMRFDVP : 266
ABW83384.1 : AILFAPGETFGTNVSIHAIAHDDGPYAMDVVWMRFDVP : 266
ABW83394.1 : AILFAPGETFGTNVSIHAIAHDDGPYAMDVVWMRFDVP : 263
ABW83386.1 : AILFAPGETFGTNVSIHAIAHDDGPYAMDVVWMRFDVP : 266
ABW83388.1 : AILFAPGETFGTNVSIHAIAHDDGPYAMDVVWMRFDVP : 266
ABW83390.1 : AILFAPGETFGTNVSIHAIAHDDGPYAMDVVWMRFDVP : 266
ABW83392.1 : AILFAPGETFGTNVSIHAIAHDDGPYAMDVVWMRFDVP : 266
ABW83400.1 : AILFAPGETFGTNVSIHAIAHDDGPYAMDVVWMRFDVP : 266
AHG54732.1 : AILFAPGETFGTNVSIHAIAHDDGPYAMDVVWMRFDVP : 266
ABW83342.1 : AILFAPGETFGTNVSIHAIAHDDGPYAMDVVWMRFDVP : 266
ABW83340.1 : AILFAPGETFGTNVSIHAIAHDDGPYAMDVVWMRFDVP : 266
ABW83346.1 : AILFAPGETFGTNVSIHAIAHDDGPYAMDVVWMRFDVP : 266
ABW83348.1 : AILFAPGETFGTNVSIHAIAHDDGPYAMDVVWMRFDVP : 266
ABW83326.1 : AILFAPGETFGTNVSIHAIAHDDGPYAMDVVWMRFDVP : 266
ABW83350.1 : AILFAPGETFGTNVSIHAIAHDDGPYAMDVVWMRFDVP : 266
```

FIG. 77(contd)

```
ABW83352.1 : AILFAPGETFGTNVSIHAIAHDDGPYAMDVVWMRFDVP : 266
ABW83336.1 : AILFAPGETFGTNVSIHAIAHDDGPYAMDVVWMRFDVP : 266
ABW83334.1 : AILFAPGETFGTNVSIHAIAHDDGPYAMDVVWMRFDVP : 266
ABW83354.1 : AILFAPGETFGTNVSIHAIAHDDGPYAMDVVWMRFDVP : 266
ABW83338.1 : AILFAPGETFGTNVSIHAIAHDDGPYAMDVVWMRFDVP : 266

                      *        280         *        300
AKC59449.1 : SSCAEMRIYEACLYHPQLPECLSPADAPCAVSSWAYRL : 304
AKC42830.1 : SSCAEMRIYEACLYHPQLPECLSPADAPCAVSSWAYRL : 304
ABU45436.1 : SSCAEMRIYEACLYHPQLPECLSPADAPCAVSSWAYRL : 304
ABU45439.1 : SSCAEMRIYEACLYHPQLPECLSPADAPCAVSSWAYRL : 304
ABU45437.1 : SSCAEMRIYEACLYHPQLPECLSPADAPCAVSSWAYRL : 304
ABU45438.1 : SSCAEMRIYEACLYHPQLPECLSPADAPCAVSSWAYRL : 304
AMB66104.1 : SSCAEMRIYEACLYHPQLPECLSPADAPCAVSSWAYRL : 301
AMB66173.1 : SSCAEMRIYEACLYHPQLPECLSPADAPCAVSSWAYRL : 301
AMB66246.1 : SSCAEMRIYEACLYHPQLPECLSPADAPCAVSSWAYRL : 304
AKC59520.1 : SSCAEMRIYEACLYHPQLPECLSPADAPCAVSSWAYRL : 304
AKC59591.1 : SSCAEMRIYEACLYHPQLPECLSPADAPCAVSSWAYRL : 304
AKC59307.1 : SSCAEMRIYEACLYHPQLPECLSPADAPCAVSSWAYRL : 304
AMB66465.1 : SSCAEMRIYEACLYHPQLPECLSPADAPCAVSSWAYRL : 304
AKC59378.1 : SSCAEMRIYEACLYHPQLPECLSPADAPCAVSSWAYRL : 304
AEV91407.1 : SSCAEMRIYEACLYHPQLPECLSPADAPCAVSSWAYRL : 301
CAB06715.1 : SSCADMRIYEACLYHPQLPECLSPADAPCAVSSWAYRL : 301
YP_0091372 : SSCAEMRIYEACLYHPQLPECLSPADAPCAVSSWAYRL : 301
ABW83306.1 : SSCAEMRIYEACLYHPQLPECLSPADAPCAVSSWAYRL : 304
ABW83324.1 : SSCAEMRIYEACLYHPQLPECLSPADAPCAVSSWAYRL : 304
ABW83308.1 : SSCAEMRIYEACLYHPQLPECLSPADAPCAVSSWAYRL : 304
ABW83310.1 : SSCAEMRIYEACLYHPQLPECLSPADAPCAVSSWAYRL : 304
ABW83312.1 : SSCAEMRIYEACLYHPQLPECLSPADAPCAVSSWAYRL : 304
ABW83314.1 : SSCAEMRIYEACLYHPQLPECLSPADAPCAVSSWAYRL : 304
ABW83316.1 : SSCAEMRIYEACLYHPQLPECLSPADAPCAVSSWAYRL : 304
ABW83318.1 : SSCAEMRIYEACLYHPQLPECLSPADAPCAVSSWAYRL : 304
ABW83320.1 : SSCAEMRIYEACLYHPQLPECLSPADAPCAVSSWAYRL : 304
ABW83322.1 : SSCAEMRIYEACLYHPQLPECLSPADAPCAVSSWAYRL : 304
ABW83398.1 : SSCAEMRIYEACLYHPQLPECLSPADAPCAVSSWAYRL : 304
ABW83380.1 : SSCAEMRIYEACLYHPQLPECLSPADAPCAVSSWAYRL : 304
ABW83396.1 : SSCAEMRIYEACLYHPQLPECLSPADAPCAVSSWAYRL : 301
ABW83382.1 : SSCAEMRIYEACLYHPQLPECLSPADAPCAVSSWAYRL : 304
ABW83384.1 : SSCAEMRIYEACLYHPQLPECLSPADAPCAVSSWAYRL : 304
ABW83394.1 : SSCAEMRIYEACLYHPQLPECLSPADAPCAVSSWAYRL : 301
ABW83386.1 : SSCAEMRIYEACLYHPQLPECLSPADAPCAVSSWAYRL : 304
ABW83388.1 : SSCAEMRIYEACLYHPQLPECLSPADAPCAVSSWAYRL : 304
ABW83390.1 : SSCAEMRIYEACLYHPQLPECLSPADAPCAVSSWAYRL : 304
ABW83392.1 : SSCAEMRIYEACLYHPQLPECLSPADAPCAVSSWAYRL : 304
ABW83400.1 : SSCAEMRIYEACLYHPQLPECLSPADAPCAVSSWAYRL : 304
AHG54732.1 : SSCAEMRIYEACLYHPQLPECLSPADAPCAVSSWAYRL : 304
ABW83342.1 : SSCAEMRIYEACLYHPQLPECLSPADAPCAVSSWAYRL : 304
ABW83340.1 : SSCAEMRIYEACLYHPQLPECLSPADAPCAVSSWAYRL : 304
ABW83346.1 : SSCAEMRIYEACLYHPQLPECLSPADAPCAVSSWAYRL : 304
ABW83348.1 : SSCAEMRIYEACLYHPQLPECLSPADAPCAVSSWAYRL : 304
ABW83326.1 : SSCAEMRIYEACLYHPQLPECLSPADAPCAVSSWAYRL : 304
ABW83350.1 : SSCAEMRIYEACLYHPQLPECLSPADAPCAVSSWAYRL : 304
ABW83352.1 : SSCAEMRIYEACLYHPQLPECLSPADAPCAVSSWAYRL : 304
ABW83336.1 : SSCAEMRIYEACLYHPQLPECLSPADAPCAVSSWAYRL : 304
ABW83334.1 : SSCAEMRIYEACLYHPQLPECLSPADAPCAVSSWAYRL : 304
ABW83354.1 : SSCAEMRIYEACLYHPQLPECLSPADAPCAVSSWAYRL : 304
ABW83338.1 : SSCAEMRIYEACLYHPQLPECLSPADAPCAVSSWAYRL : 304
```

FIG. 77(contd)

```
                              *        320          *        340
AKC59449.1 : AVRSYAGCSRTTPPPRCFAEARMEPVPGLAWLASTVNL : 342
AKC42830.1 : AVRSYAGCSRTTPPPRCFAEARMEPVPGLAWLASTVNL : 342
ABU45436.1 : AVRSYAGCSRTTPPPRCFAEARMEPVPGLAWLASTVNL : 342
ABU45439.1 : AVRSYAGCSRTTPPPRCFAEARMEPVPGLAWLASTVNL : 342
ABU45437.1 : AVRSYAGCSRTTPPPRCFAEARMEPVPGLAWLASTVNL : 342
ABU45438.1 : AVRSYAGCSRTTPPPRCFAEARMEPVPGLAWLASTVNL : 342
AMB66104.1 : AVRSYAGCSRTTPPPRCFAEARMEPVPGLAWLASTVNL : 339
AMB66173.1 : AVRSYAGCSRTTPPPRCFAEARMEPVPGLAWLASTVNL : 339
AMB66246.1 : AVRSYAGCSRTTPPPRCFAEARMEPVPGLAWLASTVNL : 342
AKC59520.1 : AVRSYAGCSRTTPPPRCFAEARMEPVPGLAWLASTVNL : 342
AKC59591.1 : AVRSYAGCSRTTPPPRCFAEARMEPVPGLAWLASTVNL : 342
AKC59307.1 : AVRSYAGCSRTTPPPRCFAEARMEPVPGLAWLASTVNL : 342
AMB66465.1 : AVRSYAGCSRTTPPPRCFAEARMEPVPGLAWLASTVNL : 342
AKC59378.1 : AVRSYAGCSRTTPPPRCFAEARMEPVPGLAWLASTVNL : 342
AEV91407.1 : AVRSYAGCSRTTPPPRCFAEARMEPVPGLAWLASTVNL : 339
CAB06715.1 : AVRSYAGCSRTTPPPRCFAEARMEPVPGLAWLASTVNL : 339
YP_0091372 : AVRSYAGCSRTTPPPRCFAEARMEPVPGLAWLASTVNL : 339
ABW83306.1 : AVRSYAGCSRTTPPPRCFAEARMEPVPGLAWLASTVNL : 342
ABW83324.1 : AVRSYAGCSRTTPPPRCFAEARMEPVPGLAWLASTVNL : 342
ABW83308.1 : AVRSYAGCSRTTPPPRCFAEARMEPVPGLAWLASTVNL : 342
ABW83310.1 : AVRSYAGCSRTTPPPRCFAEARMEPVPGLAWLASTVNL : 342
ABW83312.1 : AVRSYAGCSRTTPPPRCFAEARMEPVPGLAWLASTVNL : 342
ABW83314.1 : AVRSYAGCSRTTPPPRCFAEARMEPVPGLAWLASTVNL : 342
ABW83316.1 : AVRSYAGCSRTTPPPRCFAEARMEPVPGLAWLASTVNL : 342
ABW83318.1 : AVRSYAGCSRTTPPPRCFAEARMEPVPGLAWLASTVNL : 342
ABW83320.1 : AVRSYAGCSRTTPPPRCFAEARMEPVPGLAWLASTVNL : 342
ABW83322.1 : AVRSYAGCSRTTPPPRCFAEARMEPVPGLAWLASTVNL : 342
ABW83398.1 : AVRSYAGCSRTTPPPRCFAEARMEPVPGLAWLASTVNL : 342
ABW83380.1 : AVRSYAGCSRTTPPPRCFAEARMEPVPGLAWLASTVNL : 342
ABW83396.1 : AVRSYAGCSRTTPPPRCFAEARMEPVPGLAWLASTVNL : 339
ABW83382.1 : AVRSYAGCSRTTPPPRCFAEARMEPVPGLAWLASTVNL : 342
ABW83384.1 : AVRSYAGCSRTTPPPRCFAEARMEPVPGLAWLASTVNL : 342
ABW83394.1 : AVRSYAGCSRTTPPPRCFAEARMEPVPGLAWLASTVNL : 339
ABW83386.1 : AVRSYAGCSRTTPPPRCFAEARMEPVPGLAWLASTVNL : 342
ABW83388.1 : AVRSYAGCSRTTPPPRCFAEARMEPVPGLAWLASTVNL : 342
ABW83390.1 : AVRSYAGCSRTTPPPRCFAEARMEPVPGLAWLASTVNL : 342
ABW83392.1 : AVRSYAGCSRTTPPPRCFAEARMEPVPGLAWLASTVNL : 342
ABW83400.1 : AVRSYAGCSRTTPPPRCFAEARMEPVPGLAWLASTVNL : 342
AHG54732.1 : AVRSYAGCSRTTPPPRCFAEARMEPVPGLAWLASTVNL : 342
ABW83342.1 : AVRSYAGCSRTTPPPRCFAEARMEPVPGLAWLASTVNL : 342
ABW83340.1 : AVRSYAGCSRTTPPPRCFAEARMEPVPGLAWLASTVNL : 342
ABW83346.1 : AVRSYAGCSRTTPPPRCFAEARMEPVPGLAWLASTVNL : 342
ABW83348.1 : AVRSYAGCSRTTPPPRCFAEARMEPVPGLAWLASTVNL : 342
ABW83326.1 : AVRSYAGCSRTTPPPRCFAEARMEPVPGLAWLASTVNL : 342
ABW83350.1 : AVRSYAGCSRTTPPPRCFAEARMEPVPGLAWLASTVNL : 342
ABW83352.1 : AVRSYAGCSRTTPPPRCFAEARMEPVPGLAWLASTVNL : 342
ABW83336.1 : AVRSYAGCSRTTPPPRCFAEARMEPVPGLAWLASTVNL : 342
ABW83334.1 : AVRSYAGCSRTTPPPRCFAEARMEPVPGLAWLASTVNL : 342
ABW83354.1 : AVRSYAGCSRTTPPPRCFAEARMEPVPGLAWLASTVNL : 342
ABW83338.1 : AVRSYAGCSRTTPPPRCFAEARMEPVPGLAWLASTVNL : 342

                              *        360          *        380
AKC59449.1 : EFQHASPQHAGLYLCVVYVDDHIHAWGHMTISTAAQYR : 380
AKC42830.1 : EFQHASPQHAGLYLCVVYVDDHIHAWGHMTISTAAQYR : 380
ABU45436.1 : EFQHASPQHAGLYLCVVYVDDHIHAWGHMTISTAAQYR : 380
ABU45439.1 : EFQHASPQHAGLYLCVVYVDDHIHAWGHMTISTAAQYR : 380
ABU45437.1 : EFQHASPQHAGLYLCVVYVDDHIHAWGHMTISTAAQYR : 380
ABU45438.1 : EFQHASPQHAGLYLCVVYVDDHIHAWGHMTISTAAQYR : 380
AMB66104.1 : XFQHASPQHAGLYLCVVYVDDHIHAWGHMTISTAAQYR : 377
```

FIG. 77(contd)

```
AMB66173.1 : EFQHASPQHAGLYLCVVYVDDHIHAWGHMTISTAAQYR : 377
AMB66246.1 : EFQHASPQHAGLYLCVVYVDDHIHAWGHMTISTAXXXX : 380
AKC59520.1 : EFQHASPQHAGLYLCVVYVDDHIHAWGHMTISTAAQYR : 380
AKC59591.1 : EFQHASPQHAGLYLCVVYVDDHIHAWGHMTISTAAQYR : 380
AKC59307.1 : EFQHASPQHAGLYLCVVYVDDHIHAWGHMTISTAAQYR : 380
AMB66465.1 : EFQHASPQHAGLYLCVVYVDDHIHAWGHMTISTAAQYR : 380
AKC59378.1 : EFQHASPQHAGLYLCVVYVDDHIHAWGHMTISTAAQYR : 380
AEV91407.1 : EFQHASPQHAGLYLCVVYVDDHIHAWGHMTISTAAQYR : 377
CAB06715.1 : EFQHASPQHAGLYLCVVYVDDHIHAWGHMTISTAAQYR : 377
YP_0091372 : EFQHASPQHAGLYLCVVYVDDHIHAWGHMTISTAAQYR : 377
ABW83306.1 : EFQHASPQHAGLYLCVVYVDDHIHAWGHMTISTAAQYR : 380
ABW83324.1 : EFQHASPQHAGLYLCVVYVDDHIHAWGHMTISTAAQYR : 380
ABW83308.1 : EFQHASPQHAGLYLCVVYVDDHIHAWGHMTISTAAQYR : 380
ABW83310.1 : EFQHASPQHAGLYLCVVYVDDHIHAWGHMTISTAAQYR : 380
ABW83312.1 : EFQHASPQHAGLYLCVVYVDDHIHAWGHMTISTAAQYR : 380
ABW83314.1 : EFQHASPQHAGLYLCVVYVDDHIHAWGHMTISTAAQYR : 380
ABW83316.1 : EFQHASPQHAGLYLCVVYVDDHIHAWGHMTISTAAQYR : 380
ABW83318.1 : EFQHASPQHAGLYLCVVYVDDHIHAWGHMTISTAAQYR : 380
ABW83320.1 : EFQHASPQHAGLYLCVVYVDDHIHAWGHMTISTAAQYR : 380
ABW83322.1 : EFQHASPQHAGLYLCVVYVDDHIHAWGHMTISTAAQYR : 380
ABW83398.1 : EFQHASPQHAGLYLCVVYVDDHIHAWGHMTISTAAQYR : 380
ABW83380.1 : EFQHASPQHAGLYLCVVYVDDHIHAWGHMTISTAAQYR : 380
ABW83396.1 : EFQHASPQHAGLYLCVVYVDDHIHAWGHMTISTAAQYR : 377
ABW83382.1 : EFQHASPQHAGLYLCVVYVDDHIHAWGHMTISTAAQYR : 380
ABW83384.1 : EFQHASPQHAGLYLCVVYVDDHIHAWGHMTISTAAQYR : 380
ABW83394.1 : EFQHASPQHAGLYLCVVYVDDHIHAWGHMTISTAAQYR : 377
ABW83386.1 : EFQHASPQHAGLYLCVVYVDDHIHAWGHMTISTAAQYR : 380
ABW83388.1 : EFQHASPQHAGLYLCVVYVDDHIHAWGHMTISTAAQYR : 380
ABW83390.1 : EFQHASPQHAGLYLCVVYVDDHIHAWGHMTISTAAQYR : 380
ABW83392.1 : EFQHASPQHAGLYLCVVYVDDHIHAWGHMTISTAAQYR : 380
ABW83400.1 : EFQHASPQHAGLYLCVVYVDDHIHAWGHMTISTAAQYR : 380
AHG54732.1 : EFQHASPQHAGLYLCVVYVDDHIHAWGHMTISTAAQYR : 380
ABW83342.1 : EFQHASPQHAGLYLCVVYVDDHIHAWGHMTISTAAQYR : 380
ABW83340.1 : EFQHASPQHAGLYLCVVYVDDHIHAWGHMTISTAAQYR : 380
ABW83346.1 : EFQHASPQHAGLYLCVVYVDDHIHAWGHMTISTAAQYR : 380
ABW83348.1 : EFQHASPQHAGLYLCVVYVDDHIHAWGHMTISTAAQYR : 380
ABW83326.1 : EFQHASPQHAGLYLCVVYVDDHIHAWGHMTISTAAQYR : 380
ABW83350.1 : EFQHASPQHAGLYLCVVYVDDHIHAWGHMTISTAAQYR : 380
ABW83352.1 : EFQHASPQHAGLYLCVVYVDDHIHAWGHMTISTAAQYR : 380
ABW83336.1 : EFQHASPQHAGLYLCVVYVDDHIHAWGHMTISTAAQYR : 380
ABW83334.1 : EFQHASPQHAGLYLCVVYVDDHIHAWGHMTISTAAQYR : 380
ABW83354.1 : EFQHASPQHAGLYLCVVYVDDHIHAWGHMTISTAAQYR : 380
ABW83338.1 : EFQHASPQHAGLYLCVVYVDDHIHAWGHMTISTAAQYR : 380

                         *         400         *
AKC59449.1 : NAVVEQHLPQRQPEPVEPTRPHVRAPHPAPSARGPLRL : 418
AKC42830.1 : NAVVEQHLPQRQPEPVEPTRPHVRAPPPAPSARGPLRL : 418
ABU45436.1 : NAVVEQHLPQRQPEPVEPTRPHVRAPPPAPSARGPLRL : 418
ABU45439.1 : NAVVEQHLPQRQPEPVEPTRPHVRAPPPAPSARGPLRL : 418
ABU45437.1 : NAVVEQHLPQRQPEPVEPTRPHVRAPPPAPSARGPLRL : 418
ABU45438.1 : NAVVEQHLPQRQPEPVEPTRPHVRAPPPAPSARGPLRL : 418
AMB66104.1 : NAVVEQHLPQRQPEPVEPTRPHVRAPHPAPSARGPLRL : 415
AMB66173.1 : NAVVEQHLPXXXXXXXXXXXPHVRAPHPAPSARGPLRL : 415
AMB66246.1 : XXXXXXXXXXXXXXXXXXXXXXXXXPHPAPSARGPLRL : 418
AKC59520.1 : NAVVEQHLPQRQPEPVEPTRPHVRAPPPAPSARGPLRL : 418
AKC59591.1 : NAVVEQHLPQRQPEPVEPTRPHVRAPPPAPSARGPLRL : 418
AKC59307.1 : NAVVEQHLPQRQPEPVEPTRPHVRAPPPAPSARGPLRL : 418
AMB66465.1 : NAVVEQHLPQRQPEPVEPTRPHMRAPHPAPSARGPLRL : 418
AKC59378.1 : NAVVEQHLPQRQPEPVEPTRPHVRAPPPAPSARGPLRL : 418
AEV91407.1 : NAVVEQHLPQRQPEPVEPTRPHVRAPHPAPSARGPLRL : 415
```

FIG. 77(contd)

```
CAB06715.1 : NAVVEQHLPQRQPEPVEPTRPHVRAPHPAPSARGPLRL : 415
YP_0091372 : NAVVEQHLPQRQPEPVEPTRPHVRAPHPAPSARGPLRL : 415
ABW83306.1 : NAVVEQHLPQRQPEPVEPTRPHVRAPPPAPSARGPLRL : 418
ABW83324.1 : NAVVEQHLPQRQPEPVEPTRPHVRAPPPAPSARGPLRL : 418
ABW83308.1 : NAVVEQHLPQRQPEPVEPTRPHVRAPHPAPSARGPLRL : 418
ABW83310.1 : NAVVEQHLPQRQPEPVEPTRPHVRAPHPAPSARGPLRL : 418
ABW83312.1 : NAVVEQHLPQRQPEPVEPTRPHVRAPHPAPSARGPLRL : 418
ABW83314.1 : NAVVEQHLPQRQPEPVEPTRPHVRAPPPAPSARGPLRL : 418
ABW83316.1 : NAVVEQHLPQRQPEPVEPTRPHVRAPPPAPSARGPLRL : 418
ABW83318.1 : NAVVEQHLPQRQPEPVEPTRPHVRAPPPAPSARGPLRL : 418
ABW83320.1 : NAVVEQHLPQRQPEPVEPTRPHVRAPHPAPSARGPLRL : 418
ABW83322.1 : NAVVEQHLPQRQPEPVEPTRPHVRAPHPAPSARGPLRL : 418
ABW83398.1 : NAVVEQHLPQRQPEPVEPTRPHVRAPPPAPSARGPLRL : 418
ABW83380.1 : NAVVEQHLPQRQPEPVEPTRPHVRAPHPAPSARGPLRL : 418
ABW83396.1 : NAVVEQHLPQRQPEPVEPTRPHVRAPHPAPSARGPLRL : 415
ABW83382.1 : NAVVEQHLPQRQPEPVEPTRPHVRAPHPAPSARGPLRL : 418
ABW83384.1 : NAVVEQHLPQRQPEPVEPTRPHVRAPPPAPSARGPLRL : 418
ABW83394.1 : NAVVEQHLPQRQPEPVEPTRPHVRAPHPAPSARGPLRL : 415
ABW83386.1 : NAVVEQHLPQRQPEPVEPTRPHVRAPHPAPSARGPLRL : 418
ABW83388.1 : NAVVEQHLPQRQPEPVEPTRPHVRAPPPAPSARGPLRL : 418
ABW83390.1 : NAVVEQHLPQRQPEPVEPTRPHVRAPHPAPSARGPLRL : 418
ABW83392.1 : NAVVEQHLPQRQPEPVEPTRPHVRAPPPAPSARGPLRL : 418
ABW83400.1 : NAVVEQHLPQRQPEPVEPTRPHVRAPPPAPSARGPLRL : 418
AHG54732.1 : NAVVEQHLPQRQPEPVEPTRPHVRAPPPAPSARGPLRL : 418
ABW83342.1 : NAVVEQHLPQRQPEPVEPTRPHVRAPHPAPSARGPLRL : 418
ABW83340.1 : NAVVEQHLPQRQPEPVEPTRPHVRAPHPAPSARGPLRL : 418
ABW83346.1 : NAVVEQHLPQRQPEPVEPTRPHVRAPHPAPSARGPLRL : 418
ABW83348.1 : NAVVEQHLPQRQPEPVEPTRPHVRAPHPAPSARGPLRL : 418
ABW83326.1 : NAVVEQHLPQRQPEPVEPTRPHVRAPHPAPSARGPLRL : 418
ABW83350.1 : NAVVEQHLPQRQPEPVEPTRPHVRAPHPAPSARGPLRL : 418
ABW83352.1 : NAVVEQHLPQRQPEPVEPTRPHVRAPHPAPSARGPLRL : 418
ABW83336.1 : NAVVEQHLPQRQPEPVEPTRPHVRAPHPAPSARGPLRL : 418
ABW83334.1 : NAVVEQHLPQRQPEPVEPTRPHVRAPHPAPSARGPLRL : 418
ABW83354.1 : NAVVEQHLPQRQPEPVEPTRPHVRAPHPAPSARGPLRL : 418
ABW83338.1 : NAVVEQHLPQRQPEPVEPTRPHVRAPHPAPSARGPLRL : 418

AKC59449.1 : G : 419
AKC42830.1 : G : 419
ABU45436.1 : G : 419
ABU45439.1 : R : 419
ABU45437.1 : G : 419
ABU45438.1 : G : 419
AMB66104.1 : G : 416
AMB66173.1 : G : 416
AMB66246.1 : G : 419
AKC59520.1 : G : 419
AKC59591.1 : G : 419
AKC59307.1 : G : 419
AMB66465.1 : G : 419
AKC59378.1 : G : 419
AEV91407.1 : G : 416
CAB06715.1 : G : 416
YP_0091372 : G : 416
ABW83306.1 : G : 419
ABW83324.1 : G : 419
ABW83308.1 : G : 419
ABW83310.1 : G : 419
ABW83312.1 : G : 419
ABW83314.1 : G : 419
ABW83316.1 : G : 419
```

| | | | | |
|---|---|---|---|---|
| ABW83318.1 | : | G | : | 419 |
| ABW83320.1 | : | G | : | 419 |
| ABW83322.1 | : | G | : | 419 |
| ABW83398.1 | : | G | : | 419 |
| ABW83380.1 | : | G | : | 419 |
| ABW83396.1 | : | G | : | 416 |
| ABW83382.1 | : | G | : | 419 |
| ABW83384.1 | : | G | : | 419 |
| ABW83394.1 | : | G | : | 416 |
| ABW83386.1 | : | G | : | 419 |
| ABW83388.1 | : | G | : | 419 |
| ABW83390.1 | : | G | : | 419 |
| ABW83392.1 | : | G | : | 419 |
| ABW83400.1 | : | G | : | 419 |
| AHG54732.1 | : | G | : | 419 |
| ABW83342.1 | : | G | : | 419 |
| ABW83340.1 | : | G | : | 419 |
| ABW83346.1 | : | G | : | 419 |
| ABW83348.1 | : | G | : | 419 |
| ABW83326.1 | : | G | : | 419 |
| ABW83350.1 | : | G | : | 419 |
| ABW83352.1 | : | G | : | 419 |
| ABW83336.1 | : | G | : | 419 |
| ABW83334.1 | : | G | : | 419 |
| ABW83354.1 | : | G | : | 419 |
| ABW83338.1 | : | G | : | 419 |

FIG. 77(contd)

# FIG. 78

```
                                   *         20         *
AKC59448.1 : MPGRSLQGLAILGLWVCATGLVVRGPTVSLVSDSLVDA : 38
AKC42829.1 : MPGRSLQGLAILGLWVCATGLVVRGPTVSLVSDSLVDA : 38
AMB66029.1 : MPGRSLQGLAMLGLWVCATGLVVRGPTVSLVSDSLVDA : 38
AMB66103.1 : MPGRSLQGLAILGLWVCATGLVVRGPTVSLVSDSLVDA : 38
AMB66172.1 : MPGRSLQGLAILGLWVCATGLVVRGPTVSLVSDSLVDA : 38
AMB66245.1 : MPGRSLQGLAILGLWVCATGLVVRGPTVSLVSDSLVDA : 38
AMB66322.1 : MPGRSLQGLAILGLWVCATGLVVRGPTVSLVSDSLVDA : 38
AKC59519.1 : MPGRSLQGLAILGLWVCATGLVVRGPTVSLVSDSLVDA : 38
AKC59590.1 : MPGRSLQGLAILGLWVCATGLVVRGPTVSLVSDSLVDA : 38
AKC59306.1 : MPGRSLQGLAILGLWVCATGLVVRGPTVSLVSDSLVDA : 38
AKC59377.1 : MPGRSLQGLAILGLWVCATGLVVRGPTVSLVSDSLVDA : 38
YP_0091372 : MPGRSLQGLAILGLWVCATGLVVRGPTVSLVSDSLVDA : 38
CAB06714.1 : MPGRSLQGLAILGLWVCATGLVVRGPTVSLVSDSLVDA : 38
AEV91406.1 : MPGRSLQGLAILGLWVCATGLVVRGPTVSLVSDSLVDA : 38
ABW83305.1 : MPGRSLQGLAILGLWVCATGLVVRGPTVSLVSDSLVDA : 38
ABW83323.1 : MPGRSLQGLAILGLWVCATGLVVRGPTVSLVSDSLVDA : 38
ABW83307.1 : MPGRSLQGLAILGLWVCATGLVVRGPTVSLVSDSLVDA : 38
ABW83309.1 : MPGRSLQGLAILGLWVCATGLVVRGPTVSLVSDSLVDA : 38
ABW83311.1 : MPGRSLQGLAILGLWVCATGLVVRGPTVSLVSDSLVDA : 38
ABW83313.1 : MPGRSLQGLAILGLWVCATGLVVRGPTVSLVSDSLVDA : 38
ABW83315.1 : MPGRSLQGLAILGLWVCATGLVVRGPTVSLVSDSLVDA : 38
ABW83317.1 : MPGRSLQGLAILGLWVCATGLVVRGPTVSLVSDSLVDA : 38
ABW83319.1 : MPGRSLQGLAILGLWVCATGLVVRGPTVSLVSDSLVDA : 38
ABW83321.1 : MPGRSLQGLAILGLWVCATGLVVRGPTVSLVSDSLVDA : 38
ABW83397.1 : MPGRSLQGLAILGLWVCATGLVVRGPTVSLVSDSLVDA : 38
ABW83379.1 : MPGRSLQGLAILGLWVCATGLVVRGPTVSLVSDSLVDA : 38
ABW83395.1 : MPGRSLQGLAILGLWVCATGLVVRGPTVSLVSDSLVDA : 38
ABW83381.1 : MPGRSLQGLAILGLWVCATGLVVRGPTVSLVSDSLVDA : 38
ABW83383.1 : MPGRSLQGLAILGLWVCATGLVVRGPTVSLVSDSLVDA : 38
ABW83393.1 : MPGRSLQGLAILGLWVCATGLVVRGPTVSLVSDSLVDA : 38
ABW83385.1 : MPGRSLQGLAILGLWVCATGLVVRGPTVSLVSDSLVDA : 38
ABW83387.1 : MPGRSLQGLAILGLWVCATGLVVRGPTVSLVSDSLVDA : 38
ABW83389.1 : MPGRSLQGLAILGLWVCATGLVVRGPTVSLVSDSLVDA : 38
ABW83391.1 : MPGRSLQGLAILGLWVCATGLVVRGPTVSLVSDSLVDA : 38
ABW83399.1 : MPGRSLQGLAILGLWVCATGLVVRGPTVSLVSDSLVDA : 38
AHG54731.1 : MPGRSLQGLAILGLWVCATGLVVRGPTVSLVSDSLVDA : 38
ABW83341.1 : MPGRSLQGLAILGLWVCATGLVVRGPTVSLVSDSLVDA : 38
ABW83339.1 : MPGRSLQGLAILGLWVCATGLVVRGPTVSLVSDSLVDA : 38
ABW83345.1 : MPGRSLQGLVILGLWVCTTGLVVRGPTVSLVSDSLVDA : 38
ABW83347.1 : MPGRSLQGLAILGLWVCATGLVVRGPTVSLVSDSLVDA : 38
ABW83325.1 : MPGRSLQGLAILGLWVCATGLVVRGPTVSLVSDSLVDA : 38
ABW83349.1 : MPGRSLQGLAILGLWVCATGLVVRGPTVSLVSDSLVDA : 38
ABW83351.1 : MPGRSLQGLAILGLWVCATGLVVRGPTVSLVSDSLVDA : 38
ABW83335.1 : MPGRSLQGLVILGLWVCATGLVVRGPTVSLVSDSLVDA : 38
ABW83333.1 : MPGRSLQGLVILGLWVCATGLVVRGPTVSLVSDSLVDA : 38
ABW83353.1 : MPGRSLQGLVILGLWVCATGLVVRGPTVSLVSDSLVDA : 38
ABW83337.1 : MPGRSLQGLAILGLWVCATGLVVRGPTVSLVSDSLVDA : 38
ABW83355.1 : MPGRSLQGLAILGLWVCATGLVVRGPTVSLVSDSLVDA : 38
ABW83327.1 : MPGRSLQGLAILGLWVCATGLVVRGPTVSLVSDSLVDA : 38
ABW83343.1 : MPGRSLQGLAILGLWVCATGLVVRGPTVSLVSDSLVDA : 38
ABW83329.1 : MPGRSLQGLAILGLWVCATGLVVRGPTVSLVSDSLVDA : 38
ABW83357.1 : MPGRSLQGLAILGLWVCATGLVVRGPTVSLVSDSLVDA : 38
ABW83359.1 : MPGRSLQGLAILGLWVCATGLVVRGPTVSLVSDSLVDA : 38
ABW83361.1 : MPGRSLQGLAILGLWVCATGLVVRGPTVSLVSDSLVDA : 38
ABW83363.1 : MPGRSLQGLAILGLWVCATGLVVRGPTVSLVSDSLVDA : 38
ABW83331.1 : MPGRSLQGLVILGLWVCATGLVVRGPTVSLVSDSLVDA : 38
ABW83365.1 : MPGRSLQGLAILGLWVCATGLVVRGPTVSLVSDSLVDA : 38
```

FIG. 78(contd)

```
ABW83367.1 : MPGRSLQGLAILGLWVCATGLVVRGPTVSLVSDSLVDA : 38
ABW83369.1 : MPGRSLQGLVILGLWVCATGLVVRGPTVSLVSDSLVDA : 38
ABW83371.1 : MPGRSLQGLAILGLWVCATGLVVRGPTVSLVSDSLVDA : 38
ABW83375.1 : MPGRSLQGLAILGLWVCATGLVVRGPTVSLVSDSLVDA : 38
ABW83373.1 : MPGRSLQGLAILGLWVCATGLVVRGPTVSLVSDSLVDA : 38
ABW83377.1 : MPGRSLQGLAILGLWVCATGLVVRGPTVSLVSDSLVDA : 38

                  40         *         60         *
AKC59448.1 : GAVGPQGFVEEDLRVFGELHFVGAQVPHTNYYDGIIEL : 76
AKC42829.1 : GAVGPQGFVEEDLRVFGELHFVGAQVPHTNYYDGIIEL : 76
AMB66029.1 : GAVGPQGFVEEDLRVFGELHFVGAQVPHTNYYDGIIEL : 76
AMB66103.1 : GAVGPQGFVEEDLRVFGELHFVGAQVPHTNYYDGIIEL : 76
AMB66172.1 : GAVGPQGFVEEDLRVFGELHFVGAQVPHTNYYDGIIEL : 76
AMB66245.1 : GAVGPQGFVEEDLRVFGELHFVGAQVPHTNYYDGIIEL : 76
AMB66322.1 : GAVGPQGFVEEDLRVFGELHFVGAQVPHTNYYDGIIEL : 76
AKC59519.1 : GAVGPQGFVEEDLRVFGELHFVGAQVPHTNYYDGIIEL : 76
AKC59590.1 : GAVGPQGFVEEDLRVFGELHFVGAQVPHTNYYDGIIEL : 76
AKC59306.1 : GAVGPQGFVEEDLRVFGELHFVGAQVPHTNYYDGIIEL : 76
AKC59377.1 : GAVGPQGFVEEDLRVFGELHFVGAQVPHTNYYDGIIEL : 76
YP_0091372 : GAVGPQGFVEEDLRVFGELHFVGAQVPHTNYYDGIIEL : 76
CAB06714.1 : GAVGPQGFVEEDLRVFGELHFVGAQVPHTNYYDGIIEL : 76
AEV91406.1 : GAVGPQGFVEEDLRVFGELHFVGAQVPHTNYYDGIIEL : 76
ABW83305.1 : GAVGPQGFVEEDLRVFGELHFVGAQVPHTNYYDGIIEL : 76
ABW83323.1 : GAVGPQGFVEEDLRVFGELHFVGAQVPHTNYYDGIIEL : 76
ABW83307.1 : GAVGPQGFVEEDLRVFGELHFVGAQVPHTNYYDGIIEL : 76
ABW83309.1 : GAVGPQGFVEEDLRVFGELHFVGAQVPHTNYYDGIIEL : 76
ABW83311.1 : GAVGPQGFVEEDLRVFGELHFVGAQVPHTNYYDGIIEL : 76
ABW83313.1 : GAVGPQGFVEEDLRVFGELHFVGAQVPHTNYYDGIIEL : 76
ABW83315.1 : GAVGPQGFVEEDLRVFGELHFVGAQVPHTNYYDGIIEL : 76
ABW83317.1 : GAVGPQGFVEEDLRVFGELHFVGAQVPHTNYYDGIIEL : 76
ABW83319.1 : GAVGPQGFVEEDLRVFGELHFVGAQVPHTNYYDGIIEL : 76
ABW83321.1 : GAVGPQGFVEEDLRVFGELHFVGAQVPHTNYYDGIIEL : 76
ABW83397.1 : GAVGPQGFVEEDLRVFGELHFVGAQVPHTNYYDGIIEL : 76
ABW83379.1 : GAVGPQGFVEEDLRVFGELHFVGAQVPHTNYYDGIIEL : 76
ABW83395.1 : GAVGPQGFVEEDLRVFGELHFVGAQVPHTNYYDGIIEL : 76
ABW83381.1 : GAVGPQGFVEEDLRVFGELHFVGAQVPHTNYYDGIIEL : 76
ABW83383.1 : GAVGPQGFVEEDLRVFGELHFVGAQVPHTNYYDGIIEL : 76
ABW83393.1 : GAVGPQGFVEEDLRVFGELHFVGAQVPHTNYYDGIIEL : 76
ABW83385.1 : GAVGPQGFVEEDLRVFGELHFVGAQVPHTNYYDGIIEL : 76
ABW83387.1 : GAVGPQGFVEEDLRVFGELHFVGAQVPHTNYYDGIIEL : 76
ABW83389.1 : GAVGPQGFVEEDLRVFGELHFVGAQVPHTNYYDGIIEL : 76
ABW83391.1 : GAVGPQGFVEEDLRVFGELHFVGAQVPHTNYYDGIIEL : 76
ABW83399.1 : GAVGPQGFVEEDLRVFGELHFVGAQVPHTNYYDGIIEL : 76
AHG54731.1 : GAVGPQGFVEEDLRVFGELHFVGAQVPHTNYYDGIIEL : 76
ABW83341.1 : GAVGPQGFVEEDLRVFGELHFVGAQVPHTNYYDGIIEL : 76
ABW83339.1 : GAVGPQGFVEEDLRVFGELHFVGAQVPHTNYYDGIIEL : 76
ABW83345.1 : GAVGPQGFVEEDLRVFGELHFVGAQVPHTNYYDGIIEL : 76
ABW83347.1 : GAVGPQGFVEEDLRVFGELHFVGAQVPHTNYYDGIIEL : 76
ABW83325.1 : GAVGPQGFVEEDLRVFGELHFVGAQVPHTNYYDGIIEL : 76
ABW83349.1 : GAVGPQGFVEEDLRVFGELHFVGAQVPHTNYYDGIIEL : 76
ABW83351.1 : GAVGPQGFVEEDLRVFGELHFVGAQVPHTNYYDGIIEL : 76
ABW83335.1 : GAVGPQGFVEEDLRVFGELHFVGAQVPHTNYYDGIIEL : 76
ABW83333.1 : GAVGPQGFVEEDLRVFGELHFVGAQVPHTNYYDGIIEL : 76
ABW83353.1 : GAVGPQGFVEEDLRVFGELHFVGAQVPHTNYYDGIIEL : 76
ABW83337.1 : GAVGPQGFVEEDLRVFGELHFVGAQVPHTNYYDGIIEL : 76
ABW83355.1 : GAVGPQGFVEEDLRVFGELHFVGAQVPHTNYYDGIIEL : 76
ABW83327.1 : GAVGPQGFVEEDLRVFGELHFVGAQVPHTNYYDGIIEL : 76
ABW83343.1 : GAVGPQGFVEEDLRVFGELHFVGAQVPHTNYYDGIIEL : 76
ABW83329.1 : GAVGPQGFVEEDLRVFGELHFVGAQVPHTNYYDGIIEL : 76
ABW83357.1 : GAVGPQGFVEEDLRVFGELHFVGAQVPHTNYYDGIIEL : 76
```

FIG. 78(contd)

```
ABW83359.1 : GAVGPQGFVEEDLRVFGELHFVGAQVPHTNYYDGIIEL :  76
ABW83361.1 : GAVGPQGFVEEDLRVFGELHFVGAQVPHTNYYDGIIEL :  76
ABW83363.1 : GAVGPQGFVEEDLRVFGELHFVGAQVPHTNYYDGIIEL :  76
ABW83331.1 : GAVGPQGFVEEDLRVFGELHFVGAQVPHTNYYDGIIEL :  76
ABW83365.1 : GAVGPQGFVEEDLRVFGELHFVGAQVPHTNYYDGIIEL :  76
ABW83367.1 : GAVGPQGFVEEDLRVFGELHFVGAQVPHTNYYDGIIEL :  76
ABW83369.1 : GAVGPQGFVEEDLRVFGELHFVGAQVPHTNYYDGIIEL :  76
ABW83371.1 : GAVGPQGFVEEDLRVFGELHFVGAQVPHTNYYDGIIEL :  76
ABW83375.1 : GAVGPQGFVEEDLRVFGELHFVGAQVPHTNYYDGIIEL :  76
ABW83373.1 : GAVGPQGFVEEDLRVFGELHFVGAQVPHTNYYDGIIEL :  76
ABW83377.1 : GAVGPQGFVEEDLRVFGELHFVGAQVPHTNYYDGIIEL :  76

                     80         *        100          *
AKC59448.1 : FHYPLGNHCPRVVHVVTLTACPRRPAVAFTLCRSTHHA : 114
AKC42829.1 : FHYPLGNHCPRVVHVVTLTACPRRPAVAFTLCRSTHHA : 114
AMB66029.1 : FHYPLGNHCPRVVHVVTLTACPRRPAVAFTLCRSTHHA : 114
AMB66103.1 : FHYPLGNHCPRVVHVVTLTACPRRPAVAFTLCRSTHHA : 114
AMB66172.1 : FHYPLGNHCPRVVHVVTLTACPRRPAVAFTLCRSTHHA : 114
AMB66245.1 : FHYPLGNHCPRVVHMITLTACPRRPAVAFTLCRSTHHA : 114
AMB66322.1 : FHYPLGNHCPRVVHVVTLTACPRRPAVAFTLCRSTHHA : 114
AKC59519.1 : FHYPLGNHCPRVVHVVTLTACPRRPAVAFTLCRSTHHA : 114
AKC59590.1 : FHYPLGNHCPRVVHVVTLTACPRRPAVAFTLCRSTHHA : 114
AKC59306.1 : FHYPLGNHCPRVVHVVTLTACPRRPAVAFTLCRSTHHA : 114
AKC59377.1 : FHYPLGNHCPRVVHVVTLTACPRRPAVAFTLCRSTHHA : 114
YP_0091372 : FHYPLGNHCPRVVHVVTLTACPRRPAVAFTLCRSTHHA : 114
CAB06714.1 : FHYPLGNHCPRVVHVVTLTACPRRPAVAFTLCRSTHHA : 114
AEV91406.1 : FHYPLGNHCPRVVHVVTLTACPRRPAVAFTLCRSTHHA : 114
ABW83305.1 : FHYPLGNHCPRVVHVVTLTACPRRPAVAFTLCRSTHHA : 114
ABW83323.1 : FHYPLGNHCPRVVHVVTLTACPRRPAVAFTLCRSTHHA : 114
ABW83307.1 : FHYPLGNHCPRVVHVVTLTACPRRPAVAFTLCRSTHHA : 114
ABW83309.1 : FHYPLGNHCPRVVHVVTLTACPRRPAVAFTLCRSTHRA : 114
ABW83311.1 : FHYPLGNHCPRVVHVVTLTACPRRPAVAFTLCRSTHRA : 114
ABW83313.1 : FHYPLGNHCPRVVHVVTLTACPRRPAVAFTLCRSTHHA : 114
ABW83315.1 : FHYPLGNHCPRVVHVVTLTACPRRPAVAFTLCRSTHHA : 114
ABW83317.1 : FHYPLGNHCPRVVHVVTLTACPRRPAVAFTLCRSTHHA : 114
ABW83319.1 : FHYPLGNHCPRVVHVVTLTACPRRPAVAFTLCRSTHHA : 114
ABW83321.1 : FHYPLGNHCPRVVHVVTLTACPRRPAVAFTLCRSTHHA : 114
ABW83397.1 : FHYPLGNHCPRVVHVVTLTACPRRPAVAFTLCRSTHHA : 114
ABW83379.1 : FHYPLGNHCPRVVHVVTLTACPRRPAVAFTLCRSTHHA : 114
ABW83395.1 : FHYPLGNHCPRVVHVVTLTACPRRPAVAFTLCRSTHHA : 114
ABW83381.1 : FHYPLGNHCPRVVHVVTLTACPRRPAVAFTLCRSTHHA : 114
ABW83383.1 : FHYPLGNHCPRVVHVVTLTACPRRPAVAFTLCRSTHHA : 114
ABW83393.1 : FHYPLGNHCPRVVHVVTLTACPRRPAVAFTLCRSTHHA : 114
ABW83385.1 : FHYPLGNHCPRVVHVVTLTACPRRPAVAFTLCRSTHHA : 114
ABW83387.1 : FHYPLGNHCPRVVHVVTLTACPRRPAVAFTLCRSTHHA : 114
ABW83389.1 : FHYPLGNHCPRVVHVVTLTACPRRPAVAFTLCRSTHHA : 114
ABW83391.1 : FHYPLGNHCPRVVHVVTLTACPRRPAVAFTLCRSTHHA : 114
ABW83399.1 : FHYPLGNHCPRVVHVVTLTACPRRPAVAFTLCRSTHHA : 114
AHG54731.1 : FHYPLGNHCPRVVHVVTLTACPRRPAVAFTLCRSTHHA : 114
ABW83341.1 : FHYPLGNHCPRVVHVVTLTACPRRPAVAFTLCRSTHHA : 114
ABW83339.1 : FHYPLGNHCPRVVHVVTLTACPRRPAVAFTLCRSTHHA : 114
ABW83345.1 : FHYPLGNHCPRVVHVVTLTACPRRPAVAFTLCRSTHHA : 114
ABW83347.1 : FHYPLGNHCPRVVHVVTLTACPRRPAVAFTLCRSTHHA : 114
ABW83325.1 : FHYPLGNHCPRVVHVVTLTACPRRPAVAFTLCRSTHHA : 114
ABW83349.1 : FHYPLGNHCPRVVHVVTLTACPRRPAVAFTLCRSTHHA : 114
ABW83351.1 : FHYPLGNHCPRVVHVVTLTACPRRPAVAFTLCRSTHHA : 114
ABW83335.1 : FHYPLGNHCPRVVHVVTLTACPRRPAVAFTLCRSTHHA : 114
ABW83333.1 : FHYPLGNHCPRVVHVVTLTACPRRPAVAFTLCRSTHHA : 114
ABW83353.1 : FHYPLGNHCPRVVHVVTLTACPRRPAVAFTLCRSTHHA : 114
ABW83337.1 : FHYPLGNHCPRVVHVVTLTACPRRPAVAFTLCRSTHHA : 114
```

FIG. 78(contd)

```
ABW83355.1 : FHYPLGNHCPRVVHVVTLTACPRRPAVAFTLCRSTHHA : 114
ABW83327.1 : FHYPLGNHCPRVVHVVTLTACPRRPAVAFTLCRSTHHA : 114
ABW83343.1 : FHYPLGNHCPRVVHVVTLTACPRRPAVAFTLCRSTHHA : 114
ABW83329.1 : FHYPLGNHCPRVVHVVTLTACPRRPAVAFTLCRSTHHA : 114
ABW83357.1 : FHYPLGNHCPRVVHVVTLTACPRRPAVAFTLCRSTHHA : 114
ABW83359.1 : FHYPLGNHCPRVVHVVTLTACPRRPAVAFTLCRSTHHA : 114
ABW83361.1 : FHYPLGNHCPRVVHVVTLTACPRRPAVAFTLCRSTHHA : 114
ABW83363.1 : FHYPLGNHCPRVVHVVTLTACPRRPAVAFTLCRSTHHA : 114
ABW83331.1 : FHYPLGNHCPRVVHVVTLTACPRRPAVAFTLCRSTHHA : 114
ABW83365.1 : FHYPLGNHCPRVVHVVTLTACPRRPAVAFTLCRSTHHA : 114
ABW83367.1 : FHYPLGNHCPRVVHVVTLTACPRRPAVAFTLCRSTHHA : 114
ABW83369.1 : FHYPLGNHCPRVVHVVTLTACPRRPAVAFTLCRSTHHA : 114
ABW83371.1 : FHYPLGNHCPRVVHVVTLTACPRRPAVAFTLCRSTHHA : 114
ABW83375.1 : FHYPLGNHCPRVVHVITLTACPRRPAVAFTLCRSTHHA : 114
ABW83373.1 : FHYPLGNHCPRVVHVITLTACPRRPAVAFTLCRSTHHA : 114
ABW83377.1 : FHYPLGNHCPRVVHVVTLTACPRRPAVAFTLCRSTHHA : 114
```

```
                   120         *        140        *
AKC59448.1 : HSPAYPTLELGLARQPLLRVRTATRDYAGLYVLRVWVG : 152
AKC42829.1 : HSPAYPTLELGLARQPLLRVRTATRDYAGLYVLRVWVG : 152
AMB66029.1 : HSPAYPTLELGLARQPLLRVRTATRDYAGLYVLRVWVG : 152
AMB66103.1 : HSPAYPTLELGLARQPLLRVRTATRDYAGLYVLRVWVG : 152
AMB66172.1 : HSPAYPTLELGLARQPLLRVRTATRDYAGLYVLRVWVG : 152
AMB66245.1 : HSPAYPTLELGLARQPLLRVRTATRDYAGLYVLRVWVG : 152
AMB66322.1 : HSPAYPTLELGLARQPLLRVRTATRDYAGLYVLRVWVG : 152
AKC59519.1 : HSPAYPTLELGLARQPLLRVRTATRDYAGLYVLRVWVG : 152
AKC59590.1 : HSPAYPTLELGLARQPLLRVRTATRDYAGLYVLRVWVG : 152
AKC59306.1 : HSPAYPTLELGLARQPLLRVRTATRDYAGLYVLRVWVG : 152
AKC59377.1 : HSPAYPTLELGLARQPLLRVRTATRDYAGLYVLRVWVG : 152
YP_0091372 : HSPAYPTLELGLARQPLLRVRTATRDYAGLYVLRVWVG : 152
CAB06714.1 : HSPAYPTLELGLARQPLLRVRTATRDYAGLYVLRVWVG : 152
AEV91406.1 : HSPAYPTLELGLARQPLLRVRTATRDYAGLYVLRVWVG : 152
ABW83305.1 : HSPAYPTLELGLARQPLLRVRTATRDYAGLYVLRVWVG : 152
ABW83323.1 : HSPAYPTLELGLARQPLLRVRTATRDYAGLYVLRVWVG : 152
ABW83307.1 : HSPAYPTLELGLARQPLLRVRTATRDYAGLYVLRVWVG : 152
ABW83309.1 : HSPAYPTLELGLARQPLLRVRTATRDYAGLYVLRVWVG : 152
ABW83311.1 : HSPAYPTLELGLARQPLLRVRTATRDYAGLYVLRVWVG : 152
ABW83313.1 : HSPAYPTLELGLARQPLLRVRTATRDYAGLYVLRVWVG : 152
ABW83315.1 : HSPAYPTLELGLARQPLLRVRTATRDYAGLYVLRVWVG : 152
ABW83317.1 : HSPAYPTLELGLARQPLLRVRTATRDYAGLYVLRVWVG : 152
ABW83319.1 : HSPAYPTLELGLARQPLLRVRTATRDYAGLYVLRVWVG : 152
ABW83321.1 : HSPAYPTLELGLARQPLLRVRTATRDYAGLYVLRVRVG : 152
ABW83397.1 : HSPAYPTLELGLARQPLLRVRTATRDYAGLYVLRVWVG : 152
ABW83379.1 : HSPAYPTLELGLARPPLLRVRTATRDYAGLYVLRVWVG : 152
ABW83395.1 : HSPAYPTLELGLARQPLLRVRTATRDYAGLYVLRVWVG : 152
ABW83381.1 : HSPAYPTLELGLARQPLLRVRTATRDYAGLYVLRVWVG : 152
ABW83383.1 : HSPAYPTLELGLARQPLLRVRTATRDYAGLYVLRVWVG : 152
ABW83393.1 : HSPAYPTLELGLARQPLLRVRTATRDYAGLYVLRVWVG : 152
ABW83385.1 : HSPAYPTLELGLARQPLLRVRTATRDYAGLYVLRVWVG : 152
ABW83387.1 : HSPAYPTLELGLARQPLLRVRTATRDYAGLYVLRVWVG : 152
ABW83389.1 : HSPAYPTLELGLARQPLLRVRTATRDYAGLYVLRVWVG : 152
ABW83391.1 : HSPAYPTLELGLARQPLLRVRTATRDYAGLYVLRVWVG : 152
ABW83399.1 : HSPAYPTLELGLARQPLLRVRTATRDYAGLYVLRVWVG : 152
AHG54731.1 : HSPAYPTLELGLARQPLLRVRTATRDYAGLYVLRVWVG : 152
ABW83341.1 : HSPAYPTLELGLARQPLLRVRTATRDYAGLYVLRVWVG : 152
ABW83339.1 : HSPAYPTLELGLARQPLLRVRTATRDYAGLYVLRVWVG : 152
ABW83345.1 : HSPAYPTLELGLARQPLLRVRTATRDYAGLYVLRVWVG : 152
ABW83347.1 : HSPAYPTLELGLARQPLLRVRTATRDYAGLYVLRVWVG : 152
ABW83325.1 : HSPAYPTLELGLARQPLLRVRTATRDYAGLYVLRVWVG : 152
ABW83349.1 : HSPAYPTLELGLARQPLLRVRTATRDYAGLYVLRVWVG : 152
```

FIG. 78(contd)

```
ABW83351.1 : HSPAYPTLELGLARQPLLRVRTATRDYAGLYVLRVWVG : 152
ABW83335.1 : HSPAYPTLELGLARQPLLRVRTATRDYAGLYVLRVWVG : 152
ABW83333.1 : HSPAYPTLELGLARQPLLRVRTATRDYAGLYVLRVWVG : 152
ABW83353.1 : HSPAYPTLELGLARQPLLRVRTATRDYAGLYVLRVWVG : 152
ABW83337.1 : HSPAYPTLELGLARQPLLRVRTATRDYAGLYVLRVWVG : 152
ABW83355.1 : HSPAYPTLELGLARQPLLRVRTATRDYAGLYVLRVWVG : 152
ABW83327.1 : HSPAYPTLELGLARQPLLRVRTATRDYAGLYVLRVWVG : 152
ABW83343.1 : HSPAYPTLELGLARQPLLRVRTATRDYAGLYVLRVWVG : 152
ABW83329.1 : HSPAYPTLELGLARQPLLRVRTATRDYAGLYVLRVWVG : 152
ABW83357.1 : HSPAYPTLELGLARQPLLRVRTATRDYAGLYVLRVWVG : 152
ABW83359.1 : HSPAYPTLELGLARQPLLRVRTATRDYAGLYVLRVWVG : 152
ABW83361.1 : HSPAYPTLELGLARQPLLRVRTATRDYAGLYVLRVWVG : 152
ABW83363.1 : HSPAYPTLELGLARQPLLRVRTATRDYAGLYVLRVWVG : 152
ABW83331.1 : HSPAYPTLELGLARQPLLRVRTATRDYAGLYVLRVWVG : 152
ABW83365.1 : HSPAYPTLELGLARQPLLRVRTATRDYAGLYVLRVWVG : 152
ABW83367.1 : HSPAYPTLELGLARQPLLRVRTATRDYAGLYVLRVWVG : 152
ABW83369.1 : HSPAYPTLELGLARQPLLRVRTATRDYAGLYVLRVWVG : 152
ABW83371.1 : HSPAYPTLELGLARQPLLRVRTATRDYAGLYVLRVWVG : 152
ABW83375.1 : HSPAYPTLELGLARQPLLRVRTATRDYAGLYVLRVWVG : 152
ABW83373.1 : HSPAYPTLELGLARQPLLRVRTATRDYAGLYVLRVWVG : 152
ABW83377.1 : HSPAYPTLELGLARQPLLRVRTATRDYAGLYVLRVWVG : 152

                      160         *        180          *
AKC59448.1 : SATNASRFVLGVALSANGTFVYNGSDYGSCDPAQLPFS : 190
AKC42829.1 : SATNASLFVLGVALSANGTFVYNGSDYGSCDPAQLPFS : 190
AMB66029.1 : SATNASLFVLGVALSANGTFVYNGSDYGSCDPAQLPFS : 190
AMB66103.1 : SATNASRFVLGVALSANGTFVYNGSDYGSCDPAQLPFS : 190
AMB66172.1 : SATNASLFVLGVALSANGTFVYNGSDYGSCDPAQLPFS : 190
AMB66245.1 : SATNASRFVLGVALSANGTFVYNGSDYGSCDPAQLPFS : 190
AMB66322.1 : SATNASRFVLGVALSANGTFVYNGSDYGSCDPAQLPFS : 190
AKC59519.1 : SATNASLFVLGVALSANGTFVYNGSDYGSCDPAQLPFS : 190
AKC59590.1 : SATNASLFVLGVALSANGTFVYNGSDYGSCDPAQLPFS : 190
AKC59306.1 : SATNASLFVLGVALSANGTFVYNGSDYGSCDPAQLPFS : 190
AKC59377.1 : SATNASLFVLGVALSANGTFVYNGSDYGSCDPAQLPFS : 190
YP_0091372 : SATNASRFVLGVALSANGTFVYNGSDYGSCDPAQLPFS : 190
CAB06714.1 : SATNASRFVLGVALSANGTFVYNGSDYGSCDPAQLPFS : 190
AEV91406.1 : SATNASRFVLGVALSANGTFVYNGSDYGSCDPAQLPFS : 190
ABW83305.1 : SATNASRFVLGVALSANGTFVYNGSDYGSCDPAQLPFS : 190
ABW83323.1 : SATNASRFVLGVALSANGTFVYNGSDYGSCDPAQLPFS : 190
ABW83307.1 : SATNASRFVLGVALSANGTFVYNGSDYGSCDPAQLPFS : 190
ABW83309.1 : SATNASRFVLGVALSVNGTFVYNGSDYGSCDPAQLPFS : 190
ABW83311.1 : SATNASRFVLGVALSANGTFVYNGSDYGSCDPAQLPFS : 190
ABW83313.1 : SATNASLFVLGVALSANGTFVYNGSDYGSCDPAQLPFS : 190
ABW83315.1 : SATNASRFVLGVALSANGTFVYNGSDYGSCDPAQLPFS : 190
ABW83317.1 : SATNASRFVLGVALSANGTFVYNGSDYGSCDPAQLPFS : 190
ABW83319.1 : SATNASRFVLGVALSANGTFVYNGSDYGSCDPAQLPFS : 190
ABW83321.1 : SATNASRFVLGVALSANGTFVYNGSDYGSCDPAQLPFS : 190
ABW83397.1 : SATNASLFVLGVALSANGTFVYNGSDYGSCDPAQLPFS : 190
ABW83379.1 : SATNASLFVLGVALSANGTFVYNGSDYGSCDPAQLPFS : 190
ABW83395.1 : SATNASRFVLGVALSANGTFVYNGSDYGSCDPAQLPFS : 190
ABW83381.1 : SATNASRFVLGVALSANGTFVYNGSDYGSCDPAQLPFS : 190
ABW83383.1 : SATNASRFVLGVALSANGTFVYNGSDYGSCDPAQLPFS : 190
ABW83393.1 : SATNASRFVLGVALSANGTFVYNGSDYGSCDPAQLPFS : 190
ABW83385.1 : SATNASLFVLGVALSANGTFVYNGSDYGSCDPAQLPFS : 190
ABW83387.1 : SATNASRFVLGVALSANGTFVYNGSDYGSCDPAQLPFS : 190
ABW83389.1 : SATNASRFVLGVALSANGTFVYNGSDYGSCDPAQLPFS : 190
ABW83391.1 : SATNASRFVLGVALSANGTFVYNGSDYGSCDPAQLPFS : 190
ABW83399.1 : SATNASRFVLGVALSANGTFVYNGSDYGSCDPAQLPFS : 190
AHG54731.1 : SATNASLFVLGVALSANGTFVYNGSDYGSCDPAQLPFS : 190
ABW83341.1 : SATNASLFVLGVALSANGTFVYNGSDYGSCDPAQLPFS : 190
```

FIG. 78(contd)

```
ABW83339.1 : SATNASLFVLGVALSANGTFVYNGSDYGSCDPAQLPFS : 190
ABW83345.1 : SATNASLFVLGVALSANGTFVYNGSDYGSCDPAQLPFS : 190
ABW83347.1 : SATNASLFVLGVALSANGTFVYNGLDYGSCDPAQLPFS : 190
ABW83325.1 : SATNASRFVLGVALSANGTFVYNGSDYGSCDPAQLPFS : 190
ABW83349.1 : SATNASRFVLGVALSANGTFVYNGSDYGSCDPAQLPFS : 190
ABW83351.1 : SATNASRFVLGVALSANGTFVYNGSDYGSCDPAQLPFS : 190
ABW83335.1 : SATNASLFVLGVALSANGTFVYNGSDYGSCDPAQLPFS : 190
ABW83333.1 : SATNASLFVLGVALSANGTFVYNGSDYGSCDPAQLPFS : 190
ABW83353.1 : SATNASLFVLGVALSANGTFVYNGSDYGSCDPAQLPFS : 190
ABW83337.1 : SATNASLFVLGVALSANGTFVYNGSDYGSCDPAQLPFS : 190
ABW83355.1 : SATNASLFVLGVALSANGTFVYNGLDYGSCDPAQLPFS : 190
ABW83327.1 : SATNASLFVLGVALSANGTFVYNGSDYGSCDPAQLPFS : 190
ABW83343.1 : SATNASLFVLGVALSANGTFVYNGSDYGSCDPAQLPFS : 190
ABW83329.1 : SATNASRFVLGVALSANGTFVYNGSDYGSCDPAQLPFS : 190
ABW83357.1 : SATNASLFVLGVALSANGTFVYNGSDYGSCDPAQLPFS : 190
ABW83359.1 : SATNASLFVLGVALSANGTFVYNGLDYGSCDPAQLPFS : 190
ABW83361.1 : SATNASRFVLGVALSANGTFVYNGSDYGSCDPAQLPFS : 190
ABW83363.1 : SATNASRFVLGVALSANGTFVYNGSDYGSCDPAQLPFS : 190
ABW83331.1 : SATNASLFVLGVALSANGTFVYNGSDYGSCDPAQLPFS : 190
ABW83365.1 : SATNASRFVLGVALSANGTFVYNGSDYGSCDPAQLPFS : 190
ABW83367.1 : SATNASRFVLGVALSANGTFVYNGSDYGSCDPAQLPFS : 190
ABW83369.1 : SATNASLFVLGVALSANGTFVYNGSDYGSCDPAQLPFS : 190
ABW83371.1 : SATNASLFVLGVALSANGTFVYNGSDYGSCDPAQLPFS : 190
ABW83375.1 : SATNASRFVLGVALSANGTFVYNGSDYGSCDPAQLPFS : 190
ABW83373.1 : SATNASRFVLGVALSANGTFVYNGSDYGSCDPAQLPFS : 190
ABW83377.1 : SATNASRFVLGVALSANGTFVYNGSDYGSCDPAQLPFS : 190
```

```
                          200         *         220
AKC59448.1 : APRLGPSSVYTPGASRPTPPRTTTPPSSPRDPTPAPGD : 228
AKC42829.1 : APRLGPSSVYTPGASRPTPPRTTTSPSSPRDPTPAPGD : 228
AMB66029.1 : APRLGPSSVYTPGASRPTPPRTTTXXXXXRDPTPAPGD : 228
AMB66103.1 : APRLGPSSVYTPGASRPTPPRTTTXXXXXXXXXXAPGD : 228
AMB66172.1 : APRLGPSSVYTPGASRPTPPRTTTPPSSPRDXTPAPGD : 228
AMB66245.1 : APRLGPSSVYXXXXXXXXXXXXXXXXX--XXXXXXXXX : 226
AMB66322.1 : APRLGPSSVYTPGASRPTPPRXXXXXXXXXXXXX--XX : 226
AKC59519.1 : APRLGPSSVYTPGASRPTPPRTTTSPSSPRDPTPAPGD : 228
AKC59590.1 : APRLGPSSVYTPGASRPTPPRTTTSPSSPRDPTPAPGD : 228
AKC59306.1 : APRLGPSSVYTPGASRPTPPRTTTSPSSPRDPTPAPGD : 228
AKC59377.1 : APRLGPSSVYTPGASRPTPPRTTTSPSSPRDPTPAPGD : 228
YP_0091372 : APRLGPSSVYTPGASRPTPPRTTTPPSSPRDPTPAPGD : 228
CAB06714.1 : APRLGPSSVYTPGASRPTPPRTTTPPSSPRDPTPAPGD : 228
AEV91406.1 : APRLGPSSVYTPGASRPTPPRTTTPPSSPRDPTPAPGD : 228
ABW83305.1 : APRLGPSSVYTPGASRPTPPRTTTPPSSPRDPTPAPGD : 228
ABW83323.1 : APRLGPSSVYTPGASRPTPPRTTTPPSSPRDPTPAPGD : 228
ABW83307.1 : APRLGPSSVYTPGASRPTPPRTTTPPSSPRDPTPAPGD : 228
ABW83309.1 : APRLGPSSVYTPGASRPTPPRTTTPPSSPRDPTPAPGD : 228
ABW83311.1 : APRLGPSSVYTPGASRPTPPRTTTPPSSPRDPTPAPGD : 228
ABW83313.1 : APRLGPSSVYTPGASRPTPPRTTTSPSSPRDPTPAPGD : 228
ABW83315.1 : APRLGPSSVYTPGASRPTPPRTTTPPSSPRDPTPAPGD : 228
ABW83317.1 : APRLGPSSVYTPGASRPTPPRTTTPPSSPRDPTPAPGD : 228
ABW83319.1 : APRLGPSSVYTPGASRPTPPRTTTPPSSPRDPTPAPGD : 228
ABW83321.1 : APRLGPSSVYTPGASRPTPPRTTTPPSSPRDPTPAPGD : 228
ABW83397.1 : APRLGPSSVYTPGASRPTPPRTTTSPSSPRDPTPAPGD : 228
ABW83379.1 : APRLGPSSVYTPGASRPTPPRTTTPPSSPRDPTPAPGD : 228
ABW83395.1 : APRLGPSSVYTPGASRPTPPRTTTPPSSPRDPTPAPGD : 228
ABW83381.1 : APRLGPSSVYTPGASRPTPPRTTTPPSSPRDPTPAPGD : 228
ABW83383.1 : APRLGPSSVYTPGASRPTPPRTTTPPSSPRDPTPAPGD : 228
ABW83393.1 : APRLGPSSVYTPGASRPTPPRTTTPPSSPRDPTPAPGD : 228
ABW83385.1 : APRLGPSSVYTPGASRPTPPRTTTSPSSPRDPTPAPGD : 228
```

FIG. 78(contd)

```
ABW83387.1 : APRLGPSSVYTPGASRPTPPRTTTPPSSPRDPTPAPGD : 228
ABW83389.1 : APRLGPSSVYTPGASRPTPPRTTTSPSSPRDPTPAPGD : 228
ABW83391.1 : APRLGPSSVYTPGASRPTPPRTTTPPSSPRDPTPAPGD : 228
ABW83399.1 : APRLGPSSVYTPGASRPTPPRTTTPPSSPRDPTPAPGD : 228
AHG54731.1 : APRLGPSSVYTPGASRPTPPRTTTSPSSPRDPTPAPGD : 228
ABW83341.1 : APRLGPSSVYTPGASRPTPPRTTTPPSSPRDPTPAPGD : 228
ABW83339.1 : APRLGPSSVYTPGASRPTPPRTTTPPSSPRDPTPAPGD : 228
ABW83345.1 : APRLGPSSVYTPGASRPTPPRTTTLPSSPRDPTPAPGD : 228
ABW83347.1 : APRLGPSSVYTPGASRPTPPRTTTPPSSPRDPTPAPGD : 228
ABW83325.1 : APRLGPSSVYTPGASRPTPPRTTTSPSSPRDPTPAPGD : 228
ABW83349.1 : APRLGPSSVYTPGASRPTPPRTTTPPSSPRDPTPAPGD : 228
ABW83351.1 : APRLGPSSVYTPGASRPTPPRTTTSPSSPRDPTPAPGD : 228
ABW83335.1 : APRLGPSSVYTPGASRPTPPRTTTLPSSPRDPTPAPGD : 228
ABW83333.1 : APRLGPSSVYTPGASRPTPPRTTTLPSSPRDPTPAPGD : 228
ABW83353.1 : APRLGPSSVYTPGASRPTPPRTTTLPSSPRDPTPAPGD : 228
ABW83337.1 : APRLGPSSVYTPGASRPTPPRTTTPPSSPRDPTPAPGD : 228
ABW83355.1 : APRLGPSSVYTPGASRPTPPRTTTSPSSPRDPTPAPGD : 228
ABW83327.1 : APRLGPSSVYTPGASRPTPPRTTTSPSSPRDPTPAPGD : 228
ABW83343.1 : APRLGPSSVYTPGASRPTPPRTTTPPSSPRDPTPAPGD : 228
ABW83329.1 : APRLGPSSVYTPGASRPTPPRTTTSPSSPRDPTPAPGD : 228
ABW83357.1 : APRLGPSSVYTPGASRPTPPRTTTPPSSPRDPTPAPGD : 228
ABW83359.1 : APRLGPSSVYTPGASRPTPPRTTTPPSSPRDPTPAPGD : 228
ABW83361.1 : APRLGPSSVYTPGASRPTPPRTTTPPSSPRDPTPAPGD : 228
ABW83363.1 : APRLGPSSVYTPGASRPTPPRTTTPPSSPRDPTPAPGD : 228
ABW83331.1 : APRLGPSSVYTPGASRPTPPRTTTLPSSPRDPTPAPGD : 228
ABW83365.1 : APRLGPSSVYTPGASRPTPPRTTTSPSSPRDPTPAPGD : 228
ABW83367.1 : APRLGPSSVYTPGASRPTPPRTTTSPSSPRDPTPAPGD : 228
ABW83369.1 : APRLGPSSVYTPGASRPTPPRTTTLPSSPRDPTPAPGD : 228
ABW83371.1 : APRLGPSSVYTPGASRPTPPRTTTLPSSPRDPTPAPGD : 228
ABW83375.1 : APRLGPSSVYTPGASRPTPPRTTTSPSSPRDPTPAPGD : 228
ABW83373.1 : APRLGPSSVYTPGASRPTPPRTTTPPSSPRDPTPAPGD : 228
ABW83377.1 : APRLGPSSVYTPGASRPTPPRTTTSPSSPRDPTPAPGD : 228
```

```
                      *        240        *
AKC59448.1 : TGTPA--PASGERAPPNSTRSASESRHRLT : 256
AKC42829.1 : TGTPA--PASGERAPPNSTRSASESRHRLT : 256
AMB66029.1 : TGTPA--PASGERAPPNSTRSASESRHRLT : 256
AMB66103.1 : TGTPA--PASGEIAPPNSTRSASESRHRLT : 256
AMB66172.1 : TGTPA--PASGEIAPPNSTRSASESRHRLT : 256
AMB66245.1 : XXXXXPAPASGERAPPNSTRSASESRHRLT : 256
AMB66322.1 : XXXXXXXXXXXXIAPPNSTRSASESRHRLT : 256
AKC59519.1 : TGTPA--PASGERAPPNSTRSASESRHRLT : 256
AKC59590.1 : TGTPA--PASGERAPPNSTRSASESRHRLT : 256
AKC59306.1 : TGTPA--PASGERAPPNSTRSASESRHRLT : 256
AKC59377.1 : TGTPA--PASGERAPPNSTRSASESRHRLT : 256
YP_0091372 : TGTPA--PASGEIAPPNSTRSASESRHRLT : 256
CAB06714.1 : TGTPA--PASGEIAPPNSTRSASESRHRLT : 256
AEV91406.1 : TGTPA--PASGEIAPPNSTRSASESRHRLT : 256
ABW83305.1 : TGTPA--PASGESAPPNSTRSASESRHRLT : 256
ABW83323.1 : TGTPA--PASGESAPPNSTRSASESRHRLT : 256
ABW83307.1 : TGTPA--PASGEKAPPNSTRSASESRHRLT : 256
ABW83309.1 : TGTPA--PASGERAPPNSTRSASESRHRLT : 256
ABW83311.1 : TGTPA--PASGERAPPNSTRSASESRHRLT : 256
ABW83313.1 : TGTPA--PASGERAPPNSTRSASESRHRLT : 256
ABW83315.1 : TGTPA--PASGESAPPNSTRSASESRHRLT : 256
ABW83317.1 : TGTPA--PASGESAPPNSTRSASESRHRLT : 256
ABW83319.1 : TGTPA--PASGERAPPNSTRSASESRHRLT : 256
ABW83321.1 : TGTPA--PASGERAPPNSTRSASESRHRLT : 256
ABW83397.1 : TGTPA--PASGERAPPNSTRSASESRHRLT : 256
ABW83379.1 : TGTPA--PASGERAPPNSTRSASESRHRLT : 256
```

| | | | | |
|---|---|---|---|---|
| ABW83395.1 | : | TGTPA--PASGEIAPPNSTRSASESRHRLT | : | 256 |
| ABW83381.1 | : | TGTPA--PASGERAPPNSTRSASESRHRLT | : | 256 |
| ABW83383.1 | : | TGTPA--PASGERAPPNSTRSASESRHRLT | : | 256 |
| ABW83393.1 | : | TGTPA--PASGEIAPPNSTRSASESRHRLT | : | 256 |
| ABW83385.1 | : | TGTPA--PASGERAPPNSTRSASESRHRLT | : | 256 |
| ABW83387.1 | : | TGTPA--PASGESAPPNSTRSASESRHRLT | : | 256 |
| ABW83389.1 | : | TGTPAPAPASGESAPPNSTRSASESRHRLT | : | 258 |
| ABW83391.1 | : | TGTPA--PASGESAPPNSTRSASESRHRLT | : | 256 |
| ABW83399.1 | : | TGTPA--PASGESAPPNSTRSASESRHRLT | : | 256 |
| AHG54731.1 | : | TGTPA--PASGERAPPNSTRSASESRHRLT | : | 256 |
| ABW83341.1 | : | TGTPA--PASGERAPPNSTRSASESRHRLT | : | 256 |
| ABW83339.1 | : | TGTPA--PASGERAPPNSTRSASESRHRLT | : | 256 |
| ABW83345.1 | : | TGTPA--PASGERAPPNSTRSASESRHRLT | : | 256 |
| ABW83347.1 | : | TGTPA--PASGERAPPNSTRSASESRHRLT | : | 256 |
| ABW83325.1 | : | TGTPA--PASGERAPPNSTRSASESRHRLT | : | 256 |
| ABW83349.1 | : | TGTPA--PASGERAPPNSTRSASESRHRLT | : | 256 |
| ABW83351.1 | : | TGTPA--PASGERAPPNSTRSASESRHRLT | : | 256 |
| ABW83335.1 | : | TGTPA--PASGERAPPNSTRSASESRHRLT | : | 256 |
| ABW83333.1 | : | TGTPA--PASGERAPPNSTRSASESRHRLT | : | 256 |
| ABW83353.1 | : | TGTPA--PASGERAPPNSTRSASESRHRLT | : | 256 |
| ABW83337.1 | : | TGTPA--PASGERAPPNSTRSASESRHRLT | : | 256 |
| ABW83355.1 | : | TGTPA--PASGERAPPNSTRSASESRHRLT | : | 256 |
| ABW83327.1 | : | TGTPA--PASGERAPPNSTRSASESRHRLT | : | 256 |
| ABW83343.1 | : | TGTPA--PASGERAPPNSTRSASESRHRLT | : | 256 |
| ABW83329.1 | : | TGTPA--PASGERAPPNSTRSASESRHRLT | : | 256 |
| ABW83357.1 | : | TGTPA--PASGERAPPNSTRSASESRHRLT | : | 256 |
| ABW83359.1 | : | TGTPA--PASGERAPPNSTRSASESRHRLT | : | 256 |
| ABW83361.1 | : | TGTPA--PASGESAPPNSTRSASESRHRLT | : | 256 |
| ABW83363.1 | : | TGTPA--PASGERAPPNSTRSASESRHRLT | : | 256 |
| ABW83331.1 | : | TGTPA--PASGERAPPNSTRSASESRHRLT | : | 256 |
| ABW83365.1 | : | TGTPA--PASGERAPPNSTRSASESRHRLT | : | 256 |
| ABW83367.1 | : | TGTPA--PASGERAPPNSTRSASESRHRLT | : | 256 |
| ABW83369.1 | : | TGTPA--PASGERAPPNSTRSASESRHRLT | : | 256 |
| ABW83371.1 | : | TGTPA--PASGERAPPNSTRSASESRHRLT | : | 256 |
| ABW83375.1 | : | TGTPAPAPASGERAPPNSTRSASESRHRLT | : | 258 |
| ABW83373.1 | : | TGTPA--PASGESAPPNSTRSASESRHRLT | : | 256 |
| ABW83377.1 | : | TGTPA--PASGERAPPNSTRSASESRHRLT | : | 256 |

FIG. 78(contd)

# FIG. 79

ATAGGCGGCGCATGAGAGAAGCCCAGACCAATTACCTACCCAAAATGGAGAAAGTTCACGTTGACATCGAGG
AAGACAGCCCATTCCTCAGAGCTTTGCAGCGGAGCTTCCCGCAGTTTGAGGTAGAAGCCAAGCAGGTCACTG
ATAATGACCATGCTAATGCCAGAGCGTTTTCGCATCTGGCTTCAAAACTGATCGAAACGGAGGTGGACCCATC
CGACACGATCCTTGACATTGGAAGTGCGCCCGCCCGCAGAATGTATTCTAAGCACAAGTATCATTGTATCTGTC
CGATGAGATGTGCGGAAGATCCGGACAGATTGTATAAGTATGCAACTAAGCTGAAGAAAAACTGTAAGGAAA
TAACTGATAAGGAATTGGACAAGAAAATGAAGGAGCTCGCCGCCGTCATGAGCGACCCTGACCTGGAAACTG
AGACTATGTGCCTCCACGACGACGAGTCGTGTCGCTACGAAGGGCAAGTCGCTGTTTACCAGGATGTATACGC
GGTTGACGGACCGACAAGTCTCTATCACCAAGCCAATAAGGGAGTTAGAGTCGCCTACTGGATAGGCTTTGA
CACCACCCCTTTTATGTTTAAGAACTTGGCTGGAGCATATCCATCATACTCTACCAACTGGGCCGACGAAACCG
TGTTAACGGCTCGTAACATAGGCCTATGCAGCTCTGACGTTATGGAGCGGTCACGTAGAGGGATGTCCATTCT
TAGAAAGAAGTATTTGAAACCATCCAACAATGTTCTATTCTCTGTTGGCTCGACCATCTACCACGAGAAGAGG
GACTTACTGAGGAGCTGGCACCTGCCGTCTGTATTTCACTTACGTGGCAAGCAAAATTACACATGTCGGTGTG
AGACTATAGTTAGTTGCGACGGGTACGTCGTTAAAAGAATAGCTATCAGTCCAGGCCTGTATGGGAAGCCTTC
AGGCTATGCTGCTACGATGCACCGCGAGGGATTCTTGTGCTGCAAAGTGACAGACACATTGAACGGGGAGAG
GGTCTCTTTTCCCGTGTGCACGTATGTGCCAGCTACATTGTGTGACCAAATGACTGGCATACTGGCAACAGAT
GTCAGTGCGGACGACGCGCAAAAACTGCTGGTTGGGCTCAACCAGCGTATAGTCGTCAACGGTCGCACCCAG
AGAAACACCAATACCATGAAAAATTACCTTTTGCCCGTAGTGGCCCAGGCATTTGCTAGGTGGGCAAAGGAAT
ATAAGGAAGATCAAGAAGATGAAAGGCCACTAGGACTACGAGATAGACAGTTAGTCATGGGGTGTTGTTGG
GCTTTTAGAAGGCACAAGATAACATCTATTTATAAGCGCCCGGATACCCAAACCATCATCAAAGTGAACAGCG
ATTTCCACTCATTCGTGCTGCCCAGGATAGGCAGTAACACATTGGAGATCGGGCTGAGAACAAGAATCAGGA
AAATGTTAGAGGAGCACAAGGAGCCGTCACCTCTCATTACCGCCGAGGACGTACAAGAAGCTAAGTGCGCAG
CCGATGAGGCTAAGGAGGTGCGTGAAGCCGAGGAGTTGCGCGCAGCTCTACCACCTTTGGCAGCTGATGTTG
AGGAGCCCACTCTGGAAGCCGATGTCGACTTGATGTTACAAGAGGCTGGGGCCGGCTCAGTGGAGACACCTC
GTGGCTTGATAAAGGTTACCAGCTACGATGGCGAGGACAAGATCGGCTCTTACGCTGTGCTTTCTCCGCAGGC
TGTACTCAAGAGTGAAAAATTATCTTGCATCCACCCTCTCGCTGAACAAGTCATAGTGATAACACACTCTGGCC
GAAAAGGGCGTTATGCCGTGGAACCATACCATGGTAAAGTAGTGGTGCCAGAGGGACATGCAATACCCGTCC
AGGACTTTCAAGCTCTGAGTGAAAGTGCCACCATTGTGTACAACGAACGTGAGTTCGTAAACAGGTACCTGCA
CCATATTGCCACACATGGAGGAGCGCTGAACACTGATGAAGAATATTACAAAACTGTCAAGCCCAGCGAGCA
CGACGGCGAATACCTGTACGACATCGACAGGAAACAGTGCGTCAAGAAAGAACTAGTCACTGGGCTAGGGCT
CACAGGCGAGCTGGTGGATCCTCCCTTCCATGAATTCGCCTACGAGAGTCTGAGAACACGACCAGCCGCTCCT
TACCAAGTACCAACCATAGGGGTGTATGGCGTGCCAGGATCAGGCAAGTCTGGCATCATTAAAAGCGCAGTC
ACCAAAAAAGATCTAGTGGTGAGCGCCAAGAAAGAAAACTGTGCAGAAATTATAAGGGACGTCAAGAAAAT
GAAAGGGCTGGACGTCAATGCCAGAACTGTGGACTCAGTGCTCTTGAATGGATGCAAACACCCCGTAGAGAC
CCTGTATATTGACGAAGCTTTTGCTTGTCATGCAGGTACTCTCAGAGCGCTCATAGCCATTATAAGACCTAAAA
AGGCAGTGCTCTGCGGGGATCCCAAACAGTGCGGTTTTTTTAACATGATGTGCCTGAAAGTGCATTTTAACCA
CGAGATTTGCACACAAGTCTTCCACAAAAGCATCTCTCGCCGTTGCACTAAATCTGTGACTTCGGTCGTCTCAA
CCTTGTTTTACGACAAAAAAATGAGAACGACGAATCCGAAAGAGACTAAGATTGTGATTGACACTACCGGCA
GTACCAAACCTAAGCAGGACGATCTCATTCTCACTTGTTTCAGAGGGTGGGTGAAGCAGTTGCAAATAGATTA
CAAAGGCAACGAAATAATGACGGCAGCTGCCTCTCAAGGGCTGACCCGTAAAGGTGTGTATGCCGTTCGGTA
CAAGGTGAATGAAAATCCTCTGTACGCACCCACCTCAGAACATGTGAACGTCCTACTGACCCGCACGGAGGAC
CGCATCGTGTGGAAAACACTAGCCGGCGACCCATGGATAAAAACACTGACTGCCAAGTACCCTGGGAATTTC
ACTGCCACGATAGAGGAGTGGCAAGCAGAGCATGATGCCATCATGAGGCACATCTTGGAGAGACCGGACCCT
ACCGACGTCTTCCAGAATAAGGCAAACGTGTGTTGGGCCAAGGCTTTAGTGCCGGTGCTGAAGACCGCTGGC
ATAGACATGACCACTGAACAATGGAACACTGTGGATTATTTTGAAACGGACAAAGCTCACTCAGCAGAGATA
GTATTGAACCAACTATGCGTGAGGTTCTTTGGACTCGATCTGGACTCCGGTCTATTTTCTGCACCCACTGTTCC
GTTATCCATTAGGAATAATCACTGGGATAACTCCCCGTCGCCTAACATGTACGGGCTGAATAAAGAAGTGGTC

FIG 79 (CONTD)

CGTCAGCTCTCTCGCAGGTACCCACAACTGCCTCGGGCAGTTGCCACTGGAAGAGTCTATGACATGAACACTG
GTACACTGCGCAATTATGATCCGCGCATAAACCTAGTACCTGTAAACAGAAGACTGCCTCATGCTTTAGTCCTC
CACCATAATGAACACCCACAGAGTGACTTTTCTTCATTCGTCAGCAAATTGAAGGGCAGAACTGTCCTGGTGG
TCGGGGAAAAGTTGTCCGTCCCAGGCAAAATGGTTGACTGGTTGTCAGACCGGCCTGAGGCTACCTTCAGAG
CTCGGCTGGATTTAGGCATCCCAGGTGATGTGCCCAAATATGACATAATATTTGTTAATGTGAGGACCCCATAT
AAATACCATCACTATCAGCAGTGTGAAGACCATGCCATTAAGCTTAGCATGTTGACCAAGAAAGCTTGTCTGC
ATCTGAATCCCGGCGGAACCTGTGTCAGCATAGGTTATGGTTACGCTGACAGGGCCAGCGAAAGCATCATTG
GTGCTATAGCGCGGCAGTTCAAGTTTTCCCGGGTATGCAAACCGAAATCCTCACTTGAAGAGACGGAAGTTCT
GTTTGTATTCATTGGGTACGATCGCAAGGCCCGTACGCACAATCCTTACAAGCTTTCATCAACCTTGACCAACA
TTTATACAGGTTCCAGACTCCACGAAGCCGGATGTGCACCCTCATATCATGTGGTGCGAGGGGATATTGCCAC
GGCCACCGAAGGAGTGATTATAAATGCTGCTAACAGCAAAGGACAACCTGGCGGAGGGGTGTGCGGAGCGC
TGTATAAGAAATTCCCGGAAAGCTTCGATTTACAGCCGATCGAAGTAGGAAAAGCGCGACTGGTCAAAGGTG
CAGCTAAACATATCATTCATGCCGTAGGACCAAACTTCAACAAAGTTTCGGAGGTTGAAGGTGACAAACAGTT
GGCAGAGGCTTATGAGTCCATCGCTAAGATTGTCAACGATAACAATTACAAGTCAGTAGCGATTCCACTGTTG
TCCACCGGCATCTTTTCCGGGAACAAAGATCGACTAACCCAATCATTGAACCATTTGCTGACAGCTTTAGACAC
CACTGATGCAGATGTAGCCATATACTGCAGGGACAAGAAATGGGAAATGACTCTCAAGGAAGCAGTGGCTAG
GAGAGAAGCAGTGGAGGAGATATGCATATCCGACGACTCTTCAGTGACAGAACCTGATGCAGAGCTGGTGA
GGGTGCATCCGAAGAGTTCTTTGGCTGGAAGGAAGGGCTACAGCACAAGCGATGGCAAAACTTTCTCATATT
TGGAAGGGACCAAGTTTCACCAGGCGGCCAAGGATATAGCAGAAATTAATGCCATGTGGCCCGTTGCAACGG
AGGCCAATGAGCAGGTATGCATGTATATCCTCGGAGAAAGCATGAGCAGTATTAGGTCGAAATGCCCCGTCG
AAGAGTCGGAAGCCTCCACACCACCTAGCACGCTGCCTTGCTTGTGCATCCATGCCATGACTCCAGAAAGAGT
ACAGCGCCTAAAAGCCTCACGTCCAGAACAAATTACTGTGTGCTCATCCTTTCCATTGCCGAAGTATAGAATCA
CTGGTGTGCAGAAGATCCAATGCTCCCAGCCTATATTGTTCTCACCGAAAGTGCCTGCGTATATTCATCCAAGG
AAGTATCTCGTGGAAACACCACCGGTAGACGAGACTCCGGAGCCATCGGCAGAGAACCAATCCACAGAGGG
GACACCTGAACAACCACCACTTATAACCGAGGATGAGACCAGGACTAGAACGCCTGAGCCGATCATCATCGA
AGAGGAAGAAGAGGATAGCATAAGTTTGCTGTCAGATGGCCCGACCCACCAGGTGCTGCAAGTCGAGGCAG
ACATTCACGGGCCGCCCTCTGTATCTAGCTCATCCTGGTCCATTCCTCATGCATCCGACTTTGATGTGGACAGTT
TATCCATACTTGACACCCTGGAGGGAGCTAGCGTGACCAGCGGGGCAACGTCAGCCGAGACTAACTCTTACTT
CGCAAAGAGTATGGAGTTTCTGGCGCGACCGGTGCCTGCGCCTCGAACAGTATTCAGGAACCCTCCACATCCC
GCTCCGCGCACAAGAACACCGTCACTTGCACCCAGCAGGGCCTGCTCGAGAACCAGCCTAGTTTCCACCCCGC
CAGGCGTGAATAGGGTGATCACTAGAGAGGAGCTCGAGGCGCTTACCCCGTCACGCACTCCTAGCAGGTCGG
TCTCGAGAACCAGCCTGGTCTCCAACCCGCCAGGCGTAAATAGGGTGATTACAAGAGAGGAGTTTGAGGCGT
TCGTAGCACAACAACAATGACGGTTTGATGCGGGTGCATACATCTTTTCCTCCGACACCGGTCAAGGGCATTT
ACAACAAAAATCAGTAAGGCAAACGGTGCTATCCGAAGTGGTGTTGGAGAGGACCGAATTGGAGATTTCGTA
TGCCCCGCGCCTCGACCAAGAAAAAGAAGAATTACTACGCAAGAAATTACAGTTAAATCCCACACCTGCTAAC
AGAAGCAGATACCAGTCCAGGAAGGTGGAGAACATGAAAGCCATAACAGCTAGACGTATTCTGCAAGGCCTA
GGGCATTATTTGAAGGCAGAAGGAAAAGTGGAGTGCTACCGAACCCTGCATCCTGTTCCTTTGTATTCATCTA
GTGTGAACCGTGCCTTTTCAAGCCCCAAGGTCGCAGTGGAAGCCTGTAACGCCATGTTGAAAGAGAACTTTCC
GACTGTGGCTTCTTACTGTATTATTCCAGAGTACGATGCCTATTTGGACATGGTTGACGGAGCTTCATGCTGCT
TAGACACTGCCAGTTTTTGCCCTGCAAAGCTGCGCAGCTTTCCAAAGAAACACTCCTATTTGGAACCCACAATA
CGATCGGCAGTGCCTTCAGCGATCCAGAACACGCTCCAGAACGTCCTGGCAGCTGCCACAAAAAGAAATTGC
AATGTCACGCAAATGAGAGAATTGCCCGTATTGGATTCGGCGGCCTTTAATGTGGAATGCTTCAAGAAATATG
CGTGTAATAATGAATATTGGGAAACGTTTAAAGAAAACCCCATCAGGCTTACTGAAGAAAACGTGGTAAATTA
CATTACCAAATTAAAAGGACCAAAAGCTGCTGCTCTTTTTGCGAAGACACATAATTTGAATATGTTGCAGGAC
ATACCAATGGACAGGTTTGTAATGGACTTAAAGAGAGACGTGAAAGTGACTCCAGGAACAAAACATACTGAA
GAACGGCCCAAGGTACAGGTGATCCAGGCTGCCGATCCGCTAGCAACAGCGTATCTGTGCGGAATCCACCGA
GAGCTGGTTAGGAGATTAAATGCGGTCCTGCTTCCGAACATTCATACACTGTTTGATATGTCGGCTGAAGACT
TTGACGCTATTATAGCCGAGCACTTCCAGCCTGGGGATTGTGTTCTGGAAACTGACATCGCGTCGTTTGATAA

AAGTGAGGACGACGCCATGGCTCTGACCGCGTTAATGATTCTGGAAGACTTAGGTGTGGACGCAGAGCTGTT
GACGCTGATTGAGGCGGCTTTCGGCGAAATTTCATCAATACATTTGCCCACTAAAACTAAATTTAAATTCGGAG
CCATGATGAAATCTGGAATGTTCCTCACACTGTTTGTGAACACAGTCATTAACATTGTAATCGCAAGCAGAGTG
TTGAGAGAACGGCTAACCGGATCACCATGTGCAGCATTCATTGGAGATGACAATATCGTGAAAGGAGTCAAA
TCGGACAAATTAATGGCAGACAGGTGCGCCACCTGGTTGAATATGGAAGTCAAGATTATAGATGCTGTGGTG
GGCGAGAAAGCGCCTTATTTCTGTGGAGGGTTTATTTTGTGTGACTCCGTGACCGGCACAGCGTGCCGTGTGG
CAGACCCCCTAAAAAGGCTGTTTAAGCTTGGCAAACCTCTGGCAGCAGACGATGAACATGATGATGACAGGA
GAAGGGCATTGCATGAAGAGTCAACACGCTGGAACCGAGTGGGTATTCTTTCAGAGCTGTGCAAGGCAGTAG
AATCAAGGTATGAAACCGTAGGAACTTCCATCATAGTTATGGCCATGACTACTCTAGCTAGCAGTGTTAAATC
ATTCAGCTACCTGAGAGGGGCCCCTATAACTCTCTACGGCTAACCTGAATGGACTACGACATAGTCTAGTCCG
CCAAG................INSERT.................GGCGCGCCCACCCAGCGGCCGC**ATACAGCAGCAATTGGCAAGCTGCT
TACATAGAACTCGCGGCGATTGGCATGCCGCCTTAAAATTTTTATTTTATTTTTCTTTTCTTTTCCGAATCGGA
TTTTGTTTTTAATATTTC**AAAAAAAAAAAAAAAAAAAAAAAAAAAAAAAAAAAAAAAAgaagagcgtttaaacacgt
gatatctggcctcatgggccttcctttcactgcccgctttccagtcgggaaacctgtcgtgccagctgcattaacatggtcatagctgtttccttgcg
tattgggcgctctccgcttcctcgctcactgactcgctgcgctcggtcgttcgggtaaagcctggggtgcctaatgagcaaaaggccagcaaaag
gccaggaaccgtaaaaaggccgcgttgctggcgtttttccataggctccgcccccctgacgagcatcacaaaaatcgacgctcaagtcagaggt
ggcgaaacccgacaggactataaagataccaggcgtttccccctggaagctccctcgtgcgctctcctgttccgaccctgccgcttaccggatac
ctgtccgcctttctcccttcgggaagcgtggcgctttctcatagctcacgctgtaggtatctcagttcggtgtaggtcgttcgctccaagctgggctg
tgtgcacgaaccccccgttcagcccgaccgctgcgccttatccggtaactatcgtcttgagtccaacccggtaagacacgacttatcgccactggc
agcagccactggtaacaggattagcagagcgaggtatgtaggcggtgctacagagttcttgaagtggtggcctaactacggctacactagaag
aacagtatttggtatctgcgctctgctgaagccagttaccttcggaaaaagagttggtagctcttgatccggcaaacaaaccaccgctggtagcg
gtggtttttttgtttgcaagcagcagattacgcgcagaaaaaaaggatctcaagaagatcctttgatcttttctacggggtctgacgctcagtgga
acgaaaactcacgttaagggattttggtcatgaatacacggtgcctgactgcgttagcaatttaactgtgataaactaccgcattaaagcttatcg
atgataagctgtcaaacatgagaattcttagaaaaactcatcgagcatcaaatgaaactgcaatttattcatatcaggattatcaataccatattt
ttgaaaaagccgtttctgtaatgaaggagaaaactcaccgaggcagttccataggatggcaagatcctggtatcggtctgcgattccgactcgtc
caacatcaatacaacctattaatttcccctcgtcaaaaataaggttatcaagtgagaaatcaccatgagtgacgactgaatccggtgagaatggc
aaaagcttatgcatttctttccagacttgttcaacaggccagccattacgctcgtcatcaaaatcactcgcatcaaccaaaccgttattcattcgtg
attgcgcctgagcgagacgaaatacgcgatcgctgttaaaaggacaattacaaacaggaatcgaatgcaaccggcgcaggaacactgccagc
gcatcaacaatattttcacctgaatcaggatattcttctaatacctggaatgctgttttcccggggatcgcagtggtgagtaaccatgcatcatcag
gagtacggataaaatgcttgatggtcggaagaggcataaattccgtcagccagtttagtctgaccatctcatctgtaacatcattggcaacgctac
ctttgccatgtttcagaaacaactctggcgcatcgggcttcccatacaatcgatagattgtcgcacctgattgcccgacattatcgcgagcccattt
atacccatataaatcagcatccatgttggaatttaatcgcggcctcgagcaagacgtttcccgttgaatatggctcataacaccccttgtattactg
tttatgtaagcagacagttttattgttcatgagcggatacatatttgaatgtatttagaaaaataaacaaataggggttccgcgcacatttccccga
aaagtgccacctaaattgtaagcgttaatattttgttaaaattcgcgttaaatttttgttaaatcagctcattttttaaccaataggccgaaatcggc
aaaatcccttataaatcaaaagaatagaccgagatagggttgagtggccgctacagggcgctcccattcgccattcaggctgcgcaactgttgg
gaagggcgtttcggtgcgggcctcttcgctattacgccagctggcgaaagggggatgtgctgcaaggcgattaagttgggtaacgccagggtttt
cccagtcacacgcgtaatacgactcactatag

# FIG. 79(contd)

# FIG. 80A

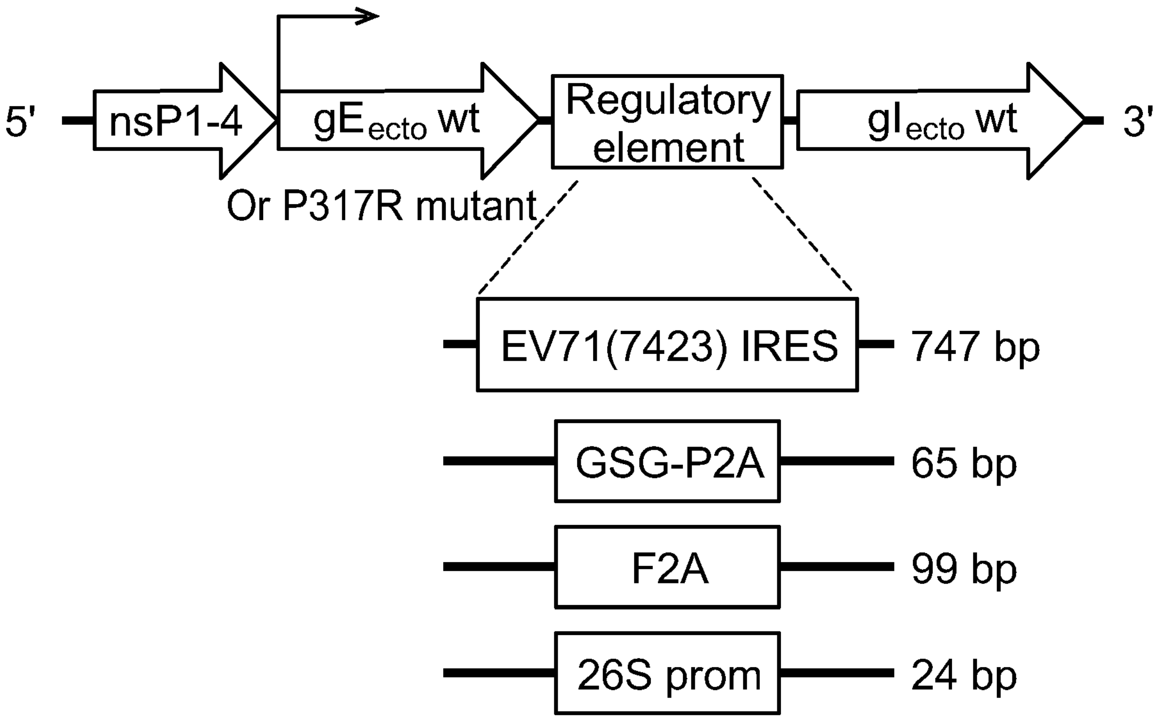


# FIG. 80B

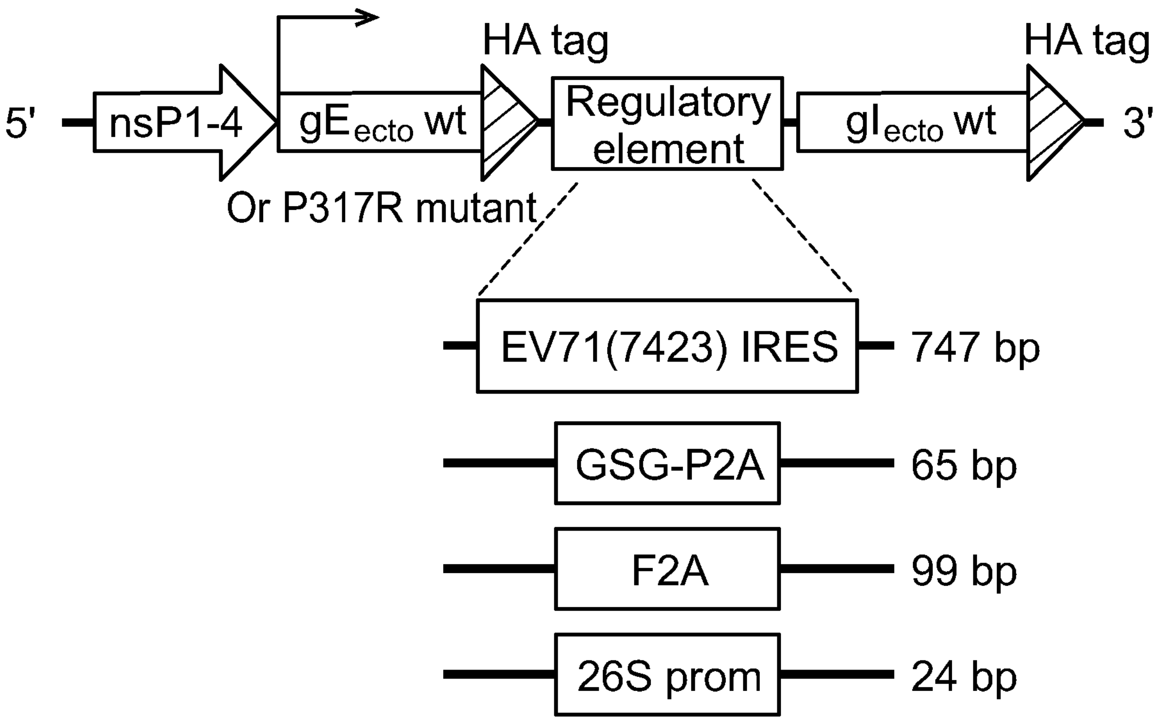

Europäisches Patentamt
European Patent Office
Office européen des brevets

# EUROPEAN SEARCH REPORT

Application Number
EP 21 15 2594

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EGAN KEVIN ET AL: "Vaccines to prevent genital herpes", TRANSLATIONAL RESEARCH, ELSEVIER, AMSTERDAM, NL, vol. 220, 16 March 2020 (2020-03-16), pages 138-152, XP086179500, ISSN: 1931-5244, DOI: 10.1016/J.TRSL.2020.03.004 [retrieved on 2020-03-16] * abstract * | 1,4-6, 8-10, 12-15, 17-20 | INV. A61K39/245 C07K14/035 |
| Y,D | SITA AWASTHI ET AL: "A trivalent subunit antigen glycoprotein vaccine as immunotherapy for genital herpes in the guinea pig genital infection model", HUMAN VACCINES & IMMUNOTHERAPEUTICS, vol. 13, no. 12, 8 May 2017 (2017-05-08), pages 2785-2793, XP055668432, US ISSN: 2164-5515, DOI: 10.1080/21645515.2017.1323604 * figures 3-5 * | 1,4-6, 8-10, 12-15, 17-20 | |
| Y | US 2013/028925 A1 (FRIEDMAN HARVEY [US] ET AL) 31 January 2013 (2013-01-31) * paragraph [0576]; figure 54 * | 1,4-6, 8-10, 12-15, 17-20 | TECHNICAL FIELDS SEARCHED (IPC) A61K C07K |

-/--

~~The present search report has been drawn up for all claims~~

6

| Place of search | Date of completion of the search | Examiner |
|---|---|---|
| Munich | 24 September 2021 | Meyer, Wolfram |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

page 1 of 2

Europäisches Patentamt
European Patent Office
Office européen des brevets

# EUROPEAN SEARCH REPORT

Application Number
EP 21 15 2594

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | S Basu ET AL: "Characterization of regions of herpes simplex virus type 1 glycoprotein E involved in binding the Fc domain of monomeric IgG and in forming a complex with glycoprotein I", The Journal of Immunology, 1 January 1995 (1995-01-01), page 260, XP055667398, United States Retrieved from the Internet: URL:https://www.jimmunol.org/content/jimmunol/154/1/260.full.pdf * figure 1 * ----- | 1,4-6, 8-10, 12-15, 17-20 | |
| T | WO 2006/004878 A1 (WYETH CORP [US]; VISALLI ROBERT J [US] ET AL.) 12 January 2006 (2006-01-12) * abstract * ----- | | |
| A | M. KALANTARI-DEHAGHI ET AL: "Discovery of Potential Diagnostic and Vaccine Antigens in Herpes Simplex Virus 1 and 2 by Proteome-Wide Antibody Profiling", JOURNAL OF VIROLOGY, vol. 86, no. 8, 15 April 2012 (2012-04-15), pages 4328-4339, XP055075306, ISSN: 0022-538X, DOI: 10.1128/JVI.05194-11 * table 1 * ----- | 2,4,5,7, 8,11,13, 14,16-20 | TECHNICAL FIELDS SEARCHED (IPC) |
| A | CUNNINGHAM ANTHONY L ET AL: "Vaccine development: From concept to early clinical testing", VACCINE, ELSEVIER, AMSTERDAM, NL, vol. 34, no. 52, 18 October 2016 (2016-10-18), pages 6655-6664, XP029837839, ISSN: 0264-410X, DOI: 10.1016/J.VACCINE.2016.10.016 * the whole document * ----- | 1-20 | |

~~The present search report has been drawn up for all claims~~

6

| Place of search | Date of completion of the search | Examiner |
|---|---|---|
| Munich | 24 September 2021 | Meyer, Wolfram |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

Europäisches Patentamt
European Patent Office
Office européen des brevets

Application Number
EP 21 15 2594

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☒ Only part of the further search fees have been paid within the fixed time limit. The present European search report has been drawn up for those parts of the European patent application which relate to the inventions in respect of which search fees have been paid, namely claims:

1, 2, 17-20(completely); 3-16(partially)

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 21 15 2594

The Search Division considers that the present European patent application does not comply with the requirements of unity of invention and relates to several inventions or groups of inventions, namely:

1. claims: 1, 17, 19(completely); 3-6, 8-10, 12-15(partially)

   Fc receptor from HSV2 for use in treating a subject infected with HSV1.

   ---

2. claims: 2, 18, 20(completely); 3-5, 7, 8, 13, 14, 16(partially)

   Fc receptor from HSV1 for use in treating a subject infected with HSV2.

   ---

3. claims: 3-5, 7, 8, 10, 13, 14, 16(all partially)

   Fc receptor from HSV1 for use in treating a subject infected with HSV1.

   ---

4. claims: 3-6, 8-16(all partially)

   Fc receptor from HSV2 for use in treating a subject infected with HSV2.

   ---

# ANNEX TO THE EUROPEAN SEARCH REPORT ON EUROPEAN PATENT APPLICATION NO. EP 21 15 2594

This annex lists the patent family members relating to the patent documents cited in the above-mentioned European search report.
The members are as contained in the European Patent Office EDP file on
The European Patent Office is in no way liable for these particulars which are merely given for the purpose of information.

24-09-2021

| Patent document cited in search report | Publication date | Patent family member(s) | Publication date |
|---|---|---|---|
| US 2013028925 A1 | 31-01-2013 | NONE | |
| WO 2006004878 A1 | 12-01-2006 | US 2008089910 A1 | 17-04-2008 |
| | | WO 2006004878 A1 | 12-01-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5340740 A
- US 5656479 A
- US 5830510 A
- US 6114168 A
- US 6500668 B
- EP 0787180 B
- EP 03291813
- WO 03043415 A
- WO 03076601 A
- WO 2005042728 A
- US 4436727 A
- US 4912094 A
- WO 9850399 A
- WO 0134617 A
- WO 0212258 A
- WO 3065806 A
- WO 04062599 A
- WO 06016997 A
- WO 0612425 A
- WO 03066065 A
- WO 0190129 A
- WO 9633739 A
- WO 2007068907 A
- US 5057540 A
- WO 9611711 A
- EP 0822831 A
- WO 9517210 A
- EP 0382271 B1


### Non-patent literature cited in the description


- **HOWARD, PAUL W. et al.** Herpes simplex virus gE/gI extracellular domains promote axonal transport and spread from neurons to epithelial cells. *Journal of virology,* 2014, vol. 88.19, 11178-11186
- **NDJAMEN, BLAISE et al.** The herpes virus Fc receptor gE-gI mediates antibody bipolar bridging to clear viral antigens from the cell surface. *PLoS pathogens,* 2014, vol. 10.3, e1003961
- **DUBIN, G. et al.** Herpes simplex virus type 1 Fc receptor protects infected cells from antibody-dependent cellular cytotoxicity. *Journal of virology,* 1991, vol. 65.12, 7046-7050
- **SPRAGUE, ELIZABETH R. et al.** Crystal structure of the HSV1 Fc receptor bound to Fc reveals a mechanism for antibody bipolar bridging. *PLoS biology,* 2006, vol. 4.6, e148
- **JOHNSTON ; CHRISTINE ; SAMI L. GOTTLIEB ; ANNA WALD.** Status of vaccine research and development of vaccines for herpes simplex virus. *Vaccine,* 2016, vol. 34.26, 2948-2952
- **VAN WAGONER, NICHOLAS et al.** Effects of different doses of GEN-003, a therapeutic vaccine for genital herpes simplex virus-2, on viral shedding and lesions: results of a randomized placebo-controlled trial. *The Journal of infectious diseases,* 2018, vol. 218.12, 1890-1899
- **AWASTHI, SITA et al.** Blocking herpes simplex virus 2 glycoprotein E immune evasion as an approach to enhance efficacy of a trivalent subunit antigen vaccine for genital herpes. *Journal of virology,* 2014, vol. 88.15, 8421-8432
- **AWASTHI, SITA et al.** An HSV2 trivalent vaccine is immunogenic in rhesus macaques and highly efficacious in guinea pigs. *PLoS pathogens,* 2017, vol. 13.1, e1006141
- **AWASTHI, SITA et al.** A trivalent subunit antigen glycoprotein vaccine as immunotherapy for genital herpes in the guinea pig genital infection model. *Human vaccines & immunotherapeutics,* 2017, vol. 13.12, 2785-2793
- **HOOK, LAUREN M. et al.** A trivalent gC2/gD2/gE2 vaccine for herpes simplex virus generates antibody responses that block immune evasion domains on gC2 better than natural infection. *Vaccine,* 2019, vol. 37.4, 664-669
- **CAIRNS, TINA M. et al.** Patient-specific neutralizing antibody responses to herpes simplex virus are attributed to epitopes on gD, gB, or both and can be type specific. *Journal of virology,* 2015, vol. 89.18, 9213-9231
- **CAIRNS, TINA M. et al.** Dissection of the antibody response against herpes simplex virus glycoproteins in naturally infected humans. *Journal of virology,* 2014, vol. 88.21, 12612-12622
- **MOKREJŠ, MARTIN et al.** IRESite: the database of experimentally verified IRES structures. *Nucleic acids research,* 2006, vol. 34 (1), D125-D130, www. iresite. org
- **SZYMCZAK et al.** *Nature Biotechnology,* 2004, vol. 22, 589-594
- **DONNELLY et al.** *J Gen Virol.,* 2001, vol. 82, 1013-25

- *Vaccine,* 2009, vol. 27, 4975-4982
- **SANDANA.** Bioseparation of Proteins. Academic Press, Inc, 1997
- **BOLLAG et al.** Protein Methods. Wiley-Liss, 1996
- **WALKER.** The Protein Protocols Handbook. Humana Press, 1996
- **HARRIS ; ANGAL.** Protein Purification Applications: A Practical Approach. IRL Press at Oxford, 1990
- **SCOPES.** Protein Purification: Principles and Practice. Springer Verlag, 1993
- **JANSON ; RYDEN.** Protein Purification: Principles, High Resolution Methods and Applications. Wiley-VCH, 1998
- **WALKER.** Protein Protocols on CD-ROM. Humana Press, 1998
- **FOX et al.** *Clin. Vaccine Immunol,* 2012, vol. 19, 1633
- **ALVING et al.** *Curr Opin in Immunol,* 2012, vol. 24, 310
- **LACAILLE-DUBOIS ; WAGNER.** A review of the biological and pharmacological activities of saponins. *Phytomedicine,* 1996, vol. 2, 363
- Saponin adjuvants. **DALSGAARD et al.** Archiv. fur die gesamte Virusforschung. Springer Verlag, 1974, vol. 44, 243
- **KENSIL et al.** *J. Immunol.,* 1991, vol. 146, 431
- **KENSIL, C. R.** Saponins as vaccine adjuvants. *Crit Rev Ther Drug Carrier Syst,* 1996, vol. 12 (1-2), 1-55
- **KENSIL.** O'Hagan: Vaccine Adjuvants: preparation methods and research protocols. Homana Press, 2000
- **STRYER et al.** Biochemistry. 2002, 44-49
- **BENJAMIN LEWIN.** Genes V. Oxford University Press, 1994
- The Encyclopedia of Molecular Biology. Blackwell Science Ltd, 1994
- Molecular Biology and Biotechnology: a Comprehensive Desk Reference. VCH Publishers, Inc, 1995
- *Eur. J. Biochem.,* 1984, vol. 138, 9-37
- **HUANG ; PANG.** *Assay Drug Dev Technol.,* 2012, vol. 10 (1), 88-96

SEQUENCE LISTING

<110> GlaxoSmithKline Biologicals s.a.

<120> THERAPEUTIC VIRAL VACCINE

<130> VB66997P EP

<160> 135

<170> PatentIn version 3.5

<210> 1
<211> 548
<212> PRT
<213> herpes simplex virus 2

<400> 1

```
Met Ala Arg Gly Ala Gly Leu Val Phe Phe Val Gly Val Trp Val Val
1               5                   10                  15

Ser Cys Leu Ala Ala Ala Pro Arg Thr Ser Trp Lys Arg Val Thr Ser
            20                  25                  30

Gly Glu Asp Val Val Leu Leu Pro Ala Pro Ala Gly Pro Glu Glu Arg
        35                  40                  45

Thr Arg Ala His Lys Leu Leu Trp Ala Ala Glu Pro Leu Asp Ala Cys
    50                  55                  60

Gly Pro Leu Arg Pro Ser Trp Val Ala Leu Trp Pro Pro Arg Arg Val
65                  70                  75                  80

Leu Glu Thr Val Val Asp Ala Ala Cys Met Arg Ala Pro Glu Pro Leu
                85                  90                  95

Ala Ile Ala Tyr Ser Pro Pro Phe Pro Ala Gly Asp Glu Gly Leu Tyr
            100                 105                 110

Ser Glu Leu Ala Trp Arg Asp Arg Val Ala Val Val Asn Glu Ser Leu
        115                 120                 125

Val Ile Tyr Gly Ala Leu Glu Thr Asp Ser Gly Leu Tyr Thr Leu Ser
    130                 135                 140

Val Val Gly Leu Ser Asp Glu Ala Arg Gln Val Ala Ser Val Val Leu
145                 150                 155                 160

Val Val Glu Pro Ala Pro Val Pro Thr Pro Thr Pro Asp Asp Tyr Asp
                165                 170                 175
```

Glu Glu Asp Asp Ala Gly Val Ser Glu Arg Thr Pro Val Ser Val Pro
180 185 190

Pro Pro Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro Pro Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr Ser
530 535 540

Ser Val Leu Trp
545

<210> 2
<211> 372
<212> PRT
<213> herpes simplex virus 2

<400> 2

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

```
Cys Pro Arg Arg Pro Ala Val Ala Phe Thr Leu Cys Arg Ser Thr His
            100                 105                 110

His Ala His Ser Pro Ala Tyr Pro Thr Leu Glu Leu Gly Leu Ala Arg
        115                 120                 125

Gln Pro Leu Leu Arg Val Arg Thr Ala Thr Arg Asp Tyr Ala Gly Leu
    130                 135                 140

Tyr Val Leu Arg Val Trp Val Gly Ser Ala Thr Asn Ala Ser Leu Phe
145                 150                 155                 160

Val Leu Gly Val Ala Leu Ser Ala Asn Gly Thr Phe Val Tyr Asn Gly
                165                 170                 175

Ser Asp Tyr Gly Ser Cys Asp Pro Ala Gln Leu Pro Phe Ser Ala Pro
            180                 185                 190

Arg Leu Gly Pro Ser Ser Val Tyr Thr Pro Gly Ala Ser Arg Pro Thr
        195                 200                 205

Pro Pro Arg Thr Thr Thr Ser Pro Ser Ser Pro Arg Asp Pro Thr Pro
    210                 215                 220

Ala Pro Gly Asp Thr Gly Thr Pro Ala Pro Ala Ser Gly Glu Arg Ala
225                 230                 235                 240

Pro Pro Asn Ser Thr Arg Ser Ala Ser Glu Ser Arg His Arg Leu Thr
                245                 250                 255

Val Ala Gln Val Ile Gln Ile Ala Ile Pro Ala Ser Ile Ile Ala Phe
            260                 265                 270

Val Phe Leu Gly Ser Cys Ile Cys Phe Ile His Arg Cys Gln Arg Arg
        275                 280                 285

Tyr Arg Arg Pro Arg Gly Gln Ile Tyr Asn Pro Gly Gly Val Ser Cys
    290                 295                 300

Ala Val Asn Glu Ala Ala Met Ala Arg Leu Gly Ala Glu Leu Arg Ser
305                 310                 315                 320

His Pro Asn Thr Pro Pro Lys Pro Arg Arg Arg Ser Ser Ser Ser Thr
                325                 330                 335

Thr Met Pro Ser Leu Thr Ser Ile Ala Glu Glu Ser Glu Pro Gly Pro
            340                 345                 350
```

```
Val Val Leu Leu Ser Val Ser Pro Arg Pro Arg Ser Gly Pro Thr Ala
        355                 360                 365

Pro Gln Glu Val
    370

<210>  3
<211>  550
<212>  PRT
<213>  herpes simplex virus 1

<400>  3

Met Asp Arg Gly Ala Val Val Gly Phe Leu Leu Gly Val Cys Val Val
1               5                   10                  15

Ser Cys Leu Ala Gly Thr Pro Lys Thr Ser Trp Arg Arg Val Ser Val
            20                  25                  30

Gly Glu Asp Val Ser Leu Leu Pro Ala Pro Gly Pro Thr Gly Arg Gly
        35                  40                  45

Pro Thr Gln Lys Leu Leu Trp Ala Val Glu Pro Leu Asp Gly Cys Gly
    50                  55                  60

Pro Leu His Pro Ser Trp Val Ser Leu Met Pro Pro Lys Gln Val Pro
65                  70                  75                  80

Glu Thr Val Val Asp Ala Ala Cys Met Arg Ala Pro Val Pro Leu Ala
                85                  90                  95

Met Ala Tyr Ala Pro Pro Ala Pro Ser Ala Thr Gly Gly Leu Arg Thr
            100                 105                 110

Asp Phe Val Trp Gln Glu Arg Ala Ala Val Val Asn Arg Ser Leu Val
        115                 120                 125

Ile His Gly Val Arg Glu Thr Asp Ser Gly Leu Tyr Thr Leu Ser Val
    130                 135                 140

Gly Asp Ile Lys Asp Pro Ala Arg Gln Val Ala Ser Val Val Leu Val
145                 150                 155                 160

Val Gln Pro Ala Pro Val Pro Thr Pro Pro Pro Thr Pro Ala Asp Tyr
                165                 170                 175

Asp Glu Asp Asp Asn Asp Glu Gly Glu Asp Glu Ser Leu Ala Gly Thr
            180                 185                 190
```

Pro Ala Ser Gly Thr Pro Arg Leu Pro Pro Pro Pro Ala Pro Pro Arg
195 200 205

Ser Trp Pro Ser Ala Pro Glu Val Ser His Val Arg Gly Val Thr Val
210 215 220

Arg Met Glu Thr Pro Glu Ala Ile Leu Phe Ser Pro Gly Glu Thr Phe
225 230 235 240

Ser Thr Asn Val Ser Ile His Ala Ile Ala His Asp Asp Gln Thr Tyr
245 250 255

Ser Met Asp Val Val Trp Leu Arg Phe Asp Val Pro Thr Ser Cys Ala
260 265 270

Glu Met Arg Ile Tyr Glu Ser Cys Leu Tyr His Pro Gln Leu Pro Glu
275 280 285

Cys Leu Ser Pro Ala Asp Ala Pro Cys Ala Ala Ser Thr Trp Thr Ser
290 295 300

Arg Leu Ala Val Arg Ser Tyr Ala Gly Cys Ser Arg Thr Asn Pro Pro
305 310 315 320

Pro Arg Cys Ser Ala Glu Ala His Met Glu Pro Val Pro Gly Leu Ala
325 330 335

Trp Gln Ala Ala Ser Val Asn Leu Glu Phe Arg Asp Ala Ser Pro Gln
340 345 350

His Ser Gly Leu Tyr Leu Cys Val Val Tyr Val Asn Asp His Ile His
355 360 365

Ala Trp Gly His Ile Thr Ile Ser Thr Ala Ala Gln Tyr Arg Asn Ala
370 375 380

Val Val Glu Gln Pro Leu Pro Gln Arg Gly Ala Asp Leu Ala Glu Pro
385 390 395 400

Thr His Pro His Val Gly Ala Pro Pro His Ala Pro Pro Thr His Gly
405 410 415

Ala Leu Arg Leu Gly Ala Val Met Gly Ala Ala Leu Leu Leu Ser Ala
420 425 430

Leu Gly Leu Ser Val Trp Ala Cys Met Thr Cys Trp Arg Arg Arg Ala

```
        435                 440                 445

Trp Arg Ala Val Lys Ser Arg Ala Ser Gly Lys Gly Pro Thr Tyr Ile
    450                 455                 460

Arg Val Ala Asp Ser Glu Leu Tyr Ala Asp Trp Ser Ser Asp Ser Glu
465                 470                 475                 480

Gly Glu Arg Asp Gln Val Pro Trp Leu Ala Pro Pro Glu Arg Pro Asp
                485                 490                 495

Ser Pro Ser Thr Asn Gly Ser Gly Phe Glu Ile Leu Ser Pro Thr Ala
            500                 505                 510

Pro Ser Val Tyr Pro Arg Ser Asp Gly His Gln Ser Arg Arg Gln Leu
        515                 520                 525

Thr Thr Phe Gly Ser Gly Arg Pro Asp Arg Arg Tyr Ser Gln Ala Ser
    530                 535                 540

Asp Ser Ser Val Phe Trp
545                 550


<210>  4
<211>  390
<212>  PRT
<213>  herpes simplex virus 1

<400>  4

Met Pro Cys Arg Pro Leu Gln Gly Leu Val Leu Val Gly Leu Trp Val
1               5                   10                  15

Cys Ala Thr Ser Leu Val Val Arg Gly Pro Thr Val Ser Leu Val Ser
            20                  25                  30

Asn Ser Phe Val Asp Ala Gly Ala Leu Gly Pro Asp Gly Val Val Glu
        35                  40                  45

Glu Asp Leu Leu Ile Leu Gly Glu Leu Arg Phe Val Gly Asp Gln Val
    50                  55                  60

Pro His Thr Thr Tyr Tyr Asp Gly Gly Val Glu Leu Trp His Tyr Pro
65                  70                  75                  80

Met Gly His Lys Cys Pro Arg Val Val His Val Val Thr Val Thr Ala
                85                  90                  95

Cys Pro Arg Arg Pro Ala Val Ala Phe Ala Leu Cys Arg Ala Thr Asp
```

100 105 110

Ser Thr His Ser Pro Ala Tyr Pro Thr Leu Glu Leu Asn Leu Ala Gln
115 120 125

Gln Pro Leu Leu Arg Val Gln Arg Ala Thr Arg Asp Tyr Ala Gly Val
130 135 140

Tyr Val Leu Arg Val Trp Val Gly Asp Ala Pro Asn Ala Ser Leu Phe
145 150 155 160

Val Leu Gly Met Ala Ile Ala Ala Glu Gly Thr Leu Ala Tyr Asn Gly
165 170 175

Ser Ala Tyr Gly Ser Cys Asp Pro Lys Leu Leu Pro Ser Ser Ala Pro
180 185 190

Arg Leu Ala Pro Ala Ser Val Tyr Gln Pro Ala Pro Asn Gln Ala Ser
195 200 205

Thr Pro Ser Thr Thr Thr Ser Thr Pro Ser Thr Thr Ile Pro Ala Pro
210 215 220

Ser Thr Thr Ile Pro Ala Pro Gln Ala Ser Thr Thr Pro Phe Pro Thr
225 230 235 240

Gly Asp Pro Lys Pro Gln Pro Pro Gly Val Asn His Glu Pro Pro Ser
245 250 255

Asn Ala Thr Arg Ala Thr Arg Asp Ser Arg Tyr Ala Leu Thr Val Thr
260 265 270

Gln Ile Ile Gln Ile Ala Ile Pro Ala Ser Ile Ile Ala Leu Val Phe
275 280 285

Leu Gly Ser Cys Ile Cys Phe Ile His Arg Cys Gln Arg Arg Tyr Arg
290 295 300

Arg Ser Arg Arg Pro Ile Tyr Ser Pro Gln Met Pro Thr Gly Ile Ser
305 310 315 320

Cys Ala Val Asn Glu Ala Ala Met Ala Arg Leu Gly Ala Glu Leu Lys
325 330 335

Ser His Pro Ser Thr Pro Pro Lys Ser Arg Arg Arg Ser Ser Arg Thr
340 345 350

Pro Met Pro Ser Leu Thr Ala Ile Ala Glu Glu Ser Glu Pro Ala Gly
355 360 365

Ala Ala Gly Leu Pro Thr Pro Pro Val Asp Pro Thr Thr Pro Thr Pro
370 375 380

Thr Pro Pro Leu Leu Val
385 390

<210> 5
<211> 234
<212> PRT
<213> Human cytomegalovirus

<400> 5

Met Gln Thr Tyr Ser Thr Pro Leu Thr Leu Val Ile Val Thr Ser Leu
1 5 10 15

Phe Leu Phe Thr Thr Gln Gly Ser Ser Ser Asn Ala Val Glu Pro Thr
20 25 30

Lys Lys Pro Leu Lys Leu Ala Asn Tyr Arg Ala Thr Cys Glu Asp Arg
35 40 45

Thr Arg Thr Leu Val Thr Arg Leu Asn Thr Ser His His Ser Val Val
50 55 60

Trp Gln Arg Tyr Asp Ile Tyr Ser Arg Tyr Met Arg Arg Met Pro Pro
65 70 75 80

Leu Cys Ile Ile Thr Asp Ala Tyr Lys Glu Thr Thr Arg Gln Gly Gly
85 90 95

Ala Ala Phe Ala Cys Thr Arg Gln Asn Leu Thr Leu Tyr Asn Leu Thr
100 105 110

Val Lys Asp Thr Gly Val Tyr Leu Leu Gln Asp Gln Tyr Thr Gly Asp
115 120 125

Val Glu Ala Phe Tyr Leu Ile Ile His Pro Arg Ser Phe Cys Arg Ala
130 135 140

Leu Glu Thr Arg Arg Cys Phe Tyr Pro Gly Pro Gly Arg Val Val Val
145 150 155 160

Thr Asp Ser Gln Glu Ala Asp Arg Ala Ile Ile Ser Asp Leu Lys Arg
165 170 175

Gln Trp Ser Gly Leu Ser Leu His Cys Ala Trp Val Ser Gly Met Met
180 185 190

Ile Phe Val Gly Ala Leu Val Ile Cys Phe Leu Arg Ser Gln Arg Ile
195 200 205

Gly Glu Gln Asp Ala Glu His Leu Arg Thr Asp Leu Asp Thr Glu Pro
210 215 220

Leu Leu Leu Thr Val Asp Gly Asp Leu Gln
225 230

<210> 6
<211> 345
<212> PRT
<213> Human cytomegalovirus

<400> 6

Met Cys Ser Val Leu Ala Ile Ala Leu Val Val Ala Leu Leu Gly Asp
1 5 10 15

Met His Pro Gly Val Lys Ser Ser Thr Thr Ser Ala Val Thr Ser Pro
20 25 30

Ser Asn Thr Thr Val Thr Ser Thr Thr Ser Ile Ser Thr Ser Asn Asn
35 40 45

Val Thr Ser Ala Val Thr Thr Thr Val Gln Thr Ser Thr Ser Ser Ala
50 55 60

Ser Thr Ser Val Ile Ala Thr Thr Gln Lys Glu Gly His Leu Tyr Thr
65 70 75 80

Val Asn Cys Glu Ala Ser Tyr Ser His Asp Gln Val Ser Leu Asn Ala
85 90 95

Thr Cys Lys Val Ile Leu Leu Asn Asn Thr Lys Asn Pro Asp Ile Leu
100 105 110

Ser Val Thr Cys Tyr Ala Arg Thr Asp Cys Lys Gly Pro Phe Thr Gln
115 120 125

Val Gly Tyr Leu Ser Ala Phe Pro Pro Asp Asn Glu Gly Lys Leu His
130 135 140

Leu Ser Tyr Asn Ala Thr Ala Gln Glu Leu Leu Ile Ser Gly Leu Arg
145 150 155 160

Pro Gln Glu Thr Thr Glu Tyr Thr Cys Ser Phe Phe Ser Trp Gly Arg
165 170 175

His His Asn Ala Thr Trp Asp Leu Phe Thr Tyr Pro Ile Tyr Ala Val
180 185 190

Tyr Gly Thr Arg Leu Asn Ala Thr Thr Met Arg Val Arg Val Leu Leu
195 200 205

Gln Glu His Glu His Cys Leu Leu Asn Gly Ser Ser Leu Tyr His Pro
210 215 220

Asn Ser Thr Val His Leu His Gln Gly Asn Gln Leu Ile Pro Pro Trp
225 230 235 240

Asn Ile Ser Asn Val Thr Tyr Asn Gly Gln Arg Leu Arg Glu Phe Val
245 250 255

Phe Tyr Leu Asn Gly Thr Tyr Thr Val Val Arg Leu His Val Gln Ile
260 265 270

Ala Gly Arg Ser Phe Thr Thr Thr Tyr Val Phe Ile Lys Ser Asp Pro
275 280 285

Leu Phe Glu Asp Arg Leu Leu Ala Tyr Gly Val Leu Ala Phe Leu Val
290 295 300

Phe Met Val Ile Ile Leu Leu Tyr Val Thr Tyr Met Leu Ala Arg Arg
305 310 315 320

Arg Asp Trp Ser Tyr Lys Arg Leu Glu Glu Pro Val Glu Glu Lys Lys
325 330 335

His Pro Val Pro Tyr Phe Lys Gln Trp
340 345

<210> 7
<211> 419
<212> PRT
<213> Artificial Sequence

<220>
<223> HSV2 gE ectodomain

<400> 7

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser

20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

Val Ile Tyr Gly Ala Leu Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Pro Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro Pro Pro Ala Pro Ser Ala Arg Gly Pro Leu
405 410 415

Arg Leu Gly

<210> 8
<211> 256
<212> PRT
<213> Artificial Sequence

<220>
<223> HSV2 gI ectodomain

<400> 8

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val

50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Leu Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Ser Pro Ser Ser Pro Arg Asp Pro Thr Pro
210 215 220

Ala Pro Gly Asp Thr Gly Thr Pro Ala Pro Ala Ser Gly Glu Arg Ala
225 230 235 240

Pro Pro Asn Ser Thr Arg Ser Ala Ser Glu Ser Arg His Arg Leu Thr
245 250 255

<210> 9
<211> 256
<212> PRT
<213> Artificial Sequence

<220>
<223> HSV2 gI ectodomain

<400> 9

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Leu Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Ser Pro Ser Ser Pro Arg Asp Pro Thr Pro
210 215 220

Ala Pro Gly Asp Thr Gly Thr Pro Ala Pro Ala Ser Gly Glu Arg Ala
225 230 235 240

Pro Pro Asn Ser Thr Arg Ser Ala Ser Glu Ser Arg His Arg Leu Thr
245 250 255

<210> 10

<211> 270
<212> PRT
<213> Artificial Sequence

<220>
<223> HSV1 gI ectodomain

<400> 10

Met Pro Cys Arg Pro Leu Gln Gly Leu Val Leu Val Gly Leu Trp Val
1               5                   10                  15

Cys Ala Thr Ser Leu Val Val Arg Gly Pro Thr Val Ser Leu Val Ser
            20                  25                  30

Asn Ser Phe Val Asp Ala Gly Ala Leu Gly Pro Asp Gly Val Val Glu
        35                  40                  45

Glu Asp Leu Leu Ile Leu Gly Glu Leu Arg Phe Val Gly Asp Gln Val
    50                  55                  60

Pro His Thr Thr Tyr Tyr Asp Gly Gly Val Glu Leu Trp His Tyr Pro
65                  70                  75                  80

Met Gly His Lys Cys Pro Arg Val Val His Val Val Thr Val Thr Ala
                85                  90                  95

Cys Pro Arg Arg Pro Ala Val Ala Phe Ala Leu Cys Arg Ala Thr Asp
            100                 105                 110

Ser Thr His Ser Pro Ala Tyr Pro Thr Leu Glu Leu Asn Leu Ala Gln
        115                 120                 125

Gln Pro Leu Leu Arg Val Gln Arg Ala Thr Arg Asp Tyr Ala Gly Val
    130                 135                 140

Tyr Val Leu Arg Val Trp Val Gly Asp Ala Pro Asn Ala Ser Leu Phe
145                 150                 155                 160

Val Leu Gly Met Ala Ile Ala Ala Glu Gly Thr Leu Ala Tyr Asn Gly
                165                 170                 175

Ser Ala Tyr Gly Ser Cys Asp Pro Lys Leu Leu Pro Ser Ser Ala Pro
            180                 185                 190

Arg Leu Ala Pro Ala Ser Val Tyr Gln Pro Ala Pro Asn Gln Ala Ser
        195                 200                 205

Thr Pro Ser Thr Thr Thr Ser Thr Pro Ser Thr Thr Ile Pro Ala Pro
    210                 215                 220

Ser Thr Thr Ile Pro Ala Pro Gln Ala Ser Thr Thr Pro Phe Pro Thr
225 230 235 240

Gly Asp Pro Lys Pro Gln Pro Pro Gly Val Asn His Glu Pro Pro Ser
245 250 255

Asn Ala Thr Arg Ala Thr Arg Asp Ser Arg Tyr Ala Leu Thr
260 265 270

<210> 11
<211> 180
<212> PRT
<213> Artificial Sequence

<220>
<223> HCMV gp34 ectodomain

<400> 11

Met Gln Thr Tyr Ser Thr Pro Leu Thr Leu Val Ile Val Thr Ser Leu
1 5 10 15

Phe Leu Phe Thr Thr Gln Gly Ser Ser Ser Asn Ala Val Glu Pro Thr
20 25 30

Lys Lys Pro Leu Lys Leu Ala Asn Tyr Arg Ala Thr Cys Glu Asp Arg
35 40 45

Thr Arg Thr Leu Val Thr Arg Leu Asn Thr Ser His His Ser Val Val
50 55 60

Trp Gln Arg Tyr Asp Ile Tyr Ser Arg Tyr Met Arg Arg Met Pro Pro
65 70 75 80

Leu Cys Ile Ile Thr Asp Ala Tyr Lys Glu Thr Thr Arg Gln Gly Gly
85 90 95

Ala Ala Phe Ala Cys Thr Arg Gln Asn Leu Thr Leu Tyr Asn Leu Thr
100 105 110

Val Lys Asp Thr Gly Val Tyr Leu Leu Gln Asp Gln Tyr Thr Gly Asp
115 120 125

Val Glu Ala Phe Tyr Leu Ile Ile His Pro Arg Ser Phe Cys Arg Ala
130 135 140

Leu Glu Thr Arg Arg Cys Phe Tyr Pro Gly Pro Gly Arg Val Val Val
145 150 155 160

Thr Asp Ser Gln Glu Ala Asp Arg Ala Ile Ile Ser Asp Leu Lys Arg
165 170 175

Gln Trp Ser Gly
180

<210> 12
<211> 291
<212> PRT
<213> Artificial Sequence

<220>
<223> HCMV gp68 ectodomain

<400> 12

Met Cys Ser Val Leu Ala Ile Ala Leu Val Val Ala Leu Leu Gly Asp
1 5 10 15

Met His Pro Gly Val Lys Ser Ser Thr Thr Ser Ala Val Thr Ser Pro
20 25 30

Ser Asn Thr Thr Val Thr Ser Thr Thr Ser Ile Ser Thr Ser Asn Asn
35 40 45

Val Thr Ser Ala Val Thr Thr Thr Val Gln Thr Ser Thr Ser Ser Ala
50 55 60

Ser Thr Ser Val Ile Ala Thr Thr Gln Lys Glu Gly His Leu Tyr Thr
65 70 75 80

Val Asn Cys Glu Ala Ser Tyr Ser His Asp Gln Val Ser Leu Asn Ala
85 90 95

Thr Cys Lys Val Ile Leu Leu Asn Asn Thr Lys Asn Pro Asp Ile Leu
100 105 110

Ser Val Thr Cys Tyr Ala Arg Thr Asp Cys Lys Gly Pro Phe Thr Gln
115 120 125

Val Gly Tyr Leu Ser Ala Phe Pro Pro Asp Asn Glu Gly Lys Leu His
130 135 140

Leu Ser Tyr Asn Ala Thr Ala Gln Glu Leu Leu Ile Ser Gly Leu Arg
145 150 155 160

Pro Gln Glu Thr Thr Glu Tyr Thr Cys Ser Phe Phe Ser Trp Gly Arg
165 170 175

His His Asn Ala Thr Trp Asp Leu Phe Thr Tyr Pro Ile Tyr Ala Val
180 185 190

Tyr Gly Thr Arg Leu Asn Ala Thr Thr Met Arg Val Arg Val Leu Leu
195 200 205

Gln Glu His Glu His Cys Leu Leu Asn Gly Ser Ser Leu Tyr His Pro
210 215 220

Asn Ser Thr Val His Leu His Gln Gly Asn Gln Leu Ile Pro Pro Trp
225 230 235 240

Asn Ile Ser Asn Val Thr Tyr Asn Gly Gln Arg Leu Arg Glu Phe Val
245 250 255

Phe Tyr Leu Asn Gly Thr Tyr Thr Val Val Arg Leu His Val Gln Ile
260 265 270

Ala Gly Arg Ser Phe Thr Thr Thr Tyr Val Phe Ile Lys Ser Asp Pro
275 280 285

Leu Phe Glu
290

<210> 13
<211> 548
<212> PRT
<213> herpes simplex virus 2

<400> 13

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

Val Ile Tyr Gly Ala Leu Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Ala Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro His Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr Ser
530 535 540

Ser Val Leu Trp
545

<210> 14
<211> 548
<212> PRT
<213> herpes simplex virus 2

<400> 14

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

Val Ile Tyr Gly Ala Leu Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Pro Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro Pro Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr

515 520 525

Phe Gly Ser Gly Ser Pro Gly Arg Arg His Ser Gln Ala Ser Tyr Ser
530 535 540

Ser Val Leu Trp
545

<210> 15
<211> 548
<212> PRT
<213> herpes simplex virus 2

<400> 15

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

Val Ile Tyr Gly Ala Leu Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro

180 185 190

Pro Pro Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro Pro Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr Ser
530 535 540

Ser Val Leu Trp
545

<210> 16
<211> 548
<212> PRT
<213> herpes simplex virus 2

<400> 16

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

Val Ile Tyr Gly Ala Leu Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Pro Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro Pro Pro Ala Pro Ser Ala Arg Gly Pro Leu
405 410 415

Arg Leu Arg Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr Ser
530 535 540

Ser Val Leu Trp
545

<210> 17
<211> 548
<212> PRT
<213> herpes simplex virus 2

<400> 17

Met Ala Arg Gly Ala Gly Leu Val Phe Phe Val Gly Val Trp Val Val
1 5 10 15

```
Ser Cys Leu Ala Ala Ala Pro Arg Thr Ser Trp Lys Arg Val Thr Ser
            20                  25                  30

Gly Glu Asp Val Val Leu Leu Pro Ala Pro Ala Gly Pro Glu Glu Arg
        35                  40                  45

Thr Arg Ala His Lys Leu Leu Trp Ala Ala Glu Pro Leu Asp Ala Cys
    50                  55                  60

Gly Pro Leu Arg Pro Ser Trp Val Ala Leu Trp Pro Pro Arg Arg Val
65                  70                  75                  80

Leu Glu Thr Val Val Asp Ala Ala Cys Met Arg Ala Pro Glu Pro Leu
                85                  90                  95

Ala Ile Ala Tyr Ser Pro Pro Phe Pro Ala Gly Asp Glu Gly Leu Tyr
            100                 105                 110

Ser Glu Leu Ala Trp Arg Asp Arg Val Ala Val Val Asn Glu Ser Leu
        115                 120                 125

Val Ile Tyr Gly Ala Leu Glu Thr Asp Ser Gly Leu Tyr Thr Leu Ser
    130                 135                 140

Val Val Gly Leu Ser Asp Glu Ala Arg Gln Val Ala Ser Val Val Leu
145                 150                 155                 160

Val Val Glu Pro Ala Pro Val Pro Thr Pro Thr Pro Asp Asp Tyr Asp
                165                 170                 175

Glu Glu Asp Asp Ala Gly Val Ser Glu Arg Thr Pro Val Ser Val Pro
            180                 185                 190

Pro Pro Thr Pro Pro Arg Arg Pro Pro Val Ala Pro Pro Thr His Pro
        195                 200                 205

Arg Val Ile Pro Glu Val Ser His Val Arg Gly Val Thr Val His Met
    210                 215                 220

Glu Thr Pro Glu Ala Ile Leu Phe Ala Pro Gly Glu Thr Phe Gly Thr
225                 230                 235                 240

Asn Val Ser Ile His Ala Ile Ala His Asp Asp Gly Pro Tyr Ala Met
                245                 250                 255

Asp Val Val Trp Met Arg Phe Asp Val Pro Ser Ser Cys Ala Glu Met
            260                 265                 270
```

```
Arg Ile Tyr Glu Ala Cys Leu Tyr His Pro Gln Leu Pro Glu Cys Leu
        275                 280                 285

Ser Pro Ala Asp Ala Pro Cys Ala Val Ser Ser Trp Ala Tyr Arg Leu
    290                 295                 300

Ala Val Arg Ser Tyr Ala Gly Cys Ser Arg Thr Thr Pro Pro Pro Arg
305                 310                 315                 320

Cys Phe Ala Glu Ala Arg Met Glu Pro Val Pro Gly Leu Ala Trp Leu
                325                 330                 335

Ala Ser Thr Val Asn Leu Glu Phe Gln His Ala Ser Pro Gln His Ala
            340                 345                 350

Gly Leu Tyr Leu Cys Val Val Tyr Val Asp Asp His Ile His Ala Trp
        355                 360                 365

Gly His Met Thr Ile Ser Thr Ala Ala Gln Tyr Arg Asn Ala Val Val
    370                 375                 380

Glu Gln His Leu Pro Gln Arg Gln Pro Glu Pro Val Glu Pro Thr Arg
385                 390                 395                 400

Pro His Val Arg Ala Pro Pro Pro Ala Pro Ser Ala Arg Gly Pro Leu
                405                 410                 415

Arg Leu Gly Ala Val Leu Gly Ala Ala Leu Leu Leu Ala Ala Leu Gly
            420                 425                 430

Leu Ser Ala Trp Ala Cys Met Thr Cys Trp Arg Arg Arg Ser Trp Arg
        435                 440                 445

Ala Val Lys Ser Arg Ala Ser Ala Thr Gly Pro Thr Tyr Ile Arg Val
    450                 455                 460

Ala Asp Ser Glu Leu Tyr Ala Asp Trp Ser Ser Asp Ser Glu Gly Glu
465                 470                 475                 480

Arg Asp Gly Ser Leu Trp Gln Asp Pro Pro Glu Arg Pro Asp Ser Pro
                485                 490                 495

Ser Thr Asn Gly Ser Gly Phe Glu Ile Leu Ser Pro Thr Ala Pro Ser
            500                 505                 510

Val Tyr Pro His Ser Glu Gly Arg Lys Ser Arg Arg Pro Leu Thr Thr
```

515 520 525

Phe Gly Ser Gly Ser Pro Gly Arg Arg His Ser Gln Ala Ser Tyr Ser
530 535 540

Ser Val Leu Trp
545

<210> 18
<211> 548
<212> PRT
<213> herpes simplex virus 2

<400> 18

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

Val Ile Tyr Gly Ala Leu Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro

180 185 190

Pro Pro Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro Pro Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr Ser
530 535 540

Ser Val Leu Trp
545

<210> 19
<211> 545
<212> PRT
<213> herpes simplex virus 2

<220>
<221> misc_feature
<222> (109)..(109)
<223> Xaa can be any naturally occurring amino acid

<220>
<221> misc_feature
<222> (146)..(195)
<223> Xaa can be any naturally occurring amino acid

<220>
<221> misc_feature
<222> (340)..(340)
<223> Xaa can be any naturally occurring amino acid

<220>
<221> misc_feature
<222> (475)..(475)
<223> Xaa can be any naturally occurring amino acid

<400> 19

Met Ala Arg Gly Ala Gly Leu Val Phe Phe Val Gly Val Trp Val Val
1 5 10 15

```
Ser Cys Leu Ala Ala Ala Pro Arg Thr Ser Trp Lys Arg Val Thr Ser
            20                  25                  30

Gly Glu Asp Val Val Leu Leu Pro Ala Pro Ala Glu Arg Thr Arg Ala
        35                  40                  45

His Lys Leu Leu Trp Ala Ala Glu Pro Leu Asp Ala Cys Gly Pro Leu
    50                  55                  60

Arg Pro Ser Trp Val Ala Leu Trp Pro Pro Arg Arg Val Leu Glu Thr
65                  70                  75                  80

Val Val Asp Ala Ala Cys Met Arg Ala Pro Glu Pro Leu Ala Ile Ala
                85                  90                  95

Tyr Ser Pro Pro Phe Pro Ala Gly Asp Glu Gly Leu Xaa Ser Glu Leu
            100                 105                 110

Ala Trp Arg Asp Arg Val Ala Val Val Asn Glu Ser Leu Val Ile Tyr
        115                 120                 125

Gly Ala Leu Glu Thr Asp Ser Gly Leu Tyr Thr Leu Ser Val Val Gly
    130                 135                 140

Leu Xaa Xaa Xaa Xaa Xaa Xaa Xaa Xaa Xaa Xaa Xaa Xaa Xaa Xaa Xaa
145                 150                 155                 160

Xaa Xaa Xaa Xaa Xaa Xaa Xaa Xaa Xaa Xaa Xaa Xaa Xaa Xaa Xaa Xaa
                165                 170                 175

Xaa Xaa Xaa Xaa Xaa Xaa Xaa Xaa Xaa Xaa Xaa Xaa Xaa Xaa Xaa Xaa
            180                 185                 190

Xaa Xaa Xaa Arg Pro Pro Val Ala Pro Pro Thr His Pro Arg Val Ile
        195                 200                 205

Pro Glu Val Ser His Val Arg Gly Val Thr Val His Met Glu Thr Pro
    210                 215                 220

Glu Ala Ile Leu Phe Ala Pro Gly Glu Thr Phe Gly Thr Asn Val Ser
225                 230                 235                 240

Ile His Ala Ile Ala His Asp Asp Gly Pro Tyr Ala Met Asp Val Val
                245                 250                 255

Trp Met Arg Phe Asp Val Pro Ser Ser Cys Ala Glu Met Arg Ile Tyr
            260                 265                 270
```

Glu Ala Cys Leu Tyr His Pro Gln Leu Pro Glu Cys Leu Ser Pro Ala
275 280 285

Asp Ala Pro Cys Ala Val Ser Ser Trp Ala Tyr Arg Leu Ala Val Arg
290 295 300

Ser Tyr Ala Gly Cys Ser Arg Thr Thr Pro Pro Pro Arg Cys Phe Ala
305 310 315 320

Glu Ala Arg Met Glu Pro Val Pro Gly Leu Ala Trp Leu Ala Ser Thr
325 330 335

Val Asn Leu Xaa Phe Gln His Ala Ser Pro Gln His Ala Gly Leu Tyr
340 345 350

Leu Cys Val Val Tyr Val Asp Asp His Ile His Ala Trp Gly His Met
355 360 365

Thr Ile Ser Thr Ala Ala Gln Tyr Arg Asn Ala Val Val Glu Gln His
370 375 380

Leu Pro Gln Arg Gln Pro Glu Pro Val Glu Pro Thr Arg Pro His Val
385 390 395 400

Arg Ala Pro His Pro Ala Pro Ser Ala Arg Gly Pro Leu Arg Leu Gly
405 410 415

Ala Val Leu Gly Ala Ala Leu Leu Leu Ala Ala Leu Gly Leu Ser Ala
420 425 430

Trp Ala Cys Met Thr Cys Trp Arg Arg Arg Ser Trp Arg Ala Val Lys
435 440 445

Ser Arg Ala Ser Ala Thr Gly Pro Thr Tyr Ile Arg Val Ala Asp Ser
450 455 460

Glu Leu Tyr Ala Asp Trp Ser Ser Asp Ser Xaa Gly Glu Arg Asp Gly
465 470 475 480

Ser Leu Trp Gln Asp Pro Pro Glu Arg Pro Asp Ser Pro Ser Thr Asn
485 490 495

Gly Ser Gly Phe Glu Ile Leu Ser Pro Thr Ala Pro Ser Val Tyr Pro
500 505 510

His Ser Glu Gly Arg Lys Ser Arg Arg Pro Leu Thr Thr Phe Gly Ser

515 520 525

Gly Ser Pro Gly Arg Arg His Ser Gln Ala Ser Tyr Pro Ser Val Leu
530 535 540

Trp
545

<210> 20
<211> 545
<212> PRT
<213> herpes simplex virus 2

<220>
<221> misc_feature
<222> (44)..(46)
<223> Xaa can be any naturally occurring amino acid

<220>
<221> misc_feature
<222> (58)..(58)
<223> Xaa can be any naturally occurring amino acid

<220>
<221> misc_feature
<222> (69)..(69)
<223> Xaa can be any naturally occurring amino acid

<220>
<221> misc_feature
<222> (81)..(206)
<223> Xaa can be any naturally occurring amino acid

<220>
<221> misc_feature
<222> (387)..(397)
<223> Xaa can be any naturally occurring amino acid

<400> 20

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Val Leu Pro Ala Pro Ala Xaa Xaa Xaa Arg Ala
35 40 45

His Lys Leu Leu Trp Ala Ala Glu Pro Xaa Asp Ala Cys Gly Pro Leu
50 55 60

Arg Pro Ser Trp Xaa Ala Leu Trp Pro Pro Arg Arg Val Leu Glu Thr
65 70 75 80

```
Xaa Xaa Xaa Xaa Xaa Xaa Xaa Xaa Xaa Xaa Xaa Xaa Xaa Xaa Xaa Xaa
                85                  90                  95

Xaa Xaa Xaa Xaa Xaa Xaa Xaa Xaa Xaa Xaa Xaa Xaa Xaa Xaa Xaa Xaa
            100                 105                 110

Xaa Xaa Xaa Xaa Xaa Xaa Xaa Xaa Xaa Xaa Xaa Xaa Xaa Xaa Xaa Xaa
        115                 120                 125

Xaa Xaa Xaa Xaa Xaa Xaa Xaa Xaa Xaa Xaa Xaa Xaa Xaa Xaa Xaa Xaa
    130                 135                 140

Xaa Xaa Xaa Xaa Xaa Xaa Xaa Xaa Xaa Xaa Xaa Xaa Xaa Xaa Xaa Xaa
145                 150                 155                 160

Xaa Xaa Xaa Xaa Xaa Xaa Xaa Xaa Xaa Xaa Xaa Xaa Xaa Xaa Xaa Xaa
                165                 170                 175

Xaa Xaa Xaa Xaa Xaa Xaa Xaa Xaa Xaa Xaa Xaa Xaa Xaa Xaa Xaa Xaa
            180                 185                 190

Xaa Xaa Xaa Xaa Xaa Xaa Xaa Xaa Xaa Xaa Xaa Xaa Xaa Xaa Val Ile
        195                 200                 205

Pro Glu Val Ser His Val Arg Gly Val Thr Val His Met Glu Thr Pro
    210                 215                 220

Glu Ala Ile Leu Phe Ala Pro Gly Glu Thr Phe Gly Thr Asn Val Ser
225                 230                 235                 240

Ile His Ala Ile Ala His Asp Asp Gly Pro Tyr Ala Met Asp Val Val
                245                 250                 255

Trp Met Arg Phe Asp Val Pro Ser Ser Cys Ala Glu Met Arg Ile Tyr
            260                 265                 270

Glu Ala Cys Leu Tyr His Pro Gln Leu Pro Glu Cys Leu Ser Pro Ala
        275                 280                 285

Asp Ala Pro Cys Ala Val Ser Ser Trp Ala Tyr Arg Leu Ala Val Arg
    290                 295                 300

Ser Tyr Ala Gly Cys Ser Arg Thr Thr Pro Pro Pro Arg Cys Phe Ala
305                 310                 315                 320

Glu Ala Arg Met Glu Pro Val Pro Gly Leu Ala Trp Leu Ala Ser Thr
                325                 330                 335
```

Val Asn Leu Glu Phe Gln His Ala Ser Pro Gln His Ala Gly Leu Tyr
340 345 350

Leu Cys Val Val Tyr Val Asp Asp His Ile His Ala Trp Gly His Met
355 360 365

Thr Ile Ser Thr Ala Ala Gln Tyr Arg Asn Ala Val Val Glu Gln His
370 375 380

Leu Pro Xaa Xaa Xaa Xaa Xaa Xaa Xaa Xaa Xaa Xaa Xaa Pro His Val
385 390 395 400

Arg Ala Pro His Pro Ala Pro Ser Ala Arg Gly Pro Leu Arg Leu Gly
405 410 415

Ala Val Leu Gly Ala Ala Leu Leu Leu Ala Ala Leu Gly Leu Ser Ala
420 425 430

Trp Ala Cys Met Thr Cys Trp Arg Arg Arg Ser Trp Arg Ala Val Lys
435 440 445

Ser Arg Ala Ser Ala Thr Gly Pro Thr Tyr Ile Arg Val Ala Asp Ser
450 455 460

Glu Leu Tyr Ala Asp Trp Ser Ser Asp Ser Glu Gly Glu Arg Asp Gly
465 470 475 480

Ser Leu Trp Gln Asp Pro Pro Glu Arg Pro Asp Ser Pro Ser Thr Asn
485 490 495

Gly Ser Gly Phe Glu Ile Leu Ser Pro Thr Ala Pro Ser Val Tyr Pro
500 505 510

His Ser Glu Gly Arg Lys Ser Arg Arg Pro Leu Thr Thr Phe Gly Ser
515 520 525

Gly Ser Pro Gly Arg Arg His Ser Gln Ala Ser Tyr Ser Ser Val Leu
530 535 540

Trp
545

<210> 21
<211> 548
<212> PRT
<213> herpes simplex virus 2

<220>
<221> misc_feature
<222> (181)..(203)
<223> Xaa can be any naturally occurring amino acid

<220>
<221> misc_feature
<222> (377)..(405)
<223> Xaa can be any naturally occurring amino acid

<400> 21

```
Met Ala Arg Gly Ala Gly Leu Val Phe Phe Val Gly Val Trp Val Val
1               5                   10                  15

Ser Cys Leu Ala Ala Ala Pro Arg Thr Ser Trp Lys Arg Val Thr Ser
            20                  25                  30

Gly Glu Asp Val Val Leu Leu Pro Ala Pro Ala Gly Pro Glu Glu Arg
        35                  40                  45

Thr Arg Ala His Lys Leu Leu Trp Ala Ala Glu Pro Leu Asp Ala Cys
    50                  55                  60

Gly Pro Leu Arg Pro Ser Trp Val Ala Leu Trp Pro Pro Arg Arg Val
65                  70                  75                  80

Leu Glu Thr Val Val Asp Ala Ala Cys Met Arg Ala Pro Glu Pro Leu
                85                  90                  95

Ala Ile Ala Tyr Ser Pro Pro Phe Pro Ala Gly Asp Glu Gly Leu Tyr
            100                 105                 110

Ser Glu Leu Ala Trp Arg Asp Arg Val Ala Val Val Asn Glu Ser Leu
        115                 120                 125

Val Ile Tyr Gly Ala Leu Glu Thr Asp Ser Gly Leu Tyr Thr Leu Ser
    130                 135                 140

Val Val Gly Leu Ser Asp Glu Ala Arg Gln Val Ala Ser Val Val Leu
145                 150                 155                 160

Val Val Glu Pro Ala Pro Val Pro Thr Pro Thr Pro Asp Asp Tyr Asp
                165                 170                 175

Glu Glu Asp Asp Xaa Xaa Xaa Xaa Xaa Xaa Xaa Xaa Xaa Xaa Xaa Xaa
            180                 185                 190

Xaa Xaa Xaa Xaa Xaa Xaa Xaa Xaa Xaa Xaa Xaa Pro Pro Thr His Pro
        195                 200                 205
```

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Xaa Xaa Xaa Xaa Xaa Xaa Xaa Xaa
370 375 380

Xaa Xaa Xaa Xaa Xaa Xaa Xaa Xaa Xaa Xaa Xaa Xaa Xaa Xaa Xaa Xaa
385 390 395 400

Xaa Xaa Xaa Xaa Xaa Pro His Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val

450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr Ser
530 535 540

Ser Val Leu Trp
545

<210> 22
<211> 548
<212> PRT
<213> herpes simplex virus 2

<400> 22

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu

115 120 125

Val Ile Tyr Gly Ala Leu Glu Thr Asp Ser Gly Leu Tyr Thr Leu Ser
130 135 140

Val Val Gly Leu Ser Asp Lys Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Pro Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro Pro Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr Ser
530 535 540

Ser Val Leu Trp
545

<210> 23
<211> 548
<212> PRT
<213> herpes simplex virus 2

<400> 23

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

Val Ile Tyr Gly Ala Leu Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Pro Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
    290                 295                 300

Ala Val Arg Ser Tyr Ala Gly Cys Ser Arg Thr Thr Pro Pro Pro Arg
305                 310                 315                 320

Cys Phe Ala Glu Ala Arg Met Glu Pro Val Pro Gly Leu Ala Trp Leu
                325                 330                 335

Ala Ser Thr Val Asn Leu Glu Phe Gln His Ala Ser Pro Gln His Ala
            340                 345                 350

Gly Leu Tyr Leu Cys Val Val Tyr Val Asp Asp His Ile His Ala Trp
        355                 360                 365

Gly His Met Thr Ile Ser Thr Ala Ala Gln Tyr Arg Asn Ala Val Val
    370                 375                 380

Glu Gln His Leu Pro Gln Arg Gln Pro Glu Pro Val Glu Pro Thr Arg
385                 390                 395                 400

Pro His Val Arg Ala Pro Pro Pro Ala Pro Ser Ala Arg Gly Pro Leu
                405                 410                 415

Arg Leu Gly Ala Val Leu Gly Ala Ala Leu Leu Leu Ala Ala Leu Gly
            420                 425                 430

Leu Ser Ala Trp Ala Cys Met Thr Cys Trp Arg Arg Arg Ser Trp Arg
        435                 440                 445

Ala Val Lys Ser Arg Ala Ser Ala Thr Gly Pro Thr Tyr Ile Arg Val
    450                 455                 460

Ala Asp Ser Glu Leu Tyr Ala Asp Trp Ser Ser Asp Ser Glu Gly Glu
465                 470                 475                 480

Arg Asp Gly Ser Leu Trp Gln Asp Pro Pro Glu Arg Pro Asp Ser Pro
                485                 490                 495

Ser Thr Asn Gly Ser Gly Phe Glu Ile Leu Ser Pro Thr Ala Pro Ser
            500                 505                 510

Val Tyr Pro His Ser Glu Gly Arg Lys Ser Arg Arg Pro Leu Thr Thr
        515                 520                 525

Phe Gly Ser Gly Ser Pro Gly Arg Arg His Ser Gln Ala Ser Tyr Ser
    530                 535                 540

Ser Val Leu Trp
545

<210> 24
<211> 548
<212> PRT
<213> herpes simplex virus 2

<400> 24

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

Val Ile Tyr Gly Ala Leu Glu Thr Asp Ser Gly Leu Tyr Thr Leu Ser
130 135 140

Val Val Gly Leu Ser Asp Lys Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Pro Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro Pro Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val

450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr Ser
530 535 540

Ser Val Leu Trp
545

<210> 25
<211> 548
<212> PRT
<213> herpes simplex virus 2

<220>
<221> misc_feature
<222> (120)..(120)
<223> Xaa can be any naturally occurring amino acid

<220>
<221> misc_feature
<222> (141)..(198)
<223> Xaa can be any naturally occurring amino acid

<400> 25

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Xaa Val Ala Val Val Asn Glu Ser Leu
115 120 125

Val Ile Tyr Gly Ala Leu Glu Thr Asp Ser Gly Leu Xaa Xaa Xaa Xaa
130 135 140

Xaa Xaa Xaa Xaa Xaa Xaa Xaa Xaa Xaa Xaa Xaa Xaa Xaa Xaa Xaa Xaa
145 150 155 160

Xaa Xaa Xaa Xaa Xaa Xaa Xaa Xaa Xaa Xaa Xaa Xaa Xaa Xaa Xaa Xaa
165 170 175

Xaa Xaa Xaa Xaa Xaa Xaa Xaa Xaa Xaa Xaa Xaa Xaa Xaa Xaa Xaa Xaa
180 185 190

Xaa Xaa Xaa Xaa Xaa Xaa Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu

325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Met Arg Ala Pro His Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr Ser
530 535 540

Ser Val Leu Trp
545

<210> 26
<211> 548
<212> PRT
<213> herpes simplex virus 2

<400> 26

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

Val Ile Tyr Gly Ala Leu Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Pro Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro Pro Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr Ser
530 535 540

Ser Val Leu Trp
545

<210> 27
<211> 545
<212> PRT
<213> herpes simplex virus 2

<400> 27

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Glu Arg Thr Arg Ala
35 40 45

His Lys Leu Leu Trp Ala Ala Glu Pro Leu Asp Ala Cys Gly Pro Leu
50 55 60

Arg Pro Ser Trp Val Ala Leu Trp Pro Pro Arg Arg Val Leu Glu Thr
65 70 75 80

Val Val Asp Ala Ala Cys Met Arg Ala Pro Glu Pro Leu Ala Ile Ala
85 90 95

Tyr Ser Pro Pro Phe Pro Ala Gly Asp Glu Gly Leu Tyr Ser Glu Leu
100 105 110

Ala Trp Arg Asp Arg Val Ala Val Val Asn Glu Ser Leu Val Ile Tyr
115 120 125

Gly Ala Leu Glu Thr Asp Ser Gly Leu Tyr Thr Leu Ser Val Val Gly
130 135 140

Leu Ser Asp Glu Ala Arg Gln Val Ala Ser Val Val Leu Val Val Glu
145 150 155 160

Pro Ala Pro Val Pro Thr Pro Thr Pro Asp Asp Tyr Asp Glu Glu Asp
165 170 175

Asp Ala Gly Val Ser Glu Arg Thr Pro Val Ser Val Pro Pro Pro Thr
180 185 190

Pro Pro Arg Arg Pro Pro Val Ala Pro Pro Thr His Pro Arg Val Ile
195 200 205

Pro Glu Val Ser His Val Arg Gly Val Thr Val His Met Glu Thr Pro
210 215 220

Glu Ala Ile Leu Phe Ala Pro Gly Glu Thr Phe Gly Thr Asn Val Ser
225 230 235 240

Ile His Ala Ile Ala His Asp Asp Gly Pro Tyr Ala Met Asp Val Val
245 250 255

Trp Met Arg Phe Asp Val Pro Ser Ser Cys Ala Glu Met Arg Ile Tyr
260 265 270

Glu Ala Cys Leu Tyr His Pro Gln Leu Pro Glu Cys Leu Ser Pro Ala
275 280 285

Asp Ala Pro Cys Ala Val Ser Ser Trp Ala Tyr Arg Leu Ala Val Arg
290 295 300

Ser Tyr Ala Gly Cys Ser Arg Thr Thr Pro Pro Pro Arg Cys Phe Ala
305 310 315 320

Glu Ala Arg Met Glu Pro Val Pro Gly Leu Ala Trp Leu Ala Ser Thr
325 330 335

Val Asn Leu Glu Phe Gln His Ala Ser Pro Gln His Ala Gly Leu Tyr
340 345 350

Leu Cys Val Val Tyr Val Asp Asp His Ile His Ala Trp Gly His Met
355 360 365

Thr Ile Ser Thr Ala Ala Gln Tyr Arg Asn Ala Val Val Glu Gln His
370 375 380

Leu Pro Gln Arg Gln Pro Glu Pro Val Glu Pro Thr Arg Pro His Val
385 390 395 400

Arg Ala Pro His Pro Ala Pro Ser Ala Arg Gly Pro Leu Arg Leu Gly
405 410 415

Ala Val Leu Gly Ala Ala Leu Leu Leu Ala Ala Leu Gly Leu Ser Ala
420 425 430

Trp Ala Cys Met Thr Cys Trp Arg Arg Arg Ser Trp Arg Ala Val Lys
435 440 445

Ser Arg Ala Ser Ala Thr Gly Pro Thr Tyr Ile Arg Val Ala Asp Ser
450 455 460

Glu Leu Tyr Ala Asp Trp Ser Ser Asp Ser Glu Gly Glu Arg Asp Gly
465 470 475 480

Ser Leu Trp Gln Asp Pro Pro Glu Arg Pro Asp Ser Pro Ser Thr Asn
485 490 495

Gly Ser Gly Phe Glu Ile Leu Ser Pro Thr Ala Pro Ser Val Tyr Pro
500 505 510

His Ser Glu Gly Arg Lys Ser Arg Arg Pro Leu Thr Thr Phe Gly Ser
515 520 525

Gly Ser Pro Gly Arg Arg His Ser Gln Ala Ser Tyr Pro Ser Val Leu
530 535 540

Trp
545

<210> 28
<211> 545
<212> PRT
<213> herpes simplex virus 2

<400> 28

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Glu Arg Thr Arg Ala
35 40 45

His Lys Leu Leu Trp Ala Ala Glu Pro Leu Asp Ala Cys Gly Pro Leu
50 55 60

Arg Pro Ser Trp Val Ala Leu Trp Pro Pro Arg Arg Val Leu Glu Thr
65 70 75 80

```
Val Val Asp Ala Ala Cys Met Arg Ala Pro Glu Pro Leu Ala Ile Ala
                85                  90                  95

Tyr Ser Pro Pro Phe Pro Ala Gly Asp Glu Gly Leu Tyr Ser Glu Leu
            100                 105                 110

Ala Trp Arg Asp Arg Val Ala Val Val Asn Glu Ser Leu Val Ile Tyr
        115                 120                 125

Gly Ala Leu Glu Thr Asp Ser Gly Leu Tyr Thr Leu Ser Val Val Gly
    130                 135                 140

Leu Ser Asp Glu Ala Arg Gln Val Ala Ser Val Val Leu Val Val Glu
145                 150                 155                 160

Pro Ala Pro Val Pro Thr Pro Thr Pro Asp Asp Tyr Asp Glu Glu Asp
                165                 170                 175

Asp Ala Gly Val Thr Asn Ala Arg Arg Ser Ala Phe Pro Pro Gln Pro
            180                 185                 190

Pro Pro Arg Arg Pro Pro Val Ala Pro Pro Thr His Pro Arg Val Ile
        195                 200                 205

Pro Glu Val Ser His Val Arg Gly Val Thr Val His Met Glu Thr Leu
    210                 215                 220

Glu Ala Ile Leu Phe Ala Pro Gly Glu Thr Phe Gly Thr Asn Val Ser
225                 230                 235                 240

Ile His Ala Ile Ala His Asp Asp Gly Pro Tyr Ala Met Asp Val Val
                245                 250                 255

Trp Met Arg Phe Asp Val Pro Ser Ser Cys Ala Asp Met Arg Ile Tyr
            260                 265                 270

Glu Ala Cys Leu Tyr His Pro Gln Leu Pro Glu Cys Leu Ser Pro Ala
        275                 280                 285

Asp Ala Pro Cys Ala Val Ser Ser Trp Ala Tyr Arg Leu Ala Val Arg
    290                 295                 300

Ser Tyr Ala Gly Cys Ser Arg Thr Thr Pro Pro Pro Arg Cys Phe Ala
305                 310                 315                 320

Glu Ala Arg Met Glu Pro Val Pro Gly Leu Ala Trp Leu Ala Ser Thr
```

325 330 335

Val Asn Leu Glu Phe Gln His Ala Ser Pro Gln His Ala Gly Leu Tyr
340 345 350

Leu Cys Val Val Tyr Val Asp Asp His Ile His Ala Trp Gly His Met
355 360 365

Thr Ile Ser Thr Ala Ala Gln Tyr Arg Asn Ala Val Val Glu Gln His
370 375 380

Leu Pro Gln Arg Gln Pro Glu Pro Val Glu Pro Thr Arg Pro His Val
385 390 395 400

Arg Ala Pro His Pro Ala Pro Ser Ala Arg Gly Pro Leu Arg Leu Gly
405 410 415

Ala Val Leu Gly Ala Ala Leu Leu Leu Ala Ala Leu Gly Leu Ser Ala
420 425 430

Trp Ala Cys Met Thr Cys Trp Arg Arg Arg Ser Trp Arg Ala Val Lys
435 440 445

Ser Arg Ala Ser Ala Thr Gly Pro Thr Tyr Ile Arg Val Ala Asp Ser
450 455 460

Glu Leu Tyr Ala Asp Trp Ser Ser Asp Ser Glu Gly Glu Arg Asp Gly
465 470 475 480

Ser Leu Trp Gln Asp Pro Pro Glu Arg Pro Asp Ser Pro Ser Thr Asn
485 490 495

Gly Ser Gly Phe Glu Ile Leu Ser Pro Thr Ala Pro Ser Val Tyr Pro
500 505 510

His Ser Glu Gly Arg Lys Ser Arg Arg Pro Leu Thr Thr Phe Gly Ser
515 520 525

Gly Ser Pro Gly Arg Arg His Ser Gln Ala Ser Tyr Pro Ser Val Leu
530 535 540

Trp
545

<210> 29
<211> 545
<212> PRT
<213> herpes simplex virus 2

<400> 29

Met Ala Arg Gly Ala Gly Leu Val Phe Phe Val Gly Val Trp Val Val
1               5                   10                  15

Ser Cys Leu Ala Ala Ala Pro Arg Thr Ser Trp Lys Arg Val Thr Ser
            20                  25                  30

Gly Glu Asp Val Val Leu Leu Pro Ala Pro Ala Glu Arg Thr Arg Ala
        35                  40                  45

His Lys Leu Leu Trp Ala Ala Glu Pro Leu Asp Ala Cys Gly Pro Leu
    50                  55                  60

Arg Pro Ser Trp Val Ala Leu Trp Pro Pro Arg Arg Val Leu Glu Thr
65                  70                  75                  80

Val Val Asp Ala Ala Cys Met Arg Ala Pro Glu Pro Leu Ala Ile Ala
                85                  90                  95

Tyr Ser Pro Pro Phe Pro Ala Gly Asp Glu Gly Leu Tyr Ser Glu Leu
            100                 105                 110

Ala Trp Arg Asp Arg Val Ala Val Val Asn Glu Ser Leu Val Ile Tyr
        115                 120                 125

Gly Ala Leu Glu Thr Asp Ser Gly Leu Tyr Thr Leu Ser Val Val Gly
    130                 135                 140

Leu Ser Asp Glu Ala Arg Gln Val Ala Ser Val Val Leu Val Val Glu
145                 150                 155                 160

Pro Ala Pro Val Pro Thr Pro Thr Pro Asp Asp Tyr Asp Glu Glu Asp
                165                 170                 175

Asp Ala Gly Val Ser Glu Arg Thr Pro Val Ser Val Pro Pro Pro Thr
            180                 185                 190

Pro Pro Arg Arg Pro Pro Val Ala Pro Pro Thr His Pro Arg Val Ile
        195                 200                 205

Pro Glu Val Ser His Val Arg Gly Val Thr Val His Met Glu Thr Pro
    210                 215                 220

Glu Ala Ile Leu Phe Ala Pro Gly Glu Thr Phe Gly Thr Asn Val Ser
225                 230                 235                 240

Ile His Ala Ile Ala His Asp Asp Gly Pro Tyr Ala Met Asp Val Val
245 250 255

Trp Met Arg Phe Asp Val Pro Ser Ser Cys Ala Glu Met Arg Ile Tyr
260 265 270

Glu Ala Cys Leu Tyr His Pro Gln Leu Pro Glu Cys Leu Ser Pro Ala
275 280 285

Asp Ala Pro Cys Ala Val Ser Ser Trp Ala Tyr Arg Leu Ala Val Arg
290 295 300

Ser Tyr Ala Gly Cys Ser Arg Thr Thr Pro Pro Pro Arg Cys Phe Ala
305 310 315 320

Glu Ala Arg Met Glu Pro Val Pro Gly Leu Ala Trp Leu Ala Ser Thr
325 330 335

Val Asn Leu Glu Phe Gln His Ala Ser Pro Gln His Ala Gly Leu Tyr
340 345 350

Leu Cys Val Val Tyr Val Asp Asp His Ile His Ala Trp Gly His Met
355 360 365

Thr Ile Ser Thr Ala Ala Gln Tyr Arg Asn Ala Val Val Glu Gln His
370 375 380

Leu Pro Gln Arg Gln Pro Glu Pro Val Glu Pro Thr Arg Pro His Val
385 390 395 400

Arg Ala Pro His Pro Ala Pro Ser Ala Arg Gly Pro Leu Arg Leu Gly
405 410 415

Ala Val Leu Gly Ala Ala Leu Leu Leu Ala Ala Leu Gly Leu Ser Ala
420 425 430

Trp Ala Cys Met Thr Cys Trp Arg Arg Arg Ser Trp Arg Ala Val Lys
435 440 445

Ser Arg Ala Ser Ala Thr Gly Pro Thr Tyr Ile Arg Val Ala Asp Ser
450 455 460

Glu Leu Tyr Ala Asp Trp Ser Ser Asp Ser Glu Gly Glu Arg Asp Gly
465 470 475 480

Ser Leu Trp Gln Asp Pro Pro Glu Arg Pro Asp Ser Pro Ser Thr Asn
485 490 495

```
Gly Ser Gly Phe Glu Ile Leu Ser Pro Thr Ala Pro Ser Val Tyr Pro
            500                 505                 510

His Ser Glu Gly Arg Lys Ser Arg Arg Pro Leu Thr Thr Phe Gly Ser
        515                 520                 525

Gly Ser Pro Gly Arg Arg His Ser Gln Ala Ser Tyr Pro Ser Val Leu
    530                 535                 540

Trp
545


<210>  30
<211>  548
<212>  PRT
<213>  herpes simplex virus 2

<400>  30

Met Ala Arg Gly Ala Gly Leu Val Phe Phe Val Gly Val Trp Val Val
1               5                   10                  15

Ser Cys Leu Ala Ala Ala Pro Arg Thr Ser Trp Lys Arg Val Thr Ser
            20                  25                  30

Gly Glu Asp Val Val Leu Leu Pro Ala Pro Ala Gly Pro Glu Glu Arg
        35                  40                  45

Thr Arg Ala His Lys Leu Leu Trp Ala Ala Glu Pro Leu Asp Ala Cys
    50                  55                  60

Gly Pro Leu Arg Pro Ser Trp Val Ala Leu Trp Pro Pro Arg Arg Val
65                  70                  75                  80

Leu Glu Thr Val Val Asp Ala Ala Cys Met Arg Ala Pro Glu Pro Leu
                85                  90                  95

Ala Ile Ala Tyr Ser Pro Pro Phe Pro Ala Gly Asp Glu Gly Leu Tyr
            100                 105                 110

Ser Glu Leu Ala Trp Arg Asp Arg Val Ala Val Val Asn Glu Ser Leu
        115                 120                 125

Val Ile Tyr Gly Ala Leu Glu Thr Asp Ser Gly Leu Tyr Thr Leu Ser
    130                 135                 140

Val Val Gly Leu Ser Asp Glu Ala Arg Gln Val Ala Ser Val Val Leu
145                 150                 155                 160
```

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Pro Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro Pro Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr Ser
530 535 540

Ser Val Leu Trp
545

<210> 31
<211> 548
<212> PRT
<213> herpes simplex virus 2

<400> 31

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

Val Ile Tyr Gly Ala Leu Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Pro Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu

```
                325                 330                 335

Ala Ser Thr Val Asn Leu Glu Phe Gln His Ala Ser Pro Gln His Ala
            340                 345                 350

Gly Leu Tyr Leu Cys Val Val Tyr Val Asp Asp His Ile His Ala Trp
        355                 360                 365

Gly His Met Thr Ile Ser Thr Ala Ala Gln Tyr Arg Asn Ala Val Val
    370                 375                 380

Glu Gln His Leu Pro Gln Arg Gln Pro Glu Pro Val Glu Pro Thr Arg
385                 390                 395                 400

Pro His Val Arg Ala Pro Pro Pro Ala Pro Ser Ala Arg Gly Pro Leu
                405                 410                 415

Arg Leu Gly Ala Val Leu Gly Ala Ala Leu Leu Leu Ala Ala Leu Gly
            420                 425                 430

Leu Ser Ala Trp Ala Cys Met Thr Cys Trp Arg Arg Arg Ser Trp Arg
        435                 440                 445

Ala Val Lys Ser Arg Ala Ser Ala Thr Gly Pro Thr Tyr Ile Arg Val
    450                 455                 460

Ala Asp Ser Glu Leu Tyr Ala Asp Trp Ser Ser Asp Ser Glu Gly Glu
465                 470                 475                 480

Arg Asp Gly Ser Leu Trp Gln Asp Pro Pro Glu Arg Pro Asp Ser Pro
                485                 490                 495

Ser Thr Asn Gly Ser Gly Phe Glu Ile Leu Ser Pro Thr Ala Pro Ser
            500                 505                 510

Val Tyr Pro His Ser Glu Gly Arg Lys Ser Arg Arg Pro Leu Thr Thr
        515                 520                 525

Phe Gly Ser Gly Ser Pro Gly Arg Arg His Ser Gln Ala Ser Tyr Ser
    530                 535                 540

Ser Val Leu Trp
545
```

<210>  32
<211>  548
<212>  PRT
<213>  herpes simplex virus 2

<400> 32

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

Val Ile Tyr Gly Ala Leu Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Ala Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro His Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr Ser
530 535 540

Ser Val Leu Trp
545

<210> 33
<211> 548
<212> PRT
<213> herpes simplex virus 2

<400> 33

Met Ala Arg Gly Ala Gly Leu Val Phe Phe Val Gly Val Trp Val Val
1 5 10 15

Ser Cys Leu Gly Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

Val Ile Tyr Gly Ala Leu Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Pro Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro His Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr Ser
530 535 540

Ser Val Leu Trp
545

<210> 34
<211> 548
<212> PRT
<213> herpes simplex virus 2

<400> 34

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

Val Ile Tyr Gly Ala Leu Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Pro Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu

325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro His Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr Ser
530 535 540

Ser Val Leu Trp
545

<210> 35
<211> 548
<212> PRT
<213> herpes simplex virus 2

<400> 35

Met Ala Arg Gly Ala Gly Leu Val Phe Phe Val Gly Val Trp Val Val
1               5                   10                  15

Ser Cys Leu Ala Ala Ala Pro Arg Thr Ser Trp Lys Arg Val Thr Ser
            20                  25                  30

Gly Glu Asp Val Val Leu Leu Pro Ala Pro Ala Gly Pro Glu Glu Arg
        35                  40                  45

Thr Arg Ala His Lys Leu Leu Trp Ala Ala Glu Pro Leu Asp Ala Cys
    50                  55                  60

Gly Pro Leu Arg Pro Ser Trp Val Ala Leu Trp Pro Pro Arg Arg Val
65                  70                  75                  80

Leu Glu Thr Val Val Asp Ala Ala Cys Met Arg Ala Pro Glu Pro Leu
                85                  90                  95

Ala Ile Ala Tyr Ser Pro Pro Phe Pro Ala Gly Asp Glu Gly Leu Tyr
            100                 105                 110

Ser Glu Leu Ala Trp Arg Asp Arg Val Ala Val Val Asn Glu Ser Leu
        115                 120                 125

Val Ile Tyr Gly Ala Leu Glu Thr Asp Ser Gly Leu Tyr Thr Leu Ser
    130                 135                 140

Val Val Gly Leu Ser Asp Glu Ala Arg Gln Val Ala Ser Val Val Leu
145                 150                 155                 160

Val Val Glu Pro Ala Pro Val Pro Thr Pro Thr Pro Asp Asp Tyr Asp
                165                 170                 175

Glu Glu Asp Asp Ala Gly Val Ser Glu Arg Thr Pro Val Ser Val Pro
            180                 185                 190

Pro Pro Thr Pro Pro Arg Arg Pro Pro Val Ala Pro Pro Thr His Pro
        195                 200                 205

Arg Val Ile Pro Glu Val Ser His Val Arg Gly Val Thr Val His Met
    210                 215                 220

Glu Thr Pro Glu Ala Ile Leu Phe Ala Pro Gly Glu Thr Phe Gly Thr
225                 230                 235                 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro Pro Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr Ser
530 535 540

Ser Val Leu Trp
545

<210> 36
<211> 548
<212> PRT
<213> herpes simplex virus 2

<400> 36

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

Val Ile Tyr Gly Ala Leu Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Pro Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro Pro Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr Ser
530 535 540

Ser Val Leu Trp
545

<210> 37
<211> 548
<212> PRT
<213> herpes simplex virus 2

<400> 37

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

```
Leu Glu Thr Val Val Asp Ala Ala Cys Met Arg Ala Pro Glu Pro Leu
                85                  90                  95

Ala Ile Ala Tyr Ser Pro Pro Phe Pro Ala Gly Asp Glu Gly Leu Tyr
            100                 105                 110

Ser Glu Leu Ala Trp Arg Asp Arg Val Ala Val Val Asn Glu Ser Leu
        115                 120                 125

Val Ile Tyr Gly Ala Leu Glu Thr Asp Ser Gly Leu Tyr Thr Leu Ser
    130                 135                 140

Val Val Gly Leu Ser Asp Glu Ala Arg Gln Val Ala Ser Val Val Leu
145                 150                 155                 160

Val Val Glu Pro Ala Pro Val Pro Thr Pro Thr Pro Asp Asp Tyr Asp
                165                 170                 175

Glu Glu Asp Asp Ala Gly Val Ser Glu Arg Thr Pro Val Ser Val Pro
            180                 185                 190

Pro Pro Thr Pro Pro Arg Arg Pro Pro Val Ala Pro Pro Thr His Pro
        195                 200                 205

Arg Val Ile Pro Glu Val Ser His Val Arg Gly Val Thr Val His Met
    210                 215                 220

Glu Thr Pro Glu Ala Ile Leu Phe Ala Pro Gly Glu Thr Phe Gly Thr
225                 230                 235                 240

Asn Val Ser Ile His Ala Ile Ala His Asp Asp Gly Pro Tyr Ala Met
                245                 250                 255

Asp Val Val Trp Met Arg Phe Asp Val Pro Ser Ser Cys Ala Glu Met
            260                 265                 270

Arg Ile Tyr Glu Ala Cys Leu Tyr His Pro Gln Leu Pro Glu Cys Leu
        275                 280                 285

Ser Pro Ala Asp Ala Pro Cys Ala Val Ser Ser Trp Ala Tyr Arg Leu
    290                 295                 300

Ala Val Arg Ser Tyr Ala Gly Cys Ser Arg Thr Thr Pro Pro Pro Arg
305                 310                 315                 320

Cys Phe Ala Glu Ala Arg Met Glu Pro Val Pro Gly Leu Ala Trp Leu
```

325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro Pro Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr Ser
530 535 540

Ser Val Leu Trp
545

<210> 38
<211> 548
<212> PRT
<213> herpes simplex virus 2

<400> 38

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

Val Ile Tyr Gly Ala Leu Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Pro Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro His Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr Ser
530 535 540

Ser Val Leu Trp
545

<210> 39
<211> 548
<212> PRT
<213> herpes simplex virus 2

<400> 39

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

Val Ile Tyr Gly Ala Leu Asp Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Pro Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro His Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr Ser
530 535 540

Ser Val Leu Trp
545

<210> 40
<211> 548
<212> PRT
<213> herpes simplex virus 2

<400> 40

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

Val Ile Tyr Gly Ala Leu Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Pro Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu

325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro Pro Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr Ser
530 535 540

Ser Val Leu Trp
545

<210> 41
<211> 539
<212> PRT
<213> herpes simplex virus 2

<400> 41

Met Ala Arg Gly Ala Gly Leu Val Phe Phe Val Gly Val Trp Val Val
1               5                   10                  15

Ser Cys Leu Ala Ala Ala Pro Arg Thr Ser Trp Lys Arg Val Thr Ser
            20                  25                  30

Gly Glu Asp Val Val Leu Leu Pro Ala Pro Ala Gly Pro Glu Glu Arg
        35                  40                  45

Thr Arg Ala His Lys Leu Leu Trp Ala Ala Glu Pro Leu Asp Ala Cys
    50                  55                  60

Gly Pro Leu Arg Pro Ser Trp Val Ala Leu Trp Pro Pro Arg Arg Val
65                  70                  75                  80

Leu Glu Thr Val Val Asp Ala Ala Cys Met Arg Ala Pro Glu Pro Leu
                85                  90                  95

Ala Ile Ala Tyr Ser Pro Pro Phe Pro Ala Gly Asp Glu Gly Leu Tyr
            100                 105                 110

Ser Glu Leu Ala Trp Arg Asp Arg Val Ala Val Val Asn Glu Ser Leu
        115                 120                 125

Val Ile Tyr Gly Ala Arg Glu Thr Asp Ser Gly Leu Tyr Thr Leu Ser
    130                 135                 140

Val Val Gly Leu Ser Asp Glu Ala Arg Gln Val Ala Ser Val Val Leu
145                 150                 155                 160

Val Val Glu Pro Ala Pro Val Pro Thr Pro Thr Pro Asp Asp Tyr Asp
                165                 170                 175

Glu Glu Asp Asp Ala Gly Val Ser Glu Arg Thr Pro Val Ser Val Pro
            180                 185                 190

Pro Pro Thr Pro Pro Arg Arg Pro Pro Val Ala Pro Pro Thr His Pro
        195                 200                 205

Arg Val Ile Pro Glu Val Ser His Val Arg Gly Val Thr Val His Met
    210                 215                 220

Glu Thr Pro Glu Ala Ile Leu Phe Ala Pro Gly Glu Thr Phe Gly Thr
225                 230                 235                 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro His Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser
530 535

<210> 42
<211> 545
<212> PRT
<213> herpes simplex virus 2

<400> 42

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Glu Arg Thr Arg Ala
35 40 45

His Lys Leu Leu Trp Ala Ala Glu Pro Leu Asp Ala Cys Gly Pro Leu
50 55 60

Arg Pro Ser Trp Val Ala Leu Trp Pro Pro Arg Arg Val Leu Glu Thr
65 70 75 80

Val Val Asp Ala Ala Cys Met Arg Ala Pro Glu Pro Leu Ala Ile Ala
85 90 95

Tyr Ser Pro Pro Phe Pro Ala Gly Asp Glu Gly Leu Tyr Ser Glu Leu
100 105 110

Ala Trp Arg Asp Arg Val Ala Val Val Asn Glu Ser Leu Val Ile Tyr
115 120 125

Gly Ala Leu Glu Thr Asp Ser Gly Leu Tyr Thr Leu Ser Val Val Gly
130 135 140

Leu Ser Asp Glu Ala Arg Gln Val Ala Ser Val Val Leu Val Val Glu
145 150 155 160

Pro Ala Pro Val Pro Thr Pro Thr Pro Asp Asp Tyr Asp Glu Glu Asp
165 170 175

Asp Ala Gly Val Ser Glu Arg Thr Pro Val Ser Val Pro Pro Pro Thr
180 185 190

Pro Pro Arg Arg Pro Pro Val Ala Pro Pro Thr His Pro Arg Val Ile
195 200 205

Pro Glu Val Ser His Val Arg Gly Val Thr Val His Met Glu Thr Pro
210 215 220

Glu Ala Ile Leu Phe Ala Pro Gly Glu Thr Phe Gly Thr Asn Val Ser
225 230 235 240

Ile His Ala Ile Ala His Asp Asp Gly Pro Tyr Ala Met Asp Val Val
245 250 255

Trp Met Arg Phe Asp Val Pro Ser Ser Cys Ala Glu Met Arg Ile Tyr
260 265 270

Glu Ala Cys Leu Tyr His Pro Gln Leu Pro Glu Cys Leu Ser Pro Ala
275 280 285

Asp Ala Pro Cys Ala Val Ser Ser Trp Ala Tyr Arg Leu Ala Val Arg
290 295 300

Ser Tyr Ala Gly Cys Ser Arg Thr Thr Pro Pro Pro Arg Cys Phe Ala
305 310 315 320

Glu Ala Arg Met Glu Pro Val Pro Gly Leu Ala Trp Leu Ala Ser Thr
325 330 335

Val Asn Leu Glu Phe Gln His Ala Ser Pro Gln His Ala Gly Leu Tyr
340 345 350

Leu Cys Val Val Tyr Val Asp Asp His Ile His Ala Trp Gly His Met
355 360 365

Thr Ile Ser Thr Ala Ala Gln Tyr Arg Asn Ala Val Val Glu Gln His
370 375 380

Leu Pro Gln Arg Gln Pro Glu Pro Val Glu Pro Thr Arg Pro His Val
385 390 395 400

Arg Ala Pro His Pro Ala Pro Ser Ala Arg Gly Pro Leu Arg Leu Gly
405 410 415

Ala Val Leu Gly Ala Ala Leu Leu Leu Ala Ala Leu Gly Leu Ser Ala
420 425 430

```
Trp Ala Cys Met Thr Cys Trp Arg Arg Arg Ser Trp Arg Ala Val Lys
        435                 440                 445

Ser Arg Ala Ser Ala Thr Gly Pro Thr Tyr Ile Arg Val Ala Asp Ser
    450                 455                 460

Glu Leu Tyr Ala Asp Trp Ser Ser Asp Ser Glu Gly Glu Arg Asp Gly
465                 470                 475                 480

Ser Leu Trp Gln Asp Pro Pro Glu Arg Pro Asp Ser Pro Ser Thr Asn
                485                 490                 495

Gly Ser Gly Phe Glu Ile Leu Ser Pro Thr Ala Pro Ser Val Tyr Pro
            500                 505                 510

His Ser Glu Gly Arg Lys Ser Arg Arg Pro Leu Thr Thr Phe Gly Ser
        515                 520                 525

Gly Ser Pro Gly Arg Arg His Ser Gln Ala Ser Tyr Pro Ser Val Leu
    530                 535                 540

Trp
545

<210>  43
<211>  543
<212>  PRT
<213>  herpes simplex virus 2

<400>  43

Met Ala Arg Gly Ala Gly Leu Val Phe Phe Val Gly Val Trp Val Val
1               5                   10                  15

Ser Cys Leu Ala Ala Ala Pro Arg Thr Ser Trp Lys Arg Val Thr Ser
            20                  25                  30

Gly Glu Asp Val Val Leu Leu Pro Ala Pro Ala Gly Pro Glu Glu Arg
        35                  40                  45

Thr Arg Ala His Lys Leu Leu Trp Ala Ala Glu Pro Leu Asp Ala Cys
    50                  55                  60

Gly Pro Leu Arg Pro Ser Trp Val Ala Leu Trp Pro Pro Arg Arg Val
65                  70                  75                  80

Leu Glu Thr Val Val Asp Ala Ala Cys Met Arg Ala Pro Glu Pro Leu
                85                  90                  95
```

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

Val Ile Tyr Gly Ala Leu Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Pro Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala

340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro His Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr
530 535 540

<210> 44
<211> 544
<212> PRT
<213> herpes simplex virus 2

<400> 44

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser

20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

Val Ile Tyr Gly Ala Leu Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Pro Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
        275                 280                 285

Ser Pro Ala Asp Ala Pro Cys Ala Val Ser Ser Trp Ala Tyr Arg Leu
    290                 295                 300

Ala Val Arg Ser Tyr Ala Gly Cys Ser Arg Thr Thr Pro Pro Pro Arg
305                 310                 315                 320

Cys Phe Ala Glu Ala Arg Met Glu Pro Val Pro Gly Leu Ala Trp Leu
                325                 330                 335

Ala Ser Thr Val Asn Leu Glu Phe Gln His Ala Ser Pro Gln His Ala
            340                 345                 350

Gly Leu Tyr Leu Cys Val Val Tyr Val Asp Asp His Ile His Ala Trp
        355                 360                 365

Gly His Met Thr Ile Ser Thr Ala Ala Gln Tyr Arg Asn Ala Val Val
    370                 375                 380

Glu Gln His Leu Pro Gln Arg Gln Pro Glu Pro Val Glu Pro Thr Arg
385                 390                 395                 400

Pro His Val Arg Ala Pro Pro Pro Ala Pro Ser Ala Arg Gly Pro Leu
                405                 410                 415

Arg Leu Gly Ala Val Leu Gly Ala Ala Leu Leu Leu Ala Ala Leu Gly
            420                 425                 430

Leu Ser Ala Trp Ala Cys Met Thr Cys Trp Arg Arg Arg Ser Trp Arg
        435                 440                 445

Ala Val Lys Ser Arg Ala Ser Ala Thr Gly Pro Thr Tyr Ile Arg Val
    450                 455                 460

Ala Asp Ser Glu Leu Tyr Ala Asp Trp Ser Ser Asp Ser Glu Gly Glu
465                 470                 475                 480

Arg Asp Gly Ser Leu Trp Gln Asp Pro Pro Glu Arg Pro Asp Ser Pro
                485                 490                 495

Ser Thr Asn Gly Ser Gly Phe Glu Ile Leu Ser Pro Thr Ala Pro Ser
            500                 505                 510

Val Tyr Pro His Ser Glu Gly Arg Lys Ser Arg His Pro Leu Thr Thr
        515                 520                 525

Phe Gly Ser Gly Ser Pro Gly Arg Arg His Ser Gln Ala Ser Tyr Ser
530 535 540

<210> 45
<211> 545
<212> PRT
<213> herpes simplex virus 2

<400> 45

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Glu Arg Thr Arg Ala
35 40 45

His Lys Leu Leu Trp Ala Ala Glu Pro Leu Asp Ala Cys Gly Pro Leu
50 55 60

Arg Pro Ser Trp Val Ala Leu Trp Pro Pro Arg Arg Val Leu Glu Thr
65 70 75 80

Val Val Asp Ala Ala Cys Met Arg Ala Pro Glu Pro Leu Ala Ile Ala
85 90 95

Tyr Ser Pro Pro Phe Pro Ala Gly Asp Glu Gly Leu Tyr Ser Glu Leu
100 105 110

Ala Trp Arg Asp Arg Val Ala Val Val Asn Glu Ser Leu Val Ile Tyr
115 120 125

Gly Ala Leu Glu Thr Asp Ser Gly Leu Tyr Thr Leu Ser Val Val Gly
130 135 140

Leu Ser Asp Glu Ala Arg Gln Val Ala Ser Val Val Leu Val Val Glu
145 150 155 160

Pro Ala Pro Val Pro Thr Pro Thr Pro Asp Asp Tyr Asp Glu Glu Asp
165 170 175

Asp Ala Gly Val Ser Glu Arg Thr Pro Val Ser Val Pro Pro Pro Thr
180 185 190

Pro Pro Arg Arg Pro Pro Val Ala Pro Pro Thr His Pro Arg Val Ile
195 200 205

```
Pro Glu Val Ser His Val Arg Gly Val Thr Val His Met Glu Thr Pro
    210                 215                 220

Glu Ala Ile Leu Phe Ala Pro Gly Glu Thr Phe Gly Thr Asn Val Ser
225                 230                 235                 240

Ile His Ala Ile Ala His Asp Asp Gly Pro Tyr Ala Met Asp Val Val
                245                 250                 255

Trp Met Arg Phe Asp Val Pro Ser Ser Cys Ala Glu Met Arg Ile Tyr
            260                 265                 270

Glu Ala Cys Leu Tyr His Pro Gln Leu Pro Glu Cys Leu Ser Pro Ala
        275                 280                 285

Asp Ala Pro Cys Ala Val Ser Ser Trp Ala Tyr Arg Leu Ala Val Arg
    290                 295                 300

Ser Tyr Ala Gly Cys Ser Arg Thr Thr Pro Pro Pro Arg Cys Phe Ala
305                 310                 315                 320

Glu Ala Arg Met Glu Pro Val Pro Gly Leu Ala Trp Leu Ala Ser Thr
                325                 330                 335

Val Asn Leu Glu Phe Gln His Ala Ser Pro Gln His Ala Gly Leu Tyr
            340                 345                 350

Leu Cys Val Val Tyr Val Asp Asp His Ile His Ala Trp Gly His Met
        355                 360                 365

Thr Ile Ser Thr Ala Ala Gln Tyr Arg Asn Ala Val Val Glu Gln His
    370                 375                 380

Leu Pro Gln Arg Gln Pro Glu Pro Val Glu Pro Thr Arg Pro His Val
385                 390                 395                 400

Arg Ala Pro His Pro Ala Pro Ser Ala Arg Gly Pro Leu Arg Leu Gly
                405                 410                 415

Ala Val Leu Gly Ala Ala Leu Leu Leu Ala Ala Leu Gly Leu Ser Ala
            420                 425                 430

Trp Ala Cys Met Thr Cys Trp Arg Arg Arg Ser Trp Arg Ala Val Lys
        435                 440                 445

Ser Arg Ala Ser Ala Thr Gly Pro Thr Tyr Ile Arg Val Ala Asp Ser
    450                 455                 460
```

Glu Leu Tyr Ala Asp Trp Ser Ser Asp Ser Glu Gly Glu Arg Asp Gly
465 470 475 480

Ser Leu Trp Gln Asp Pro Pro Glu Arg Pro Asp Ser Pro Ser Thr Asn
485 490 495

Gly Ser Gly Phe Glu Ile Leu Ser Pro Thr Ala Pro Ser Val Tyr Pro
500 505 510

His Ser Glu Gly Arg Lys Ser Arg Arg Pro Leu Thr Thr Phe Gly Ser
515 520 525

Gly Ser Pro Gly Arg Arg His Ser Gln Ala Ser Tyr Pro Ser Val Leu
530 535 540

Trp
545

<210> 46
<211> 543
<212> PRT
<213> herpes simplex virus 2

<400> 46

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

```
Val Ile Tyr Gly Ala Leu Glu Thr Asp Ser Gly Leu Tyr Thr Leu Ser
    130                 135                 140

Val Val Gly Leu Ser Asp Glu Ala Arg Gln Val Ala Ser Val Val Leu
145                 150                 155                 160

Val Val Glu Pro Ala Pro Val Pro Thr Pro Thr Pro Asp Asp Tyr Asp
                165                 170                 175

Glu Glu Asp Asp Ala Gly Val Ser Glu Arg Thr Pro Val Ser Val Pro
            180                 185                 190

Pro Pro Thr Pro Pro Arg Arg Pro Pro Val Ala Pro Pro Thr His Pro
        195                 200                 205

Arg Val Ile Pro Glu Val Ser His Val Arg Gly Val Thr Val His Met
    210                 215                 220

Glu Thr Pro Glu Ala Ile Leu Phe Ala Pro Gly Glu Thr Phe Gly Thr
225                 230                 235                 240

Asn Val Ser Ile His Ala Ile Ala His Asp Asp Gly Pro Tyr Ala Met
                245                 250                 255

Asp Val Val Trp Met Arg Phe Asp Val Pro Ser Ser Cys Ala Glu Met
            260                 265                 270

Arg Ile Tyr Glu Ala Cys Leu Tyr His Pro Gln Leu Pro Glu Cys Leu
        275                 280                 285

Ser Pro Ala Asp Ala Pro Cys Ala Val Ser Ser Trp Ala Tyr Arg Leu
    290                 295                 300

Ala Val Arg Ser Tyr Ala Gly Cys Ser Arg Thr Thr Pro Pro Pro Arg
305                 310                 315                 320

Cys Phe Ala Glu Ala Arg Met Glu Pro Val Pro Gly Leu Ala Trp Leu
                325                 330                 335

Ala Ser Thr Val Asn Leu Glu Phe Gln His Ala Ser Pro Gln His Ala
            340                 345                 350

Gly Leu Tyr Leu Cys Val Val Tyr Val Asp Asp His Ile His Ala Trp
        355                 360                 365

Gly His Met Thr Ile Ser Thr Ala Ala Gln Tyr Arg Asn Ala Val Val
```

370 375 380

Glu Gln His Leu Pro Gln Arg Gln Pro Glu Pro Val Glu Pro Thr Arg
385 390 395 400

Pro His Val Arg Ala Pro His Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr
530 535 540

<210> 47
<211> 542
<212> PRT
<213> herpes simplex virus 2

<400> 47

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys

50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

Val Ile Tyr Gly Ala Leu Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Pro Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro Pro Pro Ala Pro Ser Ala Arg Gly Pro Leu
405 410 415

Arg Leu Gly Ala Val Leu Gly Ala Ala Leu Leu Leu Ala Gly Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser
530 535 540

<210> 48
<211> 538
<212> PRT

<213> herpes simplex virus 2

<400> 48

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Val His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

Val Ile Tyr Gly Ala Leu Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Pro Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro His Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His
530 535

<210> 49
<211> 534
<212> PRT
<213> herpes simplex virus 2

<400> 49

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

Val Ile Tyr Gly Ala Leu Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Pro Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro Pro Pro Ala Pro Ser Ala Arg Gly Pro Leu
405 410 415

Arg Leu Gly Ala Val Leu Gly Ala Ala Leu Leu Leu Ala Ala Leu Gly

420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro
530

<210> 50
<211> 548
<212> PRT
<213> herpes simplex virus 2

<400> 50

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr

100 105 110

Ser Glu Leu Ala Trp Arg Asp Arg Val Ala Val Val Asn Glu Ser Leu
115 120 125

Val Ile Tyr Gly Ala Leu Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Leu Val Ser Val Pro
180 185 190

Pro Pro Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

```
Gly Leu Tyr Leu Cys Val Val Tyr Val Asp Asp His Ile His Ala Trp
        355                 360                 365

Gly His Met Thr Ile Ser Thr Ala Ala Gln Tyr Arg Asn Ala Val Val
    370                 375                 380

Glu Gln His Leu Pro Gln Arg Gln Pro Glu Pro Val Glu Pro Thr Arg
385                 390                 395                 400

Pro His Val Arg Ala Pro Pro Pro Ala Pro Ser Ala Arg Gly Pro Leu
                405                 410                 415

Arg Leu Gly Ala Val Leu Gly Ala Ala Leu Leu Leu Ala Ala Leu Gly
            420                 425                 430

Leu Ser Ala Trp Ala Cys Met Thr Cys Trp Arg Arg Arg Ser Trp Arg
        435                 440                 445

Ala Val Lys Ser Arg Ala Ser Ala Thr Gly Pro Thr Tyr Ile Arg Val
    450                 455                 460

Ala Asp Ser Glu Leu Tyr Ala Asp Trp Ser Ser Asp Ser Glu Gly Glu
465                 470                 475                 480

Arg Asp Gly Ser Leu Trp Gln Asp Pro Pro Glu Arg Pro Asp Ser Pro
                485                 490                 495

Ser Thr Asn Gly Ser Gly Phe Glu Ile Leu Ser Pro Thr Ala Pro Ser
            500                 505                 510

Val Tyr Pro His Ser Glu Gly Arg Lys Ser Arg Arg Pro Leu Thr Thr
        515                 520                 525

Phe Gly Ser Gly Ser Pro Gly Arg Arg His Ser Gln Ala Ser Tyr Ser
    530                 535                 540

Ser Val Leu Trp
545
```

<210> 51
<211> 548
<212> PRT
<213> herpes simplex virus 2

<400> 51

```
Met Ala Arg Gly Ala Gly Leu Val Phe Phe Val Gly Val Trp Val Val
1               5                   10                  15
```

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

Val Ile Tyr Gly Ala Arg Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Thr Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro His Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr Ser
530 535 540

Ser Val Leu Trp
545

<210> 52
<211> 548
<212> PRT
<213> herpes simplex virus 2

<400> 52

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

Val Ile Tyr Gly Ala Arg Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Thr Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro His Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg

435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr Ser
530 535 540

Ser Val Leu Trp
545

<210> 53
<211> 548
<212> PRT
<213> herpes simplex virus 2

<400> 53

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr

```
            100                 105                 110

Ser Glu Leu Ala Trp Arg Asp Arg Val Ala Val Val Asn Glu Ser Leu
        115                 120                 125

Val Ile Tyr Gly Ala Arg Glu Thr Asp Ser Gly Leu Tyr Thr Leu Ser
    130                 135                 140

Val Val Gly Leu Ser Asp Glu Ala Arg Gln Val Ala Ser Val Val Leu
145                 150                 155                 160

Val Val Glu Pro Ala Pro Val Pro Thr Pro Thr Pro Asp Asp Tyr Asp
                165                 170                 175

Glu Glu Asp Asp Ala Gly Val Ser Glu Arg Thr Pro Val Ser Val Pro
            180                 185                 190

Pro Pro Thr Pro Pro Arg Arg Pro Pro Val Ala Pro Gln Thr His Pro
        195                 200                 205

Arg Val Ile Pro Glu Val Ser His Val Arg Gly Val Thr Val His Met
    210                 215                 220

Glu Thr Pro Glu Ala Ile Leu Phe Ala Pro Gly Glu Thr Phe Gly Thr
225                 230                 235                 240

Asn Val Ser Ile His Ala Ile Ala His Asp Asp Gly Pro Tyr Ala Met
                245                 250                 255

Asp Val Val Trp Met Arg Phe Asp Val Pro Ser Ser Cys Ala Glu Met
            260                 265                 270

Arg Ile Tyr Glu Ala Cys Leu Tyr His Pro Gln Leu Pro Glu Cys Leu
        275                 280                 285

Ser Pro Ala Asp Ala Pro Cys Ala Val Ser Ser Trp Ala Tyr Arg Leu
    290                 295                 300

Ala Val Arg Ser Tyr Ala Gly Cys Ser Arg Thr Thr Pro Pro Pro Arg
305                 310                 315                 320

Cys Phe Ala Glu Ala Arg Met Glu Pro Val Pro Gly Leu Ala Trp Leu
                325                 330                 335

Ala Ser Thr Val Asn Leu Glu Phe Gln His Ala Ser Pro Gln His Ala
            340                 345                 350
```

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro His Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr Ser
530 535 540

Ser Val Leu Trp
545

<210> 54
<211> 548
<212> PRT
<213> herpes simplex virus 2

<400> 54

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Thr His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

Val Ile Tyr Gly Ala Leu Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Ala Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro His Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg
435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Ile Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr Ser
    530                 535                 540

Ser Val Leu Trp
545

<210> 55
<211> 548
<212> PRT
<213> herpes simplex virus 2

<400> 55

Met Ala Arg Gly Ala Gly Leu Val Phe Phe Val Gly Val Trp Val Val
1               5                   10                  15

Ser Cys Leu Ala Ala Ala Pro Arg Thr Ser Trp Lys Arg Val Thr Ser
            20                  25                  30

Gly Glu Asp Val Val Leu Leu Pro Ala Pro Ala Gly Pro Glu Glu Arg
        35                  40                  45

Thr Arg Ala His Lys Leu Leu Trp Ala Ala Glu Pro Leu Asp Ala Cys
    50                  55                  60

Gly Pro Leu Arg Pro Ser Trp Val Ala Leu Trp Pro Pro Arg Arg Val
65                  70                  75                  80

Leu Glu Thr Val Val Asp Ala Ala Cys Met Arg Ala Pro Glu Pro Leu
                85                  90                  95

Ala Ile Ala Tyr Ser Pro Pro Phe Pro Ala Gly Asp Glu Gly Leu Tyr
            100                 105                 110

Ser Glu Leu Ala Trp Arg Asp Arg Val Ala Val Val Asn Glu Ser Leu
        115                 120                 125

Val Ile Tyr Gly Ala Leu Glu Thr Asp Ser Gly Leu Tyr Thr Leu Ser
    130                 135                 140

Val Val Gly Leu Ser Asp Glu Ala Arg Gln Val Ala Ser Val Val Leu
145                 150                 155                 160

Val Val Glu Pro Ala Pro Val Pro Thr Pro Thr Pro Asp Asp Tyr Asp
                165                 170                 175

Glu Glu Asp Asp Ala Gly Val Ser Glu Arg Thr Pro Val Ser Val Pro
            180                 185                 190

Pro Pro Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro His Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg

435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr Ser
530 535 540

Ser Val Leu Trp
545

<210> 56
<211> 548
<212> PRT
<213> herpes simplex virus 2

<400> 56

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr

100 105 110

Ser Glu Leu Ala Trp Arg Asp Arg Val Ala Val Val Asn Glu Ser Leu
115 120 125

Val Ile Tyr Gly Ala Leu Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Pro Thr Pro Pro Arg Arg Pro Pro Val Ala Pro Pro Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

```
Gly Leu Tyr Leu Cys Val Val Tyr Val Asp Asp His Ile His Ala Trp
        355                 360                 365

Gly His Met Thr Ile Ser Thr Ala Ala Gln Tyr Arg Asn Ala Val Val
    370                 375                 380

Glu Gln His Leu Pro Gln Arg Gln Pro Glu Pro Val Glu Pro Thr Arg
385                 390                 395                 400

Pro His Val Arg Ala Pro His Pro Ala Pro Ser Ala Arg Gly Pro Leu
                405                 410                 415

Arg Leu Gly Ala Val Leu Gly Ala Ala Leu Leu Leu Ala Ala Leu Gly
            420                 425                 430

Leu Ser Ala Trp Ala Cys Met Thr Cys Trp Arg Arg Arg Ser Trp Arg
        435                 440                 445

Ala Val Lys Ser Arg Ala Ser Ala Thr Gly Pro Thr Tyr Ile Arg Val
    450                 455                 460

Ala Asp Ser Glu Leu Tyr Ala Asp Trp Ser Ser Asp Ser Glu Gly Glu
465                 470                 475                 480

Arg Asp Gly Ser Leu Trp Gln Asp Pro Pro Glu Arg Pro Asp Ser Pro
                485                 490                 495

Ser Thr Asn Gly Ser Gly Phe Glu Ile Leu Ser Pro Thr Ala Pro Ser
            500                 505                 510

Val Tyr Pro His Ser Glu Gly Arg Lys Ser Arg Arg Pro Leu Thr Thr
        515                 520                 525

Phe Gly Ser Gly Ser Pro Gly Arg Arg His Ser Gln Ala Ser Tyr Ser
    530                 535                 540

Ser Val Leu Trp
545


<210>  57
<211>  548
<212>  PRT
<213>  herpes simplex virus 2

<400>  57

Met Ala Arg Gly Ala Gly Leu Val Phe Phe Val Gly Val Trp Val Val
1               5                   10                  15
```

```
Ser Cys Leu Ala Ala Ala Pro Arg Thr Ser Trp Lys Arg Val Thr Ser
            20                  25                  30

Gly Glu Asp Val Val Leu Leu Pro Ala Pro Ala Gly Pro Glu Glu Arg
        35                  40                  45

Thr Arg Ala His Lys Leu Leu Trp Ala Ala Glu Pro Leu Asp Ala Cys
    50                  55                  60

Gly Pro Leu Arg Pro Ser Trp Val Ala Leu Trp Pro Pro Arg Arg Val
65                  70                  75                  80

Leu Glu Thr Val Val Asp Ala Ala Cys Met Arg Ala Pro Glu Pro Leu
                85                  90                  95

Ala Ile Ala Tyr Ser Pro Pro Phe Pro Ala Gly Asp Glu Gly Leu Tyr
            100                 105                 110

Ser Glu Leu Ala Trp Arg Asp Arg Val Ala Val Val Asn Glu Ser Leu
        115                 120                 125

Val Ile Tyr Gly Ala Leu Glu Thr Asp Ser Gly Leu Tyr Thr Leu Ser
    130                 135                 140

Val Val Gly Leu Ser Asp Glu Ala Arg Gln Val Ala Ser Val Val Leu
145                 150                 155                 160

Val Val Glu Pro Ala Pro Val Pro Thr Pro Thr Pro Asp Asp Tyr Asp
                165                 170                 175

Glu Glu Asp Asp Ala Gly Val Ser Glu Arg Thr Pro Val Ser Val Pro
            180                 185                 190

Pro Pro Thr Pro Pro Arg Arg Pro Pro Val Ala Pro Pro Thr His Pro
        195                 200                 205

Arg Val Ile Pro Glu Val Ser His Val Arg Gly Val Thr Val His Met
    210                 215                 220

Glu Thr Pro Glu Ala Ile Leu Phe Ala Pro Gly Glu Thr Phe Gly Thr
225                 230                 235                 240

Asn Val Ser Ile His Ala Ile Ala His Asp Asp Gly Pro Tyr Ala Met
                245                 250                 255

Asp Val Val Trp Met Arg Phe Asp Val Pro Ser Ser Cys Ala Glu Met
            260                 265                 270
```

Arg Ile Tyr Glu Ala Cys Leu Tyr His Pro Gln Leu Pro Glu Cys Leu
        275                 280                 285

Ser Pro Ala Asp Ala Pro Cys Ala Val Ser Ser Trp Ala Tyr Arg Leu
    290                 295                 300

Ala Val Arg Ser Tyr Ala Gly Cys Ser Arg Thr Thr Pro Pro Pro Arg
305                 310                 315                 320

Cys Phe Ala Glu Ala Arg Met Glu Pro Val Pro Gly Leu Ala Trp Leu
                325                 330                 335

Ala Ser Thr Val Asn Leu Glu Phe Gln His Ala Ser Pro Gln His Ala
            340                 345                 350

Gly Leu Tyr Leu Cys Val Val Tyr Val Asp Asp His Ile His Ala Trp
        355                 360                 365

Gly His Met Thr Ile Ser Thr Ala Ala Gln Tyr Arg Asn Ala Val Val
    370                 375                 380

Glu Gln His Leu Pro Gln Arg Gln Pro Glu Pro Val Glu Pro Thr Arg
385                 390                 395                 400

Pro His Val Arg Ala Pro His Pro Ala Pro Ser Ala Arg Gly Pro Leu
                405                 410                 415

Arg Leu Gly Ala Val Leu Gly Ala Ala Leu Leu Leu Ala Ala Leu Gly
            420                 425                 430

Leu Ser Ala Trp Ala Cys Met Thr Cys Trp Arg Arg Arg Ser Trp Arg
        435                 440                 445

Ala Val Lys Ser Arg Ala Ser Ala Thr Gly Pro Thr Tyr Ile Arg Val
    450                 455                 460

Ala Asp Ser Glu Leu Tyr Ala Asp Trp Ser Ser Asp Ser Glu Gly Glu
465                 470                 475                 480

Arg Asp Gly Ser Leu Trp Gln Asp Pro Pro Glu Arg Pro Asp Ser Pro
                485                 490                 495

Ser Thr Asn Gly Ser Gly Phe Glu Ile Leu Ser Pro Thr Ala Pro Ser
            500                 505                 510

Val Tyr Pro His Ser Glu Gly Arg Lys Ser Arg Arg Pro Leu Thr Thr
        515                 520                 525

Phe Gly Ser Gly Ser Pro Gly Arg Arg His Ser Gln Ala Ser Tyr Ser
530 535 540

Ser Val Leu Trp
545

<210> 58
<211> 548
<212> PRT
<213> herpes simplex virus 2

<400> 58

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Thr Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Cys Arg Asp Arg Val Ala Val Val Asn Glu Ser Leu
115 120 125

Val Ile Tyr Gly Ala Arg Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Pro Thr Pro Pro Arg Arg Pro Pro Val Ala Pro Gln Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

Gly Leu Tyr Leu Cys Val Val Tyr Val Asp Asp His Ile His Ala Trp
355 360 365

Gly His Met Thr Ile Ser Thr Ala Ala Gln Tyr Arg Asn Ala Val Val
370 375 380

Glu Gln His Leu Pro Gln Arg Gln Pro Glu Pro Val Glu Pro Thr Arg
385 390 395 400

Pro His Val Arg Ala Pro His Pro Ala Pro Ser Ala Arg Gly Pro Leu
405 410 415

Arg Leu Gly Ala Val Leu Gly Ala Ala Leu Leu Leu Ala Ala Leu Gly
420 425 430

Leu Ser Ala Trp Ala Cys Met Thr Cys Trp Arg Arg Arg Ser Trp Arg

            435                 440                 445

Ala Val Lys Ser Arg Ala Ser Ala Thr Gly Pro Thr Tyr Ile Arg Val
    450                 455                 460

Ala Asp Ser Glu Leu Tyr Ala Asp Trp Ser Ser Asp Ser Glu Gly Glu
465                 470                 475                 480

Arg Asp Gly Ser Leu Trp Gln Asp Pro Pro Glu Arg Pro Asp Ser Pro
                485                 490                 495

Ser Thr Asn Gly Ser Gly Phe Glu Ile Leu Ser Pro Thr Ala Pro Ser
            500                 505                 510

Val Tyr Pro His Ser Glu Gly Arg Lys Ser Arg Arg Pro Leu Thr Thr
        515                 520                 525

Phe Gly Ser Gly Ser Pro Gly Arg Arg His Ser Gln Ala Ser Tyr Ser
    530                 535                 540

Ser Val Leu Trp
545

<210>  59
<211>  548
<212>  PRT
<213>  herpes simplex virus 2

<400>  59

Met Ala Arg Gly Ala Gly Leu Val Phe Phe Val Gly Val Trp Val Val
1               5                   10                  15

Ser Cys Leu Ala Ala Ala Pro Arg Thr Ser Trp Lys Arg Val Thr Ser
            20                  25                  30

Gly Glu Asp Val Val Leu Leu Pro Ala Pro Thr Gly Pro Glu Glu Arg
        35                  40                  45

Thr Arg Ala His Lys Leu Leu Trp Ala Ala Glu Pro Leu Asp Ala Cys
    50                  55                  60

Gly Pro Leu Arg Pro Ser Trp Val Ala Leu Trp Pro Pro Arg Arg Val
65                  70                  75                  80

Leu Glu Thr Val Val Asp Ala Ala Cys Met Arg Ala Pro Glu Pro Leu
                85                  90                  95

Ala Ile Ala Tyr Ser Pro Pro Phe Pro Ala Gly Asp Glu Gly Leu Tyr

100 105 110

Ser Glu Leu Ala Cys Arg Asp Arg Val Ala Val Val Asn Glu Ser Leu
115 120 125

Val Ile Tyr Gly Ala Arg Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Pro Thr Pro Pro Arg Arg Pro Pro Val Ala Pro Gln Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

Arg Ile Tyr Glu Ala Cys Leu Tyr His Pro Gln Leu Pro Glu Cys Leu
275 280 285

Ser Pro Ala Asp Ala Pro Cys Ala Val Ser Ser Trp Ala Tyr Arg Leu
290 295 300

Ala Val Arg Ser Tyr Ala Gly Cys Ser Arg Thr Thr Pro Pro Pro Arg
305 310 315 320

Cys Phe Ala Glu Ala Arg Met Glu Pro Val Pro Gly Leu Ala Trp Leu
325 330 335

Ala Ser Thr Val Asn Leu Glu Phe Gln His Ala Ser Pro Gln His Ala
340 345 350

```
Gly Leu Tyr Leu Cys Val Val Tyr Val Asp Asp His Ile His Ala Trp
        355                 360                 365

Gly His Met Thr Ile Ser Thr Ala Ala Gln Tyr Arg Asn Ala Val Val
    370                 375                 380

Glu Gln His Leu Pro Gln Arg Gln Pro Glu Pro Val Glu Pro Thr Arg
385                 390                 395                 400

Pro His Val Arg Ala Pro His Pro Ala Pro Ser Ala Arg Gly Pro Leu
                405                 410                 415

Arg Leu Gly Ala Val Leu Gly Ala Ala Leu Leu Leu Ala Ala Leu Gly
            420                 425                 430

Leu Ser Ala Trp Ala Cys Met Thr Cys Trp Arg Arg Arg Ser Trp Arg
        435                 440                 445

Ala Val Lys Ser Arg Ala Ser Ala Thr Gly Pro Thr Tyr Ile Arg Val
    450                 455                 460

Ala Asp Ser Glu Leu Tyr Ala Asp Trp Ser Ser Asp Ser Glu Gly Glu
465                 470                 475                 480

Arg Asp Gly Ser Leu Trp Gln Asp Pro Pro Glu Arg Pro Asp Ser Pro
                485                 490                 495

Ser Thr Asn Gly Ser Gly Phe Glu Ile Leu Ser Pro Thr Ala Pro Ser
            500                 505                 510

Val Tyr Pro His Ser Glu Gly Arg Lys Ser Arg Arg Pro Leu Thr Thr
        515                 520                 525

Phe Gly Ser Gly Ser Pro Gly Arg Arg His Ser Gln Ala Ser Tyr Ser
    530                 535                 540

Ser Val Leu Trp
545


<210>  60
<211>  548
<212>  PRT
<213>  herpes simplex virus 2

<400>  60

Met Ala Arg Gly Ala Gly Leu Val Phe Phe Val Gly Val Trp Val Val
1               5                   10                  15
```

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

Val Ile Tyr Gly Ala Arg Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Pro Thr Pro Pro Arg Arg Pro Pro Val Ala Pro Gln Thr His Pro
195 200 205

Arg Val Ile Pro Glu Val Ser His Val Arg Gly Val Thr Val His Met
210 215 220

Glu Thr Pro Glu Ala Ile Leu Phe Ala Pro Gly Glu Thr Phe Gly Thr
225 230 235 240

Asn Val Ser Ile His Ala Ile Ala His Asp Asp Gly Pro Tyr Ala Met
245 250 255

Asp Val Val Trp Met Arg Phe Asp Val Pro Ser Ser Cys Ala Glu Met
260 265 270

```
Arg Ile Tyr Glu Ala Cys Leu Tyr His Pro Gln Leu Pro Glu Cys Leu
        275                 280                 285

Ser Pro Ala Asp Ala Pro Cys Ala Val Ser Ser Trp Ala Tyr Arg Leu
    290                 295                 300

Ala Val Arg Ser Tyr Ala Gly Cys Ser Arg Thr Thr Pro Pro Pro Arg
305                 310                 315                 320

Cys Phe Ala Glu Ala Arg Met Glu Pro Val Pro Gly Leu Ala Trp Leu
                325                 330                 335

Ala Ser Thr Val Asn Leu Glu Phe Gln His Ala Ser Pro Gln His Ala
            340                 345                 350

Gly Leu Tyr Leu Cys Val Val Tyr Val Asp Asp His Ile His Ala Trp
        355                 360                 365

Gly His Met Thr Ile Ser Thr Ala Ala Gln Tyr Arg Asn Ala Val Val
    370                 375                 380

Glu Gln His Leu Pro Gln Arg Gln Pro Glu Pro Val Glu Pro Thr Arg
385                 390                 395                 400

Pro His Val Arg Ala Pro His Pro Ala Pro Ser Ala Arg Gly Pro Leu
                405                 410                 415

Arg Leu Gly Ala Val Leu Gly Ala Ala Leu Leu Leu Ala Ala Leu Gly
            420                 425                 430

Leu Ser Ala Trp Ala Cys Met Thr Cys Trp Arg Arg Arg Ser Trp Arg
        435                 440                 445

Ala Val Lys Ser Arg Ala Ser Ala Thr Gly Pro Thr Tyr Ile Arg Val
    450                 455                 460

Ala Asp Ser Glu Leu Tyr Ala Asp Trp Ser Ser Asp Ser Glu Gly Glu
465                 470                 475                 480

Arg Asp Gly Ser Leu Trp Gln Asp Pro Pro Glu Arg Pro Asp Ser Pro
                485                 490                 495

Ser Thr Asn Gly Ser Gly Phe Glu Ile Leu Ser Pro Thr Ala Pro Ser
            500                 505                 510

Val Tyr Pro His Ser Glu Gly Arg Lys Ser Arg Arg Pro Leu Thr Thr
        515                 520                 525
```

Phe Gly Ser Gly Ser Pro Gly Arg Arg His Ser Gln Ala Ser Tyr Ser
530 535 540

Ser Val Leu Trp
545

<210> 61
<211> 548
<212> PRT
<213> herpes simplex virus 2

<400> 61

Met Ala Arg Gly Ala Gly Leu Val Phe Phe Val Gly Val Trp Val Val
1 5 10 15

Ser Cys Leu Ala Ala Ala Pro Arg Thr Ser Trp Lys Arg Val Thr Ser
20 25 30

Gly Glu Asp Val Val Leu Leu Pro Ala Pro Ala Gly Pro Glu Glu Arg
35 40 45

Thr Arg Ala His Lys Leu Leu Trp Ala Ala Glu Pro Leu Asp Ala Cys
50 55 60

Gly Pro Leu Arg Pro Ser Trp Val Ala Leu Trp Pro Pro Arg Arg Val
65 70 75 80

Leu Glu Thr Val Val Asp Ala Ala Cys Met Arg Ala Pro Glu Pro Leu
85 90 95

Ala Ile Ala Tyr Ser Pro Pro Phe Pro Ala Gly Asp Glu Gly Leu Tyr
100 105 110

Ser Glu Leu Ala Trp Arg Asp Arg Val Ala Val Val Asn Glu Ser Leu
115 120 125

Val Ile Tyr Gly Ala Arg Glu Thr Asp Ser Gly Leu Tyr Thr Leu Ser
130 135 140

Val Val Gly Leu Ser Asp Glu Ala Arg Gln Val Ala Ser Val Val Leu
145 150 155 160

Val Val Glu Pro Ala Pro Val Pro Thr Pro Thr Pro Asp Asp Tyr Asp
165 170 175

Glu Glu Asp Asp Ala Gly Val Ser Glu Arg Thr Pro Val Ser Val Pro
180 185 190

Pro Thr Thr Pro Pro Arg Arg Pro Pro Val Ala Pro Pro Thr His Pro
        195                 200                 205

Arg Val Ile Pro Glu Val Ser His Val Arg Gly Val Thr Val His Met
    210                 215                 220

Glu Thr Pro Glu Ala Ile Leu Phe Ala Pro Gly Glu Thr Phe Gly Thr
225                 230                 235                 240

Asn Val Ser Ile His Ala Ile Ala His Asp Asp Gly Pro Tyr Ala Met
                245                 250                 255

Asp Val Val Trp Met Arg Phe Asp Val Pro Ser Ser Cys Ala Glu Met
            260                 265                 270

Arg Ile Tyr Glu Ala Cys Leu Tyr His Pro Gln Leu Pro Glu Cys Leu
        275                 280                 285

Ser Pro Ala Asp Ala Pro Cys Ala Val Ser Ser Trp Ala Tyr Arg Leu
    290                 295                 300

Ala Val Arg Ser Tyr Ala Gly Cys Ser Arg Thr Thr Pro Pro Pro Arg
305                 310                 315                 320

Cys Phe Ala Glu Ala Arg Met Glu Pro Val Pro Gly Leu Ala Trp Leu
                325                 330                 335

Ala Ser Thr Val Asn Leu Glu Phe Gln His Ala Ser Pro Gln His Ala
            340                 345                 350

Gly Leu Tyr Leu Cys Val Val Tyr Val Asp Asp His Ile His Ala Trp
        355                 360                 365

Gly His Met Thr Ile Ser Thr Ala Ala Gln Tyr Arg Asn Ala Val Val
    370                 375                 380

Glu Gln His Leu Pro Gln Arg Gln Pro Glu Pro Val Glu Pro Thr Arg
385                 390                 395                 400

Pro His Val Arg Ala Pro His Pro Ala Pro Ser Ala Arg Gly Pro Leu
                405                 410                 415

Arg Leu Gly Ala Val Leu Gly Ala Ala Leu Leu Leu Ala Ala Leu Gly
            420                 425                 430

Leu Ser Ala Trp Ala Cys Met Thr Cys Trp Arg Arg Arg Ser Trp Arg

435 440 445

Ala Val Lys Ser Arg Ala Ser Ala Thr Gly Pro Thr Tyr Ile Arg Val
450 455 460

Ala Asp Ser Glu Leu Tyr Ala Asp Trp Ser Ser Asp Ser Glu Gly Glu
465 470 475 480

Arg Asp Gly Ser Leu Trp Gln Asp Pro Pro Glu Arg Pro Asp Ser Pro
485 490 495

Ser Thr Asn Gly Ser Gly Phe Glu Ile Leu Ser Pro Thr Ala Pro Ser
500 505 510

Val Tyr Pro His Ser Glu Gly Arg Lys Ser Arg Arg Pro Leu Thr Thr
515 520 525

Phe Gly Ser Gly Ser Pro Gly Arg Arg His Ser Gln Ala Ser Tyr Ser
530 535 540

Ser Val Leu Trp
545

<210> 62
<211> 372
<212> PRT
<213> herpes simplex virus 2

<400> 62

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His

100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Leu Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Ser Pro Ser Ser Pro Arg Asp Pro Thr Pro
210 215 220

Ala Pro Gly Asp Thr Gly Thr Pro Ala Pro Ala Ser Gly Glu Arg Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 63
<211> 372
<212> PRT
<213> herpes simplex virus 2

<400> 63

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Leu Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
        195                 200                 205

Pro Pro Arg Thr Thr Thr Ser Pro Ser Ser Pro Arg Asp Pro Thr Pro
    210                 215                 220

Ala Pro Gly Asp Thr Gly Thr Pro Ala Pro Ala Ser Gly Glu Arg Ala
225                 230                 235                 240

Pro Pro Asn Ser Thr Arg Ser Ala Ser Glu Ser Arg His Arg Leu Thr
                245                 250                 255

Val Ala Gln Val Ile Gln Ile Ala Ile Pro Ala Ser Ile Ile Ala Phe
            260                 265                 270

Val Phe Leu Gly Ser Cys Ile Cys Phe Ile His Arg Cys Gln Arg Arg
        275                 280                 285

Tyr Arg Arg Pro Arg Gly Gln Ile Tyr Asn Pro Gly Gly Val Ser Cys
    290                 295                 300

Ala Val Asn Glu Ala Ala Met Ala Arg Leu Gly Ala Glu Leu Arg Ser
305                 310                 315                 320

His Pro Asn Thr Pro Pro Lys Pro Arg Arg Arg Ser Ser Ser Ser Thr
                325                 330                 335

Thr Met Pro Ser Leu Thr Ser Ile Ala Glu Glu Ser Glu Pro Gly Pro
            340                 345                 350

Val Val Leu Leu Ser Val Ser Pro Arg Pro Arg Ser Gly Pro Thr Ala
        355                 360                 365

Pro Gln Glu Val
    370

<210> 64
<211> 372
<212> PRT
<213> herpes simplex virus 2

<400> 64

Met Pro Gly Arg Ser Leu Gln Gly Leu Ala Ile Leu Gly Leu Trp Val
1               5                   10                  15

Cys Ala Thr Gly Leu Val Val Arg Gly Pro Thr Val Ser Leu Val Ser
            20                  25                  30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Leu Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Ser Pro Ser Ser Pro Arg Asp Pro Thr Pro
210 215 220

Ala Pro Gly Asp Thr Gly Thr Pro Ala Pro Ala Ser Gly Glu Arg Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 65
<211> 372
<212> PRT
<213> herpes simplex virus 2

<400> 65

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
    130                 135                 140

Tyr Val Leu Arg Val Trp Val Gly Ser Ala Thr Asn Ala Ser Leu Phe
145                 150                 155                 160

Val Leu Gly Val Ala Leu Ser Ala Asn Gly Thr Phe Val Tyr Asn Gly
                165                 170                 175

Ser Asp Tyr Gly Ser Cys Asp Pro Ala Gln Leu Pro Phe Ser Ala Pro
            180                 185                 190

Arg Leu Gly Pro Ser Ser Val Tyr Thr Pro Gly Ala Ser Arg Pro Thr
        195                 200                 205

Pro Pro Arg Thr Thr Thr Ser Pro Ser Ser Pro Arg Asp Pro Thr Pro
    210                 215                 220

Ala Pro Gly Asp Thr Gly Thr Pro Ala Pro Ala Ser Gly Glu Arg Ala
225                 230                 235                 240

Pro Pro Asn Ser Thr Arg Ser Ala Ser Glu Ser Arg His Arg Leu Thr
                245                 250                 255

Val Ala Gln Val Ile Gln Ile Ala Ile Pro Ala Ser Ile Ile Ala Phe
            260                 265                 270

Val Phe Leu Gly Ser Cys Ile Cys Phe Ile His Arg Cys Gln Arg Arg
        275                 280                 285

Tyr Arg Arg Pro Arg Gly Gln Ile Tyr Asn Pro Gly Gly Val Ser Cys
    290                 295                 300

Ala Val Asn Glu Ala Ala Met Ala Arg Leu Gly Ala Glu Leu Arg Ser
305                 310                 315                 320

His Pro Asn Thr Pro Pro Lys Pro Arg Arg Arg Ser Ser Ser Ser Thr
                325                 330                 335

Thr Met Pro Ser Leu Thr Ser Ile Ala Glu Glu Ser Glu Pro Gly Pro
            340                 345                 350

Val Val Leu Leu Ser Val Ser Pro Arg Pro Arg Ser Gly Pro Thr Ala
        355                 360                 365

Pro Gln Glu Val
    370

<210> 66
<211> 372
<212> PRT
<213> herpes simplex virus 2

<400> 66

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Leu Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Ser Pro Ser Ser Pro Arg Asp Pro Thr Pro
210 215 220

Ala Pro Gly Asp Thr Gly Thr Pro Ala Pro Ala Ser Gly Glu Arg Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 67
<211> 372
<212> PRT
<213> herpes simplex virus 2

<400> 67

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

```
Pro His Thr Asn Tyr Tyr Asp Gly Ile Ile Glu Leu Phe His Tyr Pro
65                  70                  75                  80

Leu Gly Asn His Cys Pro Arg Val Val His Val Val Thr Leu Thr Ala
                85                  90                  95

Cys Pro Arg Arg Pro Ala Val Ala Phe Thr Leu Cys Arg Ser Thr His
            100                 105                 110

His Ala His Ser Pro Ala Tyr Pro Thr Leu Glu Leu Gly Leu Ala Arg
        115                 120                 125

Gln Pro Leu Leu Arg Val Arg Thr Ala Thr Arg Asp Tyr Ala Gly Leu
    130                 135                 140

Tyr Val Leu Arg Val Trp Val Gly Ser Ala Thr Asn Ala Ser Leu Phe
145                 150                 155                 160

Val Leu Gly Val Ala Leu Ser Ala Asn Gly Thr Phe Val Tyr Asn Gly
                165                 170                 175

Ser Asp Tyr Gly Ser Cys Asp Pro Ala Gln Leu Pro Phe Ser Ala Pro
            180                 185                 190

Arg Leu Gly Pro Ser Ser Val Tyr Thr Pro Gly Ala Ser Arg Pro Thr
        195                 200                 205

Pro Pro Arg Thr Thr Thr Ser Pro Ser Ser Pro Arg Asp Pro Thr Pro
    210                 215                 220

Ala Pro Gly Asp Thr Gly Thr Pro Ala Pro Ala Ser Gly Glu Arg Ala
225                 230                 235                 240

Pro Pro Asn Ser Thr Arg Ser Ala Ser Glu Ser Arg His Arg Leu Thr
                245                 250                 255

Val Ala Gln Val Ile Gln Ile Ala Ile Pro Ala Ser Ile Ile Ala Phe
            260                 265                 270

Val Phe Leu Gly Ser Cys Ile Cys Phe Ile His Arg Cys Gln Arg Arg
        275                 280                 285

Tyr Arg Arg Pro Arg Gly Gln Ile Tyr Asn Pro Gly Gly Val Ser Cys
    290                 295                 300

Ala Val Asn Glu Ala Ala Met Ala Arg Leu Gly Ala Glu Leu Arg Ser
```

305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 68
<211> 372
<212> PRT
<213> herpes simplex virus 2

<400> 68

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Leu Phe

```
145                 150                 155                 160

Val Leu Gly Val Ala Leu Ser Ala Asn Gly Thr Phe Val Tyr Asn Gly
                165                 170                 175

Ser Asp Tyr Gly Ser Cys Asp Pro Ala Gln Leu Pro Phe Ser Ala Pro
            180                 185                 190

Arg Leu Gly Pro Ser Ser Val Tyr Thr Pro Gly Ala Ser Arg Pro Thr
        195                 200                 205

Pro Pro Arg Thr Thr Thr Ser Pro Ser Ser Pro Arg Asp Pro Thr Pro
    210                 215                 220

Ala Pro Gly Asp Thr Gly Thr Pro Ala Pro Ala Ser Gly Glu Arg Ala
225                 230                 235                 240

Pro Pro Asn Ser Thr Arg Ser Ala Ser Glu Ser Arg His Arg Leu Thr
                245                 250                 255

Val Ala Gln Val Ile Gln Ile Ala Ile Pro Ala Ser Ile Ile Ala Phe
            260                 265                 270

Val Phe Leu Gly Ser Cys Ile Cys Phe Ile His Arg Cys Gln Arg Arg
        275                 280                 285

Tyr Arg Arg Pro Arg Gly Gln Ile Tyr Asn Pro Gly Gly Val Ser Cys
    290                 295                 300

Ala Val Asn Glu Ala Ala Met Ala Arg Leu Gly Ala Glu Leu Arg Ser
305                 310                 315                 320

His Pro Asn Thr Pro Pro Lys Pro Arg Arg Arg Ser Ser Ser Ser Thr
                325                 330                 335

Thr Met Pro Ser Leu Thr Ser Ile Ala Glu Glu Ser Glu Pro Gly Pro
            340                 345                 350

Val Val Leu Leu Ser Val Ser Pro Arg Pro Arg Ser Gly Pro Thr Ala
        355                 360                 365

Pro Gln Glu Val
    370


<210>  69
<211>  372
<212>  PRT
<213>  herpes simplex virus 2
```

<400> 69

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Leu Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Ser Pro Ser Ser Pro Arg Asp Pro Thr Pro
210 215 220

Ala Pro Gly Asp Thr Gly Thr Pro Ala Pro Ala Ser Gly Glu Arg Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 70
<211> 372
<212> PRT
<213> herpes simplex virus 2

<400> 70

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Leu Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Ser Pro Ser Ser Pro Arg Asp Pro Thr Pro
210 215 220

Ala Pro Gly Asp Thr Gly Thr Pro Ala Pro Ala Ser Gly Glu Arg Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 71
<211> 372
<212> PRT
<213> herpes simplex virus 2

<400> 71

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Leu Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Pro Pro Ser Ser Pro Arg Asp Pro Thr Pro
210 215 220

Ala Pro Gly Asp Thr Gly Thr Pro Ala Pro Ala Ser Gly Glu Arg Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 72
<211> 372
<212> PRT
<213> herpes simplex virus 2

<400> 72

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Leu Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Pro Pro Ser Ser Pro Arg Asp Pro Thr Pro
210 215 220

Ala Pro Gly Asp Thr Gly Thr Pro Ala Pro Ala Ser Gly Glu Arg Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

```
Val Phe Leu Gly Ser Cys Ile Cys Phe Ile His Arg Cys Gln Arg Arg
        275                 280                 285

Tyr Arg Arg Pro Arg Gly Gln Ile Tyr Asn Pro Gly Gly Val Ser Cys
    290                 295                 300

Ala Val Asn Glu Ala Ala Met Ala Arg Leu Gly Ala Glu Leu Arg Ser
305                 310                 315                 320

His Pro Asn Thr Pro Pro Lys Pro Arg Arg Arg Ser Ser Ser Ser Thr
                325                 330                 335

Thr Met Pro Ser Leu Thr Ser Ile Ala Glu Glu Ser Glu Pro Gly Pro
            340                 345                 350

Val Val Leu Leu Ser Val Ser Pro Arg Pro Arg Ser Gly Pro Thr Ala
        355                 360                 365

Pro Gln Glu Val
    370


<210>  73
<211>  372
<212>  PRT
<213>  herpes simplex virus 2

<400>  73

Met Pro Gly Arg Ser Leu Gln Gly Leu Ala Ile Leu Gly Leu Trp Val
1               5                   10                  15

Cys Ala Thr Gly Leu Val Val Arg Gly Pro Thr Val Ser Leu Val Ser
            20                  25                  30

Asp Ser Leu Val Asp Ala Gly Ala Val Gly Pro Gln Gly Phe Val Glu
        35                  40                  45

Glu Asp Leu Arg Val Phe Gly Glu Leu His Phe Val Gly Ala Gln Val
    50                  55                  60

Pro His Thr Asn Tyr Tyr Asp Gly Ile Ile Glu Leu Phe His Tyr Pro
65                  70                  75                  80

Leu Gly Asn His Cys Pro Arg Val Val His Val Val Thr Leu Thr Ala
                85                  90                  95

Cys Pro Arg Arg Pro Ala Val Ala Phe Thr Leu Cys Arg Ser Thr His
            100                 105                 110
```

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Arg Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Ser Pro Ser Ser Pro Arg Asp Pro Thr Pro
210 215 220

Ala Pro Gly Asp Thr Gly Thr Pro Ala Pro Ala Ser Gly Glu Arg Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala

355 360 365

Pro Gln Glu Val
370

<210> 74
<211> 372
<212> PRT
<213> herpes simplex virus 2

<400> 74

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Arg Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr

```
        195                 200                 205

Pro Pro Arg Thr Thr Thr Ser Pro Ser Ser Pro Arg Asp Pro Thr Pro
    210                 215                 220

Ala Pro Gly Asp Thr Gly Thr Pro Ala Pro Ala Ser Gly Glu Arg Ala
225                 230                 235                 240

Pro Pro Asn Ser Thr Arg Ser Ala Ser Glu Ser Arg His Arg Leu Thr
                245                 250                 255

Val Ala Gln Val Ile Gln Ile Ala Ile Pro Ala Ser Ile Ile Ala Phe
            260                 265                 270

Val Phe Leu Gly Ser Cys Ile Cys Phe Ile His Arg Cys Gln Arg Arg
        275                 280                 285

Tyr Arg Arg Pro Arg Gly Gln Ile Tyr Asn Pro Gly Gly Val Ser Cys
    290                 295                 300

Ala Val Asn Glu Ala Ala Met Ala Arg Leu Gly Ala Glu Leu Arg Ser
305                 310                 315                 320

His Pro Asn Thr Pro Pro Lys Pro Arg Arg Arg Ser Ser Ser Ser Thr
                325                 330                 335

Thr Met Pro Ser Leu Thr Ser Ile Ala Glu Glu Ser Glu Pro Gly Pro
            340                 345                 350

Val Val Leu Leu Ser Val Ser Pro Arg Pro Arg Ser Gly Pro Thr Ala
        355                 360                 365

Pro Gln Glu Val
    370


<210>  75
<211>  372
<212>  PRT
<213>  herpes simplex virus 2

<400>  75

Met Pro Gly Arg Ser Leu Gln Gly Leu Ala Ile Leu Gly Leu Trp Val
1               5                   10                  15

Cys Ala Thr Gly Leu Val Val Arg Gly Pro Thr Val Ser Leu Val Ser
            20                  25                  30

Asp Ser Leu Val Asp Ala Gly Ala Val Gly Pro Gln Gly Phe Val Glu
```

35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Leu Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Pro Pro Ser Ser Pro Arg Asp Pro Thr Pro
210 215 220

Ala Pro Gly Asp Thr Gly Thr Pro Ala Pro Ala Ser Gly Glu Arg Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
    290                 295                 300

Ala Val Asn Glu Ala Ala Met Ala Arg Leu Gly Ala Glu Leu Arg Ser
305                 310                 315                 320

His Pro Asn Thr Pro Pro Lys Pro Arg Arg Arg Ser Ser Ser Ser Thr
                325                 330                 335

Thr Met Pro Ser Leu Thr Ser Ile Ala Glu Glu Ser Glu Pro Gly Pro
            340                 345                 350

Val Val Leu Leu Ser Val Ser Pro Arg Pro Arg Ser Gly Pro Thr Ala
        355                 360                 365

Pro Gln Glu Val
    370

<210> 76
<211> 372
<212> PRT
<213> herpes simplex virus 2

<400> 76

Met Pro Gly Arg Ser Leu Gln Gly Leu Ala Ile Leu Gly Leu Trp Val
1               5                   10                  15

Cys Ala Thr Gly Leu Val Val Arg Gly Pro Thr Val Ser Leu Val Ser
            20                  25                  30

Asp Ser Leu Val Asp Ala Gly Ala Val Gly Pro Gln Gly Phe Val Glu
        35                  40                  45

Glu Asp Leu Arg Val Phe Gly Glu Leu His Phe Val Gly Ala Gln Val
    50                  55                  60

Pro His Thr Asn Tyr Tyr Asp Gly Ile Ile Glu Leu Phe His Tyr Pro
65                  70                  75                  80

Leu Gly Asn His Cys Pro Arg Val Val His Val Val Thr Leu Thr Ala
                85                  90                  95

Cys Pro Arg Arg Pro Ala Val Ala Phe Thr Leu Cys Arg Ser Thr His
            100                 105                 110

His Ala His Ser Pro Ala Tyr Pro Thr Leu Glu Leu Gly Leu Ala Arg
        115                 120                 125

```
Gln Pro Leu Leu Arg Val Arg Thr Ala Thr Arg Asp Tyr Ala Gly Leu
    130                 135                 140

Tyr Val Leu Arg Val Trp Val Gly Ser Ala Thr Asn Ala Ser Leu Phe
145                 150                 155                 160

Val Leu Gly Val Ala Leu Ser Ala Asn Gly Thr Phe Val Tyr Asn Gly
                165                 170                 175

Leu Asp Tyr Gly Ser Cys Asp Pro Ala Gln Leu Pro Phe Ser Ala Pro
            180                 185                 190

Arg Leu Gly Pro Ser Ser Val Tyr Thr Pro Gly Ala Ser Arg Pro Thr
        195                 200                 205

Pro Pro Arg Thr Thr Thr Ser Pro Ser Ser Pro Arg Asp Pro Thr Pro
    210                 215                 220

Ala Pro Gly Asp Thr Gly Thr Pro Ala Pro Ala Ser Gly Glu Arg Ala
225                 230                 235                 240

Pro Pro Asn Ser Thr Arg Ser Ala Ser Glu Ser Arg His Arg Leu Thr
                245                 250                 255

Val Ala Gln Val Ile Gln Ile Ala Ile Pro Ala Ser Ile Ile Ala Phe
            260                 265                 270

Val Phe Leu Gly Ser Cys Ile Cys Phe Ile His Arg Cys Gln Arg Arg
        275                 280                 285

Tyr Arg Arg Pro Arg Gly Gln Ile Tyr Asn Pro Gly Gly Val Ser Cys
    290                 295                 300

Ala Val Asn Glu Ala Ala Met Ala Arg Leu Gly Ala Glu Leu Arg Ser
305                 310                 315                 320

His Pro Asn Thr Pro Pro Lys Pro Arg Arg Arg Ser Ser Ser Ser Thr
                325                 330                 335

Thr Met Pro Ser Leu Thr Ser Ile Ala Glu Glu Ser Glu Pro Gly Pro
            340                 345                 350

Val Val Leu Leu Ser Val Ser Pro Arg Pro Arg Ser Gly Pro Thr Ala
        355                 360                 365

Pro Gln Glu Val
    370
```

<210> 77
<211> 372
<212> PRT
<213> herpes simplex virus 2

<400> 77

Met Pro Gly Arg Ser Leu Gln Gly Leu Ala Ile Leu Gly Leu Trp Val
1               5                   10                  15

Cys Ala Thr Gly Leu Val Val Arg Gly Pro Thr Val Ser Leu Val Ser
            20                  25                  30

Asp Ser Leu Val Asp Ala Gly Ala Val Gly Pro Gln Gly Phe Val Glu
        35                  40                  45

Glu Asp Leu Arg Val Phe Gly Glu Leu His Phe Val Gly Ala Gln Val
    50                  55                  60

Pro His Thr Asn Tyr Tyr Asp Gly Ile Ile Glu Leu Phe His Tyr Pro
65                  70                  75                  80

Leu Gly Asn His Cys Pro Arg Val Val His Val Val Thr Leu Thr Ala
                85                  90                  95

Cys Pro Arg Arg Pro Ala Val Ala Phe Thr Leu Cys Arg Ser Thr His
            100                 105                 110

His Ala His Ser Pro Ala Tyr Pro Thr Leu Glu Leu Gly Leu Ala Arg
        115                 120                 125

Gln Pro Leu Leu Arg Val Arg Thr Ala Thr Arg Asp Tyr Ala Gly Leu
    130                 135                 140

Tyr Val Leu Arg Val Trp Val Gly Ser Ala Thr Asn Ala Ser Leu Phe
145                 150                 155                 160

Val Leu Gly Val Ala Leu Ser Ala Asn Gly Thr Phe Val Tyr Asn Gly
                165                 170                 175

Ser Asp Tyr Gly Ser Cys Asp Pro Ala Gln Leu Pro Phe Ser Ala Pro
            180                 185                 190

Arg Leu Gly Pro Ser Ser Val Tyr Thr Pro Gly Ala Ser Arg Pro Thr
        195                 200                 205

Pro Pro Arg Thr Thr Thr Pro Pro Ser Ser Pro Arg Asp Pro Thr Pro
    210                 215                 220

Ala Pro Gly Asp Thr Gly Thr Pro Ala Pro Ala Ser Gly Glu Arg Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 78
<211> 372
<212> PRT
<213> herpes simplex virus 2

<400> 78

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Arg Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Ser Pro Ser Ser Pro Arg Asp Pro Thr Pro
210 215 220

Ala Pro Gly Asp Thr Gly Thr Pro Ala Pro Ala Ser Gly Glu Arg Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 79
<211> 372
<212> PRT
<213> herpes simplex virus 2

<400> 79

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Leu Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Pro Pro Ser Ser Pro Arg Asp Pro Thr Pro
210 215 220

Ala Pro Gly Asp Thr Gly Thr Pro Ala Pro Ala Ser Gly Glu Arg Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 80
<211> 372
<212> PRT
<213> herpes simplex virus 2

<400> 80

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Arg Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Ser Pro Ser Ser Pro Arg Asp Pro Thr Pro
210 215 220

Ala Pro Gly Asp Thr Gly Thr Pro Ala Pro Ala Ser Gly Glu Arg Ala
225 230 235 240

Pro Pro Asn Ser Thr Arg Ser Ala Ser Glu Ser Arg His Arg Leu Thr

245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 81
<211> 372
<212> PRT
<213> herpes simplex virus 2

<400> 81

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala

85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Arg Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Ser Pro Ser Ser Pro Arg Asp Pro Thr Pro
210 215 220

Ala Pro Gly Asp Thr Gly Thr Pro Ala Pro Ala Ser Gly Glu Arg Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 82
<211> 372
<212> PRT
<213> herpes simplex virus 2

<400> 82

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Leu Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Leu Pro Ser Ser Pro Arg Asp Pro Thr Pro
210 215 220

Ala Pro Gly Asp Thr Gly Thr Pro Ala Pro Ala Ser Gly Glu Arg Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 83
<211> 372
<212> PRT
<213> herpes simplex virus 2

<400> 83

Met Pro Gly Arg Ser Leu Gln Gly Leu Ala Ile Leu Gly Leu Trp Val
1 5 10 15

```
Cys Ala Thr Gly Leu Val Val Arg Gly Pro Thr Val Ser Leu Val Ser
            20                  25                  30

Asp Ser Leu Val Asp Ala Gly Ala Val Gly Pro Gln Gly Phe Val Glu
        35                  40                  45

Glu Asp Leu Arg Val Phe Gly Glu Leu His Phe Val Gly Ala Gln Val
    50                  55                  60

Pro His Thr Asn Tyr Tyr Asp Gly Ile Ile Glu Leu Phe His Tyr Pro
65                  70                  75                  80

Leu Gly Asn His Cys Pro Arg Val Val His Val Val Thr Leu Thr Ala
                85                  90                  95

Cys Pro Arg Arg Pro Ala Val Ala Phe Thr Leu Cys Arg Ser Thr His
            100                 105                 110

His Ala His Ser Pro Ala Tyr Pro Thr Leu Glu Leu Gly Leu Ala Arg
        115                 120                 125

Gln Pro Leu Leu Arg Val Arg Thr Ala Thr Arg Asp Tyr Ala Gly Leu
    130                 135                 140

Tyr Val Leu Arg Val Trp Val Gly Ser Ala Thr Asn Ala Ser Arg Phe
145                 150                 155                 160

Val Leu Gly Val Ala Leu Ser Ala Asn Gly Thr Phe Val Tyr Asn Gly
                165                 170                 175

Ser Asp Tyr Gly Ser Cys Asp Pro Ala Gln Leu Pro Phe Ser Ala Pro
            180                 185                 190

Arg Leu Gly Pro Ser Ser Val Tyr Thr Pro Gly Ala Ser Arg Pro Thr
        195                 200                 205

Pro Pro Arg Thr Thr Thr Ser Pro Ser Ser Pro Arg Asp Pro Thr Pro
    210                 215                 220

Ala Pro Gly Asp Thr Gly Thr Pro Ala Pro Ala Ser Gly Glu Arg Ala
225                 230                 235                 240

Pro Pro Asn Ser Thr Arg Ser Ala Ser Glu Ser Arg His Arg Leu Thr
                245                 250                 255

Val Ala Gln Val Ile Gln Ile Ala Ile Pro Ala Ser Ile Ile Ala Phe
            260                 265                 270
```

Val Phe Leu Gly Ser Cys Ile Cys Phe Ile His Arg Cys Gln Arg Arg
        275                 280                 285

Tyr Arg Arg Pro Arg Gly Gln Ile Tyr Asn Pro Gly Gly Val Ser Cys
    290                 295                 300

Ala Val Asn Glu Ala Ala Met Ala Arg Leu Gly Ala Glu Leu Arg Ser
305                 310                 315                 320

His Pro Asn Thr Pro Pro Lys Pro Arg Arg Arg Ser Ser Ser Ser Thr
                325                 330                 335

Thr Met Pro Ser Leu Thr Ser Ile Ala Glu Glu Ser Glu Pro Gly Pro
            340                 345                 350

Val Val Leu Leu Ser Val Ser Pro Arg Pro Arg Ser Gly Pro Thr Ala
        355                 360                 365

Pro Gln Glu Val
    370

<210> 84
<211> 372
<212> PRT
<213> herpes simplex virus 2

<400> 84

Met Pro Gly Arg Ser Leu Gln Gly Leu Ala Ile Leu Gly Leu Trp Val
1               5                   10                  15

Cys Ala Thr Gly Leu Val Val Arg Gly Pro Thr Val Ser Leu Val Ser
            20                  25                  30

Asp Ser Leu Val Asp Ala Gly Ala Val Gly Pro Gln Gly Phe Val Glu
        35                  40                  45

Glu Asp Leu Arg Val Phe Gly Glu Leu His Phe Val Gly Ala Gln Val
    50                  55                  60

Pro His Thr Asn Tyr Tyr Asp Gly Ile Ile Glu Leu Phe His Tyr Pro
65                  70                  75                  80

Leu Gly Asn His Cys Pro Arg Val Val His Val Val Thr Leu Thr Ala
                85                  90                  95

Cys Pro Arg Arg Pro Ala Val Ala Phe Thr Leu Cys Arg Ser Thr His
            100                 105                 110

```
His Ala His Ser Pro Ala Tyr Pro Thr Leu Glu Leu Gly Leu Ala Arg
        115                 120                 125

Gln Pro Leu Leu Arg Val Arg Thr Ala Thr Arg Asp Tyr Ala Gly Leu
    130                 135                 140

Tyr Val Leu Arg Val Trp Val Gly Ser Ala Thr Asn Ala Ser Arg Phe
145                 150                 155                 160

Val Leu Gly Val Ala Leu Ser Ala Asn Gly Thr Phe Val Tyr Asn Gly
                165                 170                 175

Ser Asp Tyr Gly Ser Cys Asp Pro Ala Gln Leu Pro Phe Ser Ala Pro
            180                 185                 190

Arg Leu Gly Pro Ser Ser Val Tyr Thr Pro Gly Ala Ser Arg Pro Thr
        195                 200                 205

Pro Pro Arg Thr Thr Thr Pro Pro Ser Ser Pro Arg Asp Pro Thr Pro
    210                 215                 220

Ala Pro Gly Asp Thr Gly Thr Pro Ala Pro Ala Ser Gly Glu Arg Ala
225                 230                 235                 240

Pro Pro Asn Ser Thr Arg Ser Ala Ser Glu Ser Arg His Arg Leu Thr
                245                 250                 255

Val Ala Gln Val Ile Gln Ile Ala Ile Pro Ala Ser Ile Ile Ala Phe
            260                 265                 270

Val Phe Leu Gly Ser Cys Ile Cys Phe Ile His Arg Cys Gln Arg Arg
        275                 280                 285

Tyr Arg Arg Pro Arg Gly Gln Ile Tyr Asn Pro Gly Gly Val Ser Cys
    290                 295                 300

Ala Val Asn Glu Ala Ala Met Ala Arg Leu Gly Ala Glu Leu Arg Ser
305                 310                 315                 320

His Pro Asn Thr Pro Pro Lys Pro Arg Arg Arg Ser Ser Ser Ser Thr
                325                 330                 335

Thr Met Pro Ser Leu Thr Ser Ile Ala Glu Glu Ser Glu Pro Gly Pro
            340                 345                 350

Val Val Leu Leu Ser Val Ser Pro Arg Pro Arg Ser Gly Pro Thr Ala
        355                 360                 365
```

Pro Gln Glu Val
370

<210> 85
<211> 372
<212> PRT
<213> herpes simplex virus 2

<400> 85

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Arg Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Pro Pro Ser Ser Pro Arg Asp Pro Thr Pro
    210                 215                 220

Ala Pro Gly Asp Thr Gly Thr Pro Ala Pro Ala Ser Gly Glu Arg Ala
225                 230                 235                 240

Pro Pro Asn Ser Thr Arg Ser Ala Ser Glu Ser Arg His Arg Leu Thr
                245                 250                 255

Val Ala Gln Val Ile Gln Ile Ala Ile Pro Ala Ser Ile Ile Ala Phe
            260                 265                 270

Val Phe Leu Gly Ser Cys Ile Cys Phe Ile His Arg Cys Gln Arg Arg
        275                 280                 285

Tyr Arg Arg Pro Arg Gly Gln Ile Tyr Asn Pro Gly Gly Val Ser Cys
    290                 295                 300

Ala Val Asn Glu Ala Ala Met Ala Arg Leu Gly Ala Glu Leu Arg Ser
305                 310                 315                 320

His Pro Asn Thr Pro Pro Lys Pro Arg Arg Arg Ser Ser Ser Ser Thr
                325                 330                 335

Thr Met Pro Ser Leu Thr Ser Ile Ala Glu Glu Ser Glu Pro Gly Pro
            340                 345                 350

Val Val Leu Leu Ser Val Ser Pro Arg Pro Arg Ser Gly Pro Thr Ala
        355                 360                 365

Pro Gln Glu Val
    370

<210> 86
<211> 372
<212> PRT
<213> herpes simplex virus 2

<400> 86

Met Pro Gly Arg Ser Leu Gln Gly Leu Ala Ile Leu Gly Leu Trp Val
1               5                   10                  15

Cys Ala Thr Gly Leu Val Val Arg Gly Pro Thr Val Ser Leu Val Ser
            20                  25                  30

Asp Ser Leu Val Asp Ala Gly Ala Val Gly Pro Gln Gly Phe Val Glu
        35                  40                  45

Glu Asp Leu Arg Val Phe Gly Glu Leu His Phe Val Gly Ala Gln Val
    50                  55                  60

Pro His Thr Asn Tyr Tyr Asp Gly Ile Ile Glu Leu Phe His Tyr Pro
65                  70                  75                  80

Leu Gly Asn His Cys Pro Arg Val Val His Val Val Thr Leu Thr Ala
                85                  90                  95

Cys Pro Arg Arg Pro Ala Val Ala Phe Thr Leu Cys Arg Ser Thr His
            100                 105                 110

His Ala His Ser Pro Ala Tyr Pro Thr Leu Glu Leu Gly Leu Ala Arg
        115                 120                 125

Pro Pro Leu Leu Arg Val Arg Thr Ala Thr Arg Asp Tyr Ala Gly Leu
    130                 135                 140

Tyr Val Leu Arg Val Trp Val Gly Ser Ala Thr Asn Ala Ser Leu Phe
145                 150                 155                 160

Val Leu Gly Val Ala Leu Ser Ala Asn Gly Thr Phe Val Tyr Asn Gly
                165                 170                 175

Ser Asp Tyr Gly Ser Cys Asp Pro Ala Gln Leu Pro Phe Ser Ala Pro
            180                 185                 190

Arg Leu Gly Pro Ser Ser Val Tyr Thr Pro Gly Ala Ser Arg Pro Thr
        195                 200                 205

Pro Pro Arg Thr Thr Thr Pro Pro Ser Ser Pro Arg Asp Pro Thr Pro
    210                 215                 220

Ala Pro Gly Asp Thr Gly Thr Pro Ala Pro Ala Ser Gly Glu Arg Ala
225                 230                 235                 240

Pro Pro Asn Ser Thr Arg Ser Ala Ser Glu Ser Arg His Arg Leu Thr
                245                 250                 255

Val Ala Gln Val Ile Gln Ile Ala Ile Pro Ala Ser Ile Ile Ala Phe
            260                 265                 270

Val Phe Leu Gly Ser Cys Ile Cys Phe Ile His Arg Cys Gln Arg Arg
        275                 280                 285

Tyr Arg Arg Pro Arg Gly Gln Ile Tyr Asn Pro Gly Gly Val Ser Cys

290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 87
<211> 372
<212> PRT
<213> herpes simplex virus 2

<400> 87

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu

130 135 140

Tyr Val Leu Arg Val Trp Val Gly Ser Ala Thr Asn Ala Ser Arg Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Pro Pro Ser Ser Pro Arg Asp Pro Thr Pro
210 215 220

Ala Pro Gly Asp Thr Gly Thr Pro Ala Pro Ala Ser Gly Glu Arg Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 88
<211> 372
<212> PRT
<213> herpes simplex virus 2

<400> 88

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Arg Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Pro Pro Ser Ser Pro Arg Asp Pro Thr Pro
210 215 220

Ala Pro Gly Asp Thr Gly Thr Pro Ala Pro Ala Ser Gly Glu Arg Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 89
<211> 374
<212> PRT
<213> herpes simplex virus 2

<400> 89

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

```
Pro His Thr Asn Tyr Tyr Asp Gly Ile Ile Glu Leu Phe His Tyr Pro
65                  70                  75                  80

Leu Gly Asn His Cys Pro Arg Val Val His Val Val Thr Leu Thr Ala
                85                  90                  95

Cys Pro Arg Arg Pro Ala Val Ala Phe Thr Leu Cys Arg Ser Thr His
            100                 105                 110

His Ala His Ser Pro Ala Tyr Pro Thr Leu Glu Leu Gly Leu Ala Arg
        115                 120                 125

Gln Pro Leu Leu Arg Val Arg Thr Ala Thr Arg Asp Tyr Ala Gly Leu
    130                 135                 140

Tyr Val Leu Arg Val Trp Val Gly Ser Ala Thr Asn Ala Ser Arg Phe
145                 150                 155                 160

Val Leu Gly Val Ala Leu Ser Ala Asn Gly Thr Phe Val Tyr Asn Gly
                165                 170                 175

Ser Asp Tyr Gly Ser Cys Asp Pro Ala Gln Leu Pro Phe Ser Ala Pro
            180                 185                 190

Arg Leu Gly Pro Ser Ser Val Tyr Thr Pro Gly Ala Ser Arg Pro Thr
        195                 200                 205

Pro Pro Arg Thr Thr Thr Ser Pro Ser Ser Pro Arg Asp Pro Thr Pro
    210                 215                 220

Ala Pro Gly Asp Thr Gly Thr Pro Ala Pro Ala Pro Ala Ser Gly Glu
225                 230                 235                 240

Ser Ala Pro Pro Asn Ser Thr Arg Ser Ala Ser Glu Ser Arg His Arg
                245                 250                 255

Leu Thr Val Ala Gln Val Ile Gln Ile Ala Ile Pro Ala Ser Ile Ile
            260                 265                 270

Ala Phe Val Phe Leu Gly Ser Cys Ile Cys Phe Ile His Arg Cys Gln
        275                 280                 285

Arg Arg Tyr Arg Arg Pro Arg Gly Gln Ile Tyr Asn Pro Gly Gly Val
    290                 295                 300

Ser Cys Ala Val Asn Glu Ala Ala Met Ala Arg Leu Gly Ala Glu Leu
305                 310                 315                 320
```

Arg Ser His Pro Asn Thr Pro Pro Lys Pro Arg Arg Arg Ser Ser Ser
325 330 335

Ser Thr Thr Met Pro Ser Leu Thr Ser Ile Ala Glu Glu Ser Glu Pro
340 345 350

Gly Pro Val Val Leu Leu Ser Val Ser Pro Arg Pro Arg Ser Gly Pro
355 360 365

Thr Ala Pro Gln Glu Val
370

<210> 90
<211> 372
<212> PRT
<213> herpes simplex virus 2

<400> 90

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Leu Phe
145 150 155 160

Val Leu Gly Val Ala Leu Ser Ala Asn Gly Thr Phe Val Tyr Asn Gly
165 170 175

Leu Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Pro Pro Ser Ser Pro Arg Asp Pro Thr Pro
210 215 220

Ala Pro Gly Asp Thr Gly Thr Pro Ala Pro Ala Ser Gly Glu Arg Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 91
<211> 372
<212> PRT
<213> herpes simplex virus 2

<400> 91

Met Pro Gly Arg Ser Leu Gln Gly Leu Ala Ile Leu Gly Leu Trp Val
1               5                   10                  15

Cys Ala Thr Gly Leu Val Val Arg Gly Pro Thr Val Ser Leu Val Ser
            20                  25                  30

Asp Ser Leu Val Asp Ala Gly Ala Val Gly Pro Gln Gly Phe Val Glu
        35                  40                  45

Glu Asp Leu Arg Val Phe Gly Glu Leu His Phe Val Gly Ala Gln Val
    50                  55                  60

Pro His Thr Asn Tyr Tyr Asp Gly Ile Ile Glu Leu Phe His Tyr Pro
65                  70                  75                  80

Leu Gly Asn His Cys Pro Arg Val Val His Val Val Thr Leu Thr Ala
                85                  90                  95

Cys Pro Arg Arg Pro Ala Val Ala Phe Thr Leu Cys Arg Ser Thr His
            100                 105                 110

His Ala His Ser Pro Ala Tyr Pro Thr Leu Glu Leu Gly Leu Ala Arg
        115                 120                 125

Gln Pro Leu Leu Arg Val Arg Thr Ala Thr Arg Asp Tyr Ala Gly Leu
    130                 135                 140

Tyr Val Leu Arg Val Trp Val Gly Ser Ala Thr Asn Ala Ser Arg Phe
145                 150                 155                 160

Val Leu Gly Val Ala Leu Ser Ala Asn Gly Thr Phe Val Tyr Asn Gly
                165                 170                 175

Ser Asp Tyr Gly Ser Cys Asp Pro Ala Gln Leu Pro Phe Ser Ala Pro
            180                 185                 190

Arg Leu Gly Pro Ser Ser Val Tyr Thr Pro Gly Ala Ser Arg Pro Thr
        195                 200                 205

Pro Pro Arg Thr Thr Thr Pro Pro Ser Ser Pro Arg Asp Pro Thr Pro
    210                 215                 220

Ala Pro Gly Asp Thr Gly Thr Pro Ala Pro Ala Ser Gly Glu Arg Ala
225                 230                 235                 240

Pro Pro Asn Ser Thr Arg Ser Ala Ser Glu Ser Arg His Arg Leu Thr
                245                 250                 255

```
Val Ser Gln Val Ile Gln Ile Ala Ile Pro Ala Ser Ile Ile Ala Phe
            260                 265                 270

Val Phe Leu Gly Ser Cys Ile Cys Phe Ile His Arg Cys Gln Arg Arg
        275                 280                 285

Tyr Arg Arg Pro Arg Gly Gln Ile Tyr Asn Pro Gly Gly Val Ser Cys
    290                 295                 300

Ala Val Asn Glu Ala Ala Met Ala Arg Leu Gly Ala Glu Leu Arg Ser
305                 310                 315                 320

His Pro Asn Thr Pro Pro Lys Pro Arg Arg Arg Ser Ser Ser Ser Thr
                325                 330                 335

Thr Met Pro Ser Leu Thr Ser Ile Ala Glu Glu Ser Glu Pro Gly Pro
            340                 345                 350

Val Val Leu Leu Ser Val Ser Pro Arg Pro Arg Ser Gly Pro Thr Ala
        355                 360                 365

Pro Gln Glu Val
    370


<210>  92
<211>  372
<212>  PRT
<213>  herpes simplex virus 2

<400>  92

Met Pro Gly Arg Ser Leu Gln Gly Leu Val Ile Leu Gly Leu Trp Val
1               5                   10                  15

Cys Ala Thr Gly Leu Val Val Arg Gly Pro Thr Val Ser Leu Val Ser
            20                  25                  30

Asp Ser Leu Val Asp Ala Gly Ala Val Gly Pro Gln Gly Phe Val Glu
        35                  40                  45

Glu Asp Leu Arg Val Phe Gly Glu Leu His Phe Val Gly Ala Gln Val
    50                  55                  60

Pro His Thr Asn Tyr Tyr Asp Gly Ile Ile Glu Leu Phe His Tyr Pro
65                  70                  75                  80

Leu Gly Asn His Cys Pro Arg Val Val His Val Val Thr Leu Thr Ala
                85                  90                  95
```

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Leu Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Leu Pro Ser Ser Pro Arg Asp Pro Thr Pro
210 215 220

Ala Pro Gly Asp Thr Gly Thr Pro Ala Pro Ala Ser Gly Glu Arg Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro

340 345 350

Val Val Leu Leu Ser Val Ser Pro Arg Pro Arg Ser Gly Pro Thr Ala
355 360 365

Pro Gln Glu Val
370

<210> 93
<211> 372
<212> PRT
<213> herpes simplex virus 2

<400> 93

Met Pro Gly Arg Ser Leu Gln Gly Leu Val Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Leu Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro

180 185 190

Arg Leu Gly Pro Ser Ser Val Tyr Thr Pro Gly Ala Ser Arg Pro Thr
195 200 205

Pro Pro Arg Thr Thr Thr Leu Pro Ser Ser Pro Arg Asp Pro Thr Pro
210 215 220

Ala Pro Gly Asp Thr Gly Thr Pro Ala Pro Ala Ser Gly Glu Arg Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 94
<211> 372
<212> PRT
<213> herpes simplex virus 2

<400> 94

Met Pro Gly Arg Ser Leu Gln Gly Leu Val Ile Leu Gly Leu Trp Val
1 5 10 15

Cys Ala Thr Gly Leu Val Val Arg Gly Pro Thr Val Ser Leu Val Ser

```
            20                  25                  30

Asp Ser Leu Val Asp Ala Gly Ala Val Gly Pro Gln Gly Phe Val Glu
        35                  40                  45

Glu Asp Leu Arg Val Phe Gly Glu Leu His Phe Val Gly Ala Gln Val
    50                  55                  60

Pro His Thr Asn Tyr Tyr Asp Gly Ile Ile Glu Leu Phe His Tyr Pro
65                  70                  75                  80

Leu Gly Asn His Cys Pro Arg Val Val His Val Val Thr Leu Thr Ala
                85                  90                  95

Cys Pro Arg Arg Pro Ala Val Ala Phe Thr Leu Cys Arg Ser Thr His
            100                 105                 110

His Ala His Ser Pro Ala Tyr Pro Thr Leu Glu Leu Gly Leu Ala Arg
        115                 120                 125

Gln Pro Leu Leu Arg Val Arg Thr Ala Thr Arg Asp Tyr Ala Gly Leu
    130                 135                 140

Tyr Val Leu Arg Val Trp Val Gly Ser Ala Thr Asn Ala Ser Leu Phe
145                 150                 155                 160

Val Leu Gly Val Ala Leu Ser Ala Asn Gly Thr Phe Val Tyr Asn Gly
                165                 170                 175

Ser Asp Tyr Gly Ser Cys Asp Pro Ala Gln Leu Pro Phe Ser Ala Pro
            180                 185                 190

Arg Leu Gly Pro Ser Ser Val Tyr Thr Pro Gly Ala Ser Arg Pro Thr
        195                 200                 205

Pro Pro Arg Thr Thr Thr Leu Pro Ser Ser Pro Arg Asp Pro Thr Pro
    210                 215                 220

Ala Pro Gly Asp Thr Gly Thr Pro Ala Pro Ala Ser Gly Glu Arg Ala
225                 230                 235                 240

Pro Pro Asn Ser Thr Arg Ser Ala Ser Glu Ser Arg His Arg Leu Thr
                245                 250                 255

Val Ala Gln Val Ile Gln Ile Ala Ile Pro Ala Ser Ile Ile Ala Phe
            260                 265                 270
```

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 95
<211> 372
<212> PRT
<213> herpes simplex virus 2

<400> 95

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

```
His Ala His Ser Pro Ala Tyr Pro Thr Leu Glu Leu Gly Leu Ala Arg
        115                 120                 125

Gln Pro Leu Leu Arg Val Arg Thr Ala Thr Arg Asp Tyr Ala Gly Leu
    130                 135                 140

Tyr Val Leu Arg Val Trp Val Gly Ser Ala Thr Asn Ala Ser Leu Phe
145                 150                 155                 160

Val Leu Gly Val Ala Leu Ser Ala Asn Gly Thr Phe Val Tyr Asn Gly
                165                 170                 175

Leu Asp Tyr Gly Ser Cys Asp Pro Ala Gln Leu Pro Phe Ser Ala Pro
            180                 185                 190

Arg Leu Gly Pro Ser Ser Val Tyr Thr Pro Gly Ala Ser Arg Pro Thr
        195                 200                 205

Pro Pro Arg Thr Thr Thr Pro Pro Ser Ser Pro Arg Asp Pro Thr Pro
    210                 215                 220

Ala Pro Gly Asp Thr Gly Thr Pro Ala Pro Ala Ser Gly Glu Arg Ala
225                 230                 235                 240

Pro Pro Asn Ser Thr Arg Ser Ala Ser Glu Ser Arg His Arg Leu Thr
                245                 250                 255

Val Ala Gln Val Ile Gln Ile Ala Ile Pro Ala Ser Ile Ile Ala Phe
            260                 265                 270

Val Phe Leu Gly Ser Cys Ile Cys Phe Ile His Arg Cys Gln Arg Arg
        275                 280                 285

Tyr Arg Arg Pro Arg Gly Gln Ile Tyr Asn Pro Gly Gly Val Ser Cys
    290                 295                 300

Ala Val Asn Glu Ala Ala Met Ala Arg Leu Gly Ala Glu Leu Arg Ser
305                 310                 315                 320

His Pro Asn Thr Pro Pro Lys Pro Arg Arg Arg Ser Ser Ser Ser Thr
                325                 330                 335

Thr Met Pro Ser Leu Thr Ser Ile Ala Glu Glu Ser Glu Pro Gly Pro
            340                 345                 350

Val Val Leu Leu Ser Val Ser Pro Arg Pro Arg Ser Gly Pro Thr Ala
        355                 360                 365
```

Pro Gln Glu Val
    370

<210> 96
<211> 372
<212> PRT
<213> herpes simplex virus 2

<400> 96

Met Pro Gly Arg Ser Leu Gln Gly Leu Ala Ile Leu Gly Leu Trp Val
1               5                   10                  15

Cys Ala Thr Gly Leu Val Val Arg Gly Pro Thr Val Ser Leu Val Ser
            20                  25                  30

Asp Ser Leu Val Asp Ala Gly Ala Val Gly Pro Gln Gly Phe Val Glu
        35                  40                  45

Glu Asp Leu Arg Val Phe Gly Glu Leu His Phe Val Gly Ala Gln Val
    50                  55                  60

Pro His Thr Asn Tyr Tyr Asp Gly Ile Ile Glu Leu Phe His Tyr Pro
65                  70                  75                  80

Leu Gly Asn His Cys Pro Arg Val Val His Val Val Thr Leu Thr Ala
                85                  90                  95

Cys Pro Arg Arg Pro Ala Val Ala Phe Thr Leu Cys Arg Ser Thr His
            100                 105                 110

His Ala His Ser Pro Ala Tyr Pro Thr Leu Glu Leu Gly Leu Ala Arg
        115                 120                 125

Gln Pro Leu Leu Arg Val Arg Thr Ala Thr Arg Asp Tyr Ala Gly Leu
    130                 135                 140

Tyr Val Leu Arg Val Trp Val Gly Ser Ala Thr Asn Ala Ser Arg Phe
145                 150                 155                 160

Val Leu Gly Val Ala Leu Ser Ala Asn Gly Thr Phe Val Tyr Asn Gly
                165                 170                 175

Ser Asp Tyr Gly Ser Cys Asp Pro Ala Gln Leu Pro Phe Ser Ala Pro
            180                 185                 190

Arg Leu Gly Pro Ser Ser Val Tyr Thr Pro Gly Ala Ser Arg Pro Thr
        195                 200                 205

```
Pro Pro Arg Thr Thr Thr Pro Pro Ser Ser Pro Arg Asp Pro Thr Pro
    210                 215                 220

Ala Pro Gly Asp Thr Gly Thr Pro Ala Pro Ala Ser Gly Glu Arg Ala
225                 230                 235                 240

Pro Pro Asn Ser Thr Arg Ser Ala Ser Glu Ser Arg His Arg Leu Thr
                245                 250                 255

Val Ser Gln Val Ile Gln Ile Ala Ile Pro Ala Ser Ile Ile Ala Phe
            260                 265                 270

Val Phe Leu Gly Ser Cys Ile Cys Phe Ile His Arg Cys Gln Arg Arg
        275                 280                 285

Tyr Arg Arg Pro Arg Gly Gln Ile Tyr Asn Pro Gly Gly Val Ser Cys
    290                 295                 300

Ala Val Asn Glu Ala Ala Met Ala Arg Leu Gly Ala Glu Leu Arg Ser
305                 310                 315                 320

His Pro Asn Thr Pro Pro Lys Pro Arg Arg Arg Ser Ser Ser Ser Thr
                325                 330                 335

Thr Met Pro Ser Leu Thr Ser Ile Ala Glu Glu Ser Glu Pro Gly Pro
            340                 345                 350

Val Val Leu Leu Ser Val Ser Pro Arg Pro Arg Ser Gly Pro Thr Ala
        355                 360                 365

Pro Gln Glu Val
    370


<210>  97
<211>  372
<212>  PRT
<213>  herpes simplex virus 2

<400>  97

Met Pro Gly Arg Ser Leu Gln Gly Leu Val Ile Leu Gly Leu Trp Val
1               5                   10                  15

Cys Ala Thr Gly Leu Val Val Arg Gly Pro Thr Val Ser Leu Val Ser
            20                  25                  30

Asp Ser Leu Val Asp Ala Gly Ala Val Gly Pro Gln Gly Phe Val Glu
        35                  40                  45
```

```
Glu Asp Leu Arg Val Phe Gly Glu Leu His Phe Val Gly Ala Gln Val
    50                  55                  60

Pro His Thr Asn Tyr Tyr Asp Gly Ile Ile Glu Leu Phe His Tyr Pro
65                  70                  75                  80

Leu Gly Asn His Cys Pro Arg Val Val His Val Val Thr Leu Thr Ala
                85                  90                  95

Cys Pro Arg Arg Pro Ala Val Ala Phe Thr Leu Cys Arg Ser Thr His
            100                 105                 110

His Ala His Ser Pro Ala Tyr Pro Thr Leu Glu Leu Gly Leu Ala Arg
        115                 120                 125

Gln Pro Leu Leu Arg Val Arg Thr Ala Thr Arg Asp Tyr Ala Gly Leu
    130                 135                 140

Tyr Val Leu Arg Val Trp Val Gly Ser Ala Thr Asn Ala Ser Leu Phe
145                 150                 155                 160

Val Leu Gly Val Ala Leu Ser Ala Asn Gly Thr Phe Val Tyr Asn Gly
                165                 170                 175

Ser Asp Tyr Gly Ser Cys Asp Pro Ala Gln Leu Pro Phe Ser Ala Pro
            180                 185                 190

Arg Leu Gly Pro Ser Ser Val Tyr Thr Pro Gly Ala Ser Arg Pro Thr
        195                 200                 205

Pro Pro Arg Thr Thr Thr Leu Pro Ser Ser Pro Arg Asp Pro Thr Pro
    210                 215                 220

Ala Pro Gly Asp Thr Gly Thr Pro Ala Pro Ala Ser Gly Glu Arg Ala
225                 230                 235                 240

Pro Pro Asn Ser Thr Arg Ser Ala Ser Glu Ser Arg His Arg Leu Thr
                245                 250                 255

Val Ala Gln Val Ile Gln Ile Ala Ile Pro Ala Ser Ile Ile Ala Phe
            260                 265                 270

Val Phe Leu Gly Ser Cys Ile Cys Phe Ile His Arg Cys Gln Arg Arg
        275                 280                 285

Tyr Arg Arg Pro Arg Gly Gln Ile Tyr Asn Pro Gly Gly Val Ser Cys
    290                 295                 300
```

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 98
<211> 372
<212> PRT
<213> herpes simplex virus 2

<400> 98

Met Pro Gly Arg Ser Leu Gln Gly Leu Val Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Leu Phe
145                 150                 155                 160

Val Leu Gly Val Ala Leu Ser Ala Asn Gly Thr Phe Val Tyr Asn Gly
                165                 170                 175

Ser Asp Tyr Gly Ser Cys Asp Pro Ala Gln Leu Pro Phe Ser Ala Pro
            180                 185                 190

Arg Leu Gly Pro Ser Ser Val Tyr Thr Pro Gly Ala Ser Arg Pro Thr
        195                 200                 205

Pro Pro Arg Thr Thr Thr Leu Pro Ser Ser Pro Arg Asp Pro Thr Pro
    210                 215                 220

Ala Pro Gly Asp Thr Gly Thr Pro Ala Pro Ala Ser Gly Glu Arg Ala
225                 230                 235                 240

Pro Pro Asn Ser Thr Arg Ser Ala Ser Glu Ser Arg His Arg Leu Thr
                245                 250                 255

Val Ala Gln Val Ile Gln Ile Ala Ile Pro Ala Ser Ile Ile Ala Phe
            260                 265                 270

Val Phe Leu Gly Ser Cys Ile Cys Phe Ile His Arg Cys Gln Arg Arg
        275                 280                 285

Tyr Arg Arg Pro Arg Gly Gln Ile Tyr Asn Pro Gly Gly Val Ser Cys
    290                 295                 300

Ala Val Asn Glu Ala Ala Met Ala Arg Leu Gly Ala Glu Leu Arg Ser
305                 310                 315                 320

His Pro Asn Thr Pro Pro Lys Pro Arg Arg Arg Ser Ser Ser Ser Thr
                325                 330                 335

Thr Met Pro Ser Leu Thr Ser Ile Ala Glu Glu Ser Glu Pro Gly Pro
            340                 345                 350

Val Val Leu Leu Ser Val Ser Pro Arg Pro Arg Ser Gly Pro Thr Ala
        355                 360                 365

Pro Gln Glu Val
    370

<210>  99

<211> 371
<212> PRT
<213> herpes simplex virus 2

<400> 99

Met Pro Gly Arg Ser Leu Gln Gly Leu Ala Ile Leu Gly Leu Trp Val
1               5                   10                  15

Cys Ala Thr Gly Leu Val Val Arg Gly Pro Thr Val Ser Leu Val Ser
            20                  25                  30

Asp Ser Leu Val Asp Ala Gly Ala Val Gly Pro Gln Gly Phe Val Glu
        35                  40                  45

Glu Asp Leu Arg Val Phe Gly Glu Leu His Phe Val Gly Ala Gln Val
    50                  55                  60

Pro His Thr Asn Tyr Tyr Asp Gly Ile Ile Glu Leu Phe His Tyr Pro
65                  70                  75                  80

Leu Gly Asn His Cys Pro Arg Val Val His Val Ile Thr Leu Thr Ala
                85                  90                  95

Cys Pro Arg Arg Pro Ala Val Ala Phe Thr Leu Cys Arg Ser Thr His
            100                 105                 110

His Ala His Ser Pro Ala Tyr Pro Thr Leu Glu Leu Gly Leu Ala Arg
        115                 120                 125

Gln Pro Leu Leu Arg Val Arg Thr Ala Thr Arg Asp Tyr Ala Gly Leu
    130                 135                 140

Tyr Val Leu Arg Val Trp Val Gly Ser Ala Thr Asn Ala Ser Arg Phe
145                 150                 155                 160

Val Leu Gly Val Ala Leu Ser Ala Asn Gly Thr Phe Val Tyr Asn Gly
                165                 170                 175

Ser Asp Tyr Gly Ser Cys Asp Pro Ala Gln Leu Pro Phe Ser Ala Pro
            180                 185                 190

Arg Leu Gly Pro Ser Ser Val Tyr Thr Pro Gly Ala Ser Arg Pro Thr
        195                 200                 205

Pro Pro Arg Thr Thr Thr Ser Pro Ser Ser Pro Arg Asp Pro Thr Pro
    210                 215                 220

Ala Pro Gly Asp Thr Gly Thr Pro Ala Pro Ala Pro Ala Ser Gly Glu

225 230 235 240

Arg Ala Pro Pro Asn Ser Thr Arg Ser Ala Ser Glu Ser Arg His Arg
245 250 255

Leu Thr Val Ala Gln Val Ile Gln Ile Ala Ile Pro Ala Ser Ile Ile
260 265 270

Ala Phe Val Phe Leu Gly Ser Cys Ile Cys Phe Ile His Arg Cys Gln
275 280 285

Arg Arg Tyr Arg Arg Pro Arg Gly Gln Ile Tyr Asn Pro Gly Gly Val
290 295 300

Ser Cys Ala Val Asn Glu Ala Ala Met Ala Arg Leu Gly Ala Glu Leu
305 310 315 320

Arg Ser His Pro Asn Thr Pro Pro Lys Pro Arg Arg Arg Ser Ser Ser
325 330 335

Ser Thr Thr Met Pro Ser Leu Thr Ser Ile Ala Glu Glu Ser Glu Pro
340 345 350

Gly Pro Val Val Leu Leu Ser Val Ser Pro Arg Pro Arg Ser Gly Pro
355 360 365

Thr Ala Pro
370

<210> 100
<211> 372
<212> PRT
<213> herpes simplex virus 2

<220>
<221> misc_feature
<222> (222)..(222)
<223> Xaa can be any naturally occurring amino acid

<400> 100

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
    50                  55                  60

Pro His Thr Asn Tyr Tyr Asp Gly Ile Ile Glu Leu Phe His Tyr Pro
65                  70                  75                  80

Leu Gly Asn His Cys Pro Arg Val Val His Val Val Thr Leu Thr Ala
                85                  90                  95

Cys Pro Arg Arg Pro Ala Val Ala Phe Thr Leu Cys Arg Ser Thr His
            100                 105                 110

His Ala His Ser Pro Ala Tyr Pro Thr Leu Glu Leu Gly Leu Ala Arg
        115                 120                 125

Gln Pro Leu Leu Arg Val Arg Thr Ala Thr Arg Asp Tyr Ala Gly Leu
    130                 135                 140

Tyr Val Leu Arg Val Trp Val Gly Ser Ala Thr Asn Ala Ser Leu Phe
145                 150                 155                 160

Val Leu Gly Val Ala Leu Ser Ala Asn Gly Thr Phe Val Tyr Asn Gly
                165                 170                 175

Ser Asp Tyr Gly Ser Cys Asp Pro Ala Gln Leu Pro Phe Ser Ala Pro
            180                 185                 190

Arg Leu Gly Pro Ser Ser Val Tyr Thr Pro Gly Ala Ser Arg Pro Thr
        195                 200                 205

Pro Pro Arg Thr Thr Thr Pro Pro Ser Ser Pro Arg Asp Xaa Thr Pro
    210                 215                 220

Ala Pro Gly Asp Thr Gly Thr Pro Ala Pro Ala Ser Gly Glu Ile Ala
225                 230                 235                 240

Pro Pro Asn Ser Thr Arg Ser Ala Ser Glu Ser Arg His Arg Leu Thr
                245                 250                 255

Val Ala Gln Val Ile Gln Ile Ala Ile Pro Ala Ser Ile Ile Ala Phe
            260                 265                 270

Val Phe Leu Gly Ser Cys Ile Cys Phe Ile His Arg Cys Gln Arg Arg
        275                 280                 285

Tyr Arg Arg Pro Arg Gly Gln Ile Tyr Asn Pro Gly Gly Val Ser Cys
    290                 295                 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 101
<211> 372
<212> PRT
<213> herpes simplex virus 2

<400> 101

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

```
Tyr Val Leu Arg Val Trp Val Gly Ser Ala Thr Asn Ala Ser Arg Phe
145                 150                 155                 160

Val Leu Gly Val Ala Leu Ser Ala Asn Gly Thr Phe Val Tyr Asn Gly
                165                 170                 175

Ser Asp Tyr Gly Ser Cys Asp Pro Ala Gln Leu Pro Phe Ser Ala Pro
            180                 185                 190

Arg Leu Gly Pro Ser Ser Val Tyr Thr Pro Gly Ala Ser Arg Pro Thr
        195                 200                 205

Pro Pro Arg Thr Thr Thr Pro Pro Ser Ser Pro Arg Asp Pro Thr Pro
    210                 215                 220

Ala Pro Gly Asp Thr Gly Thr Pro Ala Pro Ala Ser Gly Glu Ile Ala
225                 230                 235                 240

Pro Pro Asn Ser Thr Arg Ser Ala Ser Glu Ser Arg His Arg Leu Thr
                245                 250                 255

Val Ala Gln Val Ile Gln Ile Ala Ile Pro Ala Ser Ile Ile Ala Phe
            260                 265                 270

Val Phe Leu Gly Ser Cys Ile Cys Phe Ile His Arg Cys Gln Arg Arg
        275                 280                 285

Tyr Arg Arg Pro Arg Gly Gln Ile Tyr Asn Pro Gly Gly Val Ser Cys
    290                 295                 300

Ala Val Asn Glu Ala Ala Met Ala Arg Leu Gly Ala Glu Leu Arg Ser
305                 310                 315                 320

His Pro Asn Thr Pro Pro Lys Pro Arg Arg Arg Ser Ser Ser Ser Thr
                325                 330                 335

Thr Met Pro Ser Leu Thr Ser Ile Ala Glu Glu Ser Glu Pro Gly Pro
            340                 345                 350

Val Val Leu Leu Ser Val Ser Pro Arg Pro Arg Ser Gly Pro Thr Ala
        355                 360                 365

Pro Gln Glu Val
    370

<210>  102
```

<211> 372
<212> PRT
<213> herpes simplex virus 2

<400> 102

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Arg Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Pro Pro Ser Ser Pro Arg Asp Pro Thr Pro
210 215 220

Ala Pro Gly Asp Thr Gly Thr Pro Ala Pro Ala Ser Gly Glu Ile Ala

225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 103
<211> 372
<212> PRT
<213> herpes simplex virus 2

<400> 103

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro

65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Arg Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Pro Pro Ser Ser Pro Arg Asp Pro Thr Pro
210 215 220

Ala Pro Gly Asp Thr Gly Thr Pro Ala Pro Ala Ser Gly Glu Ile Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

```
His Pro Asn Thr Pro Pro Lys Pro Arg Arg Arg Ser Ser Ser Ser Thr
                325                 330                 335

Thr Met Pro Ser Leu Thr Ser Ile Ala Glu Glu Ser Glu Pro Gly Pro
            340                 345                 350

Val Val Leu Leu Ser Val Ser Pro Arg Pro Arg Ser Gly Pro Thr Ala
        355                 360                 365

Pro Gln Glu Val
    370


<210>  104
<211>  372
<212>  PRT
<213>  herpes simplex virus 2

<400>  104

Met Pro Gly Arg Ser Leu Gln Gly Leu Ala Ile Leu Gly Leu Trp Val
1               5                   10                  15

Cys Ala Thr Gly Leu Val Val Arg Gly Pro Thr Val Ser Leu Val Ser
            20                  25                  30

Asp Ser Leu Val Asp Ala Gly Ala Val Gly Pro Gln Gly Phe Val Glu
        35                  40                  45

Glu Asp Leu Arg Val Phe Gly Glu Leu His Phe Val Gly Ala Gln Val
    50                  55                  60

Pro His Thr Asn Tyr Tyr Asp Gly Ile Ile Glu Leu Phe His Tyr Pro
65                  70                  75                  80

Leu Gly Asn His Cys Pro Arg Val Val His Val Val Thr Leu Thr Ala
                85                  90                  95

Cys Pro Arg Arg Pro Ala Val Ala Phe Thr Leu Cys Arg Ser Thr His
            100                 105                 110

His Ala His Ser Pro Ala Tyr Pro Thr Leu Glu Leu Gly Leu Ala Arg
        115                 120                 125

Gln Pro Leu Leu Arg Val Arg Thr Ala Thr Arg Asp Tyr Ala Gly Leu
    130                 135                 140

Tyr Val Leu Arg Val Trp Val Gly Ser Ala Thr Asn Ala Ser Arg Phe
145                 150                 155                 160
```

```
Val Leu Gly Val Ala Leu Ser Ala Asn Gly Thr Phe Val Tyr Asn Gly
                165                 170                 175

Ser Asp Tyr Gly Ser Cys Asp Pro Ala Gln Leu Pro Phe Ser Ala Pro
            180                 185                 190

Arg Leu Gly Pro Ser Ser Val Tyr Thr Pro Gly Ala Ser Arg Pro Thr
        195                 200                 205

Pro Pro Arg Thr Thr Thr Pro Pro Ser Ser Pro Arg Asp Pro Thr Pro
    210                 215                 220

Ala Pro Gly Asp Thr Gly Thr Pro Ala Pro Ala Ser Gly Glu Ser Ala
225                 230                 235                 240

Pro Pro Asn Ser Thr Arg Ser Ala Ser Glu Ser Arg His Arg Leu Thr
                245                 250                 255

Val Ala Gln Val Ile Gln Ile Ala Ile Pro Ala Ser Ile Ile Ala Phe
            260                 265                 270

Val Phe Leu Gly Ser Cys Ile Cys Phe Ile His Arg Cys Gln Arg Arg
        275                 280                 285

Tyr Arg Arg Pro Arg Gly Gln Ile Tyr Asn Pro Gly Gly Val Ser Cys
    290                 295                 300

Ala Val Asn Glu Ala Ala Met Ala Arg Leu Gly Ala Glu Leu Arg Ser
305                 310                 315                 320

His Pro Asn Thr Pro Pro Lys Pro Arg Arg Arg Ser Ser Ser Ser Thr
                325                 330                 335

Thr Met Pro Ser Leu Thr Ser Ile Ala Glu Glu Ser Glu Pro Gly Pro
            340                 345                 350

Val Val Leu Leu Ser Val Ser Pro Arg Pro Arg Ser Gly Pro Thr Ala
        355                 360                 365

Pro Gln Glu Val
    370
```

<210>  105
<211>  372
<212>  PRT
<213>  herpes simplex virus 2

<400>  105

```
Met Pro Gly Arg Ser Leu Gln Gly Leu Ala Ile Leu Gly Leu Trp Val
1               5                   10                  15

Cys Ala Thr Gly Leu Val Val Arg Gly Pro Thr Val Ser Leu Val Ser
            20                  25                  30

Asp Ser Leu Val Asp Ala Gly Ala Val Gly Pro Gln Gly Phe Val Glu
        35                  40                  45

Glu Asp Leu Arg Val Phe Gly Glu Leu His Phe Val Gly Ala Gln Val
    50                  55                  60

Pro His Thr Asn Tyr Tyr Asp Gly Ile Ile Glu Leu Phe His Tyr Pro
65                  70                  75                  80

Leu Gly Asn His Cys Pro Arg Val Val His Val Val Thr Leu Thr Ala
                85                  90                  95

Cys Pro Arg Arg Pro Ala Val Ala Phe Thr Leu Cys Arg Ser Thr His
            100                 105                 110

His Ala His Ser Pro Ala Tyr Pro Thr Leu Glu Leu Gly Leu Ala Arg
        115                 120                 125

Gln Pro Leu Leu Arg Val Arg Thr Ala Thr Arg Asp Tyr Ala Gly Leu
    130                 135                 140

Tyr Val Leu Arg Val Trp Val Gly Ser Ala Thr Asn Ala Ser Arg Phe
145                 150                 155                 160

Val Leu Gly Val Ala Leu Ser Ala Asn Gly Thr Phe Val Tyr Asn Gly
                165                 170                 175

Ser Asp Tyr Gly Ser Cys Asp Pro Ala Gln Leu Pro Phe Ser Ala Pro
            180                 185                 190

Arg Leu Gly Pro Ser Ser Val Tyr Thr Pro Gly Ala Ser Arg Pro Thr
        195                 200                 205

Pro Pro Arg Thr Thr Thr Pro Pro Ser Ser Pro Arg Asp Pro Thr Pro
    210                 215                 220

Ala Pro Gly Asp Thr Gly Thr Pro Ala Pro Ala Ser Gly Glu Ser Ala
225                 230                 235                 240

Pro Pro Asn Ser Thr Arg Ser Ala Ser Glu Ser Arg His Arg Leu Thr
                245                 250                 255
```

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Leu Glu Pro Gly Pro
340 345 350

Val Val Leu Leu Ser Val Ser Pro Arg Pro Arg Ser Gly Pro Thr Ala
355 360 365

Pro Gln Glu Val
370

<210> 106
<211> 372
<212> PRT
<213> herpes simplex virus 2

<400> 106

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Arg Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Pro Pro Ser Ser Pro Arg Asp Pro Thr Pro
210 215 220

Ala Pro Gly Asp Thr Gly Thr Pro Ala Pro Ala Ser Gly Glu Lys Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 107
<211> 372
<212> PRT
<213> herpes simplex virus 2

<400> 107

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

Arg Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Arg Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Pro Pro Ser Ser Pro Arg Asp Pro Thr Pro
210 215 220

Ala Pro Gly Asp Thr Gly Thr Pro Ala Pro Ala Ser Gly Glu Arg Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 108
<211> 372
<212> PRT
<213> herpes simplex virus 2

<400> 108

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

```
Asp Ser Leu Val Asp Ala Gly Ala Val Gly Pro Gln Gly Phe Val Glu
        35                  40                  45

Glu Asp Leu Arg Val Phe Gly Glu Leu His Phe Val Gly Ala Gln Val
    50                  55                  60

Pro His Thr Asn Tyr Tyr Asp Gly Ile Ile Glu Leu Phe His Tyr Pro
65                  70                  75                  80

Leu Gly Asn His Cys Pro Arg Val Val His Val Val Thr Leu Thr Ala
                85                  90                  95

Cys Pro Arg Arg Pro Ala Val Ala Phe Thr Leu Cys Arg Ser Thr His
            100                 105                 110

His Ala His Ser Pro Ala Tyr Pro Thr Leu Glu Leu Gly Leu Ala Arg
        115                 120                 125

Gln Pro Leu Leu Arg Val Arg Thr Ala Thr Arg Asp Tyr Ala Gly Leu
    130                 135                 140

Tyr Val Leu Arg Val Trp Val Gly Ser Ala Thr Asn Ala Ser Arg Phe
145                 150                 155                 160

Val Leu Gly Val Ala Leu Ser Ala Asn Gly Thr Phe Val Tyr Asn Gly
                165                 170                 175

Ser Asp Tyr Gly Ser Cys Asp Pro Ala Gln Leu Pro Phe Ser Ala Pro
            180                 185                 190

Arg Leu Gly Pro Ser Ser Val Tyr Thr Pro Gly Ala Ser Arg Pro Thr
        195                 200                 205

Pro Pro Arg Thr Thr Thr Pro Pro Ser Ser Pro Arg Asp Pro Thr Pro
    210                 215                 220

Ala Pro Gly Asp Thr Gly Thr Pro Ala Pro Ala Ser Gly Glu Ser Ala
225                 230                 235                 240

Pro Pro Asn Ser Thr Arg Ser Ala Ser Glu Ser Arg His Arg Leu Thr
                245                 250                 255

Val Ala Gln Val Ile Gln Ile Ala Ile Pro Ala Ser Ile Ile Ala Phe
            260                 265                 270

Val Phe Leu Gly Ser Cys Ile Cys Phe Ile His Arg Cys Gln Arg Arg
```

275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 109
<211> 372
<212> PRT
<213> herpes simplex virus 2

<400> 109

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg

115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Arg Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Pro Pro Ser Ser Pro Arg Asp Pro Thr Pro
210 215 220

Ala Pro Gly Asp Thr Gly Thr Pro Ala Pro Ala Ser Gly Glu Ser Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 110
<211> 372
<212> PRT
<213> herpes simplex virus 2

<400> 110

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Arg Val Gly Ser Ala Thr Asn Ala Ser Arg Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Pro Pro Ser Ser Pro Arg Asp Pro Thr Pro
210 215 220

Ala Pro Gly Asp Thr Gly Thr Pro Ala Pro Ala Ser Gly Glu Arg Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 111
<211> 372
<212> PRT
<213> herpes simplex virus 2

<400> 111

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Arg Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Pro Pro Ser Ser Pro Arg Asp Pro Thr Pro
210 215 220

Ala Pro Gly Asp Thr Gly Thr Pro Ala Pro Ala Ser Gly Glu Ile Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 112
<211> 372
<212> PRT
<213> herpes simplex virus 2

<400> 112

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Arg Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Pro Pro Ser Ser Pro Arg Asp Pro Thr Pro
210 215 220

Ala Pro Gly Asp Thr Gly Thr Pro Ala Pro Ala Ser Gly Glu Ile Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 113
<211> 372

<212> PRT
<213> herpes simplex virus 2

<400> 113

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Arg Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Pro Pro Ser Ser Pro Arg Asp Pro Thr Pro
210 215 220

Ala Pro Gly Asp Thr Gly Thr Pro Ala Pro Ala Ser Gly Glu Ser Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 114
<211> 372
<212> PRT
<213> herpes simplex virus 2

<400> 114

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

```
Leu Gly Asn His Cys Pro Arg Val Val His Val Val Thr Leu Thr Ala
                85                  90                  95

Cys Pro Arg Arg Pro Ala Val Ala Phe Thr Leu Cys Arg Ser Thr His
            100                 105                 110

His Ala His Ser Pro Ala Tyr Pro Thr Leu Glu Leu Gly Leu Ala Arg
        115                 120                 125

Gln Pro Leu Leu Arg Val Arg Thr Ala Thr Arg Asp Tyr Ala Gly Leu
    130                 135                 140

Tyr Val Leu Arg Val Trp Val Gly Ser Ala Thr Asn Ala Ser Arg Phe
145                 150                 155                 160

Val Leu Gly Val Ala Leu Ser Ala Asn Gly Thr Phe Val Tyr Asn Gly
                165                 170                 175

Ser Asp Tyr Gly Ser Cys Asp Pro Ala Gln Leu Pro Phe Ser Ala Pro
            180                 185                 190

Arg Leu Gly Pro Ser Ser Val Tyr Thr Pro Gly Ala Ser Arg Pro Thr
        195                 200                 205

Pro Pro Arg Thr Thr Thr Pro Pro Ser Ser Pro Arg Asp Pro Thr Pro
    210                 215                 220

Ala Pro Gly Asp Thr Gly Thr Pro Ala Pro Ala Ser Gly Glu Ser Ala
225                 230                 235                 240

Pro Pro Asn Ser Thr Arg Ser Ala Ser Glu Ser Arg His Arg Leu Thr
                245                 250                 255

Val Ala Gln Val Ile Gln Ile Ala Ile Pro Ala Ser Ile Ile Ala Phe
            260                 265                 270

Val Phe Leu Gly Ser Cys Ile Cys Phe Ile His Arg Cys Gln Arg Arg
        275                 280                 285

Tyr Arg Arg Pro Arg Gly Gln Ile Tyr Asn Pro Gly Gly Val Ser Cys
    290                 295                 300

Ala Val Asn Glu Ala Ala Met Ala Arg Leu Gly Ala Glu Leu Arg Ser
305                 310                 315                 320

His Pro Asn Thr Pro Pro Lys Pro Arg Arg Arg Ser Ser Ser Ser Thr
```

325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 115
<211> 372
<212> PRT
<213> herpes simplex virus 2

<400> 115

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Arg Phe
145 150 155 160

Val Leu Gly Val Ala Leu Ser Ala Asn Gly Thr Phe Val Tyr Asn Gly

165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Pro Pro Ser Ser Pro Arg Asp Pro Thr Pro
210 215 220

Ala Pro Gly Asp Thr Gly Thr Pro Ala Pro Ala Ser Gly Glu Ser Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 116
<211> 372
<212> PRT
<213> herpes simplex virus 2

<400> 116

Met Pro Gly Arg Ser Leu Gln Gly Leu Val Ile Leu Gly Leu Trp Val

1 5 10 15

Cys Thr Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Leu Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Leu Pro Ser Ser Pro Arg Asp Pro Thr Pro
210 215 220

Ala Pro Gly Asp Thr Gly Thr Pro Ala Pro Ala Ser Gly Glu Arg Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 117
<211> 372
<212> PRT
<213> herpes simplex virus 2

<400> 117

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Arg Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Pro Pro Ser Ser Pro Arg Asp Pro Thr Pro
210 215 220

Ala Pro Gly Asp Thr Gly Thr Pro Ala Pro Ala Ser Gly Glu Ser Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 118
<211> 372
<212> PRT
<213> herpes simplex virus 2

<400> 118

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

Arg Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Arg Phe
145 150 155 160

Val Leu Gly Val Ala Leu Ser Val Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Pro Pro Ser Ser Pro Arg Asp Pro Thr Pro
210 215 220

Ala Pro Gly Asp Thr Gly Thr Pro Ala Pro Ala Ser Gly Glu Arg Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 119
<211> 372
<212> PRT
<213> herpes simplex virus 2

<400> 119

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Ile Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Arg Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Pro Pro Ser Ser Pro Arg Asp Pro Thr Pro
210 215 220

Ala Pro Gly Asp Thr Gly Thr Pro Ala Pro Ala Ser Gly Glu Ser Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
    290                 295                 300

Ala Val Asn Glu Ala Ala Met Ala Arg Leu Gly Ala Glu Leu Arg Ser
305                 310                 315                 320

His Pro Asn Thr Pro Pro Lys Pro Arg Arg Arg Ser Ser Ser Ser Thr
                325                 330                 335

Thr Met Pro Ser Leu Thr Ser Ile Ala Glu Glu Ser Glu Pro Gly Pro
            340                 345                 350

Val Val Leu Leu Ser Val Ser Pro Arg Pro Arg Ser Gly Pro Thr Ala
        355                 360                 365

Pro Gln Glu Val
    370

<210>  120
<211>  372
<212>  PRT
<213>  herpes simplex virus 2

<220>
<221>  misc_feature
<222>  (215)..(219)
<223>  Xaa can be any naturally occurring amino acid

<400>  120

Met Pro Gly Arg Ser Leu Gln Gly Leu Ala Met Leu Gly Leu Trp Val
1               5                   10                  15

Cys Ala Thr Gly Leu Val Val Arg Gly Pro Thr Val Ser Leu Val Ser
            20                  25                  30

Asp Ser Leu Val Asp Ala Gly Ala Val Gly Pro Gln Gly Phe Val Glu
        35                  40                  45

Glu Asp Leu Arg Val Phe Gly Glu Leu His Phe Val Gly Ala Gln Val
    50                  55                  60

Pro His Thr Asn Tyr Tyr Asp Gly Ile Ile Glu Leu Phe His Tyr Pro
65                  70                  75                  80

Leu Gly Asn His Cys Pro Arg Val Val His Val Val Thr Leu Thr Ala
                85                  90                  95

```
Cys Pro Arg Arg Pro Ala Val Ala Phe Thr Leu Cys Arg Ser Thr His
            100                 105                 110

His Ala His Ser Pro Ala Tyr Pro Thr Leu Glu Leu Gly Leu Ala Arg
        115                 120                 125

Gln Pro Leu Leu Arg Val Arg Thr Ala Thr Arg Asp Tyr Ala Gly Leu
    130                 135                 140

Tyr Val Leu Arg Val Trp Val Gly Ser Ala Thr Asn Ala Ser Leu Phe
145                 150                 155                 160

Val Leu Gly Val Ala Leu Ser Ala Asn Gly Thr Phe Val Tyr Asn Gly
                165                 170                 175

Ser Asp Tyr Gly Ser Cys Asp Pro Ala Gln Leu Pro Phe Ser Ala Pro
            180                 185                 190

Arg Leu Gly Pro Ser Ser Val Tyr Thr Pro Gly Ala Ser Arg Pro Thr
        195                 200                 205

Pro Pro Arg Thr Thr Thr Xaa Xaa Xaa Xaa Xaa Arg Asp Pro Thr Pro
    210                 215                 220

Ala Pro Gly Asp Thr Gly Thr Pro Ala Pro Ala Ser Gly Glu Arg Ala
225                 230                 235                 240

Pro Pro Asn Ser Thr Arg Ser Ala Ser Glu Ser Arg His Arg Leu Thr
                245                 250                 255

Val Ala Gln Val Ile Gln Ile Ala Ile Pro Ala Ser Ile Ile Ala Phe
            260                 265                 270

Val Phe Leu Gly Ser Cys Ile Cys Phe Ile His Arg Cys Gln Arg Arg
        275                 280                 285

Tyr Arg Arg Pro Arg Gly Gln Ile Tyr Asn Pro Gly Gly Val Ser Cys
    290                 295                 300

Ala Val Asn Glu Ala Ala Met Ala Arg Leu Gly Ala Glu Leu Arg Ser
305                 310                 315                 320

His Pro Asn Thr Pro Pro Lys Pro Arg Arg Arg Ser Ser Ser Ser Thr
                325                 330                 335

Thr Met Pro Ser Leu Thr Ser Ile Ala Glu Glu Ser Glu Pro Gly Pro
            340                 345                 350
```

Val Val Leu Leu Ser Val Ser Pro Arg Pro Arg Ser Gly Pro Thr Ala
355 360 365

Pro Gln Glu Val
370

<210> 121
<211> 372
<212> PRT
<213> herpes simplex virus 2

<220>
<221> misc_feature
<222> (215)..(224)
<223> Xaa can be any naturally occurring amino acid

<400> 121

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Arg Phe
145 150 155 160

Val Leu Gly Val Ala Leu Ser Ala Asn Gly Thr Phe Val Tyr Asn Gly

165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Thr Thr Thr Xaa Xaa Xaa Xaa Xaa Xaa Xaa Xaa Xaa Xaa
210 215 220

Ala Pro Gly Asp Thr Gly Thr Pro Ala Pro Ala Ser Gly Glu Ile Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

Tyr Arg Arg Pro Arg Gly Gln Ile Tyr Asn Pro Gly Gly Val Ser Cys
290 295 300

Ala Val Asn Glu Ala Ala Met Ala Arg Leu Gly Ala Glu Leu Arg Ser
305 310 315 320

His Pro Asn Thr Pro Pro Lys Pro Arg Arg Arg Ser Ser Ser Ser Thr
325 330 335

Thr Met Pro Ser Leu Thr Ser Ile Ala Glu Glu Ser Glu Pro Gly Pro
340 345 350

Val Val Leu Leu Ser Val Ser Pro Arg Pro Arg Ser Gly Pro Thr Ala
355 360 365

Pro Gln Glu Val
370

<210> 122
<211> 372
<212> PRT
<213> herpes simplex virus 2

<220>
<221> misc_feature

<222> (212)..(238)
<223> Xaa can be any naturally occurring amino acid

<400> 122

Met Pro Gly Arg Ser Leu Gln Gly Leu Ala Ile Leu Gly Leu Trp Val
1 5 10 15

Cys Ala Thr Gly Leu Val Val Arg Gly Pro Thr Val Ser Leu Val Ser
20 25 30

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Val Val Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Arg Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Thr Pro Gly Ala Ser Arg Pro Thr
195 200 205

Pro Pro Arg Xaa Xaa Xaa Xaa Xaa Xaa Xaa Xaa Xaa Xaa Xaa Xaa Xaa
210 215 220

Xaa Xaa Xaa Xaa Xaa Xaa Xaa Xaa Xaa Xaa Xaa Xaa Xaa Xaa Ile Ala
225 230 235 240

```
Pro Pro Asn Ser Thr Arg Ser Ala Ser Glu Ser Arg His Arg Leu Thr
                245                 250                 255

Val Ala Gln Val Ile Gln Ile Ala Ile Pro Ala Ser Ile Ile Ala Phe
            260                 265                 270

Val Phe Leu Gly Ser Cys Ile Cys Phe Ile His Arg Cys Gln Arg Arg
        275                 280                 285

Tyr Arg Arg Pro Arg Gly Gln Ile Tyr Asn Pro Gly Gly Val Ser Cys
    290                 295                 300

Ala Val Asn Glu Ala Ala Met Ala Arg Leu Gly Ala Glu Leu Arg Ser
305                 310                 315                 320

His Pro Asn Thr Pro Pro Lys Pro Arg Arg Arg Ser Ser Ser Ser Thr
                325                 330                 335

Thr Met Pro Ser Leu Thr Ser Ile Ala Glu Glu Ser Glu Pro Gly Pro
            340                 345                 350

Val Val Leu Leu Ser Val Ser Pro Arg Pro Arg Ser Gly Pro Thr Ala
        355                 360                 365

Pro Gln Glu Val
    370


<210>  123
<211>  424
<212>  PRT
<213>  herpes simplex virus 2


<220>
<221>  misc_feature
<222>  (201)..(231)
<223>  Xaa can be any naturally occurring amino acid

<220>
<221>  misc_feature
<222>  (309)..(396)
<223>  Xaa can be any naturally occurring amino acid

<400>  123

Met Pro Gly Arg Ser Leu Gln Gly Leu Ala Ile Leu Gly Leu Trp Val
1               5                   10                  15

Cys Ala Thr Gly Leu Val Val Arg Gly Pro Thr Val Ser Leu Val Ser
            20                  25                  30
```

Asp Ser Leu Val Asp Ala Gly Ala Val Gly Pro Gln Gly Phe Val Glu
35 40 45

Glu Asp Leu Arg Val Phe Gly Glu Leu His Phe Val Gly Ala Gln Val
50 55 60

Pro His Thr Asn Tyr Tyr Asp Gly Ile Ile Glu Leu Phe His Tyr Pro
65 70 75 80

Leu Gly Asn His Cys Pro Arg Val Val His Met Ile Thr Leu Thr Ala
85 90 95

Cys Pro Arg Arg Pro Ala Val Ala Phe Thr Leu Cys Arg Ser Thr His
100 105 110

His Ala His Ser Pro Ala Tyr Pro Thr Leu Glu Leu Gly Leu Ala Arg
115 120 125

Gln Pro Leu Leu Arg Val Arg Thr Ala Thr Arg Asp Tyr Ala Gly Leu
130 135 140

Tyr Val Leu Arg Val Trp Val Gly Ser Ala Thr Asn Ala Ser Arg Phe
145 150 155 160

Val Leu Gly Val Ala Leu Ser Ala Asn Gly Thr Phe Val Tyr Asn Gly
165 170 175

Ser Asp Tyr Gly Ser Cys Asp Pro Ala Gln Leu Pro Phe Ser Ala Pro
180 185 190

Arg Leu Gly Pro Ser Ser Val Tyr Xaa Xaa Xaa Xaa Xaa Xaa Xaa Xaa
195 200 205

Xaa Xaa Xaa Xaa Xaa Xaa Xaa Xaa Xaa Xaa Xaa Xaa Xaa Xaa Xaa Xaa
210 215 220

Xaa Xaa Xaa Xaa Xaa Xaa Xaa Pro Ala Pro Ala Ser Gly Glu Arg Ala
225 230 235 240

Pro Pro Asn Ser Thr Arg Ser Ala Ser Glu Ser Arg His Arg Leu Thr
245 250 255

Val Ala Gln Val Ile Gln Ile Ala Ile Pro Ala Ser Ile Ile Ala Phe
260 265 270

Val Phe Leu Gly Ser Cys Ile Cys Phe Ile His Arg Cys Gln Arg Arg
275 280 285

```
Tyr Arg Arg Pro Arg Gly Gln Ile Tyr Asn Pro Gly Gly Val Ser Cys
    290                 295                 300

Ala Val Asn Glu Xaa Xaa Xaa Xaa Xaa Xaa Xaa Xaa Xaa Xaa Xaa Xaa
305                 310                 315                 320

Xaa Xaa Xaa Xaa Xaa Xaa Xaa Xaa Xaa Xaa Xaa Xaa Xaa Xaa Xaa Xaa
                325                 330                 335

Xaa Xaa Xaa Xaa Xaa Xaa Xaa Xaa Xaa Xaa Xaa Xaa Xaa Xaa Xaa Xaa
            340                 345                 350

Xaa Xaa Xaa Xaa Xaa Xaa Xaa Xaa Xaa Xaa Xaa Xaa Xaa Xaa Xaa Xaa
        355                 360                 365

Xaa Xaa Xaa Xaa Xaa Xaa Xaa Xaa Xaa Xaa Xaa Xaa Xaa Xaa Xaa Xaa
    370                 375                 380

Xaa Xaa Xaa Xaa Xaa Xaa Xaa Xaa Xaa Xaa Xaa Xaa Asn Lys Pro Met
385                 390                 395                 400

Leu Phe Ala Tyr Met Arg Val Leu Asp Pro Phe Val Ile Val Arg His
                405                 410                 415

Ser Pro Asp Gly Met Gly Gly Gly
            420


<210>  124
<211>  66
<212>  DNA
<213>  Artificial Sequence

<220>
<223>  GSG-P2A

<400>  124
ggaagcggag ctactaactt cagcctgctg aagcaggctg gagacgtgga ggagaaccct       60

ggacct                                                                  66


<210>  125
<211>  99
<212>  DNA
<213>  Artificial Sequence

<220>
<223>  F2A long

<400>  125
gaggcccggc acaagcagaa gatcgtggcc cccgtgaagc agaccctgaa cttcgacctg       60
```

ctgaagctgg ccggcgacgt ggagagcaac cccggcccc 99

<210> 126
<211> 24
<212> DNA
<213> Artificial Sequence

<220>
<223> Subgenomic promoter

<400> 126
ctctctacgg ctaacctgaa tgga 24

<210> 127
<211> 750
<212> DNA
<213> Artificial Sequence

<220>
<223> IRES EV71

<400> 127
tgattaaaac agctgtgggt tgttcccacc cacagggccc actgggcgct agcactctga 60

ttttacgaaa tccttgtgcg cctgttttat atcccttccc taattcgaaa cgtagaagca 120

atgcgcacca ctgatcaata gtaggcgtaa cgcgccagtt acgtcatgat caagcatatc 180

tgttcccccg gactgagtat caatagactg cttacgcggt tgaaggagaa aacgttcgtt 240

atccggctaa ctacttcgag aagcccagta acaccatgga agctgcaggg tgtttcgctc 300

agcacttccc ccgtgtagat caggtcgatg agccactgca atccccacag gtgactgtgg 360

cagtggctgc gttggcggcc tgcctatggg gagacccata ggacgctcta atgtggacat 420

ggtgcgaaga gtctattgag ctagttagta gtcctccggc ccctgaatgc ggctaatcct 480

aactgcggag cacatgcctt caacccagag ggtagtgtgt cgtaacgggc aactctgcag 540

cggaaccgac tactttgggt gtccgtgttt cttttttatt cttatattgg ctgcttatgg 600

tgacaattac agaattgtta ccatatagct attggattgg ccatccggtg tgtaatagag 660

ctgttatata cctatttgtt ggctttgtac cactaacttt aaaatctata actaccctca 720

actttatatt aaccctcaat acagttgacc 750

<210> 128
<211> 1257
<212> DNA
<213> Artificial Sequence

<220>
<223> gE P317R ectodomain codon-optimized

<400> 128
atggccaggg gagccggcct cgtgttcttc gtgggcgtgt gggtggtgag ctgtctggcc 60

gctgccccta gaaccagctg gaagagagtg acctccggag aggacgtggt gctgctccct 120

gctcccgctg gacctgagga aagaaccaga gcccacaagc tgctgtgggc tgctgagcct 180

ctggacgcct gcggacctct gagaccttcc tgggtggctc tgtggcctcc cagaagagtg 240

ctggagacag tggtggacgc cgcctgcatg agagcccctg aacccctggc catcgcctac 300

tcccccctt ttcccgccgg agacgagggc ctgtatagcg agctggcctg gagagacagg 360

gtggccgtgg tcaatgagag cctggtcatc tacggagccc tggagaccga cagcggcctg 420

tataccctga gcgtggtggg cctgagcgac gaagccagac aggtggcttc cgtcgtgctg 480

gtggtggaac ctgcccccgt ccctacacct accccctgacg actacgacga ggaggacgac 540

gccggcgtgt ccgaaagaac ccccgtgagc gtgcctcccc ccacccctcc tagaagacct 600

cctgtggctc ctcctaccca ccccagggtg atccctgagg tgagccacgt cagaggcgtg 660

accgtgcaca tggagacacc tgaggccatc ctgttcgccc ctggcgagac cttcggaacc 720

aatgtgtcca tccacgccat tgcccacgac gacggcccct acgccatgga cgtggtgtgg 780

atgaggtttg acgtgccctc cagctgcgcc gagatgagga tctacgaggc ctgcctgtac 840

cacccccagc tgcccgagtg tctgtcccct gctgacgctc cttgcgccgt cagcagctgg 900

gcctatagac tggccgtgag atcctacgct ggctgtagca gaaccaccag gccccccaga 960

tgcttcgccg aagccagaat ggagcctgtg cctggactgg cctggctggc ttccaccgtg 1020

aatctggagt ttcagcacgc cagcccccaa cacgccggac tgtacctgtg cgtggtgtac 1080

gtggacgacc acatccatgc ctggggacac atgaccatca gcaccgccgc ccagtacagg 1140

aacgctgtgg tggaacagca cctgccccag aggcaacctg agcccgtgga gcccaccaga 1200

cctcatgtga gagcccctcc ccctgctccc tccgccagag gacctctgag actgggc 1257

<210> 129
<211> 768
<212> DNA
<213> Artificial Sequence

<220>
<223> gI ectodomain codon-optimized

<400> 129
atgcctggca ggagcctgca aggcctggcc atcctgggcc tgtgggtctg tgctaccggc 60

ctggtggtga gaggacccac cgtgtccctg gtgagcgaca gcctggtgga tgccggagct 120

gtgggacctc agggcttcgt ggaagaggac ctgagggtgt tcggcgaact ccactttgtg 180

ggcgcccagg tgccccacac caactactac gacggcatca tcgagctgtt ccactacccc 240

ctgggtaatc attgtcccag agtggtgcac gtggtgaccc tgaccgcctg tcctagaagg 300

cccgctgtgg ccttcaccct gtgcagaagc acccaccacg cccacagccc tgcctacccc 360

acactggaac tgggactggc cagacagccc ctgctgaggg tgaggacagc caccagggac 420

tatgccggac tgtacgtgct gagagtgtgg gtgggaagcg ccaccaacgc ctccctgttc 480

gtgctgggcg tggctctgag cgccaatggc accttcgtgt acaacggctc cgactatggc 540

agctgtgacc ccgcccagct cccctttagc gctcctaggc tgggacctag ctccgtgtac 600

acccccggag ccagcagacc cacacccccc agaaccacca ccagccctag ctcccctcgt 660

gatcctaccc ctgcccctgg cgatacagga accccctgccc ccgctagcgg agaaagagcc 720

cctcccaaca gcaccaggtc cgccagcgag tccaggcaca ggctgaca 768

<210> 130
<211> 9993
<212> DNA
<213> Artificial

<220>
<223> SAM backbone

<400> 130
ataggcggcg catgagagaa gcccagacca attacctacc caaaatggag aaagttcacg 60

ttgacatcga ggaagacagc ccattcctca gagctttgca gcggagcttc ccgcagtttg 120

aggtagaagc caagcaggtc actgataatg accatgctaa tgccagagcg ttttcgcatc 180

tggcttcaaa actgatcgaa acggaggtgg acccatccga cacgatcctt gacattggaa 240

gtgcgcccgc ccgcagaatg tattctaagc acaagtatca ttgtatctgt ccgatgagat 300

gtgcggaaga tccggacaga ttgtataagt atgcaactaa gctgaagaaa aactgtaagg 360

aaataactga taaggaattg gacaagaaaa tgaaggagct cgccgccgtc atgagcgacc 420

ctgacctgga aactgagact atgtgcctcc acgacgacga gtcgtgtcgc tacgaagggc 480

aagtcgctgt ttaccaggat gtatacgcgg ttgacggacc gacaagtctc tatcaccaag 540

ccaataaggg agttagagtc gcctactgga taggctttga caccacccct tttatgttta 600

agaacttggc tggagcatat ccatcatact ctaccaactg ggccgacgaa accgtgttaa 660

cggctcgtaa cataggccta tgcagctctg acgttatgga gcggtcacgt agagggatgt 720

ccattcttag aaagaagtat ttgaaaccat ccaacaatgt tctattctct gttggctcga 780

ccatctacca cgagaagagg gacttactga ggagctggca cctgccgtct gtatttcact 840

tacgtggcaa gcaaaattac acatgtcggt gtgagactat agttagttgc gacgggtacg 900

tcgttaaaag aatagctatc agtccaggcc tgtatgggaa gccttcaggc tatgctgcta 960

cgatgcaccg cgagggattc ttgtgctgca aagtgacaga cacattgaac ggggagaggg 1020

tctcttttcc cgtgtgcacg tatgtgccag ctacattgtg tgaccaaatg actggcatac 1080

tggcaacaga tgtcagtgcg gacgacgcgc aaaaactgct ggttgggctc aaccagcgta 1140

tagtcgtcaa cggtcgcacc cagagaaaca ccaataccat gaaaaattac cttttgcccg 1200

tagtggccca ggcatttgct aggtgggcaa aggaatataa ggaagatcaa gaagatgaaa 1260

ggccactagg actacgagat agacagttag tcatggggtg ttgttgggct tttagaaggc 1320

acaagataac atctatttat aagcgcccgg atacccaaac catcatcaaa gtgaacagcg 1380

atttccactc attcgtgctg cccaggatag gcagtaacac attggagatc gggctgagaa 1440

caagaatcag gaaaatgtta gaggagcaca aggagccgtc acctctcatt accgccgagg 1500

acgtacaaga agctaagtgc gcagccgatg aggctaagga ggtgcgtgaa gccgaggagt 1560

tgcgcgcagc tctaccacct ttggcagctg atgttgagga gcccactctg gaagccgatg 1620

tcgacttgat gttacaagag gctggggccg gctcagtgga gacacctcgt ggcttgataa 1680

aggttaccag ctacgatggc gaggacaaga tcggctctta cgctgtgctt tctccgcagg 1740

ctgtactcaa gagtgaaaaa ttatcttgca tccaccctct cgctgaacaa gtcatagtga 1800

taacacactc tggccgaaaa gggcgttatg ccgtggaacc ataccatggt aaagtagtgg 1860

tgccagaggg acatgcaata cccgtccagg actttcaagc tctgagtgaa agtgccacca 1920

ttgtgtacaa cgaacgtgag ttcgtaaaca ggtacctgca ccatattgcc acacatggag 1980

gagcgctgaa cactgatgaa gaatattaca aaactgtcaa gcccagcgag cacgacggcg 2040

aatacctgta cgacatcgac aggaaacagt gcgtcaagaa agaactagtc actgggctag 2100

ggctcacagg cgagctggtg gatcctccct tccatgaatt cgcctacgag agtctgagaa 2160

cacgaccagc cgctccttac caagtaccaa ccataggggt gtatggcgtg ccaggatcag 2220

gcaagtctgg catcattaaa agcgcagtca ccaaaaaaga tctagtggtg agcgccaaga 2280

aagaaaactg tgcagaaatt ataaggacg tcaagaaaat gaaagggctg gacgtcaatg 2340

ccagaactgt ggactcagtg ctcttgaatg gatgcaaaca ccccgtagag accctgtata 2400

ttgacgaagc ttttgcttgt catgcaggta ctctcagagc gctcatagcc attataagac 2460

ctaaaaaggc agtgctctgc ggggatccca aacagtgcgg tttttttaac atgatgtgcc 2520

tgaaagtgca ttttaaccac gagatttgca cacaagtctt ccacaaaagc atctctcgcc 2580

gttgcactaa atctgtgact tcggtcgtct caaccttgtt ttacgacaaa aaaatgagaa 2640

cgacgaatcc gaaagagact aagattgtga ttgacactac cggcagtacc aaacctaagc 2700

aggacgatct cattctcact tgtttcagag ggtgggtgaa gcagttgcaa atagattaca 2760

aaggcaacga aataatgacg gcagctgcct ctcaagggct gacccgtaaa ggtgtgtatg 2820

ccgttcggta caaggtgaat gaaaatcctc tgtacgcacc cacctcagaa catgtgaacg 2880

tcctactgac ccgcacggag gaccgcatcg tgtggaaaac actagccggc gacccatgga 2940

taaaaacact gactgccaag taccctggga atttcactgc cacgatagag gagtggcaag 3000

cagagcatga tgccatcatg aggcacatct tggagagacc ggaccctacc gacgtcttcc 3060

agaataaggc aaacgtgtgt tgggccaagg ctttagtgcc ggtgctgaag accgctggca 3120

tagacatgac cactgaacaa tggaacactg tggattattt tgaaacggac aaagctcact 3180
cagcagagat agtattgaac caactatgcg tgaggttctt tggactcgat ctggactccg 3240
gtctattttc tgcacccact gttccgttat ccattaggaa taatcactgg gataactccc 3300
cgtcgcctaa catgtacggg ctgaataaag aagtggtccg tcagctctct cgcaggtacc 3360
cacaactgcc tcgggcagtt gccactggaa gagtctatga catgaacact ggtacactgc 3420
gcaattatga tccgcgcata aacctagtac ctgtaaacag aagactgcct catgctttag 3480
tcctccacca taatgaacac ccacagagtg acttttcttc attcgtcagc aaattgaagg 3540
gcagaactgt cctggtggtc ggggaaaagt tgtccgtccc aggcaaaatg gttgactggt 3600
tgtcagaccg gcctgaggct accttcagag ctcggctgga tttaggcatc ccaggtgatg 3660
tgcccaaata tgacataata tttgttaatg tgaggacccc atataaatac catcactatc 3720
agcagtgtga agaccatgcc attaagctta gcatgttgac caagaaagct tgtctgcatc 3780
tgaatcccgg cggaacctgt gtcagcatag gttatggtta cgctgacagg gccagcgaaa 3840
gcatcattgg tgctatagcg cggcagttca agttttcccg ggtatgcaaa ccgaaatcct 3900
cacttgaaga gacggaagtt ctgtttgtat tcattgggta cgatcgcaag gcccgtacgc 3960
acaatcctta caagctttca tcaaccttga ccaacattta tacaggttcc agactccacg 4020
aagccggatg tgcaccctca tatcatgtgg tgcgagggga tattgccacg gccaccgaag 4080
gagtgattat aaatgctgct aacagcaaag gacaacctgg cggagggtg tgcggagcgc 4140
tgtataagaa attcccggaa agcttcgatt tacagccgat cgaagtagga aaagcgcgac 4200
tggtcaaagg tgcagctaaa catatcattc atgccgtagg accaaacttc aacaaagttt 4260
cggaggttga aggtgacaaa cagttggcag aggcttatga gtccatcgct aagattgtca 4320
acgataacaa ttacaagtca gtagcgattc cactgttgtc caccggcatc ttttccggga 4380
acaaagatcg actaacccaa tcattgaacc atttgctgac agctttagac accactgatg 4440
cagatgtagc catatactgc agggacaaga aatgggaaat gactctcaag gaagcagtgg 4500
ctaggagaga agcagtggag gagatatgca tatccgacga ctcttcagtg acagaacctg 4560
atgcagagct ggtgagggtg catccgaaga gttctttggc tggaaggaag ggctacagca 4620
caagcgatgg caaaactttc tcatatttgg aagggaccaa gtttcaccag gcggccaagg 4680
atatagcaga aattaatgcc atgtggcccg ttgcaacgga ggccaatgag caggtatgca 4740
tgtatatcct cggagaaagc atgagcagta ttaggtcgaa atgccccgtc gaagagtcgg 4800
aagcctccac accacctagc acgctgcctt gcttgtgcat ccatgccatg actccagaaa 4860
gagtacagcg cctaaaagcc tcacgtccag aacaaattac tgtgtgctca tcctttccat 4920
tgccgaagta tagaatcact ggtgtgcaga agatccaatg ctcccagcct atattgttct 4980
caccgaaagt gcctgcgtat attcatccaa ggaagtatct cgtggaaaca ccaccggtag 5040

| | | | | | | |
|---|---|---|---|---|---|---|
| acgagactcc | ggagccatcg | gcagagaacc | aatccacaga | ggggacacct | gaacaaccac | 5100 |
| cacttataac | cgaggatgag | accaggacta | gaacgcctga | gccgatcatc | atcgaagagg | 5160 |
| aagaagagga | tagcataagt | ttgctgtcag | atggcccgac | ccaccaggtg | ctgcaagtcg | 5220 |
| aggcagacat | tcacgggccg | ccctctgtat | ctagctcatc | ctggtccatt | cctcatgcat | 5280 |
| ccgactttga | tgtggacagt | ttatccatac | ttgacaccct | ggagggagct | agcgtgacca | 5340 |
| gcggggcaac | gtcagccgag | actaactctt | acttcgcaaa | gagtatggag | tttctggcgc | 5400 |
| gaccggtgcc | tgcgcctcga | acagtattca | ggaaccctcc | acatcccgct | ccgcgcacaa | 5460 |
| gaacaccgtc | acttgcaccc | agcagggcct | gctcgagaac | cagcctagtt | tccaccccgc | 5520 |
| caggcgtgaa | tagggtgatc | actagagagg | agctcgaggc | gcttaccccg | tcacgcactc | 5580 |
| ctagcaggtc | ggtctcgaga | accagcctgg | tctccaaccc | gccaggcgta | aatagggtga | 5640 |
| ttacaagaga | ggagtttgag | gcgttcgtag | cacaacaaca | atgacggttt | gatgcgggtg | 5700 |
| catacatctt | ttcctccgac | accggtcaag | ggcatttaca | acaaaaatca | gtaaggcaaa | 5760 |
| cggtgctatc | cgaagtggtg | ttggagagga | ccgaattgga | gatttcgtat | gccccgcgcc | 5820 |
| tcgaccaaga | aaaagaagaa | ttactacgca | agaaattaca | gttaaatccc | acacctgcta | 5880 |
| acagaagcag | ataccagtcc | aggaaggtgg | agaacatgaa | agccataaca | gctagacgta | 5940 |
| ttctgcaagg | cctagggcat | tatttgaagg | cagaaggaaa | agtggagtgc | taccgaaccc | 6000 |
| tgcatcctgt | tcctttgtat | tcatctagtg | tgaaccgtgc | cttttcaagc | cccaaggtcg | 6060 |
| cagtggaagc | ctgtaacgcc | atgttgaaag | agaactttcc | gactgtggct | tcttactgta | 6120 |
| ttattccaga | gtacgatgcc | tatttggaca | tggttgacgg | agcttcatgc | tgcttagaca | 6180 |
| ctgccagttt | ttgccctgca | aagctgcgca | gctttccaaa | gaaacactcc | tatttggaac | 6240 |
| ccacaatacg | atcggcagtg | ccttcagcga | tccagaacac | gctccagaac | gtcctggcag | 6300 |
| ctgccacaaa | aagaaattgc | aatgtcacgc | aaatgagaga | attgcccgta | ttggattcgg | 6360 |
| cggcctttaa | tgtggaatgc | ttcaagaaat | atgcgtgtaa | taatgaatat | tgggaaacgt | 6420 |
| ttaaagaaaa | ccccatcagg | cttactgaag | aaaacgtggt | aaattacatt | accaaattaa | 6480 |
| aaggaccaaa | agctgctgct | ctttttgcga | agacacataa | tttgaatatg | ttgcaggaca | 6540 |
| taccaatgga | caggtttgta | atggacttaa | agagagacgt | gaaagtgact | ccaggaacaa | 6600 |
| aacatactga | agaacggccc | aaggtacagg | tgatccaggc | tgccgatccg | ctagcaacag | 6660 |
| cgtatctgtg | cggaatccac | cgagagctgg | ttaggagatt | aaatgcggtc | ctgcttccga | 6720 |
| acattcatac | actgtttgat | atgtcggctg | aagactttga | cgctattata | gccgagcact | 6780 |
| tccagcctgg | ggattgtgtt | ctggaaactg | acatcgcgtc | gtttgataaa | agtgaggacg | 6840 |
| acgccatggc | tctgaccgcg | ttaatgattc | tggaagactt | aggtgtggac | gcagagctgt | 6900 |

tgacgctgat tgaggcggct ttcggcgaaa tttcatcaat acatttgccc actaaaacta 6960
aatttaaatt cggagccatg atgaaatctg gaatgttcct cacactgttt gtgaacacag 7020
tcattaacat tgtaatcgca agcagagtgt tgagagaacg gctaaccgga tcaccatgtg 7080
cagcattcat tggagatgac aatatcgtga aaggagtcaa atcggacaaa ttaatggcag 7140
acaggtgcgc cacctggttg aatatggaag tcaagattat agatgctgtg gtgggcgaga 7200
aagcgcctta tttctgtgga gggtttattt tgtgtgactc cgtgaccggc acagcgtgcc 7260
gtgtggcaga cccccctaaaa aggctgttta agcttggcaa acctctggca gcagacgatg 7320
aacatgatga tgacaggaga agggcattgc atgaagagtc aacacgctgg aaccgagtgg 7380
gtattctttc agagctgtgc aaggcagtag aatcaaggta tgaaaccgta ggaacttcca 7440
tcatagttat ggccatgact actctagcta gcagtgttaa atcattcagc tacctgagag 7500
gggcccctat aactctctac ggctaacctg aatggactac gacatagtct agtccgccaa 7560
gggcgcgccc acccagcggc cgcatacagc agcaattggc aagctgctta catagaactc 7620
gcggcgattg gcatgccgcc ttaaaatttt tattttattt ttcttttctt ttccgaatcg 7680
gattttgttt ttaatatttc aaaaaaaaaa aaaaaaaaaa aaaaaaaaaa aaaaaaaaaa 7740
gaagagcgtt taaacacgtg atatctggcc tcatgggcct tcctttcact gcccgctttc 7800
cagtcgggaa acctgtcgtg ccagctgcat taacatggtc atagctgttt ccttgcgtat 7860
tgggcgctct ccgcttcctc gctcactgac tcgctgcgct cggtcgttcg ggtaaagcct 7920
ggggtgccta atgagcaaaa ggccagcaaa aggccaggaa ccgtaaaaag gccgcgttgc 7980
tggcgttttt ccataggctc cgcccccctg acgagcatca caaaaatcga cgctcaagtc 8040
agaggtggcg aaacccgaca ggactataaa gataccaggc gtttccccct ggaagctccc 8100
tcgtgcgctc tcctgttccg accctgccgc ttaccggata cctgtccgcc tttctccctt 8160
cgggaagcgt ggcgctttct catagctcac gctgtaggta tctcagttcg gtgtaggtcg 8220
ttcgctccaa gctgggctgt gtgcacgaac cccccgttca gcccgaccgc tgcgccttat 8280
ccggtaacta tcgtcttgag tccaacccgg taagacacga cttatcgcca ctggcagcag 8340
ccactggtaa caggattagc agagcgaggt atgtaggcgg tgctacagag ttcttgaagt 8400
ggtggcctaa ctacggctac actagaagaa cagtatttgg tatctgcgct ctgctgaagc 8460
cagttacctt cggaaaaaga gttggtagct cttgatccgg caaacaaacc accgctggta 8520
gcggtggttt ttttgtttgc aagcagcaga ttacgcgcag aaaaaaagga tctcaagaag 8580
atcctttgat cttttctacg gggtctgacg ctcagtggaa cgaaaactca cgttaaggga 8640
ttttggtcat gaatacacgg tgcctgactg cgttagcaat ttaactgtga taaactaccg 8700
cattaaagct tatcgatgat aagctgtcaa acatgagaat tcttagaaaa actcatcgag 8760
catcaaatga aactgcaatt tattcatatc aggattatca ataccatatt tttgaaaaag 8820

ccgtttctgt aatgaaggag aaaactcacc gaggcagttc cataggatgg caagatcctg 8880

gtatcggtct gcgattccga ctcgtccaac atcaatacaa cctattaatt tcccctcgtc 8940

aaaaataagg ttatcaagtg agaaatcacc atgagtgacg actgaatccg gtgagaatgg 9000

caaaagctta tgcatttctt tccagacttg ttcaacaggc cagccattac gctcgtcatc 9060

aaaatcactc gcatcaacca aaccgttatt cattcgtgat tgcgcctgag cgagacgaaa 9120

tacgcgatcg ctgttaaaag gacaattaca aacaggaatc gaatgcaacc ggcgcaggaa 9180

cactgccagc gcatcaacaa tattttcacc tgaatcagga tattcttcta atacctggaa 9240

tgctgttttc ccggggatcg cagtggtgag taaccatgca tcatcaggag tacggataaa 9300

atgcttgatg gtcggaagag gcataaattc cgtcagccag tttagtctga ccatctcatc 9360

tgtaacatca ttggcaacgc tacctttgcc atgtttcaga aacaactctg gcgcatcggg 9420

cttcccatac aatcgataga ttgtcgcacc tgattgcccg acattatcgc gagcccattt 9480

atacccatat aaatcagcat ccatgttgga atttaatcgc ggcctcgagc aagacgtttc 9540

ccgttgaata tggctcataa caccccttgt attactgttt atgtaagcag acagttttat 9600

tgttcatgag cggatacata tttgaatgta tttagaaaaa taaacaaata ggggttccgc 9660

gcacatttcc ccgaaaagtg ccacctaaat tgtaagcgtt aatattttgt taaaattcgc 9720

gttaaatttt tgttaaatca gctcattttt taaccaatag gccgaaatcg gcaaaatccc 9780

ttataaatca aaagaataga ccgagatagg gttgagtggc cgctacaggg cgctcccatt 9840

cgccattcag gctgcgcaac tgttgggaag ggcgtttcgg tgcgggcctc ttcgctatta 9900

cgccagctgg cgaaaggggg atgtgctgca aggcgattaa gttgggtaac gccagggttt 9960

tcccagtcac acgcgtaata cgactcacta tag 9993

<210> 131
<211> 7561
<212> DNA
<213> Artificial

<220>
<223> 5′ region of SEQ ID NO:130

<400> 131
ataggcggcg catgagagaa gcccagacca attacctacc caaaatggag aaagttcacg 60

ttgacatcga ggaagacagc ccattcctca gagctttgca gcggagcttc ccgcagtttg 120

aggtagaagc caagcaggtc actgataatg accatgctaa tgccagagcg ttttcgcatc 180

tggcttcaaa actgatcgaa acggaggtgg acccatccga cacgatcctt gacattggaa 240

gtgcgcccgc ccgcagaatg tattctaagc acaagtatca ttgtatctgt ccgatgagat 300

gtgcggaaga tccggacaga ttgtataagt atgcaactaa gctgaagaaa aactgtaagg 360

aaataactga taaggaattg gacaagaaaa tgaaggagct cgccgccgtc atgagcgacc 420
ctgacctgga aactgagact atgtgcctcc acgacgacga gtcgtgtcgc tacgaagggc 480
aagtcgctgt ttaccaggat gtatacgcgg ttgacggacc gacaagtctc tatcaccaag 540
ccaataaggg agttagagtc gcctactgga taggctttga caccacccct tttatgttta 600
agaacttggc tggagcatat ccatcatact ctaccaactg ggccgacgaa accgtgttaa 660
cggctcgtaa cataggccta tgcagctctg acgttatgga gcggtcacgt agagggatgt 720
ccattcttag aaagaagtat ttgaaaccat ccaacaatgt tctattctct gttggctcga 780
ccatctacca cgagaagagg gacttactga ggagctggca cctgccgtct gtatttcact 840
tacgtggcaa gcaaaattac acatgtcggt gtgagactat agttagttgc gacgggtacg 900
tcgttaaaag aatagctatc agtccaggcc tgtatgggaa gccttcaggc tatgctgcta 960
cgatgcaccg cgagggattc ttgtgctgca aagtgacaga cacattgaac ggggagaggg 1020
tctcttttcc cgtgtgcacg tatgtgccag ctacattgtg tgaccaaatg actggcatac 1080
tggcaacaga tgtcagtgcg gacgacgcgc aaaaactgct ggttgggctc aaccagcgta 1140
tagtcgtcaa cggtcgcacc cagagaaaca ccaataccat gaaaaattac cttttgcccg 1200
tagtggccca ggcatttgct aggtgggcaa aggaatataa ggaagatcaa gaagatgaaa 1260
ggccactagg actacgagat agacagttag tcatggggtg ttgttgggct tttagaaggc 1320
acaagataac atctatttat aagcgcccgg atacccaaac catcatcaaa gtgaacagcg 1380
atttccactc attcgtgctg cccaggatag gcagtaacac attggagatc gggctgagaa 1440
caagaatcag gaaaatgtta gaggagcaca aggagccgtc acctctcatt accgccgagg 1500
acgtacaaga agctaagtgc gcagccgatg aggctaagga ggtgcgtgaa gccgaggagt 1560
tgcgcgcagc tctaccacct ttggcagctg atgttgagga gcccactctg gaagccgatg 1620
tcgacttgat gttacaagag gctggggccg gctcagtgga gacacctcgt ggcttgataa 1680
aggttaccag ctacgatggc gaggacaaga tcggctctta cgctgtgctt tctccgcagg 1740
ctgtactcaa gagtgaaaaa ttatcttgca tccaccctct cgctgaacaa gtcatagtga 1800
taacacactc tggccgaaaa gggcgttatg ccgtggaacc ataccatggt aaagtagtgg 1860
tgccagaggg acatgcaata cccgtccagg actttcaagc tctgagtgaa agtgccacca 1920
ttgtgtacaa cgaacgtgag ttcgtaaaca ggtacctgca ccatattgcc acacatggag 1980
gagcgctgaa cactgatgaa gaatattaca aaactgtcaa gcccagcgag cacgacggcg 2040
aatacctgta cgacatcgac aggaaacagt gcgtcaagaa agaactagtc actgggctag 2100
ggctcacagg cgagctggtg gatcctccct tccatgaatt cgcctacgag agtctgagaa 2160
cacgaccagc cgctccttac caagtaccaa ccataggggt gtatggcgtg ccaggatcag 2220
gcaagtctgg catcattaaa agcgcagtca ccaaaaaaga tctagtggtg agcgccaaga 2280

aagaaaactg tgcagaaatt ataagggacg tcaagaaaat gaaagggctg gacgtcaatg 2340
ccagaactgt ggactcagtg ctcttgaatg gatgcaaaca ccccgtagag accctgtata 2400
ttgacgaagc ttttgcttgt catgcaggta ctctcagagc gctcatagcc attataagac 2460
ctaaaaaggc agtgctctgc ggggatccca aacagtgcgg ttttttaac atgatgtgcc 2520
tgaaagtgca ttttaaccac gagatttgca cacaagtctt ccacaaaagc atctctcgcc 2580
gttgcactaa atctgtgact tcggtcgtct caaccttgtt ttacgacaaa aaaatgagaa 2640
cgacgaatcc gaaagagact aagattgtga ttgacactac cggcagtacc aaacctaagc 2700
aggacgatct cattctcact tgtttcagag ggtgggtgaa gcagttgcaa atagattaca 2760
aaggcaacga aataatgacg gcagctgcct ctcaagggct gacccgtaaa ggtgtgtatg 2820
ccgttcggta caaggtgaat gaaaatcctc tgtacgcacc cacctcagaa catgtgaacg 2880
tcctactgac ccgcacggag gaccgcatcg tgtggaaaac actagccggc gacccatgga 2940
taaaaacact gactgccaag taccctggga atttcactgc cacgatagag gagtggcaag 3000
cagagcatga tgccatcatg aggcacatct tggagagacc ggaccctacc gacgtcttcc 3060
agaataaggc aaacgtgtgt tgggccaagg ctttagtgcc ggtgctgaag accgctggca 3120
tagacatgac cactgaacaa tggaacactg tggattattt tgaaacggac aaagctcact 3180
cagcagagat agtattgaac caactatgcg tgaggttctt tggactcgat ctggactccg 3240
gtctattttc tgcacccact gttccgttat ccattaggaa taatcactgg gataactccc 3300
cgtcgcctaa catgtacggg ctgaataaag aagtggtccg tcagctctct cgcaggtacc 3360
cacaactgcc tcgggcagtt gccactggaa gagtctatga catgaacact ggtacactgc 3420
gcaattatga tccgcgcata aacctagtac ctgtaaacag aagactgcct catgctttag 3480
tcctccacca taatgaacac ccacagagtg acttttcttc attcgtcagc aaattgaagg 3540
gcagaactgt cctggtggtc ggggaaaagt tgtccgtccc aggcaaaatg gttgactggt 3600
tgtcagaccg gcctgaggct accttcagag ctcggctgga tttaggcatc ccaggtgatg 3660
tgcccaaata tgacataata tttgttaatg tgaggacccc atataaatac catcactatc 3720
agcagtgtga agaccatgcc attaagctta gcatgttgac caagaaagct tgtctgcatc 3780
tgaatcccgg cggaacctgt gtcagcatag gttatggtta cgctgacagg gccagcgaaa 3840
gcatcattgg tgctatagcg cggcagttca agttttcccg ggtatgcaaa ccgaaatcct 3900
cacttgaaga gacggaagtt ctgtttgtat tcattgggta cgatcgcaag gcccgtacgc 3960
acaatcctta caagctttca tcaaccttga ccaacattta tacaggttcc agactccacg 4020
aagccggatg tgcaccctca tatcatgtgg tgcgagggga tattgccacg gccaccgaag 4080
gagtgattat aaatgctgct aacagcaaag gacaacctgg cggaggggtg tgcggagcgc 4140

tgtataagaa attcccggaa agcttcgatt tacagccgat cgaagtagga aaagcgcgac 4200
tggtcaaagg tgcagctaaa catatcattc atgccgtagg accaaacttc aacaaagttt 4260
cggaggttga aggtgacaaa cagttggcag aggcttatga gtccatcgct aagattgtca 4320
acgataacaa ttacaagtca gtagcgattc cactgttgtc caccggcatc ttttccggga 4380
acaaagatcg actaacccaa tcattgaacc atttgctgac agctttagac accactgatg 4440
cagatgtagc catatactgc agggacaaga aatgggaaat gactctcaag gaagcagtgg 4500
ctaggagaga agcagtggag gagatatgca tatccgacga ctcttcagtg acagaacctg 4560
atgcagagct ggtgagggtg catccgaaga gttctttggc tggaaggaag ggctacagca 4620
caagcgatgg caaaactttc tcatatttgg aagggaccaa gtttcaccag gcggccaagg 4680
atatagcaga aattaatgcc atgtggcccg ttgcaacgga ggccaatgag caggtatgca 4740
tgtatatcct cggagaaagc atgagcagta ttaggtcgaa atgccccgtc gaagagtcgg 4800
aagcctccac accacctagc acgctgcctt gcttgtgcat ccatgccatg actccagaaa 4860
gagtacagcg cctaaaagcc tcacgtccag aacaaattac tgtgtgctca tcctttccat 4920
tgccgaagta tagaatcact ggtgtgcaga agatccaatg ctcccagcct atattgttct 4980
caccgaaagt gcctgcgtat attcatccaa ggaagtatct cgtggaaaca ccaccggtag 5040
acgagactcc ggagccatcg gcagagaacc aatccacaga ggggacacct gaacaaccac 5100
cacttataac cgaggatgag accaggacta gaacgcctga gccgatcatc atcgaagagg 5160
aagaagagga tagcataagt ttgctgtcag atggcccgac ccaccaggtg ctgcaagtcg 5220
aggcagacat tcacgggccg ccctctgtat ctagctcatc ctggtccatt cctcatgcat 5280
ccgactttga tgtggacagt ttatccatac ttgacaccct ggagggagct agcgtgacca 5340
gcggggcaac gtcagccgag actaactctt acttcgcaaa gagtatggag tttctggcgc 5400
gaccggtgcc tgcgcctcga acagtattca ggaaccctcc acatcccgct ccgcgcacaa 5460
gaacaccgtc acttgcaccc agcagggcct gctcgagaac cagcctagtt tccaccccgc 5520
caggcgtgaa tagggtgatc actagagagg agctcgaggc gcttaccccg tcacgcactc 5580
ctagcaggtc ggtctcgaga accagcctgg tctccaaccc gccaggcgta aatagggtga 5640
ttacaagaga ggagtttgag gcgttcgtag cacaacaaca atgacggttt gatgcgggtg 5700
catacatctt ttcctccgac accggtcaag ggcatttaca acaaaaatca gtaaggcaaa 5760
cggtgctatc cgaagtggtg ttggagagga ccgaattgga gatttcgtat gccccgcgcc 5820
tcgaccaaga aaaagaagaa ttactacgca agaaattaca gttaaatccc acacctgcta 5880
acagaagcag ataccagtcc aggaaggtgg agaacatgaa agccataaca gctagacgta 5940
ttctgcaagg cctagggcat tatttgaagg cagaaggaaa agtggagtgc taccgaaccc 6000
tgcatcctgt tcctttgtat tcatctagtg tgaaccgtgc cttttcaagc cccaaggtcg 6060

cagtggaagc ctgtaacgcc atgttgaaag agaactttcc gactgtggct tcttactgta 6120

ttattccaga gtacgatgcc tatttggaca tggttgacgg agcttcatgc tgcttagaca 6180

ctgccagttt ttgccctgca aagctgcgca gctttccaaa gaaacactcc tatttggaac 6240

ccacaatacg atcggcagtg ccttcagcga tccagaacac gctccagaac gtcctggcag 6300

ctgccacaaa aagaaattgc aatgtcacgc aaatgagaga attgcccgta ttggattcgg 6360

cggcctttaa tgtggaatgc ttcaagaaat atgcgtgtaa taatgaatat tgggaaacgt 6420

ttaaagaaaa ccccatcagg cttactgaag aaaacgtggt aaattacatt accaaattaa 6480

aaggaccaaa agctgctgct ctttttgcga agacacataa tttgaatatg ttgcaggaca 6540

taccaatgga caggtttgta atggacttaa agagagacgt gaaagtgact ccaggaacaa 6600

aacatactga agaacggccc aaggtacagg tgatccaggc tgccgatccg ctagcaacag 6660

cgtatctgtg cggaatccac cgagagctgg ttaggagatt aaatgcggtc ctgcttccga 6720

acattcatac actgtttgat atgtcggctg aagactttga cgctattata gccgagcact 6780

tccagcctgg ggattgtgtt ctggaaactg acatcgcgtc gtttgataaa agtgaggacg 6840

acgccatggc tctgaccgcg ttaatgattc tggaagactt aggtgtggac gcagagctgt 6900

tgacgctgat tgaggcggct ttcggcgaaa tttcatcaat acatttgccc actaaaacta 6960

aatttaaatt cggagccatg atgaaatctg gaatgttcct cacactgttt gtgaacacag 7020

tcattaacat tgtaatcgca agcagagtgt tgagagaacg gctaaccgga tcaccatgtg 7080

cagcattcat tggagatgac aatatcgtga aaggagtcaa atcggacaaa ttaatggcag 7140

acaggtgcgc cacctggttg aatatggaag tcaagattat agatgctgtg gtgggcgaga 7200

aagcgcctta tttctgtgga gggtttattt tgtgtgactc cgtgaccggc acagcgtgcc 7260

gtgtggcaga ccccctaaaa aggctgttta agcttggcaa acctctggca gcagacgatg 7320

aacatgatga tgacaggaga agggcattgc atgaagagtc aacacgctgg aaccgagtgg 7380

gtattctttc agagctgtgc aaggcagtag aatcaaggta tgaaaccgta ggaacttcca 7440

tcatagttat ggccatgact actctagcta gcagtgttaa atcattcagc tacctgagag 7500

gggcccctat aactctctac ggctaacctg aatggactac gacatagtct agtccgccaa 7560

g 7561

<210> 132
<211> 2439
<212> DNA
<213> Artificial

<220>
<223> 3′ region of SEQ ID NO:130

<400> 132

```
tctagacggc gcgcccaccc agcggccgca tacagcagca attggcaagc tgcttacata      60
gaactcgcgg cgattggcat gccgccttaa aatttttatt ttatttttct tttcttttcc     120
gaatcggatt ttgtttttaa tatttcaaaa aaaaaaaaaa aaaaaaaaaa aaaaaaaaaa     180
aaaaaagaag agcgtttaaa cacgtgatat ctggcctcat gggccttcct ttcactgccc     240
gctttccagt cgggaaacct gtcgtgccag ctgcattaac atggtcatag ctgtttcctt     300
gcgtattggg cgctctccgc ttcctcgctc actgactcgc tgcgctcggt cgttcgggta     360
aagcctgggg tgcctaatga gcaaaaggcc agcaaaaggc caggaaccgt aaaaaggccg     420
cgttgctggc gtttttccat aggctccgcc cccctgacga gcatcacaaa aatcgacgct     480
caagtcagag gtggcgaaac ccgacaggac tataaagata ccaggcgttt ccccctggaa     540
gctccctcgt gcgctctcct gttccgaccc tgccgcttac cggatacctg tccgcctttc     600
tcccttcggg aagcgtggcg ctttctcata gctcacgctg taggtatctc agttcggtgt     660
aggtcgttcg ctccaagctg ggctgtgtgc acgaaccccc cgttcagccc gaccgctgcg     720
ccttatccgg taactatcgt cttgagtcca acccggtaag acacgactta tcgccactgg     780
cagcagccac tggtaacagg attagcagag cgaggtatgt aggcggtgct acagagttct     840
tgaagtggtg gcctaactac ggctacacta gaagaacagt atttggtatc tgcgctctgc     900
tgaagccagt taccttcgga aaaagagttg gtagctcttg atccggcaaa caaaccaccg     960
ctggtagcgg tggttttttt gtttgcaagc agcagattac gcgcagaaaa aaaggatctc    1020
aagaagatcc tttgatcttt tctacggggt ctgacgctca gtggaacgaa aactcacgtt    1080
aagggatttt ggtcatgaat acacggtgcc tgactgcgtt agcaatttaa ctgtgataaa    1140
ctaccgcatt aaagcttatc gatgataagc tgtcaaacat gagaattctt agaaaaactc    1200
atcgagcatc aaatgaaact gcaatttatt catatcagga ttatcaatac catatttttg    1260
aaaaagccgt ttctgtaatg aaggagaaaa ctcaccgagg cagttccata ggatggcaag    1320
atcctggtat cggtctgcga ttccgactcg tccaacatca atacaaccta ttaatttccc    1380
ctcgtcaaaa ataaggttat caagtgagaa atcaccatga gtgacgactg aatccggtga    1440
gaatggcaaa agcttatgca tttctttcca gacttgttca acaggccagc cattacgctc    1500
gtcatcaaaa tcactcgcat caaccaaacc gttattcatt cgtgattgcg cctgagcgag    1560
acgaaatacg cgatcgctgt taaaaggaca attacaaaca ggaatcgaat gcaaccggcg    1620
caggaacact gccagcgcat caacaatatt ttcacctgaa tcaggatatt cttctaatac    1680
ctggaatgct gttttcccgg ggatcgcagt ggtgagtaac catgcatcat caggagtacg    1740
gataaaatgc ttgatggtcg gaagaggcat aaattccgtc agccagttta gtctgaccat    1800
ctcatctgta acatcattgg caacgctacc tttgccatgt ttcagaaaca actctggcgc    1860
atcgggcttc ccatacaatc gatagattgt cgcacctgat tgcccgacat tatcgcgagc    1920
```

ccatttatac ccatataaat cagcatccat gttggaattt aatcgcggcc tcgagcaaga 1980

cgtttcccgt tgaatatggc tcataacacc ccttgtatta ctgtttatgt aagcagacag 2040

ttttattgtt catgagcgga tacatatttg aatgtattta gaaaaataaa caaatagggg 2100

ttccgcgcac atttccccga aaagtgccac ctaaattgta agcgttaata ttttgttaaa 2160

attcgcgtta aatttttgtt aaatcagctc attttttaac caataggccg aaatcggcaa 2220

aatcccttat aaatcaaaag aatagaccga gatagggttg agtggccgct acagggcgct 2280

cccattcgcc attcaggctg cgcaactgtt gggaagggcg tttcggtgcg ggcctcttcg 2340

ctattacgcc agctggcgaa agggggatgt gctgcaaggc gattaagttg ggtaacgcca 2400

gggttttccc agtcacacgc gtaatacgac tcactatag 2439

<210> 133
<211> 7747
<212> RNA
<213> Artificial

<220>
<223> SAM backbone

<400> 133
auaggcggcg caugagagaa gcccagacca auuaccuacc caaaauggag aaaguucacg 60

uugacaucga ggaagacagc ccauuccuca gagcuuugca gcggagcuuc ccgcaguuug 120

agguagaagc caagcagguc acugauaaug accaugcuaa ugccagagcg uuuucgcauc 180

uggcuucaaa acugaucgaa acggaggugg acccauccga cacgauccuu gacauuggaa 240

gugcgcccgc ccgcagaaug uauucuaagc acaaguauca uuguaucugu ccgaugagau 300

gugcggaaga uccggacaga uuguauaagu augcaacuaa gcugaagaaa aacuguaagg 360

aaauaacuga uaaggaauug gacaagaaaa ugaaggagcu cgccgccguc augagcgacc 420

cugaccugga aacugagacu augugccucc acgacgacga gucgugucgc uacgaagggc 480

aagucgcugu uuaccaggau guauacgcgg uugacggacc gacaagucuc uaucaccaag 540

ccaauaaggg aguuagaguc gccuacugga uaggcuuuga caccaccccu uuuauguuua 600

agaacuuggc uggagcauau ccaucauacu cuaccaacug ggccgacgaa accguguuaa 660

cggcucguaa cauaggccua ugcagcucug acguuaugga gcggucacgu agagggaugu 720

ccauucuuag aaagaaguau uugaaaccau ccaacaaugu ucuauucucu guuggcucga 780

ccaucuacca cgagaagagg gacuuacuga ggagcuggca ccugccgucu guauuucacu 840

uacguggcaa gcaaaauuac acaugucggu gugagacuau aguuaguugc gacggguacg 900

ucguuaaaag aauagcuauc aguccaggcc uguaugggaa gccuucaggc uaugcugcua 960

cgaugcaccg cgagggauuc uugugcugca aagugacaga cacauugaac ggggagaggg 1020

ucucuuuucc cgugugcacg uaugugccag cuacauugug ugaccaaaug acuggcauac 1080

uggcaacaga ugucagugcg gacgacgcgc aaaaacugcu gguugggcuc aaccagcgua 1140

uagucgucaa cggucgcacc cagagaaaca ccaauaccau gaaaaauuac cuuuugcccg 1200

uaguggccca ggcauuugcu aggugggcaa aggaauauaa ggaagaucaa gaagaugaaa 1260

ggccacuagg acuacgagau agacaguuag ucaugggug uuguugggcu uuuagaaggc 1320

acaagauaac aucuauuuau aagcgcccgg auacccaaac caucaucaaa gugaacagcg 1380

auuuccacuc auucgugcug cccaggauag gcaguaacac auuggagauc gggcugagaa 1440

caagaaucag gaaaauguua gaggagcaca aggagccguc accucucauu accgccgagg 1500

acguacaaga agcuaagugc gcagccgaug aggcuaagga ggugcgugaa gccgaggagu 1560

ugcgcgcagc ucuaccaccu uuggcagcug auguugagga gcccacucug gaagccgaug 1620

ucgacuugau guuacaagag gcuggggccg gcucagugga gacaccucgu ggcuugauaa 1680

agguuaccag cuacgauggc gaggacaaga ucggcucuua cgcugugcuu ucuccgcagg 1740

cuguacucaa gagugaaaaa uuaucuugca uccacccucu cgcugaacaa gucauaguga 1800

uaacacacuc uggccgaaaa gggcguuaug ccguggaacc auaccauggu aaaguagugg 1860

ugccagaggg acaugcaaua cccguccagg acuuucaagc ucugagugaa agugccacca 1920

uuguguacaa cgaacgugag uucguaaaca gguaccugca ccauauugcc acacauggag 1980

gagcgcugaa cacugaugaa gaauauuaca aaacugucaa gcccagcgag cacgacggcg 2040

aauaccugua cgacaucgac aggaaacagu gcgucaagaa agaacuaguc acugggcuag 2100

ggcucacagg cgagcuggug gauccucccu uccaugaauu cgccuacgag agucugagaa 2160

cacgaccagc cgcuccuuac caaguaccaa ccauaggggu guauggcgug ccaggaucag 2220

gcaagucugg caucauuaaa agcgcaguca ccaaaaaaga ucuaguggug agcgccaaga 2280

aagaaaacug ugcagaaauu auaagggacg ucaagaaaau gaaagggcug gacgucaaug 2340

ccagaacugu ggacucagug cucuugaaug gaugcaaaca ccccguagag acccuguaua 2400

uugacgaagc uuuugcuugu caugcaggua cucucagagc gcucauagcc auuauaagac 2460

cuaaaaaggc agugcucugc ggggauccca aacagugcgg uuuuuuuaac augaugugcc 2520

ugaaagugca uuuuaaccac gagauuugca cacaagucuu ccacaaaagc aucucucgcc 2580

guugcacuaa aucugugacu ucggucgucu caaccuuguu uuacgacaaa aaaaugagaa 2640

cgacgaaucc gaaagagacu aagauuguga uugacacuac cggcaguacc aaaccuaagc 2700

aggacgaucu cauucucacu uguuucagag ggugggugaa gcaguugcaa auagauuaca 2760

aaggcaacga aauaaugacg gcagcugccu cucaagggcu gacccguaaa gguguguaug 2820

ccguucggua caaggugaau gaaaauccuc uguacgcacc caccucagaa caugugaacg 2880

uccuacugac ccgcacggag gaccgcaucg uguggaaaac acuagccggc gacccaugga 2940

uaaaaacacu gacugccaag uacccuggga auuucacugc cacgauagag gaguggcaag 3000
cagagcauga ugccaucaug aggcacaucu uggagagacc ggacccuacc gacgucuucc 3060
agaauaaggc aaacgugugu ugggccaagg cuuuagugcc ggugcugaag accgcuggca 3120
uagacaugac cacugaacaa uggaacacug uggauuauuu ugaaacggac aaagcucacu 3180
cagcagagau aguauugaac caacuaugcg ugagguucuu uggacucgau cuggacuccg 3240
gucuauuuuc ugcacccacu guuccguuau ccauuaggaa uaaucacugg gauaacuccc 3300
cgucgccuaa cauguacggg cugaauaaag aagugguccg ucagcucucu cgcagguacc 3360
cacaacugcc ucgggcaguu gccacuggaa gagucuauga caugaacacu gguacacugc 3420
gcaauuauga uccgcgcaua aaccuaguac cuguaaacag aagacugccu caugcuuuag 3480
uccuccacca uaaugaacac ccacagagug acuuuucuuc auucgucagc aaauugaagg 3540
gcagaacugu ccuggugguc ggggaaaagu uguccguccc aggcaaaaug guugacuggu 3600
ugucagaccg gccugaggcu accuucagag cucggcugga uuuaggcauc ccaggugaug 3660
ugcccaaaua ugacauaaua uuuguuaaug ugaggacccc auauaaauac caucacuauc 3720
agcaguguga agaccaugcc auuaagcuua gcauguugac caagaaagcu ugucugcauc 3780
ugaaucccgg cggaaccugu gucagcauag guuaugguua cgcugacagg gccagcgaaa 3840
gcaucauugg ugcuauagcg cggcaguuca aguuucccgg gguaugcaaa ccgaaauccu 3900
cacuugaaga gacggaaguu cuguuuguau ucauugggua cgaucgcaag gcccguacgc 3960
acaauccuua caagcuuuca ucaaccuuga ccaacauuua uacagguucc agacuccacg 4020
aagccggaug ugcacccuca uaucaugugg ugcgagggga uauugccacg gccaccgaag 4080
gagugauuau aaaugcugcu aacagcaaag gacaaccugg cggagggguc ugcggagcgc 4140
uguauaagaa auucccggaa agcuucgauu uacagccgau cgaaguagga aaagcgcgac 4200
uggucaaagg ugcagcuaaa cauaucauuc augccguagg accaaacuuc aacaaaguuu 4260
cggagguuga aggugacaaa caguuggcag aggcuuauga guccaucgcu aagauuguca 4320
acgauaacaa uuacaaguca guagcgauuc cacuguuguc caccggcauc uuuuccggga 4380
acaaagaucg acuaacccaa ucauugaacc auuugcugac agcuuuagac accacugaug 4440
cagauguagc cauauacugc agggacaaga aaugggaaau gacucucaag gaagcagugg 4500
cuaggagaga agcaguggag gagauaugca uauccgacga cucuucagug acagaaccug 4560
augcagagcu ggugagggug cauccgaaga guucuuuggc uggaaggaag ggcuacagca 4620
caagcgaugg caaaacuuuc ucauauuugg aagggaccaa guuucaccag gcggccaagg 4680
auauagcaga aauuaaugcc auguggcccg uugcaacgga ggccaaugag cagguaugca 4740
uguauauccu cggagaaagc augagcagua uuaggucgaa augccccguc gaagagucgg 4800

aagccuccac accaccuagc acgcugccuu gcuugugcau ccaugccaug acuccagaaa 4860

gaguacagcg ccuaaaagcc ucacguccag aacaaauuac ugugugcuca uccuuuccau 4920

ugccgaagua uagaaucacu ggugugcaga agauccaaug cucccagccu auauuguucu 4980

caccgaaagu gccugcguau auucauccaa ggaaguaucu cguggaaaca ccaccgguag 5040

acgagacucc ggagccaucg gcagagaacc aauccacaga ggggacaccu gaacaaccac 5100

cacuuauaac cgaggaugag accaggacua gaacgccuga gccgaucauc aucgaagagg 5160

aagaagagga uagcauaagu uugcugucag auggcccgac ccaccaggug cugcaagucg 5220

aggcagacau ucacgggccg cccucuguau cuagcucauc cugguccauu ccucaugcau 5280

ccgacuuuga uguggacagu uuauccauac uugacacccu ggagggagcu agcgugacca 5340

gcggggcaac gucagccgag acuaacucuu acuucgcaaa gaguauggag uuucuggcgc 5400

gaccggugcc ugcgccucga acaguauuca ggaacccucc acaucccgcu ccgcgcacaa 5460

gaacaccguc acuugcaccc agcagggccu gcucgagaac cagccuaguu uccaccccgc 5520

caggcgugaa uagggugauc acuagagagg agcucgaggc gcuuaccccg ucacgcacuc 5580

cuagcagguc ggucucgaga accagccugg ucuccaaccc gccaggcgua aauaggguga 5640

uuacaagaga ggaguuugag gcguucguag cacaacaaca augacgguuu gaugcgggug 5700

cauacaucuu uuccuccgac accggucaag ggcauuuaca acaaaaauca guaaggcaaa 5760

cggugcuauc cgaaguggug uuggagagga ccgaauugga gauuucguau gccccgcgcc 5820

ucgaccaaga aaaagaagaa uuacuacgca agaaauuaca guuaaauccc acaccugcua 5880

acagaagcag auaccagucc aggaaggugg agaacaugaa agccauaaca gcuagacgua 5940

uucugcaagg ccuagggcau uauuugaagg cagaaggaaa aguggagugc uaccgaaccc 6000

ugcauccugu uccuuuguau ucaucuagug ugaaccgugc cuuuucaagc cccaaggucg 6060

caguggaagc cuguaacgcc auguugaaag agaacuuucc gacuguggcu ucuuacugua 6120

uuauuccaga guacgaugcc uauuuggaca ugguugacgg agcuucaugc ugcuuagaca 6180

cugccaguuu uugcccugca aagcugcgca gcuuuccaaa gaaacacucc uauuuggaac 6240

ccacaauacg aucggcagug ccuucagcga uccagaacac gcuccagaac guccuggcag 6300

cugccacaaa aagaaauugc aaugucacgc aaaugagaga auugcccgua uuggauucgg 6360

cggccuuuaa uguggaaugc uucaagaaau augcguguaa uaaugaauau ugggaaacgu 6420

uuaaagaaaa ccccaucagg cuuacugaag aaaacguggu aaauuacauu accaaauuaa 6480

aaggaccaaa agcugcugcu cuuuuugcga agacacauaa uuugaauaug uugcaggaca 6540

uaccaaugga cagguuugua auggacuuaa agagagacgu gaaagugacu ccaggaacaa 6600

aacauacuga agaacggccc aagguacagg ugauccaggc ugccgauccg cuagcaacag 6660

cguaucugug cggaauccac cgagagcugg uuaggagauu aaaugcgguc cugcuuccga 6720

acauucauac acuguuugau augucggcug aagacuuuga cgcuauuaua gccgagcacu 6780

uccagccugg ggauuguguu cuggaaacug acaucgcguc guuugauaaa agugaggacg 6840

acgccauggc ucugaccgcg uuaaugauuc uggaagacuu aggugugggac gcagagcugu 6900

ugacgcugau ugaggcggcu uucggcgaaa uuucaucaau acauuugccc acuaaaacua 6960

aauuuaaauu cggagccaug augaaaucug gaauguuccu cacacuguuu gugaacacag 7020

ucauuaacau uguaaucgca agcagagugu ugagagaacg gcuaaccgga ucaccaugug 7080

cagcauucau uggagaugac aauaucguga aaggagucaa aucggacaaa uuaauggcag 7140

acaggugcgc caccugguug aauauggaag ucaagauuau agaugcugug gugggcgaga 7200

aagcgccuua uuucugugga ggguuuauuu ugugugacuc cgugaccggc acagcgugcc 7260

guguggcaga cccccuaaaa aggcuguuua agcuuggcaa accucuggca gcagacgaug 7320

aacaugauga ugacaggaga agggcauugc augaagaguc aacacgcugg aaccgagugg 7380

guauucuuuc agagcugugc aaggcaguag aaucaaggua ugaaaccgua ggaacuucca 7440

ucauaguuau ggccaugacu acucuagcua gcaguguuaa aucauucagc uaccugagag 7500

gggccccuau aacucucuac ggcuaaccug aauggacuac gacauagucu aguccgccaa 7560

gucuagacgg cgcgcccacc cagcggccgc auacagcagc aauuggcaag cugcuuacau 7620

agaacucgcg gcgauuggca ugccgccuua aaauuuuuau uuuauuuuuc uuuucuuuuc 7680

cgaaucggau uuuguuuuua auauuucaaa aaaaaaaaaa aaaaaaaaaa aaaaaaaaaa 7740

aaaaaaa 7747

<210> 134
<211> 7561
<212> RNA
<213> Artificial

<220>
<223> 5′ region of SEQ ID NO:133

<400> 134
auaggcggcg caugagagaa gcccagacca auuaccuacc caaaauggag aaaguucacg 60

uugacaucga ggaagacagc ccauuccuca gagcuuugca gcggagcuuc ccgcaguuug 120

agguagaagc caagcagguc acugauaaug accaugcuaa ugccagagcg uuuucgcauc 180

uggcuucaaa acugaucgaa acggaggugg acccauccga cacgauccuu gacauuggaa 240

gugcgcccgc ccgcagaaug uauucuaagc acaaguauca uuguaucugu ccgaugagau 300

gugcggaaga uccggacaga uuguauaagu augcaacuaa gcugaagaaa aacuguaagg 360

aaauaacuga uaaggaauug gacaagaaaa ugaaggagcu cgccgccguc augagcgacc 420

cugaccugga aacugagacu augugccucc acgacgacga gucgugucgc uacgaagggc 480

aagucgcugu uuaccaggau guauacgcgg uugacggacc gacaagucuc uaucaccaag 540
ccaauaaggg aguuagaguc gccuacugga uaggcuuuga caccaccccu uuuauguuua 600
agaacuuggc uggagcauau ccaucauacu cuaccaacug ggccgacgaa accguguuaa 660
cggcucguaa cauaggccua ugcagcucug acguuaugga gcggucacgu agagggaugu 720
ccauucuuag aaagaaguau uugaaaccau ccaacaaugu ucuauucucu guuggcucga 780
ccaucuacca cgagaagagg gacuuacuga ggagcuggca ccugccgucu guauuucacu 840
uacguggcaa gcaaaauuac acaugucggu gugagacuau aguuaguugc gacggguacg 900
ucguuaaaag aauagcuauc aguccaggcc uguaugggaa gccuucaggc uaugcugcua 960
cgaugcaccg cgagggauuc uugugcugca aagugacaga cacauugaac ggggagaggg 1020
ucucuuuucc cgugugcacg uaugugccag cuacauugug ugaccaaaug acuggcauac 1080
uggcaacaga ugucagugcg gacgacgcgc aaaaacugcu gguugggcuc aaccagcgua 1140
uagucgucaa cggucgcacc cagagaaaca ccaauaccau gaaaaauuac cuuuugcccg 1200
uaguggccca ggcauuugcu aggugggcaa aggaauauaa ggaagaucaa gaagaugaaa 1260
ggccacuagg acuacgagau agacaguuag ucauggggug uuguugggcu uuuagaaggc 1320
acaagauaac aucuauuuau aagcgcccgg auacccaaac caucaucaaa gugaacagcg 1380
auuuccacuc auucgugcug cccaggauag gcaguaacac auuggagauc gggcugagaa 1440
caagaaucag gaaaauguua gaggagcaca aggagccguc accucucauu accgccgagg 1500
acguacaaga agcuaagugc gcagccgaug aggcuaagga ggugcgugaa gccgaggagu 1560
ugcgcgcagc ucuaccaccu uuggcagcug auguugagga gcccacucug gaagccgaug 1620
ucgacuugau guuacaagag gcuggggccg gcucagugga gacaccucgu ggcuugauaa 1680
agguuaccag cuacgauggc gaggacaaga ucggcucuua cgcugugcuu ucuccgcagg 1740
cuguacucaa gagugaaaaa uuaucuugca uccacccucu cgcugaacaa gucauaguga 1800
uaacacacuc uggccgaaaa gggcguuaug ccguggaacc auaccauggu aaaguagugg 1860
ugccagaggg acaugcaaua cccguccagg acuuucaagc ucugagugaa agugccacca 1920
uuguguacaa cgaacgugag uucguaaaca gguaccugca ccauauugcc acacauggag 1980
gagcgcugaa cacugaugaa gaauauuaca aaacugucaa gcccagcgag cacgacggcg 2040
aauaccugua cgacaucgac aggaaacagu gcgucaagaa agaacuaguc acugggcuag 2100
ggcucacagg cgagcuggug gauccucccu uccaugaauu cgccuacgag agucugagaa 2160
cacgaccagc cgcuccuuac caaguaccaa ccauaggggu guauggcgug ccaggaucag 2220
gcaagucugg caucauuaaa agcgcaguca ccaaaaaaga ucuagugguc agcgccaaga 2280
aagaaaacug ugcagaaauu auaagggacg ucaagaaaau gaaagggcug gacgucaaug 2340
ccagaacugu ggacucagug cucuugaaug gaugcaaaca ccccguagag acccuguaua 2400

uugacgaagc uuuugcuugu caugcaggua cucucagagc gcucauagcc auuauaagac 2460
cuaaaaaggc agugcucugc ggggauccca aacagugcgg uuuuuuuaac augaugugcc 2520
ugaaagugca uuuuaaccac gagauuugca cacaagucuu ccacaaaagc aucucucgcc 2580
guugcacuaa aucugugacu ucggucgucu caaccuuguu uuacgacaaa aaaaugagaa 2640
cgacgaaucc gaaagagacu aagauuguga uugacacuac cggcaguacc aaaccuaagc 2700
aggacgaucu cauucucacu uguuucagag ggugggugaa gcaguugcaa auagauuaca 2760
aaggcaacga aauaaugacg gcagcugccu cucaagggcu gacccguaaa gguguguaug 2820
ccguucggua caaggugaau gaaaauccuc uguacgcacc caccucagaa caugugaacg 2880
uccuacugac ccgcacggag gaccgcaucg uguggaaaac acuagccggc gacccaugga 2940
uaaaaacacu gacugccaag uacccuggga auuucacugc cacgauagag gaguggcaag 3000
cagagcauga ugccaucaug aggcacaucu uggagagacc ggacccuacc gacgucuucc 3060
agaauaaggc aaacguguguu ugggccaagg cuuuagugcc ggugcugaag accgcuggca 3120
uagacaugac cacugaacaa uggaacacug uggauuauuu ugaaacggac aaagcucacu 3180
cagcagagau aguauugaac caacuaugcg ugagguucuu uggacucgau cuggacuccg 3240
gucuauuuuc ugcacccacu guuccguuau ccauuaggaa uaaucacugg gauaacuccc 3300
cgucgccuaa cauguacggg cugaauaaag aaguggucccg ucagcucucu cgcagguacc 3360
cacaacugcc ucgggcaguu gccacuggaa gagucuauga caugaacacu gguacacugc 3420
gcaauuauga uccgcgcaua aaccuaguac cuguaaacag aagacugccu caugcuuuag 3480
uccuccacca uaaugaacac ccacagagug acuuuucuuc auucgucagc aaauugaagg 3540
gcagaacugu ccuggugguc ggggaaaagu uguccguccc aggcaaaaug guugacuggu 3600
ugucagaccg gccugaggcu accuucagag cucggcugga uuuaggcauc ccaggugaug 3660
ugcccaaaua ugacauaaua uuuguuaaug ugaggacccc auauaaauac caucacuauc 3720
agcagugugua agaccaugcc auuaagcuua gcauguugac caagaaagcu ugucugcauc 3780
ugaaucccgg cggaaccugu gucagcauag guuaugguua cgcugacagg gccagcgaaa 3840
gcaucauugg ugcuauagcg cggcaguuca aguuuucccg gguaugcaaa ccgaaauccu 3900
cacuugaaga gacggaaguu cuguuuguau ucauugggua cgaucgcaag gcccguacgc 3960
acaauccuua caagcuuuca ucaaccuuga ccaacauuua uacagguucc agacuccacg 4020
aagccggaug ugcacccuca uaucaugugg ugcgagggga uauugccacg gccaccgaag 4080
gagugauuau aaaugcugcu aacagcaaag gacaaccugg cggagggggug ugcggagcgc 4140
uguauaagaa auucccggaa agcuucgauu uacagccgau cgaaguagga aaagcgcgac 4200
uggucaaagg ugcagcuaaa cauaucauuc augccguagg accaaacuuc aacaaaguuu 4260

cggagguuga aggugacaaa caguuggcag aggcuuauga guccaucgcu aagauuguca 4320
acgauaacaa uuacaaguca guagcgauuc cacuguuguc caccggcauc uuuuccggga 4380
acaaagaucg acuaacccaa ucauugaacc auuugcugac agcuuuagac accacugaug 4440
cagauguagc cauauacugc agggacaaga aaugggaaau gacucucaag gaagcagugg 4500
cuaggagaga agcaguggag gagauaugca uauccgacga cucuucagug acagaaccug 4560
augcagagcu ggugagggug cauccgaaga guucuuuggc uggaaggaag ggcuacagca 4620
caagcgaugg caaaacuuuc ucauauuugg aagggaccaa guuucaccag gcggccaagg 4680
auauagcaga aauuaaugcc auguggcccg uugcaacgga ggccaaugag cagguaugca 4740
uguauauccu cggagaaagc augagcagua uuaggucgaa augccccguc gaagagucgg 4800
aagccuccac accaccuagc acgcugccuu gcuugugcau ccaugccaug acuccagaaa 4860
gaguacagcg ccuaaaagcc ucacguccag aacaaauuac ugugugcuca uccuuuccau 4920
ugccgaagua uagaaucacu ggugugcaga agauccaaug cucccagccu auauuguucu 4980
caccgaaagu gccugcguau auucauccaa ggaaguaucu cguggaaaca ccaccgguag 5040
acgagacucc ggagccaucg gcagagaacc aauccacaga ggggacaccu gaacaaccac 5100
cacuuauaac cgaggaugag accaggacua gaacgccuga gccgaucauc aucgaagagg 5160
aagaagagga uagcauaagu uugcugucag auggcccgac ccaccaggug cugcaagucg 5220
aggcagacau ucacgggccg cccucuguau cuagcucauc cugguccauu ccucaugcau 5280
ccgacuuuga uguggacagu uuauccauac uugacacccu ggagggagcu agcgugacca 5340
gcggggcaac gucagccgag acuaacucuu acuucgcaaa gaguauggag uuucuggcgc 5400
gaccggugcc ugcgccucga acaguauuca ggaacccucc acaucccgcu ccgcgcacaa 5460
gaacaccguc acuugcaccc agcagggccu gcucgagaac cagccuaguu uccaccccgc 5520
caggcgugaa uagggugauc acuagagagg agcucgaggc gcuuaccccg ucacgcacuc 5580
cuagcagguc ggucucgaga accagccugg ucuccaaccc gccaggcgua aauaggguga 5640
uuacaagaga ggaguuugag gcguucguag cacaacaaca augacgguuu gaugcgggug 5700
cauacaucuu uuccuccgac accggucaag ggcauuuaca acaaaaauca guaaggcaaa 5760
cggugcuauc cgaaguggug uuggagagga ccgaauugga gauuucguau gccccgcgcc 5820
ucgaccaaga aaaagaagaa uuacuacgca agaaauuaca guuaaauccc acaccugcua 5880
acagaagcag auaccagucc aggaaggugg agaacaugaa agccauaaca gcuagacgua 5940
uucugcaagg ccuagggcau uauuugaagg cagaaggaaa aguggagugc uaccgaaccc 6000
ugcauccugu ucccuuugua ucaucuagug ugaaccgugc cuuuucaagc cccaaggucg 6060
caguggaagc cuguaacgcc auguugaaag agaacuuucc gacuguggcu ucuuacugua 6120
uuauuccaga guacgaugcc uauuuggaca ugguugacgg agcuucaugc ugcuuagaca 6180

cugccaguuu uugcccugca aagcugcgca gcuuuccaaa gaaacacucc uauuuggaac 6240

ccacaauacg aucggcagug ccuucagcga uccagaacac gcuccagaac guccuggcag 6300

cugccacaaa aagaaauugc aaugucacgc aaaugagaga auugcccgua uuggauucgg 6360

cggccuuuaa uguggaaugc uucaagaaau augcguguaa uaaugaauau ugggaaacgu 6420

uuaaagaaaa ccccaucagg cuuacugaag aaaacguggu aaauuacauu accaaauuaa 6480

aaggaccaaa agcugcugcu cuuuuugcga agacacauaa uuugaauaug uugcaggaca 6540

uaccaaugga cagguuugua auggacuuaa agagagacgu gaaagugacu ccaggaacaa 6600

aacauacuga agaacggccc aagguacagg ugauccaggc ugccgauccg cuagcaacag 6660

cguaucugug cggaauccac cgagagcugg uuaggagauu aaaugcgguc cugcuuccga 6720

acauucauac acuguuugau augucggcug aagacuuuga cgcuauuaua gccgagcacu 6780

uccagccugg ggauuguguu cuggaaacug acaucgcguc guuugauaaa agugaggacg 6840

acgccauggc ucugaccgcg uuaaugauuc uggaagacuu aggugugggac gcagagcugu 6900

ugacgcugau ugaggcggcu uucggcgaaa uuucaucaau acauuugccc acuaaaacua 6960

aauuuaaauu cggagccaug augaaaucug gaauguuccu cacacuguuu gugaacacag 7020

ucauuaacau uguaaucgca agcagagugu ugagagaacg gcuaaccgga ucaccaugug 7080

cagcauucau uggagaugac aauaucguga aaggagucaa aucggacaaa uuaauggcag 7140

acaggugcgc caccugguug aauauggaag ucaagauuau agaugcugug gugggcgaga 7200

aagcgccuua uuucugugga ggguuuauuu ugugugacuc cgugaccggc acagcgugcc 7260

guguggcaga cccccuaaaa aggcuguuua agcuuggcaa accucuggca gcagacgaug 7320

aacaugauga ugacaggaga agggcauugc augaagaguc aacacgcugg aaccgagugg 7380

guauucuuuc agagcugugc aaggcaguag aaucaaggua ugaaaccgua ggaacuucca 7440

ucauaguuau ggccaugacu acucuagcua gcaguguuaa aucauucagc uaccugagag 7500

gggccccuau aacucucuac ggcuaaccug aauggacuac gacauagucu aguccgccaa 7560

g 7561

<210> 135
<211> 186
<212> RNA
<213> Artificial

<220>
<223> 3′ region of SEQ ID NO:133

<400> 135
ucuagacggc gcgcccaccc agcggccgca uacagcagca auuggcaagc ugcuuacaua 60

gaacucgcgg cgauuggcau gccgccuuaa aauuuuuauu uuauuuuucu uuucuuuucc 120

```
gaaucggauu uuguuuuuaa uauuucaaaa aaaaaaaaaa aaaaaaaaaa aaaaaaaaaa      180

aaaaaa                                                                  186
```